(19) Europäisches Patentamt
European Patent Office
Office européen des brevets

(11) EP 4 582 427 A1

(12) **EUROPEAN PATENT APPLICATION**
published in accordance with Art. 153(4) EPC

(43) Date of publication:
**09.07.2025 Bulletin 2025/28**

(21) Application number: **23859498.0**

(22) Date of filing: **01.09.2023**

(51) International Patent Classification (IPC):
***C07D 487/04*** *(2006.01)* ***C07D 513/14*** *(2006.01)*
***C07D 519/00*** *(2006.01)* ***A61P 35/00*** *(2006.01)*
***A61K 31/519*** *(2006.01)* ***A61K 31/52*** *(2006.01)*
***A61K 45/06*** *(2006.01)*

(52) Cooperative Patent Classification (CPC):
**A61K 31/519; A61K 31/52; A61K 45/06; A61P 35/00; C07D 487/04; C07D 513/14; C07D 519/00**

(86) International application number:
**PCT/CN2023/116528**

(87) International publication number:
**WO 2024/046471 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**02.09.2022 CN 202211069731**
**18.10.2022 CN 202211272140**
**18.01.2023 CN 202310084635**
**18.04.2023 CN 202310416682**
**25.06.2023 CN 202310755391**
**25.08.2023 CN 202311082622**

(71) Applicant: **Shanghai Qilu Pharmaceutical Research and Development Centre Ltd.**
**Shanghai 201203 (CN)**

(72) Inventors:
- **QIN, Hua**
  **Shanghai 201203 (CN)**
- **SHI, Guqin**
  **Shanghai 201203 (CN)**
- **ZHANG, Yuxing**
  **Shanghai 201203 (CN)**
- **XU, Yiming**
  **Shanghai 201203 (CN)**
- **HAO, Junguo**
  **Shanghai 201203 (CN)**
- **CHEN, Xu**
  **Shanghai 201203 (CN)**
- **FENG, Hao**
  **Shanghai 201203 (CN)**
- **ZHU, Panhu**
  **Shanghai 201203 (CN)**
- **FU, Jiasheng**
  **Shanghai 201203 (CN)**
- **SUN, Weimei**
  **Shanghai 201203 (CN)**
- **SUN, Daqing**
  **Shanghai 201203 (CN)**

(74) Representative: **Schmitz, Joseph**
**Isler & Pedrazzini AG**
**Giesshübelstrasse 45**
**Postfach 1772**
**8027 Zürich (CH)**

(54) **USP1 INHIBITOR**

(57) The present application provides a class of novel compounds having USP1 inhibitory activity as shown in formula (II'), pharmaceutical compositions comprising the compounds, useful intermediates for preparing the compounds, and a method for treating related diseases mediated by a USP1 target by means of the compounds of the present application.

EP 4 582 427 A1

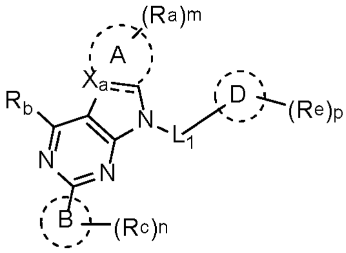

(II')

## Description

The present application claims the benefits of the priority of the Chinese patent application filed before CNIPA on September 02, 2022, with the application No. CN202211069731.2 and titled "USP1 INHIBITOR"; the Chinese patent application filed before CNIPA on October 18, 2022, with the application No. CN202211272140.5 and titled "USP1 INHIBITOR"; the Chinese patent application filed before CNIPA on January 18, 2023, with the application No. CN202310084635.3 and titled "USP1 INHIBITOR"; the Chinese patent application filed before CNIPA on April 18, 2023, with the application No. CN202310416682.3 and titled "USP1 INHIBITOR"; the Chinese patent application filed before CNIPA on June 25, 2023, with the application No. CN202310755391.7 and titled "USP1 INHIBITOR"; and the Chinese patent application filed before CNIPA on August 25, 2023, with the application No. CN202311082622.9 and titled "USP1 INHIBITOR"; which are hereby incorporated in their entirety by reference.

## FIELD OF THE INVENTION

The present application relates to the field of medicinal chemistry, and involves a novel compound having USP1 inhibitory activity, a pharmaceutical composition comprising the compound, a useful intermediate for preparing the compound, and a method for treating related diseases mediated by a USP1 target by using the compound of the present application.

## BACKGROUND OF THE INVENTION

Ubiquitin is a small, highly conserved protein consisting of 76 amino acids, which attaches to a substrate protein (including itself) in a three-step enzymatic reaction. Initial covalent attachment occurs primarily between a C-terminal glycine of ubiquitin and a ε-amino group of a lysine residue of a target protein. Additional ubiquitin molecules can be attached to one of the seven internal lysines of ubiquitin, leading to different ubiquitin chain topologies. Biological outcome of ubiquitination is determined by length and connection topology. Similar to other types of post-translational modifications, ubiquitination is a reversible process that is counter-regulated by enzymes called deubiquitinating enzymes (DUBs), which catalyze the removal of ubiquitin from modified proteins. More importantly, dysfunction of ubiquitin-dependent signaling pathway is associated with a variety of human diseases, suggesting that inhibition of ubiquitin pathway is a novel therapeutic target for drug development.

The ubiquitin-proteasome system offers additional opportunities for therapeutic interventions, which may increase specificity and the potential for improved clinical efficacy. Most obvious targets include enzymes involved in ubiquitin conjugation and de-conjugation (i.e. ubiquitin ligases and DUBs), which are upstream processes of proteasome-mediated protein degradation. Among various DUBs, ubiquitin-specific protease 1 (USP1) is an attractive anticancer target due to its involvement in the regulation of DNA damage response pathway. USP1 binds to USP1-associated factor 1 (UAF1) to produce a heterodimeric USP1/UAF1 complex required for the activity of deubiquitinating enzymes. The USP1/UAF1 complex has been shown to modulate tolerance to DNA damage induced by a DNA cross-linking agent through deubiquitination of proliferating cell nuclear antigen (PCNA) 11 and Fanconi anemia complementation group D2 (FANCD2), which are proteins involved in translesion synthesis and the Fanconi anemia pathway, respectively.

There are currently many studies focusing on this mechanism, but there is no USP1 inhibitor on the market and there is an urgent need to develop an effective USP1 inhibitor for clinical patients.

## SUMMARY OF THE INVENTION

In a first aspect, the present application provides a compound represented by formula (II'), or an isomer thereof or a pharmaceutically acceptable salt thereof,

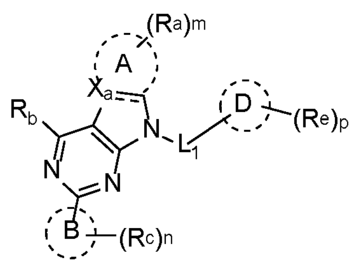

(II')

wherein,

$X_a$ is C or N;
ring A is phenyl, 5-6 membered heteroaryl, $C_{5-6}$ cycloalkyl, or 5-6 membered heterocyclyl; $R_a$ are each independently deuterium, halogen, $C_{1-4}$ alkyl, $C_{1-4}$ haloalkyl, $C_{1-4}$ alkoxy, oxo, cyano, amino, hydroxy, aminosulfonyl, $C_{1-4}$ alkylsulfonyl, carbamoyl, $C_{1-4}$ alkylamino, $C_{3-6}$ cycloalkyl, $C_{1-4}$ alkylsulfonylamino, dimethylphosphonoyl, -$C_{1-4}$ alkyl-OH, -$COOC_{1-4}$ alkyl, $C_{1-4}$ alkyl-$SO_2$-$NR_f$-, HO-$C_{1-4}$ alkyl-$SO_2$-$NR_f$-, 3-6 membered heterocyclyl, -$C_{1-4}$ haloalkyl-OH, ($C_{1-4}$ alkyl)$_2$P(O)-,

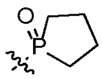 , 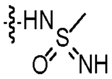 , 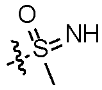 , 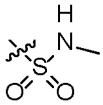 ,

deuterated $C_{1-4}$ alkyl, or 4-6 membered heterocycloalkyl; wherein C atom(s) in the $C_{1-4}$ alkyl and $C_{1-4}$ haloalkyl is/are optionally substituted with N or O; $R_f$ is $C_{3-6}$ cycloalkyl or $C_{1-4}$ alkyl; m is 0, 1, 2, 3, or 4;
$R_b$ is H or $C_{1-4}$ alkyl;
ring B is phenyl, 5-10 membered heteroaryl, or 5-10 membered heterocyclyl; $R_c$ are each independently halogen, $C_{1-4}$ alkyl, $C_{1-4}$ alkoxy, $C_{3-6}$ cycloalkyl, $C_{1-4}$ haloalkyl, $C_{1-4}$ haloalkoxy, or deuterated $C_{1-4}$ alkyl; n is 0, 1, 2, 3, or 4;
ring D is phenyl, 5-6 membered heteroaryl, or 9-18 membered fused-heterocyclyl; $R_e$ is deuterium, $C_{1-4}$ alkyl, $C_{1-4}$ haloalkyl, $C_{1-4}$ alkoxy, ring C, or halogen, wherein ring C is optionally substituted with one $R_d$; ring C is 5-10 membered heteroaryl comprising 1 to 4 N atom(s) or 8-10 membered fused-heterocyclyl comprising 1 to 4 N atom(s); $R_d$ are each independently $C_{1-4}$ alkyl, $C_{1-4}$ haloalkyl, $C_{1-4}$ alkoxy, deuterated $C_{1-4}$ alkyl, or $C_{3-6}$ cycloalkyl; 1 is 1, 2, 3, or 4; p is 1, 2, 3, or 4;
$L_1$ is $C_{1-4}$ alkylene, $C_{3-6}$ cycloalkylene or a chemical bond;
when ring A is 5-6 membered heteroaryl, structural unit

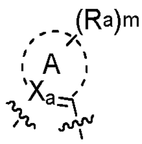

is not

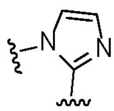 ,

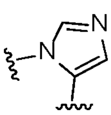 , 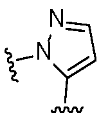 , 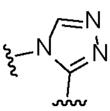 , 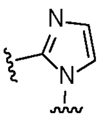 , 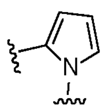 , 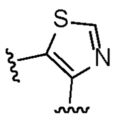 , 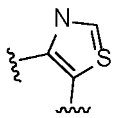 , or 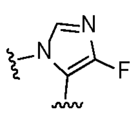 ;

and
when ring A is 5-6 membered heterocyclyl, structural unit

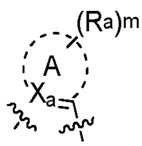

is not

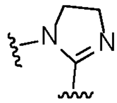.

In some embodiments of the present application, in the compound represented by formula (II'), or the isomer thereof or the pharmaceutically acceptable salt thereof, ring D is

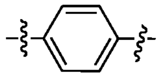,

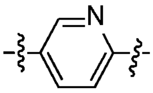, 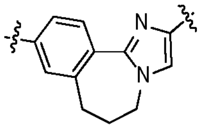, 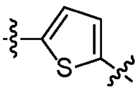, 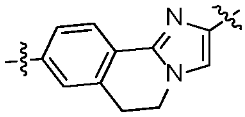, 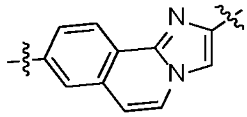,

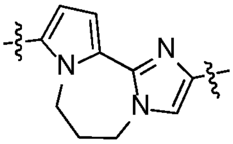, 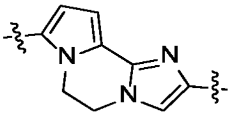, or 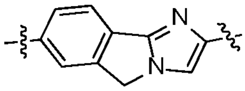.

In some embodiments of the present application, in the compound represented by formula (II'), or the isomer thereof or the pharmaceutically acceptable salt thereof, ring D is

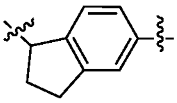, 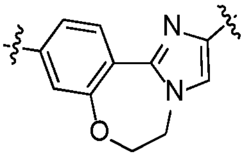, 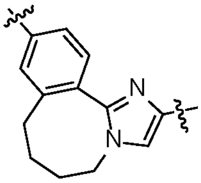, 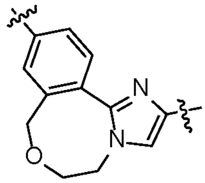, 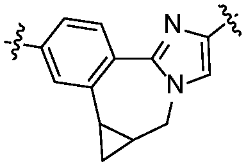,

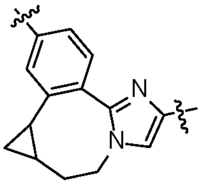, 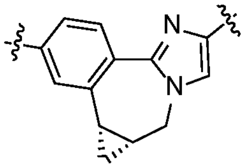, or 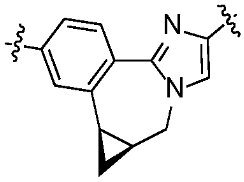.

In some embodiments of the present application, in the compound represented by formula (II'), or the isomer thereof or the pharmaceutically acceptable salt thereof, ring D is

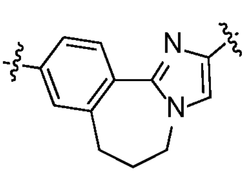 or 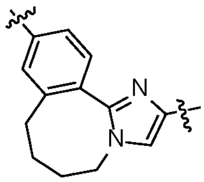.

In some embodiments of the present application, in the compound represented by formula (II'), or the isomer thereof or the pharmaceutically acceptable salt thereof, $R_e$ is $-CF_3$,

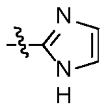

or

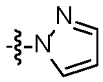

In some embodiments of the present application, in the compound represented by formula (II'), or the isomer thereof or the pharmaceutically acceptable salt thereof, $R_e$ is $-OCH_3$, -F, -D, or $-CH_3$.

In some embodiments of the present application, in the compound represented by formula (II'), or the isomer thereof or the pharmaceutically acceptable salt thereof, $R_e$ is $-CF_3$.

The present application further provides a compound represented by formula (I'), or an isomer thereof or a pharmaceutically acceptable salt thereof:

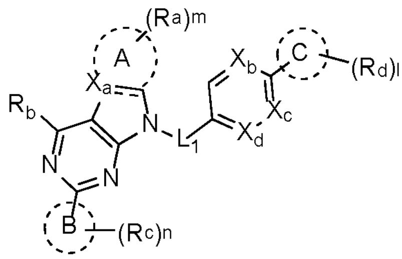

(I')

wherein,

$X_a$ is C or N;

$X_b$, $X_c$, and $X_d$ are each independently CH, N, or CR', wherein R' is halogen, $C_{1-4}$ alkyl, $C_{1-4}$ haloalkyl, or $C_{1-4}$ alkoxy; ring A is phenyl, 5-6 membered heteroaryl, $C_{5-6}$ cycloalkyl, or 5-6 membered heterocyclyl; $R_a$ are each independently deuterium, halogen, $C_{1-4}$ alkyl, $C_{1-4}$ haloalkyl, $C_{1-4}$ alkoxy, oxo, cyano, amino, hydroxy, aminosulfonyl, $C_{1-4}$ alkylsulfonyl, carbamoyl, $C_{1-4}$ alkylamino, $C_{3-6}$ cycloalkyl, $C_{1-4}$ alkylsulfonylamino, dimethylphosphonoyl, $-C_{1-4}$ alkyl-OH, $-COOC_{1-4}$ alkyl, $C_{1-4}$ alkyl-$SO_2$-$NR_f$-, HO-$C_{1-4}$ alkyl-$SO_2$-$NR_f$-, 3-6 membered heterocyclyl, $-C_{1-4}$ haloalkyl-OH, ($C_{1-4}$ alkyl)$_2$P(O)-,

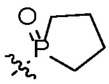, 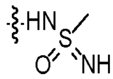, 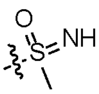, or 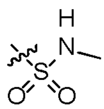;

wherein C atom(s) in the $C_{1-4}$ alkyl and $C_{1-4}$ haloalkyl is/are optionally substituted with N or O; $R_f$ is $C_{3-6}$ cycloalkyl or $C_{1-4}$ alkyl;

m is 0, 1, 2, 3, or 4;

$R_b$ is H or $C_{1-4}$ alkyl;

ring B is phenyl, 5-10 membered heteroaryl, or 5-10 membered heterocyclyl; $R_c$ are each independently halogen, $C_{1-4}$ alkyl, $C_{1-4}$ alkoxy, $C_{3-6}$ cycloalkyl, $C_{1-4}$ haloalkyl, or $C_{1-4}$ haloalkoxy; n is 0, 1, 2, 3, or 4;

ring C is 5-10 membered heteroaryl comprising 1 to 4 N atom(s); $R_d$ are each independently $C_{1-4}$ alkyl, $C_{1-4}$ haloalkyl, $C_{1-4}$ alkoxy, or deuterated $C_{1-4}$ alkyl; 1 is 0, 1, 2, 3, or 4; and

$L_1$ is $C_{1-4}$ alkylene, $C_{3-6}$ cycloalkylene or a chemical bond.

When ring A is 5-6 membered heteroaryl, structural unit

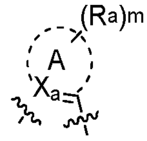

is not

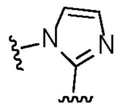,

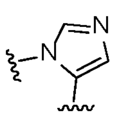, 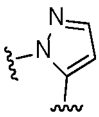, 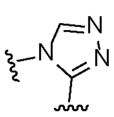, 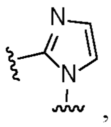, 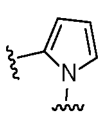, 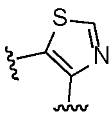, 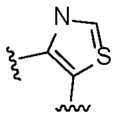, or 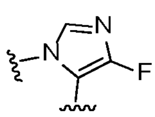;

and

when ring A is 5-6 membered heterocyclyl, structural unit

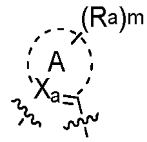

is not

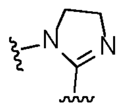.

The present application further provides a compound represented by formula (I'-1), or an isomer thereof or a pharmaceutically acceptable salt thereof,

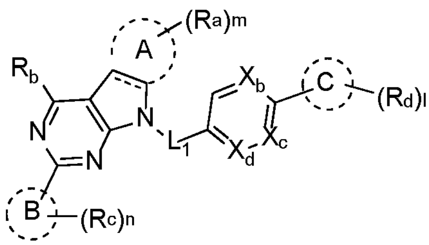

(I'-1)

wherein,

$X_b$, $X_c$, and $X_d$ are each independently CH, N, or CR', wherein R' is halogen or $C_{1-4}$ alkoxy;
ring A is phenyl, pyridinyl, or $C_{5-6}$ cycloalkyl; $R_a$ are each independently halogen, $C_{1-4}$ alkyl, $C_{1-4}$ haloalkyl, $C_{1-4}$ alkoxy, oxo, cyano, amino, hydroxy, aminosulfonyl, $C_{1-4}$ alkylsulfonyl, carbamoyl, $C_{1-4}$ alkylamino, $C_{3-6}$ cycloalkyl, $C_{1-4}$ alkylsulfonylamino, dimethylphosphonoyl, -$C_{1-4}$ alkyl-OH, -$COOC_{1-4}$ alkyl, $C_{1-4}$ alkyl-$SO_2$-$NR_f$-, HO-$C_{1-4}$ alkyl-$SO_2$-$NR_f$-, 3-6 membered heterocyclyl, -$C_{1-4}$ haloalkyl-OH, $(C_{1-4}$ alkyl$)_2P(O)$-,

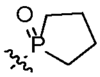, 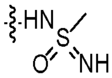, 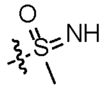, or 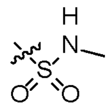;

wherein C atom(s) in the $C_{1-4}$ alkyl and $C_{1-4}$ haloalkyl is/are optionally substituted with N or O; $R_f$ is $C_{3-6}$ cycloalkyl or $C_{1-4}$ alkyl; m is 0, 1, 2, 3, or 4;

$R_b$ is H or $C_{1-4}$ alkyl;

ring B is 5-6 membered heteroaryl or 5-10 membered heterocyclyl; $R_c$ are each independently $C_{1-4}$ alkyl, $C_{1-4}$ alkoxy, $C_{3-6}$ cycloalkyl, or halogen; n is 0, 1, 2, 3, or 4;

ring C is 5-6 membered heteroaryl comprising 1 to 4 N atom(s); $R_d$ are each independently $C_{1-4}$ alkyl, $C_{1-4}$ haloalkyl, or $C_{1-4}$ alkoxy; 1 is 0, 1, 2, 3, or 4; and

$L_1$ is $C_{1-4}$ alkylene or $C_{3-6}$ cycloalkylene.

In some embodiments of the present application, provided is a compound represented by formula (I'-1), or an isomer thereof or a pharmaceutically acceptable salt thereof,

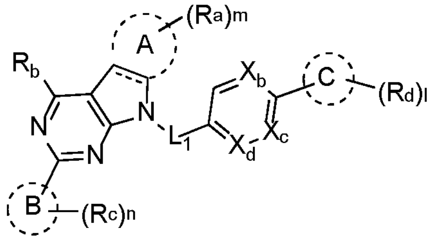

(I'-1)

wherein,

$X_b$, $X_c$, and $X_d$ are each independently CH, N, or CR', wherein R' is halogen or $C_{1-4}$ alkoxy;

ring A is phenyl, pyridinyl, or $C_{5-6}$ cycloalkyl; $R_a$ are each independently deuterium, halogen, $C_{1-4}$ alkyl, $C_{1-4}$ haloalkyl, $C_{1-4}$ alkoxy, cyano, amino, aminosulfonyl, methylsulfonyl, -COO$C_{1-4}$ alkyl, -$C_{1-4}$ alkyl-OH, or carbamoyl; m is 0, 1, 2, 3, or 4;

$R_b$ is H or $C_{1-4}$ alkyl;

ring B is 5-6 membered heteroaryl; $R_c$ are each independently $C_{1-4}$ alkyl, $C_{1-4}$ alkoxy, $C_{3-6}$ cycloalkyl, or halogen; n is 0, 1, 2, 3, or 4;

ring C is 5-6 membered heteroaryl comprising 1 to 4 N atom(s); $R_d$ are each independently $C_{1-4}$ alkyl, $C_{1-4}$ haloalkyl, or $C_{1-4}$ alkoxy; 1 is 0, 1, 2, 3, or 4; and

$L_1$ is $C_{1-4}$ alkylene or $C_{3-6}$ cycloalkylene.

In some embodiments of the present application, in the compound represented by formula (II'), (I'), or (I'-1), or the isomer thereof or the pharmaceutically acceptable salt thereof, ring A is phenyl or 5-6 membered heteroaryl.

In some embodiments of the present application, in the compound represented by formula (II'), (I'), or (I'-1), or the isomer thereof or the pharmaceutically acceptable salt thereof, ring A is

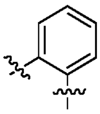, 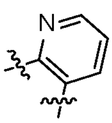, 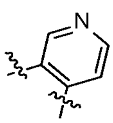, 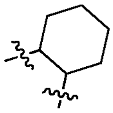, 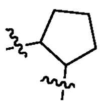, 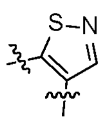, 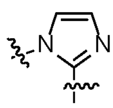, 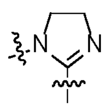, 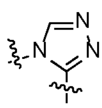,

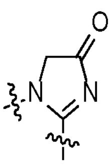, 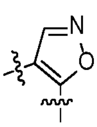, 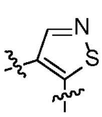, 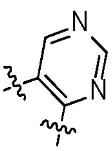, 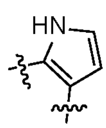, 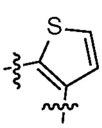, 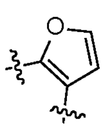, or 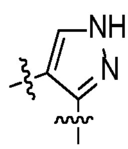.

In some embodiments of the present application, in the compound represented by formula (II'), (I'), or (I'-1), or the isomer thereof or the pharmaceutically acceptable salt thereof, ring A is

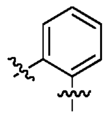, 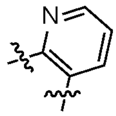, or 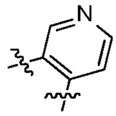.

In some embodiments of the present application, in the compound represented by formula (II'), (I'), or (I'-1), or the isomer thereof or the pharmaceutically acceptable salt thereof, ring A is

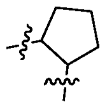.

In some embodiments of the present application, in the compound represented by formula (II'), (I'), or (I'-1), or the isomer thereof or the pharmaceutically acceptable salt thereof, ring A is

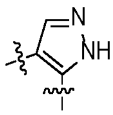 or 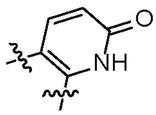.

In some embodiments of the present application, in the compound represented by formula (II'), (I'), or (I'-1), or the isomer thereof or the pharmaceutically acceptable salt thereof, ring A is

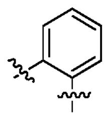, 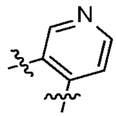, or 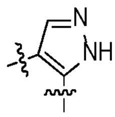.

In some embodiments of the present application, in the compound represented by formula (II'), (I'), or (I'-1), or the isomer thereof or the pharmaceutically acceptable salt thereof, R' are each independently $-OCH_3$ or -F.

In some embodiments of the present application, in the compound represented by formula (II'), (I'), or (I'-1), or the isomer thereof or the pharmaceutically acceptable salt thereof, $R_a$ are each independently -F, $-OCH_3$, $-CF_3$, $-COOCH_3$, $-C(CH_3)_2-OH$, $-CHF_2$, -CN,

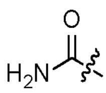, 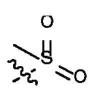,

-Cl, $-NH_2$,

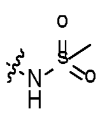, 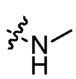,

$CH_3-NH-S(O)_2-$, or $NH_2-S(O)_2-$.

In some embodiments of the present application, in the compound represented by formula (II'), (I'), or (I'-1), or the isomer thereof or the pharmaceutically acceptable salt thereof, $R_a$ are each independently -F, $-OCH_3$ or $-CF_3$.

In some embodiments of the present application, in the compound represented by formula (II'), (I'), or (I'-1), or the isomer thereof or the pharmaceutically acceptable salt thereof, $R_a$ are each independently $-COOCH_3$, $-C(CH_3)_2-OH$, or $-CHF_2$.

In some embodiments of the present application, in the compound represented by formula (II'), (I'), or (I'-1), or the isomer thereof or the pharmaceutically acceptable salt thereof, $R_a$ are each independently $-CH_3$, -OH, -Br, $-CH_2CH_3$,

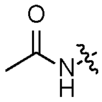, 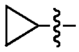, 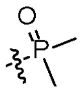, 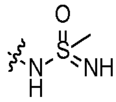,

$CH(CH_3)_2$,

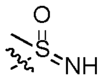, 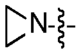, 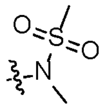, 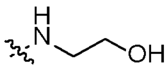, 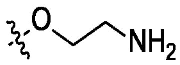, 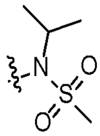, 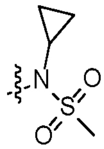,

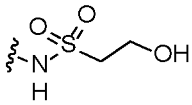, 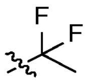, 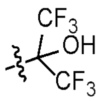, 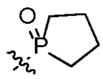, 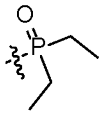,

$-OCH(CH_3)_2$,

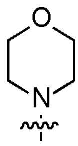,

D,

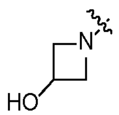,

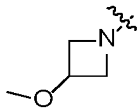,

$-CD_3$, or

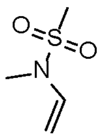.

In some embodiments of the present application, in the compound represented by formula (II') or (I'), or the isomer thereof or the pharmaceutically acceptable salt thereof, structural unit

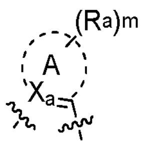

is

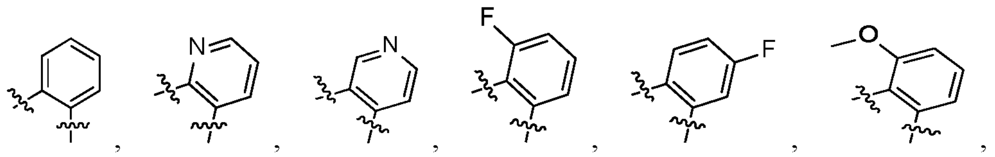

, , , , , ,

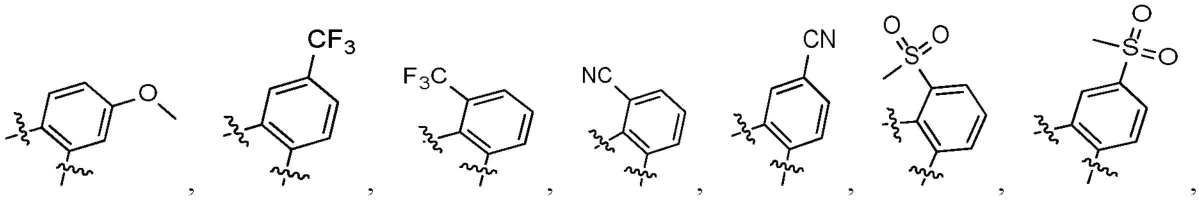

, , , , , , ,

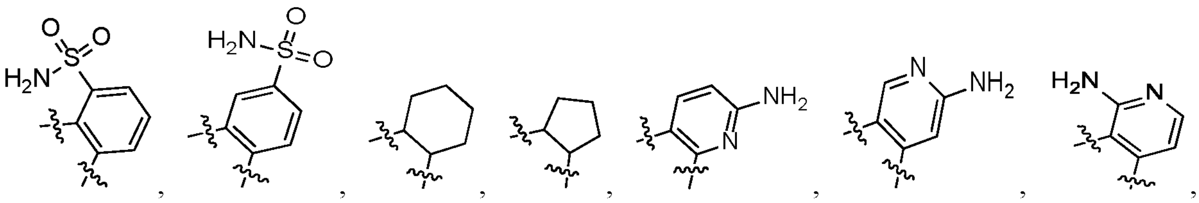

, , , , , , ,

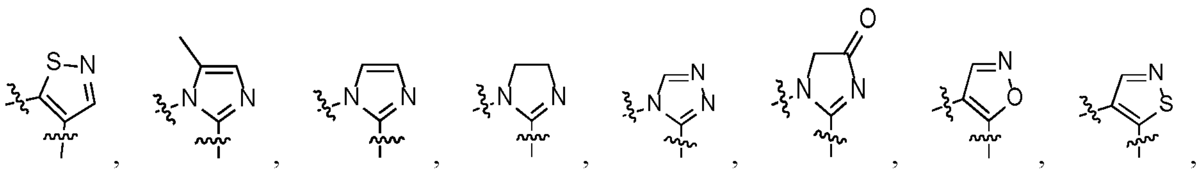

, , , , , , , ,

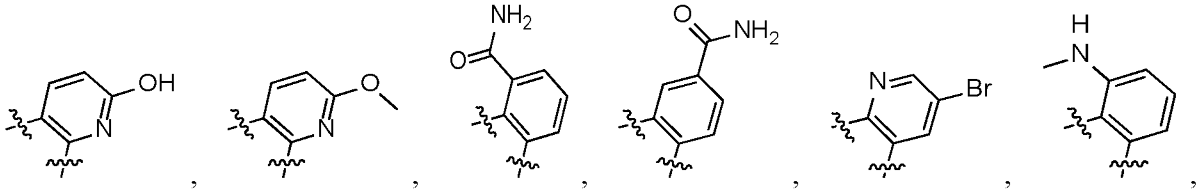

, , , , , ,

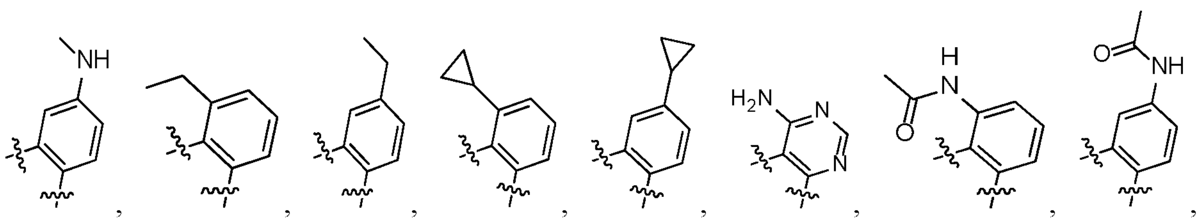

, , , , , , , ,

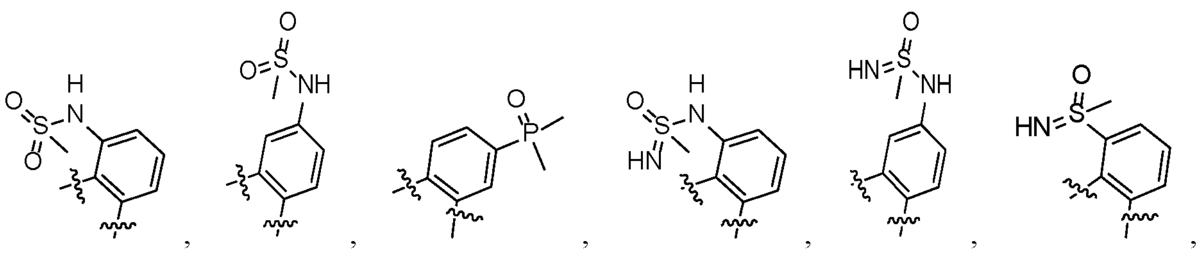

, , , , , ,

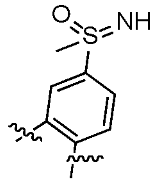 , 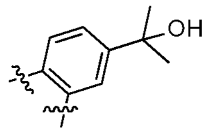 , 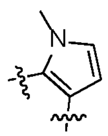 , 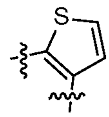 , 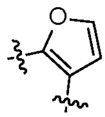 , 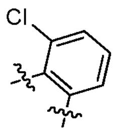 , 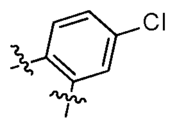 ,

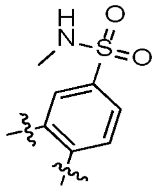 , 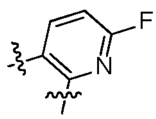 , or 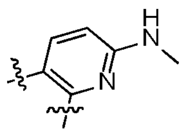 .

In some embodiments of the present application, in the compound represented by formula (II') or (I'), or the isomer thereof or the pharmaceutically acceptable salt thereof, structural unit

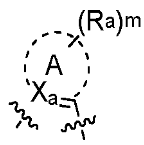

is

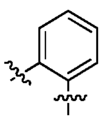 , 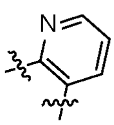 , 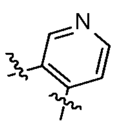 , 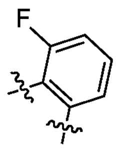 , 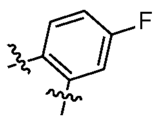 , 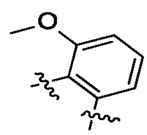 ,

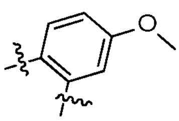 , 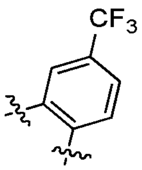 , 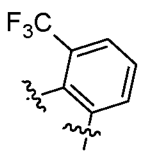 , 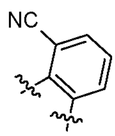 , 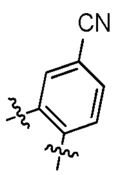 , 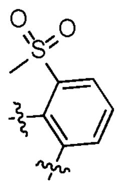 , 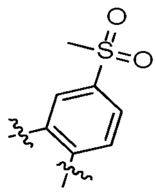 ,

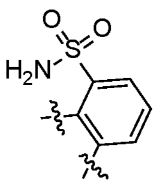 , 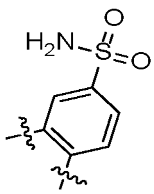 , 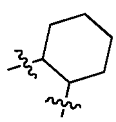 , 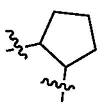 , 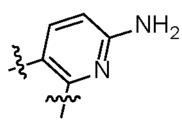 , 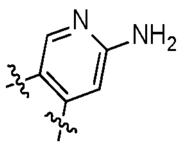 , 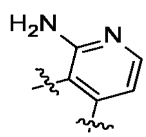 ,

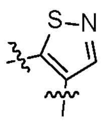 , 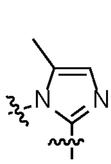 , 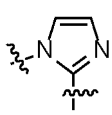 , 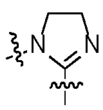 , 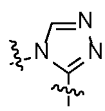 , 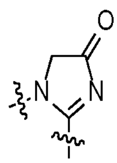 , 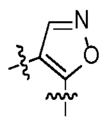 , 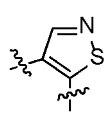 ,

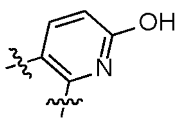 , 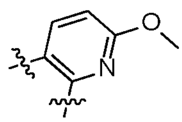 , 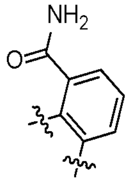 , 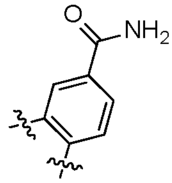 , 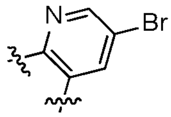 , 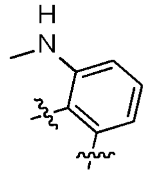 ,

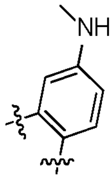 , 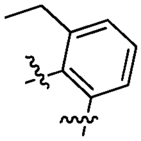 , 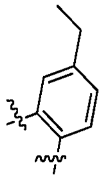 , 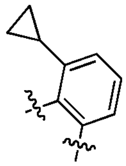 , 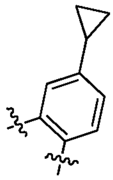 , 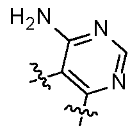 , 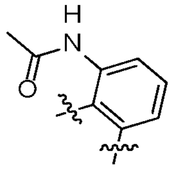 , 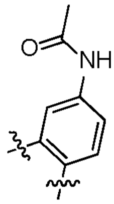 ,

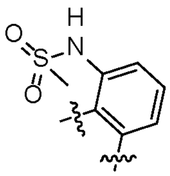 , 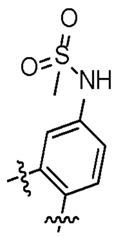 , 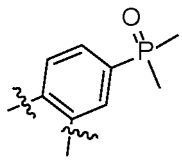 , 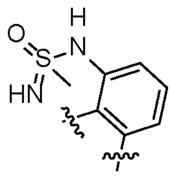 , 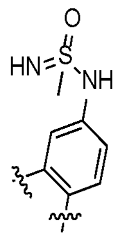 , 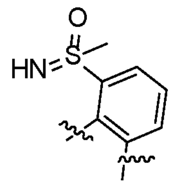 ,

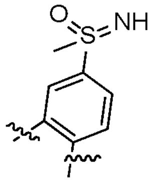 , 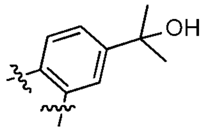 , 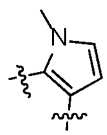 , 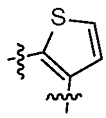 , or 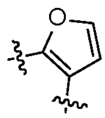 .

In some embodiments of the present application, in the compound represented by formula (II') or formula (I'), or the isomer thereof or the pharmaceutically acceptable salt thereof, structural unit

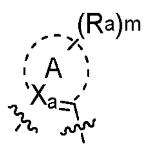

is

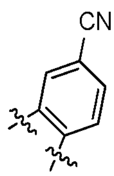 , 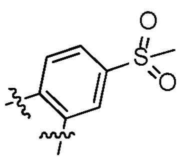 , 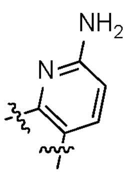 , 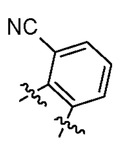 , 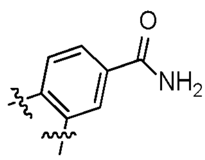 ,

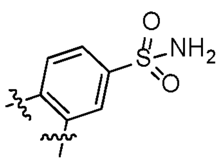 , 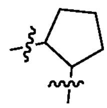 , 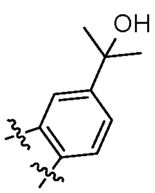 , 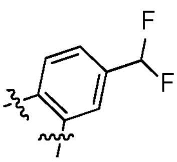 , or 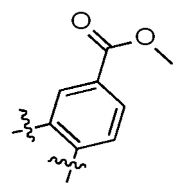 .

In some embodiments of the present application, in the compound represented by formula (II') or (I'), or the

isomer thereof or the pharmaceutically acceptable salt thereof, structural unit

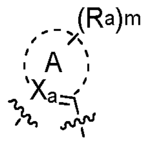

is

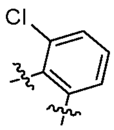 , 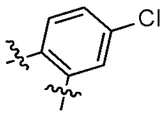 , 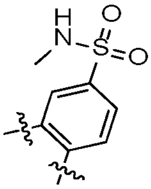 , 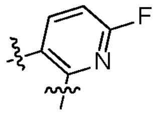 , 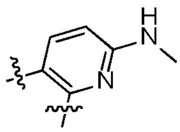 ,

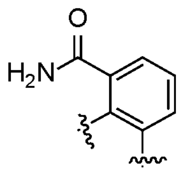 , or 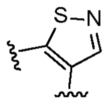 .

In some embodiments of the present application, in the compound represented by formula (II') or formula (I'), or the isomer thereof or the pharmaceutically acceptable salt thereof, structural unit

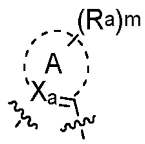

is

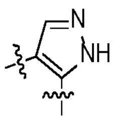 , 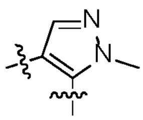 , 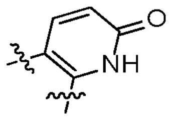 , 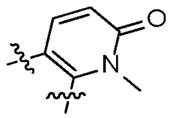 , 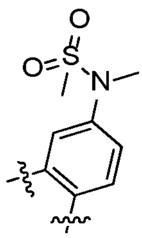 ,

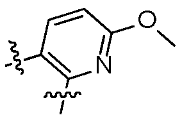 , 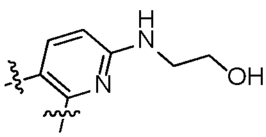 , 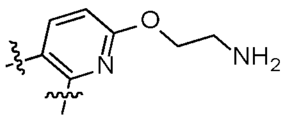 , 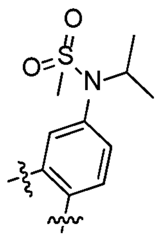 , 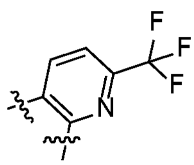 ,

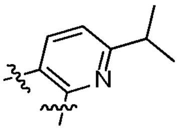, 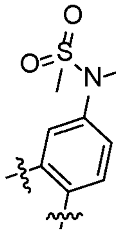, 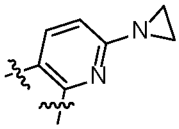, 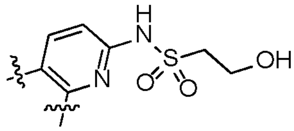, 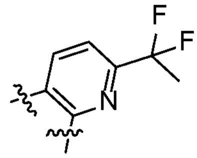,

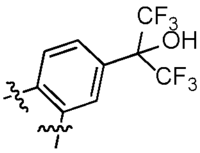, 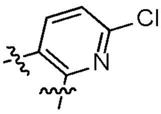, 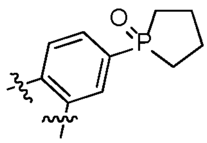, 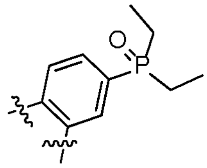, 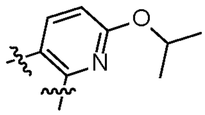,

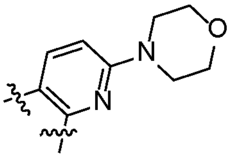, 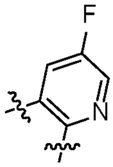, 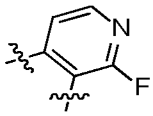, 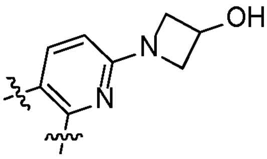, 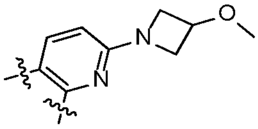,

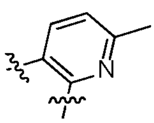, 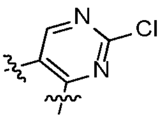, 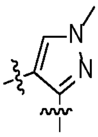, 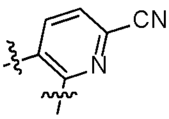, 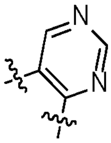, or 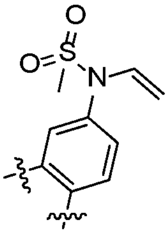.

In some embodiments of the present application, in the compound represented by formula (II') or (I'), or the isomer thereof or the pharmaceutically acceptable salt thereof, structural unit

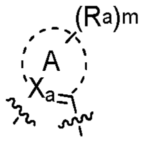

is

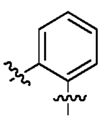, 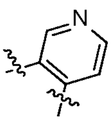, 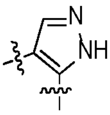, or 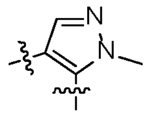.

In some embodiments of the present application, in the compound represented by formula (I'-1), or the isomer thereof or the pharmaceutically acceptable salt thereof, structural unit

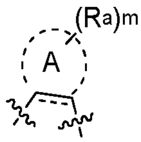

is

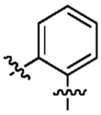, 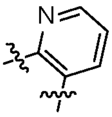, 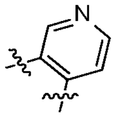, 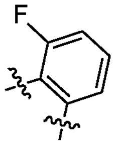, 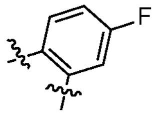, 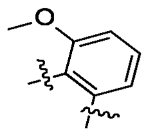,

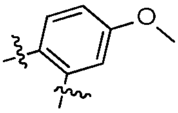, 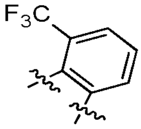, or 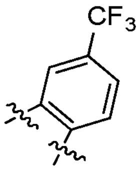.

In some embodiments of the present application, in the compound represented by formula (I'-1), or the isomer thereof or the pharmaceutically acceptable salt thereof, structural unit

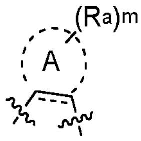

is

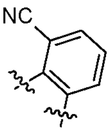, 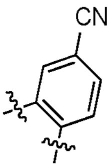, 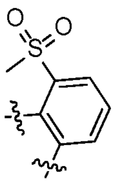, 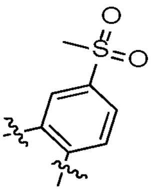, 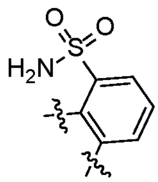, 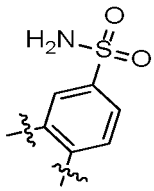,

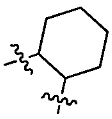, 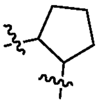, 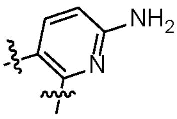, 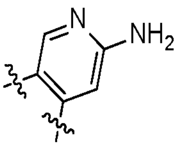, 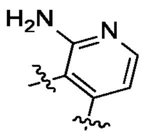, 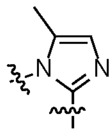, 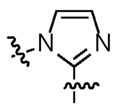,

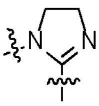, 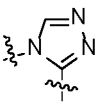, 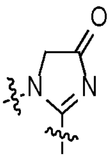, 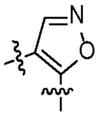, 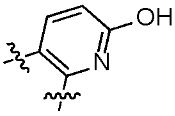, 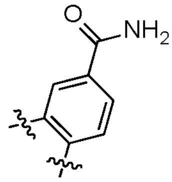, 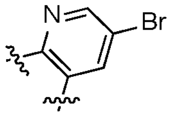,

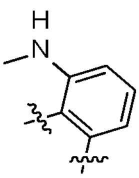, 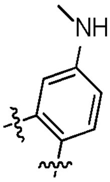, 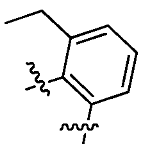, 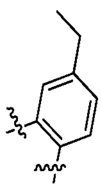, 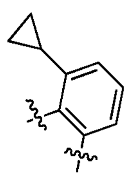, 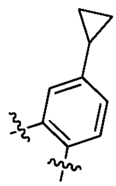, 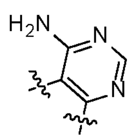,

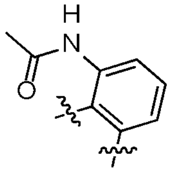, 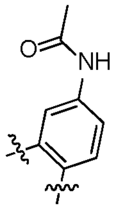, 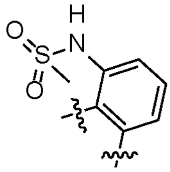, 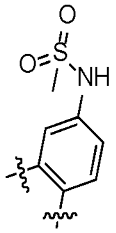, 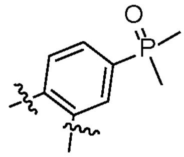, 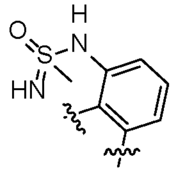, 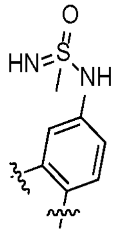,

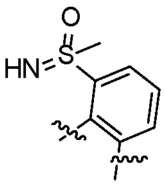, 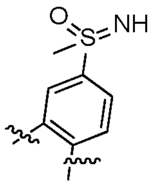, 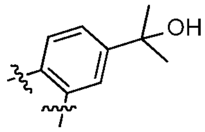, 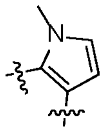, 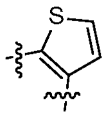, 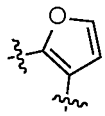, 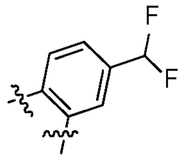,

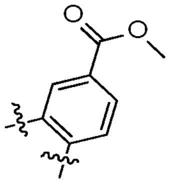, 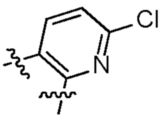, 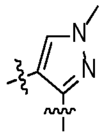, 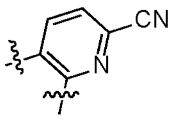, or 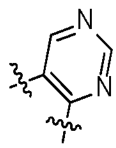.

In some embodiments of the present application, in the compound represented by formula (I'-1), or the isomer thereof or the pharmaceutically acceptable salt thereof, structural unit

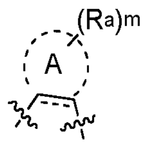

is

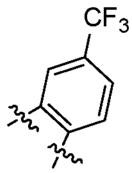, 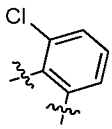, 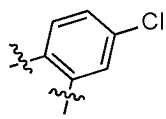, 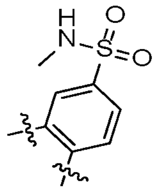, 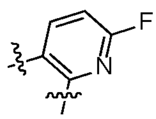,

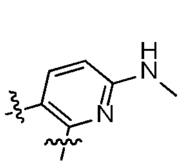, 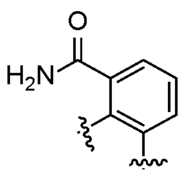, or 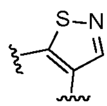.

In some embodiments of the present application, in the compound represented by formula (I'-1), or the isomer thereof or the pharmaceutically acceptable salt thereof, structural unit

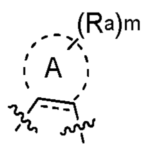

is

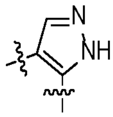, 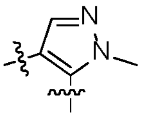, 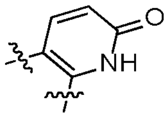, 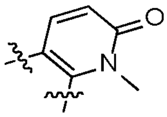, 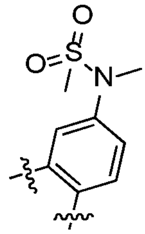,

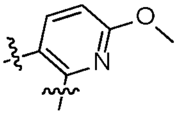, 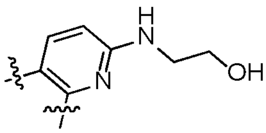, 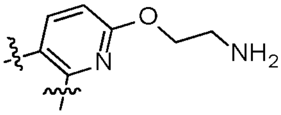, 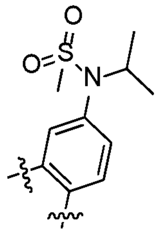, 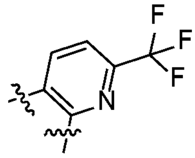,

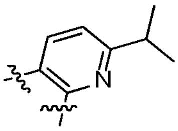, 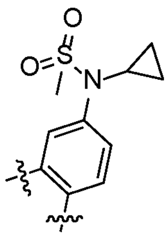, 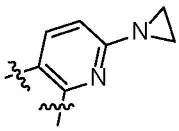, 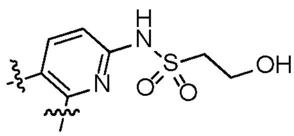, 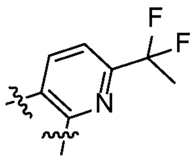,

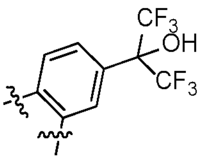, 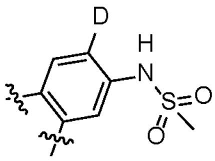, 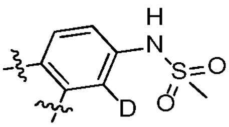, 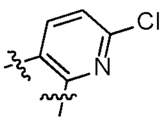, 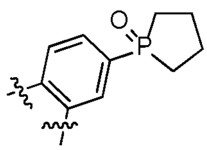,

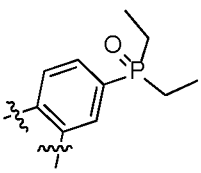, 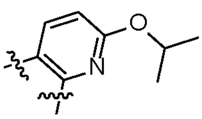, 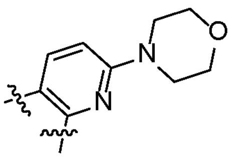, 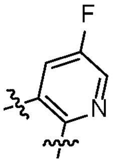, 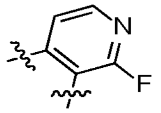,

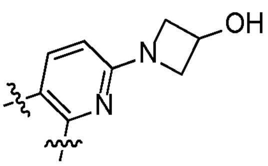, 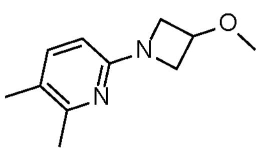, 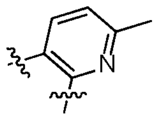, or 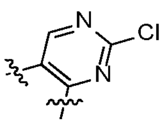.

In some embodiments of the present application, in the compound represented by formula (II') or (I'), or the isomer thereof or the pharmaceutically acceptable salt thereof, structural unit

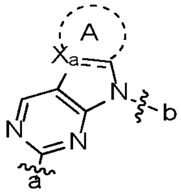

is

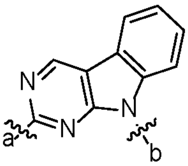, 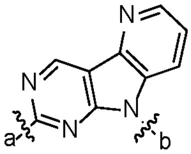, 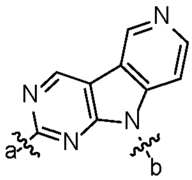, 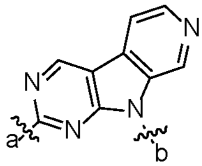,

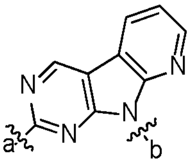, 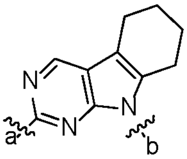, 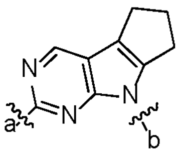, 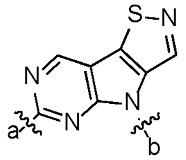, 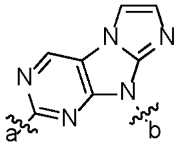,

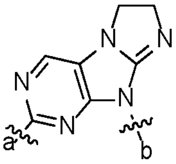, 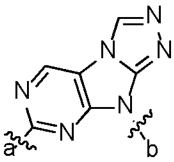, 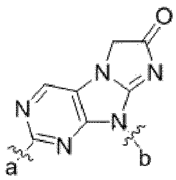, 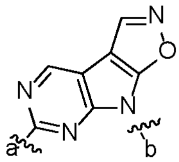, 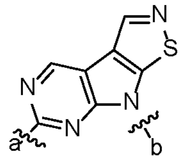,

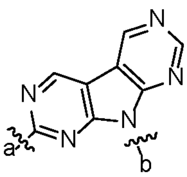, 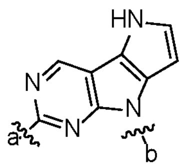, 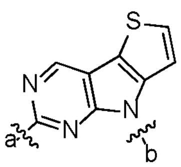, or 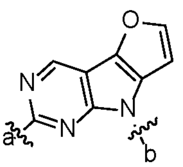,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

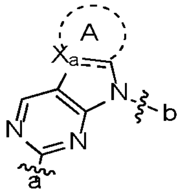

is

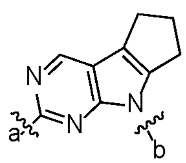,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

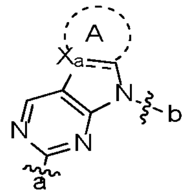

is

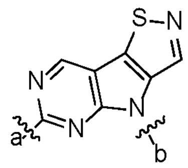

,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

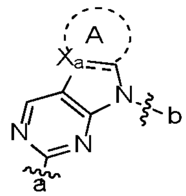

is

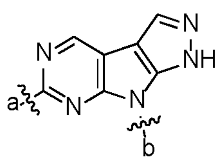

,

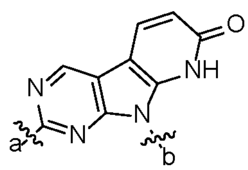

, or

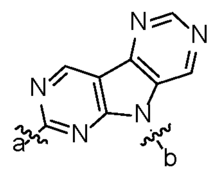

,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

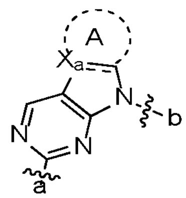

is

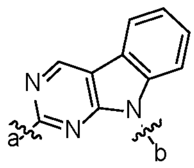, 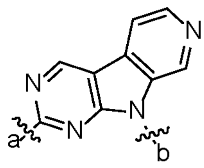, or 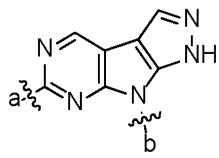,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

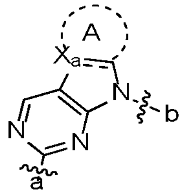

is

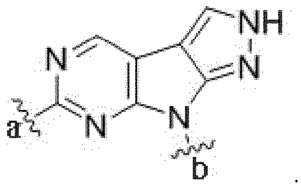.

In some embodiments of the present application, in the compound represented by formula (II') or (I'), or the isomer thereof or the pharmaceutically acceptable salt thereof, structural unit

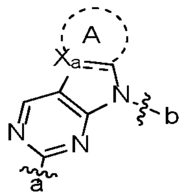

is

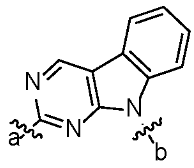, 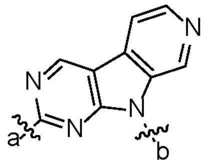, or 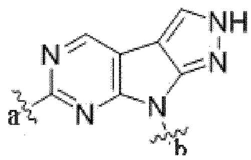,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

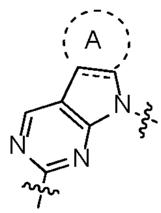

is

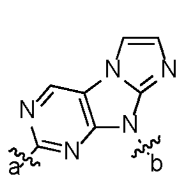, 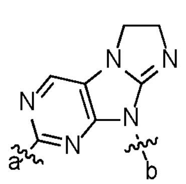, 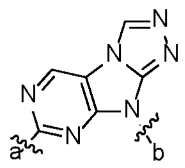, 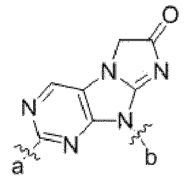,

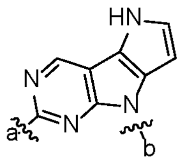, or 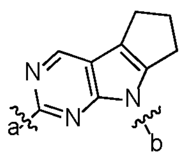,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

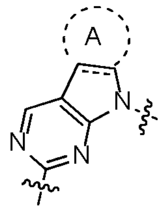

is

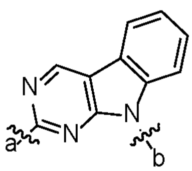, 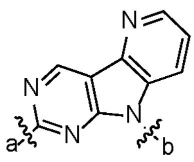, 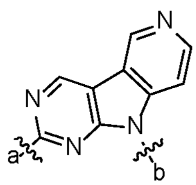, 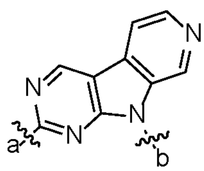,

or

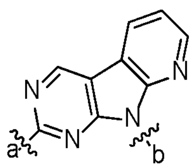,

wherein a represents the linking site with ring B, and b represents the linking site with $L_1$.

In some embodiments of the present application, in the compound represented by formula or (I'-1), or the isomer thereof or the pharmaceutically acceptable salt thereof, structural unit

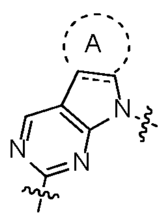

is

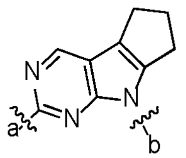

,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

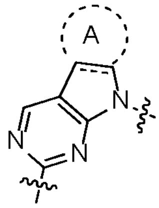

is

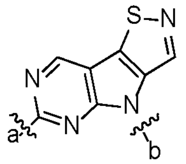

,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

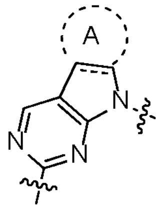

is

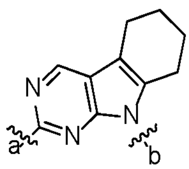 , 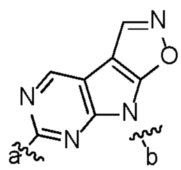 , 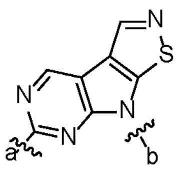 , 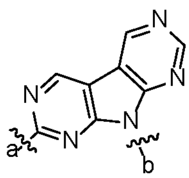 ,

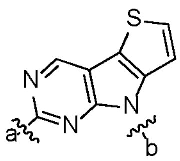 , 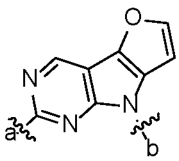 , 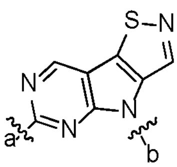 , 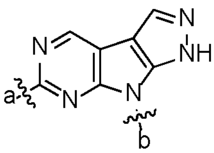 , 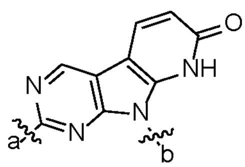 ,

or

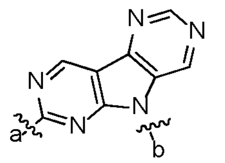

,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

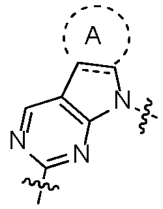

is

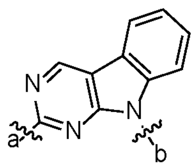

,

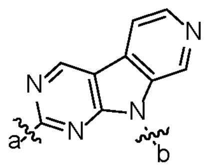

, or

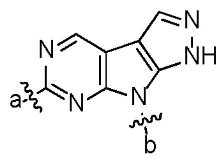

,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

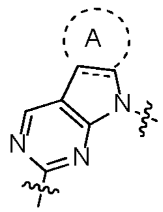

is

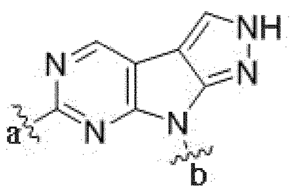

,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

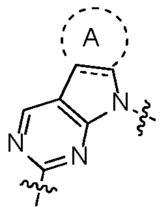

is

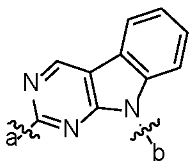, 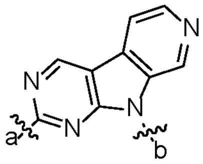, or 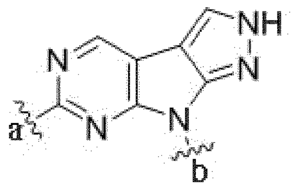,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

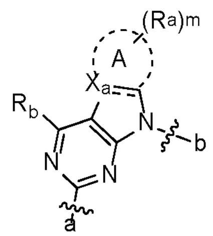

is

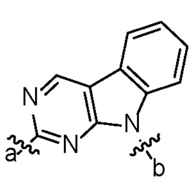, 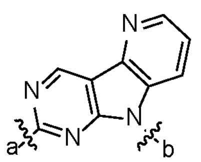, 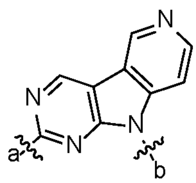, 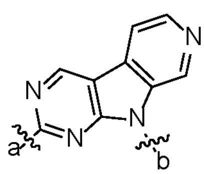,

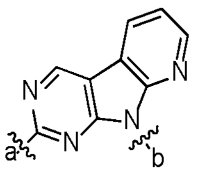, 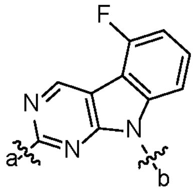, 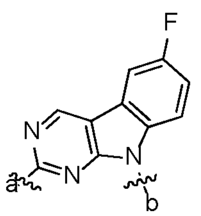, 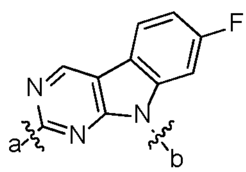, 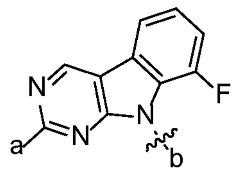,

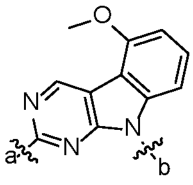, 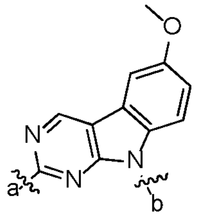, 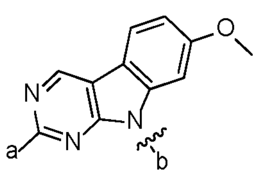, 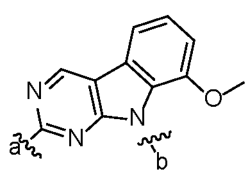, 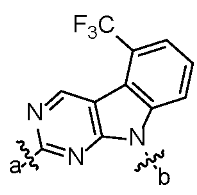,

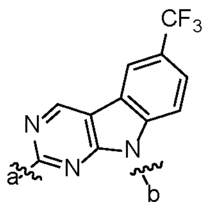 , 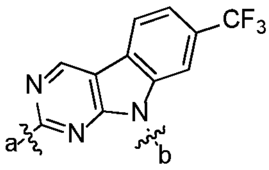 , 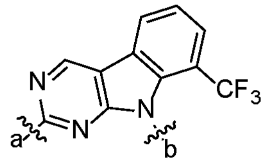 , 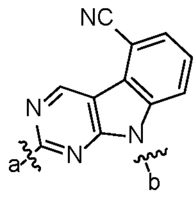 ,

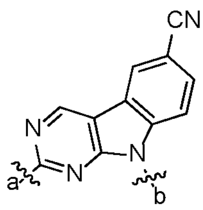 , 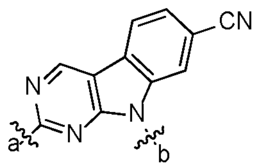 , 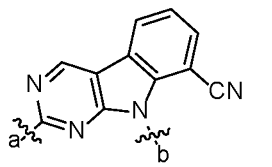 , 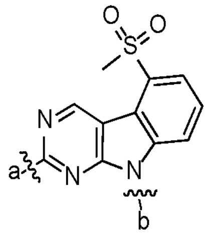 ,

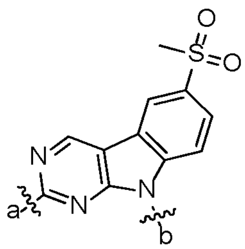 , 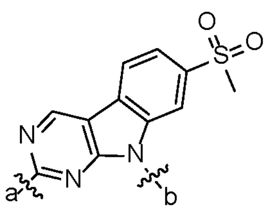 , 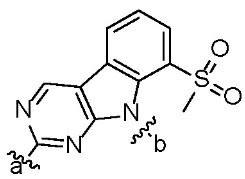 , 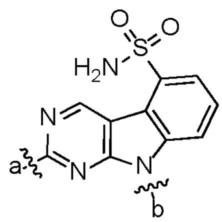 ,

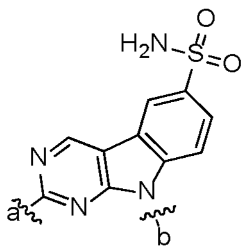 , 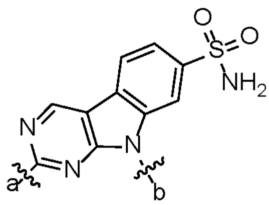 , 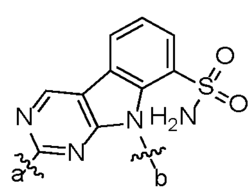 , 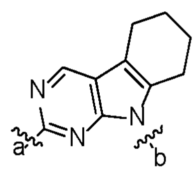 ,

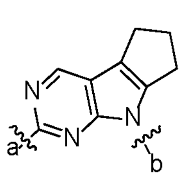 , 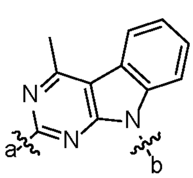 , 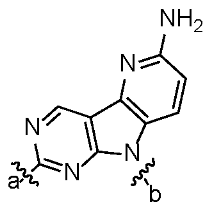 , 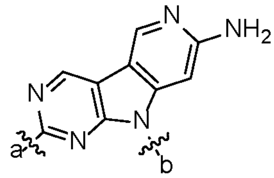 , 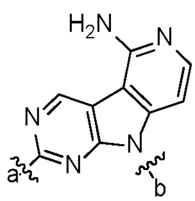 ,

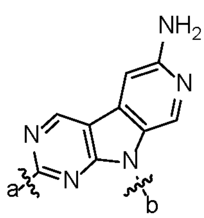 , 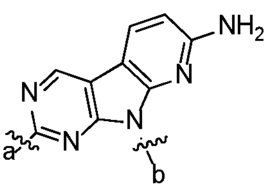 , 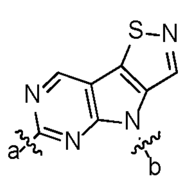 , 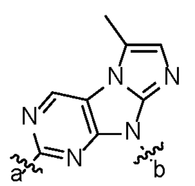 , 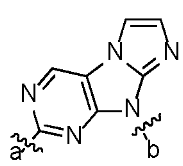 ,

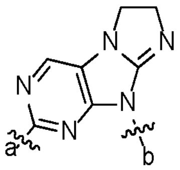 , 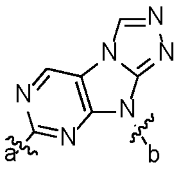 , 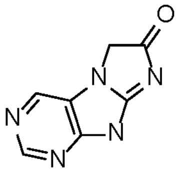 , 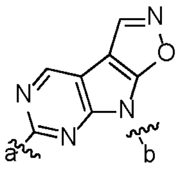 , 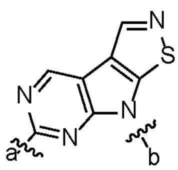 ,

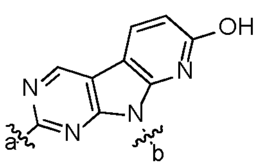 , 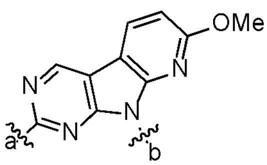 , 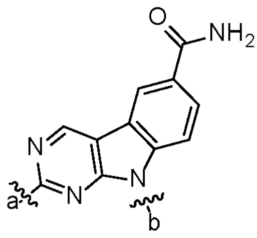 , 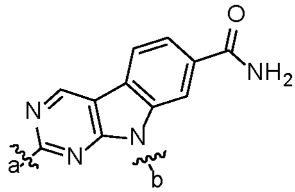 ,

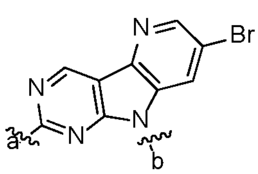 , 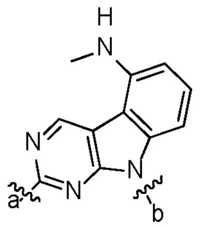 , 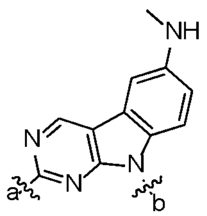 , 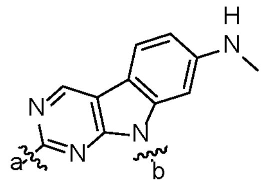 ,

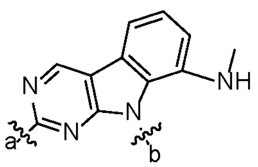 , 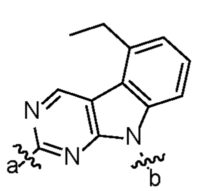 , 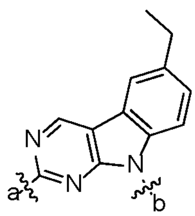 , 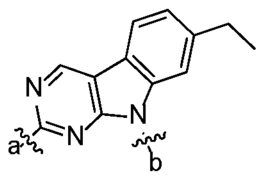 ,

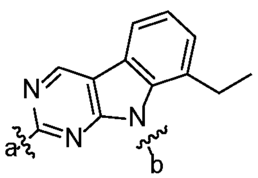 , 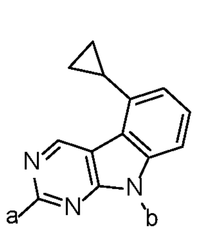 , 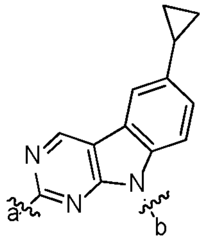 , 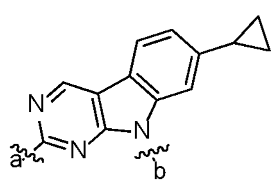 ,

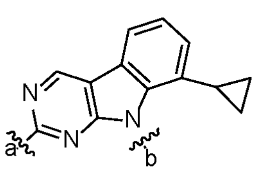 , 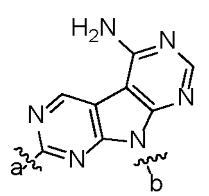 , 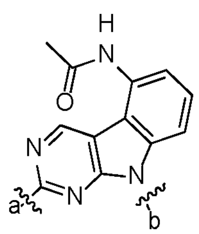 , 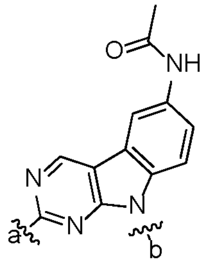 ,

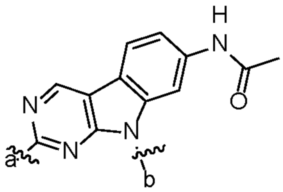, 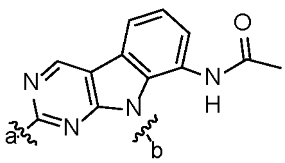, 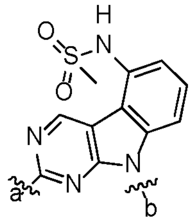, 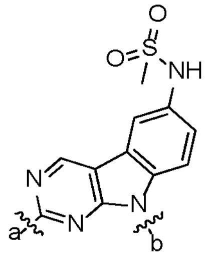,

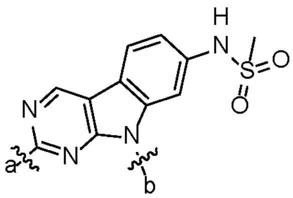, 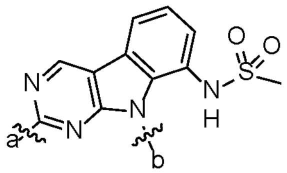, 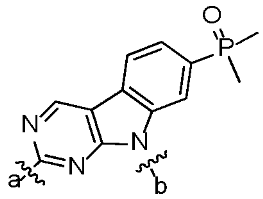, 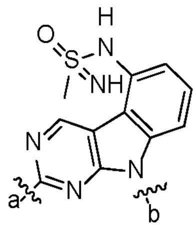,

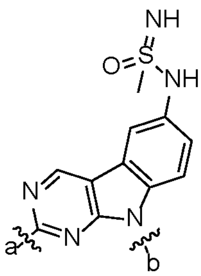, 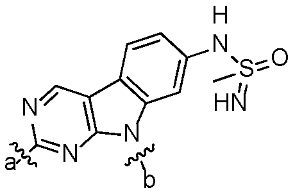, 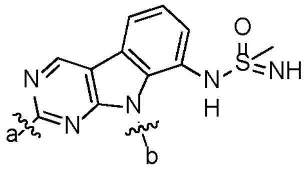, 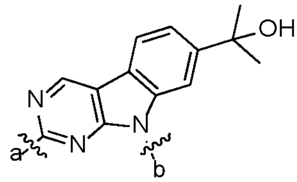,

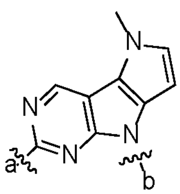, 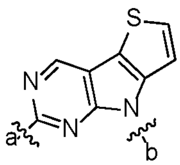, 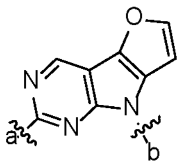, 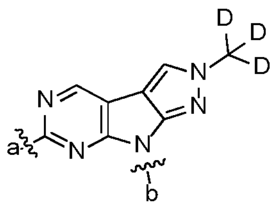, 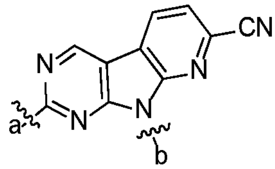,

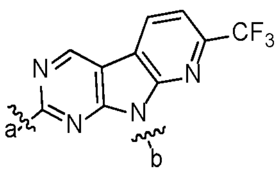, 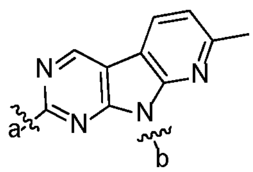, 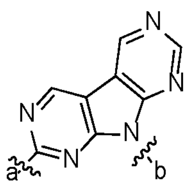, or 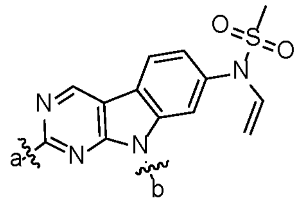,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

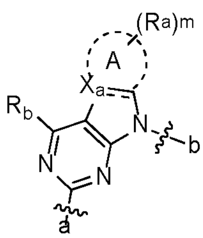

is

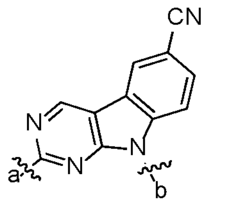,
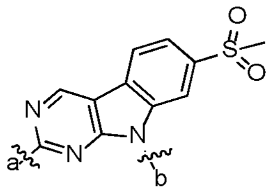,
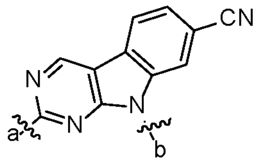,
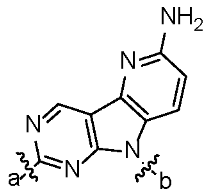,
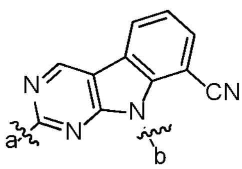,
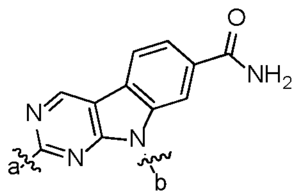,
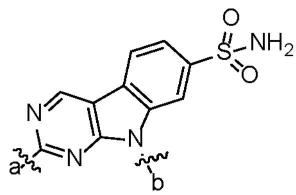,
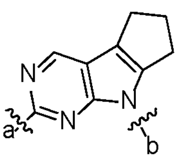,
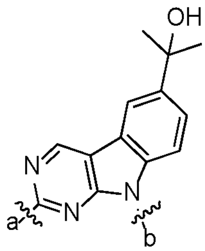,
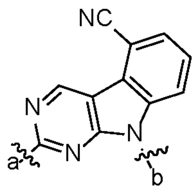,
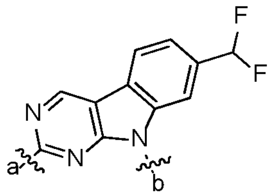,

or

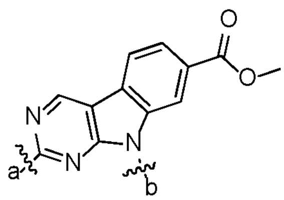,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

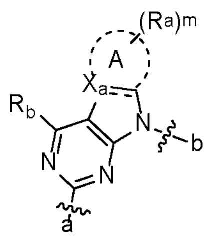

is

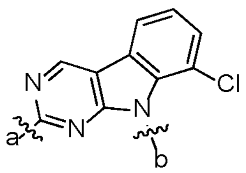, 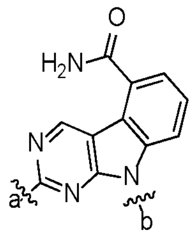, 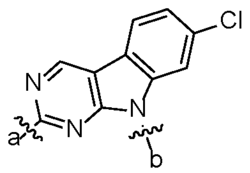,

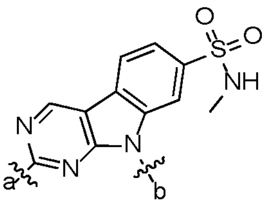, 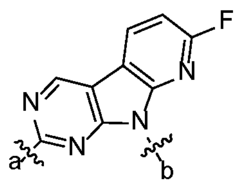, or 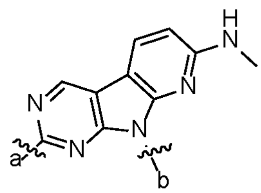,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

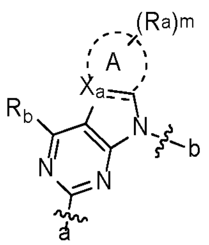

is

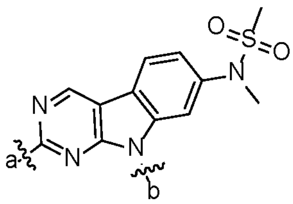, 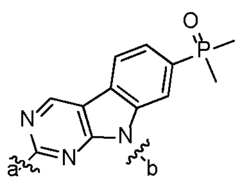, 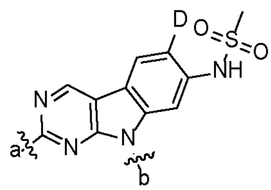,

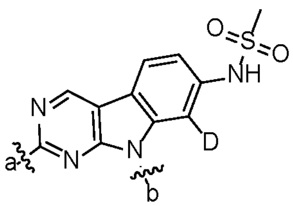, 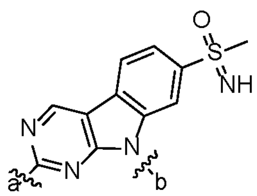, 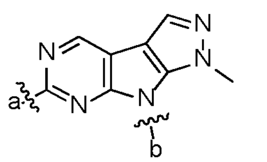, 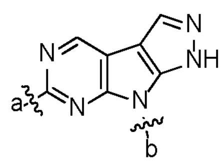,

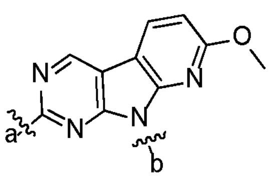, 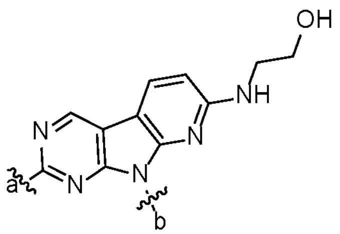, 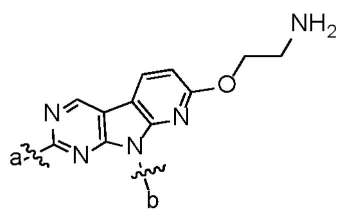,

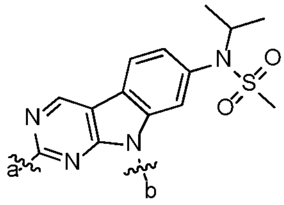 , 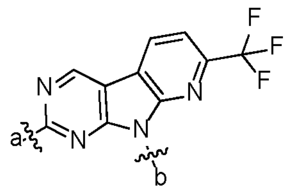 , 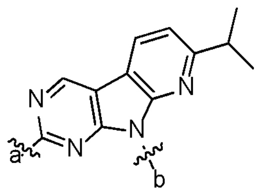 , 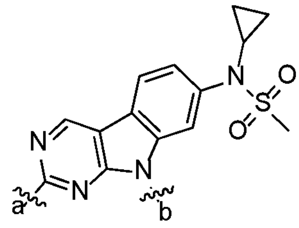 ,

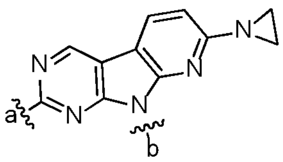 , 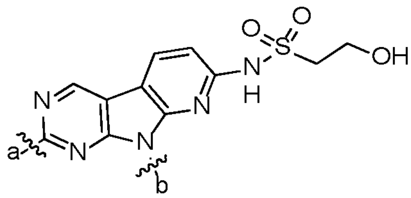 , 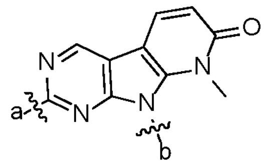 ,

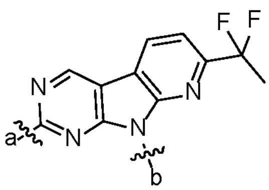 , 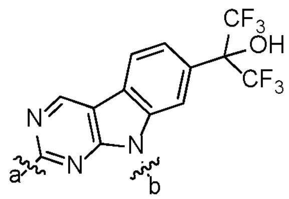 , 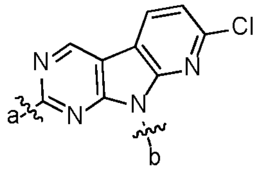 , 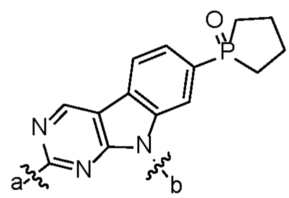 ,

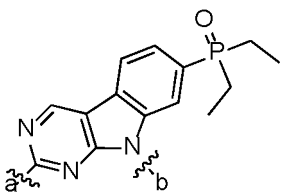 , 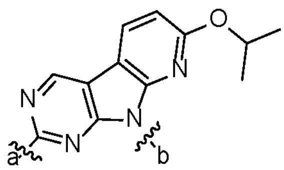 , 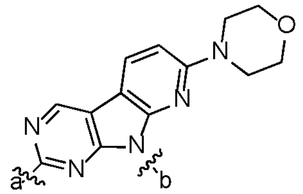 , 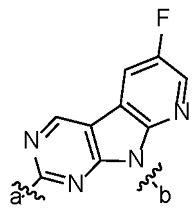 ,

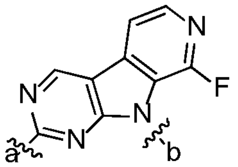 , 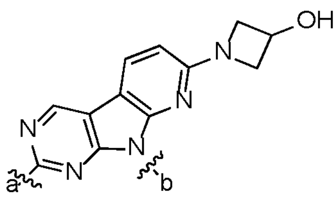 , 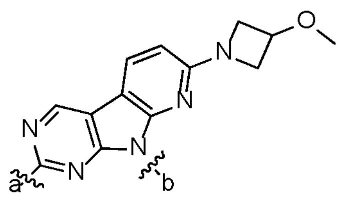 ,

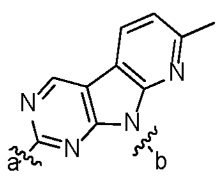 , 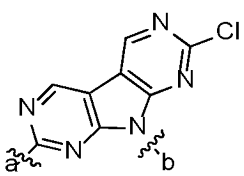 , or 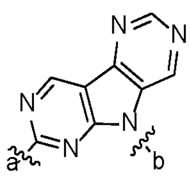 ,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

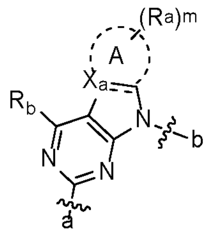

is

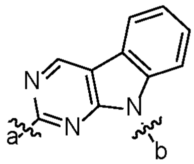, 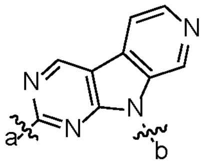, or 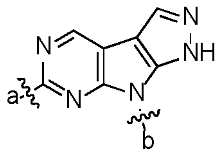,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

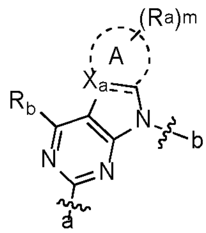

is

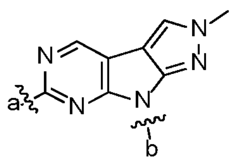,

wherein a represents the linking site with ring B, and b represents the linking site with $L_1$.

In some embodiments of the present application, in the compound represented by formula (II') or (I'), or the isomer thereof or the pharmaceutically acceptable salt thereof, structural unit

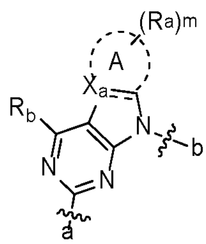

is

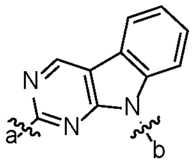 , 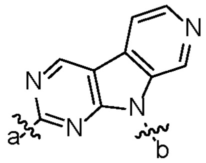 , 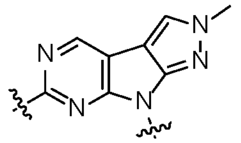 ,

or

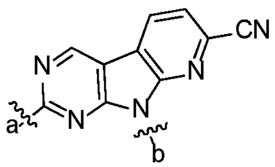 ,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

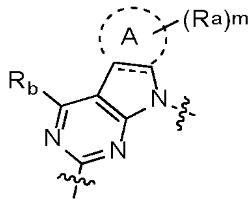

is

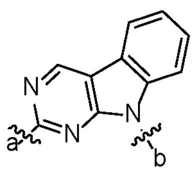 , 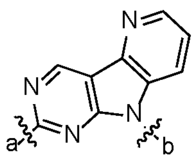 , 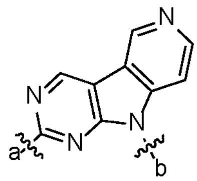 , 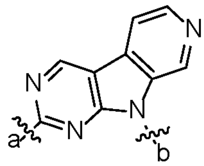 ,

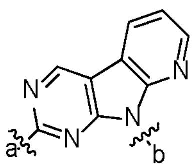 , 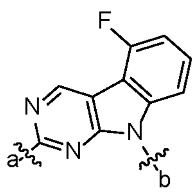 , 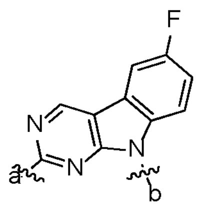 , 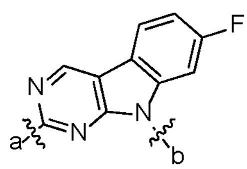 , 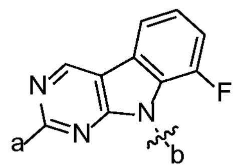 ,

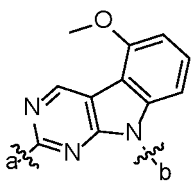 , 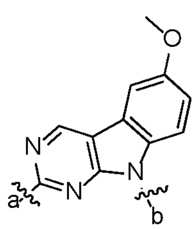 , 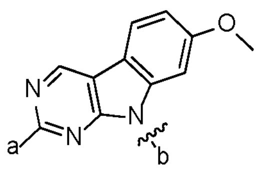 , 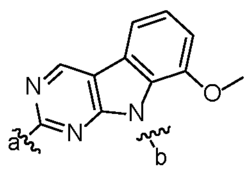 , 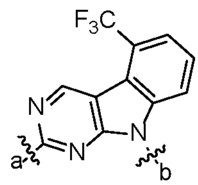 ,

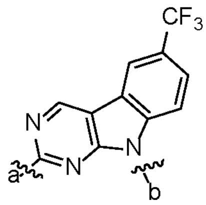 , 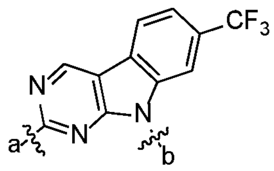 , 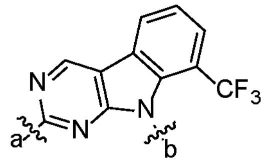 , 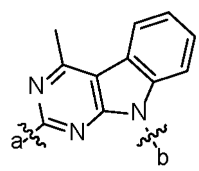 ,

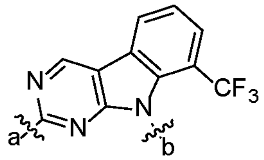 , 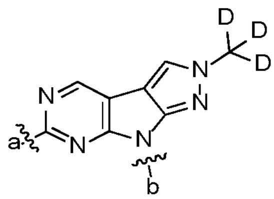 , 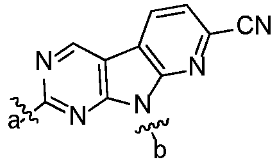 , 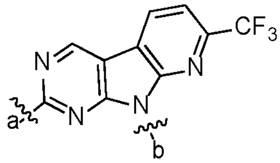 ,

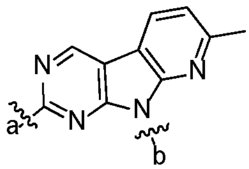 , 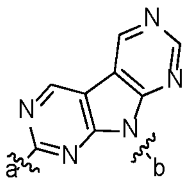 , or 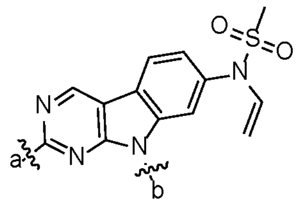 ,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

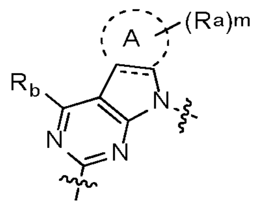

is

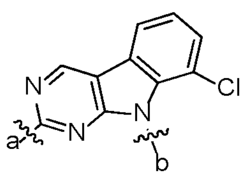 , 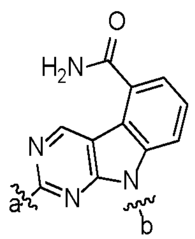 , 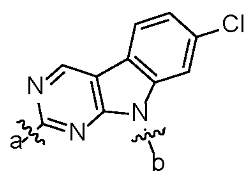 ,

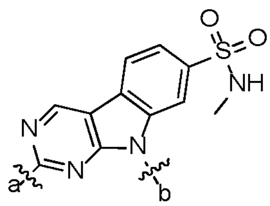 , 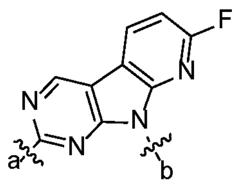 , 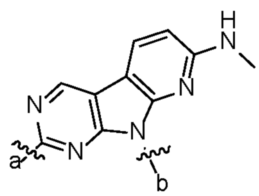 , 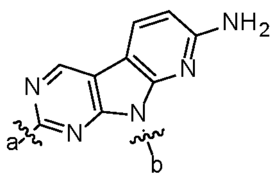 ,

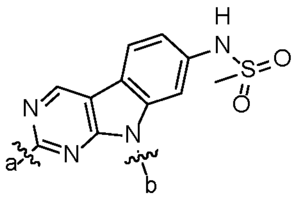, 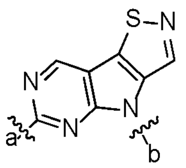, or 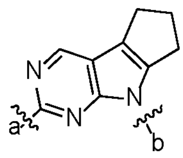,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

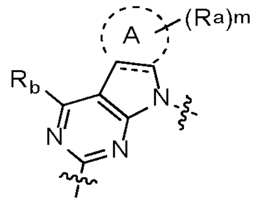

is

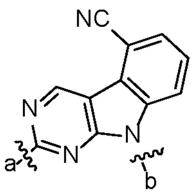, 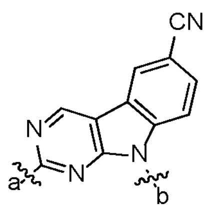, 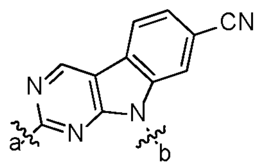,

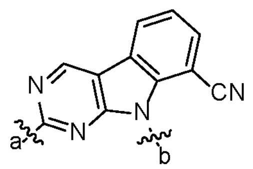, 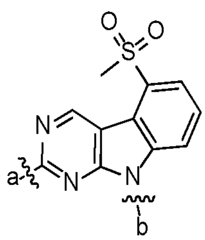, 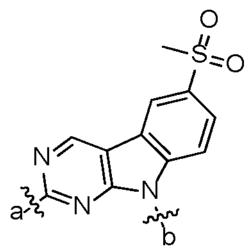, 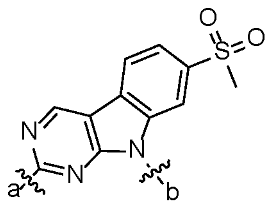,

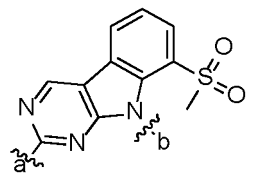, 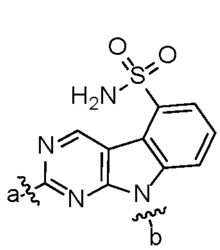, 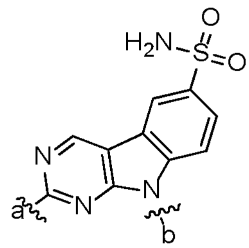, 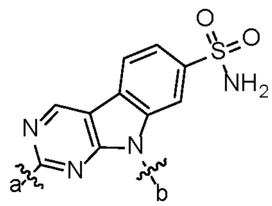,

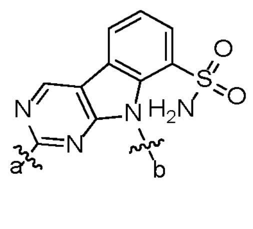, 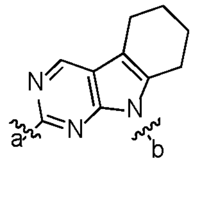, 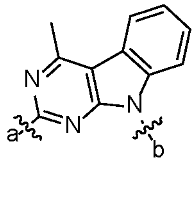, 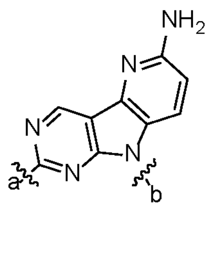,

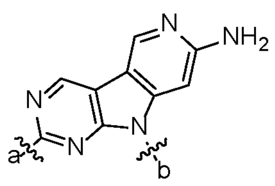 , 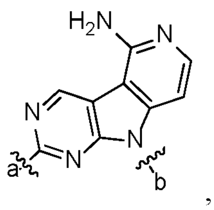 , 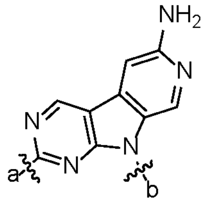 , 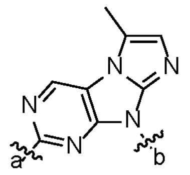 , 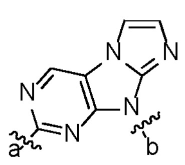 ,

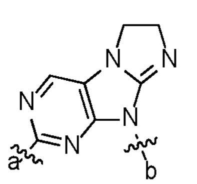 , 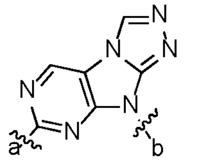 , 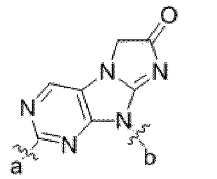 , 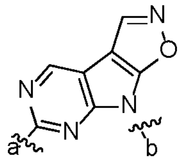 , 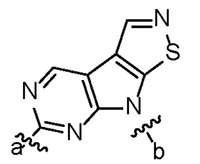 ,

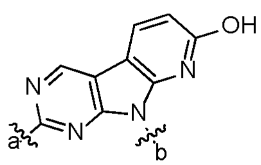 , 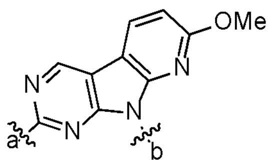 , 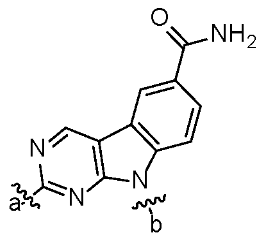 , 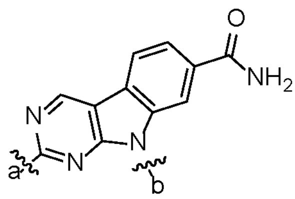 ,

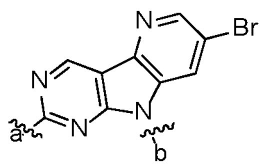 , 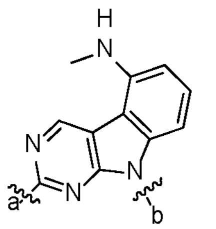 , 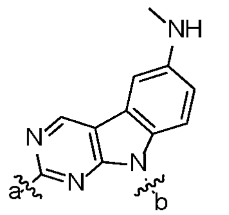 , 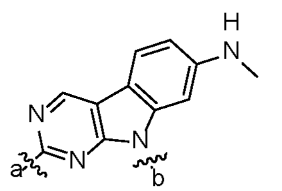 ,

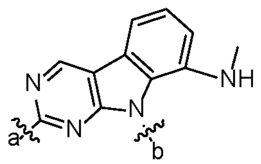 , 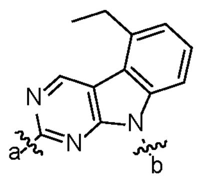 , 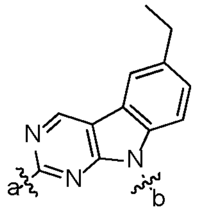 , 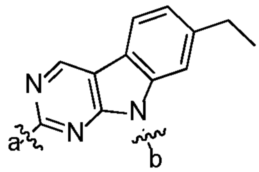 ,

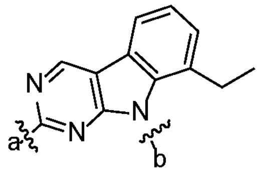 , 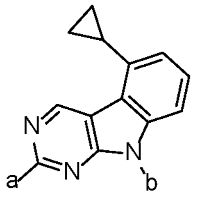 , 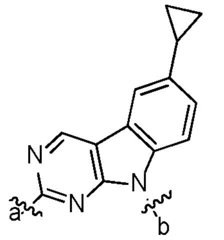 , 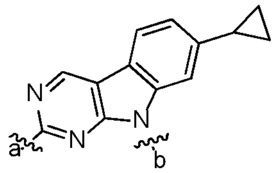 ,

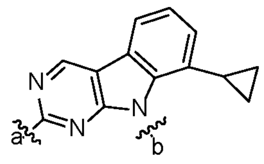, 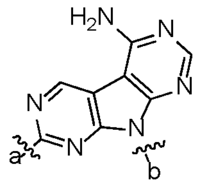, 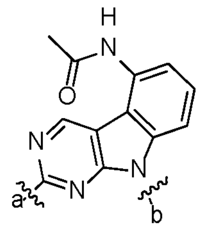, 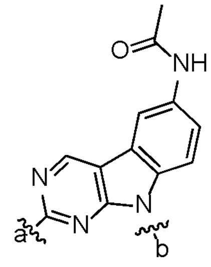,

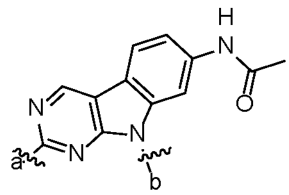, 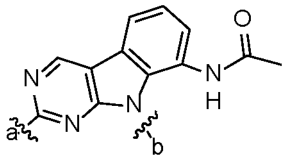, 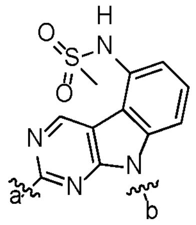, 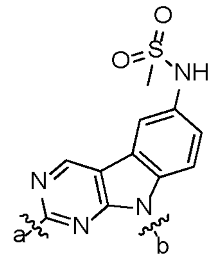,

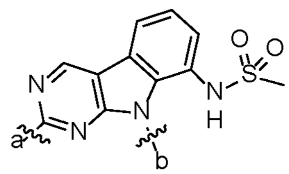, 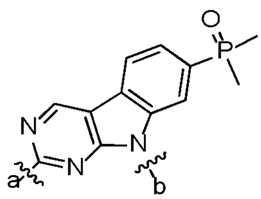, 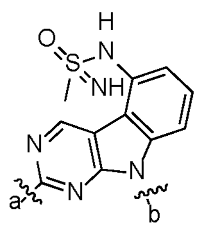, 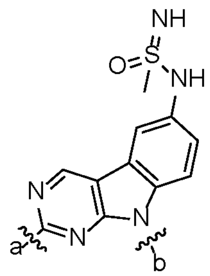,

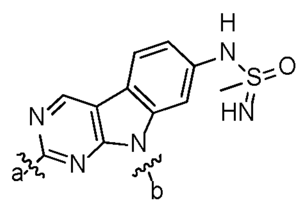, 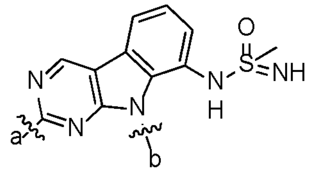, 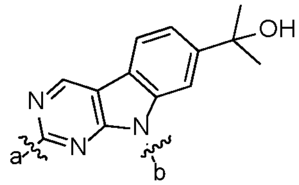, 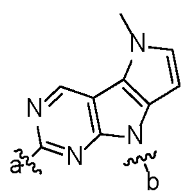,

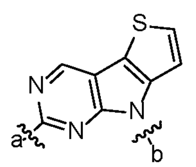, 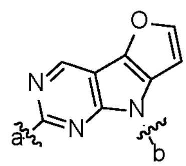, 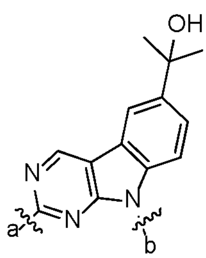, 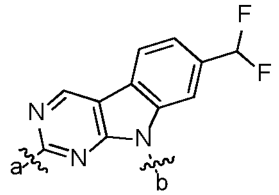,

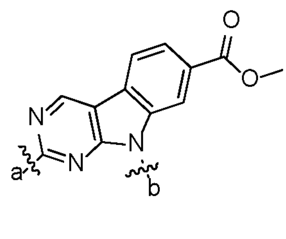, 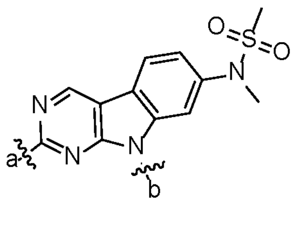, 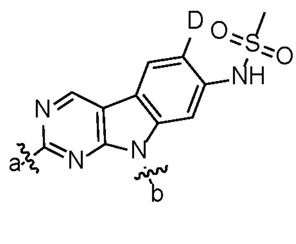,

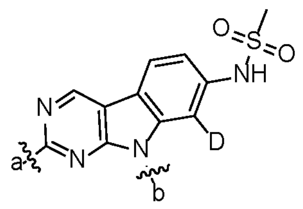,
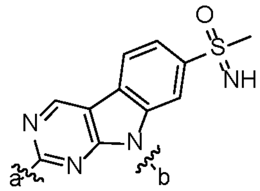,
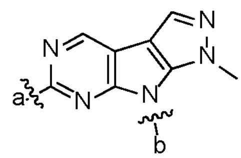,
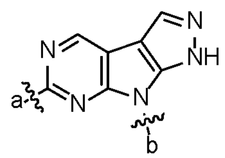,

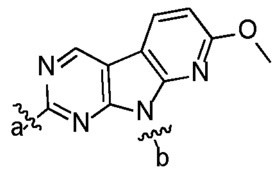,
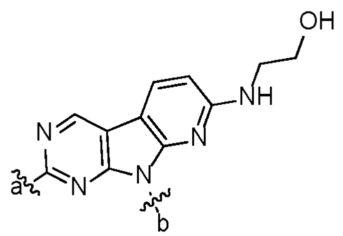,
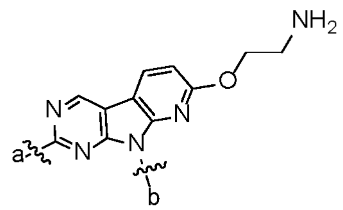,

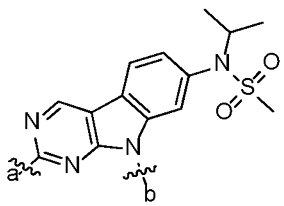,
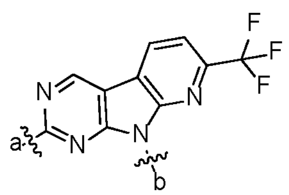,
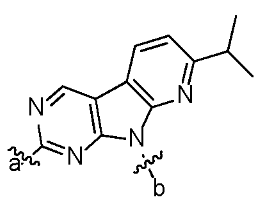,
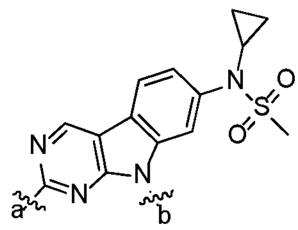,

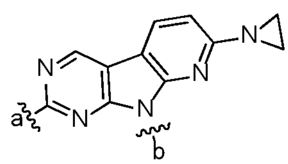,
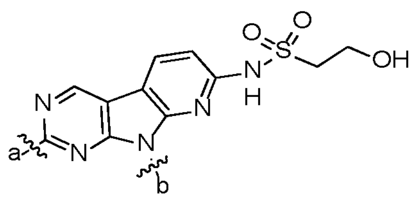,
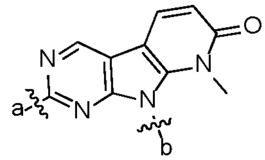,

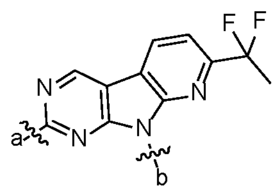,
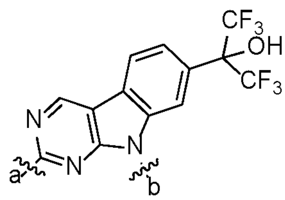,
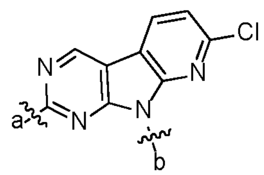,
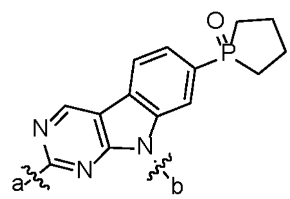,

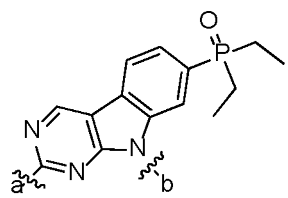,
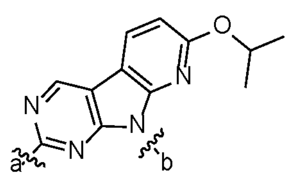,
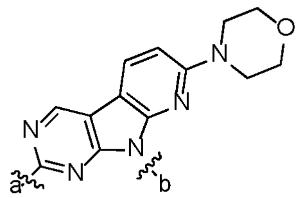,
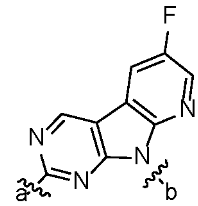,

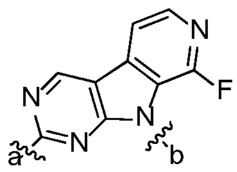, 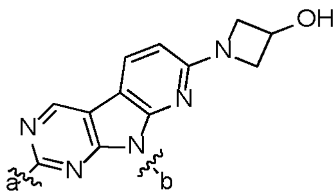, 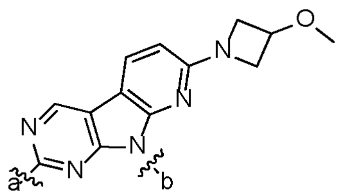,

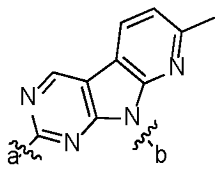, 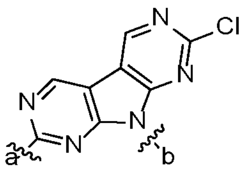, or 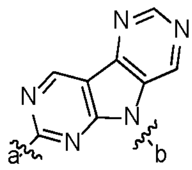,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

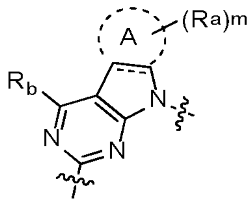

is

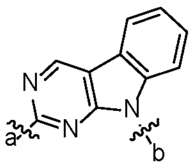, 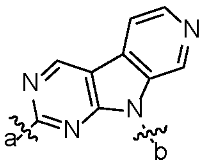, 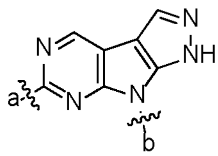,

or

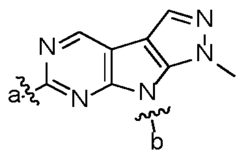,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

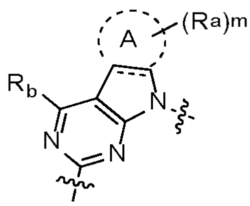

is

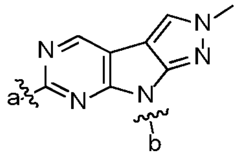

,

wherein a represents the linking site with ring B, and b represents the linking site with $L_1$.

In some embodiments of the present application, in the compound represented by formula (I'-1), or the isomer thereof or the pharmaceutically acceptable salt thereof, structural unit

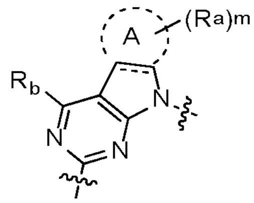

is

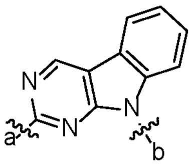

,

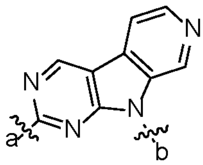

,

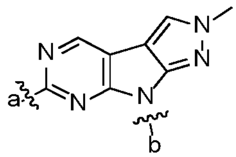

,

or

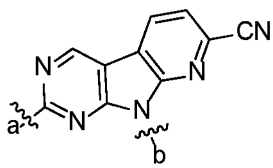

,

wherein a represents the linking site with ring B, and b represents the linking site with $L_1$.

In some embodiments of the present application, in the compound represented by formula (II'), (I'), or (I'-1), or the isomer thereof or the pharmaceutically acceptable salt thereof, $R_b$ is hydrogen.

In some embodiments of the present application, in the compound represented by formula (II'), (I'), or (I'-1), or the isomer thereof or the pharmaceutically acceptable salt thereof, ring B is

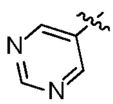

,

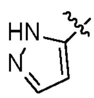

,

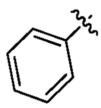

, or

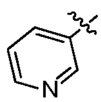

.

In some embodiments of the present application, in the compound represented by formula (II'), (I'), or (I'-1), or the isomer thereof or the pharmaceutically acceptable salt thereof, ring B is

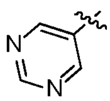

.

In some embodiments of the present application, in the compound represented by formula (II'), (I'), or (I'-1), or the isomer thereof or the pharmaceutically acceptable salt thereof, ring B is

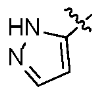.

In some embodiments of the present application, in the compound represented by formula (II'), (I'), or (I'-1), or the isomer thereof or the pharmaceutically acceptable salt thereof, ring B is

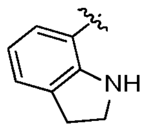 or 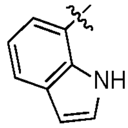.

In some embodiments of the present application, in the compound represented by formula (II'), (I'), or (I'-1), or the isomer thereof or the pharmaceutically acceptable salt thereof, $R_c$ are each independently $CH_3O$-, Cl-, $CH(CH_3)_2$-,

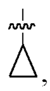,

$-OCHF_2$, or $-CF_3$.

In some embodiments of the present application, in the compound represented by formula (II'), (I'), or (I'-1), or the isomer thereof or the pharmaceutically acceptable salt thereof, $R_c$ are each independently $-OCH_3$,

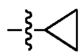,

$-CH(CH_3)_2$, or -Cl.

In some embodiments of the present application, in the compound represented by formula (II'), (I'), or (I'-1), or the isomer thereof or the pharmaceutically acceptable salt thereof, $R_c$ are each independently

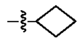,

$-CH_3$, or $-OCD_3$.

In some embodiments of the present application, in the compound represented by formula (II'), (I'), or (I'-1), or the isomer thereof or the pharmaceutically acceptable salt thereof, structural unit

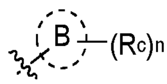

is

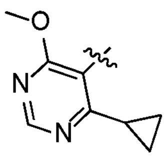, 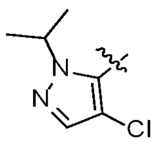, 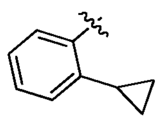, 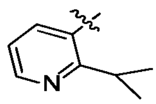,

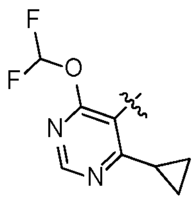, or 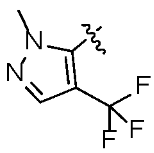.

In some embodiments of the present application, in the compound represented by formula (II'), (I'), or (I'-1), or the

isomer thereof or the pharmaceutically acceptable salt thereof, structural unit

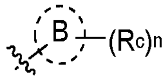

is

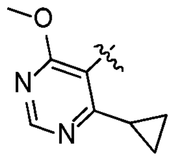.

In some embodiments of the present application, in the compound represented by formula (II'), (I'), or (I'-1), or the isomer thereof or the pharmaceutically acceptable salt thereof, structural unit

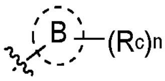

is

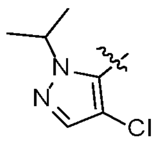.

In some embodiments of the present application, in the compound represented by formula (II'), (I'), or (I'-1), or the isomer thereof or the pharmaceutically acceptable salt thereof, structural unit

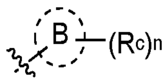

is

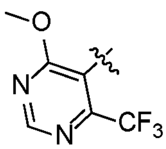, 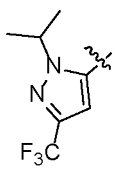, 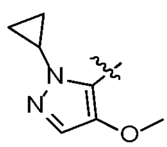, 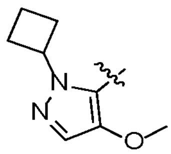, 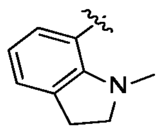,

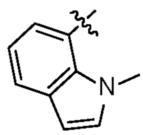, 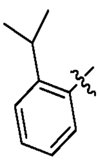, 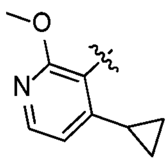, 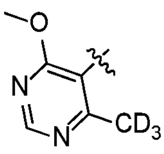, 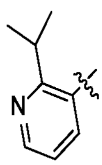, 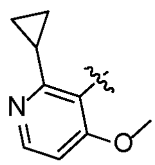, 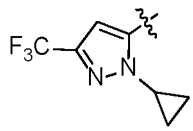,

or

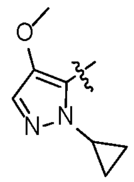

In some embodiments of the present application, in the compound represented by formula (II'), (I'), or (I'-1), or the isomer thereof or the pharmaceutically acceptable salt thereof, ring C is

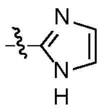 , 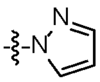 , or 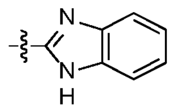 .

In some embodiments of the present application, in the compound represented by formula (II'), (I'), or (I'-1), or the isomer thereof or the pharmaceutically acceptable salt thereof, ring C is

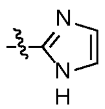

In some embodiments of the present application, in the compound represented by formula (II'), (I'), or (I'-1), or the isomer thereof or the pharmaceutically acceptable salt thereof, ring C is

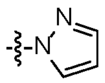

In some embodiments of the present application, in the compound represented by formula (II'), (I'), or (I'-1), or the isomer thereof or the pharmaceutically acceptable salt thereof, ring C is

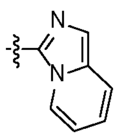 , 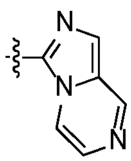 , 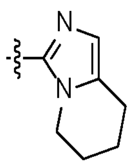 , 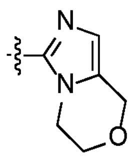 , 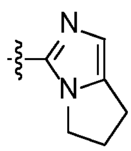 , 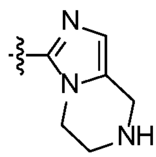 , or 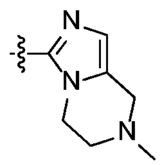 .

In some embodiments of the present application, in the compound represented by formula (II'), (I'), or (I'-1), or the isomer thereof or the pharmaceutically acceptable salt thereof, ring C is

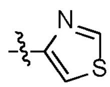 or 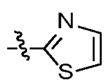 .

In some embodiments of the present application, in the compound represented by formula (II'), (I'), or (I'-1), or the isomer thereof or the pharmaceutically acceptable salt thereof, $R_d$ are each independently $-CF_3$, $-CH_3$, $-CH_2CH_3$, $-CH(CH_3)_2$, $-OCH_2CH_3$, or $-CD_3$.

In some embodiments of the present application, in the compound represented by formula (II'), (I'), or (I'-1), or the isomer thereof or the pharmaceutically acceptable salt thereof, $R_d$ are each independently $-CF_3$, $-CH_3$, or $-CH(CH_3)_2$.

In some embodiments of the present application, in the compound represented by formula (II'), (I'), or (I'-1), or the isomer thereof or the pharmaceutically acceptable salt thereof, $R_d$ are each independently $-CH_2CH_3$.

In some embodiments of the present application, in the compound represented by formula (II'), (I'), or (I'-1), or the isomer thereof or the pharmaceutically acceptable salt thereof, $R_d$ are each independently -Cl or

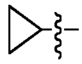.

In some embodiments of the present application, in the compound represented by formula (II'), (I'), or (I'-1), or the isomer thereof or the pharmaceutically acceptable salt thereof, $R_d$ are each independently -Br or -F.

In some embodiments of the present application, in the compound represented by formula (II'), (I'), or (I'-1), or the isomer thereof or the pharmaceutically acceptable salt thereof, structural unit

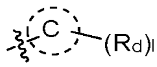

is

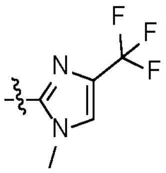, 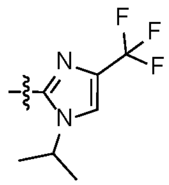, 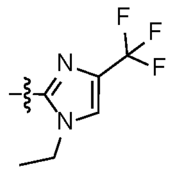, 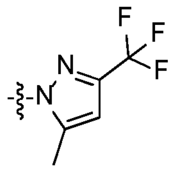,

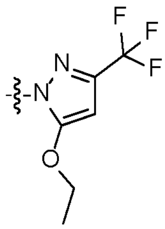, 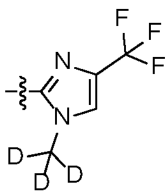, or 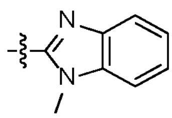.

In some embodiments of the present application, in the compound represented by formula (II'), (I'), or (I'-1), or the isomer thereof or the pharmaceutically acceptable salt thereof, structural unit

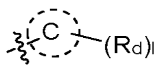

is

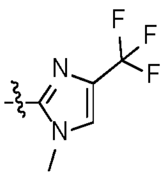, 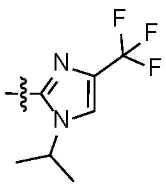, or 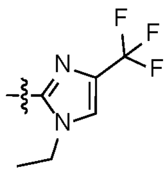.

In some embodiments of the present application, in the compound represented by formula (II'), (I'), or (I'-1), or the isomer thereof or the pharmaceutically acceptable salt thereof, structural unit

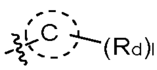

is

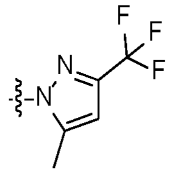 or 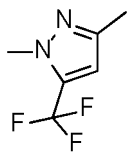 .

In some embodiments of the present application, in the compound represented by formula (II'), (I'), or (I'-1), or the isomer thereof or the pharmaceutically acceptable salt thereof, structural unit

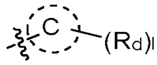

is

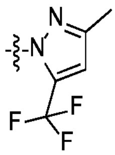 , 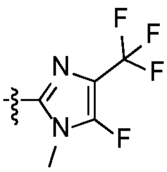 , 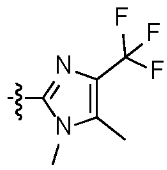 , 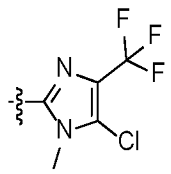 , 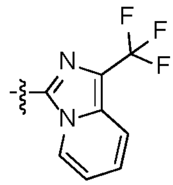 ,

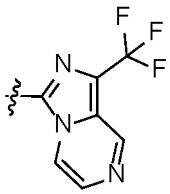 , 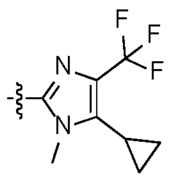 , 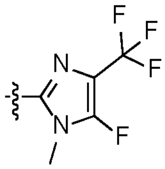 , 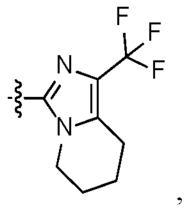 , 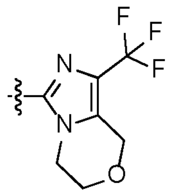 , 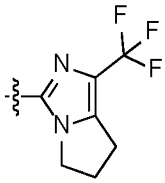 ,

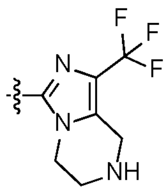 , or 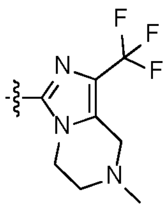 .

In some embodiments of the present application, in the compound represented by formula (II'), (I'), or (I'-1), or the isomer thereof or the pharmaceutically acceptable salt thereof, structural unit

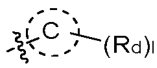

is

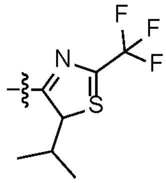 , 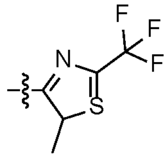 , 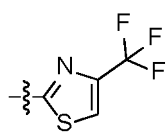 , 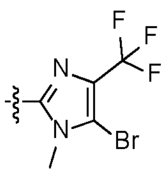 ,

or

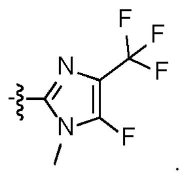

In some embodiments of the present application, in the compound represented by formula (II'), (I'), or (I'-1), or the isomer thereof or the pharmaceutically acceptable salt thereof, $L_1$ is $-CH_2-$, $-CH(CH_3)-$, $-C(CH_3)_2-$, or

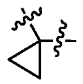

In some embodiments of the present application, in the compound represented by formula (II'), (I'), or (I'-1), or the isomer thereof or the pharmaceutically acceptable salt thereof, $L_1$ is $-CH_2-$, $-CH(CH_3)-$, or $-C(CH_3)_2-$.

In some embodiments of the present application, in the compound represented by formula (II'), (I'), or (I'-1), or the isomer thereof or the pharmaceutically acceptable salt thereof, $L_1$ is $-CH(C_2H_5)-$ or a chemical bond.

The present application further provides the following compounds, or isomers or pharmaceutically acceptable salts thereof,

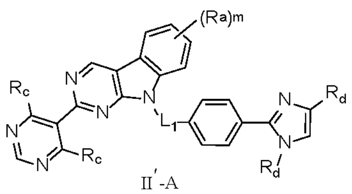

II′-A

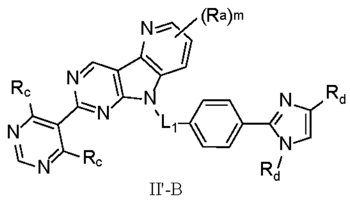

II′-B

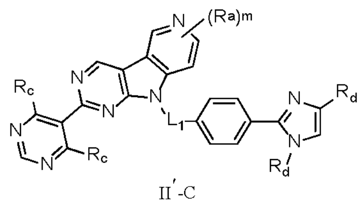

II′-C

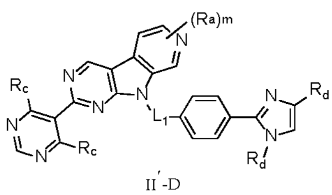

II′-D

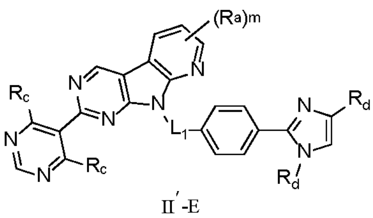

II′-E

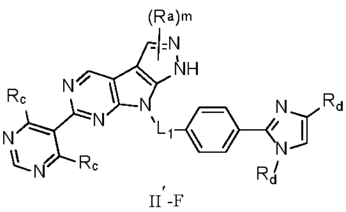

II′-F

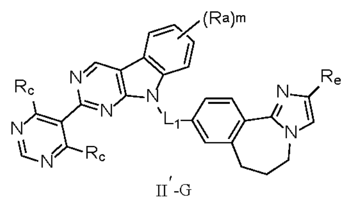

II′-G

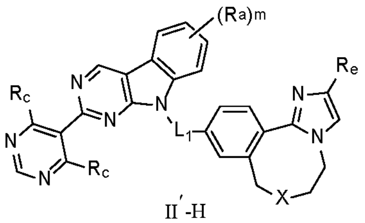

II′-H

wherein $R_a$, $R_c$, $R_d$, $R_e$, $L_1$, and m are defined as mentioned above in the present application; and X is C, N, or O.

The present application further provides the following compound, or an isomer thereof or a pharmaceutically acceptable salt thereof,

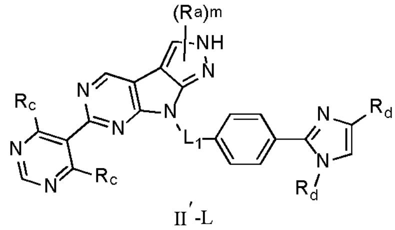

II′-L

wherein $R_a$, $R_c$, $R_d$, $L_1$, and m are defined as mentioned above in the present application.

The present application further provides the following compounds, or isomers or pharmaceutically acceptable salts thereof,

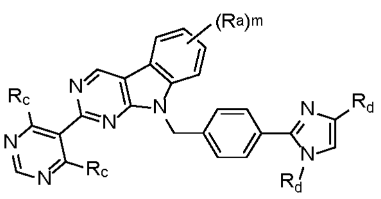
II′-1

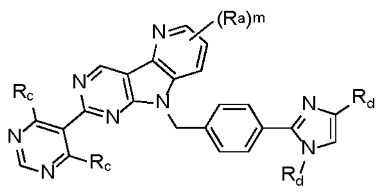
II′-2

wherein $R_a$, $R_c$, $R_d$, and m are defined as mentioned above in the present application.

The present application further provides the following compounds, or isomers or pharmaceutically acceptable salts thereof,

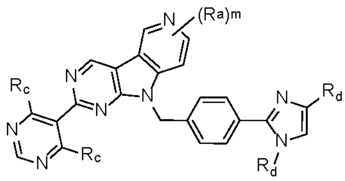
II′-3

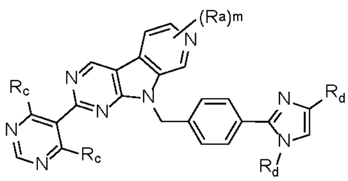
II′-4

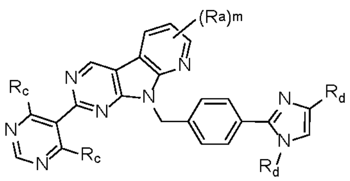
II′-5

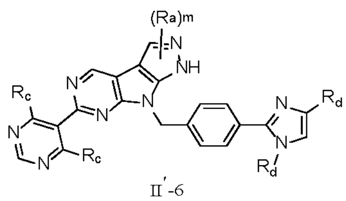
II′-6

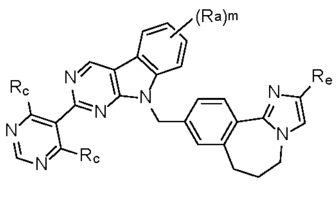
II′-7

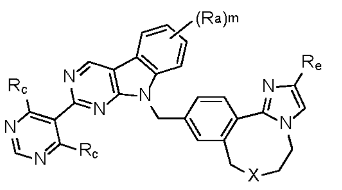
II′-8

wherein $R_a$, $R_c$, $R_d$, $R_e$, and m are defined as mentioned above in the present application; and X is C, N, or O.

The present application further provides the following compound, or an isomer thereof or a pharmaceutically acceptable salt thereof,

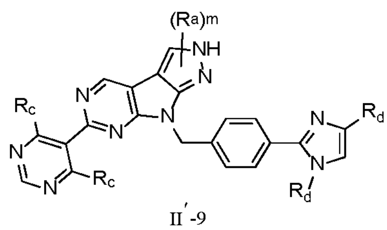
II′-9

wherein $R_a$, $R_c$, $R_d$, $R_e$, and m are defined as mentioned above in the present application.

The present application further provides the following compounds, or isomers or pharmaceutically acceptable salts thereof,

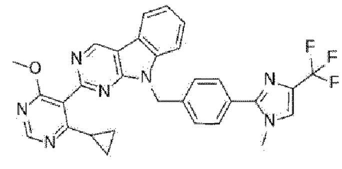

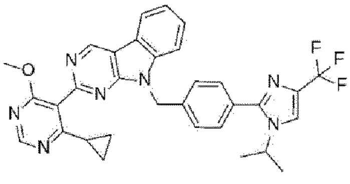

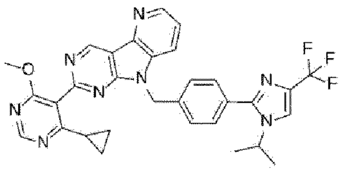

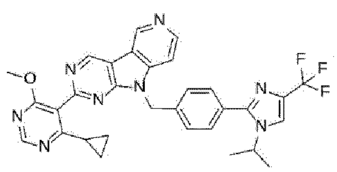 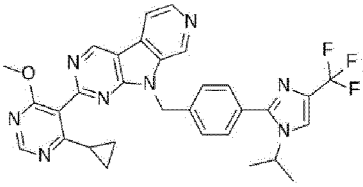 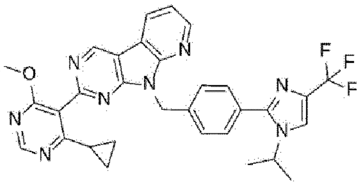

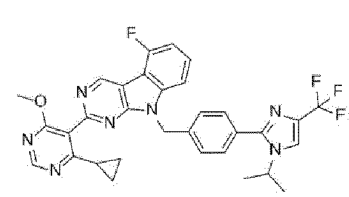 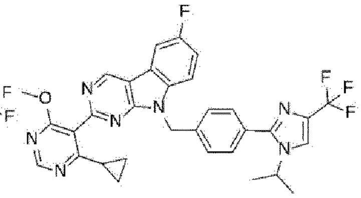 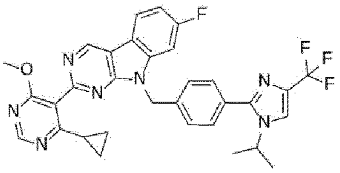

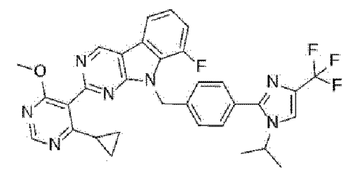 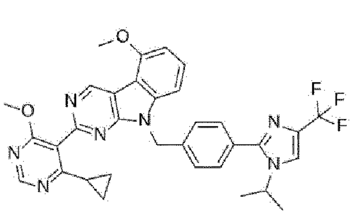 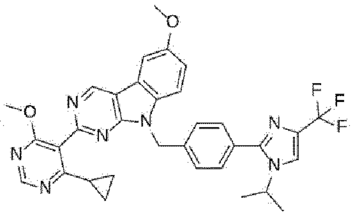

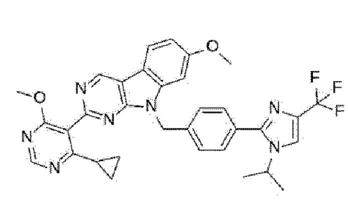 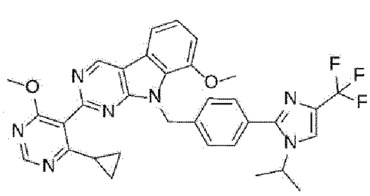 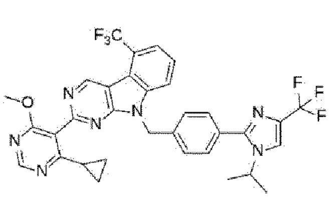

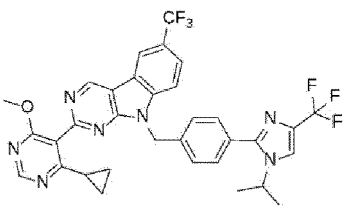 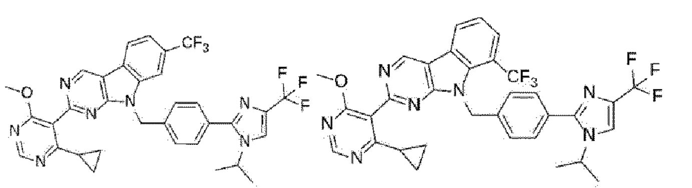

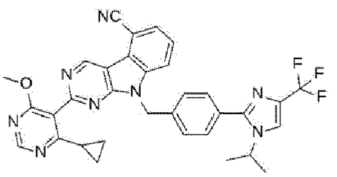 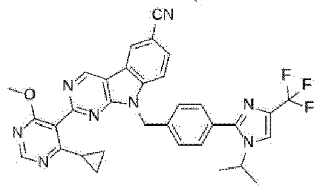 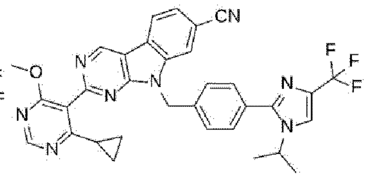

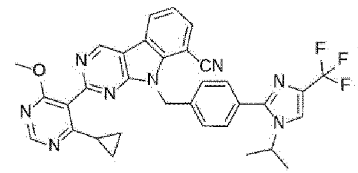 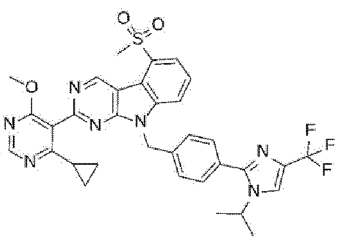 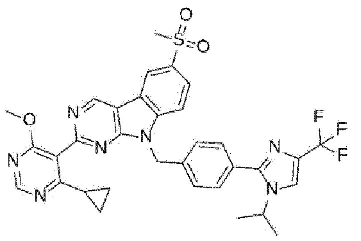

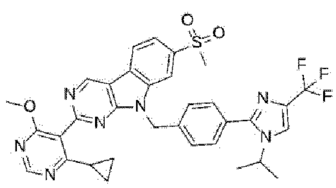
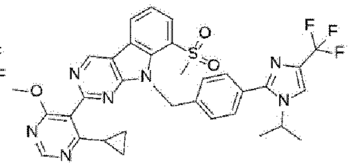
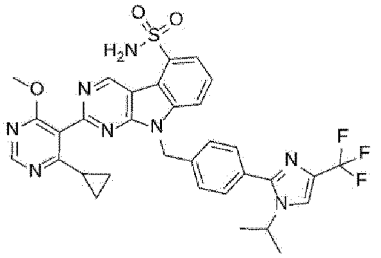

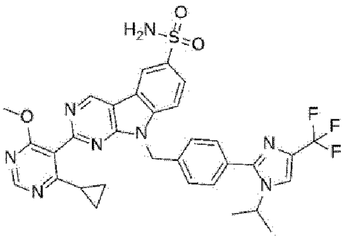
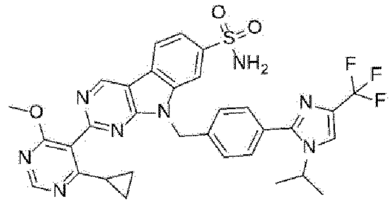
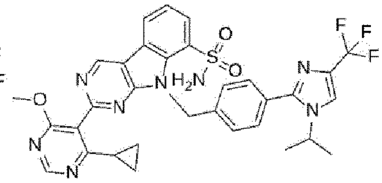

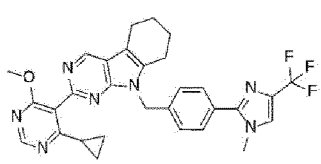
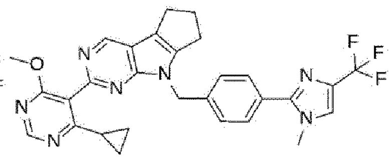
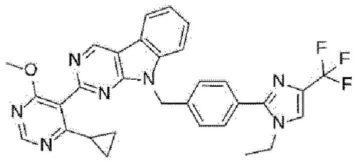

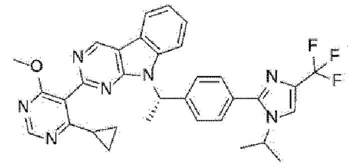
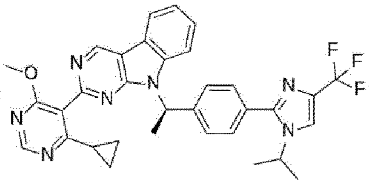
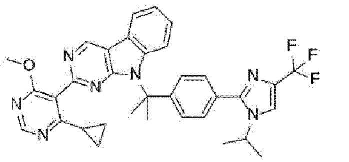

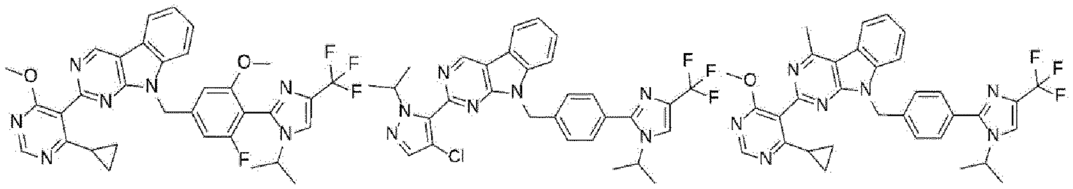

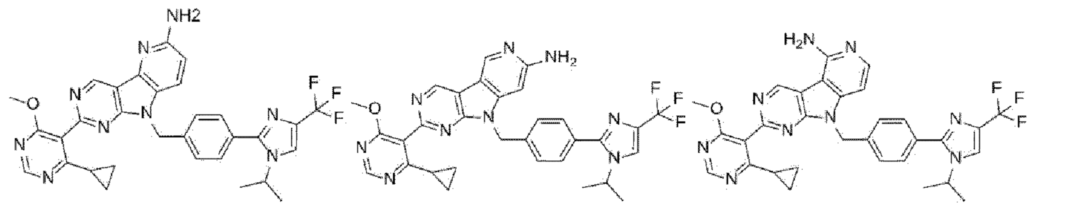

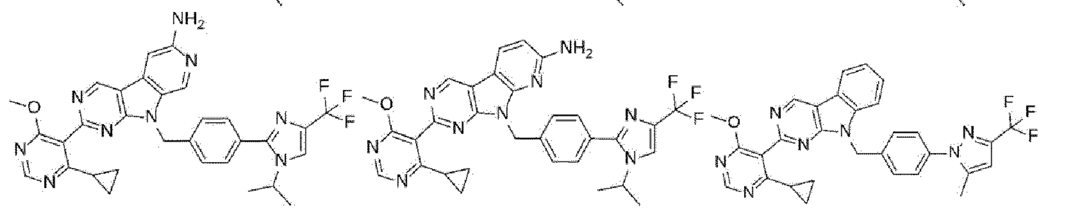

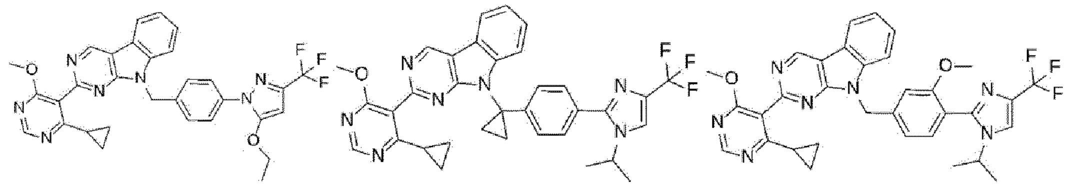

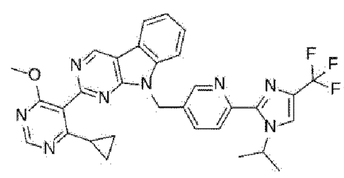
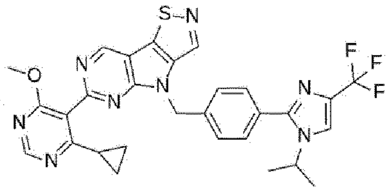
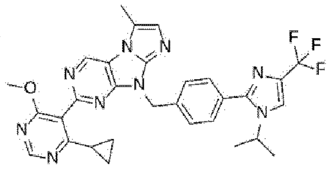

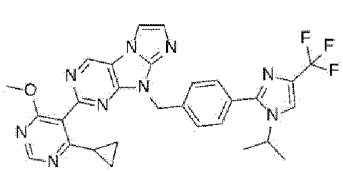
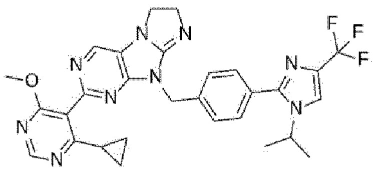
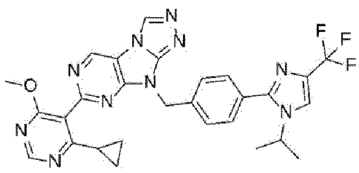

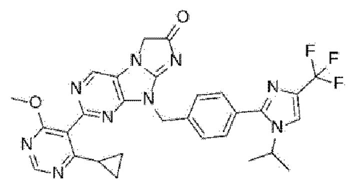
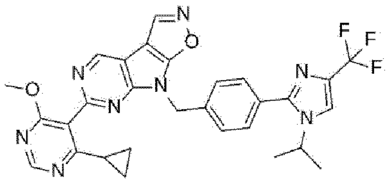
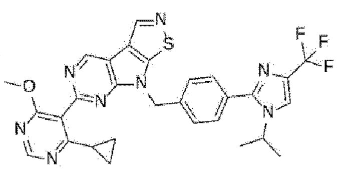

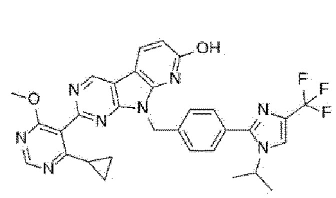
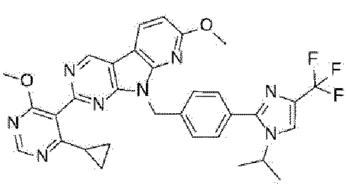
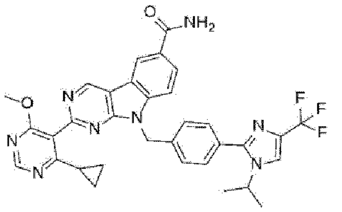

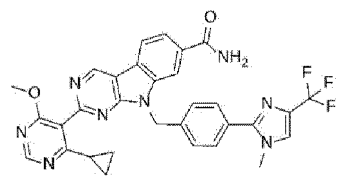
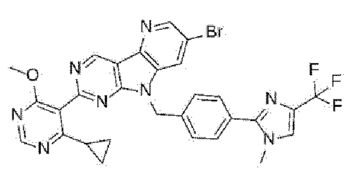
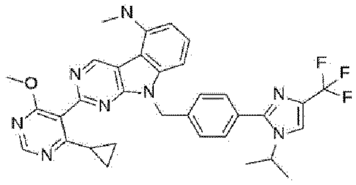

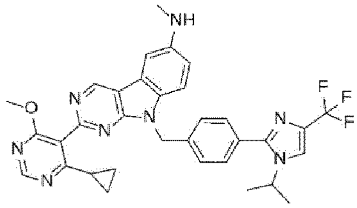
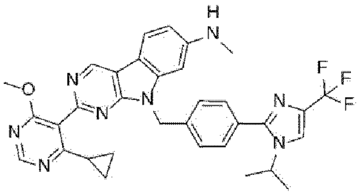
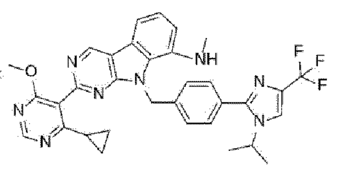
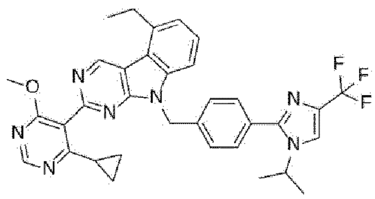
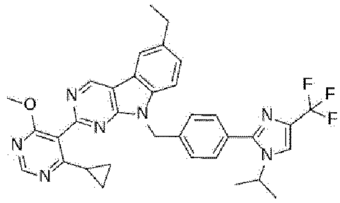
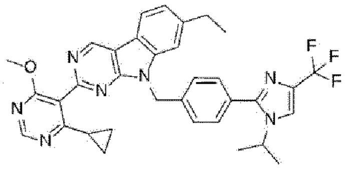
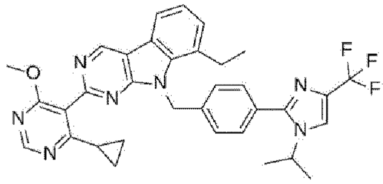
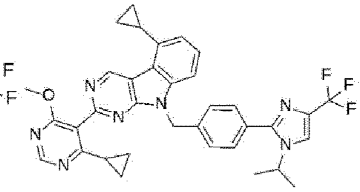
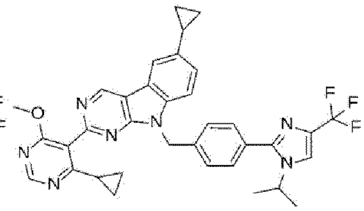
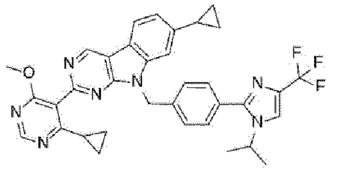
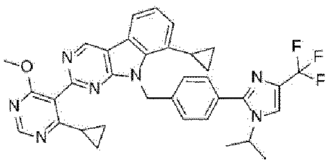
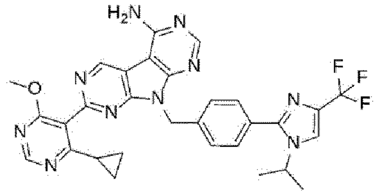
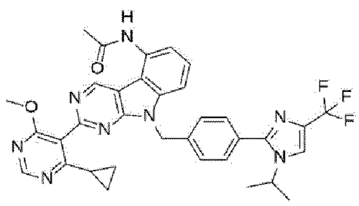
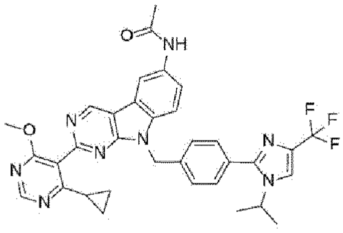
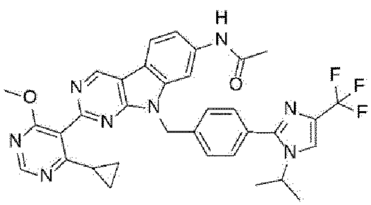
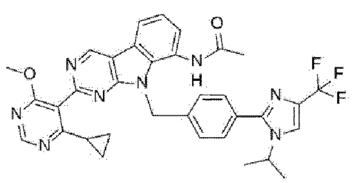
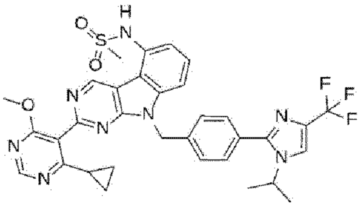
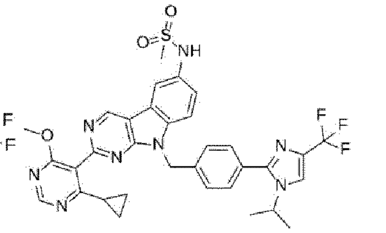
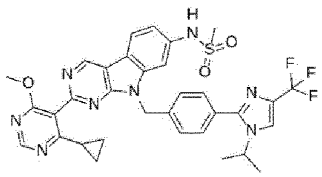
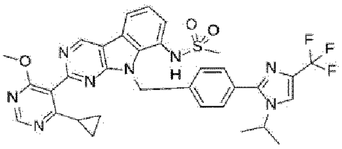
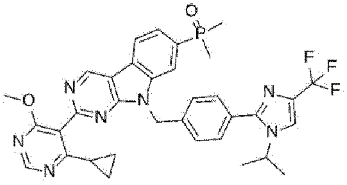
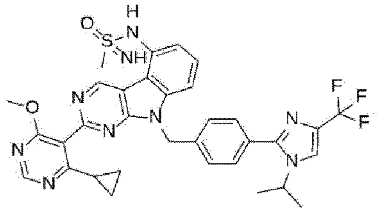
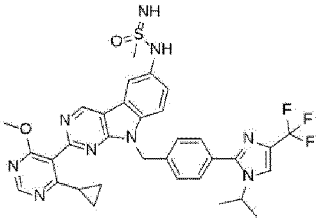
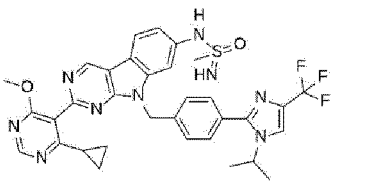

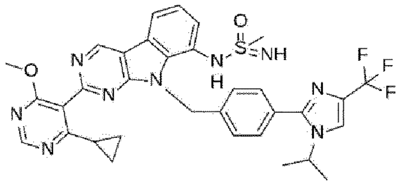
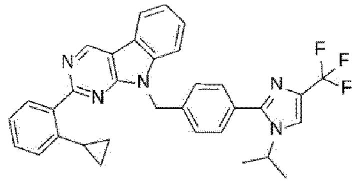
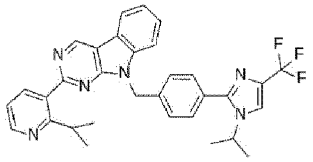
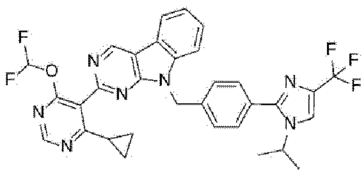
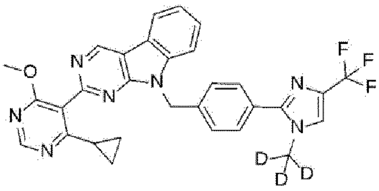
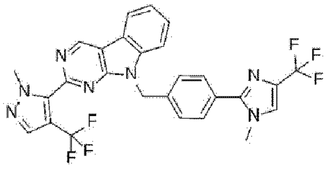
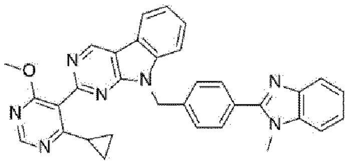
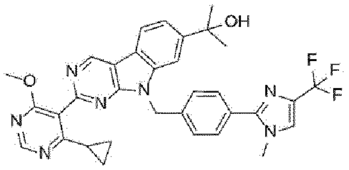
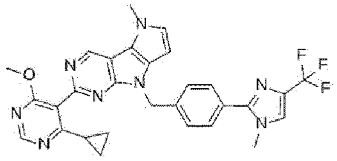
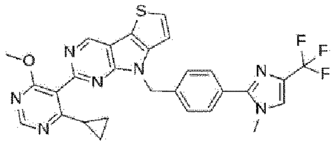
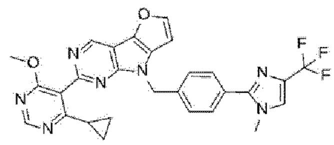
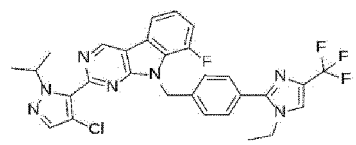
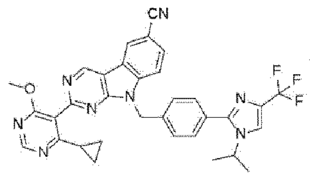
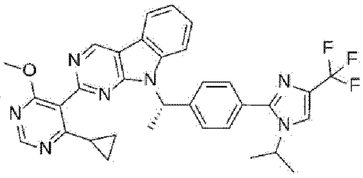
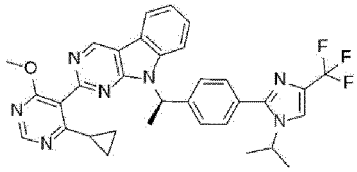
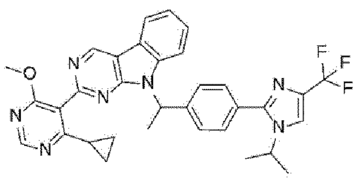
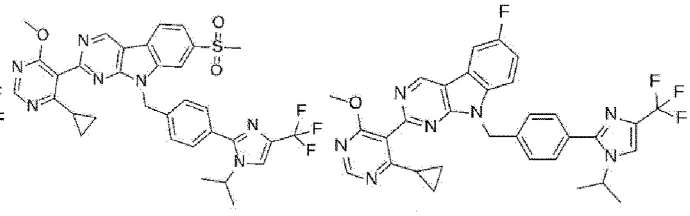
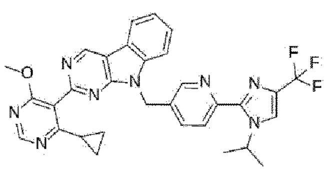
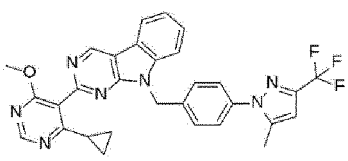
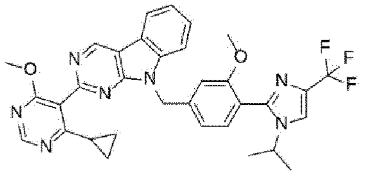
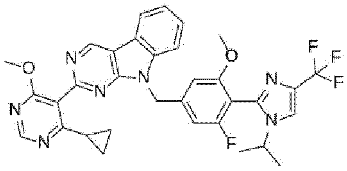
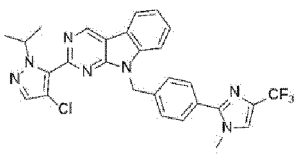
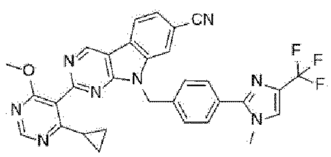
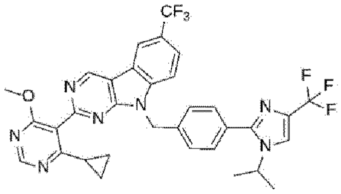
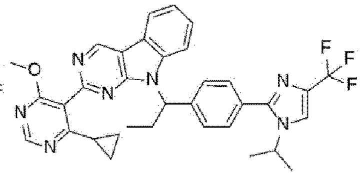
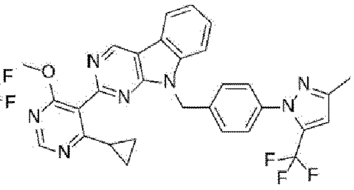

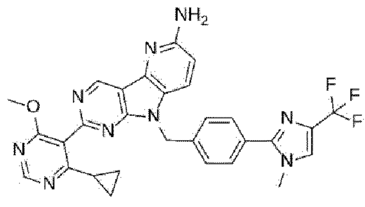
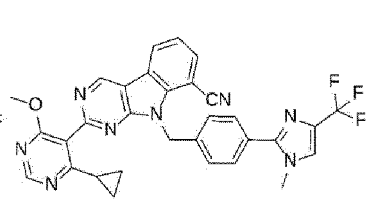
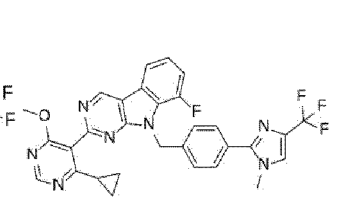

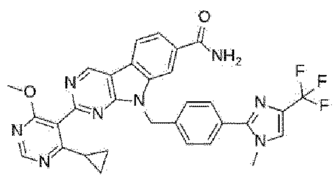
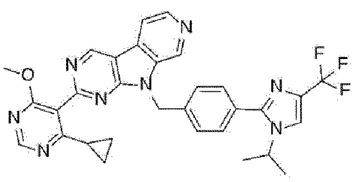
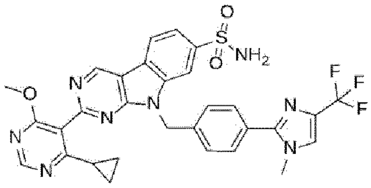

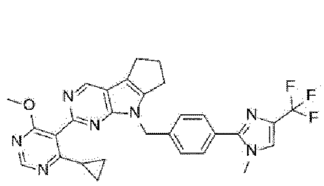
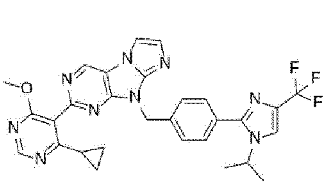
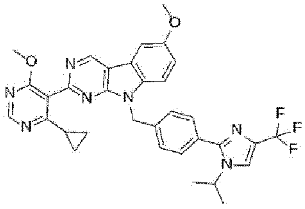

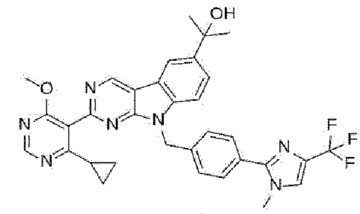
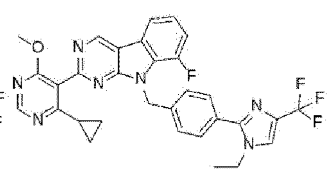
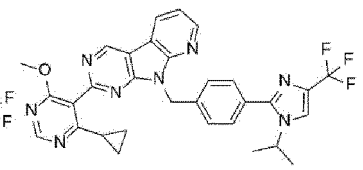

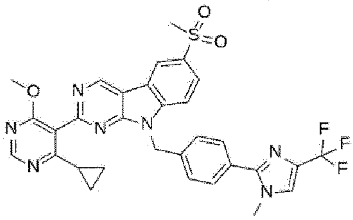
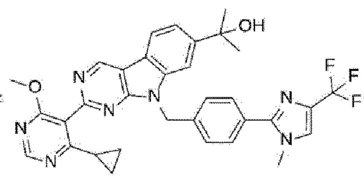
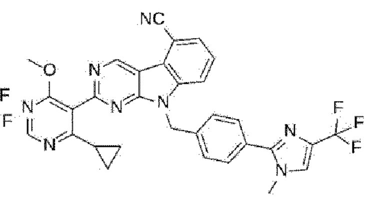

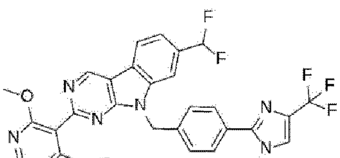
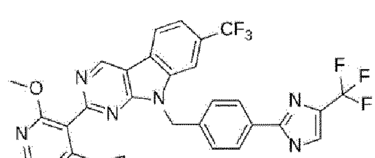
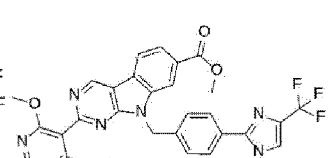

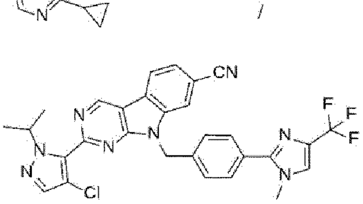
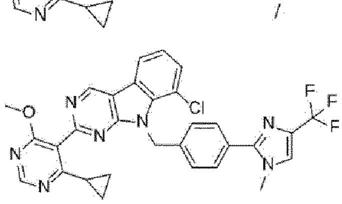
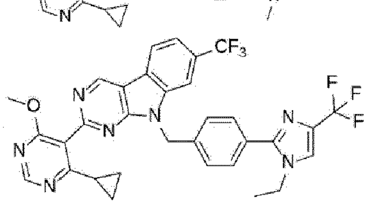

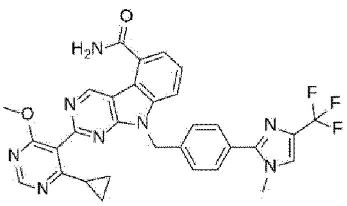
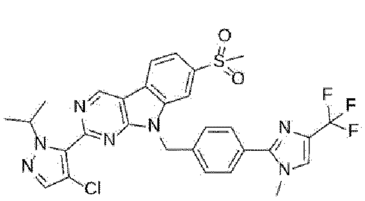
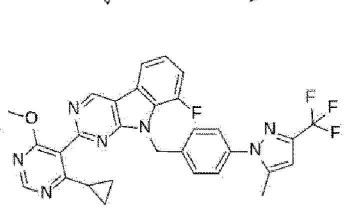

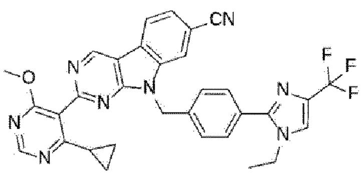 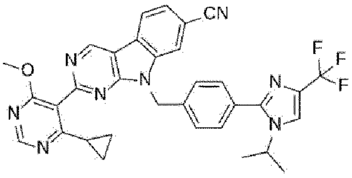 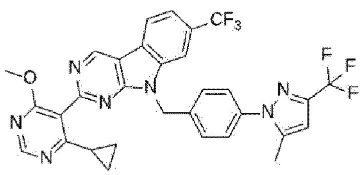

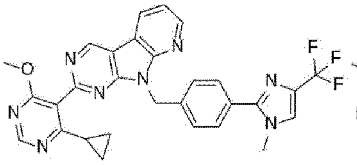 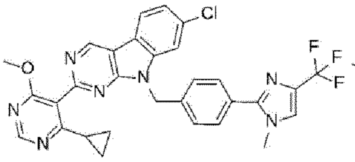 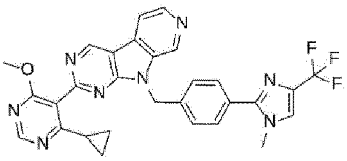

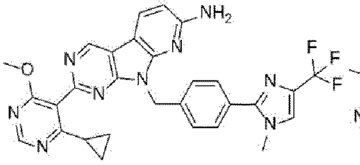 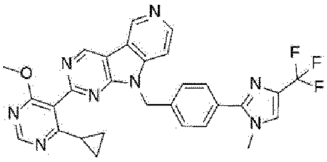 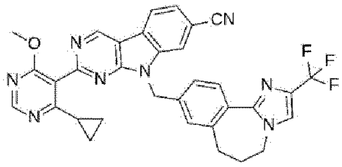

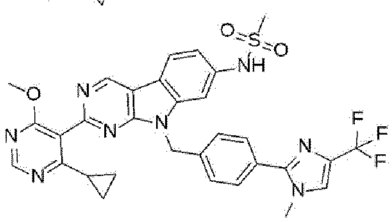 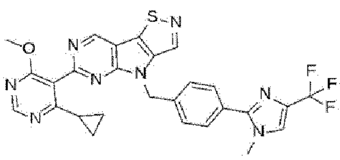 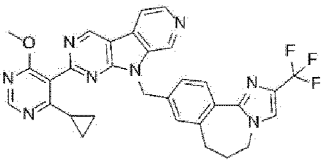

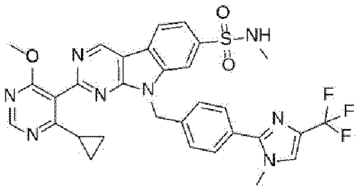 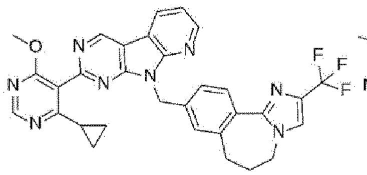 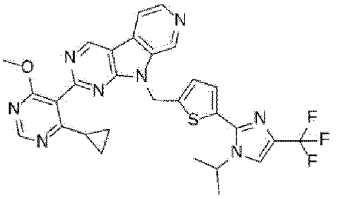

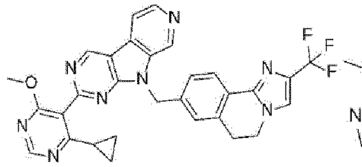 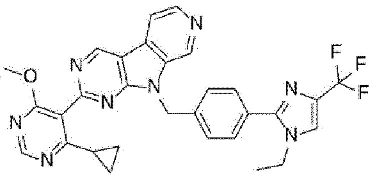 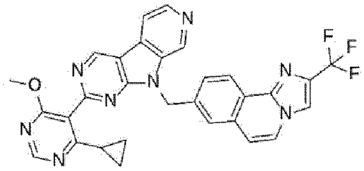

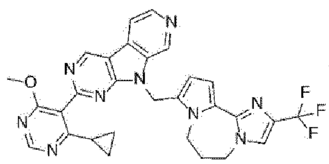 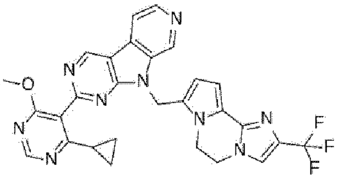 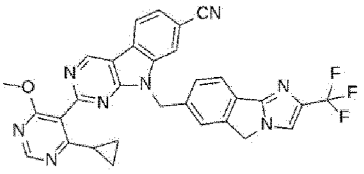

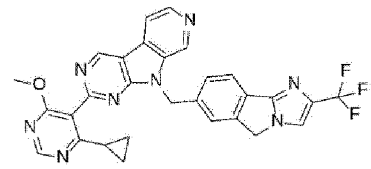 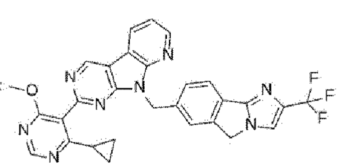 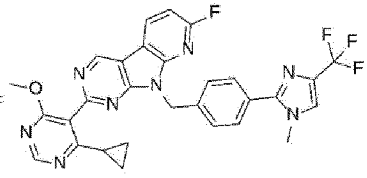

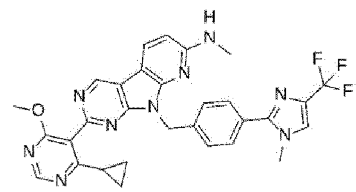 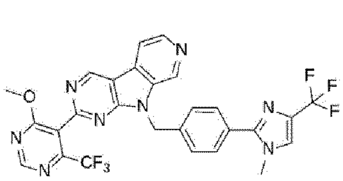 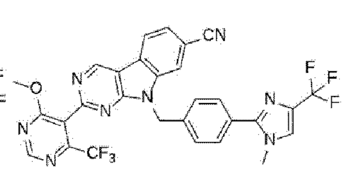

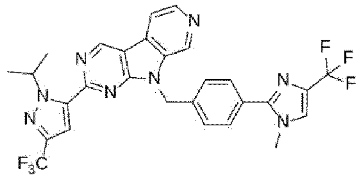
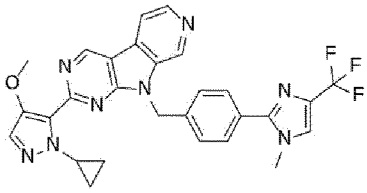
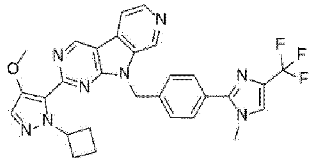

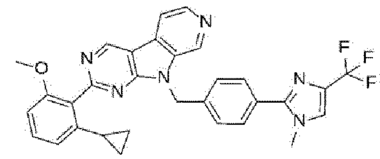
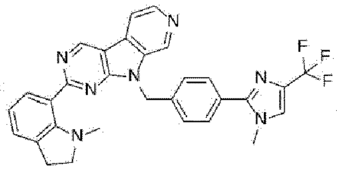
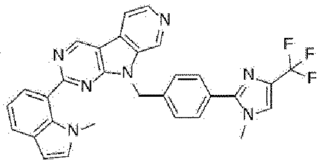

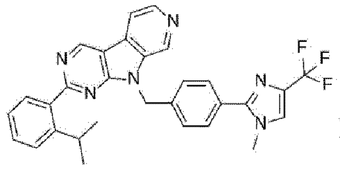
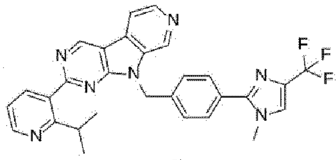
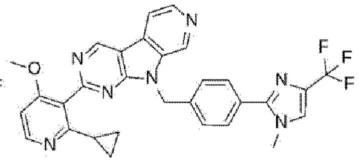

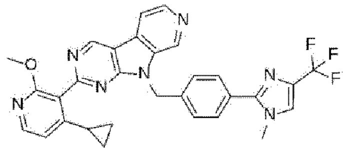
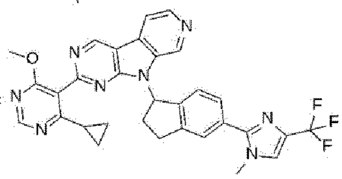
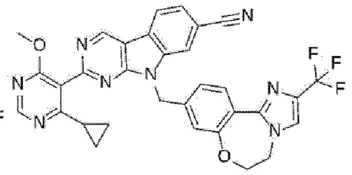

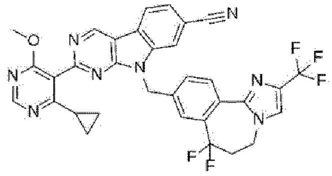
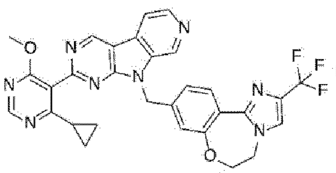
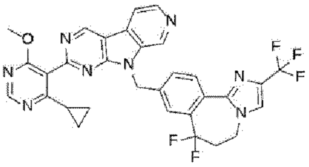

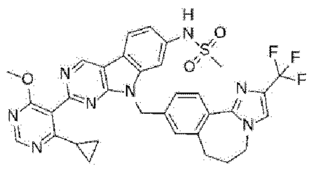
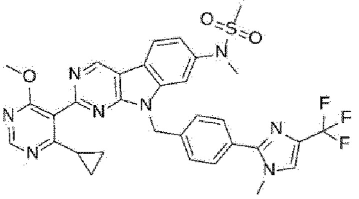
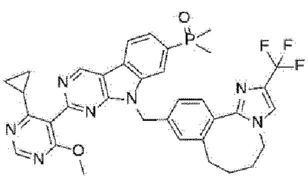

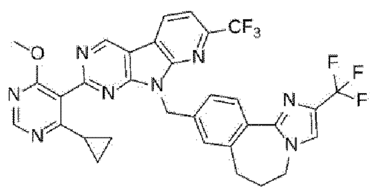
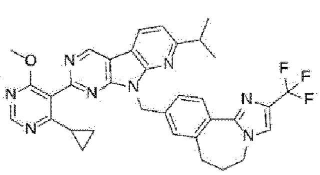
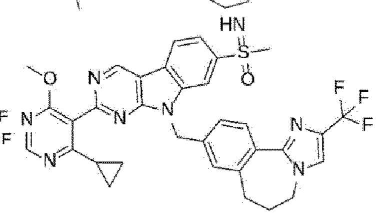

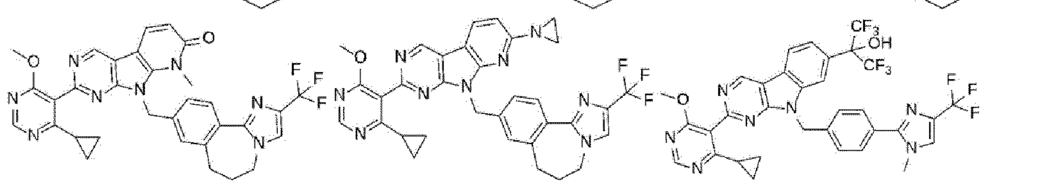

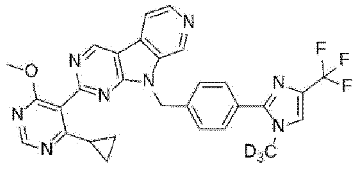
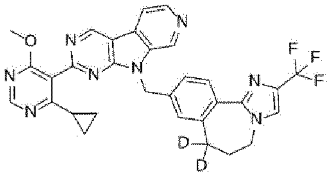
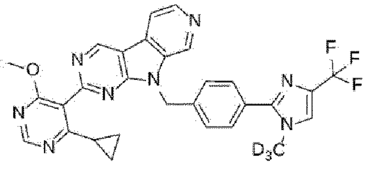

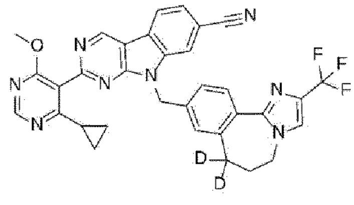
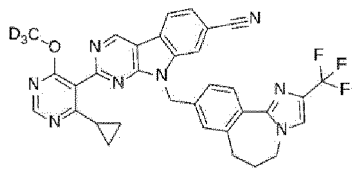
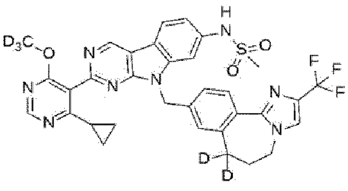
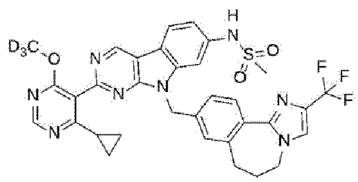
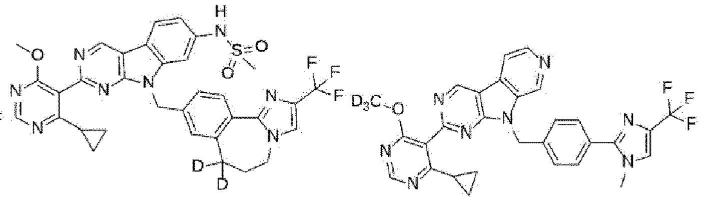
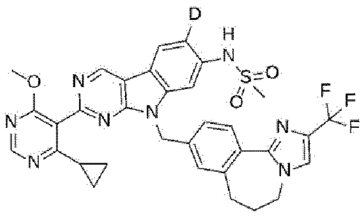
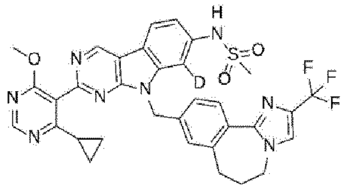
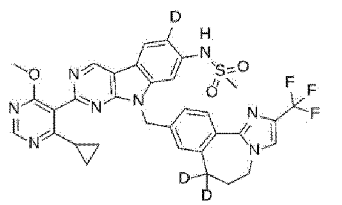
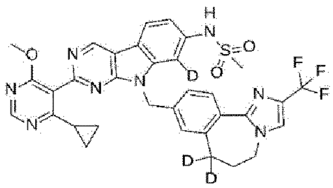
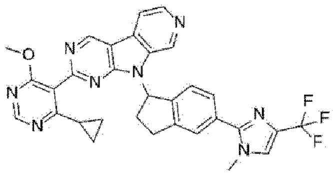
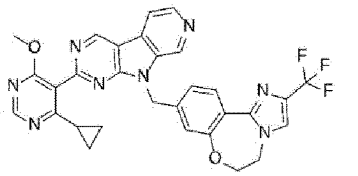
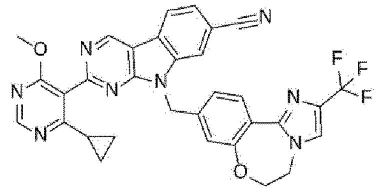
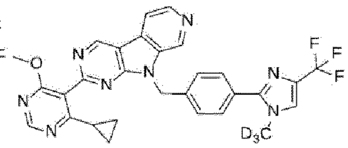
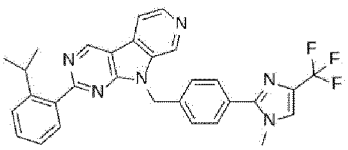
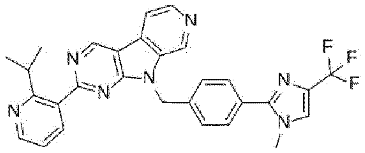
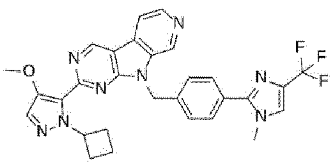
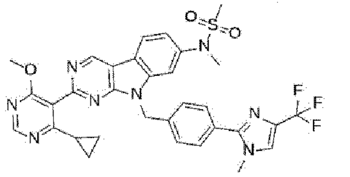
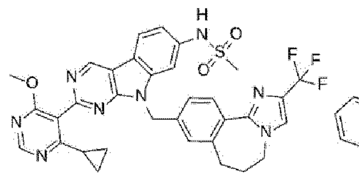
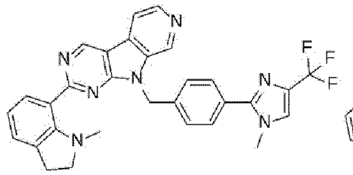
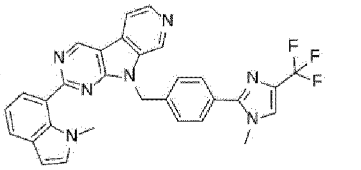
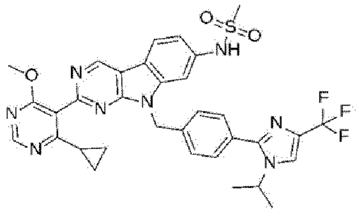
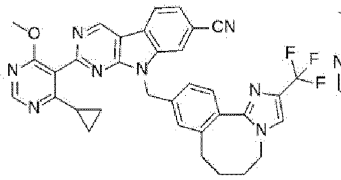
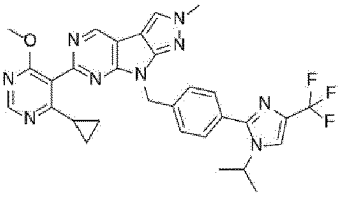

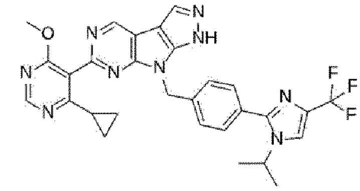
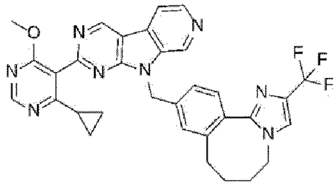
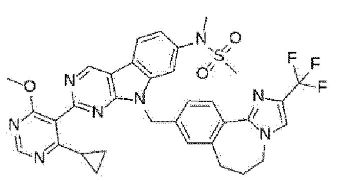

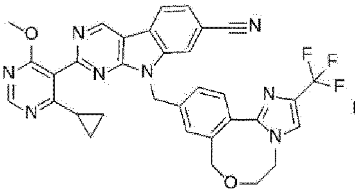
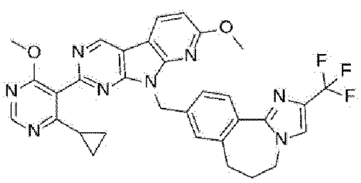
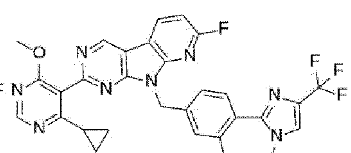

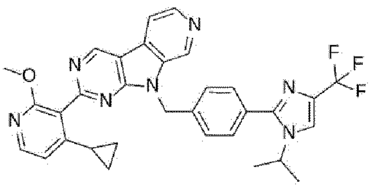
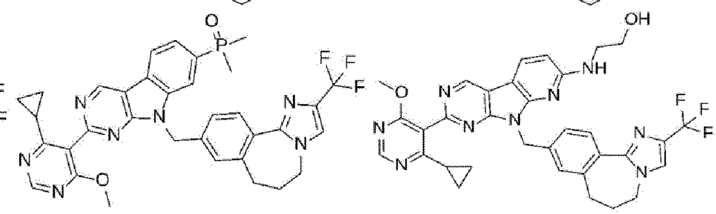

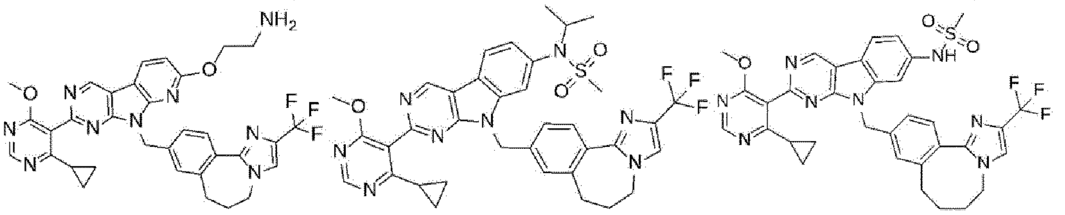

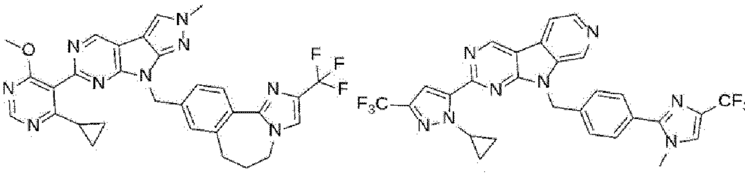
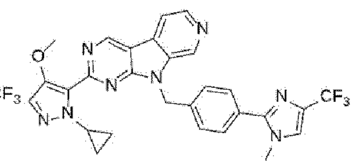

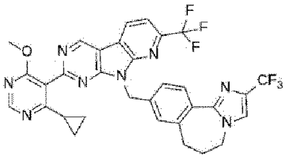
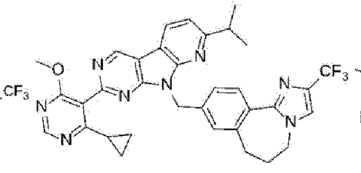
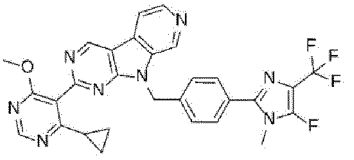

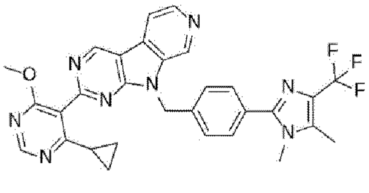
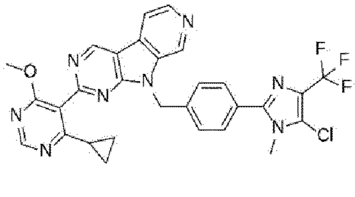
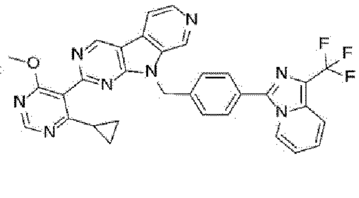

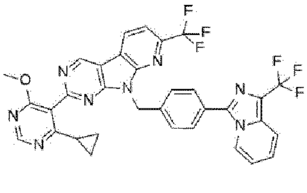
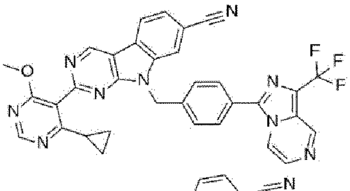
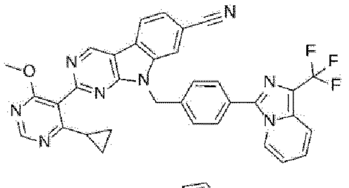

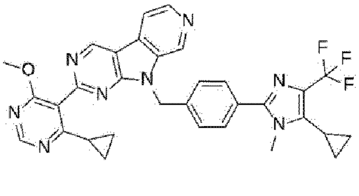
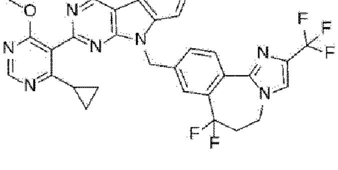
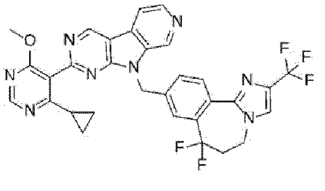

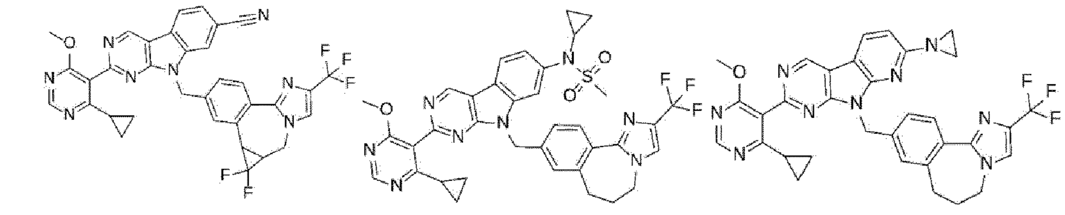

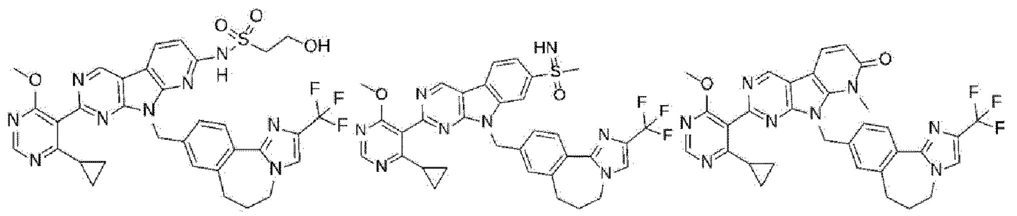

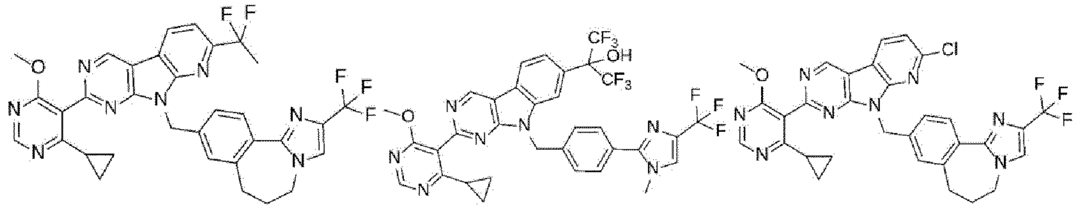

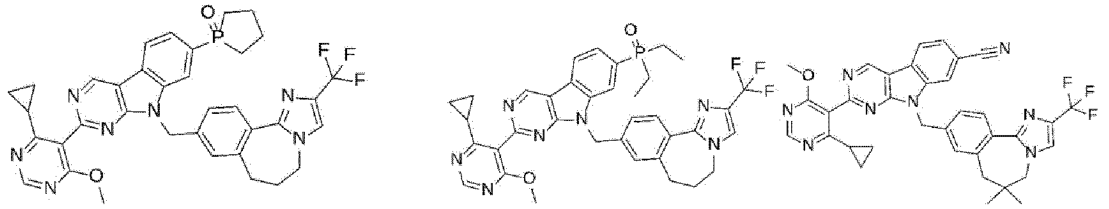

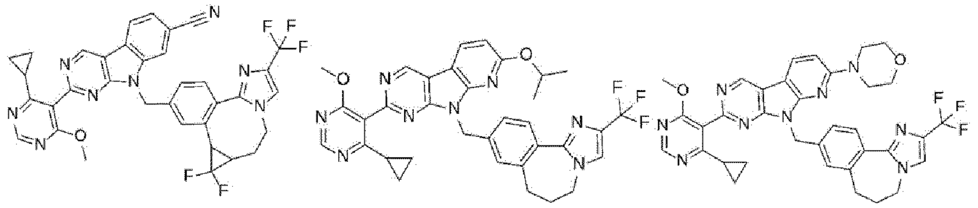

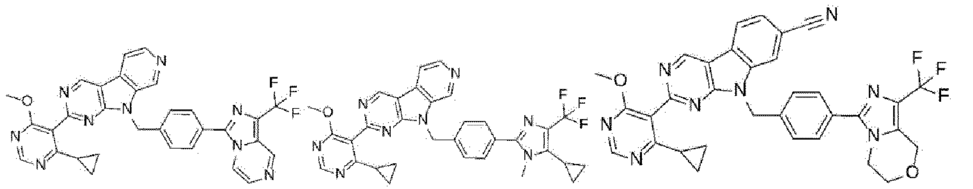

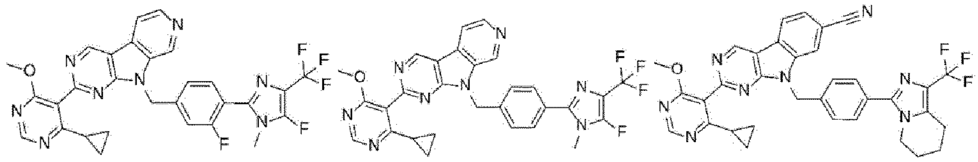

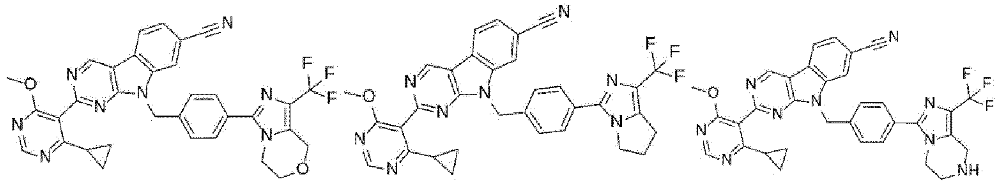

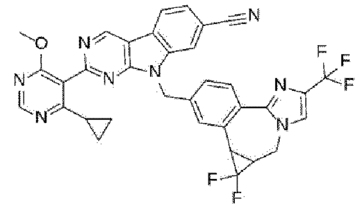 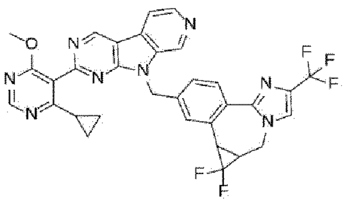 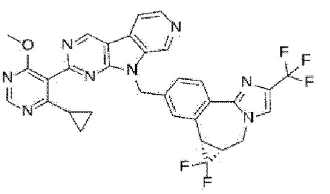

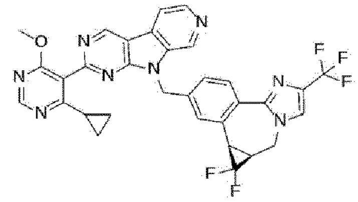 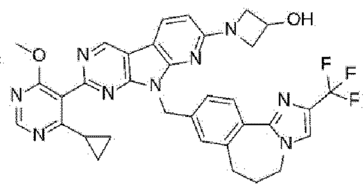 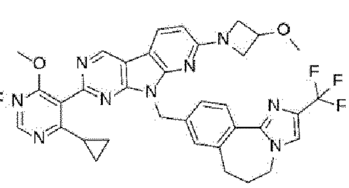

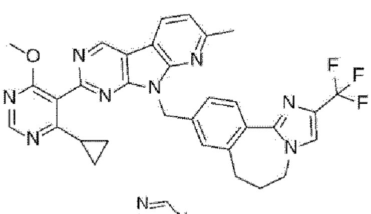 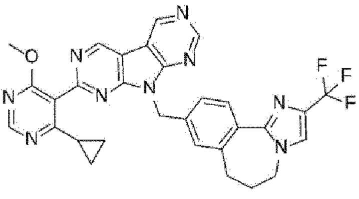 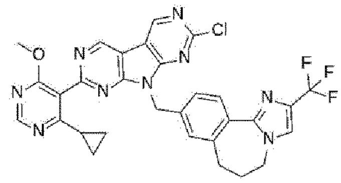

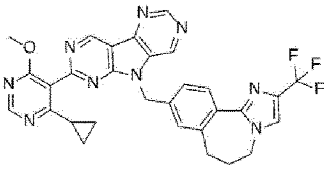 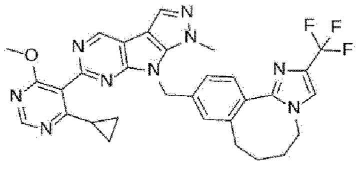 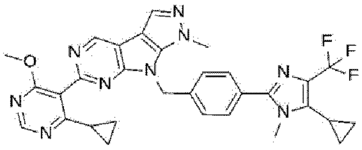

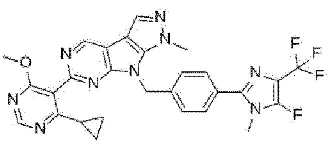 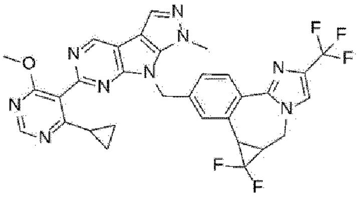 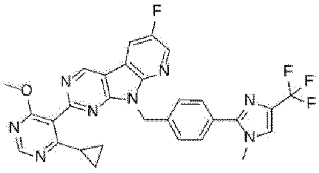

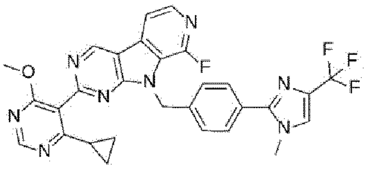 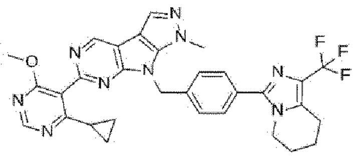 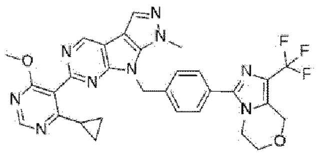

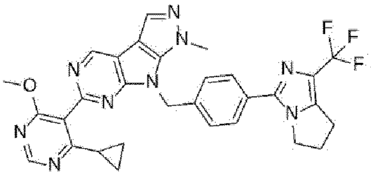 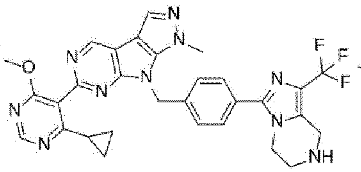 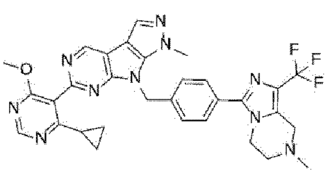

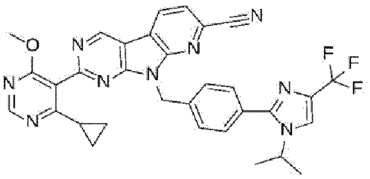 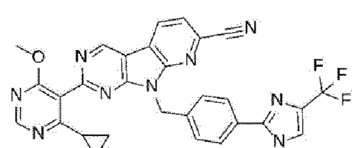 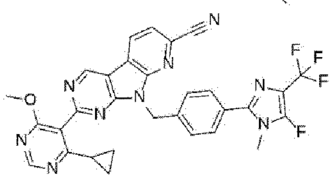

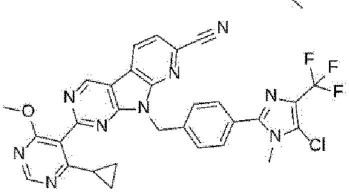 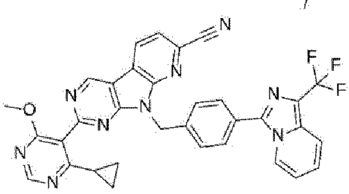 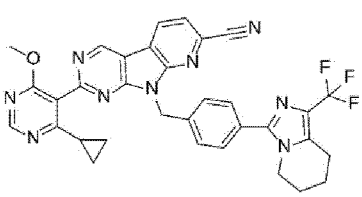

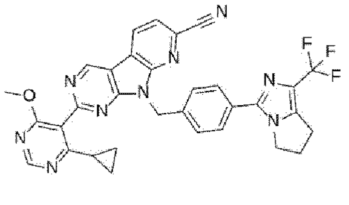 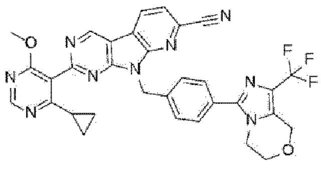 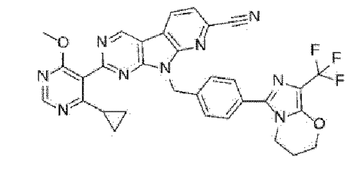

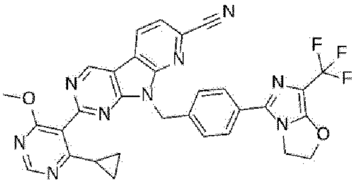 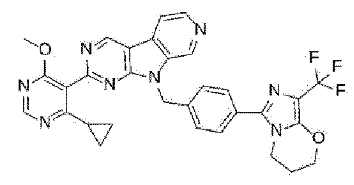 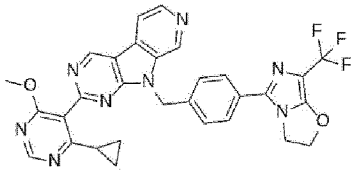

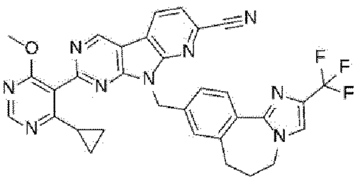 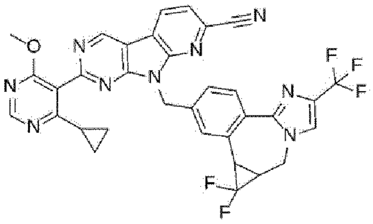 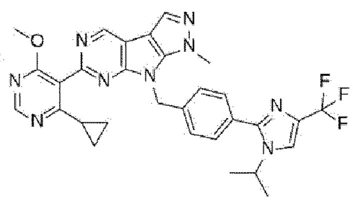

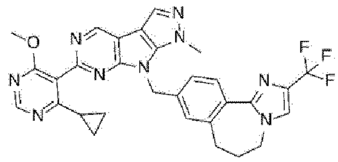 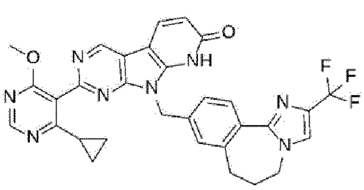 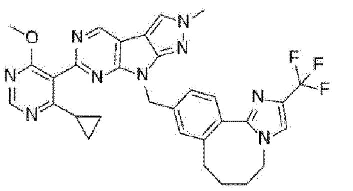

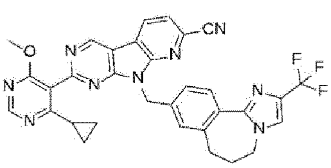 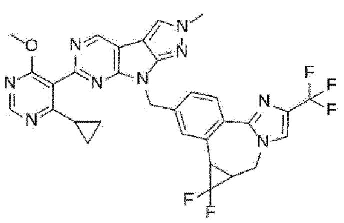 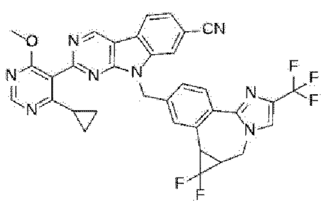

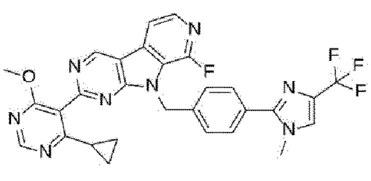 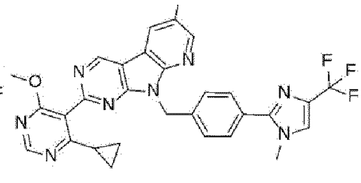 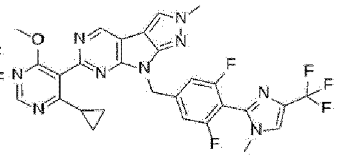

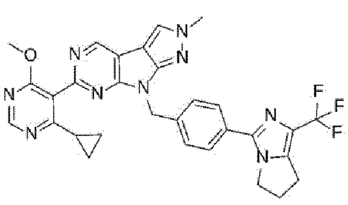 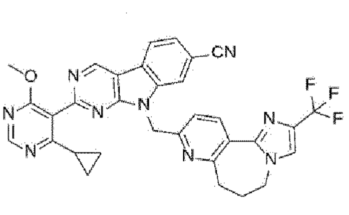 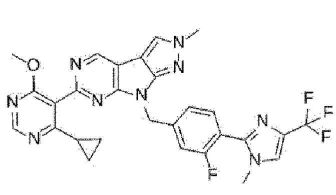

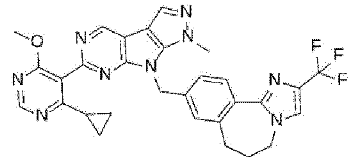
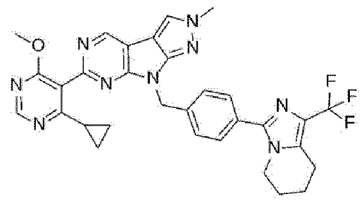
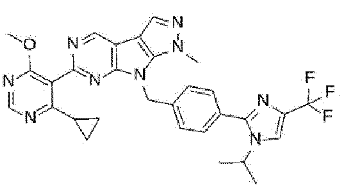

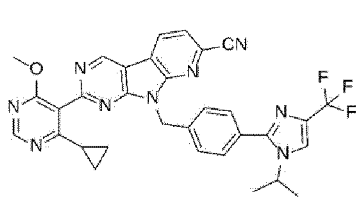
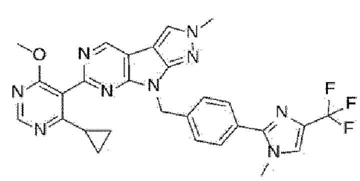
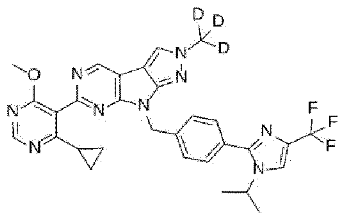

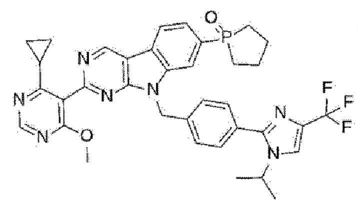
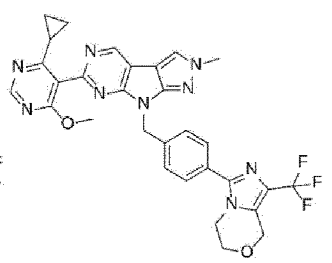
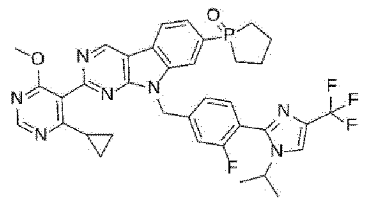

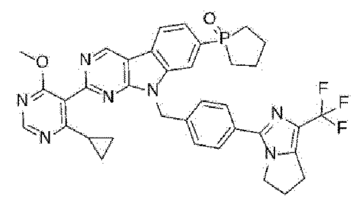
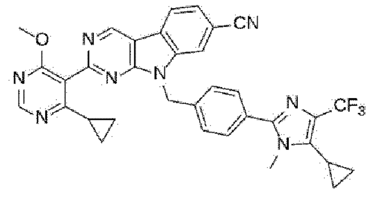
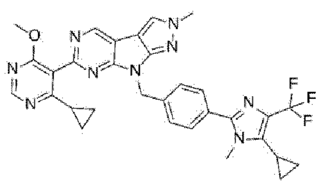

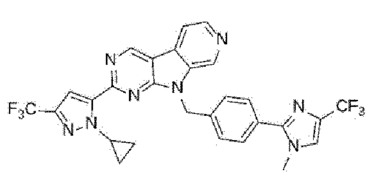
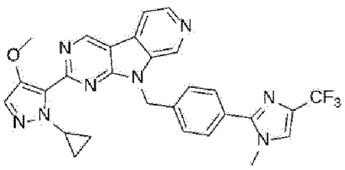
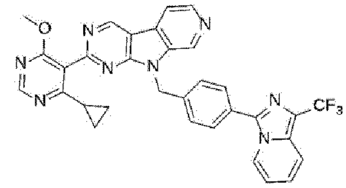

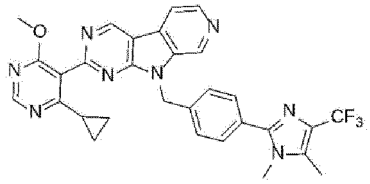
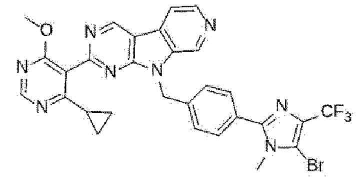
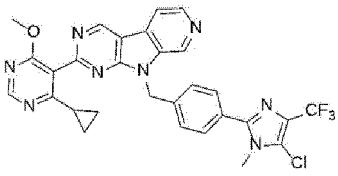

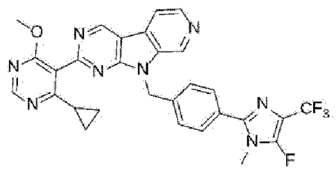
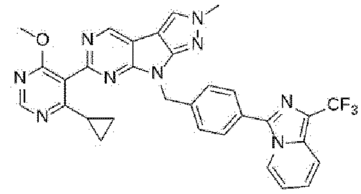
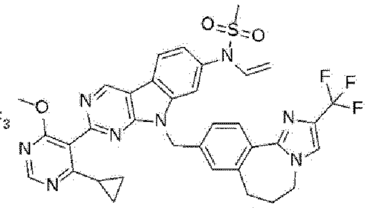

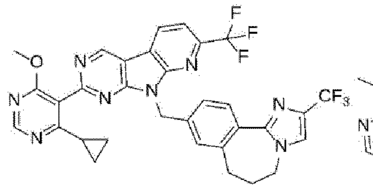
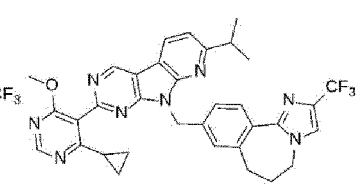
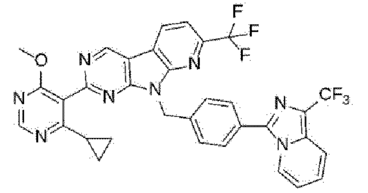

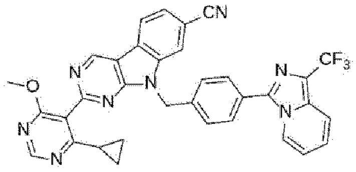
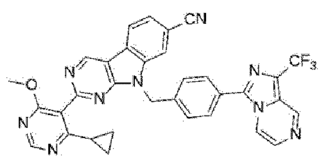
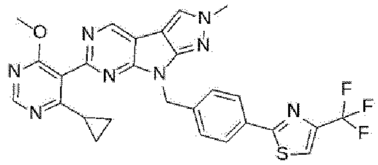

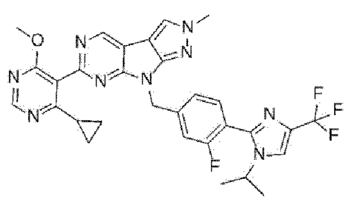
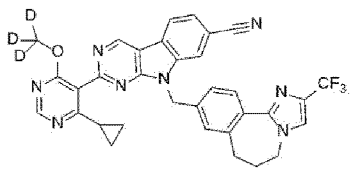
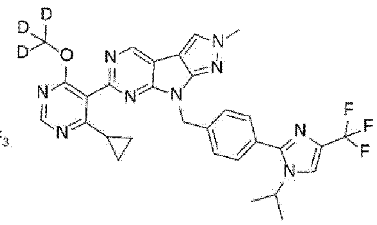

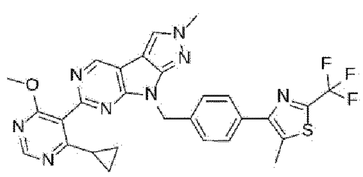
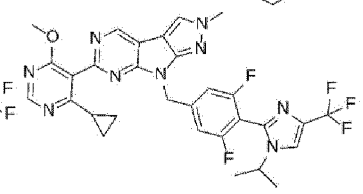
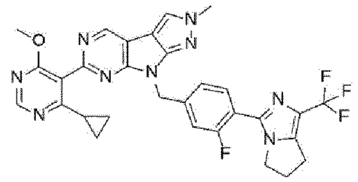

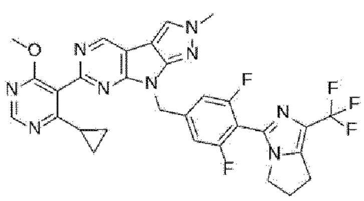
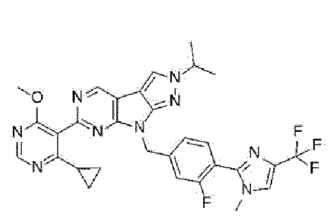
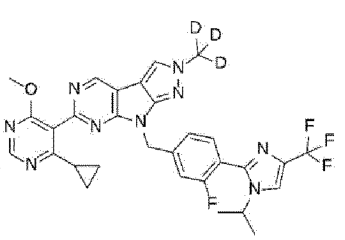

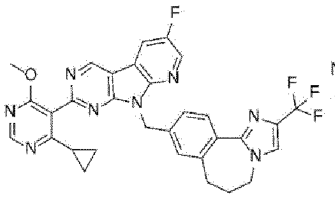
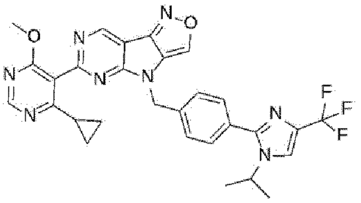
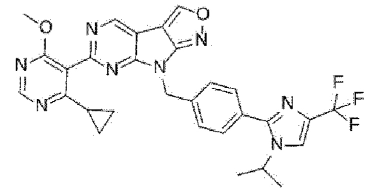

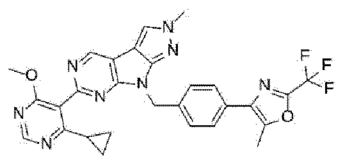
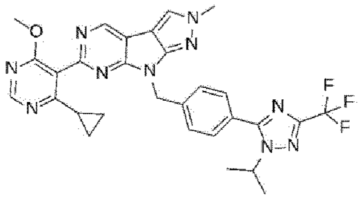
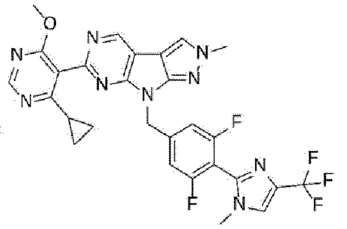

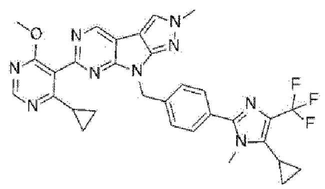
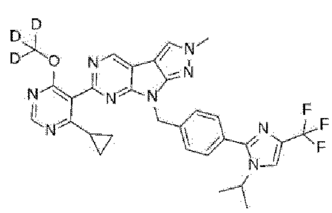
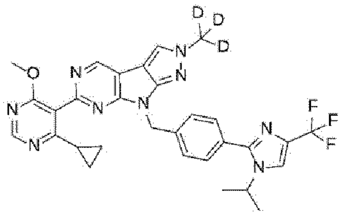

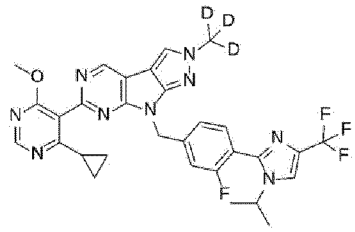
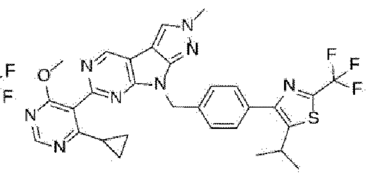
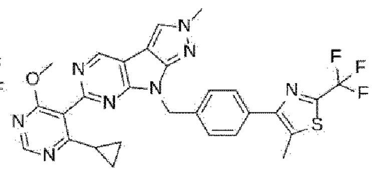

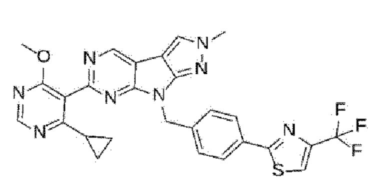
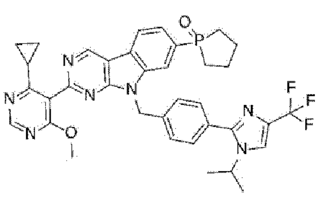
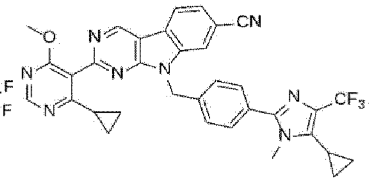

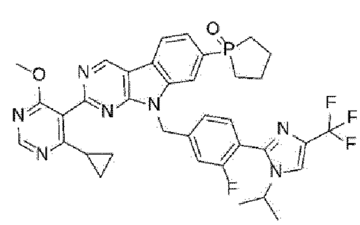
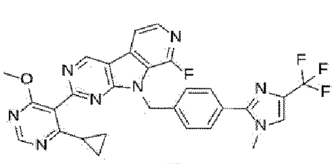
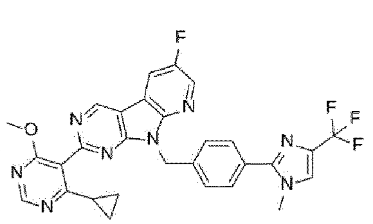

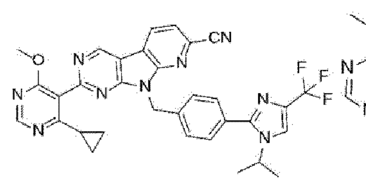
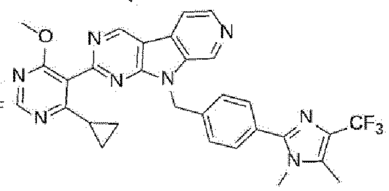
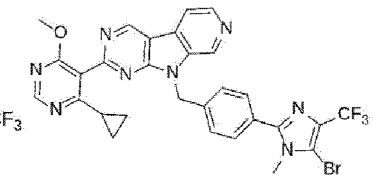

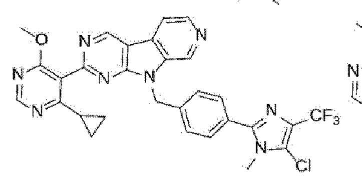
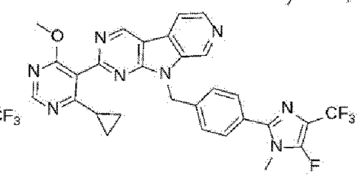
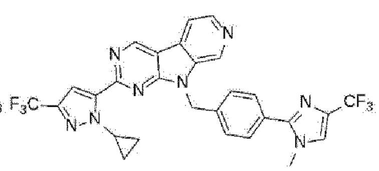

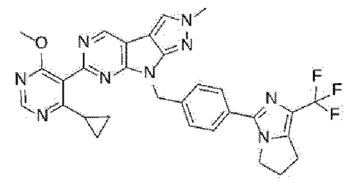
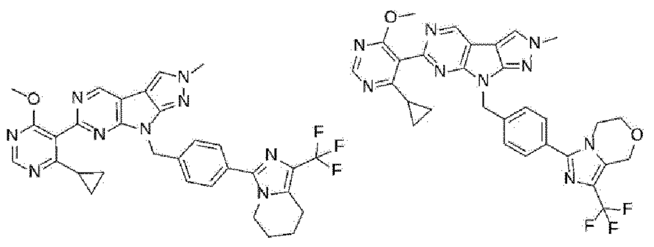

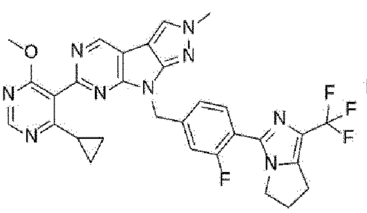
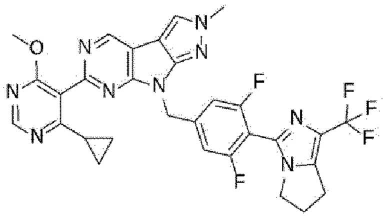
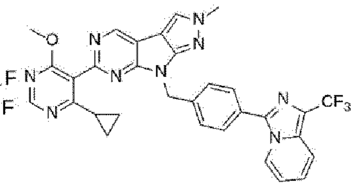

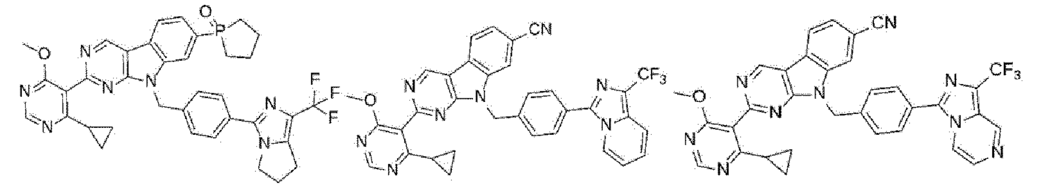

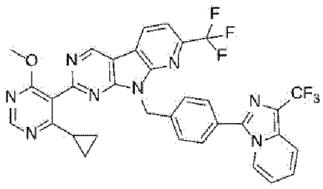
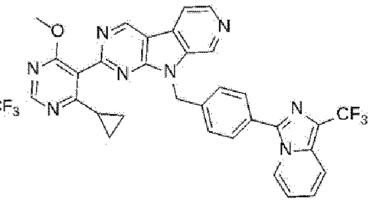
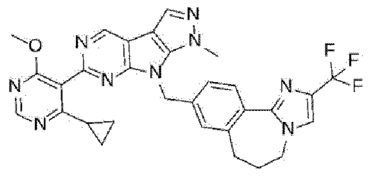

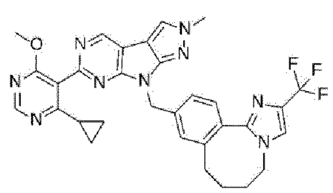
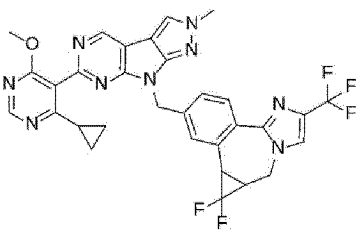
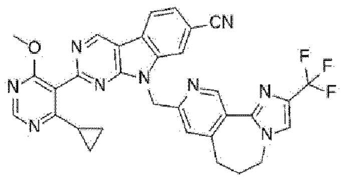

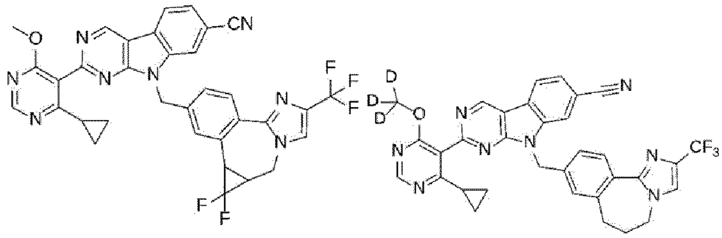
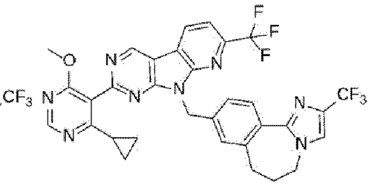

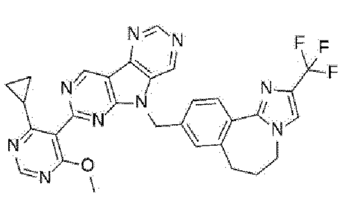
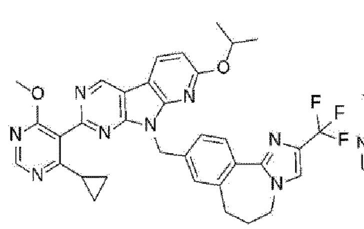
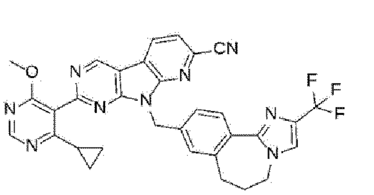.

In a second aspect, the present application provides a preparation method for the compound:

Preparation method I

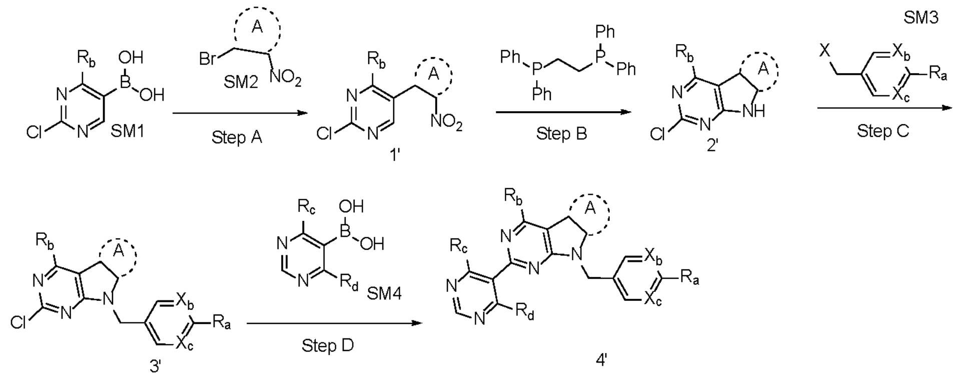


wherein, ring A, $X_b$, $X_c$, ring C, $R_b$, $R_c$, and $R_d$ are defined as mentioned above in the present application;

compound 1' is prepared by reacting SM1 and SM2 under the presence of a base and a catalyst, wherein the base comprises sodium carbonate, potassium carbonate, $K_3PO_4$, $Na_2CO_3$, CsF, $Cs_2CO_3$, t-Bu-Na and the like, and the catalyst comprises $Pd(PPh_3)_4$, $Pd(dppf)Cl_2$ and the like;

compound 2' is prepared by reacting compound 1' with 1,2-bis(diphenylphosphino)ethane;

compound 3' is prepared by a substitution reaction between compound 2' and SM3 under the presence of a base, wherein the base comprises sodium hydride, sodium hydroxide, potassium hydroxide, sodium tert-butoxide, potassium tert-butoxide and the like; and

compound 4' is prepared by a coupling reaction between compound 3' and SM4 under the presence of a base and a catalyst, wherein the base comprises sodium carbonate, potassium carbonate, $K_3PO_4$, $Na_2CO_3$, CsF, $Cs_2CO_3$, t-Bu-Na and the like, and the catalyst comprises $Pd(PPh_3)_4$, $Pd(dppf)Cl_2$ and the like.

Preparation method II

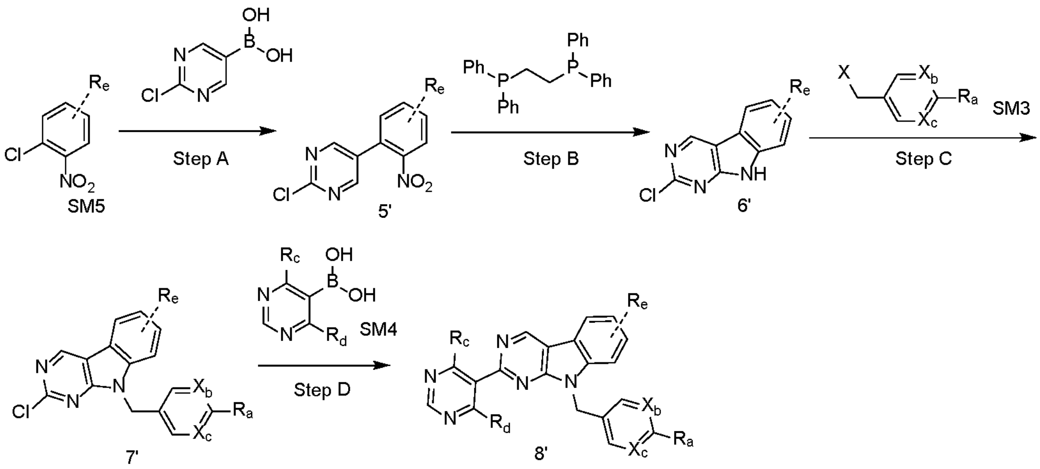


wherein, $X_b$, $X_c$, ring C, $R_c$, $R_d$, and $R_a$ are defined as mentioned above in the present application;

compound 5' is prepared by reacting SM5 under the presence of a base and a catalyst, wherein the base comprises sodium carbonate, potassium carbonate, $K_3PO_4$, $Na_2CO_3$, CsF, $Cs_2CO_3$, t-Bu-Na and the like, and the catalyst comprises $Pd(PPh_3)_4$, $Pd(dppf)Cl_2$ and the like;

compound 6' is prepared by reacting compound 5' with 1,2-bis(diphenylphosphino)ethane;

compound 7' is prepared by a substitution reaction between compound 6' and SM3 under the presence of a base,

wherein the base comprises sodium hydride, sodium hydroxide, potassium hydroxide, sodium tert-butoxide, potassium tert-butoxide and the like; and

compound 8' is prepared by reacting compound 7' under the presence of a base and a catalyst, wherein the base comprises sodium carbonate, potassium carbonate, $K_3PO_4$, $Na_2CO_3$, CsF, $Cs_2CO_3$, t-Bu-Na and the like, and the catalyst comprises $Pd(PPh_3)_4$, $Pd(dppf)Cl_2$ and the like.

In a third aspect, the present application provides a pharmaceutical composition, comprising a therapeutically effective amount of the compound, or the isomer thereof or the pharmaceutically acceptable salt thereof, and a pharmaceutically acceptable carrier.

The term "pharmaceutically acceptable carrier" refers to a medium generally acceptable in the art for the delivery of a biologically active agent to an animal, in particular a mammal. Depending on the mode of administration and the nature of dosage form, the pharmaceutically acceptable carrier comprises, for example, adjuvant, excipient or vehicle, such as diluent, preservative, filler, flow modifier, disintegrating agent, humectant, emulsifier, suspending agent, sweetener, flavoring agent, aromatizer, antibacterial agent, antifungal agent, lubricant, and dispersant. Pharmaceutically acceptable carrier may be formulated based on the capabilities of a person of ordinary skills in the art in view of a large number of factors, including but not limited to: type and nature of active agent being formulated, the subject to which the composition comprising the agent is to be administered, the intended route of administration of the composition, and the target therapeutic indication(s). Pharmaceutically acceptable carrier comprises both aqueous and non-aqueous media and various solid and semi-solid dosage forms. In addition to the active agent, the carrier comprises many different ingredients and additives, and such additional ingredients included in the composition are well known to those of ordinary skill in the art for a variety of reasons (e.g., stabilizing the active agent, binders, etc.).

A fourth aspect of the present application provides the compound, or the isomer thereof or the pharmaceutically acceptable salt thereof in the manufacture of a medicament for treating related diseases mediated by a USP1 target.

In some embodiments of the present application, the related diseases mediated by a USP1 target comprise cellular inflammatory diseases, neurodegenerative diseases, and cancers.

## TECHNICAL EFFECT

The compound of the present application has significant USP1 enzymatic inhibitory activity and can be used in the treatment of cancer.

## DESCRIPTION AND DEFINITION

Unless otherwise indicated, the following terms and phrases used herein are intended to have the meanings set forth below. A particular term or phrase should not be regarded as indefinite or unclear without special definition, but should be understood in its ordinary meaning.

The term "pharmaceutically acceptable" means those compounds, materials, compositions and/or dosage forms which, within reasonable medical judgment, are suitable for use in contact with human and animal tissues without undue toxicity, irritation, allergic reaction or other problems or complications, and which have a reasonable benefit/risk ratio.

The term "pharmaceutically acceptable salt" refers to derivatives of the compound of the application prepared with relatively non-toxic acids or bases. The salt may be prepared during the synthesis, isolation, or purification of the compound, or prepared separately by reacting the free form of a purified compound with a suitable acid or base. When the compound contains a relatively acidic functional group, the salt comprises alkali addition salts obtained by reacting with an alkali metal or alkaline earth metal hydroxid or an organic amine, including cations based on alkali metals and alkaline earth metals, as well as non-toxic ammonium, quaternary ammonium and amine cations, and also salts of amino acids and the like. When the compound contains a relatively basic functional group, the salt comprises acid addition salts obtained by reacting with an organic or inorganic acid.

The term "excipient" generally refers to the carrier, diluent and/or medium required to formulate an effective pharmaceutical composition.

The term "therapeutically effective amount" or "prophylactically effective amount" refers to a sufficient amount of the compound or the pharmaceutically acceptable salt thereof of the present application, to treat a disorder with a reasonable effect/risk ratio suitable for any medical therapy and/or prophylaxis. It should be recognized, however, that the total daily dosage of the compound represented by formula (II') or the pharmaceutically acceptable salt thereof or the composition of the present application is subject to the decision of the attending physician within the bounds of sound medical judgment. For any particular patient, the specific therapeutically effective amount level shall be based on a variety of factors, including the disorder being treated and the severity of the disorder; the activity of the specific compound employed; the specific composition employed; the age, body weight, general health, gender, and diet of the patient; the time of administration, route of administration, and rate of excretion of the specific compound employed; the duration of the

treatment; the medicament that is administrated in combination with or simultaneously with the specific compound employed; and similar factors that are well known in the medical field. For example, it is a practice in the art to start the dosage of a compound at a level below that required to obtain the desired therapeutic effect and gradually increase the dosage until the desired effect is obtained.

Unless otherwise specified, the term "5-10 membered heteroaryl" refers to a monocyclic or bicyclic group having a conjugated $\pi$-electron system composed of 5 to 10 ring atoms, 1, 2, 3 or 4 of which are heteroatom(s) independently selected from the group consisting of O, S, and N, the rest of them being carbon atoms. Among them, each N atom is optionally quaternized, and each N and S heteroatom is optionally oxidized (i.e., NO and $S(O)_p$, p being 1 or 2). The 5-6 membered heteroaryl may be attached to other moieties of the compound via the heteroatom or the carbon atom. Examples of the 5-6 membered heteroaryl include, but are not limited to, pyrrolyl (comprising N-pyrrolyl, 2-pyrrolyl, 3-pyrrolyl, and the like), pyrazolyl (comprising 2-pyrazolyl, 3-pyrazolyl, and the like), imidazolyl (comprising N-imidazolyl, 2-imidazolyl, 4-imidazolyl, 5-imidazolyl, and the like), oxazolyl (comprising 2-oxazolyl, 4-oxazolyl, 5-oxazolyl, and the like), triazolyl (1*H*-1,2,3-triazolyl, 2*H*-1,2,3-triazolyl, 1*H*-1,2,4-triazolyl, 4*H*-1,2,4-triazolyl, and the like), tetrazolyl, isoxazolyl (comprising 3-isoxazolyl, 4-isoxazolyl, and 5-isoxazolyl, and the like), thiazolyl (comprising 2-thiazolyl, 4-thiazolyl, 5-thiazolyl, and the like), furanyl (comprising 2-furanyl, 3-furanyl, and the like), thienyl (comprising 2-thienyl, 3-thienyl, and the like), pyridinyl, pyrimidinyl, benzimidazolyl, and the like.

Unless otherwise specified, the term "fused-heterocyclyl" refers to a saturated or partially saturated non-aromatic cyclic group formed by two or more ring structures sharing two adjacent atoms with each other and containing at least one heteroatom as ring atom; the heteroatom is generally N, O, or S; the carbon ring atoms and the heteroatoms in the fused-heterocyclyl may be further oxidized to form cyclic moieties containing C(O), NO, SO, or $S(O)_2$ groups, which are also encompassed within the definition of heterocyclyl as described in the present application. The term "non-aromatic" in this definition means that the group is not aromatic when it exists on its own. The 11-14 membered fused-heterocyclyl as defined in the present application comprises "11-14 membered saturated fused-heterocyclyl" and "11-14 membered partially saturated fused-heterocyclyl". The fused-heterocyclyl may be 5-6 membered heterocyclyl fused with 5-6 membered heterocyclyl, 5-6 membered heterocyclyl fused with 5-6 membered cycloalkyl, phenyl fused with 5-6 membered heterocyclyl, phenyl fused with 5-6 membered saturated heterocyclyl, 5-6 membered heteroaryl fused with 5-6 membered heterocyclyl, 5-6 membered heteroaryl fused with 5-6 membered saturated heterocyclyl, phenyl fused with 5-6 membered heterocyclyl and fused with 5-6 membered heterocyclyl, 5-6 membered heteroaryl fused with 5-6 membered heterocyclyl and fused with 5-6 membered heterocyclyl, phenyl fused with 5-6 membered cycloalkyl and fused with 5-6 membered heterocyclyl, 5-6 membered heteroaryl fused with 5-6 membered cycloalkyl and fused with 5-6 membered heterocyclyl; specific examples of the fused-heterocyclyl include, but are not limited to:

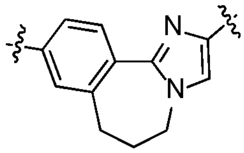 , 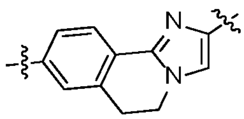 ,

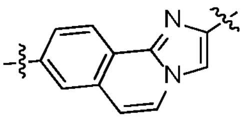 , 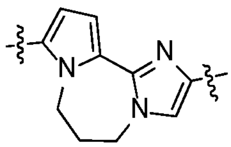 , 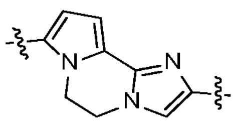 , and 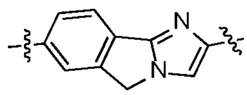 .

Unless otherwise specified, the term "heterocyclyl" refers to a substituted or unsubstituted, saturated or unsaturated non-aromatic ring comprising 1-3 heteroatoms selected from N, O and S. Any carbon group(s) or heteroatom(s) in the heterocyclyl may be oxo-substituted, e.g., the carbon group may form -C(O)-. The "heterocyclyl" of the present application refers to a non-aromatic cyclic group containing at least one heteroatom as ring atom(s), which is derived from the removal of a hydrogen atom. Heterocyclyl comprises a saturated or partially saturated monocyclic heterocyclyl; and the heterocyclyl is independent of the linking site (*i.e.,* it can be linked by either a carbon atom or a heteroatom). Examples of "heterocyclyl" include, but are not limited to,

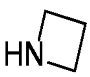 , 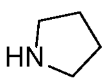 , 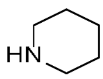 , 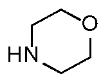 , 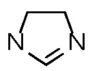 , and 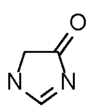 .

Unless otherwise specified, the term "cycloalkyl" refers to a saturated monocyclic or multicyclic hydrocarbyl

group. The cycloalkyl is preferably $C_{3-12}$ cycloalkyl, more preferably $C_{3-8}$ cycloalkyl, further preferably $C_{5-6}$ cycloalkyl. Examples of cycloalkyl include, but are not limited to, cyclopentyl and cyclohexyl.

Unless otherwise specified, the term "cycloalkylene" refers to a divalent group formed by further removal of a hydrogen atom from a cycloalkyl.

Unless otherwise specified, the term "halogen" refers to a fluorine, chlorine, bromine or iodine atom.

Unless otherwise specified, the term "$C_{1-4}$ alkyl" refers to a saturated hydrocarbyl group with a $C_{1-4}$ straight or branched chain. Examples of alkyl include, but are not limited to, methyl, ethyl, n-propyl, isopropyl, butyl, isobutyl, and the like.

Unless otherwise specified, the term "alkylene" refers to a straight or branched saturated aliphatic hydrocarbon group which is a residue derived from a parent alkane by removing two hydrogen atoms from the same carbon atom or from two different carbon atoms. Alkylene may be a straight or branched group comprising from 1 to 20 carbon atoms, preferably from 1 to 12 (e.g., 1, 2, 3, 4, 5, 6, 7, 8, 9, 10, 11 or 12) carbon atoms, more preferably from 1 to 4 carbon atoms. Alkylene includes, but is not limited to, methylene ($-CH_2-$), 1,1-ethylidene ($-CH(CH_3)-$), 1,2-ethylene ($-CH_2CH_2-$), 1,1-propylidene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$) and the like.

Unless otherwise specified, the term "$C_{1-4}$ haloalkyl" refers to an alkyl group in which one or more hydrogen atom(s) has been replaced by halogen atom(s). Examples include, but are not limited to, monofluoromethyl, difluoromethyl, trifluoromethyl, trichloromethyl, tribromomethyl, 2,2,2-trifluoroethyl, 2,2,2-trichloroethyl, and the like.

Unless otherwise specified, the term "$C_{1-4}$ alkoxy" refers to a $C_{1-4}$ alkyl group linked by an oxygen bridge. Examples include, but are not limited to, methoxy, ethoxy, n-propoxy, isopropoxy, n-butoxy, sec-butoxy, and tert-butoxy.

Unless otherwise specified, the term "$-C_{1-4}$ alkyl-OH" refers to a $C_{1-4}$ alkyl having a hydroxyl substituent. Examples include, but are not limited to, $-CH_2-OH$, $-(CH_2)_2-OH$, and $-CH(CH_3)_2-OH$.

Unless otherwise specified, the term "$C_{3-6}$ cycloalkyl" refers to a 3-6 membered monocycloalkyl. Examples of such monocycloalkyl groups include, but are not limited to, cyclopropyl, cyclobutyl, cyclopentyl, cyclohexyl, cycloheptyl, and cyclooctyl.

Unless otherwise specified, the term "5-6 membered heteroaryl comprising 1 to 4 N atom(s)" refers to a 5-6 membered heteroaryl in which carbon atom(s) in the ring is/are replaced by 1, 2, 3 or 4 nitrogen atoms. Examples include, but are not limited to,

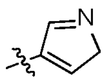, 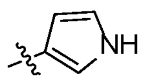, 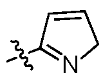,

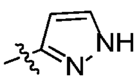, 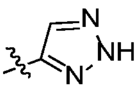, 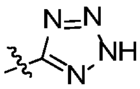, 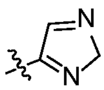, 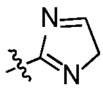, 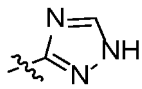, 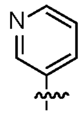, and 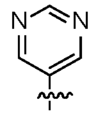.

Unless otherwise specified, in the structure

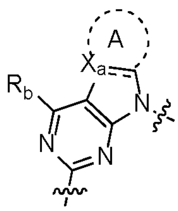,

"- - -" refers to a single bond or a double bond.

## BRIEF DESCRIPTION OF THE DRAWINGS

The accompanying drawings illustrated herein are provided for further understanding of the present application and form part of the present application, and the schematic embodiments of the present application and related description are used to explain the present application and do not constitute an undue limitation of the present application.

FIG. 1 shows tumor growth in mice in groups G1 to G4 in Test Example 5 of the present application.

FIG. 2 shows the change in body weight of mice in groups G1 to G4 in Test Example 5 of the present application.

## DETAILED DESCRIPTION OF THE INVENTION

For the sake of clarity of the purpose, technical solutions and advantages of the present application, the application is further described in detail by referring to the drawings and examples. Obviously, the described examples are only a part of the examples of the present application, rather than all the examples. All other examples obtained by those of ordinary skills in the art based on the present application shall fall within the protection scope of this application.

The compounds of the present application can be prepared by a variety of synthetic methods known to those skilled in the art, including the specific embodiments enumerated below, embodiments resulting from combinations thereof with other methods of chemical synthesis, and equivalent substitutions known to those skilled in the art, with preferred embodiments including, but not limited to, the embodiments of the present application.

The solvents used in this application are commercially available.

The structures of the compounds of the present application are determined by nuclear magnetic resonance (NMR) or/and liquid chromatography-mass spectrometry (LC-MS), or ultra performance liquid chromatography-mass spectrometry (UPLC-MS). NMR chemical shifts ($\delta$) are given in parts per million (ppm). NMR was determined using a Bruker Neo 400M or Bruker Ascend 400 NMR instrument, and the solvents were deuterated dimethyl sulfoxide (DMSO-$d_6$), deuterated methanol ($CD_3OD$), deuterated chloroform ($CDCl_3$), and heavy water ($D_2O$), and the internal standard was tetramethylsilane (TMS).

Liquid chromatography-mass spectrometry (LC-MS) was performed using an Agilent 1260-6125B single quadrupole mass spectrometer (ion source was provided by electrospray ionization).

Ultra performance liquid chromatography-mass spectrometry (UPLC-MS) was perfromed using a Waters UPLC H-class SQD mass spectrometer (ion source was provided by electrospray ionization).

HPLC was performed using a Waters e2695-2998 or Waters ARC and Agilent 1260 or Agilent Poroshell HPH high performance liquid chromatography.

Preparative HPLC was performed using Waters 2555-2489 (10 $\mu$m, ODS 250 cm $\times$ 5 cm) or GILSON Trilution LC.

Chiral HPLC was performed using Waters acquity UPC2; and the column was a Daicel chi ring Clpak AD-H (5 $\mu$m, 4.6 $\times$ 250 mm).

Supercritical fluid chromatography (SFC) was performed using Waters SFC 80Q.

The starting materials in the examples of the present application are known and commercially available, or can be synthesized using or according to methods known in the art.

Unless otherwise specified, all reactions of this application are carried out under continuous magnetic stirring under a dry nitrogen or argon atmosphere, with the solvent being a dry solvent and the reaction temperature in degrees Celsius (°C).

The present application will be described in more details by way of examples, which, however, does not imply any unfavorable limitation of the present application. The compounds of the present application can be prepared by a variety of synthetic methods known to those skilled in the art, including the specific embodiments enumerated below, embodiments resulting from combinations thereof with other methods of chemical synthesis, and equivalent substitutions known to those skilled in the art, with preferred embodiments including, but not limited to, the embodiments of the present application. Various variations and improvements to the specific examples of the present application without departing from the spirit and scope of the present application will be apparent to those skilled in the art.

### I. Preparation method

As used herein, room temperature refers to a temperature of about 20°C to 30°C.

### Intermediate INT-1: (4-(1-isopropyl-4-(trifluoromethyl)-1H-imidazol-2-yl)phenyl)methanol

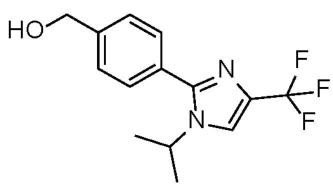

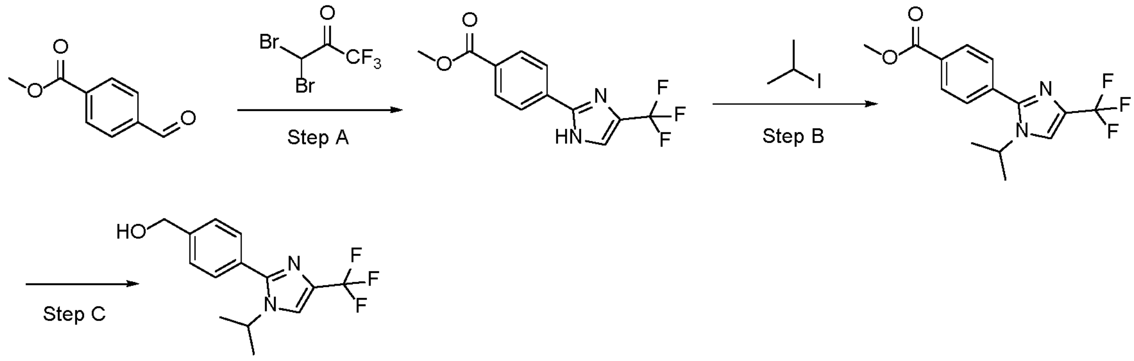


INT-1

**Step A:** At room temperature, 3,3-dibromo-1,1,1-trifluoropropan-2-one (144 g, 0.54 mol) and sodium acetate (80 g, 0.97 mol) were dissolved in water (320 mL). Subsequently, the above solution was warmed up to 90°C and stirred for 0.5 hour. Then the reaction system was cooled to 0°C, and methyl 4-formylbenzoate (80 g, 0.49 mol) and a mixture of 28 wt% aqueous ammonia (400 mL) and methanol (120 mL) were slowly added dropwise to the above reaction solution. Finally the reaction system was continued to be stirred at room temperature for 16 hours.

After the disappearance of raw materials as monitored by LCMS, the reaction solution was filtered and the filter cake was rinsed with ethyl acetate (100 mL × 3 times). The filtrate was then collected and concentrated under reduced pressure. The resulting residue was purified by silica gel column chromatography to obtain 104 g of methyl 4-(4-(trifluoromethyl)-1H-imidazol-2-yl)benzoate.

MS (ESI) M/Z: 271.0 $[M+H]^+$.

**Step B:** At room temperature, methyl 4-(4-(trifluoromethyl)-1H-imidazol-2-yl)benzoate (69 g, 0.25 mol) and cesium carbonate (250 g, 0.76 mol) were dissolved in acetonitrile (1.2 L) and stirred for 2 hours. Subsequently, 2-iodopropane (64.9 g, 0.38 mol) was added to the above solution, and the reaction system was heated to 50°C and stirred for 24 hours. The reaction system was then cooled to 0°C and further 2-iodopropane (21.6 g, 0.13 mol) was added. Then the reaction system was stirred at 50°C for 4 hours.

After the disappearance of raw materials as monitored by LCMS, the reaction solution was quenched by adding to ice water (1.5 L), and the mixture was extracted with ethyl acetate (500 mL × 3 times). Organic phases were combined and then washed with saturated saline (150 mL). Then the resultant was dried with anhydrous sodium sulfate, filtered, and concentrated under reduced pressure. The resulting residue was purified by silica gel column chromatography to obtain 33 g of methyl 4-(1-isopropyl-4-(trifluoromethyl)-1H-imidazol-2-yl)benzoate.

MS (ESI) M/Z: 313.2 $[M+H]^+$.

**Step C:** At room temperature and under nitrogen protection, methyl 4-(1-isopropyl-4-(trifluoromethyl)-1H-imidazol-2-yl)benzoate (33 g, 0.11 mol) was dissolved in dry tetrahydrofuran (528 mL). Subsequently, 2.5 M (mol/L) lithium aluminum hydride solution (85 mL, 0.22 mol) was slowly added to the above solution dropwise at 0°C. Then the reaction system was continued to be stirred at room temperature for 1 hour.

After the disappearance of raw materials as monitored by LCMS, the reaction solution was quenched by slowly adding to ice water (1 L) dropwise. The resultant was filtered and the filter cake was washed with ethyl acetate (100 mL × 3 times). The filtrate was extracted with ethyl acetate (500 mL × 3 times), and organic phases were combined and then washed with saturated saline (150 mL). Then the resultant was dried with anhydrous sodium sulfate, filtered, and concentrated under reduced pressure. The resulting residue was purified by silica gel column chromatography to obtain 28 g of (4-(1-isopropyl-4-(trifluoromethyl)-1H-imidazol-2-yl)phenyl)methanol.

MS (ESI) M/Z: 285.1 $[M+H]^+$.

$^1H$ NMR (400 MHz, $CDCl_3$): δ 7.47 - 7.32 (m, 5H), 4.72 (s, 2H), 4.59 - 4.46 (m, 1H), 2.97 (br, 1H), 1.44 (d, $J$ = 6.8 Hz, 6H).

**Intermediate INT-2: 2-(4-(bromomethyl)phenyl)-1-isopropyl-4-(trifluoromethyl)-1H-imidazole**

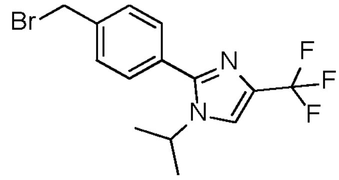

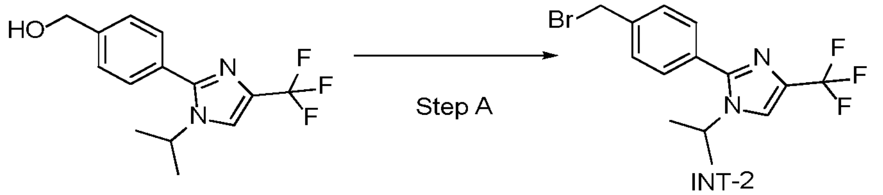


INT-2

**Step A:** At room temperature, (4-(1-isopropyl-4-(trifluoromethyl)-1H-imidazol-2-yl)phenyl)methanol (700 mg, 2.46 mmol) was dissolved in dichloromethane (12.5 mL). Then, triphenylphosphine (1.29 g, 4.93 mmol), sodium bicarbonate (414 mg, 4.93 mmol) and carbon tetrabromide (1.63 g, 4.93 mmol) were added sequentially to the above solution. Then the reaction system was continued to be stirred at room temperature for 2 hours.

After the disappearance of raw materials as monitored by LCMS, the reaction solution was quenched by adding water (20 mL), and the mixture was extracted with dichloromethane (10 mL × 3 times). Organic phases were combined and then washed with saturated saline (60 mL). Then the resultant was dried with anhydrous sodium sulfate, filtered, and concentrated under reduced pressure. The resulting residue was purified by silica gel column chromatography to obtain 820 mg of 2-(4-(bromomethyl)phenyl)-1-isopropyl-4-(trifluoromethyl)-1H-imidazole.

MS (ESI) M/Z: 347.2 [M+H]$^+$.

**Intermediate INT-3: (4-cyclopropyl-6-methoxypyrimidin-5-yl)boronic acid**

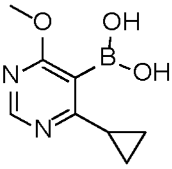

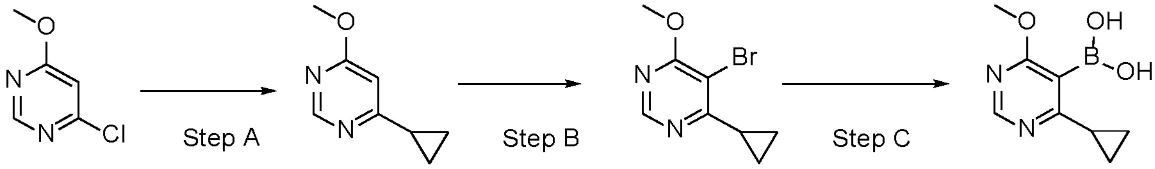


INT-3

**Step A:** At room temperature and under nitrogen protection, compound 4-chloro-6-methoxypyrimidine (25 g, 172.94 mmol), cyclopropylboronic acid (25.25 g, 294 mmol), 1,1'-bis(diphenylphosphino)ferrocene palladium dichloride (6.3 g, 8.68 mmol), potassium phosphate (73.42 g, 345.88 mmol) and silver oxide (20.04 g, 86.47 mmol) were dissolved in 1,4-dioxane (868 mL). The above solution was then heated to 90°C and stirred for 16 hours.

After the disappearance of raw materials as monitored by LCMS, the reaction solution was filtered while hot and the filtrate was concentrated under reduced pressure. The resulting residue was quenched by adding water (500 mL), and the mixture was extracted with dichloromethane (100 mL × 3 times). Organic phases were combined and then washed with saturated saline (150 mL × 2 times). Then the resultant was dried with anhydrous sodium sulfate, filtered, and concentrated under reduced pressure. The resulting residue was purified by silica gel column chromatography to obtain 18.3 g of 4-cyclopropyl-6-methoxypyrimidine.

MS (ESI) M/Z: 151.2 [M+H]$^+$.

**Step B:** At -20°C and under nitrogen protection, 4-cyclopropyl-6-methoxypyrimidine (45 g, 299.64 mmol) was dissolved in ethanol (1.5 L). Subsequently, liquid bromine (240 g, 1.5 mol) was slowly added dropwise to the above solution. Then the reaction system was continued to be stirred at room temperature for 16 hours.

After the disappearance of raw materials as monitored by LCMS, the reaction solution was concentrated through

reduced pressure distillation. The resulting crude product was pulped with ethyl acetate and filtered, and the filter cake was collected. Then the filter cake was added to water (150 mL) and stirred at 0°C, and saturated sodium bicarbonate solution was added dropwise to the solution system until pH=7. Finally, the mixture was filtered, the filter cake was rinsed with water (150 mL × 2 times), the filter cake was collected and concentrated under reduced pressure to obtain 56 g of 5-bromo-4-cyclopropyl-6-methoxypyrimidine.

MS (ESI) M/Z: 230.1 $[M+H]^+$.

$^1$H NMR (400 MHz, DMSO-$d_6$) δ 8.53 (s, 1H), 3.99 (s, 3H), 2.50 - 2.44 (m, 1H), 1.16 - 1.09 (m, 2H), 1.08 - 1.02 (m, 2H).

**Step C:** At room temperature and under nitrogen protection, 5-bromo-4-cyclopropyl-6-methoxypyrimidine (15.0 g, 65.79 mmol) and triisopropyl borate (16.08 g, 85.53 mmol) were dissolved in toluene/tetrahydrofuran (150 mL/45 mL). Subsequently, the above reaction solution was cooled to -78°C, stirred for 30 min, 2.5 M n-butyllithium solution (34.2 mL, 85.53 mmol) was slowly added dropwise, and the mixture was stirred for 30 min. Then the reaction system was warmed to -20°C and stirred for 1 hour.

After the disappearance of raw materials as monitored by LCMS, the reaction solution was quenched by adding to ice water (100 L). The mixed solution was then filtered and the filter cake was rinsed with water (20 mL × 3 times) to obtain 9.7 g of (4-cyclopropyl-6-methoxypyrimidin-5-yl)boronic acid.

MS (ESI) M/Z: 195.0 $[M+H]^+$.

**Intermediate INT-4: 2-(4-(bromomethyl)phenyl)-1-methyl-4-(trifluoromethyl)-1H-imidazole**

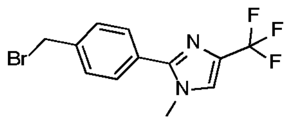

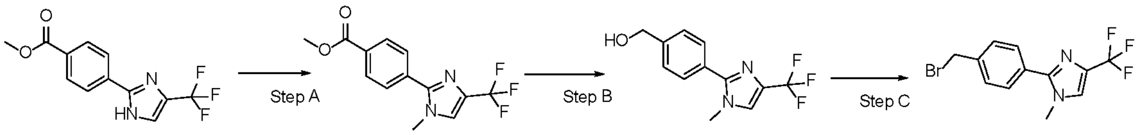


INT-4

**Step A:** At room temperature, methyl 4-(4-(trifluoromethyl)-1H-imidazol-2-yl)benzoate (5 g, 18.45 mmol) and potassium carbonate (5.1 g, 36.7 mmol) were dissolved in N,N-dimethylformamide (92 mL). The reaction system was then cooled to 0°C, and iodomethane (3.14 g, 22.14 mmol) was slowly added dropwise. Then the reaction system was continued to be stirred at room temperature for 1 hour.

After the disappearance of raw materials as monitored by LCMS, the reaction solution was quenched by adding water (200 mL), and the mixture was extracted with ethyl acetate (60 mL × 3 times). Organic phases were combined and then washed with saturated saline (100 mL × 2 times). Then the resultant was dried with anhydrous sodium sulfate, filtered, and concentrated under reduced pressure. The resulting residue was purified by silica gel column chromatography to obtain 3.2 g of methyl 4-(1-methyl-4-(trifluoromethyl)-1H-imidazol-2-yl)benzoate.

MS (ESI) M/Z: 285.0 $[M+H]^+$.

**Step B:** At room temperature, methyl 4-(1-methyl-4-(trifluoromethyl)-1H-imidazol-2-yl)benzoate (3.2 g, 11.26 mmol) was dissolved in tetrahydrofuran (28 mL). The reaction system was then cooled to 0°C, and then lithium aluminum hydride (2.14 g, 56.32 mmol) was slowly added dropwise. Then the reaction system was continued to be stirred at room temperature for 2 hours.

After the disappearance of raw materials as monitored by LCMS, the reaction solution was quenched by adding ice water (100 mL), and the mixture was extracted with ethyl acetate (60 mL × 3 times). Organic phases were combined and then washed with saturated saline (30 mL × 2 times). Then the resultant was dried with anhydrous sodium sulfate, filtered, and concentrated under reduced pressure. The resulting residue was purified by silica gel column chromatography to obtain 2.6 g of (4-(1-methyl-4-(trifluoromethyl)-1H-imidazol-2-yl)phenyl)methanol.

MS (ESI) M/Z: 257.0 $[M+H]^+$.

**Step C:** At room temperature and under nitrogen protection, (4-(1-methyl-4-(trifluoromethyl)-1H-imidazol-2-yl)phenyl)methanol (2.6 g, 10.12 mmol), sodium bicarbonate (1.7 g, 20.24 mmol), and triphenylphosphine (5.3 g, 20.24 mmol) were dissolved in dichloromethane (51 mL). The reaction system was then cooled to 0°C and then carbon tetrabromide (6.7 g, 20.24 mmol) was slowly added. Then the reaction system was warmed to room temperature and

continued to be stirred for 2 hours.

After the disappearance of raw materials as monitored by LCMS, the reaction solution was quenched by adding ice water (100 mL), and the mixture was extracted with dichloromethane (50 mL × 3 times). Organic phases were combined and then washed with saturated saline (80 mL × 2 times). Then the resultant was dried with anhydrous sodium sulfate, filtered, and concentrated under reduced pressure. The resulting residue was purified by silica gel column chromatography to obtain 2.4 g of (2-(4-(bromomethyl)phenyl)-1-methyl-4-(trifluoromethyl)-1H-imidazole.

MS (ESI) M/Z: 319.0 $[M+H]^+$.

**Intermediate INT-5: 2-(4-(bromomethyl)phenyl)-1-ethyl-4-(trifluoromethyl)-1H-imidazole**

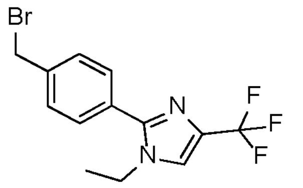

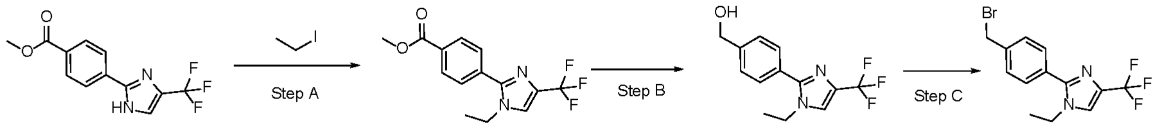


INT-5

**Step A:** At 0°C and under nitrogen protection, methyl 4-(4-(trifluoromethyl)-1H-imidazol-2-yl)benzoate (700 mg, 2.59 mmol) and sodium hydride (156 mg, 3.89 mmol) were dissolved in N,N-dimethylformamide (13 mL), and the mixture was stirred for 30 minutes. Then iodoethane (606.6 mg, 3.89 mmol) was slowly added dropwise to the above system, and the reaction system was warmed room temperature and stirred for 1 hour.

After the disappearance of raw materials as monitored by LCMS, the reaction solution was quenched by adding ice water (60 mL), and the mixture was extracted with ethyl acetate (20 mL × 3 times). Organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 300 mg of methyl 4-(1-ethyl-4-(trifluoromethyl)-1H-imidazol-2-yl)benzoate.

MS (ESI) M/Z: 299.0 $[M+H]^+$.

**Step B:** In an ice-water bath and under nitrogen protection, methyl 4-(1-ethyl-4-(trifluoromethyl)-1H-imidazol-2-yl)benzoate (300 mg, 1.00 mmol) was dissolved in dry tetrahydrofuran (5 mL). Subsequently, lithium aluminium hydride (0.8 mL, 2.00 mmol) was added to the above solution slowly, and the reaction system was warmed to room temperature and stirred for 2 hours.

After the disappearance of raw materials as monitored by LCMS, the reaction solution was quenched by adding ice water (30 mL). The mixed solution was extracted with ethyl acetate (20 mL × 3 times), and organic phases were combined and then washed with saturated saline (10 mL × 2 times). Then the resultant was dried with anhydrous sodium sulfate, filtered, and concentrated under reduced pressure. The resulting residue was purified by silica gel column chromatography to obtain 210 mg of (4-(1-ethyl-4-(trifluoromethyl)-1H-imidazol-2-yl)phenyl)methanol.

MS (ESI) M/Z: 271.0 $[M+H]^+$.

**Step C:** In an ice-water bath and under nitrogen protection, (4-(1-ethyl-4-(trifluoromethyl)-1H-imidazol-2-yl) phenyl)methanol (200 mg, 0.74 mmol), triphenylphosphine (390 mg, 1.48 mmol), sodium bicarbonate (125 mg, 1.48 mmol) and carbon tetrabromide (390 mg, 1.48 mmol) were dissolved in dichloromethane (3.7 mL). Then the reaction system was continued to be stirred at room temperature for 1 hour.

After the disappearance of raw materials as monitored by LCMS, the reaction solution was quenched by adding water (50 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 230 mg of 2-(4-(bromomethyl)phenyl)-1-ethyl-4-(trifluoromethyl)-1H-imidazole.

MS (ESI) M/Z: 333.0 $[M+H]^+$.

**Intermediate INT-6: 2-chloro-9H-pyrimido[4,5-b]indole**

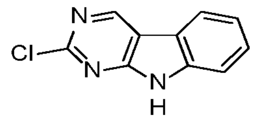

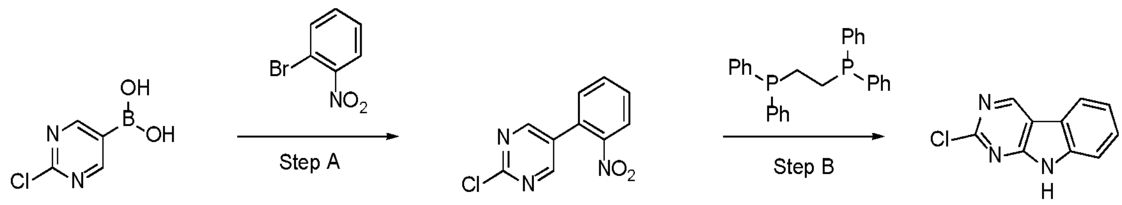


INT-6

**Step A:** At room temperature, 1-bromo-2-nitrobenzene (500 mg, 2.49 mmol), (2-chloropyrimidin-5-yl)boronic acid (590 mg, 3.74 mmol), sodium carbonate (792 mg, 7.47 mmol) and [1,1'-bis(diphenylphosphino)ferrocene]palladium dichloride dichloromethane complex (305 mg, 0.37 mmol) were dissolved in 1,4-dioxane/water (11 mL/1.4 mL). The reaction system was evacuated to remove air and purged with nitrogen for 3 times, and then stirred at 90°C for 3 h under nitrogen protection.

After the disappearance of raw materials as monitored by LCMS, the reaction solution was quenched by adding to ice water (20 mL). The mixed solution was extracted with ethyl acetate (20 mL × 3 times), and organic phases were combined and then washed with saturated saline (15 mL × 2 times). Then the resultant was dried with anhydrous sodium sulfate, filtered, and concentrated under reduced pressure to obtain a residue. The residue was purified by silica gel column chromatography to obtain 500 mg of 2-chloro-5-(2-nitrophenyl)pyrimidine.

MS (ESI) M/Z: 236.0 $[M+H]^+$.

**Step B:** At room temperature, 2-chloro-5-(2-nitrophenyl)pyrimidine (500 mg, 2.13 mmol) and 1,2-bis(diphenylphosphino)ethane (1.06 g, 2.66 mmol) were dissolved in 1,2-dichlorobenzene (7.1 mL). Then the reaction system was stirred at 160°C for 1 hour.

After the disappearance of raw materials as monitored by LCMS, the reaction solution was quenched by adding ice water (10 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure to obtain a residue. The residue was purified by silica gel column chromatography to obtain 200 mg of 2-chloro-9H-pyrimido[4,5-b]indole.

MS (ESI) M/Z: 204.0 $[M+H]^+$.

**Intermediate INT-7: 2-chloro-5,6,7,8-tetrahydrocyclopenta[4,5]pyrrolo[2,3-d]pyrimidine**

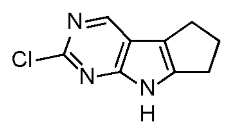

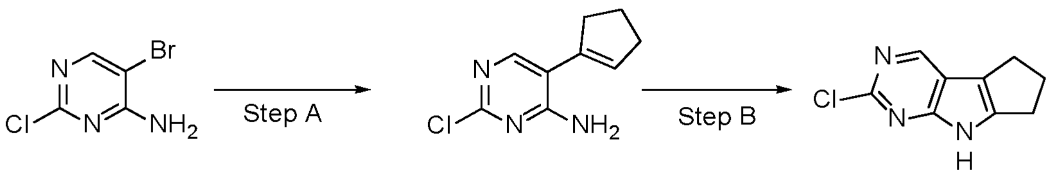


INT-7

**Step A:** At room temperature, 4-amino-5-bromo-2-chloropyrimidine (1.0 g, 4.83 mmol), 1-cyclopentenylboronic

acid pinacol ester (1.4 g, 7.25 mmol), potassium phosphate (2.6 g, 12.08 mmol) and [1,1'-bis(diphenylphosphino) ferrocene]palladium dichloride dichloromethane complex (389 mg, 0.48 mmol) were dissolved in 1,4-dioxane/water (15 mL/3 mL). The reaction system was evacuated to remove air and purged with nitrogen for 3 times, and then stirred at 90°C for 3 hours under nitrogen protection.

After the disappearance of raw materials as monitored by TLC, the reaction solution was quenched by adding to ice water (20 mL). The mixed solution was extracted with ethyl acetate (20 mL × 3 times), and organic phases were combined and then washed with saturated saline (15 mL × 2 times). Then the resultant was dried with anhydrous sodium sulfate, filtered, and concentrated under reduced pressure to obtain a residue. The resulting residue was purified by silica gel column chromatography to obtain 600 mg of 2-chloro-5-(cyclopent-1-en-1-yl)pyrimidin-4-amine.

MS (ESI) M/Z: 196.1 $[M+H]^+$.

$^1H$ NMR (400 MHz, DMSO-*d6*) δ 7.91 (s, 1H), 7.29 (br, 2H), 6.09 (t, *J* = 2.2 Hz, 1H), 2.65 - 2.57 (m, 2H), 2.49 - 2.43 (m, 2H), 1.91 (p, *J* = 7.5 Hz, 2H).

**Step B:** At 0°C, 2-chloro-5-(cyclopent-1-en-1-yl)pyrimidin-4-amine (100 mg, 0.51 mmol) was dissolved in tetrahydrofuran (2 mL) and water (1 mL). N-bromosuccinimide (100 mg, 0.56 mmol) was then added, and the mixture was stirred for 1 hour. Subsequently, 2M aqueous sodium hydroxide solution (0.2 mL) was added, and the mixture was stirred for 30 minutes.

After the disappearance of raw materials as monitored by TLC, the reaction solution was quenched by adding ice water (10 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure to obtain a residue. To the residue was added tetrahydrofuran (3 mL) followed by boron trifluoride ether (217 mg, 1.53 mmol), and the mixture was stirred at 60°C for 3 hours.

After the disappearance of raw materials as monitored by TLC, the reaction solution was quenched by adding saturated sodium bicarbonate aqueous solution (50 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure to obtain a residue. The resulting residue was purified by silica gel column chromatography to obtain 60 mg of 2-chloro-5,6,7,8-tetrahydrocyclopenta[4,5]pyrrolo[2,3-d]pyrimidine.

MS (ESI) M/Z: 194.1 $[M+H]^+$.

$^1H$ NMR (400 MHz, DMSO-*d6*) δ 12.19 (s, 1H), 8.68 (s, 1H), 2.87 (t, *J* = 7.1 Hz, 2H), 2.78 (t, *J* = 7.1 Hz, 2H), 2.48 - 2.39 (m, 2H).

**Intermediate INT-8: 2-(4-(bromomethyl)-2-methoxyphenyl)-1-isopropyl-4-(trifluoromethyl)- 1H-imidazole**

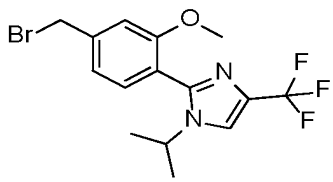

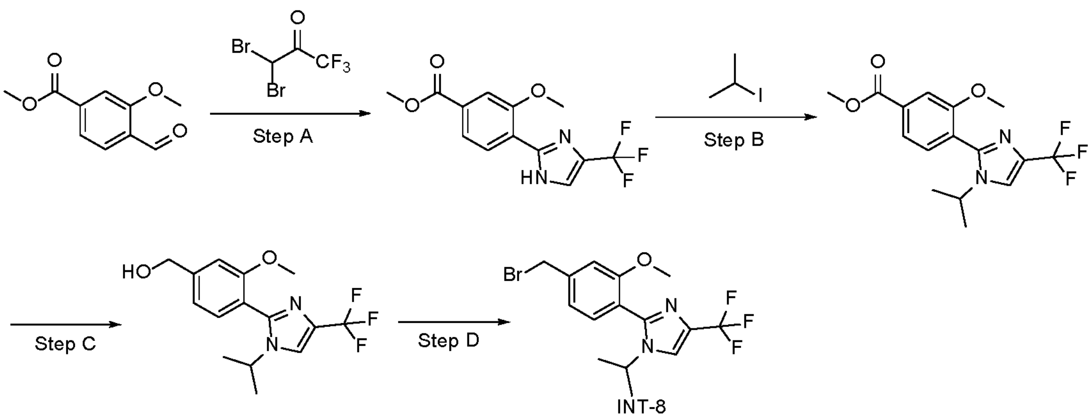


INT-8

**Step A:** At room temperature, 3,3-dibromo-1,1,1-trifluoropropan-2-one (40.2 g, 0.15 mol) and sodium acetate (12.25 g, 0.15 mol) were dissolved in water (36 mL). Subsequently, the above solution was warmed up to 90°C and stirred

for 0.5 hour. Then the reaction system was cooled to 0°C, and methyl 4-formyl-3-methoxybenzoate (10 g, 51.54 mmol) and a mixture of 28 wt% aqueous ammonia (45 mL) and methanol (135 mL) were slowly added dropwise to the above reaction solution. Finally, the reaction system was continued to be stirred at room temperature for 16 hours.

After the disappearance of raw materials as monitored by LCMS, the reaction solution was filtered and the filter cake was rinsed with ethyl acetate/petroleum ether (v/v, 3/1). The filtrate was collected and concentrated under reduced pressure. The resulting residue was purified by silica gel column chromatography to obtain 10.85 g of methyl 3-methoxy-4-(4-(trifluoromethyl)-1H-imidazol-2-yl)benzoate.

MS (ESI) M/Z: 301.0 $[M+H]^+$.

**Step B:** At room temperature, methyl 3-methoxy-4-(4-(trifluoromethyl)-1H-imidazol-2-yl)benzoate (6 g, 0.02 mol) and cesium carbonate (19.7 g, 0.06 mol) were dissolved in acetonitrile (65 mL), and the solution was stirred for 2 hours. Subsequently, 2-iodopropane (5.1 g, 0.03 mol) was added to the above solution, and the reaction system was heated to 50°C and stirred for 24 hours. The reaction system was then cooled to 0°C and further 2-iodopropane (5.1 g, 0.03 mol) was added. The reaction system was heated to 50°C and stirred for 4 hours.

After the disappearance of raw materials as monitored by LCMS, the reaction solution was quenched by adding to ice water (300 mL), and the mixture was extracted with ethyl acetate (100 mL × 3 times). Organic phases were combined and then washed with saturated saline (50 mL). Then the resultant was dried with anhydrous sodium sulfate, filtered, and concentrated under reduced pressure. The resulting residue was purified by silica gel column chromatography to obtain 6.9 g of methyl 4-(1-isopropyl-4-(trifluoromethyl)-1H-imidazol-2-yl)-3-methoxybenzoate.

MS (ESI) M/Z: 343.0 $[M+H]^+$.

**Step C:** At 0°C and under nitrogen protection, methyl 4-(1-isopropyl-4-(trifluoromethyl)-1H-imidazol-2-yl)-3-methoxybenzoate (2.9 g, 8.48 mmol) was dissolved in dry tetrahydrofuran (30 mL). Subsequently, lithium aluminium hydride (6.8 mL, 17 mmol) was slowly added to the above solution dropwise. Then the reaction system was continued to be stirred at room temperature for 1 hour.

After the disappearance of raw materials as monitored by LCMS, the reaction solution was quenched by slowly adding to ice water (100 mL) dropwise. The resultant was filtered and the filter cake was rinsed with ethyl acetate (50 mL × 3 times). The filtrate was extracted with ethyl acetate (50 mL × 3 times), and organic phases were combined and then washed with saturated saline (150 mL). Then the resultant was dried with anhydrous sodium sulfate, filtered, and concentrated under reduced pressure. The resulting residue was purified by silica gel column chromatography to obtain 1.95 g of (4-(1-isopropyl-4-(trifluoromethyl)-1H-imidazol-2-yl)-3-methoxyphenyl)methanol.

MS (ESI) M/Z: 315.4 $[M+H]^+$.

**Step D:** At 0°C, (4-(1-isopropyl-4-(trifluoromethyl)-1H-imidazol-2-yl)-3-methoxyphenyl)methanol (500 mg, 1.59 mmol), sodium bicarbonate (269 mg, 3.2 mmol), and triphenylphosphine (840 mg, 3.2 mmol) were dissolved in dry dichloromethane (8 mL). Subsequently, carbon tetrabromide (1.06 g, 3.2 mmol) was slowly added to the above reaction solution. Then the reaction system was continued to be stirred at room temperature for 1 hour.

After the disappearance of raw materials as monitored by LCMS, the reaction solution was quenched by adding ice water (50 mL), and the mixture was extracted with ethyl acetate (20 mL × 3 times). Organic phases were combined and then washed with saturated saline (30 mL × 2 times). Then the resultant was dried with anhydrous sodium sulfate, filtered, and concentrated under reduced pressure. The resulting residue was purified by silica gel column chromatography to obtain 350 mg of 2-(4-(bromomethyl)-2-methoxyphenyl)-1-isopropyl-4-(trifluoromethyl)-1H-imidazole.

MS (ESI) M/Z: 377.2 $[M+H]^+$.

**Intermediate INT-9: 2-(4-(bromomethyl)-2-fluoro-6-methoxyphenyl)-1-isopropyl-4-(trifluoromethyl)-1H-imidazol e**

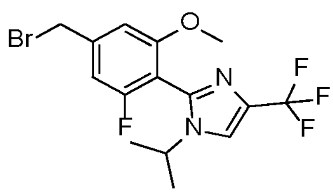

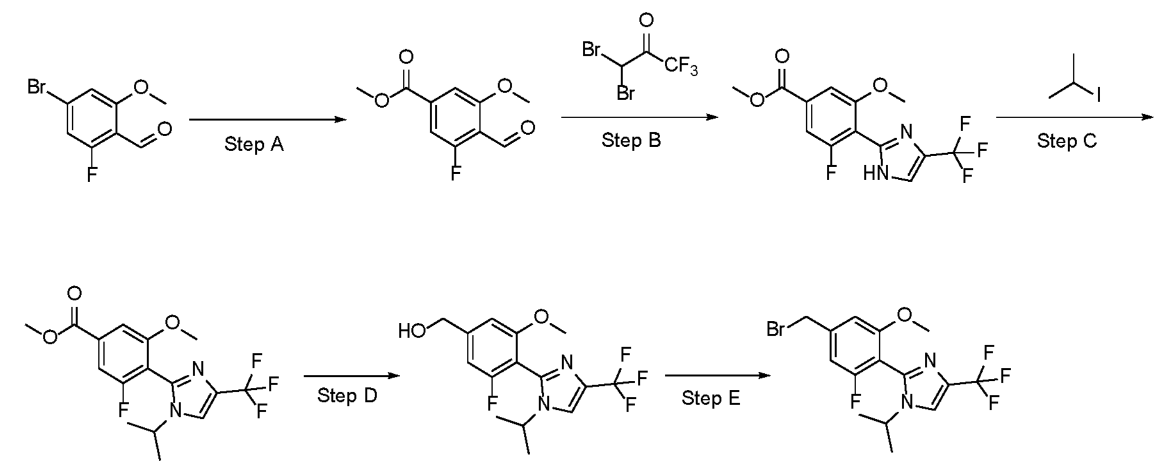


**Step A:** At room temperature, 4-bromo-2-fluoro-6-methoxybenzaldehyde (4 g, 17 mmol), triethylamine (12.38 g, 0.12 mol) and 1,1-bis(diphenylphosphino)ferrocenepalladium dichloride (1.34 g, 18 mmol) were dissolved in methanol (120 mL). The reaction system was evacuated to remove air and purged with carbon monoxide for 3 times, and then the reaction solution was heated to 90°C and stirred for 24 hours.

After the disappearance of raw materials as monitored by LCMS, the reaction solution was filtered and the filtrate was concentrated under reduced pressure. The resulting residue was purified by silica gel column chromatography to obtain 1.98 g of methyl 3-fluoro-4-formyl-5-methoxybenzoate.

MS (ESI) M/Z: 213.0 $[M+H]^+$.

**Step B:** At room temperature, 3,3-dibromo-1,1,1-trifluoropropan-2-one (6.72 g, 25 mmol) and sodium acetate (2.06 g, 25 mmol) were dissolved in water (7 mL). Subsequently, the above solution was warmed up to 90°C and stirred for 0.5 hour. Then the reaction system was cooled to 0°C, and methyl 3-fluoro-4-formyl-5-methoxybenzoate (1.98 g, 9 mmol) and a mixture of 28 wt% aqueous ammonia (17 mL) and methanol (45 mL) were slowly added dropwise to the above reaction solution.

After the disappearance of raw materials as monitored by LCMS, the reaction solution was filtered and the filter cake was rinsed with ethyl acetate/petroleum ether (v/v, 3/1). The filtrate was collected and concentrated under reduced pressure. The resulting residue was purified by silica gel column chromatography to obtain 2.06 g of methyl 3-fluoro-5-methoxy-4-(4-(trifluoromethyl)-1H-imidazol-2-yl)benzoate.

MS (ESI) M/Z: 319.0 $[M+H]^+$.

**Step C:** At room temperature, methyl 3-fluoro-5-methoxy-4-(4-(trifluoromethyl)-1H-imidazol-2-yl)benzoate (2.06 g, 6.5 mmol) and cesium carbonate (6.31 g, 19 mmol) were dissolved in acetonitrile (25 mL) and stirred for 2 hours. Subsequently, 2-iodopropane (1.65 g, 9.7 mmol) was added to the above solution, and the reaction system was heated to 50°C and stirred for 24 hours. The reaction system was then cooled to 0°C and further 2-iodopropane (1 g, 5.9 mmol) was added. The reaction system was heated to 50°C and stirred for 4 hours.

After the disappearance of raw materials as monitored by LCMS, the reaction solution was quenched by adding to ice water (50 mL), and the mixture was extracted with ethyl acetate (20 mL × 3 times). Organic phases were combined and then washed with saturated saline (50 mL). Then the resultant was dried with anhydrous sodium sulfate, filtered, and concentrated under reduced pressure. The resulting residue was purified by silica gel column chromatography to obtain 1.84 g of methyl 3-fluoro-4-(1-isopropyl-4-(trifluoromethyl)-1H-imidazol-2-yl)-5-methoxybenzoate.

MS (ESI) M/Z: 361.0 $[M+H]^+$.

**Step D:** At 0°C and under nitrogen protection, methyl 3-fluoro-4-(1-isopropyl-4-(trifluoromethyl)-1H-imidazol-2-yl)-5-methoxybenzoate (1.84 g, 5.1 mmol) was dissolved in dry tetrahydrofuran (25 mL). Subsequently, lithium aluminum hydride solution (4.2 mL, 0.01 mol) was slowly added to the above solution dropwise. Then the reaction system was continued to be stirred at room temperature for 1 hour.

After the disappearance of raw materials as monitored by LCMS, the reaction solution was quenched by slowly adding to ice water (100 mL) dropwise. The resultant was filtered and the filter cake was rinsed with ethyl acetate (50 mL × 3 times). The filtrate was extracted with ethyl acetate (50 mL × 3 times), and organic phases were combined and then washed with saturated saline (150 mL). Then the resultant was dried with anhydrous sodium sulfate, filtered, and concentrated under reduced pressure. The resulting residue was purified by silica gel column chromatography to obtain 1.38 g of (3-fluoro-4-(1-isopropyl-4-(trifluoromethyl)-1H-imidazol-2-yl)-5-methoxyphenyl)methanol.

MS (ESI) M/Z: 333.2 $[M+H]^+$.

Step E: At 0°C and under nitrogen protection, (3-fluoro-4-(1-isopropyl-4-(trifluoromethyl)-1H-imidazol-2-yl)-5-methoxyphenyl)methanol (500 mg, 1.5 mmol), sodium bicarbonate (269 mg, 3.2 mmol), and triphenylphosphine (840 mg,

3.2 mmol) were dissolved in dry dichloromethane (8 mL). Subsequently, liquid bromine (1.06 g, 3.2 mmol) was slowly added to the above solution. Then the reaction system was continued to be stirred at room temperature for 1 hour.

After the disappearance of raw materials as monitored by LCMS, the reaction solution was quenched by adding water (50 mL), and the mixture was extracted with ethyl acetate (20 mL × 3 times). Organic phases were combined and then washed with saturated saline (30 mL × 2 times). Then the resultant was dried with anhydrous sodium sulfate, filtered, and concentrated under reduced pressure. The resulting residue was purified by silica gel column chromatography to obtain 350 mg of 2-(4-(bromomethyl)-2-fluoro-6-methoxyphenyl)-1-isopropyl-4-(trifluoromethyl)-1H-imidazole.

MS (ESI) M/Z: 395.2 [M+H]$^+$.

**Intermediate INT-10: 4-chloro-1-isopropyl-5-(4,4,5,5-tetramethyl-1,3,2-dioxaborolan-2-yl)-1H-pyrazole**

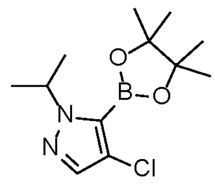

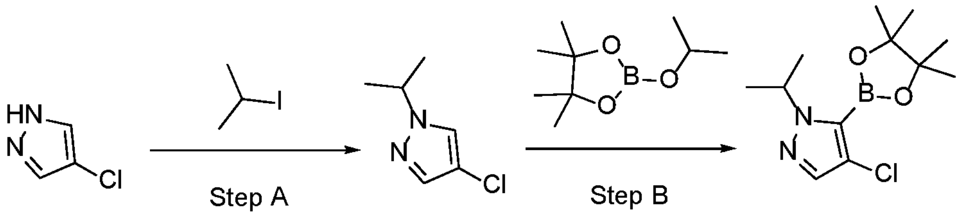


**Step A:** At room temperature, 4-chloro-1H-pyrazole (5 g, 0.049 mol), 2-iodopropane (20 g, 0.118 mol) and cesium carbonate (33 g, 0.1 mol) were dissolved in acetonitrile (40 mL). Then the reaction system was stirred at 80°C for 2 hours.

After the disappearance of raw materials as monitored by LCMS, the reaction solution was quenched by adding ice water (100 mL), and the mixture was extracted with ethyl acetate (50 mL × 3 times). Organic phases were combined and then washed with saturated saline (30 mL × 2 times). Then the resultant was dried with anhydrous sodium sulfate, filtered, and concentrated under reduced pressure. The resulting residue was purified by silica gel column chromatography to obtain 3.62 g of 4-chloro-1-isopropyl-1H-pyrazole.

MS (ESI) M/Z: 145.2 [M+H]$^+$.

**Step B:** At 0°C and under nitrogen protection, 4-chloro-1-isopropyl-1H-pyrazole (2 g, 0.014 mol) was dissolved in tetrahydrofuran (14 mL). Subsequently, n-butyllithium (11.2 mL, 0.017 mol) was added to the above solution dropwise, and the reaction system was warmed to room temperature and stirred for 1 hour. The reaction solution was then cooled to -78°C and 2-isopropoxy-4,4,5,5-tetramethyl-1,3,2-dioxaborolane (3.45 g, 0.018 mol) was slowly added dropwise, and the mixture was heated to room temperature and stirred for 2 hours.

After the disappearance of raw materials as monitored by LCMS, the reaction solution was quenched by adding saturated ammonium chloride aqueous solution (40 mL). The mixed solution was extracted with ethyl acetate (30 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 2.4 g of 4-chloro-1-isopropyl-5-(4,4,5,5-tetramethyl-1,3,2-dioxaborolan-2-yl)-1H-pyrazole.

MS (ESI) M/Z: 271.0 [M+H]$^+$.

**Intermediate INT-11: 2-(4-(1-bromopropyl)phenyl)-1-isopropyl-4-(trifluoromethyl)-1H-imidazole**

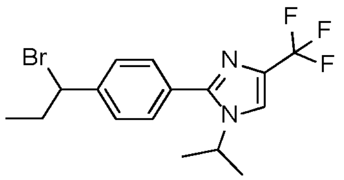

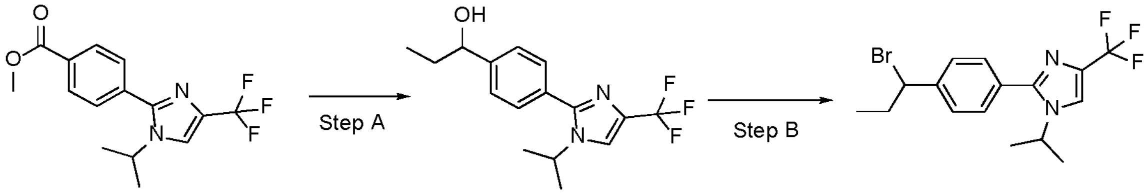


**Step A:** At 0°C and under nitrogen protection, methyl 4-(1-isopropyl-4-(trifluoromethyl)-1H-imidazol-2-yl) benzoate (300 mg, 0.96 mmol) was dissolved in dry tetrahydrofuran (5 mL). Subsequently, tetraisopropyl titanate (327 mg, 1.15 mmol) and ethylmagnesium bromide (0.46 mL, 1.15 mmol) were sequentially added to the above solution, and then the reaction system was continued to be stirred for 16 hours at room temperature.

After the disappearance of raw materials as monitored by LCMS, the reaction solution was quenched by adding to ice water (30 mL), and the mixture was filtrated, and then filtrate was extracted with ethyl acetate (20 mL × 3 times). Organic phases were combined and then washed with saturated saline (30 mL × 2 times). Then the resultant was dried with anhydrous sodium sulfate, filtered, and concentrated under reduced pressure. The resulting residue was purified by silica gel column chromatography to obtain 220 mg of 1-(4-(1-isopropyl-4-(trifluoromethyl)-1H-imidazol-2-yl)phenyl) propan-1-ol.

MS (ESI) M/Z: 313.2 $[M+H]^+$.

**Step B:** At 0°C and under nitrogen protection, 1-(4-(1-isopropyl-4-(trifluoromethyl)-1H-imidazol-2-yl)phenyl) propan-1-ol (220 mg, 0.71 mmol), triphenylphosphine (372 mg, 1.42 mmol), and sodium bicarbonate (119 mg, 1.42 mmol) were dissolved in dichloromethane (4 mL). Subsequently, carbon tetrabromide (471 mg, 1.42 mmol) was slowly added to the above reaction solution. Then the reaction system was continued to be stirred at room temperature for 30 minutes.

After the disappearance of raw materials as monitored by LCMS, the reaction solution was quenched by adding ice water (50 mL). The mixed solution was extracted with dichloromethane (20 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 80 mg of 2-(4-(1-bromopropyl)phenyl)-1-isopropyl-4-(trifluoromethyl)-1H-imidazole.

MS (ESI) M/Z: 375.0 $[M+H]^+$.

**Intermediate INT-12: 8-(bromomethyl)-2-(trifluoromethyl)imidazo[2,1-a]isoquinoline**

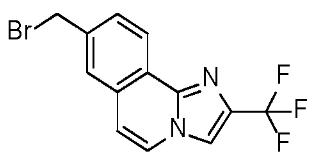

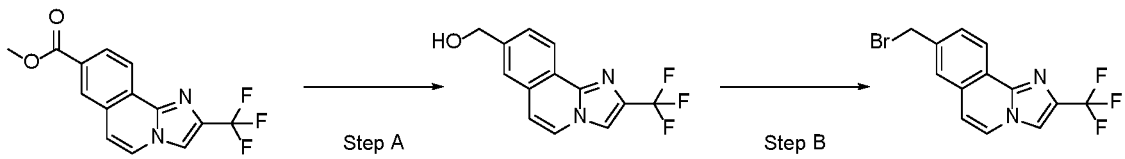


**Step A:** At room temperature, methyl 2-(trifluoromethyl)imidazo[2,1-a]isoquinoline-8-formate (52 mg, 0.18 mmol) was dissolved in tetrahydrofuran (0.9 mL). Subsequently, lithium aluminum hydride solution (0.14 mL, 0.35 mmol) was slowly added to the above solution at 0°C. Then the reaction system was continued to be stirred at room temperature for 1 hour.

After the disappearance of raw materials as monitored by LCMS, the reaction solution was quenched by adding

slowly to ice water (20 mL). The resultant was filtrated, and the filtrate was extracted with ethyl acetate (10 mL × 3 times), and organic phases were combined. Then the resultant was dried with anhydrous sodium sulfate, filtered, and concentrated under reduced pressure. The resulting residue was purified by silica gel column chromatography to obtain 32 mg of (2-(trifluoromethyl)imidazo[2,1-a]isoquinolin-8-yl)methanol.

MS (ESI) M/Z: 267.2 $[M+H]^+$.

**Step B:** At room temperature, methyl (2-(trifluoromethyl)imidazo[2,1-a]isoquinolin-8-yl)methanol (53 mg, 0.20 mmol) was dissolved in dichloromethane (1 mL). Then, triphenylphosphine (157.4 mg, 0.60 mmol), sodium bicarbonate (33.6 mg, 0.40 mmol) and carbon tetrabromide (199 mg, 0.60 mmol) were added sequentially to the above solution at 0°C. Then the reaction system was continued to be stirred at room temperature for 1.5 hours.

After the disappearance of raw materials as monitored by LCMS, the reaction solution was quenched by adding water (20 mL). The mixed solution was extracted with ethyl acetate (10 mL × 3 times), and organic phases were combined and then washed with saturated saline (30 mL × 2 times). Then the resultant was dried with anhydrous sodium sulfate, filtered, and concentrated under reduced pressure. The resulting residue was purified by silica gel column chromatography to obtain 37.8 mg of 8-(bromomethyl)-2-(trifluoromethyl)imidazo[2,1-a]isoquinoline.

MS (ESI) M/Z: 329.0 $[M+H]^+$.

**Intermediate INT-13: (2-(trifluoromethyl)-6,7-dihydro-5H-imidazo[1,2-a]pyrrolo[2,1-c][1,4]diaza-9-yl)methanol**

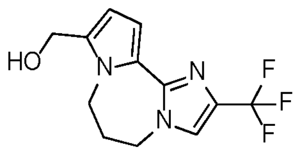

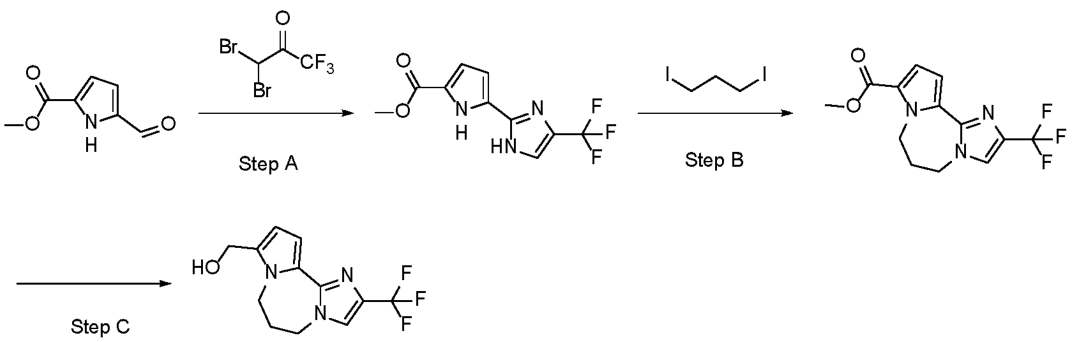


**Step A:** At room temperature, 3,3-dibromo-1,1,1-trifluoropropan-2-one (17.5 g, 65.35 mmol) and sodium acetate (5.35 g, 65.35 mmol) were dissolved in water (19.8 mL), and the solution was stirred at 90°C for 1 hour. The above reaction solution was then cooled down to 0°C, and methyl 5-formyl-1H-pyrrole-2-carboxylate (10 g, 65.35 mmol) in a mixed solution of aqueous ammonia/methanol (59.5 mL/178 mL) was added. Then the reaction system was stirred for 18 hours at room temperature.

After the disappearance of raw materials as monitored by LCMS, the reaction solution was concentrated under reduced pressure, and the resulting concentrated solution was added with ice water (200 mL). The mixed solution was extracted with ethyl acetate (50 mL × 3 times), and organic phases were combined and then washed with saturated saline (90 mL × 2 times). Then the resultant was dried with anhydrous sodium sulfate, filtered, and concentrated under reduced pressure. The resulting residue was purified by silica gel column chromatography to obtain 5.9 g of methyl 5-(4-(trifluoromethyl)-1H-imidazol-2-yl)-1H-pyrrole-2-carboxylate.

MS (ESI) M/Z: 260.0 $[M+H]^+$.

**Step B:** At room temperature, methyl 5-(4-(trifluoromethyl)-1H-imidazol-2-yl)-1H-pyrrole-2-carboxylate (2.6 g, 10.25 mmol) and cesium carbonate (10 g, 30.76 mmol) were dissolved in acetonitrile (50 mL), and the solution was stirred for 2 hours. Subsequently, 1,3-diiodopropane (4.5 g, 15.3 mmol) was added to the above solution, and the reaction system was heated to 50°C and stirred for 48 hours.

After the disappearance of raw materials as monitored by LCMS, the reaction solution was concentrated under reduced pressure, and the resulting residue was quenched by adding ice water (100 mL), and the mixture was extracted with ethyl acetate (30 mL × 3 times). Organic phases were combined and then washed with saturated saline (20 mL × 2 times). Then the resultant was dried with anhydrous sodium sulfate, filtered, and concentrated under reduced pressure.

1.4 g of methyl 2-(trifluoromethyl)-6,7-dihydro-5H-imidazo[1,2-a]pyrrolo[2,1-c][1,4]diaza-9-yl)formate was obtained.

MS (ESI) M/Z: 300.1 $[M+H]^+$.

**Step C:** At room temperature, methyl 2-(trifluoromethyl)-6,7-dihydro-5H-imidazo[1,2-a]pyrrolo[2,1-c][1,4]diaza-9-yl)formate (1.4 g, 4.68 mmol) was dissolved in tetrahydrofuran (23 mL). Subsequently, lithium aluminum hydride solution (3.74 mL, 9.36 mmol) was slowly added to the above solution at 0°C. Then the reaction system was continued to be stirred at room temperature for 0.5 hour.

After the disappearance of raw materials as monitored by LCMS, the reaction solution was quenched by adding to ice water (40 mL) slowly, and the mixture was extracted with ethyl acetate (30 mL × 3 times). Organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 680 mg of (2-(trifluoromethyl)-6,7-dihydro-5H-imidazo[1,2-a]pyrrolo[2,1-c][1,4]diaza-9-yl) methanol.

MS (ESI) M/Z: 272.1 $[M+H]^+$.

**Intermediate INT-14: (2-(trifluoromethyl)-5,6-dihydroimidazo[1,2-a]pyrrolo[2,1-c]pyrazin-8-yl)methanol**

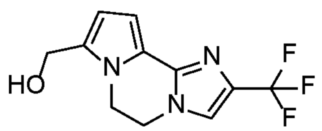

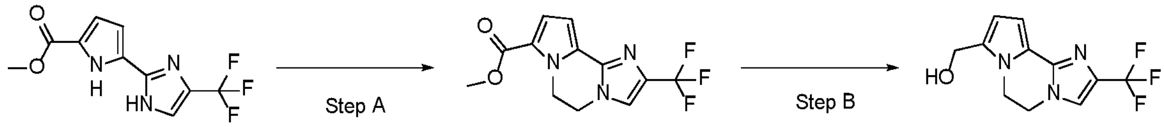


**Step A:** At room temperature, methyl 5-(4-(trifluoromethyl)-1H-imidazol-2-yl)-1H-pyrrole-2-carboxylate (2 g, 7.72 mmol) and cesium carbonate (7.54 g, 23.15 mmol) were dissolved in acetonitrile (50 mL) and stirred for 2 hours. Subsequently, 1, 2-dibromoethane (1.45 g, 7.72 mmol) was added to the above solution. Then the reaction system was stirred at 50°C for 48 hours.

After the disappearance of raw materials as monitored by LCMS, the reaction solution was concentrated under reduced pressure, and the resulting residue was quenched by adding ice water (100 mL), and the mixture was extracted with ethyl acetate (30 mL × 3 times). Organic phases were combined and then washed with saturated saline (20 mL × 2 times). Then the resultant was dried with anhydrous sodium sulfate, filtered, and concentrated under reduced pressure. 860 mg of methyl 2-(trifluoromethyl)-5,6-dihydroimidazo[1,2-a]pyrrolo[2,1-c]pyrazin-8-formate was obtained.

MS (ESI) M/Z: 286.2 $[M+H]^+$.

**Step B:** At room temperature, methyl 2-(trifluoromethyl)-5,6-dihydroimidazo[1,2-a]pyrrolo[2,1-c]pyrazin-8-formate (850 mg, 2.98 mmol) was dissolved in dry tetrahydrofuran (30 mL). Subsequently, lithium aluminum hydride solution (2.24 mL, 4.47 mmol) was slowly added to the above solution at 0°C. Then the reaction system was continued to be stirred at room temperature for 0.5 hour.

After the disappearance of raw materials as monitored by LCMS, the reaction solution was quenched by adding to ice water (40 mL) slowly, and the mixture was extracted with ethyl acetate (30 mL × 3 times). Organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 590 mg of (2-(trifluoromethyl)-5,6-dihydroimidazo[1,2-a]pyrrolo[2,1-c]pyrazin-8-yl)methanol.

MS (ESI) M/Z: 258.2 $[M+H]^+$.

$^1H$ NMR (400 MHz, DMSO-*d6*):δ7.82 (d, *J* = 1.2 Hz, 1H), 6.50 (d, *J* = 3.6 Hz, 1H), 6.11 (d, *J* = 3.6 Hz, 1H), 5.10 (t, *J* = 5.2 Hz, 1H), 4.48 (d, *J* = 5.2 Hz, 2H), 4.39 - 4.33 (m, 2H), 4.32 - 4.276 (m, 2H).

**Intermediate INT-15: 7-(bromomethyl)-2-(trifluoromethyl)-5H-imidazo[2,1-a]isoindole**

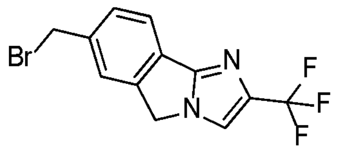

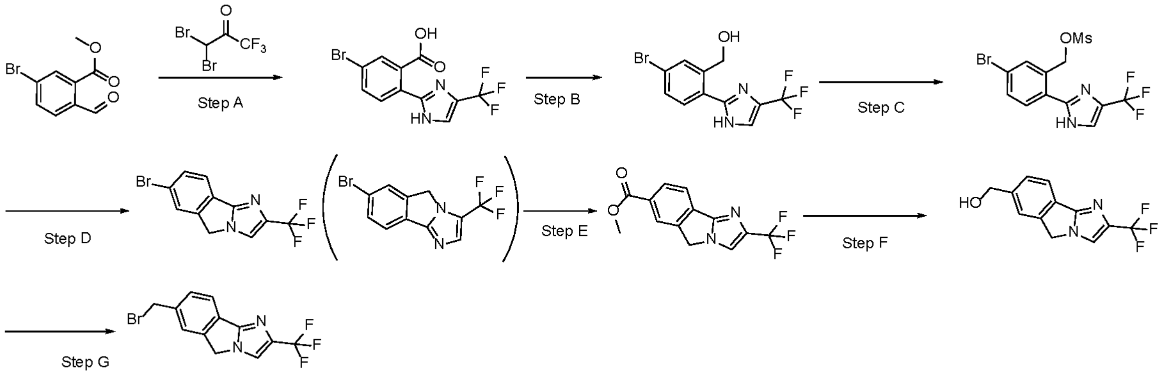


**Step A:** At room temperature, 3,3-dibromo-1,1,1-trifluoropropan-2-one (55.5 g, 205.72 mmol) was dissolved in water (65 mL). Subsequently, sodium acetate (16.9 g, 205.72 mmol) was added to the above solution, and the reaction system was heated to 90°C and stirred for 1 hour. Then the above solution was cooled down to 0°C, and methyl 5-bromo-2-formylbenzoate (25 g, 102.86 mmol) in a mixed solution of methanol (685 mL) and aqueous ammonia (150 mL) was added slowly and dropwise. Then the reaction system was stirred at 100°C for 2 hours.

After the disappearance of raw materials as monitored by LCMS, the reaction solution was concentrated under reduced pressure, and the resulting concentrated solution was quenched by adding water (100 mL). The mixed solution was extracted with ethyl acetate (200 mL × 3 times), and organic phases were combined and then dried with anhydrous sodium sulfate, filtered, and concentrated under reduced pressure. The resulting residue was pulped to obtain 41 g of 5-bromo-2-(4-(trifluoromethyl)-1H-imidazol-2-yl)benzoic acid.

MS (ESI) M/Z: 334.9 $[M+H]^+$.

**Step B:** At 0°C, 5-bromo-2-(4-(trifluoromethyl)-1H-imidazol-2-yl)benzoic acid (36 g, 107.78 mmol) was dissolved in dry tetrahydrofuran (500 mL). Subsequently, borane in tetrahydrofuran (216 mL, 215.27 mmol) was slowly added to the above solution dropwise. Then the reaction system was continued to be stirred at room temperature for 2 hours.

After the disappearance of raw materials as monitored by LCMS, the reaction solution was quenched by adding water (200 mL). The mixed solution was extracted with ethyl acetate (300 mL × 3 times), and organic phases were combined and then washed with saturated saline (300 mL × 2 times). Then the resultant was dried with anhydrous sodium sulfate, filtered, and concentrated under reduced pressure. The resulting residue was purified by silica gel column chromatography to obtain 12.3 g of (5-bromo-2-(4-(trifluoromethyl)-1H-imidazol-2-yl)phenyl)methanol.

MS (ESI) M/Z: 321.0 $[M+H]^+$.

**Step C:** At 0°C, (5-bromo-2-(4-(trifluoromethyl)-1H-imidazol-2-yl)phenyl)methanol (12.3 g, 38.44 mmol) was dissolved in dichloromethane (192 mL). Subsequently, triethylamine (10.7 mL, 76.88 mmol) and methanesulfonyl chloride (6.6 g, 57.66 mmol) were added sequentially to the above solution. Then the reaction system was continued to be stirred at room temperature for 1 hour.

After the disappearance of raw materials as monitored by LCMS, the reaction solution was quenched by adding water (200 mL). The mixed solution was extracted with ethyl acetate (200 mL × 3 times), and organic phases were combined and then washed with saturated saline (200 mL × 2 times). The resultant was dried with anhydrous sodium sulfate, filtered, and concentrated under reduced pressure to obtain 14.5 g of 5-bromo-2-(4-(trifluoromethyl)-1H-imidazol-2-yl)benzylmesylate.

MS (ESI) M/Z: 399.0 $[M+H]^+$.

**Step D:** At room temperature, 5-bromo-2-(4-(trifluoromethyl)-1H-imidazol-2-yl)benzylmesylate (14.5 g, 42.90 mmol) was dissolved in N,N-dimethylformamide (214 mL). Subsequently, cesium carbonate (16.8 g, 51.48 mmol) was added to the above solution. Then the reaction system was stirred at 50°C for 1 hour.

After the disappearance of raw materials as monitored by LCMS, the reaction solution was quenched by adding water (600 mL). The mixed solution was extracted with ethyl acetate (200 mL × 3 times), and organic phases were combined and then washed with saturated saline (200 mL × 2 times). Then the resultant was dried with anhydrous sodium sulfate, filtered, and concentrated under reduced pressure. The resulting residue was purified by silica gel column chromatography to obtain 1.83 g of 7-bromo-2-(trifluoromethyl)-5H-imidazo[2,1-a]isoindole and 500 mg of an isomer 7-bromo-3-(trifluoromethyl)-5H-imidazo[2,1-a]isoindole.

MS (ESI) M/Z: 303.0 $[M+H]^+$.

$^1H$ NMR (400 MHz, DMSO-*d6*): δ8.15 (d, *J* = 0.8 Hz, 1H), 7.92 (d, *J* = 0.8 Hz, 1H), 7.75 (d, *J* = 8.0 Hz, 1H), 7.70 (dd, *J* = 8.4, 1.8 Hz, 1H), 5.17 (s, 2H).

**Step E:** At room temperature, 7-bromo-2-(trifluoromethyl)-5H-imidazo[2,1-a]isoindole (930 mg, 3.08 mmol) was dissolved in methanol (35 mL). Subsequently, triethylamine (4.2 mL, 30.8 mmol) and [1,1'-bis(diphenylphosphino) ferrocene]palladium dichloride dichloromethane complex (251 mg, 0.31 mmol) were added sequentially to the above solution. The reaction system was then stirred at a pressure of 40 kg of carbon monoxide gas and a temperature of 85°C for 24 hours.

After the disappearance of raw materials as monitored by LCMS, the reaction solution was concentrated under reduced pressure. The resulting residue was purified by silica gel column chromatography to obtain 400 mg of methyl 2-(trifluoromethyl)-5H-imidazo[2,1-a]isoindol-7-formate.

MS (ESI) M/Z: 283.1 $[M+H]^+$.

**Step F:** At 0°C, methyl 2-(trifluoromethyl)-5H-imidazo[2,1-a]isoindol-7-formate (400 mg, 1.42 mmol) was dissolved in dry tetrahydrofuran (7 mL). Subsequently, lithium aluminum hydride solution (1.13 mL, 2.84 mmol) was slowly added to the above solution dropwise. Then the reaction system was continued to be stirred at room temperature for 1 hour.

After the disappearance of raw materials as monitored by LCMS, the reaction solution was quenched by poured into ice water (20 mL). The mixed solution was extracted with ethyl acetate (30 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 120 mg of (2-(trifluoromethyl)-5H-imidazo[2,1-a]isoindol-7-yl)methanol.

MS (ESI) M/Z: 255.1 $[M+H]^+$.

**Step G:** At 0°C, (2-(trifluoromethyl)-5H-imidazo[2,1-a]isoindol-7-yl)methanol (120 mg, 0.47 mmol) was dissolved in dichloromethane (2.4 mL). Then, triphenylphosphine (371 mg, 1.42 mmol), sodium bicarbonate (79 mg, 0.94 mmol) and carbon tetrabromide (471 mg, 1.42 mmol) were added sequentially to the above solution. Then the reaction system was continued to be stirred at room temperature for 1 hour.

After the disappearance of raw materials as monitored by LCMS, the reaction solution was quenched by adding water (10 mL). The mixed solution was extracted with ethyl acetate (10 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 100 mg of 7-(bromomethyl)-2-(trifluoromethyl)-5H-imidazo[2,1-a]isoindole.

MS (ESI) M/Z: 317.0 $[M+H]^+$.

**Intermediate INT-16:** 9-(chloromethyl)-2-(trifluoromethyl)-6,7-dihydro-5H-benzo[c]imidazo[1,2-a]azepine

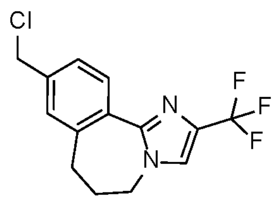

**Reaction route:**

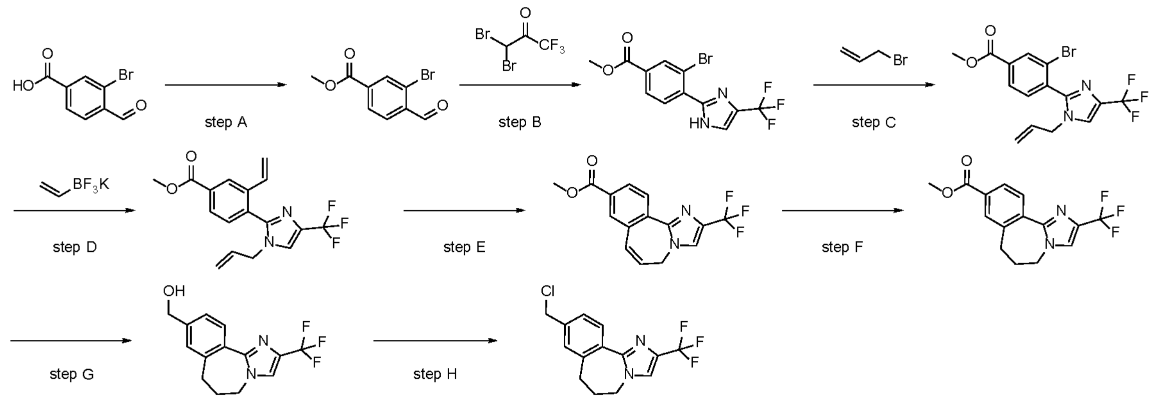


## Operation steps:

**Step A:** At room temperature, 3-bromo-4-formylbenzoic acid (100 g, 436.7 mmol) was dissolved in water (2.7 L). Subsequently, potassium carbonate (90.4 g, 655.1 mmol) and iodomethane (68.2 g, 480.4 mmol) were added to the above solution sequentially. Then the reaction system was continued to be stirred for 2 hours.

After the disappearance of raw materials as monitored by LCMS, the reaction solution was poured into ice water (20 mL), and a solid was precipitated and filtered. The filter cake was dried to obtain 95 g of methyl 3-bromo-4-formylbenzoate.

MS (ESI) M/Z: 243.0 $[M+H]^+$.

**Step B:** At room temperature, 3,3-dibromo-1,1,1-trifluoropropan-2-one (188.7 g, 699.3 mmol) was dissolved in water (340 mL). Subsequently, sodium acetate (57.5 g, 416.1 mmol) was added to the above solution, and the reaction system was heated to 90°C and stirred for 1 hour. Then the reaction solution was cooled down to 0°C, and methyl 3-bromo-4-formylbenzoate (85 g, 349.7 mmol) in a mixed solution of methanol/aqueous ammonia (1020 mL/340 mL) was added dropwise. Then the reaction system was stirred at 100°C for 2 hours.

After the disappearance of raw materials as monitored by LCMS, the reaction solution was concentrated under reduced pressure and filtrated. The resulting filter cake was pulped to obtain 87 g of methyl 3-bromo-4-(4-(trifluoromethyl)-1H-imidazol-2-yl)benzoate.

MS (ESI) M/Z: 349.0 $[M+H]^+$.

**Step C:** At 0°C and under nitrogen protection, methyl 3-bromo-4-(4-(trifluoromethyl)-1H-imidazol-2-yl)benzoate (96.7 g, 277.0 mmol) was dissolved in N,N-dimethylformamide (185 mL). Subsequently, potassium carbonate (80.7 g, 583.9 mmol) and 3-bromoprop-1-ene (40.3 g, 333.1 mmol) were added to the above solution sequentially. Then the reaction system was stirred at 25°C for 20 hours.

After the disappearance of raw materials as monitored by LCMS, the reaction solution was quenched by adding water (800 mL). The mixture was extracted with ethyl acetate (300 mL × 3 times), and organic phases were combined and then washed with saturated saline (500 mL × 2 times). Then the resultant was dried with anhydrous sodium sulfate, filtered, and concentrated under reduced pressure. The resulting residue was purified by silica gel column chromatography to obtain 83.0 g of methyl 4-(1-allyl-4-(trifluoromethyl)-1H-imidazol-2-yl)-3-bromobenzoate.

MS (ESI) M/Z: 389.0 $[M+H]^+$.

**Step D:** At 0°C and under nitrogen protection, methyl 4-(1-allyl-4-(trifluoromethyl)-1H-imidazol-2-yl)-3-bromobenzoate (107 g, 275.0 mmol) was dissolved in 1,4-dioxane/water (1370 mL/228 mL). Subsequently, potassium carbonate (76.2 g, 551.3 mmol), potassium trifluoro(vinyl)borate (96.1 g, 717.4 mmol), 2-bis(cyclohexylphosphino)-2',6'-dimethoxybiphenyl (11.3 g, 27.5 mmol), and palladium acetate (6.2 g, 27.5 mmol) were added to the above solution sequentially. Then the reaction system was stirred at 85°C for 4 hours.

After the disappearance of raw materials as monitored by LCMS, the reaction solution was filtered and the filter cake was rinsed with ethyl acetate (150 mL × 2 times). The filtrate was collected and concentrated under reduced pressure. The residue was diluted by adding water (300 mL), and the mixture was extracted with ethyl acetate (700 mL × 3 times). Organic phases were combined and then washed with saturated saline (400 mL × 2 times). Then the resultant was dried with anhydrous sodium sulfate, filtered, and concentrated under reduced pressure. The resulting residue was purified by silica gel column chromatography to obtain 48.4 g of methyl 4-(1-allyl-4-(trifluoromethyl)-1H-imidazol-2-yl)-3-vinylbenzoate.

MS (ESI) M/Z: 337.2 $[M+H]^+$.

**Step E:** At 0°C and under nitrogen protection, methyl 4-(1-allyl-4-(trifluoromethyl)-1H-imidazol-2-yl)-3-vinylbenzoate (61.4 g, 182.6 mmol) was dissolved in dichloromethane (730 mL). Subsequently, (1,3-bis(2,4,6-trimethylphenyl)-2-imidazolidinylidene)dichloro(o-isopropoxyphenylmethylene)rut henium (8.5 g, 13.7 mmol) was added to the above

solution. Then the reaction system was stirred at 25°C for 3 hours.

After the disappearance of raw materials as monitored by LCMS, the reaction solution was concentrated under reduced pressure and filtrated. The filter cake was rinsed with dichloromethane, and solid was collected and dried to obtain 39.0 g of methyl 2-(trifluoromethyl)-5H-benzo[c]imidazo[1,2-a]azepin-9-formate.

MS (ESI) M/Z: 309.2 $[M+H]^+$.

**Step F:** At room temperature, methyl 2-(trifluoromethyl)-5H-benzo[c]imidazo[1,2-a]azepin-9-formate (39.0 g, 125.8 mmol) and 10% palladium/carbon (8.0 g) were dissolved in methanol (132 mL). The reaction system was then stirred under hydrogen atmosphere for 18 hours.

After the disappearance of raw materials as monitored by LCMS, the reaction solution was filtrated. The filtrate was collected and concentrated under reduced pressure to obtain 37.2 g of methyl 2-(trifluoromethyl)-6,7-dihydro-5H-benzo[c]imidazo[1,2-a]azepin-9-formate.

MS (ESI) M/Z: 311.2 $[M+H]^+$.

**Step G:** At 0°C and under nitrogen protection, methyl 2-(trifluoromethyl)-6,7-dihydro-5H-benzo[c]imidazo[1,2-a]azepin-9-formate (37.2 g, 120.0 mmol) was dissolved in tetrahydrofuran (600 mL). Subsequently, 2.5 M lithium aluminum hydride solution (72 mL) was slowly added to the above solution dropwise. Then the reaction system was continued to be stirred at room temperature for 30 minutes.

After the disappearance of raw materials as monitored by LCMS, the reaction solution was quenched by adding water (100 mL). The reaction solution was filtered and the filter cake was rinsed with ethyl acetate (100 mL × 2 times). The filtrate was collected and layered. The aqueous phase obtained was extracted with ethyl acetate (200 mL × 3 times), and organic phases were combined and then washed with saturated saline (300 mL × 2 times). Then the resultant was dried with anhydrous sodium sulfate, filtered, and concentrated under reduced pressure. The resulting residue was purified by pulping to obtain 30 g of (2-(trifluoromethyl)-6,7-dihydro-5H-benzo[c]imidazo[1,2-a]azepin-9-yl)methanol.

MS (ESI) M/Z: 283.3 $[M+H]^+$.

**Step H:** At 0°C and under nitrogen protection, (2-(trifluoromethyl)-6,7-dihydro-5H-benzo[c]imidazo[1,2-a]azepin-9-yl)methanol (30.0 g, 106.3 mmol) was dissolved in 1,2-dichloroethane (530 mL). Subsequently, thionyl chloride (37.9 g, 319 mmol) was slowly added dropwise to the above solution. Then the reaction system was stirred at 50°C for 20 minutes.

After the disappearance of raw materials as monitored by LCMS, the residue was purified by pulping to obtain 30.4 g of 9-(chloromethyl)-2-(trifluoromethyl)-6,7-dihydro-5H-benzo[c]imidazo[1,2-a]azepine.

MS (ESI) M/Z: 301.2 $[M+H]^+$.

**Example 1: 2-(4-cyclopropyl-6-methoxypyrimidin-5-yl)-9-(4-(1-methyl-4-(trifluoromethyl)-1H-imidazol-2-yl)benzyl)-9H-pyrimido[4,5-b]indole**

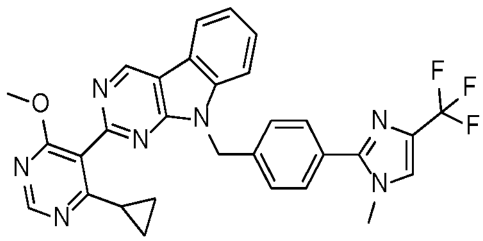

**Reaction route:**

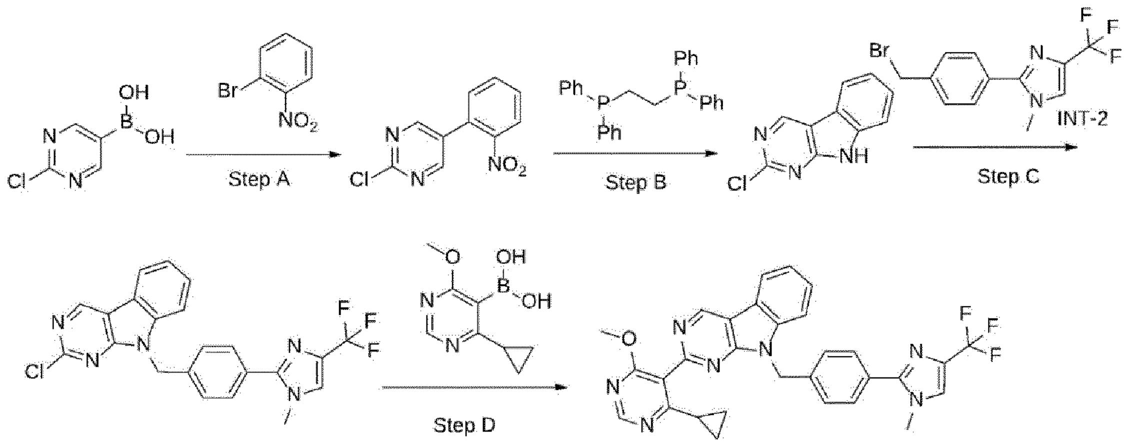


Compound 1

**Operation steps:**

**Step A:** At room temperature, 1-bromo-2-nitrobenzene (500 mg, 2.49 mmol), (2-chloropyrimidin-5-yl)boronic acid (590 mg, 3.74 mmol), sodium carbonate (792 mg, 7.47 mmol) and [1,1'-bis(diphenylphosphino)ferrocene]palladium dichloride dichloromethane complex (305 mg, 0.37 mmol) were dissolved in 1,4-dioxane/water (11 mL/1.4 mL). The reaction system was evacuated to remove air and purged with nitrogen for 3 times, and then stirred at 90°C for 3 hours under nitrogen protection.

After the disappearance of raw materials as monitored by LCMS, the reaction solution was quenched by adding to ice water (20 mL). The mixed solution was extracted with ethyl acetate (20 mL × 3 times), and organic phases were combined and then washed with saturated saline (15 mL × 2 times). Then the resultant was dried with anhydrous sodium sulfate, filtered, and concentrated under reduced pressure to obtain a residue. The residue was purified by silica gel column chromatography to obtain 500 mg of 2-chloro-5-(2-nitrophenyl)pyrimidine.

MS (ESI) M/Z: 236.0 $[M+H]^+$.

**Step B:** At room temperature, 2-chloro-5-(2-nitrophenyl)pyrimidine (500 mg, 2.13 mmol) and 1,2-bis(diphenylphosphino)ethane (1.06 g, 2.66 mmol) were dissolved in 1,2-dichlorobenzene (7.1 mL). Then the reaction system was stirred at 160°C for 1 hour.

After the disappearance of raw materials as monitored by LCMS, the reaction solution was quenched by adding ice water (10 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure to obtain a residue. The residue was purified by silica gel column chromatography to obtain 200 mg of 2-chloro-9H-pyrimido[4,5-b]indole.

MS (ESI) M/Z: 204.0 $[M+H]^+$.

**Step C:** At room temperature and under nitrogen protection, 2-chloro-9H-pyrimido[4,5-b]indole (200 mg, 0.99 mmol) was dissolved in tetrahydrofuran (5 mL). Subsequently, sodium hydride (47 mg, 1.18 mmol) was added to the above solution at 0°C and the resultant was stirred for 30 min, followed by adding 2-(4-(bromomethyl)phenyl)-1-methyl-4-(trifluoromethyl)-1H-imidazole (345 mg, 1.08 mmol). Then the reaction system was continued to be stirred at room temperature for 2 hours.

After the disappearance of raw materials as monitored by LCMS, the reaction solution was quenched by adding ice water (10 mL). The mixed solution was extracted with ethyl acetate (30 mL × 3 times), and organic phases were combined and then washed with saturated saline (20 mL × 2 times). Then the resultant was dried with anhydrous sodium sulfate, filtered, and concentrated under reduced pressure to obtain a residue. The resulting residue was purified by silica gel column chromatography to obtain 300 mg of 2-chloro-9-(4-(1-methyl-4-(trifluoromethyl)-1H-imidazol-2-yl)benzyl)-9H-pyrimido[4,5-b]indole.

MS (ESI) M/Z: 442.0 $[M+H]^+$.

**Step D:** At room temperature, 2-chloro-9-(4-(1-methyl-4-(trifluoromethyl)-1H-imidazol-2-yl)benzyl)-9H-pyrimido[4,5-b]indole (70 mg, 0.16 mmol), (4-cyclopropyl-6-methoxypyrimidin-5-yl)boronic acid (47 mg, 0.24 mmol), sodium carbonate (34 mg, 0.32 mmol) and [1,1'-bis(diphenylphosphino)ferrocene]palladium dichloride dichloromethane complex (19 mg, 0.02 mmol) were dissolved in 1,4-dioxane/water (3.2 mL/0.4 mL). The reaction system was evacuated to remove air and purged with nitrogen for 3 times, and then stirred at 90°C under microwave heating for 3 hours.

After the disappearance of raw materials as monitored by LCMS, the reaction solution was quenched by adding ice water (30 mL). The mixed solution was extracted with ethyl acetate (30 mL × 3 times), and organic phases were

combined and then washed with saturated saline (60 mL × 2 times). Then the resultant was dried with anhydrous sodium sulfate, filtered, and concentrated under reduced pressure to obtain a residue. The residue was purified by preparative high performance liquid chromatography. The product was collected and solvent was removed under reduced pressure. 15.62 mg of 2-(4-cyclopropyl-6-methoxypyrimidin-5-yl)-9-(4-(1-methyl-4-(trifluoromethyl)-1H-imidazol-2-yl) benzyl)-9H-pyrimido[4,5-b]indole was obtained.

MS (ESI) M/Z: 556.0 $[M+H]^+$.

$^1H$ NMR (400 MHz, DMSO-$d_6$): δ 9.65 (s, 1H), 8.71 (s, 1H), 8.37 (d, *J*= 8.0 Hz, 1H), 7.90 (s, 1H), 7.85 (d, *J*= 8.0 Hz, 1H), 7.70 - 7.56 (m, 3H), 7.54 - 7.33 (m, 3H), 5.80 (s, 2H), 3.88 (s, 3H), 3.71 (s, 3H), 1.80 - 1.65 (m, 1H), 1.12 - 0.96 (m, 2H), 0.91 - 0.74 (m, 2H).

**Example 2: 2-(4-cyclopropyl-6-methoxypyrimidin-5-yl)-9-(4-(1-isopropyl-4-(trifluoromethyl)-1H-imidazo l-2-yl) benzyl)-9H-pyrimido[4,5-b]indole**

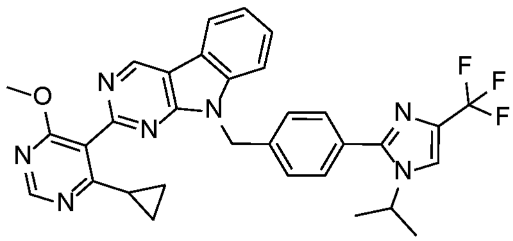

**Reaction route:**

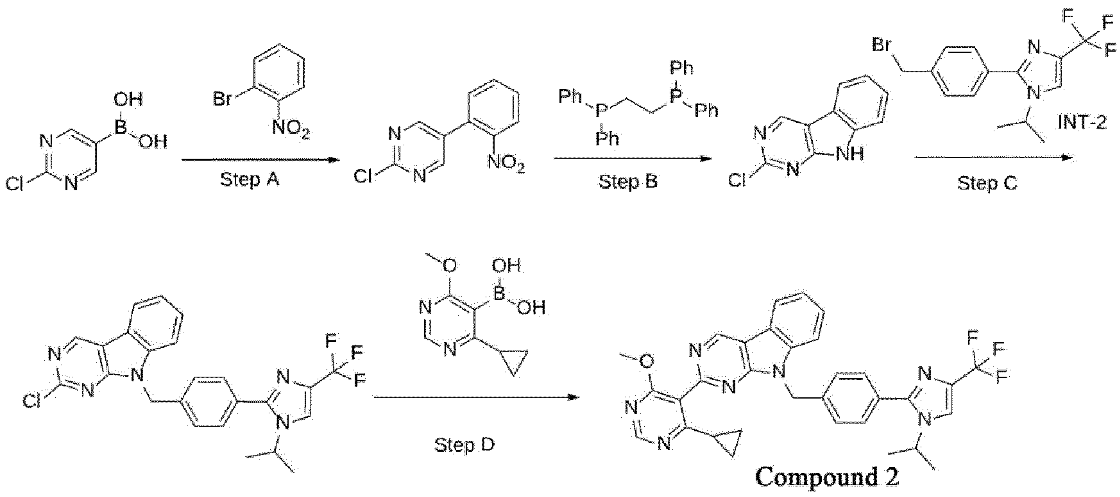


**Operation steps:**

**Step A:** At room temperature and under nitrogen protection, (2-chloropyrimidin-5-yl)boronic acid (437 mg, 2.81 mmol) was dissolved in dry 1,4-dioxane/water (8.4 mL/0.93 mL). Subsequently, 1-bromo-2-nitrobenzene (378 mg, 1.87 mmol), sodium carbonate (595 mg, 5.61 mmol) and [1,1'-bis(diphenylphosphino)ferrocene]palladium dichloride dichloromethane complex (230 mg, 0.28 mmol) were added sequentially to the above solution. The reaction system was evacuated to remove air and purged with nitrogen for 3 times, and then heated to 90°C and stirred for 16 hour.

After the disappearance of raw materials as monitored by LCMS, the reaction solution was quenched by adding water (50 mL), and the mixture was extracted with ethyl acetate (20 mL × 3 times). Organic phases were combined and then washed with saturated saline (20 mL × 2 times). Then the resultant was dried with anhydrous sodium sulfate, filtered, and concentrated under reduced pressure to obtain a residue. The residue was purified by silica gel column chromatography to obtain 280 mg of 2-chloro-5-(2-nitrophenyl)pyrimidine.

MS (ESI) M/Z: 236.0 $[M+H]^+$.

**Step B:** At room temperature, 2-chloro-5-(2-nitrophenyl)pyrimidine (280 mg, 1.19 mmol) was dissolved in 1,2-dichlorobenzene (4 mL). Subsequently, 1,2-bis(diphenylphosphino)ethane (593 mg, 1.49 mmol) was added to the above

solution. The reaction system was evacuated to remove air and purged with nitrogen for 3 times, and then heated to 160°C and stirred for 2 hours.

After the disappearance of raw materials as monitored by LCMS, the reaction solution was concentrated under reduced pressure. The residue was purified by silica gel column chromatography to obtain 120 mg of 2-chloro-9H-pyrimido [4,5-b]indole.

MS (ESI) M/Z: 204.0 $[M+H]^+$.

**Step C:** At room temperature and under nitrogen protection, 2-chloro-9H-pyrimido[4,5-b]indole (120 mg, 0.59 mmol) was dissolved in dry tetrahydrofuran (2 mL). Subsequently, sodium hydride (29 mg, 0.71 mmol) was added to the above solution at 0°C, and the resultant was stirred for 30 minutes. Then 2-(4-(bromomethyl)phenyl)-1-isopropyl-4-(trifluoromethyl)-1H-imidazole (160 mg, 0.65 mmol) was added, and the mixture was heated to room temperature and stirred for 2 hours.

After the disappearance of raw materials as monitored by LCMS, the reaction solution was concentrated under reduced pressure, and the resulting residue was quenched by adding ice water (30 mL), and the mixture was extracted with ethyl acetate (10 mL × 3 times). Organic phases were combined and then washed with saturated saline (15 mL × 2 times). Then the resultant was dried with anhydrous sodium sulfate, filtered, and concentrated under reduced pressure. The resulting residue was purified by silica gel column chromatography to obtain 60 mg of 2-chloro-9-(4-(1-isopropyl-4-(trifluoromethyl)-1H-imidazol-2-yl)benzyl)-9H-pyrimido[4,5-b]indol e.

MS (ESI) M/Z: 470.0 $[M+H]^+$.

**Step D:** At room temperature and under nitrogen protection, 2-chloro-9-(4-(1-isopropyl-4-(trifluoromethyl)-1H-imidazol-2-yl)benzyl)-9H-pyrimido[4,5-b]indol e (60 mg, 0.13 mmol) was dissolved in dry 1,4-dioxane/water (0.9 mL/0.1 mL). Subsequently, (4-cyclopropyl-6-methoxypyrimidin-5-yl)boronic acid (50 mg, 0.27 mmol), cesium carbonate (83 mg, 0.27 mmol), and chloro(2-dicyclohexylphosphino-2',4',6'-triisopropyl-1,1'-biphenyl)[2-(2'-amino-1,1'-biphenyl)]p alladium(II) (20 mg, 0.03 mmol) were added successively. The reaction system was evacuated to remove air and purged with nitrogen for 3 times and then heated to 90°C by microwave heating and stirred for 3 hours.

After the disappearance of raw materials as monitored by LCMS, the reaction solution was quenched by adding water (30 mL), and the mixture was extracted with ethyl acetate (20 mL × 3 times). Organic phases were combined and then washed with saturated saline (20 mL × 2 times). Then the resultant was dried with anhydrous sodium sulfate, filtered, and concentrated under reduced pressure. The residue was purified by preparative high performance liquid chromatography. The product was collected and solvent was removed under reduced pressure. 7.66 mg of 2-(4-cyclopropyl-6-methoxypyrimidin-5-yl)-9-(4-(1-isopropyl-4-(trifluoromethyl)-1H-imidazol-2-yl)benzyl)-9H-pyrimido[4,5-b]indole was obtained.

MS (ESI) M/Z: 583.8 $[M+H]^+$.

$^1$H NMR (400 MHz, DMSO-$d_6$): δ 9.66 (s, 1H), 8.70 (s, 1H), 8.37 (d, *J* = 8.0 Hz, 1H), 8.14 (s, 1H), 7.87 (d, *J* = 8.4 Hz, 1H), 7.64 (t, *J* = 7.8 Hz, 1H), 7.54 - 7.38 (m, 5H), 5.81 (s, 2H), 4.44 - 4.30 (m, 1H), 3.87 (s, 3H), 1.75 - 1.69 (m, 1H), 1.35 (d, *J* = 6.8 Hz, 6H), 1.10 - 1.01 (m, 2H), 0.88 - 0.79 (m, 2H).

**Example 3: 2-(4-cyclopropyl-6-methoxypyrimidin-5-yl)-9-(4-(1-isopropyl-4-(trifluoromethyl)-1H-imidazo l-2-yl) benzyl)-9H-pyridino[2',3':4,5]pyrrolo[2,3-d]pyrimidine**

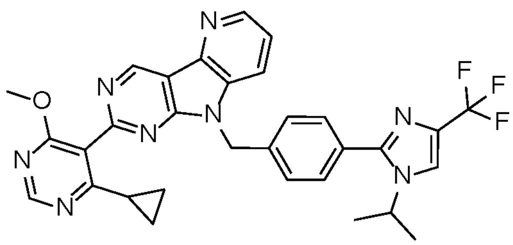

**Reaction route:**

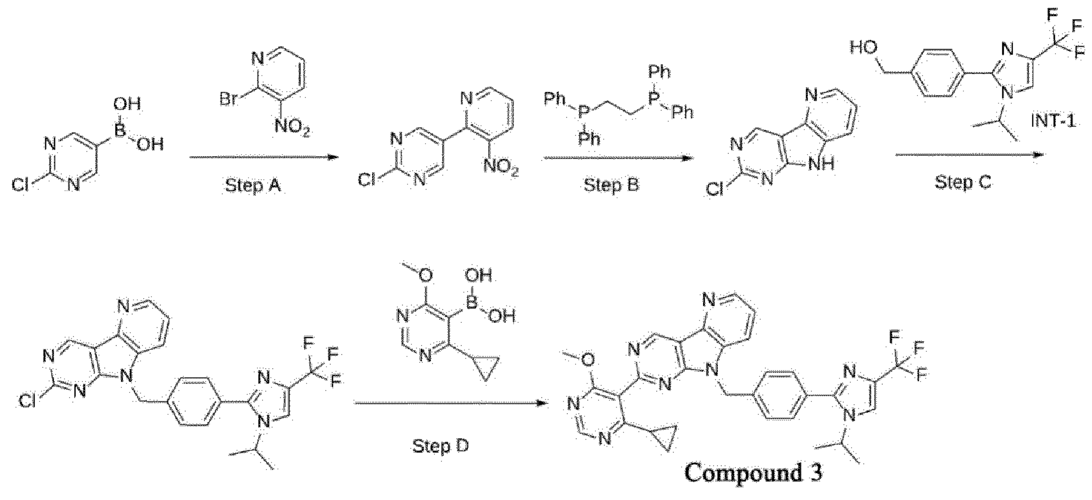


Compound 3

**Operation steps:**

**Step A:** At room temperature, (2-chloropyrimidin-5-yl)boronic acid (1 g, 6.33 mmol), 2-bromo-3-nitropyridine (857 mg, 4.2 mmol) and sodium carbonate (1.34 g, 12.64 mmol) were dissolved in 1,4-dioxane/water (21 mL/2.3 mL). Subsequently, dichloro[1,1'-bis(diphenylphosphino)ferrocene]palladium (513 mg, 0.6 mmol) was added to the above solution, and the reaction system was evacuated to remove air and purged with nitrogen for 4 times, and then heated to 100°C and stirred for 16 hours.

After the disappearance of raw materials as monitored by LCMS, the reaction solution was quenched by adding to ice water (30 mL), and the mixture was extracted with ethyl acetate (20 mL × 3 times). Organic phases were combined and then washed with saturated saline (15 mL). Then the resultant was dried with anhydrous sodium sulfate, filtered, and concentrated under reduced pressure. The residue was purified by silica gel column chromatography to obtain 600 mg of 2-chloro-5-(3-nitropyridin-2-yl)pyrimidine.

MS (ESI) M/Z: 237.0 $[M+H]^+$.

**Step B:** At room temperature, 2-chloro-5-(3-nitropyridin-2-yl)pyrimidine (600 mg, 2.54 mmol) and 1,2-bis(diphenylphosphino)ethane (1.26 g, 3.18 mmol) were dissolved in 1,2-dichlorobenzene (13 mL). The reaction system was evacuated to remove air and purged with nitrogen for 4 times, and then heated to 160°C and stirred for 2 hours.

After the disappearance of raw materials as monitored by LCMS, the reaction solution was quenched by adding to ice water (30 mL), and the mixture was extracted with ethyl acetate (20 mL × 3 times). Organic phases were combined and then washed with saturated saline (15 mL). Then the resultant was dried with anhydrous sodium sulfate, filtered, and concentrated under reduced pressure. The resulting residue was purified by silica gel column chromatography to obtain 400 mg of 2-chloro-9H-pyridino[2',3':4,5]pyrrolo[2,3-d]pyrimidine.

MS (ESI) M/Z: 205.2 $[M+H]^+$.

**Step C:** At room temperature and under nitrogen protection, 2-chloro-9H-pyridino[2',3':4,5]pyrrolo[2,3-d]pyrimidine (400 mg, 1.96 mmol), (4-(1-isopropyl-4-(trifluoromethyl)-1H-imidazol-2-yl)phenyl)methanol (555 mg, 1.96 mmol) and triphenylphosphine (771 mg, 2.94 mmol) were dissolved in dry tetrahydrofuran (10 mL). Subsequently, diisopropyl azodicarboxylate (594 mg, 2.94 mmol) was slowly added dropwise to the above solution at 0°C. Then the reaction system was continued to be stirred at room temperature for 2 hours.

After the disappearance of raw materials as monitored by LCMS, the reaction solution was quenched by adding water (30 mL), and the mixture was extracted with ethyl acetate (20 mL × 3 times). Organic phases were combined and then washed with saturated saline (15 mL). Then the resultant was dried with anhydrous sodium sulfate, filtered, and concentrated under reduced pressure. The resulting residue was purified by silica gel column chromatography to obtain 250 mg of 2-chloro-9-(4-(1-isopropyl-4-(trifluoromethyl)-1H-imidazol-2-yl)benzyl)-9H-pyridino[2',3':4,5]py rrolo[2,3-d] pyrimidine.

MS (ESI) M/Z: 470.8 $[M+H]^+$.

**Step D:** At room temperature, 2-chloro-9-(4-(1-isopropyl-4-(trifluoromethyl)-1H-imidazol-2-yl)benzyl)-9H-pyridino[2',3':4,5]py rrolo[2,3-d]pyrimidine (250 mg, 0.53 mmol), (4-cyclopropyl-6-methoxypyrimidin-5-yl)boronic acid (210 mg, 1.07 mmol), and cesium carbonate (346 mg, 1.06 mmol) were dissolved in 1,4-dioxane/water (3 mL/0.3 mL). Subsequently, chloro(2-dicyclohexylphosphino-2',4',6'-triisopropyl-1,1'-biphenyl)[2-(2'-amino-1,1'-biphenyl)]p alladium(II) (85 mg, 0.11 mmol) was added to the above solution. The reaction system was evacuated to remove air and

purged with nitrogen for 4 times, and then stirred at 100°C under microwave heating for 3 hours.

After the disappearance of raw materials as monitored by LCMS, the reaction solution was quenched by adding water (30 mL), and the mixture was extracted with ethyl acetate (20 mL × 3 times). Organic phases were combined and then washed with saturated saline (20 mL × 2 times). Then the resultant was dried with anhydrous sodium sulfate, filtered, and concentrated under reduced pressure. The residue was purified by preparative high performance liquid chromatography. The product was collected and solvent was removed under reduced pressure. 7.35 mg of 2-(4-cyclopropyl-6-methoxypyrimidin-5-yl)-9-(4-(1-isopropyl-4-(trifluoromethyl)-1H-imidazol-2-yl)benzyl)-9H-pyridino[2',3':4,5]pyrrolo[2,3-d]pyrimidine was obtained.

MS (ESI) M/Z: 585.0 $[M+H]^+$.

$^1H$ NMR (400 MHz, DMSO-$d_6$): δ 9.70 (s, 1H), 8.72 (s, 1H), 8.70 (dd, *J* = 4.8, 1.2 Hz, 1H), 8.37 (dd, *J* = 8.4, 1.2 Hz, 1H), 8.15 (d, *J* = 1.2 Hz, 1H), 7.66 (dd, *J* = 8.4, 4.8 Hz, 1H), 7.50 (brs, 4H), 5.85 (s, 2H), 4.45 - 4.32 (m, 1H), 3.88 (s, 3H), 1.80 - 1.70 (m, 1H), 1.35 (d, *J*= 6.4 Hz, 6H), 1.11 - 1.03 (m, 2H), 0.89 - 0.79 (m, 2H).

**Example 4: 2-(4-cyclopropyl-6-methoxypyrimidin-5-yl)-5-fluoro-9-(4-(1-isopropyl-4-(trifluoromethyl)-1H -imidazol-2-yl)benzyl)-9H-pyrimido[4,5-b]indole**

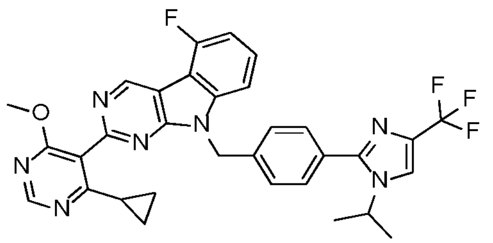

**Reaction route:**

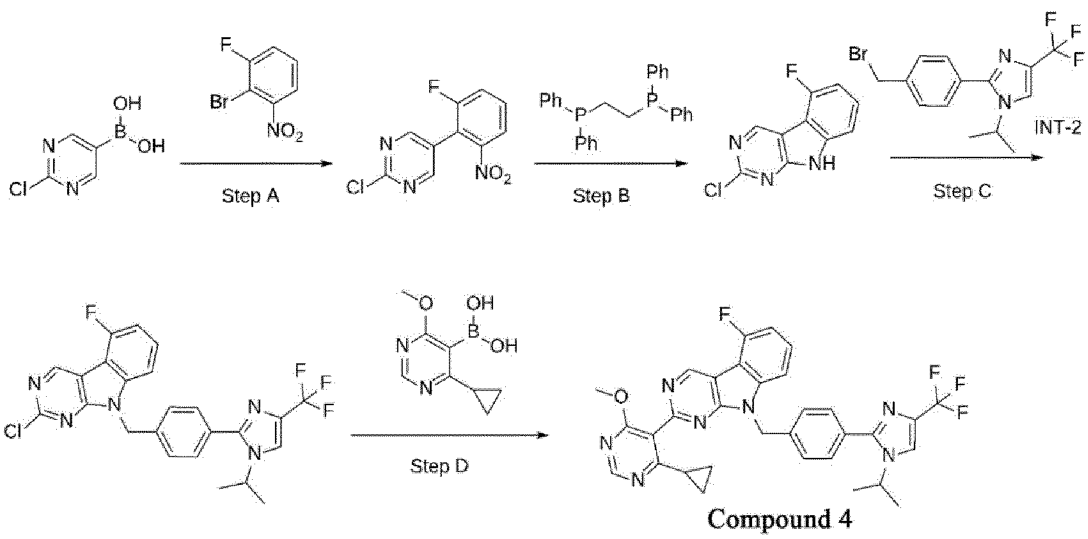


Compound 4

**Operation steps:**

**Step A:** At room temperature, 2-bromo-1-fluoro-3-nitrobenzene (914 mg, 4.15 mmol), (2-chloropyrimidin-5-yl) boronic acid (985 mg, 6.23 mmol), sodium carbonate (1.32 g, 12.45 mmol) and [1,1'-bis(diphenylphosphino)ferrocene] palladium dichloride dichloromethane complex (508 mg, 0.62 mmol) were dissolved in 1,4-dioxane/water (20.7 mL/2.3 mL). The reaction system was evacuated to remove air and purged with nitrogen for 3 times, and then stirred at 90°C for 3 hours.

After the disappearance of raw materials as monitored by LCMS, the reaction solution was quenched by adding to ice water (40 mL). The mixed solution was extracted with ethyl acetate (30 mL × 3 times), and organic phases were combined and then washed with saturated saline (60 mL × 2 times). Then the resultant was dried with anhydrous sodium sulfate, filtered, and concentrated under reduced pressure. The residue was purified by silica gel column chromatography

to obtain 516 mg of 2-chloro-5-(2-fluoro-6-nitrophenyl)pyrimidine.

MS (ESI) M/Z: 254.0 $[M+H]^+$.

**Step B:** At room temperature, 2-chloro-5-(2-fluoro-6-nitrophenyl)pyrimidine (400 mg, 1.58 mmol) and 1,2-bis(diphenylphosphino)ethane (786 mg, 1.97 mmol) were dissolved in 1,2-dichlorobenzene (7.9 mL). Then the reaction system was stirred at 160°C for 1 hour.

After the disappearance of raw materials as monitored by LCMS, the reaction solution was quenched by adding ice water (30 mL), and the mixture was extracted with ethyl acetate (20 mL × 3 times). Organic phases were combined and then washed with saturated saline (20 mL × 2 times). Then the resultant was dried with anhydrous sodium sulfate, filtered, and concentrated under reduced pressure. The residue was purified by silica gel column chromatography to obtain 276 mg of 2-chloro-5-fluoro-9H-pyrimido[4,5-b]indole.

MS (ESI) M/Z: 222.0 $[M+H]^+$.

**Step C:** At room temperature and under nitrogen protection, 2-chloro-5-fluoro-9H-pyrimido[4,5-b] indole (226 mg, 1.02 mmol) and 2-(4-(bromomethyl)phenyl)-1-isopropyl-4-(trifluoromethyl)-1H-imidazole (283 mg, 0.82 mmol) were dissolved in N,N-dimethylformamide (5 mL). Subsequently, potassium carbonate (281 mg, 2.04 mmol) was added to the above solution. Then the reaction system was stirred at 50°C for 2 hours.

After the disappearance of raw materials as monitored by LCMS, the reaction solution was quenched by adding ice water (40 mL), and the mixture was extracted with ethyl acetate (15 mL × 3 times). Organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 220 mg of 2-chloro-5-fluoro-9-(4-(1-isopropyl-4-(trifluoromethyl)-1H-imidazol-2-yl)benzyl)-9H-pyrimido[4, 5-b]indole.

MS (ESI) M/Z: 488.0 $[M+H]^+$.

**Step D:** At room temperature, 2-chloro-5-fluoro-9-(4-(1-isopropyl-4-(trifluoromethyl)-1H-imidazol-2-yl)benzyl)-9H-pyrimido[4, 5-b]indole (200 mg, 0.41 mmol), (4-cyclopropyl-6-methoxypyrimidin-5-yl)boronic acid (239 mg, 1.23 mmol), cesium carbonate (200 mg, 0.62 mmol) and [1,1'-bis(diphenylphosphino)ferrocene]palladium dichloride dichloromethane complex (65 mg, 0.08 mmol) were dissolved in 1,4-dioxane/water (2 mL/0.2 mL). The reaction system was evacuated to remove air and purged with nitrogen for 3 times, and then stirred at 100°C under microwave heating for 3 hours.

After the disappearance of raw materials as monitored by LCMS, the reaction solution was quenched by adding ice water (30 mL), and the mixture was extracted with ethyl acetate (30 mL × 3 times). Organic phases were combined and then washed with saturated saline (60 mL × 2 times). Then the resultant was dried with anhydrous sodium sulfate, filtered, and concentrated under reduced pressure. The residue was purified by preparative high performance liquid chromatography. The product was collected and solvent was removed under reduced pressure. 16.09 mg of 2-(4-cyclopropyl-6-methoxypyrimidin-5-yl)-5-fluoro-9-(4-(1-isopropyl-4-(trifluoromethyl)-1H-im idazol-2-yl)benzyl)-9H-pyrimido[4,5-b]indole was obtained.

MS (ESI) M/Z: 602.4 $[M+H]^+$.

$^1H$ NMR (400 MHz, DMSO-$d_6$): δ 9.52 (s, 1H), 8.71 (s, 1H), 8.15 (d, *J*= 1.2 Hz, 1H), 7.76 (d, *J*= 8.0 Hz, 1H), 7.71 - 7.63 (m, 1H), 7.52 - 7.47 (m, 4H), 7.31 - 7.26 (m, 1H), 5.83 (s, 2H), 4.44 - 4.33 (m, 1H), 3.88 (s, 3H), 1.78 - 1.69 (m, 1H), 1.35 (d, *J* = 6.4 Hz, 6H), 1.10 - 1.02 (m, 2H), 0.88 - 0.80 (m, 2H).

**Example 5: 2-(4-cyclopropyl-6-methoxypyrimidin-5-yl)-7-fluoro-9-(4-(1-isopropyl-4-(trifluoromethyl)-1H -imidazol-2-yl)benzyl)-9H-pyrimido[4,5-b]indole**

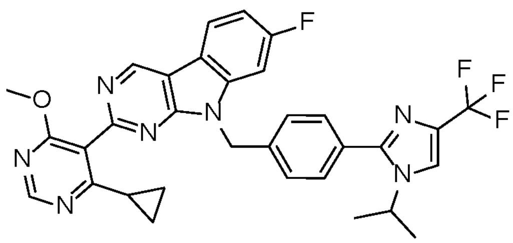

**Reaction route:**

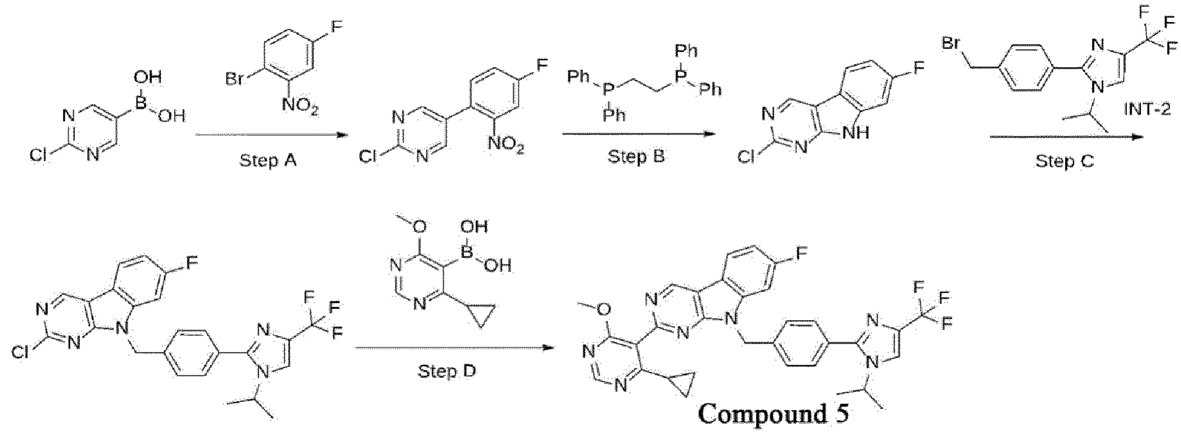


Compound 5

**Operation steps:**

**Step A:** At room temperature, 1-bromo-4-fluoro-2-nitrobenzene (1.50 g, 6.85 mmol), (2-chloropyrimidin-5-yl) boronic acid (1.62 g, 10.27 mmol), sodium carbonate (2.18 g, 20.55 mmol) and [1,1'-bis(diphenylphosphino)ferrocene] palladium dichloride dichloromethane complex (840 mg, 1.03 mmol) were dissolved in 1,4-dioxane/water (31 mL/3.5 mL). The reaction system was evacuated to remove air and purged with nitrogen for 3 times, and then stirred at 90°C for 3 hour.

After the disappearance of raw materials as monitored by LCMS, the reaction solution was quenched by adding ice water (60 mL), and the mixture was extracted with ethyl acetate (20 mL × 3 times). Organic phases were combined and then washed with saturated saline (20 mL × 2 times). Then the resultant was dried with anhydrous sodium sulfate, filtered, and concentrated under reduced pressure. The residue was purified by silica gel column chromatography to obtain 1.1 g of 2-chloro-5-(4-fluoro-2-nitrophenyl)pyrimidine.

MS (ESI) M/Z: 253.8 $[M+H]^+$.

**Step B:** At room temperature, 2-chloro-5-(4-fluoro-2-nitrophenyl)pyrimidine (1.1 g, 4.34 mmol) and 1,2-bis(diphenylphosphino)ethane (2.16 g, 5.43 mmol) were dissolved in 1,2-dichlorobenzene (15 mL). Then the reaction system was stirred at 160°C for 2 hours.

After the disappearance of raw materials as monitored by LCMS, the reaction solution was quenched by adding ice water (50 mL), and the mixture was extracted with ethyl acetate (20 mL × 3 times). Organic phases were combined and then washed with saturated saline (20 mL × 2 times). Then the resultant was dried with anhydrous sodium sulfate, filtered, and concentrated under reduced pressure. The residue was purified by silica gel column chromatography to obtain 261 mg of 2-chloro-7-fluoro-9H-pyrimido[4,5-b]indole.

MS (ESI) M/Z: 221.9 $[M+H]^+$.

**Step C:** At room temperature and under nitrogen protection, 2-chloro-7-fluoro-9H-pyrimido[4,5-b] indole (261 mg, 1.18 mmol), 2-(4-(bromomethyl)phenyl)-1-isopropyl-4-(trifluoromethyl)-1H-imidazole (490 mg, 1.42 mmol), and potassium carbonate (326 mg, 2.36 mmol) were dissolved in N,N-dimethylformamide (6 mL). Then the reaction system was stirred at 50°C for 2 hours.

After the disappearance of raw materials as monitored by LCMS, the reaction solution was quenched by adding water (30 mL), and the mixture was extracted with ethyl acetate (10 mL × 3 times). Organic phases were combined and then washed with saturated saline (30 mL × 2 times). Then the resultant was dried with anhydrous sodium sulfate, filtered, and concentrated under reduced pressure. The resulting residue was purified by silica gel column chromatography to obtain 170 mg of 2-chloro-7-fluoro-9-(4-(1-isopropyl-4-(trifluoromethyl)-1H-imidazol-2-yl)benzyl)-9H-pyrimido[4, 5-b]indole.

MS (ESI) M/Z: 487.8 $[M+H]^+$.

**Step D:** At room temperature, 2-chloro-7-fluoro-9-(4-(1-isopropyl-4-(trifluoromethyl)-1H-imidazol-2-yl)benzyl)-9H-pyrimido[4, 5-b]indole (130 mg, 0.27 mmol), (4-cyclopropyl-6-methoxypyrimidin-5-yl)boronic acid (156 mg, 0.80 mmol), cesium carbonate (174 mg, 0.53 mmol) and chloro(2-dicyclohexylphosphino-2',4',6'-triisopropyl-1,1'-biphenyl)[2-(2'-amino-1,1'-biphenyl)]p alladium(II) (42 mg, 0.05 mmol) were dissolved in 1,4-dioxane/water (1.2 mL/0.13 mL). The reaction system was evacuated to remove air and purged with nitrogen for 3 times, and then stirred at 90°C under microwave heating for 3 hours.

After the disappearance of raw materials as monitored by LCMS, the reaction solution was quenched by adding ice water (30 mL), and the mixture was extracted with ethyl acetate (20 mL × 3 times). Organic phases were combined and then washed with saturated saline (30 mL × 2 times). Then the resultant was dried with anhydrous sodium sulfate, filtered, and concentrated under reduced pressure. The residue was purified by preparative high performance liquid chromatography. The product was collected and solvent was removed under reduced pressure. 17.11 mg of 2-(4-cyclopropyl-6-methoxypyrimidin-5-yl)-7-fluoro-9-(4-(1-isopropyl-4-(trifluoromethyl)-1H-imidazol-2-yl)benzyl)-9H-pyrimido[4,5-b]indole was obtained.

MS (ESI) M/Z: 602.0 $[M+H]^+$.

$^1H$ NMR (400 MHz, DMSO-$d_6$): δ 9.65 (s, 1H), 8.70 (s, 1H), 8.41 (dd, *J* = 8.6, 5.4 Hz, 1H), 8.15 (d, *J* = 1.2 Hz, 1H), 7.85 (dd, *J* = 10.0, 2.4 Hz, 1H), 7.54 - 7.46 (m, 4H), 7.33 - 7.26 (m, 1H), 5.79 (s, 2H), 4.45 - 4.31 (m, 1H), 3.87 (s, 3H), 1.75 - 1.66 (m, 1H), 1.35 (d, *J*= 6.8 Hz, 6H), 1.09 - 1.00 (m, 2H), 0.88 - 0.77 (m, 2H).

**Example 6: 2-(4-cyclopropyl-6-methoxypyrimidin-5-yl)-8-fluoro-9-(4-(1-isopropyl-4-(trifluoromethyl)-1H -imidazol-2-yl)benzyl)-9H-pyrimido[4,5-b]indole**

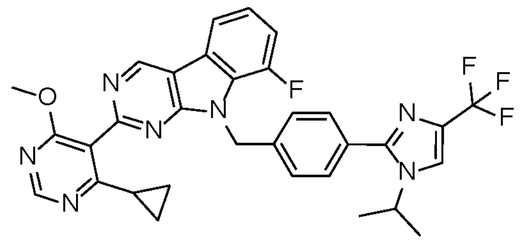

**Reaction route:**

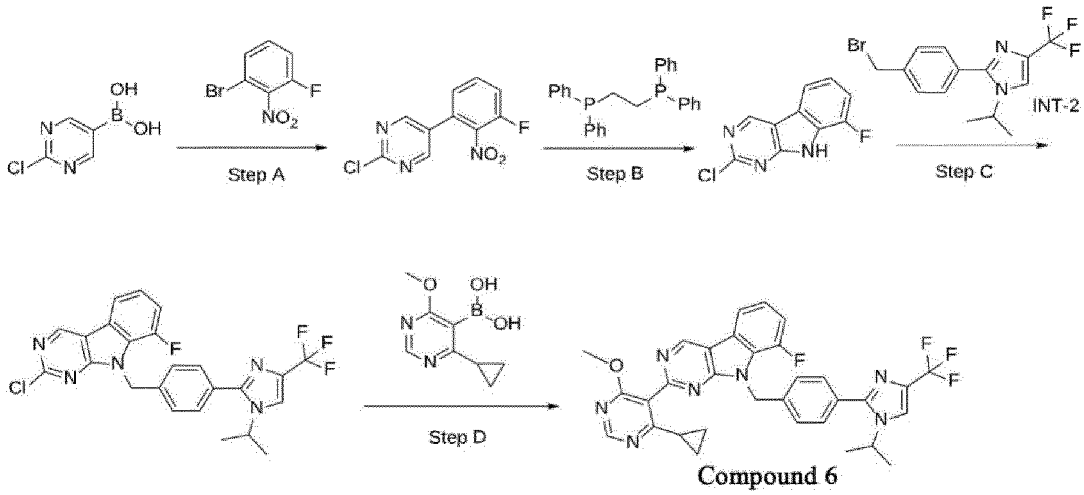

Compound 6

**Operation steps:**

**Step A:** At room temperature, 1-bromo-3-fluoro-2-nitrobenzene (1 g, 4.54 mmol), (2-chloropyrimidin-5-yl) boronic acid (1.79 g, 6.82 mmol), sodium carbonate (1.45 g, 13.63 mmol) and [1,1'-bis(diphenylphosphino)ferrocene] palladium dichloride dichloromethane complex (498 mg, 0.68 mmol) were dissolved in 1,4-dioxane/water (19.8 mL/2.2 mL). The reaction system was evacuated to remove air and purged with nitrogen for 3 times, and then stirred at 90°C for 3 hours.

After the disappearance of raw materials as monitored by LCMS, the reaction solution was quenched by adding ice water (20 mL), and the mixture was extracted with ethyl acetate (20 mL × 3 times). Organic phases were combined and then washed with saturated saline (15 mL × 2 times). Then the resultant was dried with anhydrous sodium sulfate, filtered, and concentrated under reduced pressure. The residue was purified by silica gel column chromatography to obtain 500 mg of 2-chloro-5-(3-fluoro-2-nitrophenyl)pyrimidine.

MS (ESI) M/Z: 254.0 $[M+H]^+$.

**Step B:** At room temperature, 2-chloro-5-(3-fluoro-2-nitrophenyl)pyrimidine (500 mg, 1.97 mmol) and 1,2-bis(diphenylphosphino)ethane (979 mg, 2.46 mmol) were dissolved in 1,2-dichlorobenzene (7.1 mL). Then the reaction system was stirred at 160°C for 1 hour.

After the disappearance of raw materials as monitored by LCMS, the reaction solution was quenched by adding ice water (10 mL), and the mixture was extracted with ethyl acetate (20 mL × 3 times). Organic phases were combined and

then washed with saturated saline (20 mL × 2 times). Then the resultant was dried with anhydrous sodium sulfate, filtered, and concentrated under reduced pressure. The residue was purified by silica gel column chromatography to obtain 60 mg of 2-chloro-8-fluoro-9H-pyrimido[4,5-b]indole.

MS (ESI) M/Z: 222.0 $[M+H]^+$.

**Step C:** At room temperature and under nitrogen protection, 2-chloro-8-fluoro-9H-pyrimido[4,5-b]indole (60 mg, 0.27 mmol) and anhydrous potassium carbonate (75 mg, 0.54 mmol) were dissolved in N,N-dimethylformamide (3 mL). Then 2-(4-(bromomethyl)phenyl)-1-isopropyl-4-(trifluoromethyl)-1H-imidazole (113 mg, 0.31 mmol) was added to the above solution. Then the reaction system was stirred at room temperature for 2 hours.

After the disappearance of raw materials as monitored by LCMS, the reaction solution was quenched by adding ice water (10 mL), and the mixture was extracted with ethyl acetate (30 mL × 3 times). Organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 90 mg of 2-chloro-8-fluoro-9-(4-(1-isopropyl-4-(trifluoromethyl)-1H-imidazol-2-yl)benzyl)-9H-pyrimido[4, 5-b]indole.

MS (ESI) M/Z: 488.3 $[M+H]^+$.

**Step D:** At room temperature, 2-chloro-8-fluoro-9-(4-(1-isopropyl-4-(trifluoromethyl)-1H-imidazol-2-yl)benzyl)-9H-pyrimido[4, 5-b]indole (90 mg, 0.184 mmol), (4-cyclopropyl-6-methoxypyrimidin-5-yl)boronic acid (107.8 mg, 0.55 mmol), cesium carbonate (120 mg, 0.37 mmol) and chloro(2-dicyclohexylphosphino-2',4',6'-triisopropyl-1,1'-biphenyl)[2-(2'-amino-1,1'-biphenyl)]p alladium(II) (22 mg, 0.03 mmol) were dissolved in 1,4-dioxane/water (0.8 mL/0.08 mL). The reaction system was evacuated to remove air and purged with nitrogen for 3 times, and then stirred at 90°C under microwave heating for 2.5 hours.

After the disappearance of raw materials as monitored by LCMS, the reaction solution was quenched by adding ice water (30 mL), and the mixture was extracted with ethyl acetate (30 mL × 3 times). Organic phases were combined and then washed with saturated saline (60 mL × 2 times). Then the resultant was dried with anhydrous sodium sulfate, filtered, and concentrated under reduced pressure. The residue was purified by preparative high performance liquid chromatography. The product was collected and solvent was removed under reduced pressure. 4.89 mg of 2-(4-cyclopropyl-6-methoxypyrimidin-5-yl)-8-fluoro-9-(4-(1-isopropyl-4-(trifluoromethyl)-1H-im idazol-2-yl)benzyl)-9H-pyrimido[4,5-b]indole was obtained.

MS (ESI) M/Z: 602.2 $[M+H]^+$.

$^1$H NMR (400 MHz, DMSO-$d_6$): δ 9.75 (s, 1H), 8.71 (s, 1H), 8.24 (d, *J* = 7.6 Hz, 1H), 8.15 (d, *J* = 0.8 Hz, 1H), 7.55 - 7.46 (m, 3H), 7.45 - 7.35 (m, 3H), 5.86 (s, 2H), 4.45 - 4.32 (m, 1H), 3.87 (s, 3H), 1.77 - 1.68 (m, 1H), 1.36 (d, *J* = 6.8 Hz, 6H), 1.09 - 1.02 (m, 2H), 0.88 - 0.81 (m, 2H).

**Example 7: 2-(4-cyclopropyl-6-methoxypyrimidin-5-yl)-9-(4-(1-isopropyl-4-(trifluoromethyl)-1H-imidazo l-2-yl) benzyl)-7-methoxy-9H-pyrimido[4,5-b]indole**

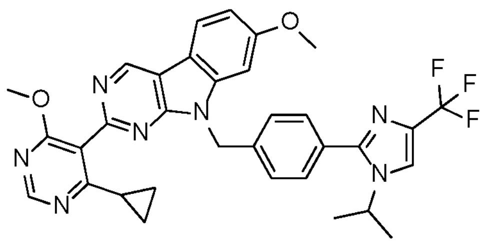

## Reaction route:

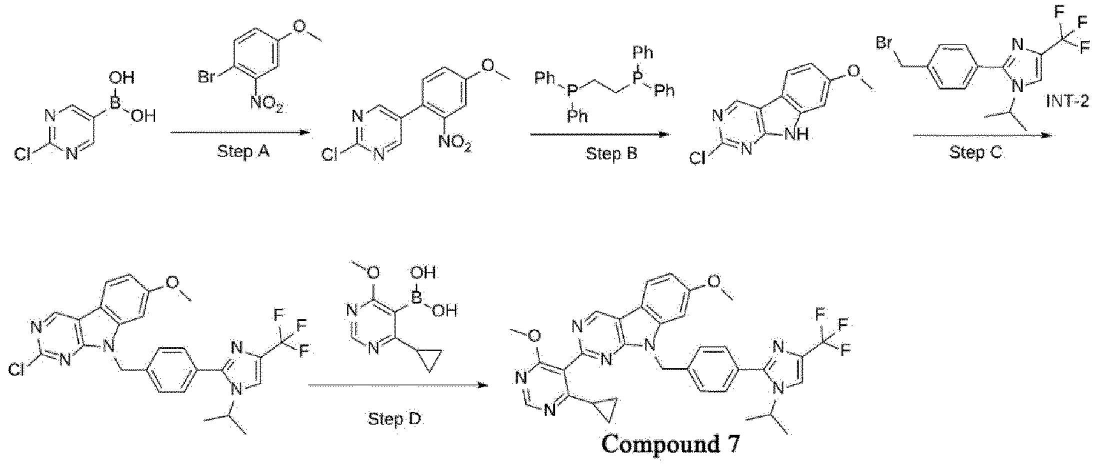


Compound 7

**Operation steps:**

**Step A:** At room temperature, (2-chloropyrimidin-5-yl)boronic acid (1.5 g, 9.49 mmol), 1-bromo-4-methoxy-2-nitrobenzene (1.5 g, 6.49 mmol) and sodium carbonate (2.1 g, 19.81 mmol) were dissolved in 1,4-dioxane/water (32 mL/3.5 mL). Subsequently, dichloro[1,1'-bis(diphenylphosphino)ferrocene]palladium (0.8 g, 0.98 mmol) was added to the above solution, and the reaction system was evacuated to remove air and purged with nitrogen for 4 times, and then stirred at 100°C for 16 hours.

After the disappearance of raw materials as monitored by LCMS, the reaction solution was quenched by adding to ice water (30 mL), and the mixture was extracted with ethyl acetate (20 mL × 3 times). Organic phases were combined and then washed with saturated saline (15 mL). Then the resultant was dried with anhydrous sodium sulfate, filtered, and concentrated under reduced pressure. The residue was purified by silica gel column chromatography to obtain 740 mg of 2-chloro-5-(4-methoxy-2-nitrophenyl)pyrimidine.

MS (ESI) M/Z: 266.0 $[M+H]^+$.

**Step B:** At room temperature, 2-chloro-5-(4-methoxy-2-nitrophenyl)pyrimidine (740 mg, 2.79 mmol) and 1,2-bis(diphenylphosphino)ethane (2.22 g, 5.58 mmol) were dissolved in 1,2-dichlorobenzene (14 mL). The reaction system was evacuated to remove air and purged with nitrogen for 4 times, and then stirred at 160°C for 2 hours.

After the disappearance of raw materials as monitored by LCMS, the reaction solution was quenched by adding to ice water (30 mL), and the mixture was extracted with ethyl acetate (20 mL × 3 times). Organic phases were combined and then washed with saturated saline (15 mL). Then the resultant was dried with anhydrous sodium sulfate, filtered, and concentrated under reduced pressure. The residue was purified by silica gel column chromatography to obtain 440 mg of 2-chloro-7-methoxy-9H-pyrimido[4,5-b]indole.

MS (ESI) M/Z: 234.0 $[M+H]^+$.

**Step C:** At room temperature and under nitrogen protection, 2-chloro-7-methoxy-9H-pyrimido[4,5-b]indole (440 mg, 1.89 mmol), 2-(4-(bromomethyl)phenyl)-1-isopropyl-4-(trifluoromethyl)-1H-imidazole (410 mg, 1.13 mmol), and potassium carbonate (521 mg, 3.78 mmol) were dissolved in dry N,N-dimethylformamide (10 mL). The reaction system was stirred at 50°C for 2 hours.

After the disappearance of raw materials as monitored by LCMS, the reaction solution was quenched by adding to ice water (50 mL), and the mixture was extracted with ethyl acetate (150 mL × 3 times). Organic phases were combined and then washed with saturated saline (25 mL). Then the resultant was dried with anhydrous sodium sulfate, filtered, and concentrated under reduced pressure. The resulting residue was purified by silica gel column chromatography to obtain 70 mg of 2-chloro-9-(4-(1-isopropyl-4-(trifluoromethyl)-1H-imidazol-2-yl)benzyl)-7-methoxy-9H-pyrimido [4,5-b]indole.

MS (ESI) M/Z: 500.0 $[M+H]^+$.

**Step D:** At room temperature, 2-chloro-9-(4-(1-isopropyl-4-(trifluoromethyl)-1H-imidazol-2-yl)benzyl)-7-methoxy-9H-pyrimido [4,5-b]indole (70 mg, 0.14 mmol), (4-cyclopropyl-6-methoxypyrimidin-5-yl)boronic acid (84 mg, 0.43 mmol), and cesium carbonate (70 mg, 0.21 mmol) were dissolved in 1,4-dioxane/water (1 mL/0.1 mL). Subsequently, chloro(2-dicyclohexylphosphino-2',4',6'-triisopropyl-1,1'-biphenyl)[2-(2'-amino-1,1'-biphenyl)]p alladium(II) (22 mg, 0.03 mmol) was added to the above solution. The reaction system was evacuated to remove air and purged with nitrogen for 4 times, and then stirred at 90°C under microwave heating for 3 hours.

After the disappearance of raw materials as monitored by LCMS, the reaction solution was quenched by adding water (30 mL), and the mixture was extracted with ethyl acetate (20 mL × 3 times). Organic phases were combined and

then washed with saturated saline (20 mL × 2 times). Then the resultant was dried with anhydrous sodium sulfate, filtered, and concentrated under reduced pressure. The residue was purified by preparative high performance liquid chromatography. The product was collected and solvent was removed under reduced pressure. 9.8 mg of 2-(4-cyclopropyl-6-methoxypyrimidin-5-yl)-9-(4-(1-isopropyl-4-(trifluoromethyl)-1H-imidazol-2-yl)benzyl)-7-methoxy-9H-pyrimido[4,5-b]indole was obtained.

MS (ESI) M/Z: 614.0 $[M+H]^+$.

$^1H$ NMR (400 MHz, DMSO-$d_6$): δ 9.51 (s, 1H), 8.69 (s, 1H), 8.24 (d, *J*= 8.4 Hz, 1H), 8.14 (s, 1H), 7.49 (brs, 4H), 7.43 (d, *J* = 2.0 Hz, 1H), 7.04 (dd, *J* =8.8, 2.4 Hz, 1H), 5.78 (s, 2H), 4.45 - 4.32 (m, 1H), 3.90 (s, 3H), 3.86 (s, 3H), 1.74 - 1.66 (m, 1H), 1.35 (d, *J* = 6.4 Hz, 6H), 1.07 - 1.00 (m, 2H), 0.85 - 0.78 (m, 2H).

**Example 8: 2-(4-cyclopropyl-6-methoxypyrimidin-5-yl)-9-(4-(1-isopropyl-4-(trifluoromethyl)-1H-imidazo l-2-yl) benzyl)-7-(trifluoroethyl)-9H-pyrimido[4,5-b]indole**

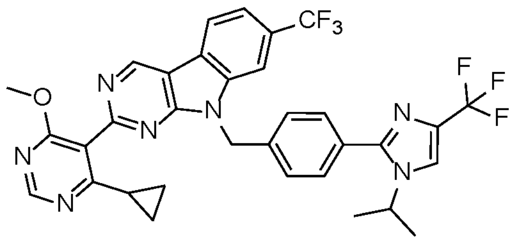

**Reaction route:**

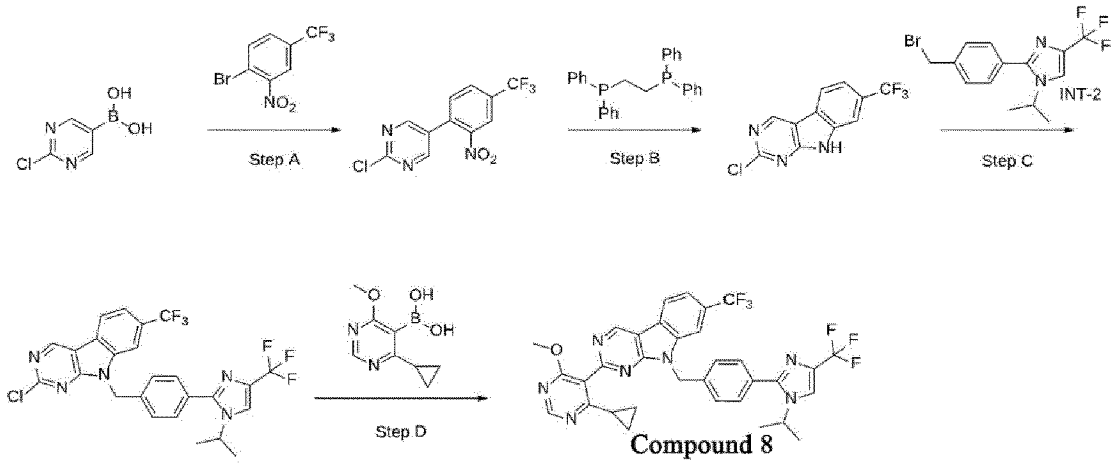

Compound 8

**Operation steps:**

**Step A:** At room temperature, 1-bromo-2-nitro-4-(trifluoromethyl)benzene (1 g, 3.70 mmol), (2-chloropyrimidin-5-yl)boronic acid (879 mg, 5.60 mmol), sodium carbonate (1.2 g, 11.1 mmol) and [1,1'-bis(diphenylphosphino) ferrocene]palladium dichloride dichloromethane complex (406 mg, 0.56 mmol) were dissolved in 1,4-dioxane/water (18 mL/2 mL). The reaction system was evacuated to remove air and purged with nitrogen for 3 times, and then stirred at 90°C for 16 hours.

After the disappearance of raw materials as monitored by LCMS, the reaction solution was quenched by adding ice water (20 mL), and the mixture was extracted with ethyl acetate (20 mL × 3 times). Organic phases were combined and then washed with saturated saline (15 mL × 2 times). Then the resultant was dried with anhydrous sodium sulfate, filtered, and concentrated under reduced pressure. The residue was purified by silica gel column chromatography to obtain 620 mg of 2-chloro-5-(4-trifluoromethyl-2-nitrophenyl)pyrimidine.

MS (ESI) M/Z: 304.0 $[M+H]^+$.

**Step B:** At room temperature, 2-chloro-5-(4-trifluoromethyl-2-nitrophenyl)pyrimidine (620 mg, 2.04 mmol) and 1,2-bis(diphenylphosphino)ethane (1.1 g, 2.55 mmol) were dissolved in 1,2-dichlorobenzene (9.5 mL). Then the reaction system was stirred at 160°C for 2 hours.

After the disappearance of raw materials as monitored by LCMS, the reaction solution was quenched by adding ice water (30 mL), and the mixture was extracted with ethyl acetate (20 mL × 3 times). Organic phases were combined and then washed with saturated saline (20 mL × 2 times). Then the resultant was dried with anhydrous sodium sulfate, filtered, and concentrated under reduced pressure. The residue was purified by silica gel column chromatography to obtain 220 mg of 2-chloro-7-(trifluoromethyl)-9H-pyrimido[4,5-b]indole.

MS (ESI) M/Z: 272.0 $[M+H]^+$.

**Step C:** At room temperature and under nitrogen protection, 2-chloro-7-(trifluoromethyl)-9H-pyrimido[4,5-b]indole (220 mg, 0.81 mmol) and anhydrous potassium carbonate (223.6 mg, 1.62 mmol) were dissolved in N,N-dimethylformamide (5 mL). Then 2-(4-(bromomethyl)phenyl)-1-isopropyl-4-(trifluoromethyl)-1H-imidazole (337 mg, 0.97 mmol) was added to the above solution. Then the reaction system was stirred at 55°C for 2 hours.

After the disappearance of raw materials as monitored by LCMS, the reaction solution was quenched by adding ice water (10 mL), and the mixture was extracted with ethyl acetate (30 mL × 3 times). Organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 450 mg of 2-chloro-9-(4-(1-isopropyl-4-(trifluoromethyl)-1H-imidazol-2-yl)benzyl)-7-(trifluoroethyl)-9H-pyr imido [4,5-b]indole.

MS (ESI) M/Z: 538.0 $[M+H]^+$.

**Step D:** At room temperature, 2-chloro-9-(4-(1-isopropyl-4-(trifluoromethyl)-1H-imidazol-2-yl)benzyl)-7-(trifluoroethyl)-9H-pyr imido[4,5-b]indole (270 mg, 0.50 mmol), (4-cyclopropyl-6-methoxypyrimidin-5-yl)boronic acid (294 mg, 1.51 mmol), cesium carbonate (327 mg, 1.0 mmol) and chloro(2-dicyclohexylphosphino-2',4',6'-triisopropyl-1,1'-biphenyl)[2-(2'-amino-1,1'-biphenyl)]p alladium(II) (60 mg, 0.075 mmol) were dissolved in 1,4-dioxane/water (2.5 mL/0.25 mL). The reaction system was evacuated to remove air and purged with nitrogen for 3 times, and then stirred at 90°C under microwave heating for 2.5 hours.

After the disappearance of raw materials as monitored by LCMS, the reaction solution was quenched by adding ice water (30 mL). The mixed solution was extracted with ethyl acetate (30 mL × 3 times), and organic phases were combined and then washed with saturated saline (60 mL × 2 times). Then the resultant was dried with anhydrous sodium sulfate, filtered, and concentrated under reduced pressure. The residue was purified by preparative high performance liquid chromatography. The product was collected and solvent was removed. 27.13 mg of 2-(4-cyclopropyl-6-methoxypyrimidin-5-yl)-9-(4-(1-isopropyl-4-(trifluoromethyl)-1H-imidazol-2-yl)benzyl)-7-(trifluoroethyl)-9H-pyrimido[4,5-b]indole was obtained.

MS (ESI) M/Z: 652.0 $[M+H]^+$.

$^1H$ NMR (400 MHz, DMSO) δ 9.82 (s, 1H), 8.72 (s, 1H), 8.62 (d, *J* = 8.0 Hz, 1H), 8.35 (s, 1H), 8.15 (d, *J* = 1.2 Hz, 1H), 7.80 (d, *J* = 8.0 Hz, 1H), 7.52 - 7.46 (m, 4H), 5.93 (s, 2H), 4.42 - 4.32 (m, 1H), 3.87 (s, 3H), 1.76 - 1.68 (m, 1H), 1.35 (d, *J* = 6.8 Hz, 6H), 1.09 - 1.03 (m, 2H), 0.86 - 0.79 (m, 2H).

**Example 9: 2-(4-cyclopropyl-6-methoxypyrimidin-5-yl)-9-(4-(1-ethyl-4-(trifluoromethyl)-1H-imidazol-2-yl)benzyl)-9H-pyrimido[4,5-b]indole**

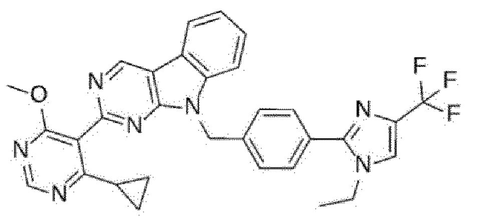

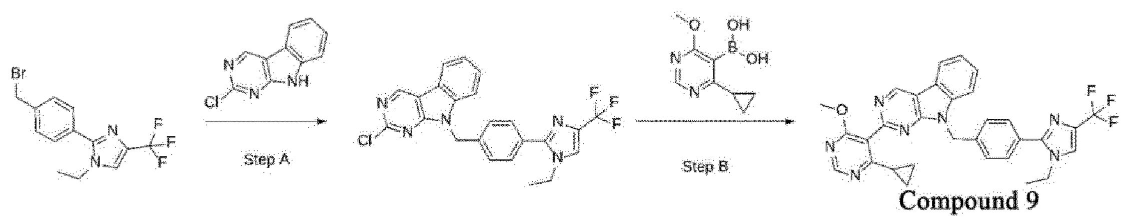


Compound 9

**Step A:** At room temperature and under nitrogen protection, 2-(4-(bromomethyl)phenyl)-1-ethyl-4-(trifluoromethyl)-1H-imidazole (200 mg, 0.60 mmol), 2-chloro-9H-pyrimido[4,5-b]indole (245 mg, 1.20 mmol) and potassium carbonate (182 mg, 1.20 mmol) were dissolved in N,N-dimethylformamide (4 mL). Then the reaction system was heated to

50°C and stirred for 2 hours.

After the disappearance of raw materials as monitored by LCMS, the reaction solution was quenched by adding water (30 mL). The mixed solution was extracted with ethyl acetate (10 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 130 mg of 2-chloro-9-(4-(1-ethyl-4-(trifluoromethyl)-1H-imidazol-2-yl)benzyl)-9H-pyrimido[4,5-b]indole.

MS (ESI) M/Z: 455.8 $[M+H]^+$.

**Step B:** At room temperature, 2-chloro-9-(4-(1-ethyl-4-(trifluoromethyl)-1H-imidazol-2-yl)benzyl)-9H-pyrimido [4,5-b]indole (110 mg, 0.24 mmol), (4-cyclopropyl-6-methoxypyrimidin-5-yl)boronic acid (94 mg, 0.48 mmol), cesium carbonate (158 mg, 0.48 mmol) and chloro(2-dicyclohexylphosphino-2',4',6'-triisopropyl-1,1'-biphenyl)[2-(2'-amino-1,1'-biphenyl)]p alladium(II) (38 mg, 0.05 mmol) were dissolved in 1,4-dioxane/water (1.1 mL/0.12 mL). The reaction system was evacuated to remove air and purged with nitrogen for 3 times, and then stirred at 90°C under microwave heating for 3 hours.

After the disappearance of raw materials as monitored by LCMS, the reaction solution was quenched by adding ice water (30 mL). The mixed solution was extracted with ethyl acetate (20 mL × 3 times), and organic phases were combined and then washed with saturated saline (30 mL × 2 times). Then the resultant was dried with anhydrous sodium sulfate, filtered, and concentrated under reduced pressure. The residue was purified by preparative high performance liquid chromatography. The product was collected and solvent was removed. 55.00 mg of 2-(4-cyclopropyl-6-methoxypyrimidin-5-yl)-9-(4-(1-ethyl-4-(trifluoromethyl)-1H-imidazol-2-yl)b enzyl)-9H-pyrimido[4,5-b]indole was obtained.

MS (ESI) M/Z: 570.1 $[M+H]^+$.

$^1H$ NMR (400 MHz, DMSO-$d_6$): δ 9.66 (s, 1H), 8.71 (s, 1H), 8.37 (d, *J* = 7.6 Hz, 1H), 7.99 (d, *J* = 1.2 Hz, 1H), 7.86 (d, *J* = 8.0 Hz, 1H), 7.63 (t, *J* = 7.6 Hz, 1H), 7.56 (d, *J* = 8.4 Hz, 2H), 7.48 (d, *J* = 8.4 Hz, 2H), 7.44 (t, *J* = 8.0 Hz, 1H), 5.80 (s, 2H), 4.01 (q, *J* = 7.2 Hz, 2H), 3.87 (s, 3H), 1.77 - 1.68 (m, 1H) ,1.26 (t, *J* = 7.2 Hz, 3H), 1.10 - 1.02 (m, 2H), 0.89 - 0.80 (m, 2H).

**Example 10: 2-(4-cyclopropyl-6-methoxypyrimidin-5-yl)-9-(4-(5-ethoxy-3-(trifluoromethyl)-1H-pyrazol-1-yl)benzyl)-9H-pyrimido[4,5-b]indole**

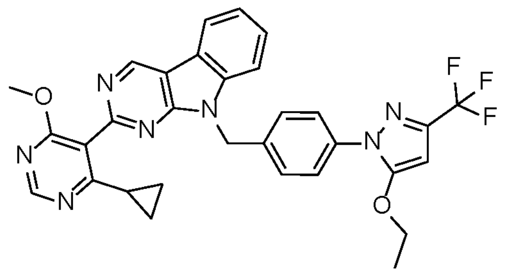

**Reaction route:**

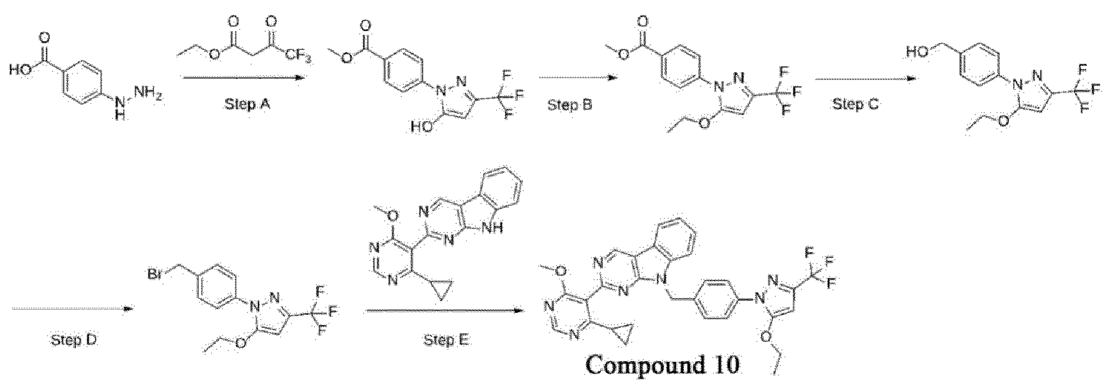


**Operation steps:**

**Step A:** At room temperature, 4-hydrazinylbenzoic acid (5 g, 32.9 mmol) and ethyl 4,4,4-trifluoro-3-oxobutanoate (6 g, 32.9 mmol) were dissolved in methanol/hydrochloric acid (73 mL/14.6 mL). Then the reaction system was stirred at room temperature for 3 hours.

After the disappearance of raw materials as monitored by LCMS, the reaction solution was concentrated under reduced pressure. The resulting residue was purified by silica gel column chromatography to obtain 8.3 g of methyl 4-(5-hydroxy-3-(trifluoromethyl)-1H-pyrazol-1-yl)benzoate.

MS (ESI) M/Z: 287.0 $[M+H]^+$.

**Step B:** At room temperature and under nitrogen protection, methyl 4-(5-hydroxy-3-(trifluoromethyl)-1H-pyrazol-1-yl)benzoate (5 g, 17.5 mmol) was dissolved in N,N-dimethylformamide (87 mL). Subsequently, sodium hydride (1.39 g, 34.96 mmol) was added to the above solution, and the resultant was stirred for 30 minutes. Then iodoethane (5.4 g, 34.96 mmol) was slowly added dropwise, and the reaction system was stirred at room temperature for 1 hour.

After the disappearance of raw materials as monitored by LCMS, the reaction solution was quenched by adding ice water (250 mL). The mixed solution was extracted with ethyl acetate (80 mL × 3 times), and organic phases were combined and then washed with saturated saline (250 mL × 2 times). Then the resultant was dried with anhydrous sodium sulfate, filtered, and concentrated under reduced pressure. The resulting residue was purified by silica gel column chromatography to obtain 3.7 g of methyl 4-(5-ethoxy-3-(trifluoromethyl)-1H-pyrazol-1-yl)benzoate.

MS (ESI) M/Z: 315.0 $[M+H]^+$.

**Step C:** At 0°C and under nitrogen protection, methyl 4-(5-ethoxy-3-(trifluoromethyl)-1H-pyrazol-1-yl)benzoate (3.7 g, 11.78 mmol) was dissolved in tetrahydrofuran (59 mL). Subsequently, lithium aluminum hydride (9.4 mL, 23.56 mmol) was added to the above solution, and the reaction system was continued to be stirred for 30 minutes.

After the disappearance of raw materials as monitored by LCMS, the reaction solution was quenched by slowly adding to ice water (150 mL) dropwise, and the mixture was filtrated. Then the filtrate was extracted with ethyl acetate (50 mL × 3 times). Organic phases were combined and then washed with saturated saline (150 mL × 2 times). Then the resultant was dried with anhydrous sodium sulfate, filtered, and concentrated under reduced pressure. The resulting residue was purified by silica gel column chromatography to obtain 3 g of (4-(5-ethoxy-3-(trifluoromethyl)-1H-pyrazol-1-yl) phenyl)methanol.

MS (ESI) M/Z: 287.0 $[M+H]^+$.

**Step D:** At 0°C and under nitrogen protection, (4-(5-ethoxy-3-(trifluoromethyl)-1H-pyrazol-1-yl)phenyl)methanol (1.5 g, 5.24 mmol) was dissolved in dichloromethane (26 mL). Then, triphenylphosphine (2.7 g, 10.48 mmol), sodium bicarbonate (880 mg, 10.48 mmol) and carbon tetrabromide (3.46 g, 10.48 mmol) were added sequentially to the above solution. Then the reaction system was continued to be stirred at room temperature for 30 minutes.

After the disappearance of raw materials as monitored by LCMS, the reaction solution was quenched by adding ice water (150 mL). The mixed solution was extracted with dichloromethane (50 mL × 3 times), and organic phases were combined and then washed with saturated saline (150 mL × 2 times). Then the resultant was dried with anhydrous sodium sulfate, filtered, and concentrated under reduced pressure. The resulting residue was purified by silica gel column chromatography to obtain 1.8 g of 1-(4-(bromomethyl)phenyl)-5-ethoxy-3-(trifluoromethyl)-1H-pyrazole.

MS (ESI) M/Z: 348.8 $[M+H]^+$.

**Step E:** At room temperature, 1-(4-(bromomethyl)phenyl)-5-ethoxy-3-(trifluoromethyl)-1H-pyrazole (43.9 mg, 0.13 mmol), 2-(4-cyclopropyl-6-methoxypyrimidin-5-yl)-9H-pyrimido[4,5-b]indole (40 mg, 0.13 mmol) and potassium carbonate (34.7 mg, 0.26 mmol) were dissolved in *N,N*-dimethylformamide (1 mL). The reaction system was evacuated to remove air and purged with nitrogen for 3 times, and then stirred at 50°C for 2 hours.

After the disappearance of raw materials as monitored by LCMS, the reaction solution was quenched by adding water (40 mL). The mixed solution was extracted with ethyl acetate (10 mL × 3 times), and organic phases were combined and then washed with saturated saline (25 mL × 2 times). Then the resultant was dried with anhydrous sodium sulfate, filtered, and concentrated under reduced pressure. The residue was purified by preparative high performance liquid chromatography. The product was collected and solvent was removed. 17.29 mg of 2-(4-cyclopropyl-6-methoxypyrimidin-5-yl)-9-(4-(5-ethoxy-3-(trifluoromethyl)-1H-pyrazol-1-yl)b enzyl)-9H-pyrimido[4,5-b]indole was obtained.

MS (ESI) M/Z: 586.2 $[M+H]^+$.

$^1H$ NMR (400 MHz, DMSO-$d_6$): δ 9.65 (s, 1H), 8.70 (s, 1H), 8.37 (d, *J* = 8.0 Hz, 1H), 7.85 (d, *J* = 8.0 Hz, 1H), 7.65 - 7.58 (m, 3H), 7.49 - 7.42 (m, 3H), 6.42 (s, 1H), 5.78 (s, 2H), 4.25 (q, *J* = 6.8 Hz, 2H), 3.87 (s, 3H), 1.77 - 1.65 (m, 1H), 1.30 (t, *J* = 6.8 Hz, 3H), 1.10 - 1.00 (m, 2H), 0.89 - 0.76 (m, 2H).

**Example 11: 2-(4-cyclopropyl-6-methoxypyrimidin-5-yl)-9-(4-(1-isopropyl-4-(trifluoromethyl)-1H-imidazo l-2-yl) benzyl)-4-methyl-9H-pyrimido[4,5-b]indole**

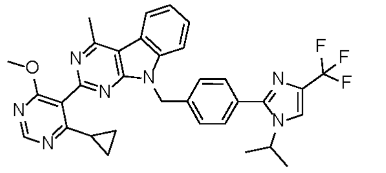

**Reaction route:**

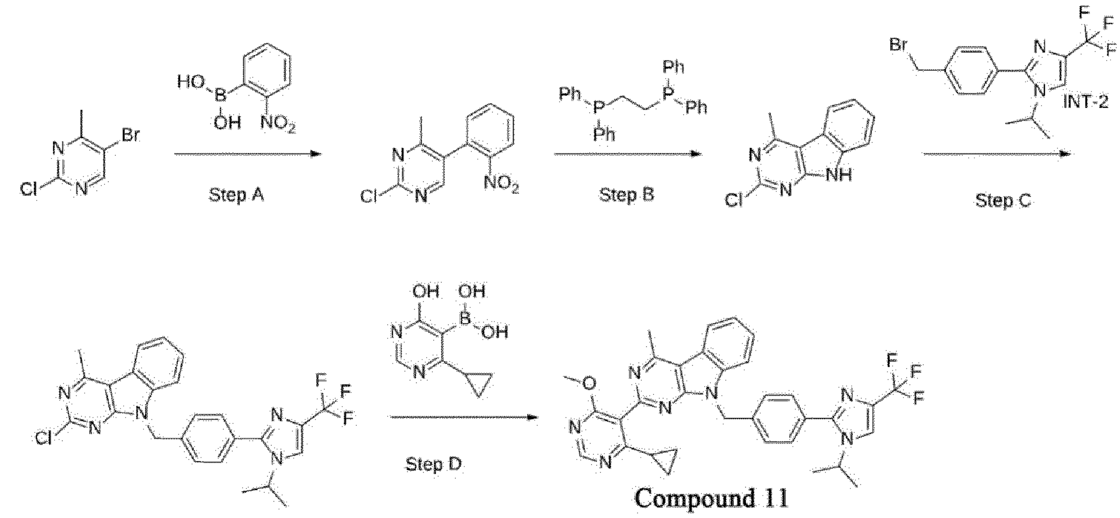


Compound 11

**Operation steps:**

**Step A:** At room temperature, (2-nitrophenyl)boronic acid (1.03 g, 6.15 mmol), 5-bromo-2-chloro-4-methylpyrimidine (850 mg, 4.10 mmol), sodium carbonate (1.3 g, 12.3 mmol), and [1,1'-bis(diphenylphosphino)ferrocene]palladium dichloride dichloromethane complex (450 mg, 0.61 mmol) were dissolved in 1,4-dioxane/water (21 mL/2.3 mL). The reaction system was evacuated to remove air and purged with nitrogen for 3 times, and then stirred at 90°C for 16 hours.

After the disappearance of raw materials as monitored by LCMS, the reaction solution was quenched by adding ice water (40 mL). The mixed solution was extracted with ethyl acetate (20 mL × 3 times), and organic phases were combined and then washed with saturated saline (15 mL × 2 times). Then the resultant was dried with anhydrous sodium sulfate, filtered, and concentrated under reduced pressure. The residue was purified by silica gel column chromatography to obtain 600 mg of 2-chloro-4-methyl-5-(2-nitrophenyl)pyrimidine.

MS (ESI) M/Z: 250.0 $[M+H]^+$.

**Step B:** At room temperature, 2-chloro-4-methyl-5-(2-nitrophenyl)pyrimidine (600 mg, 2.41 mmol) and 1,2-bis(diphenylphosphino)ethane (1198.8 mg, 3.01 mmol) were dissolved in 1,2-dichlorobenzene (12 mL). Then the reaction system was stirred at 160°C for 2 hours.

After the disappearance of raw materials as monitored by LCMS, the reaction solution was quenched by adding ice water (40 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure. The residue was purified by silica gel column chromatography to obtain 320 mg of 2-chloro-4-methyl-9H-pyrimido[4,5-b]indole.

MS (ESI) M/Z: 218.0 $[M+H]^+$.

**Step C:** At room temperature and under nitrogen protection, 2-chloro-4-methyl-9H-pyrimido[4,5-b]indole (150 mg, 0.69 mmol) and anhydrous potassium carbonate (190.2 mg, 1.38 mmol) were dissolved in N,N-dimethylformamide (5 mL). Then 2-(4-(bromomethyl)phenyl)-1-isopropyl-4-(trifluoromethyl)-1H-imidazole (286.7 mg, 0.83 mmol) was added to the above solution. Then the reaction system was stirred at 55°C for 2 hours.

After the disappearance of raw materials as monitored by LCMS, the reaction solution was quenched by adding ice water (30 mL). The mixed solution was extracted with ethyl acetate (10 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 220 mg of 2-chloro-9-(4-(1-isopropyl-4-(trifluoromethyl)-1H-imidazol-2-yl)benzyl)-4-methyl-9H-pyrimido[4 ,5-b]indole.

MS (ESI) M/Z: 484.0 $[M+H]^+$.

**Step D:** At room temperature, 2-chloro-9-(4-(1-isopropyl-4-(trifluoromethyl)-1H-imidazol-2-yl)benzyl)-4-methyl-9H-pyrimido[4 ,5-b]indole (180 mg, 0.37 mmol), (4-cyclopropyl-6-methoxypyrimidin-5-yl)boronic acid (145 mg, 0.75 mmol), cesium carbonate (243 mg, 0.75 mmol) and chloro(2-dicyclohexylphosphino-2',4',6'-triisopropyl-1,1'-biphenyl)[2-(2'-amino-1,1'-biphenyl)]p alladium(II) (59 mg, 0.08 mmol) were dissolved in 1,4-dioxane/water (1.7 mL/0.20 mL). The reaction system was evacuated to remove air and purged with nitrogen for 3 times, and then stirred at 90°C under microwave heating for 2.5 hours.

After the disappearance of raw materials as monitored by LCMS, the reaction solution was quenched by adding ice water (30 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure. The residue was purified by preparative high performance liquid chromatography. The product was collected and solvent was removed. 88.80 mg of 2-(4-cyclopropyl-6-methoxypyrimidin-5-yl)-9-(4-(1-isopropyl-4-(trifluoromethyl)-1H-imidazol-2-yl)benzyl)-4-methyl-9H-pyrimido[4,5-b]indole was obtained.

MS (ESI) M/Z: 598.2 $[M+H]^+$.

$^1$H NMR (400 MHz, DMSO) δ 8.70 (s, 1H), 8.27 (d, *J* = 7.6 Hz, 1H), 8.14 (d, *J* = 0.8 Hz, 1H), 7.88 (d, *J* = 8.0 Hz, 1H), 7.63 (t, *J* = 7.6 Hz, 1H), 7.52 - 7.41 (m, 5H), 5.81 (s, 2H), 4.43 - 4.30 (m, 1H), 3.87 (s, 3H), 3.03 (s, 3H), 1.74 - 1.63 (m, 1H), 1.35 (d, *J* = 6.8 Hz, 6H), 1.09 - 1.00 (m, 2H), 0.90 - 0.77 (m, 2H).

**Example 12: (S)-2-(4-cyclopropyl-6-methoxypyrimidin-5-yl)-9-(1-(4-(1-isopropyl-4-(trifluoromethyl)-1H-i midazol-2-yl)benzyl)ethyl)-9H-pyrimido[4,5-b]indole and (R)-2-(4-cyclopropyl-6-methoxypyrimidin-5-yl)-9-(1-(4-(1-isopropyl-4-(trifluoromethyl)-1H-i midazol-2-yl)benzyl)ethyl)-9H-pyrimido[4,5-b]indole**

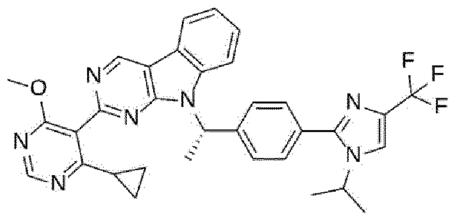

Compound 13-P1 or Compound 13-P2

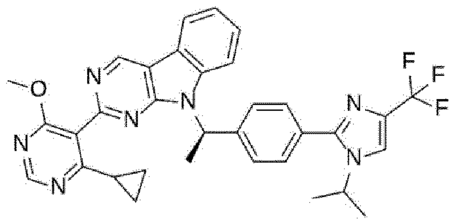

Compound 13-P2 or Compound 13-P1

The compounds were prepared with reference to the preparation method of Example 8 and obtained by chiral splitting (chiral conditions were omitted in the synthesis report). The products were separated by chiral supercritical fluid chromatography (SFC) under the following conditions: chiral column Daicel IG-3 (25×250 mm, 10 μm); mobile phase: carbon dioxide/methanol [0.2% ammonia (7 M ammonia in methanol)], and 27.65 mg of compound 13-P1 (peak time: 1.77 min) and 32.27 mg of compound 13-P2 (peak time: 3.27 min) were obtained.

**Compound 13-P1:**

MS (ESI) M/Z: 598.1 $[M+H]^+$.

$^1$H NMR (400 MHz, DMSO-$d_6$): δ 9.65 (s, 1H), 8.70 (s, 1H), 8.37 (d, *J* = 8.0 Hz, 1H), 8.15 (d, *J* = 0.8 Hz, 1H), 7.68 (d, *J* = 8.4 Hz, 1H), 7.60 - 7.47 (m, 5H), 7.41 (t, *J* = 7.4 Hz, 1H), 6.55 (q, *J* = 7.2 Hz, 1H), 4.47 - 4.31 (m, 1H), 3.87 (s, 3H), 2.15 (d, *J*= 7.2 Hz, 3H), 1.82 - 1.67 (m, 1H), 1.36 (dd, *J* = 6.6, 1.8 Hz, 6H), 1.11 - 1.01 (m, 2H), 0.90 - 0.77 (m, 2H).

**Compound 13-P2:**

MS (ESI) M/Z: 598.2 [M+H]$^+$.

$^1$H NMR (400 MHz, DMSO-$d_6$): δ 9.65 (s, 1H), 8.70 (s, 1H), 8.37 (d, *J* = 7.6 Hz, 1H), 8.15 (d, *J* = 0.8 Hz, 1H), 7.68 (d, *J* = 8.4 Hz, 1H), 7.58-7.48 (m, 5H), 7.41 (t, *J* = 7.6 Hz, 1H), 6.55 (q, *J* = 7.2 Hz, 1H), 4.46 - 4.35 (m, 1H), 3.87 (s, 3H), 2.15 (d, *J* = 7.2 Hz, 3H), 1.80 - 1.69 (m, 1H), 1.36 (dd, *J* = 6.6, 1.7 Hz, 6H), 1.09 - 1.01 (m, 2H), 0.92 - 0.79 (m, 2H).

**Example 13: 2-(4-cyclopropyl-6-methoxypyrimidin-5-yl)-9-(4-(1-isopropyl-4-(trifluoromethyl)-1H-imidazo l-2-yl) benzyl)-9H-imidazo[2,1-f]purine**

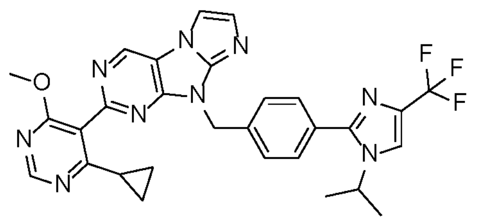

**Reaction route:**

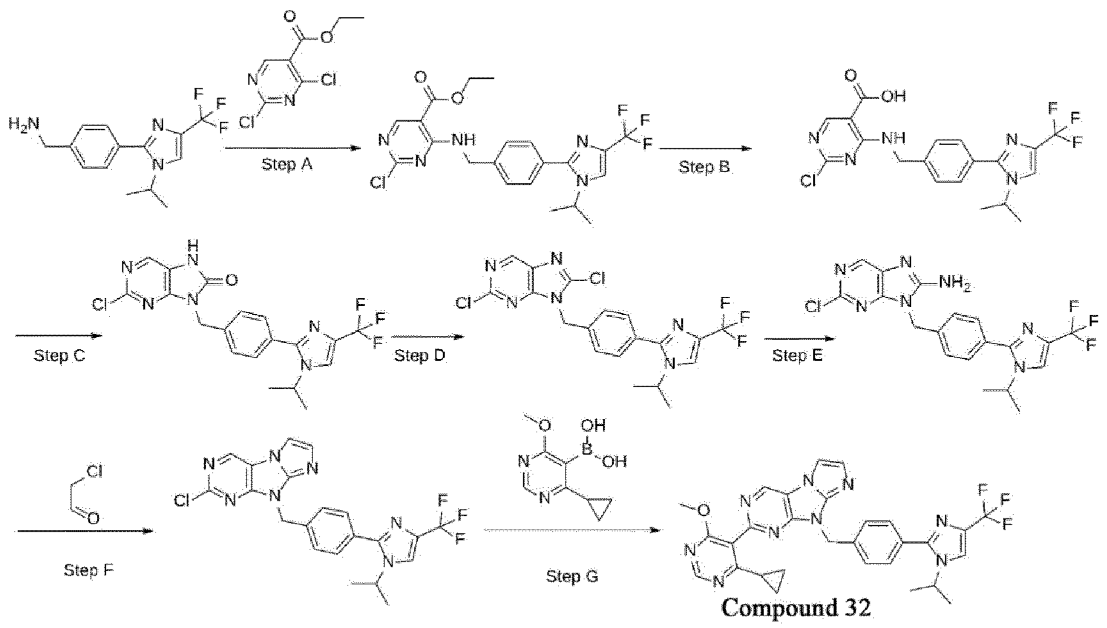

Compound 32

**Operation steps:**

**Step A:** At 0°C, (4-(1-isopropyl-4-(trifluoromethyl)-1H-imidazol-2-yl)phenyl)methylamine (2.25 g, 7.96 mmol) and ethyl 2,4-dichloropyrimidin-5-carboxylate (1.76 g, 7.96 mmol) were dissolved in acetonitrile (40 mL). Subsequently, N,N-diisopropylethylamine (267 mg, 2.07 mmol) was slowly added to the above solution dropwise. Then the reaction system was continued to be stirred for 1 hour.

After the disappearance of raw materials as monitored by LCMS, the reaction solution was quenched by adding to ice water (100 mL). The mixed solution was extracted with ethyl acetate (40 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 2.4 g of ethyl 2-chloro-4-((4-(1-isopropyl-4-(trifluoromethyl)-1H-imidazol-2-yl)benzyl)amino) pyrimidin-5-carb oxylate.

MS (ESI) M/Z: 468.0 [M+H]$^+$.

$^1$H NMR (400 MHz, DMSO-$d_6$) δ 9.05 (t, *J* = 6.2 Hz, 1H), 8.67 (s, 1H), 8.17 (d, *J* = 1.2 Hz, 1H), 7.54 (d, *J* = 8.0 Hz,

2H), 7.49 (d, *J* = 8.4 Hz, 2H), 4.78 (d, *J* = 6.4 Hz, 2H), 4.53 - 4.42 (m, 1H), 4.34 (q, *J* = 7.2 Hz, 2H), 1.40 (d, *J* = 6.8 Hz, 6H), 1.33 (t, *J* = 7.2 Hz, 3H).

**Step B:** At 0°C, ethyl 2-chloro-4-((4-(1-isopropyl-4-(trifluoromethyl)-1H-imidazol-2-yl)benzyl)amino)pyrimidin-5-carb oxylate (2.4 g, 4.9 mmol) and lithium hydroxide (413 mg, 9.8 mmol) were dissolved in tetrahydrofuran/water (12.5 mL/12.5 mL). Then the reaction system was stirred at room temperature for 2 hours.

After the disappearance of raw materials as monitored by LCMS, the reaction solution was concentrated under reduced pressure, and the resulting residue was quenched by adding to ice water (30 mL). The mixture was adjusted to pH 3-4 with 3 M dilute hydrochloric acid at 0°C and filtered. The filter cake was rinsed with water (20 mL × 3 times), and then collected and concentrated under reduced pressure to obtain 2.2 g of 2-chloro-4-((4-(1-isopropyl-4-(trifluoromethyl)-1H-imidazol-2-yl)benzyl)amino)pyrimidin-5-carb oxylic acid.

MS (ESI) M/Z: 440.0 $[M+H]^+$.

**Step C:** At room temperature, 2-chloro-4-((4-(1-isopropyl-4-(trifluoromethyl)-1H-imidazol-2-yl)benzyl)amino) pyrimidin-5-carb oxylic acid (2.2 g, 5.01 mmol), diphenyl azidophosphate (1.38 g, 5.01 mmol) and triethylamine (506 mg, 5.01 mmol) were dissolved in N,N-dimethylformamide (25 mL). Then the reaction system was stirred at 115°C for 16 hours.

After the disappearance of raw materials as monitored by LCMS, the reaction solution was concentrated under reduced pressure. The resulting residue was purified by silica gel column chromatography to obtain 1.4 g of 2-chloro-9-(4-(1-isopropyl-4-(trifluoromethyl)-1H-imidazol-2-yl)benzyl)-7,9-dihydro-8H-purin-8-one.

MS (ESI) M/Z: 437.0 $[M+H]^+$.

**Step D:** At 0°C and under nitrogen protection, 2-chloro-9-(4-(1-isopropyl-4-(trifluoromethyl)-1H-imidazol-2-yl) benzyl)-7,9-dihydro-8H-purin-8-one (2.2 g, 5.05 mmol) and triethylamine (1 g, 10.09 mmol) were dissolved in phosphorus oxychloride (25 mL). Then the reaction system was stirred at 140°C for 24 hours.

After the disappearance of raw materials as monitored by LCMS, the reaction solution was concentrated under reduced pressure. The residue was quenched by adding ice water (100 mL). The mixed solution was extracted with ethyl acetate (40 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 70 mg of 2,8-dichloro-9-(4-(1-isopropyl-4-(trifluoromethyl)-1H-imidazol-2-yl)benzyl)-9H-purine.

MS (ESI) M/Z: 455.0 $[M+H]^+$.

**Step E:** At room temperature and under nitrogen protection, 2,8-dichloro-9-(4-(1-isopropyl-4-(trifluoromethyl)-1H-imidazol-2-yl)benzyl)-9H-purine (70 mg, *0.15* mmol) was dissolved in ammonia in methanol (2 mL, 7 M). Then the reaction system was stirred at 80°C for 2 hours.

After the disappearance of raw materials as monitored by LCMS, the reaction solution was concentrated under reduced pressure. The resulting residue was purified by silica gel column chromatography to obtain 50 mg of 2-chloro-9-(4-(1-isopropyl-4-(trifluoromethyl)-1H-imidazol-2-yl)benzyl)-9H-purin-8-amine.

MS (ESI) M/Z: 436.0 $[M+H]^+$.

**Step F:** At room temperature and under nitrogen protection, 2-chloro-9-(4-(1-isopropyl-4-(trifluoromethyl)-1H-imidazol-2-yl)benzyl)-9H-purin-8-amine (25 mg, 0.06 mmol) and 2-chloroacetaldehyde (71 mg, 0.36 mmol) were dissolved in N,N-dimethylformamide (2 mL). Then the reaction system was stirred at 100°C for 18 hours.

After the disappearance of raw materials as monitored by LCMS, the reaction solution was quenched by adding ice water (50 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure. The resulting residue was purified by silica gel column chromatography to obtain 8 mg of 2-chloro-9-(4-(1-isopropyl-4-(trifluoromethyl)-1H-imidazol-2-yl)benzyl)-9H-imidazo [2,1-f]purine

MS (ESI) M/Z: 460.0 $[M+H]^+$.

**Step G:** At room temperature, 2-chloro-9-(4-(1-isopropyl-4-(trifluoromethyl)-1H-imidazol-2-yl)benzyl)-9H-imidazo[2,1-f]purine (8 mg, 0.02 mmol) was dissolved in 1,4-dioxane/water (0.5 mL/0.05 mL). Subsequently, (4-cyclopropyl-6-methoxypyrimidin-5-yl)boronic acid (8 mg, 0.04 mmol), cesium carbonate (7 mg, 0.02 mmol), and chloro(2-dicyclohexylphosphino-2',4',6'-triisopropyl-1,1'-biphenyl)[2-(2'-amino-1,1'-biphenyl)]p alladium(II) (3 mg, 0.004 mmol) were added successively to the above reaction solution. The reaction system was evacuated to remove air and purged with nitrogen for 4 times, and then stirred at 90°C under microwave heating for 2 hours.

After the disappearance of raw materials as monitored by LCMS, the reaction solution was quenched by adding ice water (50 mL). The mixed solution was extracted with ethyl acetate (20 mL × 3 times), and organic phases were combined and then washed with saturated saline (20 mL × 2 times). Then the resultant was dried with anhydrous sodium sulfate, filtered, and concentrated under reduced pressure. The residue was purified by preparative high performance liquid chromatography. The product was collected to obtain 2-(4-cyclopropyl-6-methoxypyrimidin-5-yl)-9-(4-(1-isopropyl-4-(trifluoromethyl)-1H-imidazol-2-yl)benzyl)-9H-imidazo[2,1-f]purine.

MS (ESI) M/Z: 574.2 $[M+H]^+$.

$^1$H NMR (400 MHz, DMSO-$d_6$): δ 9.24 (s, 1H), 8.70 (s, 1H), 8.16 (d, $J$= 1.4 Hz, 1H), 7.91 (d, $J$ = 1.7 Hz, 1H), 7.58 (d, $J$ = 8.4 Hz, 2H), 7.53 (d, $J$ = 8.3 Hz, 2H), 7.20 (d, $J$ = 1.6 Hz, 1H), 5.52 (s, 2H), 4.48 - 4.35 (m, 1H), 3.86 (s, 3H), 1.79 - 1.67 (m, 1H), 1.37 (d, $J$ = 6.6 Hz, 7H), 1.10 - 1.01 (m, 2H), 0.89 - 0.79 (m, 2H).

**Example 14: 6-(4-cyclopropyl-6-methoxypyrimidin-5-yl)-4-(4-(1-isopropyl-4-(trifluoromethyl)-1H-imidazo l-2-yl) benzyl)-4H-isothiazolo[5',4':4,5]pyrrolo[2,3-d]pyrimidine**

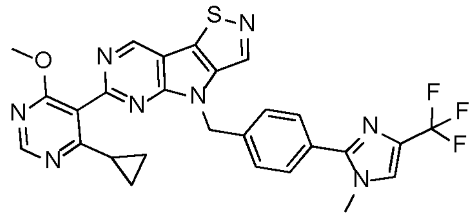

**Reaction route:**

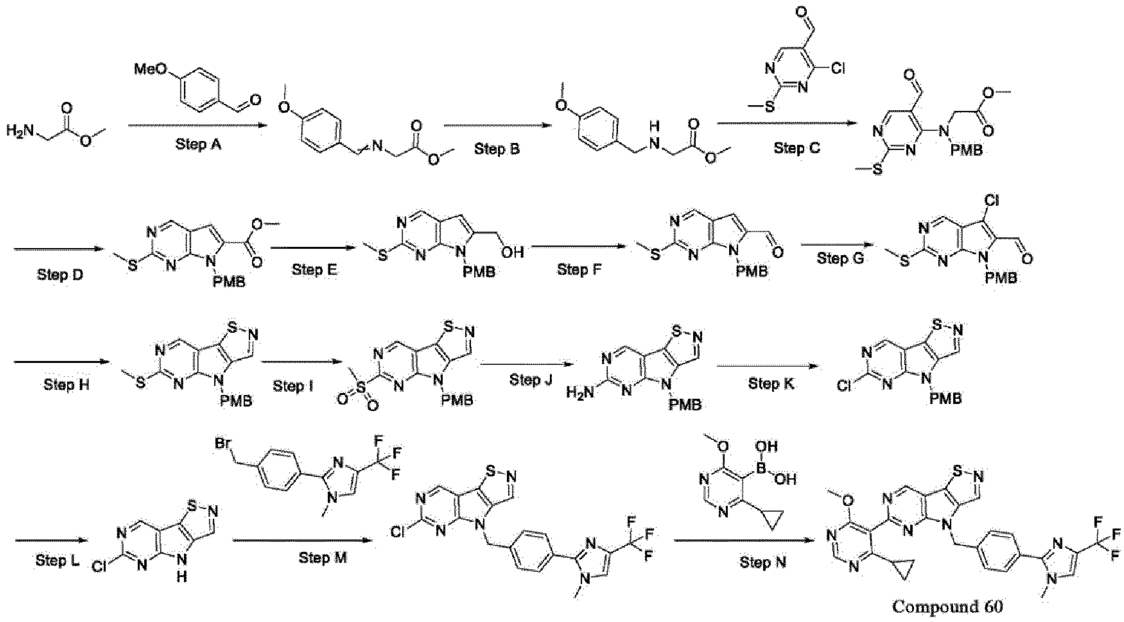


Compound 60

**Operation steps:**

**Step A:** 4-Methoxybenzaldehyde (13 g, 95.59 mmol) and triethylamine (21 g, 210.3 mmol) were dissolved in dichloromethane (200 mL) at room temperature. Subsequently, the reaction solution was cooled to 0°C, and methyl glycinate (17 g, 191.2 mmol) and anhydrous sodium sulfate (50 g, 352.1 mmol) were added to the above reaction solution. The reaction system was then stirred at room temperature for 16 hours.

After the disappearance of raw materials as monitored by LCMS, the solution was filtered and concentrated under reduced pressure. 20 g of methyl 2-((4-methoxybenzylidene)amino)acetate was obtained.

MS (ESI) M/Z: 208.1 [M+H]$^+$.

**Step B:** Methyl 2-((4-methoxybenzylidene)amino)acetate (18 g, 86.96 mmol) was dissolved in methanol (200 mL) at 0°C. Subsequently, sodium borohydride (4.96 g, 130.44 mmol) was slowly added. Then the reaction system was stirred at room temperature for 2 hours.

After the disappearance of raw materials as monitored by LCMS, ice water (500 mL) was added to the reaction solution for quenching. The mixed solution was extracted with ethyl acetate (200 mL × 3 times), and the organic phases were combined and washed with saturated saline (100 mL × 2 times). Then the resultant was dried with anhydrous sodium sulfate, filtered and concentrated under reduced pressure. The residue was purified by silica gel column chromatography to obtain 15 g of methyl (4-methoxybenzyl)glycinate.

MS (ESI) M/Z: 210.1 $[M+H]^+$.

**Step C:** Methyl (4-methoxybenzyl)glycinate (15 g, 71.77 mmol), 4-chloro-2-(methylthio)pyrimidin-5-formaldehyde (13.4 g, 71.77 mmol) and triethylamine (8.7 g, 86.12 mmol) were dissolved in tetrahydrofuran (360 mL) at room temperature. The reaction system was then stirred at room temperature for 16 hours.

After the disappearance of raw materials as monitored by LCMS, ice water (300 mL) was added to the reaction solution for quenching. The mixed solution was extracted with ethyl acetate (100 mL × 3 times), and the organic phases were combined and washed with saturated saline (50 mL × 2 times). Then the resultant was dried with anhydrous sodium sulfate, filtered and concentrated under reduced pressure. The residue was purified by silica gel column chromatography to obtain 22 g of methyl N-(5-formyl-2-(methylthio)pyrimidin-4-yl)-N-(4-methoxybenzyl)glycinate.

MS (ESI) M/Z: 362.0 $[M+H]^+$.

**Step D:** Methyl N-(5-formyl-2-(methylthio)pyrimidin-4-yl)-N-(4-methoxybenzyl)glycinate (16.5 g, 45.71 mmol) was dissolved in dry toluene (160 mL) under nitrogen protection at room temperature. Subsequently, sodium hydride (7.31 g, 182.83 mmol) was slowly added to the above solution under the condition of ice-water bath. The reaction system was then stirred at 70°C for 2 hours.

After the disappearance of raw materials as monitored by LCMS, ice water (500 mL) was added to the reaction solution for quenching. The mixed solution was extracted with ethyl acetate (100 mL × 3 times), and the organic phases were combined and washed with saturated saline (100 mL × 2 times). The resultant was dried with anhydrous sodium sulfate, filtered and concentrated under reduced pressure. The residue was purified by silica gel column chromatography to obtain 10 g of methyl 7-(4-methoxybenzyl)-2-(methylthio)-7H-pyrrolo[2,3-d]pyrimidin-6-carboxylate.

MS (ESI) M/Z: 344.0 $[M+H]^+$.

**Step E:** Methyl 7-(4-methoxybenzyl)-2-(methylthio)-7H-pyrrolo[2,3-d]pyrimidin-6-carboxylate (10 g, 29.15 mmol) was dissolved in dry dichloromethane (147 mL) at room temperature. Subsequently, at -78°C, lithium aluminum hydride (1 moL/L) (59.30 ml, 59.30 mmol) was added to the above reaction solution. Then the reaction system reacted at -78°C for 30 minutes.

After the disappearance of raw materials as monitored by LCMS, the reaction solution was added to ice water (500 mL) for quenching. The mixed solution was extracted with ethyl acetate (100 mL × 3 times), and the organic phases were combined and washed with saturated saline (100 mL × 2 times). The resultant was dried with anhydrous sodium sulfate, filtered and concentrated under reduced pressure. The residue was purified by silica gel column chromatography to obtain 10 g of (7-(4-methoxybenzyl)-2-(methylthio)-7H-pyrrolo[2,3-d]pyrimidin-6-yl)methanol.

MS (ESI) M/Z: 316.0 $[M+H]^+$.

**Step F:** (7-(4-Methoxybenzyl)-2-(methylthio)-7H-pyrrolo[2,3-d]pyrimidin-6-yl)methanol (10 g, 31.75 mmol) and manganese dioxide (13.65 g, 158.75 mmol) were dissolved in dichloromethane (635 mL) at room temperature. The reaction system was evacuated to remove air and purged with nitrogen for 3 times, and the reaction system was stirred at room temperature for 2 hours.

After the disappearance of raw materials as monitored by LCMS, the mixture was filtered and concentrated under reduced pressure. The residue was purified by silica gel column chromatography to obtain 8 g of 7-(4-methoxybenzyl)-2-(methylthio)-7H-pyrrolo[2,3-d]pyrimidin-6-formaldehyde.

MS (ESI) M/Z: 314.0 $[M+H]^+$.

**Step G:** 7-(4-Methoxybenzyl)-2-(methylthio)-7H-pyrrolo[2,3-d]pyrimidin-6-formaldehyde (8 g, 25.56 mmol) was dissolved in N,N-dimethylformamide (128 mL) at room temperature. Subsequently, N-chlorosuccinimide (5.12 g, 38.34 mmol) was slowly added to the above solution. The reaction system was then stirred at 50°C for 2 hours.

After the disappearance of raw materials as monitored by LCMS, ice water (500 mL) was added to the reaction solution for quenching. The mixed solution was extracted with ethyl acetate (100 mL × 3 times), and the organic phases were combined and washed with saturated aqueous salt solution (100 mL × 2 times). The resultant was dried with anhydrous sodium sulfate, filtered and concentrated under reduced pressure. The residue was purified by silica gel column chromatography to obtain 3.5 g of 5-chloro-7-(4-methoxybenzyl)-2-(methylthio)-7H-pyrrolo[2,3-d]pyrimidin-6-formaldehyde.

MS (ESI) M/Z: 348.0 $[M+H]^+$.

**Step H:** 5-Chloro-7-(4-methoxybenzyl)-2-(methylthio)-7H-pyrrolo[2,3-d]pyrimidin-6-formaldehyde (3.5 g, 10.09 mmol) and sulfur powder (65 mg, 2.02 mmol) were dissolved in ammonia/methanol solution (4 mol/L, 34 mL) at room temperature. The reaction system was then stirred at 80°C for 6 hours.

After the disappearance of raw materials as monitored by LCMS, ice water (40 mL) was added to the reaction solution for quenching. The mixed solution was extracted with ethyl acetate (60 mL × 3 times), and the organic phases were combined and washed with saturated saline (100 mL × 2 times). The resultant was dried with anhydrous sodium sulfate, filtered and concentrated under reduced pressure. The residue was purified by silica gel column chromatography to obtain 2.9 g of 4-(4-methoxybenzyl)-6-(methylthio)-4H-isothiazolo[5',4':4,5]pyrrolo[2,3-d]pyrimidine.

MS (ESI) M/Z: 343.0 $[M+H]^+$.

**Step I:** 4-(4-Methoxybenzyl)-6-(methylthio)-4H-isothiazolo[5',4':4,5]pyrrolo[2,3-d]pyrimidine (2.9 g, 8.48 mmol)

was dissolved in dry dichloromethane solution (43 mL) at room temperature. Subsequently, m-chloroperoxybenzoic acid (3.22 g, 18.66 mmol) was slowly added to the above reaction system at -78°C. The reaction system was then stirred at room temperature for 4 hours.

After the disappearance of raw materials as monitored by LCMS, ice water (100 mL) was added to the reaction solution for quenching. The mixed solution was extracted with dichloromethane (60 mL × 3 times), and the organic phases were combined and then washed with saturated saline (100 mL × 2 times). The resultant was dried with anhydrous sodium sulfate, filtered and concentrated under reduced pressure. The residue was purified by silica gel column chromatography to obtain 1.8 g of 4-(4-methoxybenzyl)-6-(methylsulfonyl)-4H-isothiazolo[5',4':4,5]pyrrolo[2,3-d]pyrimidine.

MS (ESI) M/Z: 375.0 $[M+H]^+$.

**Step J:** 4-(4-Methoxybenzyl)-6-(methylsulfonyl)-4H-isothiazolo[5',4':4,5]pyrrolo][2,3-d]pyrimidine (1.8 g, 4.81 mmol) was dissolved in dry 1,4-dioxane solution (16 mL) at room temperature. Subsequently, an ammonia/methanol solution (4 mol/L, 48 mL) was added to the above solution. The reaction system was then stirred at 80°C for 4 hours.

After the disappearance of raw materials as monitored by LCMS, the mixture was concentrated under reduced pressure. The residue was purified by silica gel column chromatography to obtain 840 mg of 4-(4-methoxybenzyl)-4H-isothiazolo[5',4':4,5]pyrrolo[2,3-d]pyrimidin-6-amine.

MS (ESI) M/Z: 312.0 $[M+H]^+$.

**Step K:** 4-(4-Methoxybenzyl)-4H-isothiazolo[5',4':4,5]pyrrolo[2,3-d]pyrimidin-6-amine (840 mg, 2.70 mmol) was dissolved in dry dichloromethane solution (68 mL) at room temperature. Subsequently, trimethylchlorosilane (910 mg, 8.37 mmol) and tert-butyl nitrite (1.37 g, 13.23 mmol) were successively added to the above solution under the condition of an ice-water bath. The reaction system was then stirred at room temperature for 16 hours.

After the disappearance of raw materials as monitored by LCMS, ice water (40 mL) was added to the reaction solution for quenching. The mixed solution was extracted with dichloromethane (60 mL × 3 times), the organic phases were combined and then washed with saturated saline (100 mL × 2 times). The resultant was dried with anhydrous sodium sulfate, filtered and concentrated under reduced pressure. The residue was purified by silica gel column chromatography to obtain 390 mg of 6-chloro-4-(4-methoxybenzyl)-4H-isothiazolo[5',4':4,5]pyrrolo[2,3-d]pyrimidine.

MS (ESI) M/Z: 331.0 $[M+H]^+$.

**Step L:** 6-Chloro-4-(4-methoxybenzyl)-4H-isothiazolo[5',4':4,5]pyrrolo[2,3-d]pyrimidine (390 mg, 1.18 mmol) was dissolved in trifluoromethanesulfonic acid (8 mL) at room temperature. The reaction system was then stirred at 65°C for 2 hours.

After the disappearance of raw materials as monitored by LCMS, ice water (10 mL) was added to the reaction solution for quenching. The mixed solution was extracted with ethyl acetate (20 mL × 3 times), and the organic phases were combined and washed with saturated saline (60 mL × 2 times). The resultant was dried with anhydrous sodium sulfate, filtered and concentrated under reduced pressure. The residue was purified by silica gel column chromatography to obtain 200 mg of crude 6-chloro-4H-isothiazolo[5',4':4,5]pyrrolo[2,3-d]pyrimidine.

MS (ESI) M/Z: 211.0 $[M+H]^+$.

**Step M:** 6-Chloro-4H-isothiazolo[5',4':4,5]pyrrolo[2,3-d]pyrimidine (200 mg, 0.95 mmol), 2-(4-(bromomethyl) phenyl)-1-methyl-4-(trifluoromethyl)-1H-imidazole (303 mg, 0.95 mmol) and potassium carbonate (263 mg, 1.90 mmol) were dissolved in N,N-dimethylformamide (5 mL) under nitrogen protection at room temperature. The reaction system was then stirred at 50°C for 2 hours.

After the disappearance of raw materials as monitored by LCMS, ice water (50 mL) was added to the reaction solution for quenching. The mixed solution was extracted with ethyl acetate (20 mL × 3 times), and the organic phases were combined and washed with saturated saline (60 mL × 2 times). The resultant was dried with anhydrous sodium sulfate, filtered and concentrated under reduced pressure. The residue was purified by silica gel column chromatography to obtain 170 mg of 6-chloro-4-(4-(1-methyl-4-(trifluoromethyl)-1H-imidazol-2-yl)benzyl)-4H-isothiazolo [5',4':4,5]py rrolo [2,3-d]pyrimidine.

MS (ESI) M/Z: 449.0 $[M+H]^+$.

**Step N:** 6-Chloro-4-(4-(1-methyl-4-(trifluoromethyl)-1H-imidazol-2-yl)benzyl)-4H-isothiazolo[5',4':4,5]py rrolo [2,3-d]pyrimidine (170 mg, 0.38 mmol), (4-cyclopropyl-6-methoxypyrimidin-5-yl)boric acid (110 mg, 0.57 mmol), cesium carbonate (186 mg, 0.57 mmol) and chloro(2-dicyclohexylphosphino-2',4',6'-triisopropyl-1,1'-biphenyl)[2-(2'-amino-1,1'-biphenyl)]pall adium(II) (60 mg, 0.08 mmol) were dissolved in 1,4-dioxane/water (2.7 mL/0.3 mL) at room temperature. The reaction system was evacuated to remove air and purged with nitrogen for 3 times, and the reaction system was reacted for 2 hours under the microwave condition of 100°C.

After the disappearance of raw materials as monitored by LCMS, ice water (50 mL) was added to the reaction solution for quenching. The mixed solution was extracted with ethyl acetate (30 mL × 3 times), and the organic phases were combined. The combined organic phase was washed with saturated saline (60 mL × 2 times). The resultant was dried with anhydrous sodium sulfate, filtered and concentrated under reduced pressure. The residue was purified by preparative HPLC. The product was collected to obtain 17.95 mg of 6-(4-cyclopropyl-6-methoxypyrimidin-5-yl)-4-(4-(1-isopropyl-4-(trifluoromethyl)-1H-imidazol-2-yl)benzyl)-4H-isothiazolo[5',4':4,5]pyrrolo[2,3-d]pyrimidine.

MS (ESI) M/Z: 563.6 $[M+H]^+$.

$^1H$ NMR (400 MHz, DMSO-$d_6$): δ 9.73 (s, 1H), 9.08 (s, 1H), 8.71 (s, 1H), 7.91 (d, *J* = 1.2 Hz, 1H), 7.67 (d, *J* = 8.4 Hz, 2H), 7.54 (d, *J* = 8.4 Hz, 2H), 5.82 (s, 2H), 3.88 (s, 3H), 3.73 (s, 3H), 1.77-1.68 (m, 1H), 1.10-1.04 (m, 2H), 0.90-0.83 (m, 2H).

**Example 15: 2-(4-cyclopropyl-6-methoxypyrimidin-5-yl)-9-((2-(trifluoromethyl)-6,7-dihydro-5H-benzo[c]i midazo[1,2-a]azepin-9-yl)methyl)-9H-pyrimido[4,5-b]indol-7-nitrile**

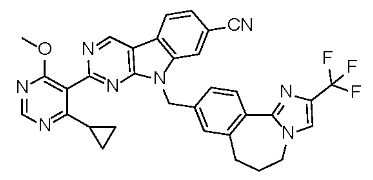

**Reaction route:**

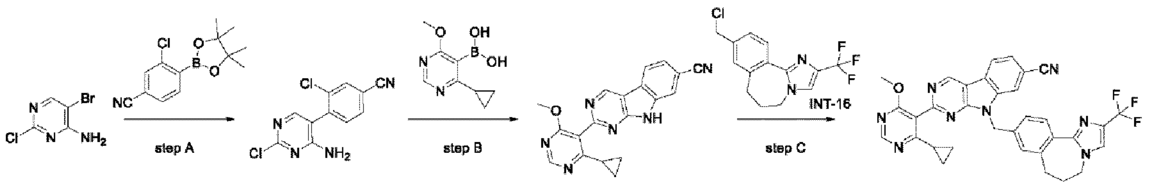


Compound 58

**Operation steps:**

**Step A:** 5-Bromo-2-chloropyrimidin-4-amine (108 g, 518 mmol) was dissolved in 1,4-dioxane/water (2119 mL/235 mL) under nitrogen protection at room temperature. Subsequently, 3-chloro-4-(4,4,5,5-tetramethyl-1,3,2-dioxaborolan-2-yl)benzonitrile (150 g, 570 mmol) and sodium carbonate (110 g, 1036 mmol) and [1,1'-bis(diphenylphosphino) ferrocene]palladium dichloride dichloromethane complex (38 g, 46.6 mmol) were sequentially added to the above solution. The reaction system was then stirred at 90°C for 16 hours.

After the disappearance of raw materials as monitored by LCMS, the reaction solution was filtered, the filtrate was collected and concentrated under reduced pressure. The residue was pulped with ethyl acetate/petroleum ether, and the filter cake was pulped again with acetonitrile/water to obtain 94.6 g of crude 4-(4-amino-2-chloropyrimidin-5-yl)-3-chlorobenzonitrile.

MS (ESI) M/Z: 265.0 $[M+H]^+$.

**Step B:** 4-(4-Amino-2-chloropyrimidin-5-yl)-3-chlorobenzonitrile (94.6 g, 358 mmol) was dissolved in 1,4-dioxane/water (1394 mL/232 mL) under nitrogen protection at room temperature. Subsequently, (4-cyclopropyl-6-methoxypyrimidin-5-yl) boric acid (69.5 g, 358 mmol), cesium carbonate (233 g, 716 mmol) and chloro(2-dicyclohexylphosphino-2',4',6'-triisopropyl-1,1'-biphenyl)[2-(2'-amino-1,1'-biphenyl)]pall adium(II) (36.5 g, 46.5 mmol) were added to the above solution successively. The reaction system was then stirred at 90°C for 16 hours.

After the disappearance of raw materials as monitored by LCMS, the reaction solution was filtered and concentrated under reduced pressure, and the residue was pulped with ethyl acetate/petroleum ether, and the filter cake was rinsed with water and dried to obtain 118 g of crude 2-(4-cyclopropyl-6-methoxypyrimidin-5-yl)-9H-pyrimido[4,5-b]indol-7-nitrile.

MS (ESI) M/Z: 343.2 $[M+H]^+$.

**Step C:** 2-(4-Cyclopropyl-6-methoxypyrimidin-5-yl)-9H-pyrimido[4,5-b]indol-7-nitrile (53.8 g, 157.2 mmol) was dissolved in N,N-dimethylformamide (436 mL) at room temperature. Subsequently, cesium carbonate (114 g, 349.2 mmol) and 9-(chloromethyl)-2-(trifluoromethyl)-6,7-dihydro-5H-benzo[c]imidazo[1,2-a]azapine (26.2 g, 87.3 mmol) were added to the reaction system successively. The reaction system was then stirred at 45°C for 16 hours.

After the disappearance of raw materials as monitored by LCMS, the reaction solution was added to water (900 mL) to precipitate a solid, and the obtained solid was purified by silica gel column chromatography to obtain 20.42 g of 2-(4-cyclopropyl-6-methoxypyrimidin-5-yl)-9-((2-(trifluoromethyl)-6,7-dihydro-5H-benzo[c]imid azo[1,2-a]azepin-9-yl) methyl)-9H-pyrimido[4,5-b]indol-7-nitrile.

MS (ESI) M/Z: 607.2 $[M+H]^+$.

$^1H$ NMR (400 MHz, DMSO-$d_6$) δ 9.82 (s, 1H), 8.72 (s, 1H), 8.59 (d, *J* = 8.4 Hz, 1H), 8.50 (s, 1H), 7.96 (d, *J* = 0.8 Hz,

1H), 7.86 (dd, *J* = 8.0, 1.2 Hz, 1H), 7.62 (d, *J* = 8.0 Hz, 1H), 7.41 (d, *J* = 0.8 Hz, 1H), 7.33 (dd, *J* = 8.0, 1.6 Hz, 1H), 5.81 (s, 2H), 3.95 (t, *J* = 6.8 Hz, 2H), 3.87 (s, 3H), 2.60 (t, *J* = 7.0 Hz, 2H), 2.27-2.15 (m, 2H), 1.77-1.67 (m, 1H), 1.10-1.02 (m, 2H), 0.88-0.77 (m, 2H).

**Example 16: 2-(4-cyclopropyl-6-methoxypyrimidin-5-yl)-9-(5-(1-methyl-4-(trifluoromethyl)-1H-imidazol-2-yl)-2,3-dihydro-1H-inden-1-yl)-9H-pyridino[4',3':4,5]pyrrolo[2,3-d]pyrimidine**

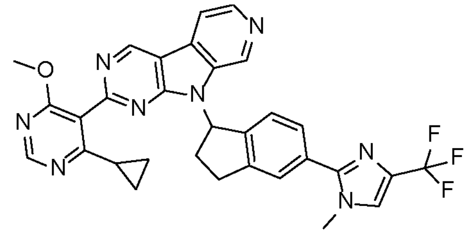

**Reaction route:**

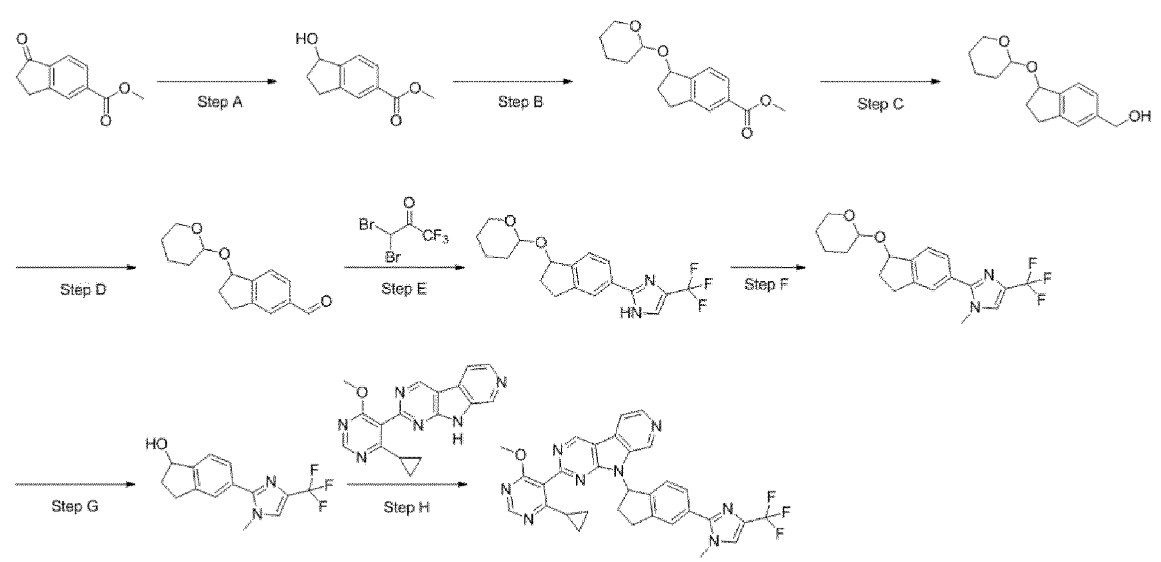


Compound 75

**Operation steps:**

**Step A:** Methyl 1-oxo-2,3-dihydro-1H-indene-5-carboxylate (1 g, 5.26 mmol) was dissolved in methanol (27.5 mL) at room temperature. Subsequently, sodium borohydride (420 mg, 10.53 mmol) was slowly added to the above solution at 0°C. Then, the reaction system was stirred at room temperature for 1 hour.

After the disappearance of raw materials as monitored by LCMS, the reaction solution was slowly added dropwise into ice water (50 mL) for quenching. The resultant was filtered, the filtrate was extracted with ethyl acetate (50 mL × 3 times), and the organic phases were combined and then washed with saturated saline (50 mL × 2 times). The resultant was dried with anhydrous sodium sulfate, filtered and concentrated under reduced pressure. The residue was purified by silica gel column chromatography to obtain 1 g of methyl 1-hydroxy-2,3-dihydro-1H-indene-5-carboxylate.

MS(ESI) M/Z: 193.1 $[M+H]^+$.

**Step B:** Methyl 1-hydroxy-2,3-dihydro-1H-indene-5-carboxylate (710 mg, 3.68 mmol), 3,4-dihydro-2H-pyran (621.25 mg, 7.40 mmol) and pyridinium p-toluenesulfonate (95 mg, 0.37 mmol) were dissolved in dichloromethane (18.50 mL) at room temperature under nitrogen protection. The reaction system was then stirred at 60°C for 1 hour.

After the disappearance of raw materials as monitored by LCMS, the reaction solution was concentrated under reduced pressure. The residue was purified by silica gel column chromatography to obtain 850 mg of methyl 1-((tetrahydro-2H-pyran-2-yl)oxy)-2,3-dihydro-1H-indene-5-carboxylate.

MS (ESI) M/Z: 299.2 $[M+23]^+$.

**Step C:** Methyl 1-((tetrahydro-2H-pyran-2-yl)oxy)-2,3-dihydro-1H-indene-5-carboxylate (850 mg, 2.90 mmol) was dissolved in tetrahydrofuran (15.50 mL) at 0°C under nitrogen protection. Subsequently, lithium aluminum hydride (2.5 mL, 6.18 mmol) was slowly added to the above solution. The reaction system was then stirred at room temperature for 1 hour.

After the disappearance of raw materials as monitored by LCMS, the reaction solution was slowly added dropwise into ice water (100 mL) for quenching. The resultant was filtered, the filtrate was extracted with ethyl acetate (50 mL × 3 times), and the organic phases were combined and then washed with saturated salt aqueous solution (100 mL × 2 times). The resultant was dried with anhydrous sodium sulfate, filtered and concentrated under reduced pressure. The residue was purified by silica gel column chromatography to obtain 690 mg of (1-((tetrahydro-2H-pyran-2-yl)oxy)-2,3-dihydro-1H-inden-5-yl)methanol.

MS (ESI) M/Z: 271.2 $[M+H]^+$.

**Step D:** (1-((Tetrahydro-2H-pyran-2-yl)oxy)-2,3-dihydro-1H-inden-5-yl)methanol (690 mg, 2.78 mmol) and manganese dioxide (2.42 g, 27.82 mmol) were dissolved in dichloromethane (14 mL) under nitrogen protection at room temperature. The reaction system was then stirred at 60°C for 1 hour.

After the disappearance of raw materials as monitored by LCMS, the reaction solution was filtered while it was hot, and the filtrate was concentrated under reduced pressure to obtain 690 mg of 1-((tetrahydro-2H-pyran-2-yl)oxy)-2,3-dihydro-1H-inden-5-formaldehyde.

MS (ESI) M/Z: 247.2 $[M+H]^+$.

**Step E:** 3,3-Dibromo-1,1,1-trifluoropropane-2-one (833.05 mg, 3.09 mmol) and sodium acetate (460 mg, 5.61 mmol) were dissolved in water (14 mL) at room temperature, and the solution was heated to 90°C and stirred for 0.5 hour. Subsequently, the temperature was lowered to 0°C, and a mixed solution of 1-((tetrahydro-2H-pyran-2-yl)oxy)-2,3-dihydro-1H-indene-5-formaldehyde (690 mg, 2.80 mmol) in aqueous ammonia/methanol (49 mL/15 mL) was added to the above solution. The reaction system was then stirred at room temperature for 16 hours.

After the disappearance of raw materials as monitored by LCMS, the reaction solution was slowly added dropwise into ice water (200 mL) for quenching. The mixed solution was extracted with ethyl acetate (100 mL × 3 times), and the organic phases were combined and then washed with saturated saline (100 mL × 2 times). The resultant was dried with anhydrous sodium sulfate, filtered and concentrated under reduced pressure. The residue was purified by silica gel column chromatography to obtain 590 mg of 2-(1-((tetrahydro-2H-pyran-2-yl)oxy)-2,3-dihydro-1H-inden-5-yl)-4-(trifluoromethyl)-1H-imidazo le.

MS (ESI) M/Z: 353.2 $[M+H]^+$.

**Step F:** 2-(1-((Tetrahydro-2H-pyran-2-yl)oxy)-2,3-dihydro-1H-inden-5-yl)-4-(trifluoromethyl)-1H-imidaz ole (590 mg, 1.67 mmol) and potassium carbonate (461.30 mg, 3.34 mmol) were dissolved in N,N-dimethylformamide (8.40 mL) at room temperature. Subsequently, methyl iodide (617.08 mg, 4.35 mmol) was added to the above solution at 0°C. The reaction system was then stirred at room temperature for 4 hours.

After the disappearance of raw materials as monitored by LCMS, the reaction solution was slowly added dropwise into ice water (100 mL) for quenching. The mixed solution was extracted with ethyl acetate (50 mL × 3 times), and the organic phases were combined and then washed with saturated saline (50 mL × 2 times). The resultant was dried with anhydrous sodium sulfate, filtered and concentrated under reduced pressure. The residue was purified by silica gel column chromatography to obtain 400 mg of 1-methyl-2-(1-((tetrahydro-2H-pyran-2-yl)oxy)-2,3-dihydro-1H-inden-5-yl)-4-(trifluoromethyl)-1 H-imidazole.

MS (ESI) M/Z: 367.2 $[M+H]^+$.

**Step G:** 1-Methyl-2-(1-((tetrahydro-2H-pyran-2-yl)oxy)-2,3-dihydro-1H-inden-5-yl)-4-(trifluoromethyl)-1 H-imidazole (400 mg, 1.09 mmol) and p-toluenesulfonic acid (41.53 mg, 0.22 mmol) were dissolved in methanol (6 mL) under nitrogen protection at room temperature, and the solution was heated to 60°C and stirred for 2 hours.

After the disappearance of raw materials as monitored by LCMS, the reaction solution was concentrated under reduced pressure, and the residue was purified by silica gel column chromatography to obtain 180 mg of 5-(1-methyl-4-(trifluoromethyl)-1H-imidazol-2-yl)-2,3-dihydro-1H-inden-1-ol.

MS (ESI) M/Z: 283.1 $[M+H]^+$.

**Step H:** 5-(1-Methyl-4-(trifluoromethyl)-1H-imidazol-2-yl)-2,3-dihydro-1H-inden-1-ol (80 mg, 0.28 mmol), 2-(4-cyclopropyl-6-methoxypyrimidin-5-yl)-9H-pyridino[4',3':4,5]pyrrolo[2,3-d]pyrimidine (90.21 mg, 0.28 mmol) and triphenylphosphine (111.49 mg, 0.43 mmol) were dissolved in anhydrous tetrahydrofuran (1.5 mL) under nitrogen protection at room temperature. Subsequently, diisopropyl azodicarboxylate (85.96 mg, 0.43 mmol) was slowly added dropwise to the above solution at 0°C. The reaction system was then stirred at room temperature for 1 hour.

After the disappearance of raw materials as monitored by LCMS, water (40 mL) was added to the reaction solution for quenching. The mixed solution was extracted with ethyl acetate (10 mL × 3 times), and the organic phases were combined. The combined organic phase was washed with saturated saline (25 mL × 2 times). The resultant was dried with anhydrous sodium sulfate, filtered and concentrated under reduced pressure. The residue was purified by preparative HPLC. 28.22 mg of 2-(4-cyclopropyl-6-methoxypyrimidin-5-yl)-9-(5-(1-methyl-4-(trifluoromethyl)-1H-imidazol-2-yl) -2,3-dihydro-1H-inden-1-yl)-9H-pyridino[4',3':4,5]pyrrolo[2,3-d]pyrimidine was obtained.

MS (ESI) M/Z: 583.3 $[M+H]^+$.

$^1H$ NMR (400 MHz, DMSO-$d_6$): δ 9.82 (s, 1H), 8.71 (s, 1H), 8.58 (d, *J* = 5.2 Hz, 1H), 8.53-8.22 (m, 2H), 7.94 (s, 1H), 7.80 (s, 1H), 7.48 (d, *J* = 8.0 Hz, 1H), 7.03 (d, *J* = 7.6 Hz, 1H), 6.92 (t, *J* = 8.4 Hz, 1H), 3.86 (s, 3H), 3.79 (s, 3H), 3.47-3.36 (m, 1H), 3.27-3.14 (m, 1H), 2.86-2.75 (m, 1H), 2.71-2.57 (m, 1H), 1.80-1.68 (m, 1H), 1.12-1.00 (m, 2H), 0.93-0.80 (m, 2H).

**Example 17: 9-((2-(4-cyclopropyl-6-methoxypyrimidin-5-yl)-9H-pyridino[4',3':4,5]pyrrolo[2,3-d]pyrimidi n-9-yl) methyl)-2-(trifluoromethyl)-5,6-dihydrobenzo[f]imidazo[1,2-d][1,4]oxazepine**

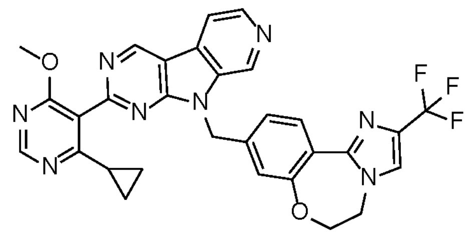

**Reaction route:**

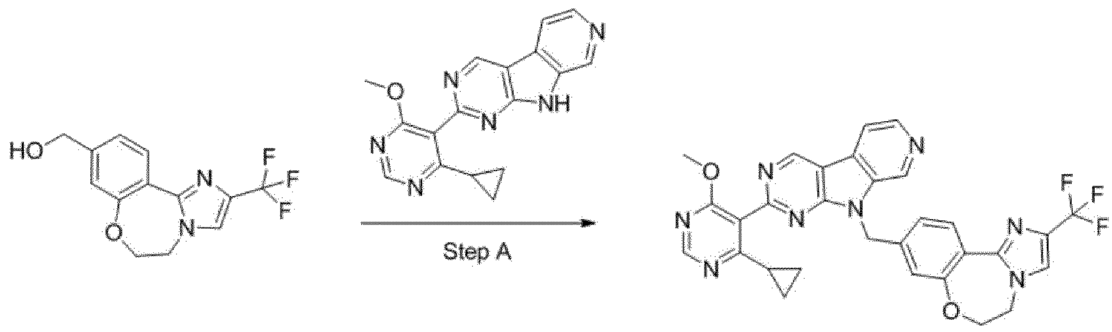


Compound 76

**Operation steps:**

**Step A:** (2-(Trifluoromethyl)-5,6-dihydrobenzo[f]imidazo[1,2-d][1,4]oxazepin-9-yl)methanol (70 mg, 0.25 mmol), 2-(4-cyclopropyl-6-methoxypyrimidin-5-yl)-9H-pyridino[4',3':4,5]pyrrolo[2,3-d]pyrimidine (79 mg, 0.25 mmol) and triphenylphosphine (97 mg, 0.37 mmol) were dissolved in dry tetrahydrofuran (1.5 mL) under nitrogen protection at room temperature. Subsequently, diisopropyl azodicarboxylate (75 mg, 0.37 mmol) was slowly added dropwise to the above solution at 0°C. The reaction system was then stirred at room temperature for 1 hour.

After the disappearance of raw materials as monitored by LCMS, ice water (20 mL) was added to the above reaction solution for quenching, and the mixed solution was extracted with ethyl acetate (30 mL × 3 times), and the organic phases were combined and then washed with saturated saline (20 mL × 2 times). The resultant was dried with anhydrous sodium sulfate, filtered and concentrated under reduced pressure. The residue was purified by preparative HPLC. 48.59 mg of 9-((2-(4-cyclopropyl-6-methoxypyrimidin-5-yl)-9H-pyridino [4',3':4,5]pyrrolo [2,3-d]pyrimidin-9-y l)methyl)-2-(trifluoromethyl)-5,6-dihydrobenzo[f]imidazo[1,2-d][1,4]oxazepine was obtained.

MS (ESI) M/Z: 585.2 $[M+H]^+$.

$^1H$ NMR (400 MHz, DMSO-$d_6$) δ 9.81 (s, 1H), 9.27 (s, 1H), 8.73 (s, 1H), 8.63 (d, *J* = 5.2 Hz, 1H), 8.34 (d, *J* = 5.2 Hz, 1H), 8.26 (d, *J* = 8.0 Hz, 1H), 7.94 (d, *J* = 0.8 Hz, 1H), 7.20 (dd, *J* = 8.4, 1.6 Hz, 1H), 7.04 (d, *J* = 1.6 Hz, 1H), 5.80 (s, 2H), 4.47-4.38 (m, 4H), 3.89 (s, 3H), 1.79-1.70 (m, 1H), 1.13-1.04 (m, 2H), 0.93-0.85 (m, 2H).

**Example** 18: **2-(4-cyclopropyl-6-methoxypyrimidin-5-yl)-9-((2-(trifluoromethyl)-5,6-dihydrobenzo[f]imida zo [1,2-d][1,4]oxazepin-9-yl)methyl)-9H-pyrimido[4,5-b]indol-7-carbonitrile**

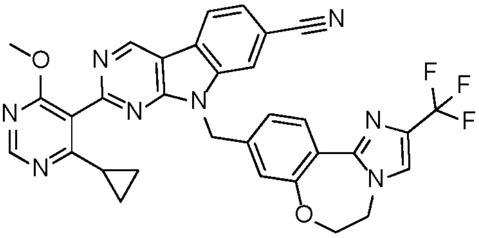

**Reaction route:**

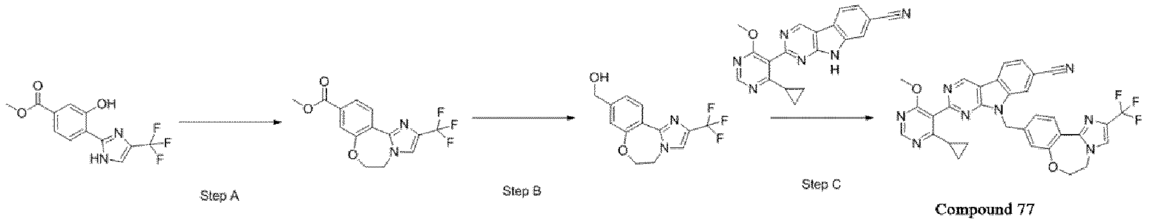

Compound 77

**Operation steps:**

**Step A:** Methyl 3-hydroxy-4-(4-(trifluoromethyl)-1H-imidazol-2-yl)benzoate (1.13 g, 3.95 mmol) and cesium carbonate (3.9 g, 11.85 mmol) were dissolved in acetonitrile (20 mL) at room temperature, and the resultant was stirred for 1 hour. Subsequently, 1,2-dibromoethane (3.7 g, 19.76 mmol) was added to the above reaction system, the temperature was raised to 60°C, and the mixture was stirred for 12 hours.

After the disappearance of raw materials as monitored by LCMS, the reaction solution was filtered, the filter cake was washed with ethyl acetate, and the filtrates were combined and concentrated under reduced pressure. The residue was purified by silica gel column chromatography to obtain 453 mg of methyl 2-(trifluoromethyl)-5,6-dihydrobenzo[f]imidazo[1,2-d][1,4]oxazepin-9-formate.

MS (ESI) M/Z: 313.2 $[M+H]^+$.

**Step B:** Methyl 2-(trifluoromethyl)-5,6-dihydrobenzo[f]imidazo[1,2-d][1,4]oxazepin-9-formate (453 mg, 1.45 mmol) was dissolved in tetrahydrofuran (7.3 mL) at 0°C under nitrogen protection. Subsequently, lithium aluminum hydride (1.2 mL, 2.9 mmol) was slowly added dropwise to the above solution, the temperature was raised to room temperature, and stirring was continued for 30 minutes.

After the disappearance of raw materials as monitored by LCMS, ice water (50 mL) was added to the reaction solution for quenching, and the filter cake was washed with ethyl acetate (20 mL× 2 times). The filtrate was collected and layered, and the water phase was extracted with ethyl acetate (30 mL × 3 times). Organic phases were combined and then washed with saturated saline (40 mL × 2 times). The resultant was dried with anhydrous sodium sulfate, filtered and concentrated under reduced pressure. The residue was purified by silica gel column chromatography to obtain 200 mg of (2-(trifluoromethyl)-5,6-dihydrobenzo[f]imidazo[1,2-d][1,4]oxazepin-9-yl)methanol.

MS (ESI) M/Z: 285.3 $[M+H]^+$.

**Step C:** (2-(Trifluoromethyl)-5,6-dihydrobenzo[f]imidazo[1,2-d][1,4]oxazepin-9-yl)methanol (50 mg, 0.18 mmol), triphenylphosphine (92 mg, 0.35 mmol) and 2-(4-cyclopropyl-6-methoxypyrimidin-5-yl)-9H-pyrimido[4,5-b]indol-7-carbonitrile (90 mg, 0.26 mmol) were dissolved in dry tetrahydrofuran (4.9 mL) at 0°C under nitrogen protection. Subsequently, diisopropyl azodicarboxylate (71 mg, 0.35 mmol) was slowly added dropwise to the above solution. The reaction system was then stirred at room temperature for 1 hour.

After the disappearance of raw materials as monitored by LCMS, ice water (50 mL) was added to the reaction solution for quenching. The mixed solution was extracted with ethyl acetate (30 mL × 3 times), the organic phases were combined, filtered and concentrated under reduced pressure. The residue was purified by preparative HPLC. 38.75 mg of 2-(4-cyclopropyl-6-methoxypyrimidin-5-yl)-9-((2-(trifluoromethyl)-5,6-dihydrobenzo[f]imidazo[1 ,2-d][1,4]oxazepin-9-yl)methyl)-9H-pyrimido[4,5-b]indol-7-carbonitrile was obtained.

MS (ESI) M/Z: 609.2 $[M+H]^+$.

$^1H$ NMR (400 MHz, DMSO-$d_6$) δ 9.81 (s, 1H), 8.72 (s, 1H), 8.57 (d, $J$ = 8.0 Hz, 1H), 8.52 (s, 1H), 8.25 (d, $J$ = 8.4 Hz, 1H), 7.94 (s, 1H), 7.85 (d, $J$ = 8.0 Hz, 1H), 7.20 (dd, $J$ = 8.4, 1.6 Hz, 1H), 7.03 (s, 1H), 5.76 (s, 2H), 4.42 (s, 4H), 3.88 (s, 3H), 1.79-1.70 (m, 1H), 1.12-1.04 (m, 2H), 0.92-0.82 (m, 2H).

**Example 19: 2-(4-cyclopropyl-6-methoxypyrimidin-5-yl)-9-(4-(1-(methyl-d3)-4-(trifluoromethyl)-1H-imid azol-2-yl)benzyl)-9H-pyridino[4',3':4,5]pyrrolo[2,3-d]pyrimidine**

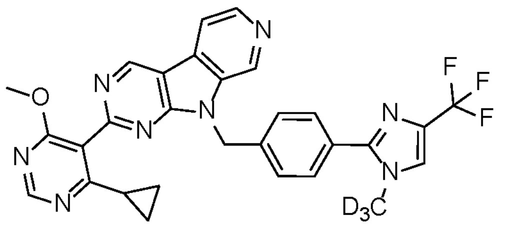

**Reaction route:**

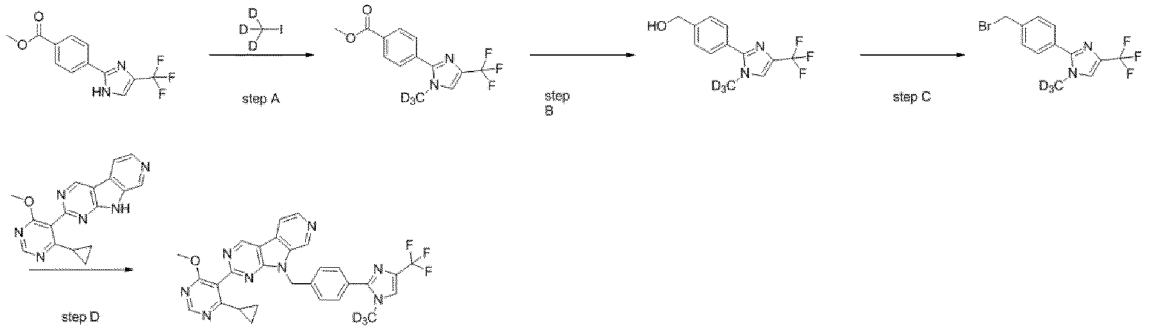


Compound 78

**Operation steps:**

**Step A:** Methyl 4-(4-(trifluoromethyl)-1H-imidazol-2-yl)benzoate (2 g, 7.4 mmol) was dissolved in N,N-dimethylformamide (37 mL) at room temperature. Subsequently, the above solution was cooled to 0°C, and potassium carbonate (2.05 g, 14.8 mmol) and methyl iodide-$D_3$ (1.29 g, 8.89 mmol) were added successively. Then, the reaction system was stirred at room temperature for 1 hour.

After the disappearance of raw materials as monitored by LCMS, water (200 mL) was added to the reaction solution for quenching. The mixed solution was extracted with ethyl acetate (80 mL × 3 times), and the organic phases were combined and then washed with saturated saline (80 mL × 2 times). The organic phases were combined and the residue was purified by silica gel column chromatography to obtain 1.6 g of methyl 4-(1-(methyl-d3)-4-(trifluoromethyl)-1H-imidazol-2-yl)benzoate.

MS (ESI) M/Z: 288.3 $[M+H]^+$.

**Step B:** Methyl 4-(1-(methyl-d3)-4-(trifluoromethyl)-1H-imidazol-2-yl)benzoate (1.6 g, 5.57 mmol) was dissolved in tetrahydrofuran (28 mL) at 0°C under nitrogen protection. Subsequently, 2 M lithium aluminum hydride solution (4.8 mL) was slowly added dropwise to the above solution. Then the reaction system was stirred for 30 minutes at room temperature.

After the disappearance of raw materials as monitored by LCMS, water (100 mL) was added to the reaction solution for quenching. The mixture was filtered, and the residue was rinsed with ethyl acetate (70 mL × 2 times). The filtrate was collected and layered. The aqueous phase was extracted with ethyl acetate (60 mL × 3 times), and all organic phases were combined and then washed with saturated saline (50 mL × 2 times). The resultant was dried with anhydrous sodium sulfate, filtered and concentrated under reduced pressure. The residue was purified by silica gel column chromatography to obtain 1.34 g of (4-(1-(methyl-d3)-4-(trifluoromethyl)-1H-imidazol-2-yl)phenyl)methanol.

MS (ESI) M/Z: 260.2 $[M+H]^+$.

**Step C:** (4-(1-(Methyl-d3)-4-(trifluoromethyl)-1H-imidazol-2-yl)phenyl)methanol (300 mg, 1.16 mmol) was dissolved in dichloromethane (5.8 mL) at room temperature. Subsequently, the above solution was cooled to 0°C, and triphenylphosphine (607 mg, 2.32 mmol), sodium bicarbonate (187 mg, 1.16 mmol) and carbon tetrabromide (767 mg, 2.32 mmol) were added successively. The reaction system was then stirred at room temperature for 2 hours.

After the disappearance of raw materials as monitored by LCMS, water (30 mL) was added to the reaction solution for quenching. The mixed solution was extracted with dichloromethane (20 mL × 3 times), and the organic phases

were combined. The combined organic phase was washed with saturated saline (30 mL × 2 times). The resultant was dried with anhydrous sodium sulfate, filtered and concentrated under reduced pressure. The residue was purified by silica gel column chromatography to obtain 210 mg of 2-(4-(bromomethyl)phenyl)-1-(methyl-d3)-4-(trifluoromethyl)-1H-imidazole.

MS (ESI) M/Z: 322.0 $[M+H]^+$.

**Step D:** 2-(4-Cyclopropyl-6-methoxypyrimidin-5-yl)-9H-pyridino[4',3':4,5]pyrrolo[2,3-d]pyrimidine (74 mg, 0.22 mmol) was dissolved in N,N-dimethylformamide (1.2 mL) at 0°C under nitrogen protection. Subsequently, sodium hydride (37 mg, 0.88 mmol) was added to the above solution, and the resultant was stirred for 20 minutes. Then, 2-(4-(bromomethyl)phenyl)-1-(methyl-d3)-4-(trifluoromethyl)-1H-imidazole (70 mg, 0.22 mmol) was added, and the mixture was raised to room temperature and stirred for 30 minutes.

After the disappearance of raw materials as monitored by LCMS, water (30 mL) was added to the reaction solution for quenching. The mixed solution was washed with ethyl acetate (10 mL × 2 times), and the organic phases were combined and then washed with saturated saline (20 mL × 2 times). The resultant was dried with anhydrous sodium sulfate, filtered and concentrated under reduced pressure. The residue was purified by preparative high performance liquid chromatography. 25.35 mg of 2-(4-cyclopropyl-6-methoxypyrimidin-5-yl)-9-(4-(1-(methyl-d3)-4-(trifluoromethyl)-1H-imidazol-2-yl)benzyl)-9H-pyridino[4',3':4,5]pyrrolo[2,3-d]pyrimidine was obtained.

MS (ESI) M/Z: 560.3 $[M+H]^+$.

$^1H$ NMR (400 MHz, DMSO-$d_6$) δ 9.82 (s, 1H), 9.30 (s, 1H), 8.73 (s, 1H), 8.64 (d, $J$ = 5.2 Hz, 1H), 8.35 (dd, $J$ = 5.2, 0.8 Hz, 1H), 7.90 (d, $J$ = 1.2 Hz, 1H), 7.67 (d, $J$ = 8.4 Hz, 2H), 7.53 (d, $J$ = 8.0 Hz, 2H), 5.88 (s, 2H), 3.89 (s, 3H), 1.79-1.70 (m, 1H), 1.12-1.04 (m, 2H), 0.92-0.82 (m, 2H).

**Example 20: 2-(2-isopropylphenyl)-9-(4-(1-methyl-4-(trifluoromethyl)-1H-imidazol-2-yl)benzyl)-9H-pyridi no [4',3':4,5]pyrrolo[2,3-d]pyrimidine**

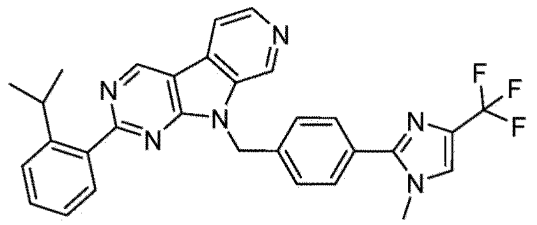

**Reaction route:**

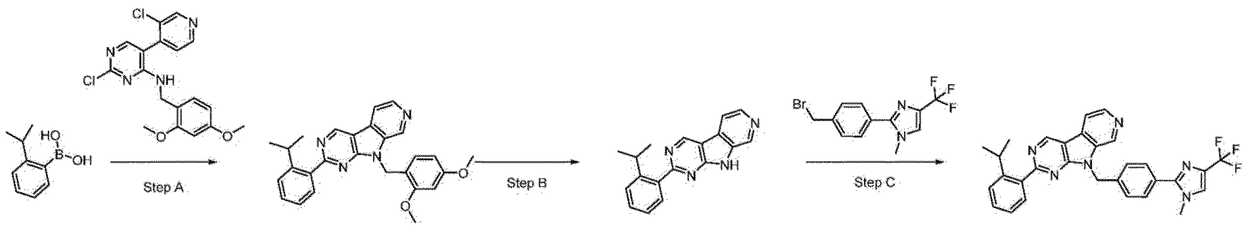


Compound 79

**Operation steps:**

**Step A:** (2-Isopropylphenyl)boric acid (170 mg, 1.04 mmol), 2-chloro-5-(3-chloropyridin-4-yl)-N-(2,4-dimethoxybenzyl)pyrimidin-4-amine (400 mg, 1.04 mmol), cesium carbonate (676 mg, 2.07 mmol) and chloro(2-dicyclohexylphosphino-2',4',6'-triisopropyl-1,1'-biphenyl)[2-(2'-amino-1,1'-biphenyl)]pall adium(II) (163 mg, 0.21 mmol) were dissolved in 1,4-dioxane/water (4.40 mL/0.74 mL) at room temperature. The reaction system was evacuated to remove air and purged with nitrogen for 3 times, and the reaction system was stirred for 8 hours under the microwave condition of 90°C.

After the disappearance of raw materials as monitored by LCMS, ice water (20 mL) was added to the reaction solution for quenching. The mixed solution was extracted with ethyl acetate (10 mL × 3 times), and the organic phases were combined. The combined organic phase was washed with saturated saline (20 mL × 2 times). The resultant was dried with anhydrous sodium sulfate, filtered and concentrated under reduced pressure. The residue was purified by silica gel column chromatography to obtain 85 mg of 9-(2,4-dimethoxybenzyl)-2-(2-isopropylphenyl)-9H-pyridino[4',3':4,5]pyrrolo[2,3-d]pyrimidine.

MS(ESI) M/Z: 439.2 $[M+H]^+$.

**Step B:** 9-(2,4-Dimethoxybenzyl)-2-(2-isopropylphenyl)-9H-pyridino[4',3':4,5]pyrrolo[2,3-d]pyrimidine (80 mg, 0.21 mmol) was dissolved in trifluoroacetic acid (3 mL) at room temperature. The reaction system was then stirred for 12 hours.

After the disappearance of raw materials as monitored by LCMS, the reaction solution was concentrated under reduced pressure. The residue was purified by silica gel column chromatography to obtain 76 mg of 2-(2-isopropylphenyl)-9H-pyridino[4',3':4,5]pyrrolo[2,3-d]pyrimidine.

MS (ESI) M/Z: 289.1 $[M+H]^+$.

**Step C:** 2-(2-Isopropylphenyl)-9H-pyridino[4',3':4,5]pyrrolo[2,3-d]pyrimidine (70 mg, 0.24 mmol) was dissolved in dry N,N-dimethylformamide (1.5 mL) at 0°C under nitrogen protection. Subsequently, sodium hydride (58 mg, 0.97 mmol) was added to the above solution, and the resultant was stirred for 20 minutes. Then, 2-(4-(bromomethyl)phenyl)-1-methyl-4-(trifluoromethyl)-1H-imidazole (77 mg, 0.24 mmol) was added, and the mixture was raised to room temperature and stirred for 10 minutes.

After the disappearance of raw materials as monitored by LCMS, the reaction solution was filtered and the filtrate was concentrated under reduced pressure. The residue was purified by preparative HPLC. The product was collected to obtain 11.73 mg of 2-(2-isopropylphenyl)-9-(4-(1-methyl-4-(trifluoromethyl)-1H-imidazol-2-yl)benzyl)-9H-pyridino[ 4',3': 4,5]pyrrolo[2,3-d]pyrimidine.

MS (ESI) M/Z: 527.2 $[M+H]^+$.

$^1H$ NMR (400 MHz, DMSO-$d_6$) δ 9.79 (s, 1H), 9.22 (s, 1H), 8.62 (d, *J* = 5.2 Hz, 1H), 8.33 (d, *J* = 5.2 Hz, 1H), 7.90 (s, 1H), 7.73 (d, *J* = 8.0 Hz, 1H), 7.67 (d, *J* = 8.4 Hz, 2H), 7.54-7.43 (m, 4H), 7.37-7.30 (m, 1H), 5.89 (s, 2H), 3.72 (s, 3H), 3.67-3.55 (m, 1H), 1.17 (d, *J* = 6.8 Hz, 6H).

**Example 21: 2-(2-isopropylpyridin-3-yl)-9-(4-(1-methyl-4-(trifluoromethyl)-1H-imidazol-2-yl)benzyl)-9H-pyridino [4',3':4,5]pyrrolo[2,3-d]pyrimidine**

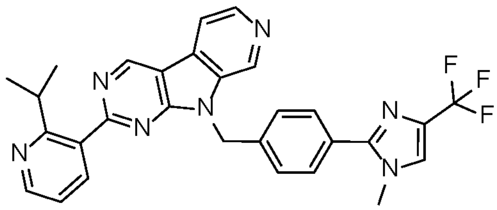

**Reaction route:**

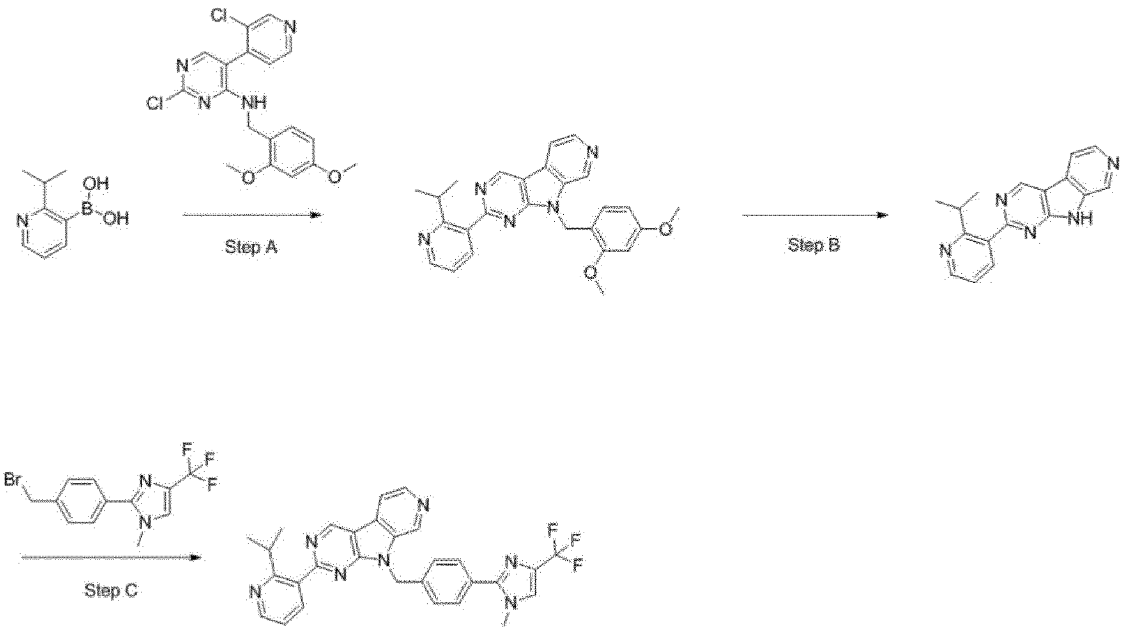


Compound 80

**Operation steps:**

**Step A:** (2-Isopropylpyridin-3-yl)boric acid (500 mg, 2.02 mmol), 2-chloro-5-(3-chloropyridin-4-yl)-N-(2,4-dimethoxybenzyl)pyrimidin-4-amine (790 mg, 2.02 mmol), cesium carbonate (1.32 g, 04.05 mmol) and chloro(2-dicyclohexylphosphino-2',4',6'-triisopropyl-1,1'-biphenyl)[2-(2'-amino-1,1'-biphenyl)]pall adium(II) (320 mg, 0.41 mmol) were dissolved in 1,4-dioxane/water (8.70 mL/1.45 mL) at room temperature. The reaction system was evacuated to remove air and purged with nitrogen for 3 times, and the reaction system was stirred for 16 hours under the microwave condition of 90°C.

After the disappearance of raw materials as monitored by LCMS, ice water (20 mL) was added to the reaction solution for quenching. The mixed solution was extracted with ethyl acetate (10 mL × 3 times), and the organic phases were combined. The combined organic phase was washed with saturated saline (20 mL × 2 times). The resultant was dried with anhydrous sodium sulfate, filtered and concentrated under reduced pressure. The residue was purified by silica gel column chromatography to obtain 235 mg of 9-(2,4-dimethoxybenzyl)-2-(2-isopropylpyridin-3-yl)-9H-pyridino[4',3':4,5]pyrrolo[2,3-d]pyrimidi ne.

MS(ESI) M/Z: 440.2 $[M+H]^+$.

**Step B:** 9-(2,4-Dimethoxybenzyl)-2-(2-isopropylpyridin-3-yl)-9H-pyridino[4',3':4,5]pyrrolo[2,3-d]pyrimid ine (210 mg, 0.48 mmol) was dissolved in trifluoroacetic acid (4 mL) at room temperature. The reaction system was then stirred for 16 hours.

After the disappearance of raw materials as monitored by LCMS, the reaction solution was concentrated under reduced pressure. The residue was purified by silica gel column chromatography to obtain 167 mg of 2-(2-isopropylpyridin-3-yl)-9H-pyridino[4',3':4,5]pyrrolo[2,3-d]pyrimidine.

MS (ESI) M/Z: 291.2 $[M+H]^+$.

**Step C:** 2-(2-Isopropylpyridin-3-yl)-9H-pyridino[4',3':4,5]pyrrolo[2,3-d]pyrimidine (157 mg, 0.54 mmol) was dissolved in dry N,N-dimethylformamide (2.7 mL) at 0°C under nitrogen protection. Subsequently, sodium hydride (87 mg, 2.17 mmol) was added to the above solution and stirred for 20 minutes. Then, 2-(4-(bromomethyl)phenyl)-1-methyl-4-(trifluoromethyl)-1H-imidazole (172 mg, 0.54 mmol) was added, and the mixture was raised to room temperature and stirred for 10 minutes.

After the disappearance of raw materials as monitored by LCMS, the reaction solution was filtered and the filtrate was concentrated under reduced pressure. The residue was purified by preparative high performance liquid chromatography. The product was collected to obtain 62.29 mg of 2-(2-isopropylpyridin-3-yl)-9-(4-(1-methyl-4-(trifluoromethyl)-1H-imidazol-2-yl)benzyl)-9H-pyri dino[4',3':4,5]pyrrolo[2,3-d]pyrimidine.

MS (ESI) M/Z: 528.2 $[M+H]^+$.

$^1$H NMR (400 MHz, DMSO-$d_6$) δ 9.82 (s, 1H), 9.24 (s, 1H), 8.68 (dd, *J* = 4.6, 1.8 Hz, 1H), 8.63 (d, *J* = 5.2 Hz, 1H), 8.35 (dd, *J* = 5.2, 0.4 Hz, 1H), 8.19 (dd, *J* = 7.6, 1.8 Hz, 1H), 7.90 (d, *J* = 1.2 Hz, 1H), 7.68 (d, *J* = 8.4 Hz, 2H), 7.48 (d, *J* = 8.0 Hz, 2H), 7.40 (dd, *J* = 8.0, 4.8 Hz, 1H), 5.91 (s, 2H), 3.86-3.75 (m, 1H), 3.73 (s, 3H), 1.22 (d, *J* = 6.4 Hz, 6H).

**Example** 22: **2-(1-cyclobutyl-4-methoxy-1H-pyrazol-5-yl)-9-(4-(1-methyl-4-(trifluoromethyl)-1H-imidazol-2-yl) benzyl)-9H-pyridino[4',3':4,5]pyrrolo[2,3-d]pyrimidine**

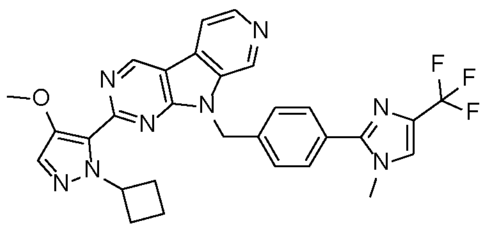

**Reaction route:**

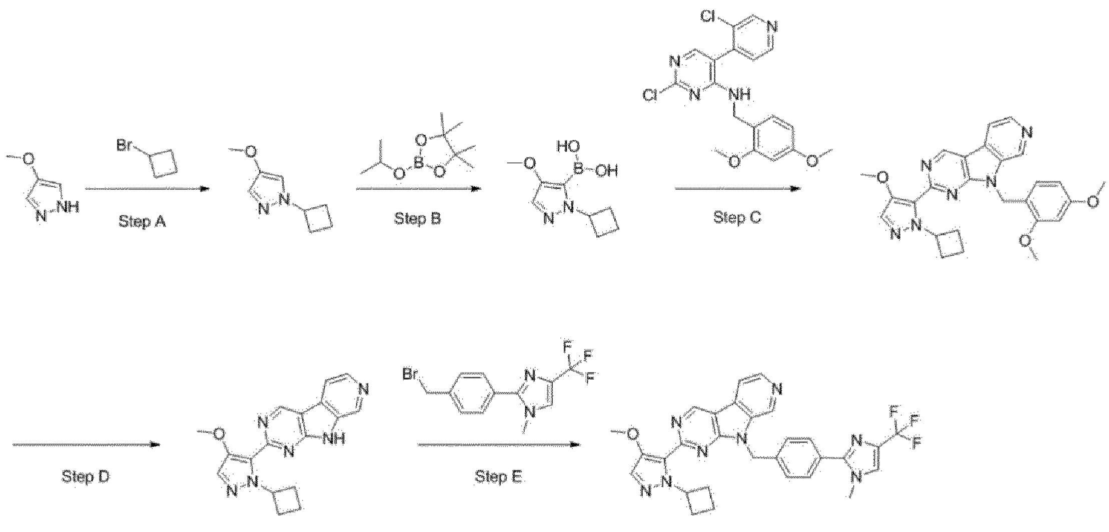


Compound 81

**Operation steps:**

**Step A:** 4-Methoxy-1H-pyrazole (6.0 g, 61.2 mmol) and potassium carbonate (33.8 g, 244.8 mmol) were dissolved in N,N-dimethylformamide (250 mL) at room temperature, and the solution was stirred for 30 min. Subsequently, cyclobutyl bromide (24.6 g, 183.6 mmol) was added dropwise to the above solution, and the solution was heated to 70°C and stirred for 24 hours.

After the disappearance of raw materials as monitored by LCMS, ice water (800 mL) was added to the reaction solution for quenching. The mixed solution was extracted with ethyl acetate (250 mL × 3 times), and the organic phases were combined. The combined organic phase was washed with saturated aqueous salt solution (250 mL × 2 times). The resultant was dried with anhydrous sodium sulfate, filtered and concentrated under reduced pressure. The residue was purified by silica gel column chromatography to obtain 600 mg of 1-cyclobutyl-4-methoxy-1H-pyrazole.

MS(ESI) M/Z: 153.2 $[M+H]^+$.

**Step B:** 1-Cyclobutyl-4-methoxy-1H-pyrazole (580 mg, 3.81 mmol) was dissolved in dry tetrahydrofuran (40 mL) at -78°C under nitrogen protection. Subsequently, 2.5 M n-butyl lithium solution (2.0 mL, 5.0 mmol) was slowly added dropwise to the above solution, and the mixture was stirred for 1 hour. Then, 2-isopropoxy-4,4,5,5-tetramethyl-1,3,2-dioxaborolane (922 mg, 4.95 mmol) was added dropwise, and the mixture was raised to room temperature and stirred for 2 hours.

After the disappearance of raw materials as monitored by LCMS, ice water (20 mL) was added to the reaction solution for quenching. The mixed solution was extracted with ethyl acetate (10 mL × 3 times), and the organic phases were combined. The combined organic phase was washed with saturated saline (20 mL × 2 times). The resultant was dried with anhydrous sodium sulfate, filtered and concentrated under reduced pressure. The residue was purified by silica gel column chromatography to obtain 600 mg of (1-cyclobutyl-4-methoxy-1H-pyrazol-5-yl)boric acid.

MS(ESI) M/Z: 197.2 $[M+H]^+$.

**Step C:** (1-Cyclobutyl-4-methoxy-1H-pyrazol-5-yl)boric acid (84 mg, 0.43 mmol), 2-chloro-5-(3-chloropyridin-4-yl)-N-(2,4-dimethoxybenzyl)pyrimidin-4-amine (168 mg, 0.43 mmol), cesium carbonate (281 mg, 0.86 mmol) and chloro(2-dicyclohexylphosphino-2',4',6'-triisopropyl-1,1'-biphenyl)[2-(2'-amino-1,1'-biphenyl)]pall adium(II) (84 mg, 0.08 mmol) were dissolved in 1,4-dioxane/water (1.85 mL/0.31 mL) at room temperature. The reaction system was evacuated to remove air and purged with nitrogen for 3 times, and the reaction system was stirred for 12 hours under the microwave condition of 90°C.

After the disappearance of raw materials as monitored by LCMS, ice water (20 mL) was added to the reaction solution for quenching. The mixed solution was extracted with ethyl acetate (10 mL × 3 times), and the organic phases were combined. The combined organic phase was washed with saturated saline (20 mL × 2 times). The resultant was dried with anhydrous sodium sulfate, filtered and concentrated under reduced pressure. The residue was purified by silica gel column chromatography to obtain 115 mg of 2-(1-cyclobutyl-4-methoxy-1H-pyrazol-5-yl)-9-(2,4-dimethoxybenzyl)-9H-pyridino[4',3':4,5]pyrro lo[2,3-d]pyrimidine.

MS(ESI) M/Z: 471.2 [M+H]$^+$.

**Step D:** 2-(1-Cyclobutyl-4-methoxy-1H-pyrazol-5-yl)-9-(2,4-dimethoxybenzyl)-9H-pyridino[4',3':4,5]pyrr olo [2,3-d]pyrimidine (110 mg, 0.23 mmol) was dissolved in trifluoroacetic acid (3 mL) at room temperature, the solution was then stirred at room temperature for 12 hours.

After the disappearance of raw materials as monitored by LCMS, the reaction solution was concentrated under reduced pressure. The residue was purified by silica gel column chromatography to obtain 100 mg of 2-(1-cyclobutyl-4-methoxy-1H-pyrazol-5-yl)-9H-pyridino[4',3':4,5]pyrrolo[2,3-d]pyrimidine.

MS (ESI) M/Z: 321.2 [M+H]$^+$.

**Step E:** 2-(1-Cyclobutyl-4-methoxy-1H-pyrazol-5-yl)-9H-pyridino[4',3':4,5]pyrrolo[2,3-d]pyrimidine (100 mg, 0.31 mmol) was dissolved in dry N,N-dimethylformamide (1.6 mL) at 0°C under nitrogen protection. Subsequently, sodium hydride (50 mg, 1.25 mmol) was added to the above solution, and the resultant was stirred for 20 minutes. Then, 2-(4-(bromomethyl)phenyl)-1-methyl-4-(trifluoromethyl)-1H-imidazole (99 mg, 0.31 mmol) was added, and the mixture was raised to room temperature and stirred for 10 minutes.

After the disappearance of raw materials as monitored by LCMS, the reaction solution was concentrated under reduced pressure. The residue was purified by preparative high performance liquid chromatography. 15.44 mg of 2-(1-cyclobutyl-4-methoxy-1H-pyrazol-5-yl)-9-(4-(1-methyl-4-(trifluoromethyl)-1H-imidazol-2-yl )benzyl)-9H-pyridino [4',3':4,5]pyrrolo[2,3-d]pyrimidine was obtained.

MS (ESI) M/Z: 559.2 [M+H]$^+$.

$^1$H NMR (400 MHz, DMSO-$d_6$) δ 9.75 (s, 1H), 9.27 (s, 1H), 8.61 (d, $J$ = 5.2 Hz, 1H), 8.29 (d, $J$ = 5.2 Hz, 1H), 7.90 (d, $J$ = 0.8 Hz, 1H), 7.69 (d, $J$ = 8.4 Hz, 2H), 7.60 (s, 1H), 7.57 (d, $J$ = 8.4 Hz, 2H), 5.89 (s, 2H), 5.68-5.56 (m, 1H), 3.84 (s, 3H), 3.72 (s, 3H), 2.61-2.53 (m, 2H), 2.31-2.19 (m, 2H), 1.79-1.54 (m, 2H).

**Example 23: N-(2-(4-cyclopropyl-6-methoxypyrimidin-5-yl)-9-(4-(1-methyl-4-(trifluoromethyl)-1H-imidaz ol-2-yl) benzyl)-9H-pyrimido[4,5-b]indol-7-yl)-N-methylmethanesulfonamide**

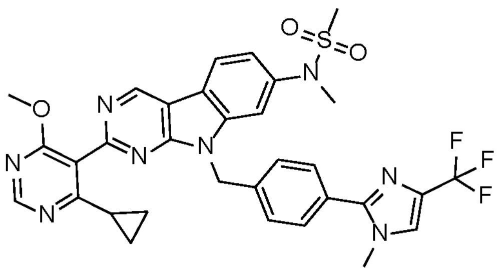

**Reaction route:**

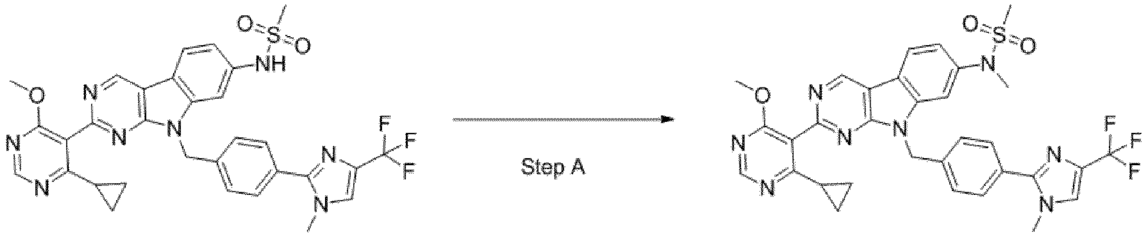


Compound 82

**Operation steps:**

**Step A:** N-(2-(4-cyclopropyl-6-methoxypyrimidin-5-yl)-9-(4-(1-methyl-4-(trifluoromethyl)-1H-imidazol-2 -yl) benzyl)-9H-pyrimido[4,5-b]indol-7-yl)methanesulfonamide (80 mg, 0.12 mmol) was dissolved in N,N-dimethylformamide (1.2 mL) at 0°C under nitrogen protection. Subsequently, sodium hydride (8.8 mg, 0.22 mmol) was added to the above solution, and the resultant was stirred for 0.5 hour. Then, methyl iodide (31 mg, 0.22 mmol) was slowly added dropwise, and the solution was heated to room temperature and stirred for 10 minutes.

After the disappearance of raw materials as monitored by LCMS, water (20 mL) was added to the reaction solution for quenching. The mixed solution was extracted with ethyl acetate (10 mL × 3 times), and the resultant was dried with anhydrous sodium sulfate, filtered and concentrated under reduced pressure. The residue was purified by preparative high performance liquid chromatography. 42.99 mg of N-(2-(4-cyclopropyl-6-methoxypyrimidin-5-yl)-9-(4-(1-methyl-4-(trifluoromethyl)-1H-imidazol-2 -yl)benzyl)-9H-pyrimido[4,5-b]indol-7-yl)-N-methylmethanesulfonamide was obtained.

MS (ESI) M/Z: 663.2 $[M+H]^+$.

$^1$H NMR (400 MHz, DMSO-$d_6$) δ 9.65 (s, 1H), 8.71 (s, 1H), 8.38 (d, *J* = 8.4 Hz, 1H), 7.95 (d, *J* = 2.0 Hz, 1H), 7.90 (d, *J* = 1.2 Hz, 1H), 7.64 (d, *J* = 8.4 Hz, 2H), 7.54-7.45 (m, 3H), 5.80 (s, 2H), 3.88 (s, 3H), 3.71 (s, 3H), 3.36 (s, 3H), 3.00 (s, 3H), 1.76-1.67 (m, 1H), 1.10-1.01 (m, 2H), 0.89-0.81 (m, 2H).

**Example 24: N-(2-(4-cyclopropyl-6-methoxypyrimidin-5-yl)-9-((2-(trifluoromethyl)-6,7-dihydro-5H-benzo [c]imidazo[1,2-a]azepan-9-yl)methyl)-9H-pyrimido[4,5-b]indol-7-yl)methanesulfonamide**

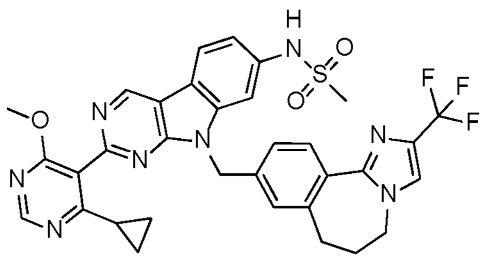

**Reaction route:**

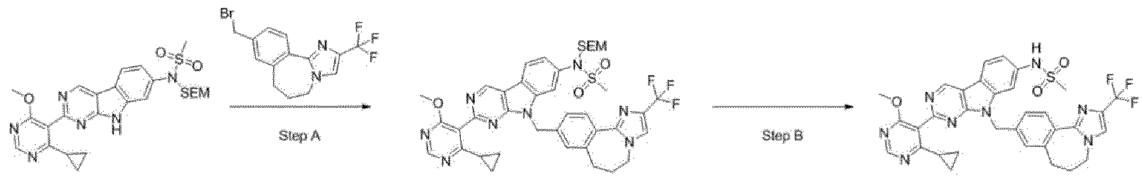


Compound 83

**Operation steps:**

**Step A:** N-(2-(4-cyclopropyl-6-methoxypyrimidin-5-yl)-9H-pyrimido[4,5-b]indol-7-yl)-N-((2-(trimethylsil yl) ethoxy)methyl)methanesulfonamide (366 mg, 0.68 mmol) was dissolved in N,N-dimethylformamide (6.8 mL) under nitrogen protection at room temperature. Subsequently, the above solution was cooled to 0°C, and sodium hydride (48.8 mg, 1.22 mmol) was slowly added, and the resultant was stirred for 0.5 hour. Then 9-(bromomethyl)-2-(trifluoromethyl)-6,7-dihydro-5H-benzo[c]imidazo[1,2-a]azepane (233 mg, 0.68 mmol) was added, and the mixture was heated to room temperature and stirred for 10 minutes.

After the disappearance of raw materials as monitored by LCMS, water (20 mL) was added to the reaction solution for quenching. The mixed solution was extracted with ethyl acetate (10 mL × 3 times), and the resultant was dried with anhydrous sodium sulfate, filtered and concentrated under reduced pressure. The residue was purified by silica gel column chromatography to obtain 350 mg of N-(2-(4-cyclopropyl-6-methoxypyrimidin-5-yl)-9-((2-(trifluoromethyl)-6,7-dihydro-5H-benzo[c]i midazo[1,2-a]azepan-9-yl)methyl)-9H-pyrimido[4,5-b]indol-7-yl)-N-(2-(trimethylsilyl)ethoxy)methyl)methanesulfonamide.

MS (ESI) M/Z: 805.2 $[M+H]^+$.

**Step B:** N-(2-(4-cyclopropyl-6-methoxypyrimidin-5-yl)-9-((2-(trifluoromethyl)-6,7-dihydro-5H-benzo[c]i midazo [1,2-a]azepan-9-yl)methyl)-9H-pyrimido[4,5-b]indol-7-yl)-N-(2-(trimethylsilyl)ethoxy)met hyl)methanesulfonamide (350 mg, 0.43 mmol) was dissolved in dichloromethane (4 mL) at room temperature. Subsequently, the above solution was cooled to 0°C, and trifluoroacetic acid (4 mL) was slowly added dropwise. The reaction system was then heated to room temperature and stirred for 1 hour.

After the disappearance of raw materials as monitored by LCMS, water (30 mL) was added to the reaction solution for quenching. The mixed solution was extracted with ethyl acetate (15 mL × 3 times), and the organic phases were combined. The combined organic phase was washed with saturated sodium bicarbonate solution (15 mL) and saturated saline (30 mL × 2 times) successively. The resultant was dried with anhydrous sodium sulfate, filtered and concentrated under reduced pressure. The obtained residue was purified by dichloromethane/ethyl acetate to obtain 168.30 mg of N-(2-(4-cyclopropyl-6-methoxypyrimidin-5-yl)-9-((2-(trifluoromethyl)-6,7-dihydro-5H-benzo[c]i midazo[1,2-a]azepan-9-yl)methyl)-9H-pyrimido[4,5-b]indol-7-yl)methanesulfonamide.

MS (ESI) M/Z: 675.2 $[M+H]^+$.

$^1$H NMR (400 MHz, DMSO-$d_6$) δ 10.12 (s, 1H), 9.56 (s, 1H), 8.71 (s, 1H), 8.28 (d, *J* = 8.4 Hz, 1H), 7.96 (d, *J* = 1.2 Hz, 1H), 7.61 (d, *J* = 8.0 Hz, 1H), 7.58 (d, *J* = 1.6 Hz, 1H), 7.39 (s, 1H), 7.27-7.23 (m, 2H), 5.70 (s, 2H), 3.95 (t, *J* = 6.8 Hz, 2H), 3.87 (s, 3H), 3.04 (s, 3H), 2.61 (t, *J* = 7.0 Hz, 2H), 2.28-2.15 (m, 2H), 1.77-1.67 (m, 1H), 1.10-1.02 (m, 2H), 0.90-0.79 (m, 2H).

**Examples 25 to 140**

Target compounds in the following Table 1 were prepared by referring to the synthesis method of Example 8 above:

Table 1

| Example | Compound | Structural formula | $^1$HNMR | MS (ESI) M/Z :$[M+H]^+$ |
|---|---|---|---|---|
| 25 | **Compound 12** | 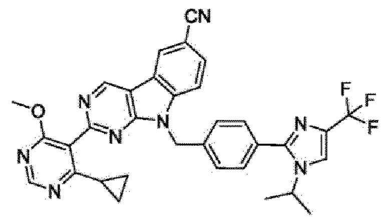 | $^1$H NMR (400 MHz, DMSO-$d_6$): δ 9.77 (s, 1H), 8.97 (d, *J* = 0.8 Hz, 1H), 8.72 (s, 1H), 8.15 (d, *J* = 0.8 Hz, 1H), 8.13 - 8.03 (m, 2H), 7.52 - 7.47 (m, 4H), 5.87 (s, 2H), 4.43 - 4.33 (m, 1H), 3.87 (s, 3H), 1.79 - 1.68 (m, 1H), 1.35 (d, *J* = 6.4 Hz, 6H), 1.10 - 1.02 (m, 2H), 0.89 - 0.79 (m, 2H). | 609.0 |
| 26 | **Compound 14** | 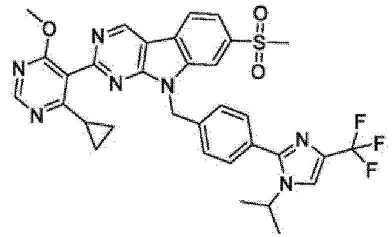 | $^1$H NMR (400 MHz, DMSO-$d_6$): δ 9.84 (s, 1H), 8.71 (s, 1H), 8.66 (d, *J* = 8.0 Hz, 1H), 8.46 (d, *J* = 1.2 Hz, 1H), 8.15 (d, *J* = 0.8 Hz, 1H), 8.00 (dd, *J* = 8.0, 1.2 Hz, 1H), 7.51(d, *J* = 8.4 Hz, 2H), 7.45 (d, *J* =8.4 Hz, 2H), 5.94 (s, 2H), 4.46 - 4.31 (m, 1H), 3.87 (s, 3H), 3.34 (s, 3H), 1.79 - 1.65 (m, 1H), 1.35 (d, *J* = 6.8 Hz, 6H), 1.10 - 1.00 (m, 2H), 0.89 - 0.75 (m, 2H). | 661.8 |
| 27 | **Compound 15** | 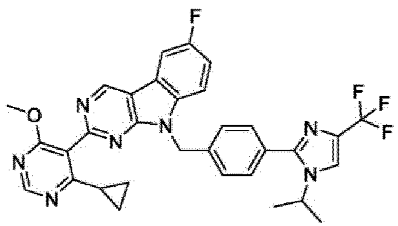 | $^1$HNMR (400 MHz, DMSO-$d_6$): δ 9.67 (s, 1H), 8.71 (s, 1H), 8.26 (dd, *J* = 8.8, 2.8 Hz, 1H), 8.15 (d, *J* = 1.2 Hz, 1H), 7.91 (dd, *J* = 9.2, 4.4 Hz, 1H), 7.58 - 7.39 (m, 5H), 5.81 (s, 2H), 4.46 - 4.28 (m, 1H), 3.87 (s, 3H), 1.78 - 1.66 (m, 1H), 1.35 (d, *J* = 6.4 Hz, 6H), 1.10 - 1.00 (m, 2H), 0.89 - 0.78 (m, 2H). | 602.2 |
| 28 | **Compound 16** | 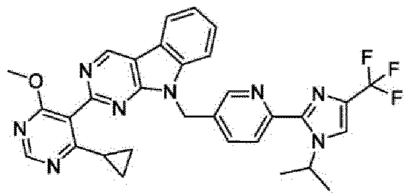 | $^1$H NMR (400 MHz, DMSO-$d_6$): δ 9.66 (s, 1H), 8.80 (d, *J* = 1.6 Hz, 1H), 8.72 (s, 1H), 8.37 (d, *J* = 8.0 Hz, 1H), 8.21 (d,*J* = 1.2 Hz, 1H), 8.02 - 7.90 (m, 3H), 7.66 (t, *J* = 7.2 Hz, 1H), 7.45 (t, *J* = 7.6 Hz, 1H), 5.83 (s, 2H), 5.70 - 5.58 (m, 1H), 3.89 (s, 3H), 1.80 - 1.70 (m, 1H), 1.40 (d, *J* = 6.8 Hz, 6H), 1.11 - 1.03 (m, 2H), 0.93 - 0.82 (m, 2H). | 585.2 |

(continued)

| Example | Compound | Structural formula | $^1$HNMR | MS (ESI) M/Z :$[M+H]^+$ |
|---|---|---|---|---|
| 29 | **Compound 17** | 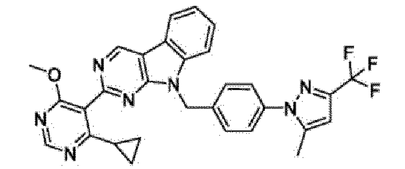 | $^1$H NMR (400 MHz, DMSO-$d_6$): δ 9.66 (s, 1H), 8.70 (s, 1H), 8.37 (d, *J* = 7.6 Hz, 1H), 7.87 (d, *J* = 8.0 Hz, 1H), 7.64 (t, *J* = 7.8 Hz, 1H), 7.51 (s, 4H), 7.44 (t, *J* = 7.64 Hz, 1H), 6.72 (s, 1H), 5.82 (s, 2H), 3.87 (s, 3H), 2.27 (s, 3H), 1.78 - 1.66 (m, 1H), 1.11 - 0.99 (m, 2H), 0.91 - 0.78 (m, 2H). | 556.2 |
| 30 | **Compound 18** | 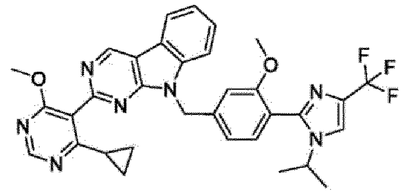 | $^1$H NMR (400 MHz, DMSO-*d6*): δ 9.66 (s, 1H), 8.71 (s, 1H), 8.38 (d, *J* = 7.6 Hz, 1H), 8.09 (d, *J* = 1.2 Hz, 1H), 7.89 (d, *J* = 8.0 Hz, 1H), 7.65 (t, *J* = 7.4 Hz, 1H), 7.45 (t, *J* = 7.4 Hz, 1H), 7.33 (s, 1H), 7.22 (d, *J* = 8.0 Hz, 1H), 6.84 (dd, *J* = 7.8, 0.5 Hz, 1H), 5.79 (s, 2H), 4.00 - 3.90 (m, 1H), 3.85 (s, 3H), 3.72 (s, 3H), 1.74 - 1.64 (m, 1H), 1.27 (d, *J* = 6.8 Hz, 6H), 1.10 - 1.02 (m, 2H), 0.88 - 0.79 (m, 2H). | 614.3 |
| 31 | **Compound 19** | 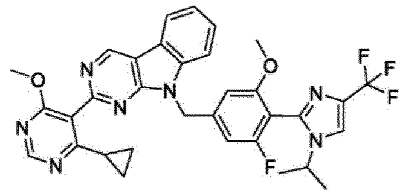 | $^1$H NMR (400 MHz, DMSO-$d_6$): δ 9.67 (s, 1H), 8.71 (s, 1H), 8.38 (d, *J* = 7.6 Hz, 1H), 8.16 (d, *J* = 0.8 Hz, 1H), 7.94 (d, *J* = 8.0 Hz, 1H), 7.67 (t, *J* = 7.2 Hz, 1H), 7.46 (t, *J* = 7.4 Hz, 1H), 7.23 (s, 1H), 6.83 (d, *J* = 9.2 Hz, 1H), 5.79 (s, 2H), 3.94 - 3.87 (m, 1H), 3.85 (s, 3H), 3.73 (s, 3H), 1.76 - 1.67 (m, 1H), 1.33 (d, *J* = 6.8 Hz, 3H), 1.20 (d, *J* = 6.4 Hz, 3H), 1.11 - 1.02 (m, 2H), 0.90 - 0.79 (m, 2H). | 632.3 |
| 32 | **Compound 20** | 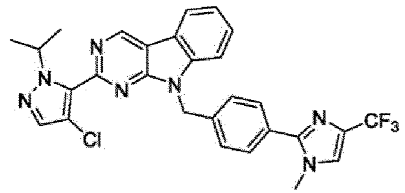 | $^1$H NMR (400 MHz, DMSO-$d_6$): δ 9.71 (s, 1H), 8.38 (d, *J* = 7.6 Hz, 1H), 7.89 (s, 1H), 7.89 (d, *J* = 8.4 Hz, 1H), 7.74 (s, 1H), 7.70 - 7.61 (m, 3H), 7.52 - 7.42 (m, 3H), 5.83 (s, 2H), 5.39 - 5.27 (m, 1H), 3.72 (s, 3H), 1.40 (d, *J* = 6.4 Hz, 6H). | 550.3 |
| 33 | **Compound 21** | 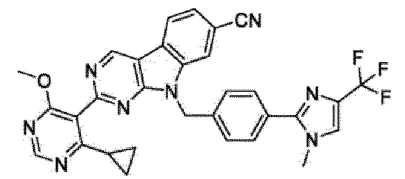 | $^1$H NMR (400 MHz, DMSO-$d_6$): δ 9.81 (s, 1H), 8.72 (s, 1H), 8.58 (d, *J* = 8.0 Hz, 1H), 8.56 (s, 1H), 7.90 (d, *J* = 1.2 Hz, 1H), 7.86 (dd, *J* =8.0, 1.2 Hz, 1H), 7.65 (d, *J* = 8.0 Hz, 2H), 7.52 (d, *J* = 8.4 Hz, 2H), 5.84 (s, 2H), 3.87 (s, 3H), 3.72 (s, 3H), 1.78 - 1.66(m, 1H), 1.10 - 1.02 (m, 2H), 0.89 - 0.81 (m, 2H). | 581.0 |

(continued)

| Example | Compound | Structural formula | $^1$HNMR | MS (ESI) M/Z :[M+H]$^+$ |
|---|---|---|---|---|
| 34 | **Compound 22** | 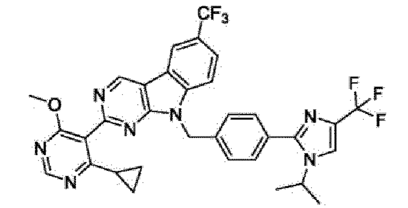 | $^1$H NMR (400 MHz, DMSO-$d_6$): δ 9.84 (s, 1H), 8.90 (s, 1H), 8.72 (s, 1H), 8.15 (s, 1H), 8.09 (d, *J* = 8.8 Hz, 1H), 7.97 (d, *J* = 8.4 Hz, 1H), 7.55 - 7.42 (m, 4H), 5.88 (s, 2H), 4.43 - 4.33 (m, 1H), 3.88 (s, 3H), 1.78 - 1.68 (m, 1H), 1.35 (d, *J* = 6.4 Hz, 6H), 1.11 - 1.01 (m, 2H), 0.90 - 0.80 (m, 2H). | 652.3 |
| 35 | **Compound 23** | 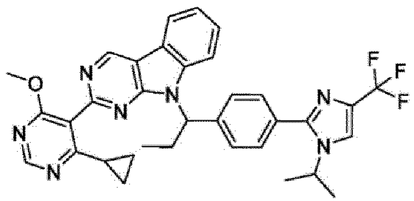 | $^1$H NMR (400 MHz, DMSO-$d_6$): δ 9.65 (s, 1H), 8.71 (s, 1H), 8.37 (d, *J*= 7.6 Hz, 1H), 8.15 (s, 1H), 7.75 (d, *J* = 8.4 Hz, 1H), 7.65 - 7.54 (m, 3H), 7.51 (d, *J* = 8.4 Hz, 2H), 7.41 (t, *J* = 7.6 Hz, 1H), 6.32 - 6.19 (m, 1H), 4.48 - 4.34 (m, 1H), 3.88 (s, 3H), 2.96 - 2.80 (m, 1H), 2.66 - 2.56 (m, 1H), 1.82 - 1.72 (m, 1H), 1.36 (d, *J* = 6.4 Hz, 6H), 1.12 - 1.02 (m, 2H), 0.91 - 0.82 (m, 2H), 0.77 (t, *J* = 7.0 Hz, 3H). | 612.0 |
| 36 | **Compound 24** | 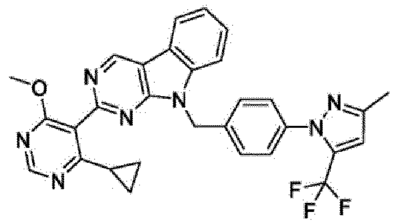 | $^1$H NMR (400 MHz, DMSO-$d_6$): δ 9.66 (s, 1H), 8.70 (s, 1H), 8.38 (d, *J* = 7.6 Hz, 1H), 7.85 (d, *J* = 8.4 Hz, 1H), 7.64 (t, *J* = 7.6 Hz, 1H), 7.50 - 7.36 (m, 5H), 6.91 (s, 1H), 5.82 (s, 2H), 3.86 (s, 3H), 2.25 (s, 3H), 1.76 - 1.66 (m, 1H), 1.10 - 1.00 (m, 2H), 0.90 - 0.76 (m, 2H), | 556.2 |
| 37 | **Compound 25** | 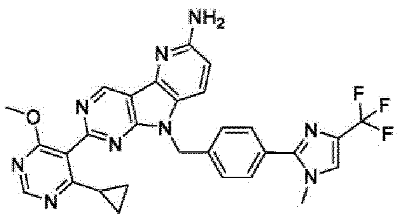 | $^1$H NMR (400 MHz, DMSO-$d_6$): δ 9.40 (s, 1H), 8.70 (s, 1H), 7.97 (d, *J* = 8.8 Hz, 1H), 7.90 (s, 1H), 7.64 (d, *J* = 8.0 Hz, 2H), 7.43 (d, *J* = 8.0 Hz, 2H), 6.77 (d, *J* = 9.2 Hz, 1H), 6.12 (s, 2H), 5.71 (s, 2H), 3.87 (s, 3H), 3.72 (m, 3H), 1.80 - 1.66 (m, 1H), 1.12 - 0.98 (m, 2H), 0.93 - 0.77 (m, 2H). | 572.1 |
| 38 | **Compound 26** | 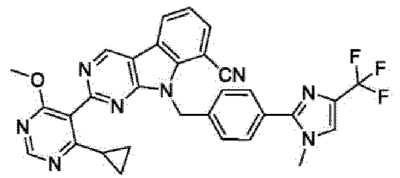 | $^1$H NMR (400 MHz, DMSO-$d_6$): δ 9.84 (s, 1H), 8.79 (dd, *J* = 7.6, 0.8 Hz, 1H), 8.70 (s, 1H), 8.09 (dd, *J* = 8.0, 1.2 Hz, 1H), 7.90 (s, 1H), 7.67 - 7.58 (m, 3H), 7.29 (d, *J* = 8.4 Hz, 2H), 6.08 (s, 2H), 3.85 (s, 3H), 3.72 (s, 3H), 1.74 - 1.63 (m, 1H), 1.07 - 1.00 (m, 2H), 0.84 - 0.77 (m, 2H). | 581.6 |

(continued)

| Example | Compound | Structural formula | $^1$HNMR | MS (ESI) M/Z :[M+H]$^+$ |
|---|---|---|---|---|
| 39 | **Compound 27** | 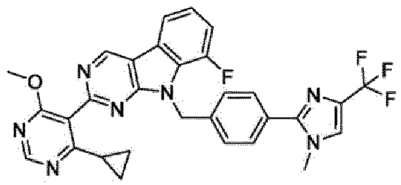 | 1H NMR (400 MHz, DMSO-$d_6$): δ 9.74 (s, 1H), 8.71 (s, 1H), 8.23 (dd, *J* = 7.6 Hz, 0.8 Hz, 1H), 7.90 (d, *J* = 1.2 Hz, 1H), 7.65 (d, *J* = 8.4 Hz, 2H), 7.53 - 7.45 (m, 1H), 7.45 - 7.40 (m, 1H), 7.37 (d, *J* = 8.4 Hz, 2H), 5.85 (s, 2H), 3.88 (s, 3H), 3.72 (s, 3H), 1.77 - 1.68 (m, 1H), 1.09 - 1.03 (m, 2H), 0.89 - 0.81 (m, 2H). | 574.0 |
| 40 | **Compound 28** | 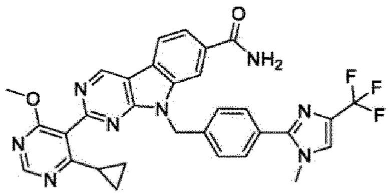 | $^1$H NMR (400 MHz, DMSO-$d_6$): δ 9.72 (s, 1H), 8.71 (s, 1H), 8.44 (d, *J* = 8.0 Hz, 1H), 8.36 (s, 1H), 8.16 (br, 1H), 7.97 (dd, *J* = 8.4, 1.2 Hz, 1H), 7.90 (d, *J* = 1.2 Hz, 1H), 7.66 (d, *J* = 8.4 Hz, 2H), 7.56 (br, 1H), 7.46 (d, *J* = 8.4 Hz, 2H), 5.82 (s, 2H), 3.87 (s, 3H), 3.71 (s, 3H), 1.76 - 1.69 (m, 1H), 1.09 - 1.02 (m, 2H), 0.88 - 0.81 (m, 2H). | 599.0 |
| 41 | **Compound 29** | 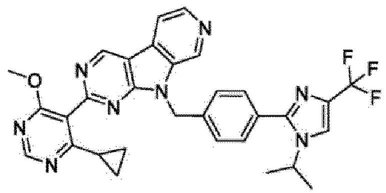 | $^1$H NMR (400 MHz, DMSO-$d_6$): δ 9.82 (s, 1H), 9.31 (s, 1H), 8.72 (s, 1H), 8.64 (d, *J* = 5.2 Hz, 1H), 8.35 (dd, *J* = 5.2 Hz, 0.8 Hz, 1H), 8.15 (d, *J* = 1.2 Hz, 1H), 7.54 (d, *J* = 8.8 Hz, 2H), 7.50 (d, *J* = 8.4 Hz, 2H), 5.89 (s, 2H), 4.45 - 4.31 (m, 1H), 3.88 (s, 3H), 1.79 - 1.68 (m, 1H), 1.35 (d, *J* = 6.4 Hz, 6H), 1.10 - 1.04 (m, 2H), 0.89 - 0.81 (m, 2H). | 585.2 |
| 42 | **Compound 30** | 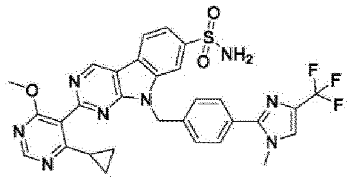 | $^1$H NMR (400 MHz, DMSO-$d_6$): δ 9.78 (s, 1H), 8.71 (s, 1H), 8.57 (d, *J* = 8.4 Hz, 1H), 8.25 (d, *J* = 1.2 Hz, 1H), 7.94 - 7.89 (m, 2H), 7.66 (d, *J* = 8.4 Hz, 2H), 7.51 (br, 2H), 7.43 (d, *J* = 8.4 Hz, 2H), 5.86 (s, 2H), 3.87 (s, 3H), 3.72 (s, 3H), 1.79 - 1.69 (m, 1H), 1.11 - 1.02 (m, 2H), 0.89 - 0.80 (m, 2H). | 635.0 |
| 43 | **Compound 31** | 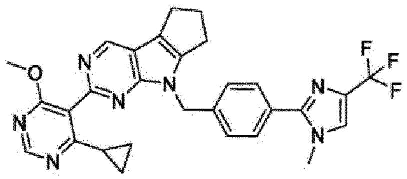 | $^1$H NMR (400 MHz, DMSO-$d_6$): δ 8.96 (s, 1H), 8.66 (s, 1H), 7.92 (d, *J* = 1.4 Hz, 1H), 7.67 (d, *J* = 8.3 Hz, 2H), 7.37 (d, *J* = 8.1 Hz, 2H), 5.49 (s, 2H), 3.84 (s, 3H), 3.75 (s, 3H), 2.95 - 2.80 (m, 4H), 2.49 - 2.42 (m, 2H), 1.72 - 1.61 (m, 1H), 1.08 - 0.98 (m, 2H), 0.88 - 0.78 (m, 2H). | 546.3 |

(continued)

| Example | Compound | Structural formula | $^1$HNMR | MS (ESI) M/Z :[M+H]$^+$ |
|---|---|---|---|---|
| 44 | **Compound 33** | 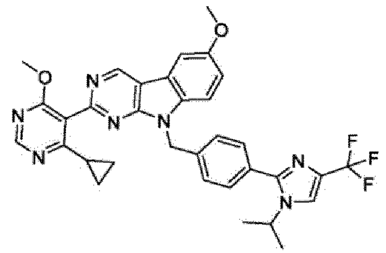 | $^1$H NMR (400 MHz, DMSO-$d_6$): δ 9.63 (s, 1H), 8.70 (s, 1H), 8.15 (d, *J* = 1.4 Hz, 1H), 7.98 (d, *J* = 2.5 Hz, 1H), 7.78 (d, *J* = 8.9 Hz, 1H), 7.50 (d, *J* = 8.4 Hz, 2H), 7.46 (d, *J* = 8.5 Hz, 2H), 7.26 (dd, *J* = 8.9, 2.6 Hz, 1H), 5.77 (s, 2H), 4.45 - 4.32 (m, 1H), 1.78 - 1.67 (m, 1H), 1.35 (d, *J* = 6.6 Hz, 6H), 1.10 - 1.02 (m, 2H), 0.88 - 0.81 (m, 2H). | 614.2 |
| 45 | **Compound 34** | 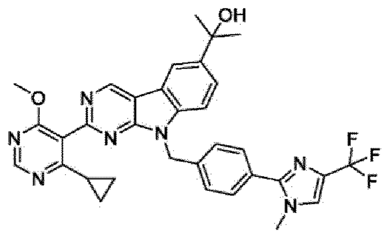 | $^1$H NMR (400 MHz, DMSO-$d_6$): δ 9.65 (s, 1H), 8.70 (s, 1H), 8.46 (s, 1H), 7.90 (d, *J* = 1.4 Hz, 1H), 7.79 - 7.72 (m, 2H), 7.64 (d, *J* = 8.3 Hz, 2H), 7.46 (d, *J* = 8.2 Hz, 2H), 5.77 (s, 2H), 5.17 (s, 1H), 3.88 (s, 3H), 3.71 (s, 3H), 1.76 - 1.68 (m, 1H), 1.54 (s, 6H), 1.10 - 1.03 (m, 2H), 0.89 - 0.80 (m, 2H), | 614.3 |
| 46 | **Compound 35** | 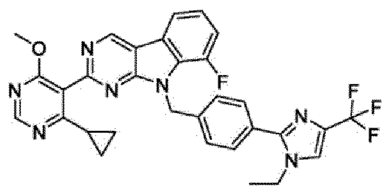 | $^1$H NMR (400 MHz, DMSO-*d6*): δ 9.74 (s, 1H), 8.71 (s, 1H), 8.23 (d, *J* = 7.7 Hz, 1H), 8.00 (d, *J* = 1.4 Hz, 1H), 7.57 (d, *J* = 8.3 Hz, 2H), 7.53 - 7.40 (m, 2H), 7.38 (d, *J* = 8.2 Hz, 2H), 5.85 (s, 2H), 4.02 (q, *J* = 7.2 Hz, 2H), 3.87 (s, 3H), 1.78 - 1.67 (m, 1H), 1.27 (t, *J* = 7.2 Hz, 3H), 1.11 - 1.02 (m, 2H), 0.90 - 0.79 (m, 2H). | 588.2 |
| 47 | **Compound 36** | 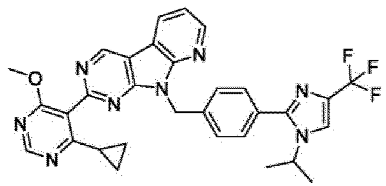 | $^1$H NMR (400 MHz, DMSO-*d6*): δ 9.71 (s, 1H), 8.77 (dd, *J* = 7.7, 1.6 Hz, 1H), 8.72 (s, 1H), 8.68 (dd, *J* = 5.0, 1.6 Hz, 1H), 8.17 (d, *J* = 1.3 Hz, 1H), 7.56 - 7.48 (m, 5H), 5.81 (s, 2H), 4.49 - 4.32 (m, 1H), 3.89 (s, 3H), 1.83 - 1.70 (m, 1H), 1.37 (d, *J* = 6.6 Hz, 6H), 1.12 - 1.02 (m, 2H), 0.89 - 0.79 (m, 2H). | 585.4 |

(continued)

| Example | Compound | Structural formula | $^1$HNMR | MS (ESI) M/Z :[M+H]$^+$ |
|---|---|---|---|---|
| 48 | **Compound 37** | 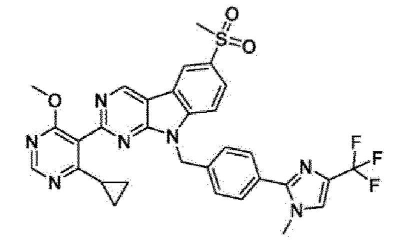 | $^1$H NMR (400 MHz, DMSO-$d_6$): δ 9.88 (s, 1H), 9.04 (d, *J* = 1.6 Hz, 1H), 8.72 (s, 1H), 8.20 - 8.09 (m, 2H), 7.90 (d, *J* = 1.4 Hz, 1H), 7.65 (d, *J* = 8.3 Hz, 2H), 7.49 (d, *J* = 8.4 Hz, 2H), 5.87 (s, 2H), 3.88 (s, 3H), 3.71 (s, 3H), 3.28 (s, 3H), 1.78 - 1.69 (m, 1H), 1.10 - 1.02 (m, 2H), 0.89 - 0.82 (m, 2H). | 634.2 |
| 49 | **Compound 38** | 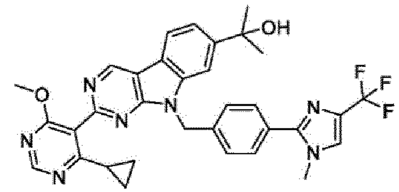 | $^1$H NMR (400 MHz, DMSO-*d6*): δ 9.59 (s, 1H), 8.70 (s, 1H), 8.26 (d, *J* = 8.2 Hz, 1H), 7.90 (s, 2H), 7.65 (d, *J* = 7.9 Hz, 2H), 7.55 (d, *J* = 8.3 Hz, 1H), 7.45 (d, *J* = 7.9 Hz, 2H), 5.79 (s, 2H), 5.22 (s, 1H), 3.87 (s, 3H), 3.72 (s, 3H), 1.77 - 1.66 (m, 1H), 1.52 (s, 3H), 1.11 - 1.02 (m, 2H), 0.89 - 0.80 (m, 2H), | 614.3 |
| 50 | **Compound 39** | 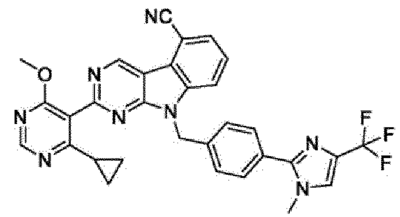 | $^1$H NMR (400 MHz, DMSO-*d6*): δ 9.69 (s, 1H), 8.73 (s, 1H), 8.29 (d, *J* = 8.4, 1H), 7.96 (d, *J* = 7.6, 1H), 7.90 (d, *J* = 1.4 Hz, 1H), 7.86 - 7.79 (m, 1H), 7.65 (d, *J* = 8.4 Hz, 2H), 7.48 (d, *J* = 8.4 Hz, 2H), 5.88 (s, 2H), 3.89 (s, 3H), 3.71 (s, 3H), 1.82 - 1.67 (m, 1H), 1.13 - 1.03 (m, 2H), 0.91 - 0.80 (m, 2H). | 581.0 |
| 51 | **Compound 40** | 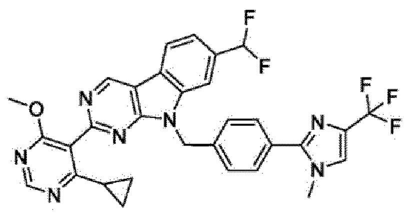 | $^1$H NMR (400 MHz, DMSO-*d6*): δ 9.75 (s, 1H), 8.71 (s, 1H), 8.52 (d, *J* = 8.2 Hz, 1H), 8.10 (s, 1H), 7.90 (d, *J* = 1.4 Hz, 1H), 7.69 - 7.60 (m, 3H), 7.46 (d, *J* = 8.3 Hz, 2H), 7.22 (t, *J* = 55.8 Hz, 1H), 5.86 (s, 2H), 3.87 (s, 3H), 3.71 (s, 3H), 1.78 - 1.67 (m, 1H), 1.10 - 1.01 (m, 2H), 0.90 - 0.75 (m, 2H). | 606.0 |

(continued)

| Example | Compound | Structural formula | $^1$HNMR | MS (ESI) M/Z :[M+H]$^+$ |
|---|---|---|---|---|
| 52 | **Compound 41** | 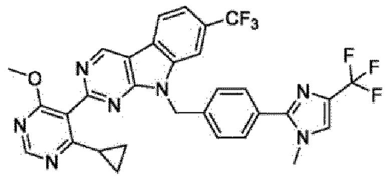 | $^1$H NMR (400 MHz, DMSO-$d_6$): δ 9.81 (s, 1H), 8.71 (s, 1H), 8.61 (d, $J$ = 8.2 Hz, 1H), 8.33 (d, $J$ = 1.5 Hz, 1H), 7.90 (d, $J$ = 1.3 Hz, 1H), 7.78 (dd, $J$ = 8.4, 1.5 Hz, 1H), 7.65 (d, $J$ = 8.4 Hz, 2H), 7.47 (d, $J$ = 8.4 Hz, 2H), 5.91 (s, 2H), 3.87 (s, 3H), 3.71 (s, 3H), 1.78 - 1.66 (m, 1H), 1.11 - 1.02 (m, 2H), 0.90 - 0.77 (m, 2H). | 624.3 |
| 53 | **Compound 42** | 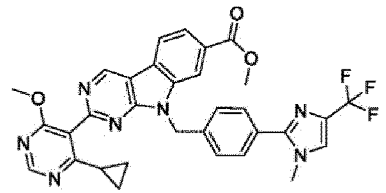 | $^1$H NMR (400 MHz, DMSO-$d_6$): δ 9.78 (s, 1H), 8.71 (s, 1H), 8.52 (d, $J$ = 8.2 Hz, 1H), 8.39 (s, 1H), 8.04 (dd, $J$ = 8.2, 1.4 Hz, 1H), 7.90 (d, $J$ = 1.4 Hz, 1H), 7.65 (d, $J$ = 8.4 Hz, 2H), 7.42 (d, $J$ = 8.3 Hz, 2H), 5.90 (s, 2H), 3.92 (s, 3H), 3.87 (s, 3H), 3.71 (s, 3H), 1.77 - 1.68 (m, 1H), 1.09 - 1.02 (m, 2H), 0.87 - 0.80 (m, 2H), | 614.2 |
| 54 | **Compound 43** | 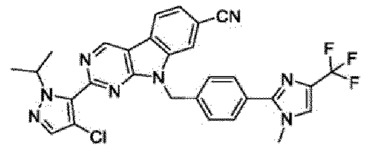 | $^1$H NMR (400 MHz, DMSO-$d_6$): δ 9.86 (s, 1H), 8.59 (d, $J$ = 8.4 Hz, 1H), 8.56 (s, 1H), 7.90 (d, $J$ = 1.2 Hz, 1H), 7.88 (dd, J = 8.0, 1.2 Hz, 1H), 7.76 (s, 1H), 7.67 (d, $J$ = 8.4 Hz, 2H), 7.51 (d, $J$ = 8.4 Hz, 2H), 5.88 (s, 2H), 5.40 - 5.27 (m, 1H), 3.72 (s, 3H), 1.39 (d, $J$ = 6.4 Hz, 6H). | 575.2 |
| 55 | **Compound 44** | 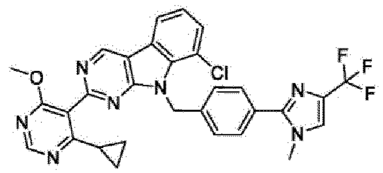 | $^1$H NMR (400 MHz, DMSO-$d_6$): δ 9.76 (s, 1H), 8.69 (s, 1H), 8.42 (dd, $J$ = 7.7, 1.1 Hz, 1H), 7.90 (d, $J$ = 1.3 Hz, 1H), 7.70 - 7.58 (m, 3H), 7.45 (t, $J$ = 7.8 Hz, 1H), 7.23 (d, $J$ = 8.5 Hz, 2H), 6.12 (s, 2H), 3.84 (s, 3H), 3.72 (s, 3H), 1.78 - 1.63 (m, 1H), 1.09 - 0.96 (m, 2H), 0.88 - 0.69 (m, 2H). | 590.0 |
| 56 | **Compound 45** | 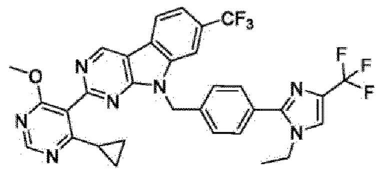 | $^1$H NMR (400 MHz, DMSO-$d_6$): δ 9.81 (s, 1H), 8.71 (s, 1H), 8.62 (d, $J$ = 8.4 Hz, 1H), 8.31 (s, 1H), 7.99 (d, $J$ = 1.2 Hz, 1H), 7.78 (d, $J$ = 7.6 Hz, 1H), 7.57 (d, $J$ = 8.0 Hz, 2H), 7.46 (d, $J$ = 8.4 Hz, 2H), 5.92 (s, 2H), 4.01 (q, $J$ = 7.2 Hz, 2H), 3.87 (s, 3H), 1.78 - 1.66 (m, 1H), 1.26 (t, $J$ = 7.2 Hz, 3H), 1.09 - 1.01 (m, 2H), 0.87 - 0.78 (m, 2H). | 638.3 |

(continued)

| Example | Compound | Structural formula | $^1$HNMR | MS (ESI) M/Z :[M+H]$^+$ |
|---|---|---|---|---|
| 57 | **Compound 46** | 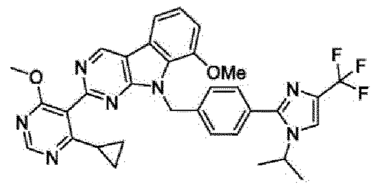 | $^1$H NMR (400 MHz, DMSO-$d_6$): δ 9.65 (s, 1H), 8.70 (s, 1H), 8.15 (s, 1H), 7.95 (d, $J$ = 7.6 Hz, 1H), 7.47 (d, $J$ = 8.4 Hz, 2H), 7.42 - 7.32 (m, 3H), 7.24 (d, $J$ = 8.0 Hz, 1H), 5.96 (s, 2H), 4.49 - 4.28 (m, 1H), 3.96 (s, 3H), 3.87 (s, 3H), 1.77 - 1.64 (m, 1H), 1.36 (d, $J$ = 6.8 Hz, 6H), 1.12 - 0.97 (m, 2H), 0.88 - 0.77 (m, 2H). | 614.2 |
| 58 | **Compound 47** | 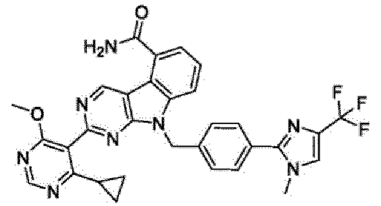 | $^1$H NMR (400 MHz, DMSO-$d_6$): δ 9.91 (s, 1H), 8.71 (s, 1H), 8.26 (s, 1H), 8.02 (dd, $J$ = 8.0, 1.2 Hz, 1H), 7.90 (d, $J$ = 1.2 Hz, 1H), 7.78 - 7.66 (m, 3H), 7.64 (d, $J$ = 8.4 Hz, 2H), 7.45 (d, $J$ = 8.0 Hz, 2H), 5.85 (s, 2H), 3.88 (s, 3H), 3.70 (s, 3H), 1.78 - 1.69 (m, 1H), 1.10 - 1.02 (m, 2H), 0.89 - 0.82 (m, 2H). | 599.3 |
| 59 | **Compound 48** | 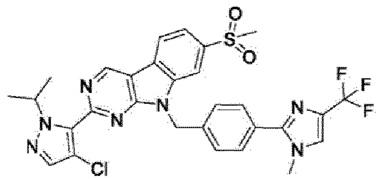 | $^1$H NMR (400 MHz, DMSO-$d_6$): δ 9.88 (s, 1H), 8.67 (d, $J$ = 8.4 Hz, 1H), 8.46 (d, $J$ = 1.2 Hz, 1H), 8.01 (dd, $J$ = 8.0, 1.2 Hz, 1H), 7.90 (d, $J$ = 1.2 Hz, 1H), 7.76 (s, 1H), 7.68 (d, $J$ = 8.4 Hz, 2H), 7.45 (d, $J$ = 8.4 Hz, 2H), 5.96 (s, 2H), 5.40 - 5.27 (m, 1H), 3.72 (s, 3H), 3.34 (s, 3H), 1.38 (d, $J$ = 6.8 Hz, 6H). | 628.0 |
| 60 | **Compound 49** | 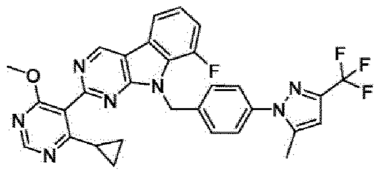 | $^1$H NMR (400 MHz, DMSO-$d_6$): δ 9.74 (s, 1H), 8.71 (s, 1H), 8.24 (d, $J$ = 6.8 Hz, 1H), 7.54 - 7.31 (m, 6H), 6.91 (s, 1H), 5.87 (s, 2H), 3.86 (s, 3H), 2.25 (s, 3H), 1.78 - 1.67 (m, 1H), 1.13 - 1.00 (m, 2H), 0.90 - 0.77 (m, 2H). | 574.4 |
| 61 | **Compound 50** | 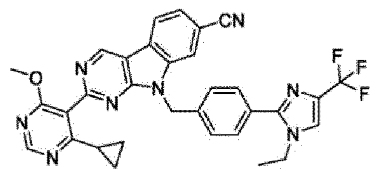 | $^1$H NMR (400 MHz, DMSO-$d_6$): δ 9.81 (s, 1H), 8.72 (s, 1H), 8.59 (d, $J$ = 8.4 Hz, 1H), 8.55 (s, 1H), 8.00 (d, $J$ = 1.2 Hz, 1H), 7.86 (dd, $J$ = 8.0, 1.2 Hz, 1H), 7.57 (d, $J$ = 8.4 Hz, 2H), 7.52 (d, $J$ = 8.4 Hz, 2H), 5.85 (s, 2H), 4.02 (q, $J$ = 7.2 Hz, 2H), 3.87 (s, 3H), 1.76 - 1.68 (m, 1H), 1.27 (t, $J$ = 7.4 Hz, 3H), 1.09 - 1.03 (m, 2H), 0.88 - 0.80 (m, 2H). | 595.2 |

(continued)

| Example | Compound | Structural formula | $^1$HNMR | MS (ESI) M/Z :$[M+H]^+$ |
|---|---|---|---|---|
| 62 | **Compound 51** | 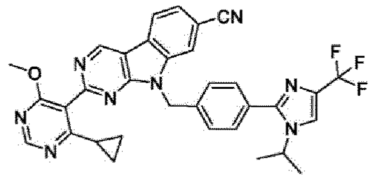 | $^1$H NMR (400 MHz, DMSO-$d_6$): δ 9.82 (s, 1H), 8.72 (s, 1H), 8.59 (m, 2H), 8.15 (d, *J* = 1.2 Hz, 1H), 7.87 (dd, *J* = 8.0, 1.2 Hz, 1H), 7.57 - 7.48 (m, 4H), 5.86 (s, 2H), 4.43 - 4.33 (m, 1H), 3.88 (s, 3H), 1.78 - 1.69 (m, 1H), 1.36 (d, *J* = 6.8 Hz, 6H), 1.10 - 1.03 (m, 2H), 0.88 - 0.80 (m, 2H). | 609.1 |
| 63 | **Compound 52** | 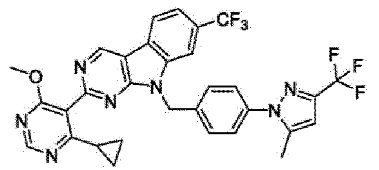 | $^1$H NMR (400 MHz, DMSO-$d_6$): δ 9.82 (s, 1H), 8.71 (s, 1H), 8.62 (d, *J* = 8.4 Hz, 1H), 8.30 (s, 1H), 7.79 (d, *J* = 7.2 Hz, 1H), 7.49 - 7.36 (m, 4H), 6.91 (s, 1H), 5.94 (s, 2H), 3.85 (s, 3H), 2.25 (s, 3H), 1.76 - 1.66 (m, 1H), 1.08 - 1.01 (m, 2H), 0.85 - 0.78 (m, 2H). | 624.2 |
| 64 | **Compound 53** | 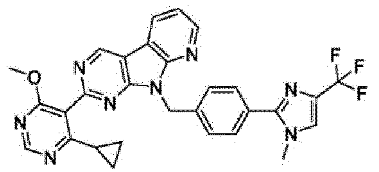 | $^1$H NMR (400 MHz, DMSO-$d_6$): δ 9.70 (s, 1H), 8.78 (dd, *J* = 7.6, 1.2 Hz, 1H), 8.71 (s, 1H), 8.68 (dd, *J* = 5.2, 1.6 Hz, 1H), 7.90 (d, *J* = 1.2 Hz, 1H), 7.63 (d, *J* = 8.4 Hz, 2H), 7.52 (dd, *J* = 8.0, 4.8 Hz, 1H), 7.49 (d, *J* = 8.3 Hz, 2H), 5.78 (s, 2H), 3.86 (s, 3H), 3.71 (s, 3H), 1.76 - 1.68 (m, 1H), 1.08 - 1.03 (m, 2H), 0.86 - 0.80 (m, 2H). | 557.3 |
| 65 | **Compound 54** | 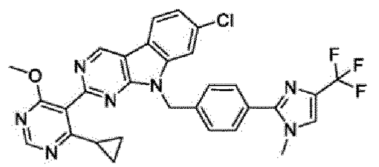 | $^1$H NMR (400 MHz, DMSO-$d_6$): δ 9.69 (s, 1H), 8.71 (s, 1H), 8.40 (d, *J* = 8.0 Hz, 1H), 8.06 (d, *J* = 1.6 Hz, 1H), 7.91 (d, *J* = 0.8 Hz, 1H), 7.66 (d, *J* = 8.0 Hz, 2H), 7.54 - 7.42 (m, 3H), 5.81 (s, 2H), 3.88 (s, 3H), 3.72 (s, 3H), 1.79 - 1.64 (m, 1H), 1.13 - 0.98 (m, 2H), 0.91 - 0.76 (m, 2H). | 590.0 |
| 66 | **Compound 55** | 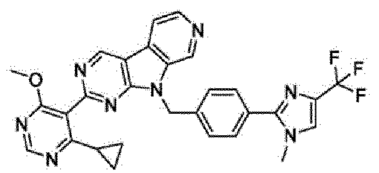 | $^1$H NMR (400 MHz, DMSO-$d_6$): δ 9.82 (s, 1H), 9.29 (s, 1H), 8.72 (s, 1H), 8.63 (d, *J* = 5.2 Hz, 1H), 8.35 (dd, *J* = 5.2, 1.1 Hz, 1H), 7.90 (d, *J* = 1.4 Hz, 1H), 7.66 (d, *J* = 8.3 Hz, 2H), 7.53 (d, *J* = 8.3 Hz, 2H), 5.88 (s, 2H), 3.88 (s, 3H), 3.72 (s, 3H), 1.81 - 1.67 (m, 1H), 1.12 - 1.02 (m, 2H), 0.93 - 0.79 (m, 2H). | 557.2 |

(continued)

| Example | Compound | Structural formula | $^1$HNMR | MS (ESI) M/Z :$[M+H]^+$ |
|---|---|---|---|---|
| 67 | **Compound 56** | 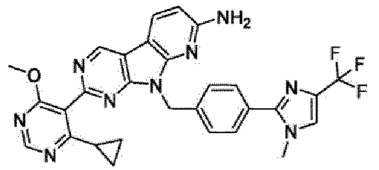 | $^1$H NMR (400 MHz, DMSO-$d_6$): δ 9.28 (s, 1H), 8.67 (s, 1H), 8.24 (d, *J* = 8.4 Hz, 1H), 7.90 (s, 1H), 7.63 (d, *J* = 8.0 Hz, 2H), 7.41 (d, *J* = 8.4 Hz, 2H), 6.72 (s, 2H), 6.54 (d, *J* = 8.4 Hz, 1H), 5.61 (s, 2H), 3.84 (s, 3H), 3.72 (s, 3H), 1.73 - 1.63 (m, 1H), 1.06 - 0.97 (m, 2H), 0.85 - 0.75 (m, 2H). | 572. 3 |
| 68 | **Compound 57** | 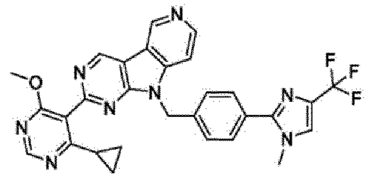 | $^1$H NMR (400 MHz, DMSO-$d_6$): δ 9.77 (s, 1H), 9.56 (d, *J* = 1.0 Hz, 1H), 8.72 (s, 1H), 8.68 (d, *J* = 5.7 Hz, 1H), 7.93 (dd, *J* = 5.8, 1.0 Hz, 1H), 7.90 (d, *J* = 1.3 Hz, 1H), 7.65 (d, *J* = 8.3 Hz, 2H), 7.49 (d, *J* = 8.3 Hz, 2H), 5.81 (s, 2H), 3.87 (s, 3H), 3.71 (s, 3H), 1.80 - 1.61 (m, 1H), 1.13 - 0.99 (m, 2H), 0.90 - 0.78 (m, 2H). | 557. 2 |
| 69 | **Compound 59** | 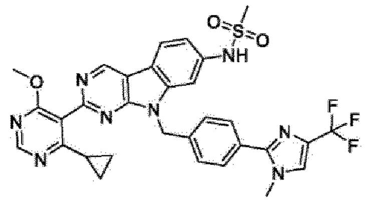 | $^1$H NMR (400 MHz, DMSO-$d_6$): δ 10.12 (s, 1H), 9.56 (s, 1H), 8.70 (s, 1H), 8.29 (d, *J* = 8.5 Hz, 1H), 7.91 (d, *J* = 1.3 Hz, 1H), 7.66 (d, *J* = 8.3 Hz, 2H), 7.56 (d, *J* = 1.9 Hz, 1H), 7.43 (d, *J* = 8.4 Hz, 2H), 7.25 (dd, *J* = 8.5, 1.9 Hz, 1H), 5.73 (s, 2H), 3.87 (s, 3H), 3.72 (s, 3H), 3.04 (s, 3H), 1.79 - 1.65 (m, 1H), 1.11 - 1.02 (m, 2H), 0.91 - 0.81 (m, 2H). | 649.0 |
| 70 | **Compound 61** | 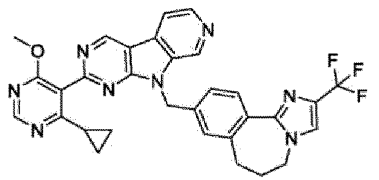 | $^1$H NMR (400 MHz, DMSO-$d_6$): δ 9.82 (s, 1H), 9.26 (d, *J* = 0.8 Hz, 1H), 8.72 (s, 1H), 8.64 (d, *J* = 5.2 Hz, 1H), 8.35 (dd, *J* = 5.2, 0.8 Hz, 1H), 7.96 (d, *J* = 1.2 Hz, 1H), 7.62 (d, *J* = 8.0 Hz, 1H), 7.44 (s, 1H), 7.35 (dd, *J* = 8.0, 1.6 Hz, 1H), 5.84 (s, 2H), 3.95 (t, *J* = 6.6 Hz, 2H), 3.88 (s, 3H), 2.61 (t, *J* = 7.0 Hz, 2H), 2.26 - 2.15 (m, 2H), 1.79 - 1.69 (m, 1H), 1.11 - 1.03 (m, 2H), 0.89 - 0.79 (m, 2H). | 583.3 |
| 71 | **Compound 62** | 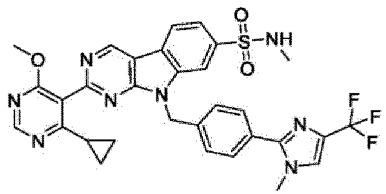 | $^1$H NMR (400 MHz, DMSO-$d_6$): δ 9.80 (s, 1H), 8.71 (s, 1H), 8.60 (d, *J* = 8.2 Hz, 1H), 8.21 (d, *J* = 1.5 Hz, 1H), 7.90 (d, *J* = 1.4 Hz, 1H), 7.83 (dd, *J* = 8.2, 1.6 Hz, 1H), 7.66 (d, *J* = 8.4 Hz, 2H), 7.55 (d, *J* = 5.0 Hz, 1H), 7.44 (d, *J* = 8.4 Hz, 2H), 5.89 (s, 2H), 3.87 (s, 3H), 3.71 (s, 3H), 2.41 (d, *J* = 4.4 Hz, 3H), 1.81 - 1.67 (m, 1H), 1.12 - 1.02 (m, 2H), 0.93 - 0.77 (m, 2H). | 649.2 |

(continued)

| Example | Compound | Structural formula | $^1$HNMR | MS (ESI) M/Z :[M+H]$^+$ |
|---|---|---|---|---|
| 72 | **Compound 63** | 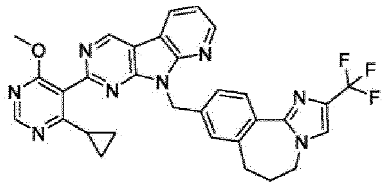 | $^1$H NMR (400 MHz, DMSO-$d_6$): δ 9.71 (s, 1H), 8.78 (dd, $J$ = 7.6, 1.6 Hz, 1H), 8.71 (s, 1H), 8.67 (dd, $J$ = 5.0, 1.4 Hz, 1H), 7.95 (d, $J$ = 1.2 Hz, 1H), 7.59 (d, $J$ = 7.6 Hz, 1H), 7.53 (dd, $J$ = 7.8, 5.0 Hz, 1H), 7.39 (d, $J$ = 1.2 Hz, 1H), 7.30 (dd, $J$ = 8.0, 1.6 Hz, 1H), 5.75 (s, 2H), 3.95 (t, $J$ = 6.6 Hz, 2H), 3.86 (s, 3H), 2.60 (t, $J$ = 6.8 Hz, 2H), 2.25 - 2.15 (m, 2H), 1.76 - 1.67 (m, 1H), 1.09 - 1.02 (m, 2H), 0.85 - 0.77 (m, 2H). | 583.3 |
| 73 | **Compound 64** | 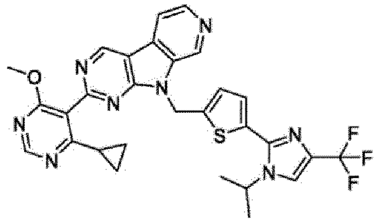 | $^1$H NMR (400 MHz, DMSO-$d_6$): δ 9.79 (s, 1H), 9.47 (d, $J$ = 1.1 Hz, 1H), 8.74 (s, 1H), 8.64 (d, $J$ = 5.2 Hz, 1H), 8.32 (dd, $J$ = 5.2, 1.1 Hz, 1H), 8.09 (d, $J$ = 1.3 Hz, 1H), 7.48 (d, $J$ = 3.8 Hz, 1H), 7.30 (d, $J$ = 3.8 Hz, 1H), 6.02 (s, 2H), 4.78 - 4.62 (m, 1H), 3.90 (s, 3H), 1.81 - 1.70 (m, 1H), 1.39 (d, $J$ = 6.6 Hz, 6H), 1.12 - 1.06 (m, 2H), 0.98 - 0.90 (m, 2H). | 579.1 |
| 74 | **Compound 65** | 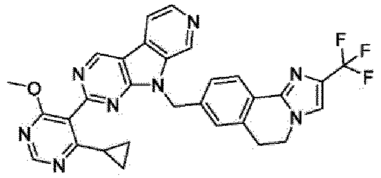 | $^1$H NMR (400 MHz, DMSO-$d_6$): δ 9.81 (s, 1H), 9.26 (d, $J$ = 1.1 Hz, 1H), 8.73 (s, 1H), 8.63 (d, $J$ = 5.2 Hz, 1H), 8.34 (dd, $J$ = 5.2, 1.1 Hz, 1H), 7.84 (d, $J$ = 7.9 Hz, 1H), 7.58 (d, $J$ = 1.5 Hz, 1H), 7.42 (s, 1H), 7.41 (d, $J$ = 7.9 Hz, 1H), 5.82 (s, 2H), 4.20 (t, $J$ = 7.0 Hz, 2H), 3.88 (s, 3H), 3.13 (t, $J$ = 7.0 Hz, 2H), 1.81 - 1.65 (m, 1H), 1.12 - 1.02 (m, 2H), 0.92 - 0.78 (m, 2H). | 569.2 |
| 75 | **Compound 66** | 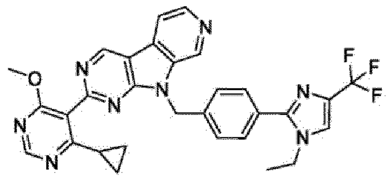 | $^1$H NMR (400 MHz, DMSO-$d_6$): δ 9.82 (s, 1H), 9.29 (s, 1H), 8.72 (s, 1H), 8.64 (d, $J$ = 5.2 Hz, 1H), 8.35 (dd, $J$ = 5.2, 0.8 Hz, 1H), 8.00 (d, $J$ = 0.8 Hz, 1H), 7.58 (d, $J$ = 8.0 Hz, 2H), 7.52 (d, $J$ = 8.4 Hz, 2H), 5.88 (s, 2H), 4.02 (q, $J$ = 7.2 Hz, 2H), 3.88 (s, 3H), 1.78 - 1.69 (m, 1H), 1.27 (t, $J$ = 7.2 Hz, 3H), 1.11 - 1.03 (m, 2H), 0.90 - 0.81 (m, 2H). | 571.3 |
| 76 | **Compound 67** | 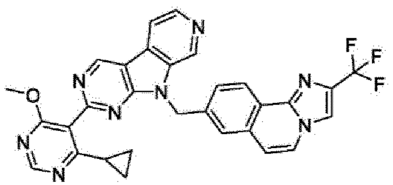 | $^1$H NMR (400 MHz, DMSO-$d_6$): δ 9.84 (s, 1H), 9.28 (s, 1H), 8.71 (s, 1H), 8.64 (d, $J$ = 5.2 Hz, 1H), 8.55 (s, 1H), 8.42 (d, $J$ = 8.4 Hz, 1H), 8.39 - 8.31 (m, 2H), 7.91 (s, 1H), 7.68 (dd, $J$ = 8.4, 1.2 Hz, 1H), 7.32 (d, $J$ = 7.2 Hz, 1H), 6.00 (s, 2H), 3.87 (s, 3H), 1.79 - 1.68 (m, 1H) , 1.09 - 0.98 (m, 2H), 0.83 - 0.71 (m, 2H). | 567.2 |

(continued)

| Example | Compound | Structural formula | $^1$HNMR | MS (ESI) M/Z :$[M+H]^+$ |
|---|---|---|---|---|
| 77 | **Compound 68** | 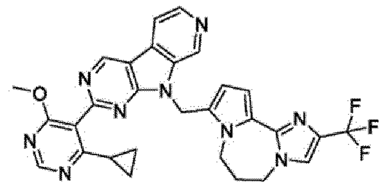 | $^1$H NMR (400 MHz, DMSO-$d_6$): δ 9.81 (s, 1H), 9.25 (d, *J* = 0.4 Hz, 1H), 8.73 (s, 1H), 8.62 (d, *J* = 5.2 Hz, 1H), 8.33 (dd, *J* = 5.2, 1.2 Hz, 1H), 7.73 (d, *J* = 1.2 Hz, 1H), 6.65 (d, *J* = 3.6 Hz, 1H), 6.39 (d, *J* = 4.0 Hz, 1H), 5.86 (s, 2H), 4.22 (t, *J* = 5.8 Hz, 2H), 4.02 (t, *J* = 5.8 Hz, 2H), 3.86 (s, 3H), 2.01 - 1.91 (m, 2H), 1.68 - 1.59 (m, 1H), 1.12 - 1.05 (m, 2H), 0.92 - 0.83 (m, 2H). | 572.0 |
| 78 | **Compound 69** | 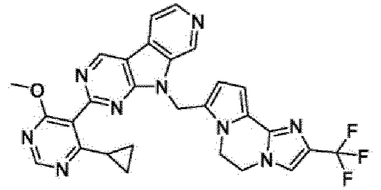 | $^1$H NMR (400 MHz, DMSO-$d_6$): δ 9.79 (s, 1H), 9.33 (s, 1H), 8.74 (s, 1H), 8.63 (d, *J* = 5.2 Hz, 1H), 8.33 (d, *J* = 5.2 Hz, 1H), 7.80 (s, 1H), 6.54 (d, *J* = 3.6 Hz, 1H), 6.43 (d, *J* = 3.6 Hz, 1H), 5.86 (s, 2H), 4.38 (t, *J* = 6.0 Hz, 2H), 4.25 (t, *J* = 5.8 Hz, 2H), 3.87 (s, 3H), 1.68 - 1.59 (m, 1H), 1.14 - 1.04 (m, 2H), 0.91 - 0.83 (m, 2H), | 558.2 |
| 79 | **Compound 70** | 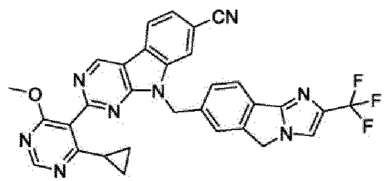 | $^1$H NMR (400 MHz, DMSO-$d_6$): δ 9.82 (s, 1H), 8.72 (s, 1H), 8.58 (d, *J* = 8.0 Hz, 1H), 8.52 (s, 1H), 8.09 (s, 1H), 7.86 (dd, *J* = 8.0, 1.2 Hz, 1H), 7.76 (d, *J* = 8.0 Hz, 1H), 7.63 (s, 1H), 7.58 (d, *J* = 8.0 Hz, 1H), 5.86 (s, 2H), 5.08 (s, 2H), 3.88 (s, 3H), 1.79 - 1.69 (m, 1H), 1.10 - 1.03 (m, 2H), 0.87 - 0.80 (m, 2H). | 579.8 |
| 80 | **Compound 71** | 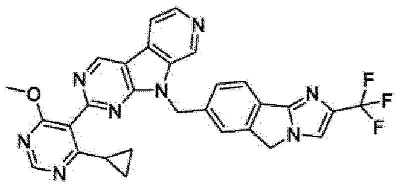 | $^1$H NMR (400 MHz, DMSO-$d_6$): δ 9.82 (s, 1H), 9.28 (s, 1H), 8.73 (s, 1H), 8.63 (d, *J* = 5.2 Hz, 1H), 8.34 (d, *J* = 5.1 Hz, 1H), 8.09 (s, 1H), 7.76 (d, *J* = 7.9 Hz, 1H), 7.65 (s, 1H), 7.59 (d, *J* = 7.9 Hz, 1H), 5.89 (s, 2H), 5.09 (s, 2H), 3.89 (s, 3H), 1.83 - 1.69 (m, 1H), 1.11 - 1.02 (m, 2H), 0.93 - 0.78 (m, 2H). | 555.2 |
| 81 | **Compound 72** | 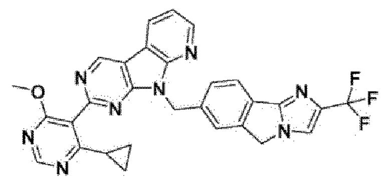 | $^1$H NMR (400 MHz, DMSO-$d_6$): δ 9.70 (s, 1H), 8.78 (dd, *J* = 7.8, 1.6 Hz, 1H), 8.71 (s, 1H), 8.67 (dd, *J* = 4.9, 1.6 Hz, 1H), 8.08 (d, *J* = 1.3 Hz, 1H), 7.73 (d, *J* = 7.9 Hz, 1H), 7.62 (s, 1H), 7.56 - 7.48 (m, 2H), 5.80 (s, 2H), 5.07 (s, 2H), 3.87 (s, 3H), 1.81 - 1.61 (m, 1H), 1.11 - 0.99 (m, 2H), 0.87 - 0.72 (m, 2H). | 555.2 |

(continued)

| Example | Compound | Structural formula | $^1$HNMR | MS (ESI) M/Z :[M+H]$^+$ |
|---|---|---|---|---|
| 82 | **Compound 73** | 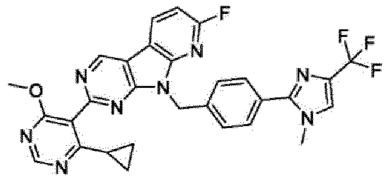 | $^1$H NMR (400 MHz, DMSO-$d_6$): δ 9.70 (s, 1H), 8.94 (t, *J* = 8.0 Hz, 1H), 8.71 (s, 1H), 7.91 (s, 1H), 7.65 (d, *J* = 8.0 Hz, 2H), 7.48 (d, *J* = 8.0 Hz, 2H), 7.28 (d, *J* = 8.0 Hz, 1H), 5.72 (s, 2H), 3.86 (s, 3H), 3.73 (s, 3H), 1.69 - 1.73 (m, 1H), 1.05 - 1.07 (m, 2H), 0.82 - 0.85 (m, 2H). | 575.2 |
| 83 | **Compound 74** | 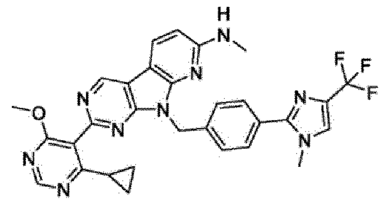 | $^1$H NMR (400 MHz, DMSO-$d_6$): δ 9.27 (s, 1H), 8.68 (s, 1H), 8.21 (d, *J* = 8.4 Hz, 1H), 7.89 (d, *J* = 1.2 Hz, 1H), 7.65 (d, *J* = 8.0 Hz, 2H), 7.54 (d, *J* = 8.0 Hz, 2H), 7.28 - 7.31 (m, 1H), 6.54 (d, *J* = 8.4 Hz, 1H), 5.62 (s, 2H), 3.86 (s, 3H), 3.72 (s, 3H), 2.95 (s, 3H), 1.69 - 1.73 (m, 1H), 1.02 - 1.05 (m, 2H), 0.82 - 0.85 (m, 2H). | 586.3 |
| 84 | **Compound 84** | 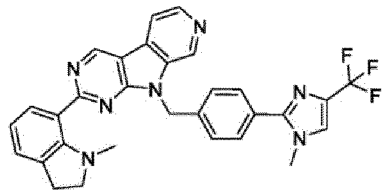 | $^1$H NMR (400 MHz, DMSO-*d6*) δ 9.71 (s, 1H), 9.15 (d, *J* = 1.0 Hz, 1H), 8.59 (d, *J* = 5.2 Hz, 1H), 8.30 (dd, *J* = 5.2, 1.1 Hz, 1H), 7.90 (d, *J* = 1.3 Hz, 1H), 7.74 - 7.63 (m, 2H), 7.53 - 7.45 (m, 3H), 7.16 (dd, *J* = 7.0, 1.3 Hz, 1H), 6.74 (dd, *J* = 7.8, 7.0 Hz, 1H), 5.88 (s, 2H), 3.73 (s, 3H), 3.45 - 3.38 (m, 2H), 2.99 (t, *J* = 8.4 Hz, 2H), 2.40 (s, 3H). | 540.2 |
| 85 | **Compound 85** | 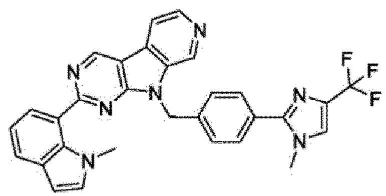 | $^1$H NMR (400 MHz, DMSO-*d6*) δ 9.82 (s, 1H), 9.18 (d, J = 1.1 Hz, 1H), 8.63 (d, *J* = 5.1 Hz, 1H), 8.36 (dd,*J* = 5.2, 1.1 Hz, 1H), 7.90 (d, *J* = 1.3 Hz, 1H), 7.72 (dd, *J* = 7.8, 1.2 Hz, 1H), 7.68 (d, *J* = 8.4 Hz, 2H), 7.52 (dd, *J* = 7.4, 1.2 Hz, 1H), 7.44 (d, *J* = 8.4 Hz, 2H), 7.34 (d, *J* = 3.1 Hz, 1H), 7.17 (t, *J* = 7.6 Hz, 1H), 6.57 (d, *J* = 3.1 Hz, 1H), 5.93 (s, 2H), 3.73 (s, 3H), 3.43 (s, 3H). | 538.2 |
| 86 | **Compound 86** | 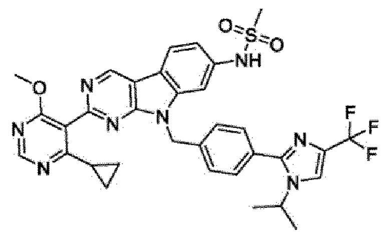 | $^1$H NMR (400 MHz, DMSO-*d6*) δ 9.90 (s, 1H), 9.56(s, 1H), 8.70 (s, 1H), 8.29 (d, *J* = 8.5 Hz, 1H), 8.15 (d, *J* = 1.4 Hz, 1H), 7.56 (d, *J* = 1.9 Hz, 1H), 7.51 (d,*J* = 8.3 Hz, 2H), 7.44 (d, *J* = 8.3 Hz, 2H), 7.25 (dd, *J* = 8.4, 1.9 Hz, 1H), 5.74 (s, 2H), 4.45 - 4.33 (m, 1H), 3.87 (s, 3H), 3.03 (s, 3H), 1.78 - 1.66 (m, 1H), 1.35 (d, J = 6.6 Hz, 6H), 1.11 - 1.01 (m, 2H), 0.89 - 0.77 (m, 2H). | 677.3 |

(continued)

| Example | Compound | Structural formula | $^1$HNMR | MS (ESI) M/Z :[M+H]$^+$ |
|---|---|---|---|---|
| 87 | **Compound 87** | 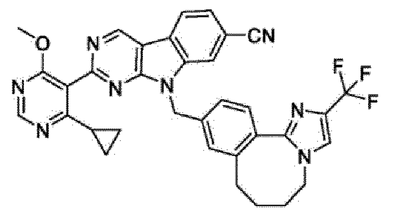 | $^1$H NMR (400 MHz, DMSO-*d6*) δ 9.82 (s, 1H), 8.72 (s, 1H), 8.58 (d, *J* = 8.3 Hz, 1H), 8.56 (s, 1H), 7.95 (d, *J* = 1.3 Hz, 1H), 7.86 (dd, *J* = 8.1, 1.3 Hz, 1H), 7.55 (d, *J* = 7.9 Hz, 1H), 7.53 (s, 1H), 7.29 (dd, *J* = 8.0, 1.7 Hz, 1H), 5.84 (s, 2H), 4.20 - 4.10 (m, 1H), 3.86 (s, 3H), 3.71 - 3.57 (m, 1H), 2.81 - 2.65 (m, 1H), 2.47 - 2.35 (m, 1H), 1.85 - 1.74 (m, 1H), 1.74 - 1.63 (m, 1H), 1.11 (d, *J* = 6.9 Hz, 3H), 1.09 - 1.03 (m, 2H), 0.87 - 0.79 (m, 2H). | 586.3 |
| 88 | **Compound 90** | 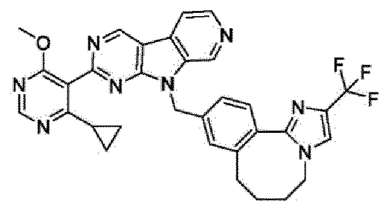 | $^1$H NMR (400 MHz, DMSO-$d_6$) δ 9.82 (s, 1H), 9.30 (d, *J* = 0.4 Hz, 1H), 8.72 (s, 1H), 8.63 (d, *J* = 5.2 Hz, 1H), 8.35 (dd, *J* = 5.2, 1.2 Hz, 1H), 7.95 (d, *J* = 1.2 Hz, 1H), 7.56 (d, *J* = 8.0 Hz, 1H), 7.53 (s, 1H), 7.31 (dd, *J* =8.0, 1.6 Hz, 1H), 5.87 (s, 2H), 4.20 - 4.10 (m, 1H), 3.87 (s, 3H), 3.70-3.59 (m, 1H), 2.80 - 2.69 (m, 1H), 2.47 - 2.36 (m, 1H), 1.87 - 1.77 (m, 1H), 1.75 - 1.65 (m, 1H), 1.15 - 1.04 (m, 5H), 0.89 - 0.79 (m, 2H). | 597.2 |
| 89 | **Compound 91** | 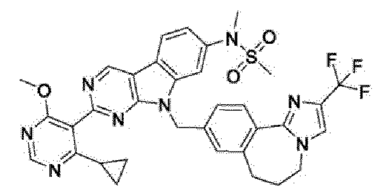 | $^1$H NMR (400 MHz, DMSO-$d_6$) δ 9.65 (s, 1H), 8.71 (s, 1H), 8.37 (d, *J* = 8.4 Hz, 1H), 7.98 - 7.92 (m, 2H), 7.59 (d, *J* = 8.0 Hz, 1H), 7.51 - 7.44 (m, 2H), 7.32 (dd, *J* = 7.8, 1.4 Hz, 1H), 5.76 (s, 2H), 3.94 (t, *J* = 6.8 Hz, 2H), 3.87 (s, 3H), 3.36 (s, 3H), 2.99 (s, 3H), 2.60 (t, *J* = 6.8 Hz, 2H), 2.26 - 2.15 (m, 2H), 1.75 - 1.66 (m, 1H), 1.11 - 1.01 (m, 2H), 0.89 - 0.78 (m, 2H). | 689.2 |
| 90 | **Compound 92** | 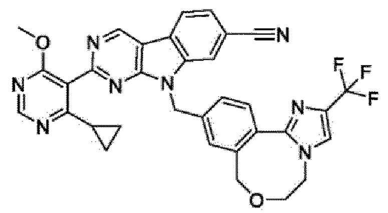 | $^1$H NMR (400 MHz, DMSO-d6) δ 9.82 (s, 1H), 8.71 (s, 1H), 8.59 (d, *J* = 8.1 Hz, 1H), 8.50 (s, 1H), 8.48 (s, 1H), 7.86 (dd, *J* = 8.1, 1.1 Hz, 1H), 7.59 (s, 1H), 7.40 (d, *J* = 7.8 Hz, 1H), 7.30 (d, *J* = 7.9 Hz, 1H), 6.58 (dd, *J* = 15.6, 8.9 Hz, 1H), 5.87 (s, 2H), 5.66 (dd, *J* = 15.6, 1.2 Hz, 1H), 5.07 (t, *J* = 5.4 Hz, 1H), 4.98 (dd, *J* = 8.8, 1.2 Hz, 1H), 4.27 (d, *J* = 5.3 Hz, 2H), 3.86 (s, 3H), 1.78 - 1.68 (m, 1H), 1.11 - 0.99 (m, 2H), 0.90 - 0.78 (m, 2H). | 623.3 |
| 91 | **Compound 93** | 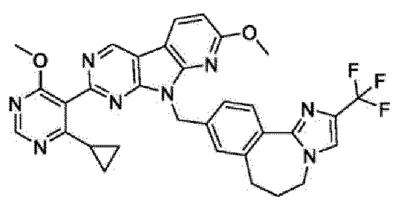 | $^1$H NMR (400 MHz, DMSO-*d6*) δ 9.53 (s, 1H), 8.71 (s, 1H), 8.61 (d, *J* = 8.5 Hz, 1H), 7.95 (d, *J* = 1.2 Hz, 1H), 7.62 (d, *J* = 7.9 Hz, 1H), 7.48 (d, *J* = 1.7 Hz, 1H), 7.41 (dd, *J* = 7.9, 1.7 Hz, 1H), 6.91 (d, *J* = 8.5 Hz, 1H), 5.67 (s, 2H), 4.05 (s, 3H), 3.95 (t, *J* = 6.8 Hz, 2H), 3.86 (s, 3H), 2.61 (t, *J* = 7.0 Hz, 2H), 2.26 - 2.15 (m, 2H), 1.78 - 1.66 (m, 1H), 1.13 - 1.00 (m, 2H), 0.89 - 0.78 (m, 2H). | 613.3 |

(continued)

| Example | Compound | Structural formula | $^1$HNMR | MS (ESI) M/Z :[M+H]$^+$ |
|---|---|---|---|---|
| 92 | **Compound 94** | 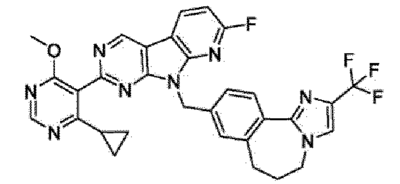 | $^1$H NMR (400 MHz, DMSO-*d6*) δ 9.71 (s, 1H), 8.94 (t, *J* = 8.0 Hz, 1H), 8.71 (s, 1H), 7.95 (d, *J* = 1.2 Hz, 1H), 7.60 (d, *J* = 7.9 Hz, 1H), 7.37 (d, *J* = 1.8 Hz, 1H), 7.29 (d, *J* = 8.0 Hz, 2H), 5.68 (s, 2H), 3.95 (t, *J* = 6.8 Hz, 2H), 3.85 (s, 3H), 2.60 (t, *J* = 7.0 Hz, 2H), 2.27 - 2.15 (m, 2H), 1.75 - 1.65 (m, 1H), 1.09 - 1.01 (m, 2H), 0.85 - 0.75 (m, 2H). | 601.2 |
| 93 | **Compound 95** | 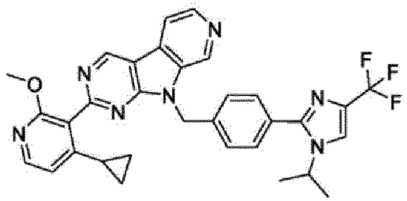 | $^1$H NMR (400 MHz, DMSO-*d6*) δ 9.81 (s, 1H), 9.30 (s, 1H), 8.64 (d, *J* = 5.2 Hz, 1H), 8.36 (d, *J* = 5.2 Hz, 1H), 8.15 (d, *J* = 4.9 Hz, 2H), 7.54 - 7.44 (m, 4H), 7.25 (d, J = 5.1 Hz, 1H), 5.90 (s, 2H), 4.46 - 4.31 (m, 1H), 3.94 (s, 3H), 1.96 - 1.85 (m, 1H), 1.35 (d, J = 6.6 Hz, 6H), 0.49 - 0.39 (m, 2H), 0.14 - 0.06 (m, 2H). | 584.2 |
| 94 | **Compound 96** | 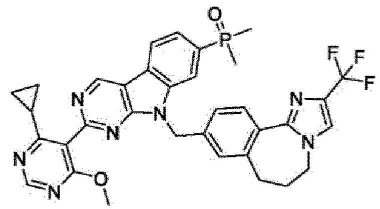 | $^1$H NMR (400 MHz, DMSO-*d6*) δ 9.75 (s, 1H), 8.71 (s, 1H), 8.52 (dd, *J* = 8.0, 2.3 Hz, 1H), 8.21 (d, *J* = 12.3 Hz, 1H), 7.95 (d, *J* = 1.4 Hz, 1H), 7.82 (ddd, *J* = 11.0, 7.9, 1.1 Hz, 1H), 7.60 (d, *J* = 7.9 Hz, 1H), 7.41 (d, *J* = 1.8 Hz, 1H), 7.23 (dd, *J* = 8.0, 1.8 Hz, 1H), 5.83 (s, 2H), 3.95 (t, *J* = 6.7 Hz, 2H), 3.87 (s, 3H), 2.60 (t, *J* = 7.0 Hz, 2H), 2.26 - 2.14 (m, 2H), 1.81 - 1.65 (m, 7H), 1.14 - 0.98 (m, 2H), 0.90 - 0.75 (m, 2H). | 658.2 |
| 95 | **Compound 97** | 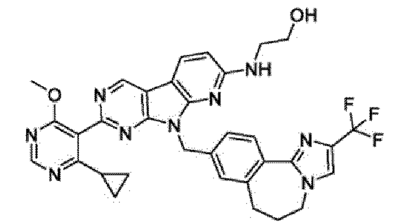 | $^1$H NMR (400 MHz, DMSO-d6) δ 9.25 (s, 1H), 8.68 (s, 1H), 8.17 (d, J = 8.6 Hz, 1H), 7.95 (d, *J* = 1.4 Hz, 1H), 7.59 (d, *J* = 7.9 Hz, 1H), 7.46 (d, *J* = 1.7 Hz, 1H), 7.42 - 7.31 (m, 2H), 6.58 (d, *J* = 8.6 Hz, 1H), 5.56 (s, 2H), 4.81 - 4.71 (m, 1H), 3.95 (t, *J* = 6.7 Hz, 2H), 3.86 (s, 3H), 3.68 - 3.58 (m, 2H), 3.55 - 3.46 (m, 2H), 2.61 (t, *J* = 7.0 Hz, 2H), 2.28 - 2.17 (m, 2H), 1.76 - 1.64 (m, 1H), 1.10 - 1.00 (m, 2H), 0.89 - 0.75 (m, 2H). | 642.2 |

(continued)

| Example | Compound | Structural formula | $^1$HNMR | MS (ESI) M/Z :[M+H]$^+$ |
|---|---|---|---|---|
| 96 | **Compound 98** | 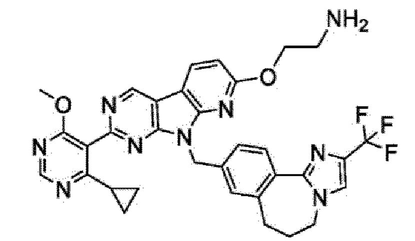 | 1H NMR (400 MHz, DMSO-d6) δ 9.54 (s, 1H), 8.71 (s, 1H), 8.61 (d, *J* = 8.5 Hz, 1H), 7.95 (d, *J* = 1.4 Hz, 1H), 7.62 (d, *J* = 7.9 Hz, 1H), 7.46 (d, *J* = 1.7 Hz, 1H), 7.40 (dd, *J* = 8.0, 1.8 Hz, 1H), 6.91 (d, *J* = 8.5 Hz, 1H), 5.67 (s, 2H), 4.47 (t, *J* = 5.8 Hz, 2H), 3.95 (t, *J* = 6.8 Hz, 2H), 3.86 (s, 3H), 3.04 (t, *J* = 5.7 Hz, 2H), 2.61 (t, *J* = 7.0 Hz, 2H), 2.22 (t, *J* = 6.9 Hz, 2H), 1.75 - 1.66 (m, 1H), 1.10 - 1.02 (m, 2H), 0.89 - 0.76 (m, 2H). | 642.3 |
| 97 | **Compound 99** | 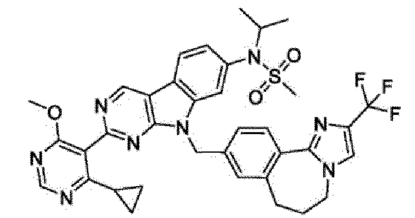 | $^1$H NMR (401 MHz, DMSO-*d6*) δ 9.68 (s, 1H), 8.72 (s, 1H), 8.39 (d, *J* = 8.3 Hz, 1H), 7.95 (d, *J* = 1.4 Hz, 1H), 7.77 (d, *J* = 1.8 Hz, 1H), 7.57 (d, *J* = 7.9 Hz, 1H), 7.49 (d, *J* = 1.7 Hz, 1H), 7.37 - 7.29 (m, 2H), 5.80 (s, 2H), 4.47 - 4.34 (m, 1H), 3.92 (t, *J* = 6.7 Hz, 2H), 3.88 (s, 3H), 3.16 (s, 3H), 2.58 (t, *J* = 7.0 Hz, 2H), 2.19 (p, *J* = 6.9 Hz, 2H), 1.75 - 1.66 (m, 1H), 1.11 - 1.01 (m, 8H), 0.89 - 0.81 (m, 2H). | 717.2 |
| 98 | **Compound 100** | 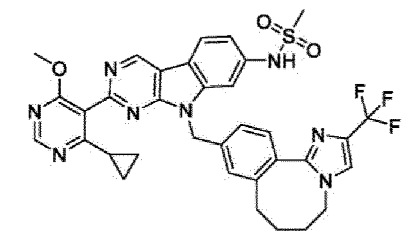 | $^1$H NMR (400 MHz, DMSO-*d6*) δ 10.05 (s, 1H), 9.56 (s, 1H), 8.70 (s, 1H), 8.28 (d, *J* = 8.4 Hz, 1H), 7.96 (d, *J* = 1.4 Hz, 1H), 7.59 (d, *J* = 1.9 Hz, 1H), 7.55 (d, *J* = 7.9 Hz, 1H), 7.48 (s, 1H), 7.26 - 7.20 (m, 2H), 5.81 - 5.63 (m, 2H), 4.22 - 4.07 (m, 1H), 3.86 (s, 3H), 3.72 - 3.57 (m, 1H), 2.78 - 2.69 (m, 1H), 2.47 - 2.39 (m, 1H), 1.88 - 1.76 (m, 1H), 1.74 - 1.61 (m, 1H), 1.19 - 1.10 (m, 3H), 1.10 - 1.01 (m, 2H), 0.91 - 0.80 (m, 2H). | 689.3 |
| 99 | **Compound 102** | 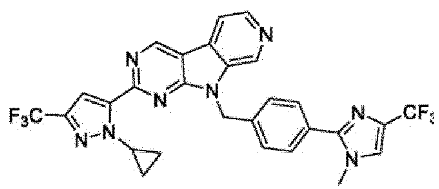 | $^1$H NMR (400 MHz, DMSO-$d_6$) δ 9.82 (s, 1H), 9.25 (s, 1H), 8.64 (d, *J* = 5.2 Hz, 1H), 8.35 (d, *J* = 5.2 Hz, 1H), 7.90 (s, 1H), 7.68 (d, *J* = 8.0 Hz, 2H), 7.54 - 7.50 (m, 3H), 5.96 (s, 2H), 4.89 - 4.85 (m, 1H), 3.72 (s, 3H), 1.19 - 1.17 (m, 2H), 1.06 - 1.03 (m, 2H). | 584.3 |

(continued)

| Example | Compound | Structural formula | $^1$HNMR | MS (ESI) M/Z :[M+H]$^+$ |
|---|---|---|---|---|
| 100 | **Compound 103** | 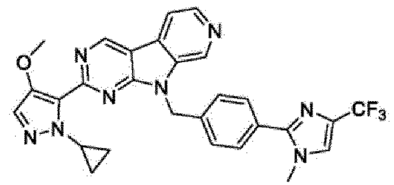 | $^1$H NMR (400 MHz, DMSO-$d_6$) δ 9.75 (s, 1H), 9.27 (s, 1H), 8.61 (d, *J* = 5.2 Hz, 1H), 8.29 (d, *J* = 5.2 Hz, 1H), 7.90 (s, 1H), 7.69 (d, *J* = 1.2 Hz, 2H), 7.56 (d, *J* = 8.4 Hz, 2H), 7.47 (s, 1H), 5.89 (s, 2H), 4.44 - 4.38 (m, 1H), 3.83 (s, 3H), 3.72 (s, 3H), 1.0 - 0.96 (m, 2H), 0.81 - 0.76 (m, 2H). | 544.3 |
| 101 | **Compound 104** | 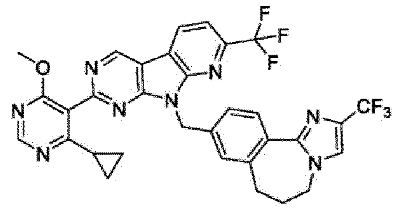 | $^1$H NMR (400 MHz, DMSO-*d6*) δ 9.84 (s, 1H), 9.05 (d,*J* = 8.0 Hz, 1H), 8.72 (s, 1H), 8.03 (d, *J* = 8.0 Hz, 1H), 7.95 (d, *J* = 4 .0Hz, 1H), 7.59 (d, *J* = 8.0 Hz, 1H), 7.44 (d, *J* = 4 .0Hz, 1H), 7.31 (dd, *J* = 8.0, 4.0 Hz, 1H), 5.75 (s, 2H), 3.95 (t, *J* = 8.0 Hz, 2H), 3.86 (s, 3H), 2.59 (t, *J* = 8.0 Hz, 2H), 2.25 - 2.15 (m, 2H), 1.76 - 1.67 (m, 1H), 1.08 - 1.03 (m, 2H), 0.84 - 0.78 (m, 2H). | 651.2 |
| 102 | **Compound 105** | 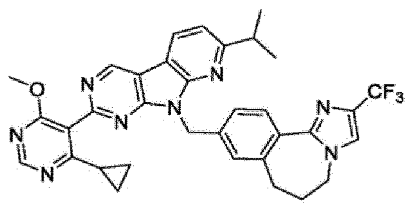 | $^1$H NMR (400 MHz, DMSO-*d6*) δ 9.61 (s, 1H), 8.72 (s, 1H), 8.64 (d, *J* = 8.0 Hz, 1H), 7.95 (d, *J* = 4.0 Hz, 1H), 7.62 - 7.51 (m, 2H), 7.46 - 7.36 (m, 2H), 5.71 (s, 2H), 3.94 (t, *J* = 8.0 Hz, 2H), 3.87 (s, 3H), 2.60 (t, *J* = 8.0 Hz, 2H), 2.27 - 2.13 (m, 2H), 1.77 - 1.68 (m, 1H), 1.37 (d, *J* = 8.0 Hz, 6H), 1.12 - 1.02 (m, 3H), 0.89 - 0.80 (m, 2H), | 626.2 |
| 103 | **Compound 109** | 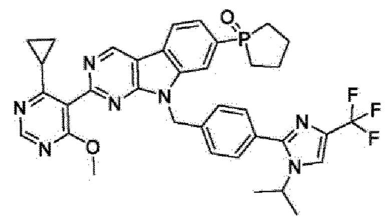 | $^1$H NMR (400 MHz, DMSO-$d_6$) δ 9.77 (s, 1H), 8.71 (s, 1H), 8.54 (dd, *J* = 8.0, 2.0 Hz, 1H), 8.18 (d, *J* = 12.0 Hz, 1H), 8.15 (d, *J* = 1.2 Hz, 1H), 7.77 (dd, *J* = 10.0, 8.4 Hz, 1H), 7.50 (d, *J* = 8.4 Hz, 2H), 7.43 (d, *J* = 8.0 Hz, 2H), 5.92 (s, 2H), 4.42 - 4.33 (m, 1H), 3.87 (m, 3H), 2.13 - 1.87 (m, 8H), 1.76 - 1.68 (m, 1H), 1.35 (d, *J* = 6.8 Hz, 6H), 1.09 - 1.01 (m, 2H), 0.87 - 0.79 (m, 2H). | 686.2 |
| 104 | **Compound 111** | 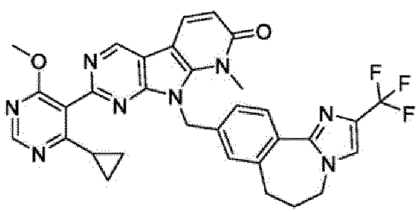 | $^1$H NMR (400 MHz, DMSO-$d_6$) δ 9.27 (s, 1H), 8.83 (s, 1H), 7.96 (d, *J* = 0.8 Hz, 1H), 7.81 (d, *J* = 8.8 Hz, 1H), 7.58 (d,*J* = 8.0 Hz, 1H), 7.31 (s, 1H), 7.24 (dd, *J* = 8.0, 1.6 Hz, 1H), 6.10 (d, *J* = 8.8 Hz, 1H), 5.55 (d, *J* = 15.6 Hz, 1H), 5.50 (d, *J* = 15.6 Hz, 1H), 3.99 (s, 3H), 3.97 - 3.91 (m, 5H), 2.59 (t, *J* = 7.0 Hz, 2H), 2.26 - 2.16 (m, 2H), 1.84 - 1.74 (m, 1H), 1.19 - 1.05 (m, 2H), 0.98 - 0.89 (m, 1H), 0.83 - 0.73 (m, 1H), | 613.2 |

(continued)

| Example | Compound | Structural formula | $^1$HNMR | MS (ESI) M/Z :[M+H]$^+$ |
|---|---|---|---|---|
| 105 | **Compound 112** | 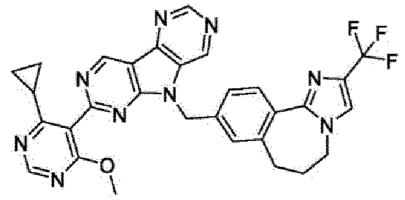 | $^1$H NMR (400 MHz, DMSO-$d_6$) δ 9.85 (s, 1H), 9.50 (s, 1H), 9.30 (s, 1H), 8.73 (s, 1H), 7.96 (s, 1H), 7.62 (d, *J* = 8.0 Hz, 1H), 7.44 (s, 1H), 7.37 (d, *J* = 6.8 Hz, 1H), 5.85 (s, 2H), 3.95 (t, *J* = 6.6 Hz, 2H), 3.88 (s, 3H), 2.61 (t, *J* = 6.8 Hz, 2H), 2.26 - 2.15 (m, 2H), 1.80 - 1.71 (m, 1H), 1.11 - 1.04 (m, 2H), 0.89 - 0.80 (m, 2H). | 584.3 |
| 106 | **Compound 113** | 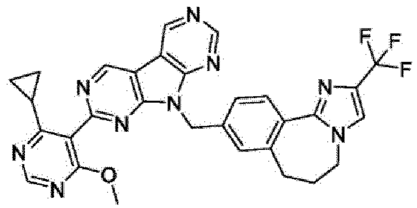 | $^1$H NMR (400 MHz, DMSO-$d_6$) δ 9.79 (s, 1H), 9.69 (s, 1H), 9.21 (s, 1H), 8.72 (s, 1H), 7.96 (s, 1H), 7.61 (d, *J* = 8.0 Hz, 1H), 7.39 (s, 1H), 7.33 (dd, *J* = 8.0, 1.2 Hz, 1H), 5.73 (s, 2H), 3.95 (t, *J* = 6.6 Hz, 2H), 3.86 (s, 3H), 2.60 (t, *J* = 7.0 Hz, 2H), 2.26 - 2.15 (m, 2H),1.76 - 1.67 (m, 1H), 1.09 - 1.02 (m, 2H), 0.85 - 0.76 (m, 2H). | 584.2 |
| 107 | **Compound 116** | 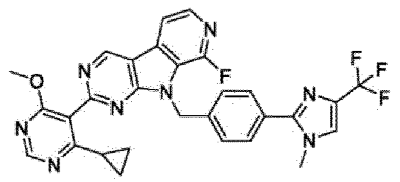 | $^1$H NMR (400 MHz, DMSO-$d_6$) δ 9.90 (s, 1H), 8.73 (s, 1H), 8.35 (dd, *J* = 5.2, 2.8 Hz, 1H), 8.19 (dd, *J* = 5.4, 1.8 Hz, 1H), 7.91 (d, *J* = 1.2 Hz, 1H), 7.66 (d, *J* = 8.4 Hz, 2H), 7.41 (d, *J* = 8.4 Hz, 2H), 5.87 (s, 2H), 3.88 (s, 3H), 3.73 (s, 3H), 1.81 - 1.65 (m, 1H), 1.14 - 1.02 (m, 2H), 0.93 - 0.77 (m, 2H). | 575.2 |
| 108 | **Compound 117** | 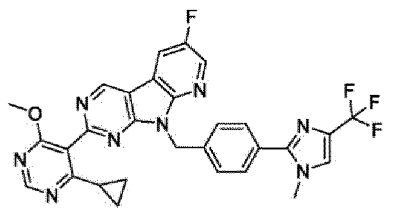 | $^1$H NMR (400 MHz, DMSO-$d_6$) δ 9.70 (s, 1H), 8.78 (dd, *J* = 8.6, 2.6 Hz, 1H), 8.74 - 8.68 (m, 2H), 7.90 (s, 1H), 7.64 (d, *J* = 8.4 Hz, 2H), 7.48 (d, *J* = 8.4 Hz, 2H), 5.77 (s, 2H), 3.87 (s, 3H), 3.72 (s, 3H), 1.77 - 1.68 (m, 1H), 1.10 - 1.02 (m, 2H), 0.88 - 0.80 (m, 2H). | 575.2 |
| 109 | **Compound 119** | 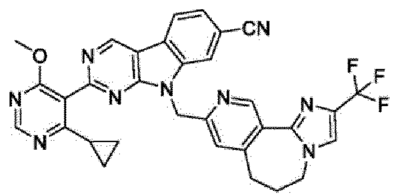 | $^1$H NMR (400 MHz, DMSO-$d_6$) δ 9.81 (s, 1H), 8.69 (s, 1H), 8.60 (d, *J* = 8.4 Hz, 1H), 8.45 (s, 1H), 8.14 (d, *J* = 8.0 Hz, 1H), 8.00 (d, *J* = 0.8 Hz, 1H), 7.86 (dd, *J* = 8.0, 1.2 Hz, 1H), 7.13 (d, *J* = 8.0 Hz, 1H), 5.90 (s, 2H), 4.10 (t, *J* = 6.6 Hz, 2H), 3.82 (s, 3H), 2.84 (t, *J* = 6.8 Hz, 2H), 2.28 - 2.18 (m, 2H), 1.69 - 1.60 (m, 1H), 1.05 - 0.98 (m, 2H), 0.80 - 0.72 (m, 2H). | 608.2 |

(continued)

| Example | Compound | Structural formula | $^1$HNMR | MS (ESI) M/Z :$[M+H]^+$ |
|---|---|---|---|---|
| 110 | **Compound 121** | 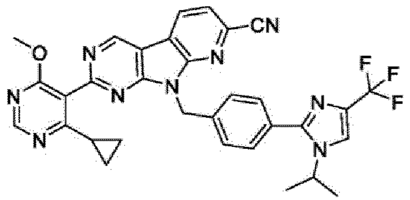 | $^1$H NMR (400 MHz, DMSO-$d_6$) δ 9.84 (s, 1H), 9.02 (d, *J* = 7.9 Hz, 1H), 8.72 (s, 1H), 8.15 (dd, *J* = 4.6, 3.2 Hz, 2H), 7.51 (s, 4H), 5.79 (s, 2H), 4.45 - 4.34 (m, 1H), 3.86 (s, 3H), 1.78 - 1.68 (m, 1H), 1.36 (d, *J* = 6.6 Hz, 6H), 1.09 - 1.02 (m, 2H), 0.86 - 0.77 (m, 2H). | 610.2 |
| 111 | **Compound 122** | 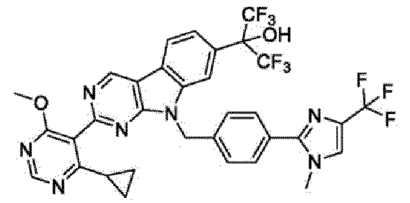 | $^1$HNMR (400 MHz, DMSO-$d_6$) δ 9.73 (s, 1H), 8.96 (s, 1H), 8.71 (s, 1H), 8.50 (d, *J* = 8.4 Hz, 1H), 8.06 (s, 1H), 7.90 (d, *J* = 1.2 Hz, 1H), 7.73 (d, *J* = 8.4 Hz, 1H), 7.65 (d, *J* = 8.4 Hz, 2H), 7.44 (d, *J* = 8.0 Hz, 2H), 5.85 (s, 2H), 3.88 (s, 3H), 3.71 (s, 3H), 1.78 - 1.68 (m, 1H), 1.11 - 1.03 (m, 2H), 0.90 - 0.80 (m, 2H). | 722.2 |
| 112 | **Compound 124** | 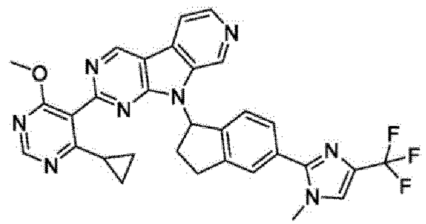 | $^1$H NMR (400 MHz, DMSO-$d_6$): δ 9.82 (s, 1H), 8.71 (s, 1H), 8.58 (d, *J* = 5.2 Hz, 1H), 8.53 - 8.22 (m, 2H), 7.94 (s, 1H), 7.80 (s, 1H), 7.48 (d, *J* = 8.0 Hz, 1H), 7.03 (d, *J* = 7.6 Hz, 1H), 6.92 (t, *J* = 8.4 Hz, 1H), 3.86 (s, 3H), 3.79 (s, 3H), 3.47 - 3.36 (m, 1H), 3.27 - 3.14 (m, 1H), 2.86 - 2.75 (m, 1H), 2.71 - 2.57 (m, 1H), 1.80 - 1.68 (m, 1H), 1.12 - 1.00 (m, 2H), 0.93 - 0.80 (m, 2H). | 583.3 |
| 113 | **Compound 125** | 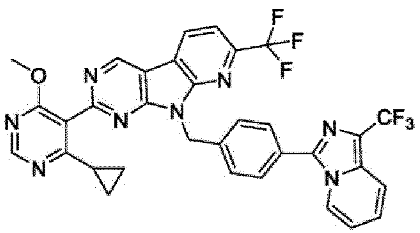 | $^1$H NMR (400 MHz, DMSO-$d_6$) δ 9.85 (s, 1H), 9.06 (d, *J* = 7.9 Hz, 1H), 8.72 (s, 1H), 8.47 (dd, *J* = 7.3, 1.1 Hz, 1H), 8.04 (d, *J* = 8.0 Hz, 1H), 7.81 - 7.75 (m, 2H), 7.75 - 7.67 (m, 1H), 7.63 - 7.56 (m, 2H), 7.19 (dd, *J* = 9.3, 6.5 Hz, 1H), 6.93 - 6.85 (m, 1H), 5.82 (s, 2H), 3.87 (s, 3H), 1.80 - 1.69 (m, 1H), 1.10 - 1.02 (m, 2H), 0.88 - 0.79 (m, 2H). | 661.5 |

(continued)

| Example | Compound | Structural formula | $^1$HNMR | MS (ESI) M/Z :[M+H]$^+$ |
|---|---|---|---|---|
| 114 | **Compound 126** | 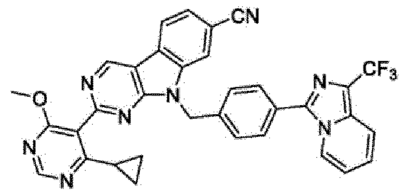 | $^1$H NMR (400 MHz, DMSO-$d_6$) δ 9.82 (s, 1H), 8.72 (s, 1H), 8.59 (d, 7.8 Hz, 2H), 8.47 (d, *J* = 7.4 Hz, 1H), 7.87 (dd, *J* = 8.3, 1.2 Hz, 1H), 7.82 - 7.76 (m, 2H), 7.71 (dd, *J* = 9.3, 1.2 Hz, 1H), 7.65 7.57 (m, 2H), 7.19 (dd, *J* = 9.3, 6.5 Hz, 1H), 6.96 - 6.83 (m, 1H), 5.88 (s, 2H), 3.88 (s, 3H), 1.79 - 1.70 (m, 1H), 1.10 - 1.03 (m, 2H), 0.90 - 0.81 (m, 2H). | 617.5 |
| 115 | **Compound 127** | 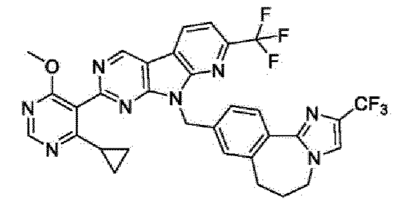 | $^1$H NMR (400 MHz, DMSO-$d_6$) δ 9.84 (s, 1H), 9.05 (d, *J* = 8.0 Hz, 1H), 8.72 (s, 1H), 8.03 (d, *J* = 8.0 Hz, 1H), 7.95 (d, *J* = 4 .0Hz, 1H), 7.59 (d, *J* = 8.0 Hz, 1H), 7.44 (d, *J* = 4 .0Hz, 1H), 7.31 (dd, *J* = 8.0, 4.0 Hz, 1H), 5.75 (s, 2H), 3.95 (t, *J* = 8.0 Hz, 2H), 3.86 (s, 3H), 2.59 (t, *J* = 8.0 Hz, 2H), 2.25 - 2.15 (m, 2H), 1.76 - 1.67 (m, 1H), 1.08 - 1.03 (m, 2H), 0.84 - 0.78 (m, 2H). | 651.2 |
| 116 | **Compound 128** | 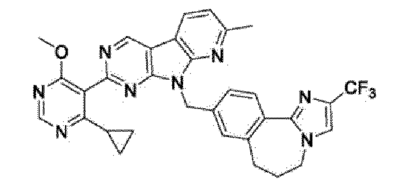 | $^1$H NMR (400 MHz, DMSO--$d_6$) δ 9.62 (s, 1H), 8.69 (s, 1H), 8.64 (d, J = 8.0 Hz, 1H), 7.95 (q, J = 4.0 Hz, 1H), 7.58 (d, J = 8.0 Hz, 1H), 7.42 - 7.34 (m, 2H), 7.25 (dd, J = 8.0, 4.0 Hz, 1H), 5.72 (s, 2H), 3.95 (t, J = 8.0 Hz, 2H), 3.84 (s, 3H), 2.68 (s, 3H), 2.59 (d, J =8.0 Hz, 2H), 2.25 - 2.15 (m, 2H), 1.72 - 1.64 (m, 1H), 1.06 - 0.99 (m, 2H), 0.83 - 0.75 (m, 2H). | 597.6 |
| 117 | **Compound 129** | 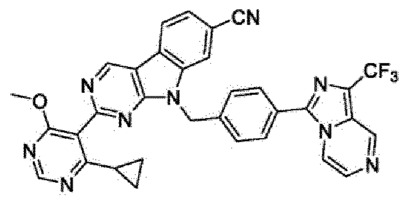 | $^1$H NMR (400 MHz, Methanol-$d_4$) δ 9.66 (s, 1H), 9.18 (s, 1H), 8.64 (s, 1H), 8.52 (dd, J = 8.1, 0.7 Hz, 1H), 8.37 (dd, J = 5.2, 1.6 Hz, 1H), 8.20 (dd, J = 1.4, 0.7 Hz, 1H), 7.87 - 7.80 (m, 2H), 7.77 (dd, J = 8.1, 1.4 Hz, 1H), 7.72 (d, J = 5.2 Hz, 1H), 7.61 (d, J = 8.3 Hz, 2H), 5.92 (s, 2H), 3.94 (s, 3H), 1.81 - 1.72 (m, 1H), 1.16 (m, 2H), 0.92 - 0.86 (m, 2H). | 618.5 |
| 118 | **Compound 130** | 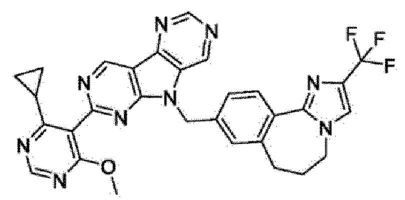 | $^1$H NMR (400 MHz, DMSO-$d_6$) δ 9.85 (s, 1H), 9.50 (s, 1H), 9.30 (s, 1H), 8.73 (s, 1H), 7.96 (s, 1H), 7.62 (d, *J* = 8.0 Hz, 1H), 7.44 (s, 1H), 7.37 (d, *J* = 6.8 Hz, 1H), 5.85 (s, 2H), 3.95 (t, *J* = 6.6 Hz, 2H), 3.88 (s, 3H), 2.61 (t, *J* = 6.8 Hz, 2H), 2.26 - 2.15 (m, 2H), 1.80 - 1.71 (m, 1H), 1.11 - 1.04 (m, 2H), 0.89 - 0.80 (m, 2H). | 584.3 |

(continued)

| Example | Compound | Structural formula | $^1$HNMR | MS (ESI) M/Z :[M+H]$^+$ |
|---|---|---|---|---|
| 119 | **Compound 132** | 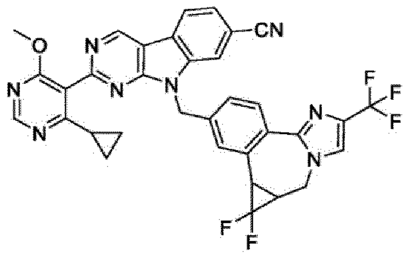 | $^1$H NMR (400 MHz, DMSO-$d_6$) δ 9.82 (s, 1H), 8.72 (s, 1H), 8.58 (d, *J* = 8.1 Hz, 1H), 8.53 (d, *J* = 1.1 Hz, 1H), 7.99 (t, *J* = 1.4 Hz, 1H), 7.86 (dd, *J* = 8.1, 1.3 Hz, 1H), 7.65 (d, *J* = 8.1 Hz, 1H), 7.62 - 7.58 (m, 1H), 7.40 (dd, *J* = 8.2, 1.8 Hz, 1H), 5.91 - 5.77 (m, 2H), 4.65 (dd, 1H), 3.86 (s, 3H), 3.82 - 3.71 (m, 1H), 3.27 (t, *J* = 12.3 Hz, 1H), 3.01 - 2.86 (m, 1H), 1.76 - 1.66 (m, 1H), 1.11 - 1.02 (m, 2H), 0.91 - 0.80 (m, 2H). | 655.2 |
| 120 | **Compound 134** | 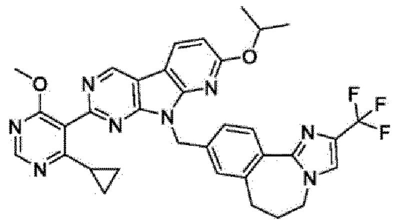 | $^1$H NMR (400 MHz, DMSO-$d_6$) δ 9.51 (s, 1H), 8.71 (s, 1H), 8.57 (d, *J* = 8.5 Hz, 1H), 7.95 (d, *J* = 1.3 Hz, 1H), 7.60 (d, *J* = 7.9 Hz, 1H), 7.48 (d, *J* = 1.7 Hz, 1H), 7.40 (dd, *J* = 7.9, 1.7 Hz, 1H), 6.82 (d, *J* = 8.5 Hz, 1H), 5.65 (s, 2H), 5.46 (h, *J* = 6.1 Hz, 1H), 3.94 (t, *J* = 6.8 Hz, 2H), 3.87 (s, 3H), 2.61 (t, *J* = 7.0 Hz, 2H), 2.26 - 2.15 (m, 2H), 1.77 - 1.66 (m, 1H), 1.38 (d, *J* = 6.1 Hz, 6H), 1.11 -. 1.03 (m, 2H), 0.89 - 0.80 (m, 2H). | 641.2 |
| 121 | **Compound 135** | 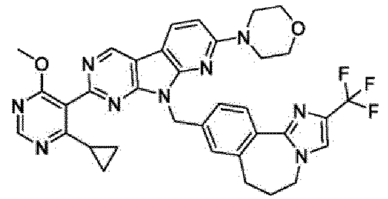 | $^1$H NMR (400 MHz, DMSO-$d_6$) δ 9.36 (s, 1H), 8.69 (s, 1H), 8.41 (d, *J* = 8.8 Hz, 1H), 7.95 (q, *J* = 1.3 Hz, 1H), 7.60 (d, *J* = 7.9 Hz, 1H), 7.44 (d, *J* = 1.7 Hz, 1H), 7.35 (dd, *J* = 8.0, 1.8 Hz, 1H), 6.94 (d, *J* = 8.8 Hz, 1H), 5.59 (s, 2H), 3.95 (t, *J* = 6.8 Hz, 2H), 3.86 (s, 3H), 3.76 (dd, *J* = 5.7, 3.6 Hz, 4H), 3.68 (t, *J* = 4.7 Hz, 4H), 2.60 (t, *J* = 7.0 Hz, 2H), 2.21 (p, *J* = 6.9 Hz, 2H), 1.71 (m, 1H), 1.04 (m, 2H), 0.83 (m, 2H). | 668.3 |
| 122 | **Compound 136** | 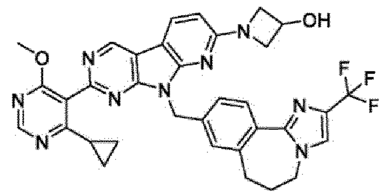 | $^1$H NMR (400 MHz, DMSO-$d_6$) δ 9.33 (s, 1H), 8.69 (s, 1H), 8.35 (dd, *J* = 8.5, 1.1 Hz, 1H), 7.95 (d, *J* = 1.3 Hz, 1H), 7.60 (d, *J* = 7.9 Hz, 1H), 7.45 (d, *J* = 1.7 Hz, 1H), 7.36 (dd, *J* = 8.0, 1.7 Hz, 1H), 6.44 (dd, *J* = 8.5, 0.9 Hz, 1H), 5.74 (d, *J* = 6.7 Hz, 1H), 5.56 (s, 2H), 4.69 - 4.59 (m, 1H), 4.38 - 4.29 (m, 2H), 3.96 (t, *J* = 6.8 Hz, 2H), 3.88 - 3.81 (m, 5H), 2.61 (t, *J* = 7.0 Hz, 2H), 2.22 (m, 2H), 1.70 (m, 1H), 1.04 (m, 2H), 0.82 (m, 2H). | 654.3 |

(continued)

| Example | Compound | Structural formula | $^1$HNMR | MS (ESI) M/Z :[M+H]$^+$ |
|---|---|---|---|---|
| 123 | **Compound 137** | 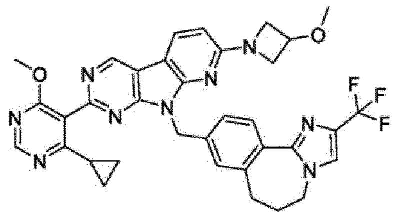 | $^1$H NMR (400 MHz, DMSO-$d_6$) δ 9.34 (s, 1H), 8.69 (s, 1H), 8.37 (d, $J$ = 8.5 Hz, 1H), 7.98 - 7.93 (m, 1H), 7.61 (d, $J$ = 7.8 Hz, 1H), 7.44 (d, $J$ = 1.8 Hz, 1H), 7.36 (dd, $J$ = 8.0, 1.8 Hz, 1H), 6.46 (d, $J$ = 8.5 Hz, 1H), 5.57 (s, 2H), 4.44 - 4.35 (m, 1H), 4.32 (dd, $J$ = 9.1, 6.3 Hz, 2H), 3.99 - 3.89 (m, 4H), 3.85 (s, 3H), 3.29 (s, 3H), 2.61 (t, $J$ = 7.0 Hz, 2H), 2.27 - 2.16 (m, 2H), 1.75 - 1.64 (m, 1H), 1.08 - 1.00 (m, 2H), 0.86 - 0.77 (m, 2H). | 668.3 |
| 124 | **Compound 140** | 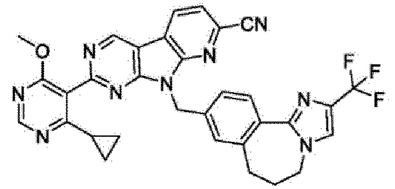 | $^1$H NMR (400 MHz, DMSO-$d_6$) δ 9.84 (s, 1H), 9.02 (d, $J$ = 7.9 Hz, 1H), 8.71 (s, 1H), 8.15 (d, $J$ = 8.0 Hz, 1H), 7.96 (d, $J$ = 1.3 Hz, 1H), 7.60 (d, $J$ = 7.9 Hz, 1H), 7.38 (d, $J$ = 1.8 Hz, 1H), 7.32 - 7.25 (m, 1H), 5.75 (s, 2H), 3.96 (t, $J$ = 6.8 Hz, 2H), 3.85 (s, 3H), 2.60 (t, $J$ = 7.0 Hz, 2H), 2.26 - 2.15 (m, 2H), 1.77 - 1.66 (m, 1H), 1.09 - 1.01 (m, 2H), 0.84 - 0.75 (m, 2H). | 608.3 |
| 125 | **Compound 141** | 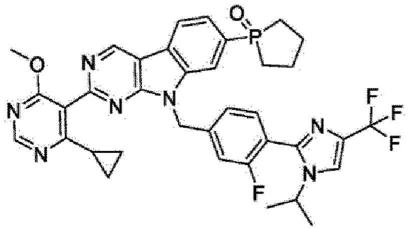 | $^1$H NMR (400 MHz, DMSO-$d_6$) δ 9.77 (s, 1H), 8.71 (s, 1H), 8.54 (dd, $J$ = 8.0, 2.3 Hz, 1H), 8.25 - 8.15 (m, 2H), 7.78 (m, 1H), 7.53 - 7.39 (m, 2H), 7.22 (dd, $J$ = 8.0, 1.6 Hz, 1H), 5.93 (s, 2H), 4.15 - 3.99 (m, 1H), 3.86 (s, 3H), 2.13 - 1.87 (m, 8H), 1.80 - 1.63 (m, 1H), 1.31 (d, $J$ = 6.6 Hz, 6H), 1.09 - 1.00 (m, 2H), 0.88 - 0.79 (m, 2H). | 704.2 |
| 126 | **Compound 142** | 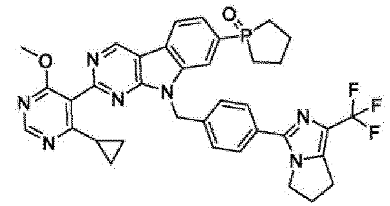 | $^1$H NMR (400 MHz, DMSO-$d_6$) δ 9.76 (s, 1H), 8.71 (s, 1H), 8.53 (dd, $J$ = 7.9, 2.3 Hz, 1H), 8.15 (d, $J$ = 12.0 Hz, 1H), 7.74 (d, $J$ = 8.4 Hz, 3H), 7.40 (d, $J$ = 8.1 Hz, 2H), 5.87 (s, 2H), 4.25 (t, $J$ = 7.2 Hz, 2H), 3.87 (s, 3H), 2.91 (t, $J$ = 7.5 Hz, 2H), 2.09 - 1.90 (m, 8H), 1.77 - 1.67 (m, 1H), 1.09 - 1.02 (m, 2H), 0.88 - 0.78 (m, 2H). | 684.2 |

(continued)

| Example | Compound | Structural formula | $^1$HNMR | MS (ESI) M/Z :[M+H]$^+$ |
|---|---|---|---|---|
| 127 | Compound 146 | 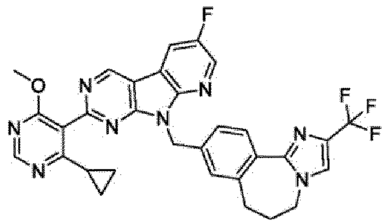 | $^1$H NMR (400 MHz, DMSO-$d_6$) δ 9.70 (s, 1H), 8.78 (dd, $J$ = 8.6, 2.8 Hz, 1H), 8.70 (d, $J$ = 3.8 Hz, 2H), 7.95 (d, $J$ = 1. 5 Hz, 1H), 7.59 (d, $J$ = 7.9 Hz, 1H), 7.37 (d, $J$ = 1.8 Hz, 1H), 7.29 (dd, $J$ = 7.9, 1.8 Hz, 1H), 5.73 (s, 2H), 3.95 (t, $J$ = 6.8 Hz, 2H), 3.86 (s, 3H), 2.60 (t, $J$ = 7.0 Hz, 2H), 2.26 - 2.14 (m, 2H), 1.77 - 1.66 (m, 1H), 1.09 - 1.01 (m, 2H), 0.86 - 0.77 (m, 2H). | 601.2 |
| 128 | **Compound 150** | 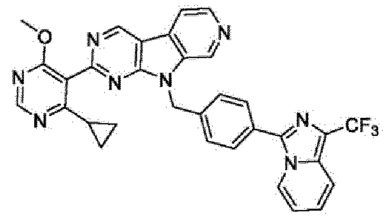 | $^1$H NMR (400 MHz, DMSO-$d_6$) δ 9.82 (s, 1H), 9.32 (s, 1H), 8.73 (s, 1H), 8.64 (d, $J$ = 5.2 Hz, 1H), 8.48 (d, $J$ = 7.2 Hz, 1H), 8.35 (d, $J$ = 5.2 Hz, 1H), 8.26 (s, 1H), 7.79 (d, $J$ = 8.0 Hz, 2H), 7.71 (d, $J$ = 9.2 Hz, 1H), 7.62 (d, $J$ = 8.1 Hz, 2H), 7.19 (dd, $J$ = 9.3, 6.5 Hz, 1H), 6.88 (t, $J$ = 6.9 Hz, 1H), 5.92 (s, 2H), 3.89 (s, 3H), 1.75 (m, 1H), 1.12 - 1.04 (m, 2H), 0.94 - 0.81 (m, 2H). | 593.2 |
| 129 | **Compound 151** | 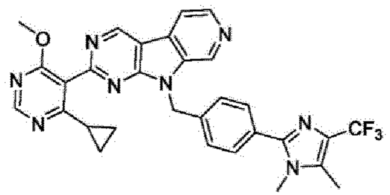 | $^1$H NMR (400 MHz, DMSO-$d_6$) δ 9.81 (s, 1H), 9.30 (s, 1H), 8.72 (s, 1H), 8.64 (d, $J$ = 5.2 Hz, 1H), 8.35 (d, $J$ = 5.2 Hz, 1H), 7.60 - 7.51 (m, 4H), 5.87 (s, 2H), 3.88 (s, 3H), 3.53 (s, 3H), 2.33 (d, $J$ = 1.8 Hz, 3H), 1.79 - 1.68 (m, 1H), 1.11 - 1.03 (m, 2H), 0.91 - 0.82 (m, 2H). | 571.5 |
| 130 | **Compound 152** | 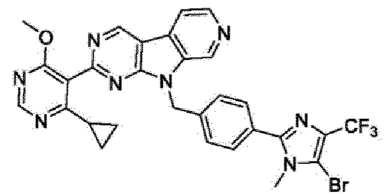 | $^1$H NMR (400 MHz, DMSO-$d_6$) δ 9.82 (s, 1H), 9.29 (s, 1H), 8.72 (s, 1H), 8.64 (d, $J$ = 5.2 Hz, 1H), 8.35 (d, $J$ = 5.2 Hz, 1H), 7.63 (d, $J$ = 8.2 Hz, 2H), 7.55 (d, $J$ = 8.2 Hz, 2H), 5.89 (s, 2H), 3.88 (s, 3H), 3.62 (s, 3H), 1.80 - 1.69 (m, 1H), 1.11 - 1.03 (m, 2H), 0.92 - 0.82 (m, 2H), | 635.4 |
| 131 | **Compound 153** | 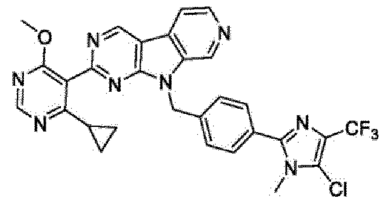 | $^1$H NMR (400 MHz, DMSO-$d_6$) δ 9.82 (s, 1H), 9.29 (s, 1H), 8.72 (s, 1H), 8.64 (d, $J$ = 5.2 Hz, 1H), 8.36 (t, $J$ = 6.6 Hz, 1H), 7.64 (d, $J$ = 8.2 Hz, 2H), 7.55 (d, $J$ = 8.1 Hz, 2H), 5.89 (s, 2H), 3.88 (s, 3H), 3.61 (s, 3H), 1.79 - 1.69 (m, 1H), 1.11 - 1.03 (m, 2H), 0.91 - 0.81 (m, 2H). | 63 5.4 |

(continued)

| Example | Compound | Structural formula | $^{1}$HNMR | MS (ESI) M/Z :[M+H]$^+$ |
|---|---|---|---|---|
| 132 | **Compound 154** | 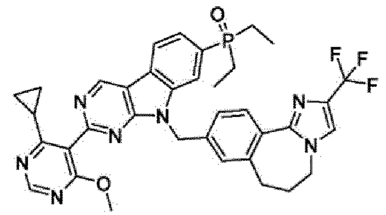 | $^{1}$H NMR (400 MHz, DMSO-$d_6$) δ 9.74 (s, 1H), 8.71 (s, 1H), 8.51 (dd, *J* = 7.9, 2.2 Hz, 1H), 8.15 (d, *J* = 11.2 Hz, 1H), 7.95 (d, *J* = 1.4 Hz, 1H), 7.76 (m, 1H), 7.58 (d, *J* = 7.9 Hz, 1H), 7.42 (d, *J* = 1.8 Hz, 1H), 7.25 (dd, *J* = 8.0, 1.8 Hz, 1H), 5.83 (s, 2H), 3.94 (t, *J* = 6.8 Hz, 2H), 3.87 (s, 3H), 2.63 - 2.52 (m, 2H), 2.25 - 2.14 (m, 2H), 2.14 - 1.90 (m, 4H), 1.76 - 1.65 (m, 1H), 1.10 - 1.02 (m, 2H), 0.99 - 0.87 (m, 6H), 0.87 - 0.78 (m, 2H), | 686.6 |
| 133 | **Compound 155** | 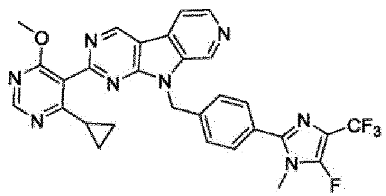 | $^{1}$H NMR (400 MHz, DMSO-$d_6$) δ 9.92 (s, 1H), 9.48 (s, 1H), 8.77 - 8.71 (m, 2H), 8.59 (d, *J* = 5.5 Hz, 1H), 7.64 (d, *J* = 8.4 Hz, 2H), 7.60 - 7.53 (m, 2H), 5.93 (s, 2H), 3.88 (s, 3H), 3.67 (s, 3H), 1.80 - 1.69 (m, 1H), 1.11 - 1.03 (m, 2H), 0.89 - 0.80 (m, 2H). | 575.5 |
| 134 | **Compound 157** | 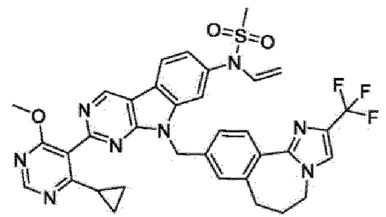 | $^{1}$H NMR (400 MHz, DMSO-$d_6$) δ 9.72 (s, 1H), 8.72 (s, 1H), 8.50 (d, *J* = 8.3 Hz, 1H), 8.04 - 7.92 (m, 2H), 7.79 - 7.67 (m, 1H), 7.61 - 7.49 (m, 3H), 7.39 (dd, *J* = 8.3, 1.8 Hz, 1H), 7.35 - 7.25 (m, 1H), 7.16 - 7.02 (m, 1H), 5.80 (s, 2H), 3.87 (s, 3H), 3.27 (s, 2H), 2.75 - 2.54 (m, 2H), 2.20 (m, 3H), 2.05 - 1.95 (m, 2H), 1.75 - 1.64 (m, 1H), 1.13 - 1.03 (m, 2H), 0.89 - 0.79 (m, 2H). | 701.6 |
| 135 | **Compound 158** | 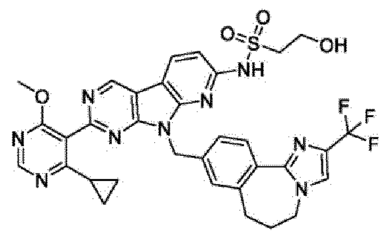 | $^{1}$H NMR (400 MHz, DMSO-$d_6$) δ 11.08 (s, 1H), 9.52 (s, 1H), 8.72 (s, 1H), 8.59 (d, *J* = 8.4 Hz, 1H), 7.95 (d, *J* = 1.4 Hz, 1H), 7.63 (d, *J* = 1.8 Hz, 1H), 7.58 (d, *J* = 7.9 Hz, 1H), 7.49 (dd, *J* = 7.9, 1.7 Hz, 1H), 6.97 (d, *J* = 8.4 Hz, 1H), 5.63 (s, 2H), 4.96 (s, 1H), 3.94 (t, *J* = 6.8 Hz, 2H), 3.88 (s, 3H), 3.85 (q, *J* = 3.3, 2.7 Hz, 4H), 2.63 (t, *J* = 7.0 Hz, 2H), 2.27 - 2.15 (m, 2H), 1.78 - 1.67 (m, 1H), 1.12 - 1.04 (m, 2H), 0.90 - 0.81 (m, 2H). | 706.2 |

(continued)

| Example | Compound | Structural formula | $^1$HNMR | MS (ESI) M/Z :[M+H]$^+$ |
|---|---|---|---|---|
| 136 | **Compound 159** | 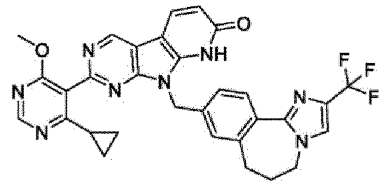 | $^1$H NMR (400 MHz, DMSO-$d_6$) δ 9.47 (s, 1H), 8.68 (s, 1H), 8.53 (d, *J* = 8.4 Hz, 1H), 7.95 (d, *J* = 1.4 Hz, 1H), 7.59 (d, *J* = 7.9 Hz, 1H), 7.33 (s, 1H), 7.23 (d, *J* = 8.0 Hz, 1H), 6.73 (d, *J* = 8.4 Hz, 1H), 5.64 (s, 2H), 3.95 (t, *J* = 6.7 Hz, 2H), 3.83 (s, 3H), 2.60 (t, *J* = 7.0 Hz, 2H), 2.25 - 2.14 (m, 2H), 1.70 - 1.62 (m, 1H), 1.02 (s, 2H), 0.81 - 0.74 (m, 2H). | 600.2 |
| 137 | **Compound 160** | 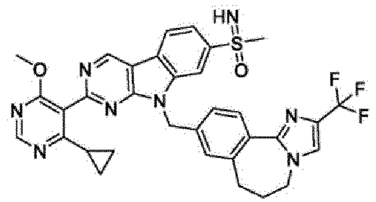 | $^1$H NMR (400 MHz, DMSO-$d_6$) δ 9.80 (s, 1H), 8.71 (s, 1H), 8.59 (d, *J* = 8.2 Hz, 1H), 8.37 (d, *J* = 1.5 Hz, 1H), 8.00 (dd, *J* = 8.2, 1.6 Hz, 1H), 7.95 (d, *J* = 1.3 Hz, 1H), 7.61 (d, *J* = 7.9 Hz, 1H), 7.37 (d, *J* = 1.8 Hz, 1H), 7.21 (dd, *J* = 8.0, 1.8 Hz, 1H), 5.93 - 5.79 (m, 2H), 4.38 (s, 1H), 3.95 (t, *J* = 6.7 Hz, 2H), 3.86 (s, 3H), 3.16 (d, *J* = 0.9 Hz, 3H), 2.60 (t, *J* = 7.0 Hz, 2H), 2.26 - 2.15 (m, 2H), 1.78 - 1.67 (m, 1H), 1.09 - 101 (m, 2H), 0.88 - 0.77 (m, 2H). | 660.2 |
| 138 | **Compound 161** | 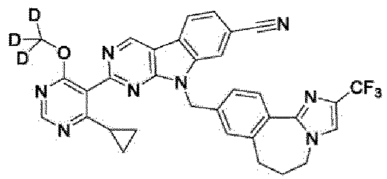 | $^1$H NMR (400 MHz, DMSO-$d_6$) δ 9.82 (s, 1H), 8.71 (s, 1H), 8.59 (d, *J* = 8.1 Hz, 1H), 8.50 (d, *J* = 1.3 Hz, 1H), 7.95 (d, *J* = 1.4 Hz, 1H), 7.86 (dd, *J* = 8.1, 1.3 Hz, 1H), 7.61 (d, *J* = 7.9 Hz, 1H), 7.40 (d, *J* = 1.8 Hz, 1H), 7.32 (dd, *J* = 8.0, 1.8 Hz, 1H), 5.80 (s, 2H), 3.95 (t, *J* = 6.8 Hz, 2H), 2.64 - 2.52 (m, 2H), 2.26 - 2.14 (m, 2H), 1.77 - 1.66 (m, 1H), 1.10 - 1.02 (m, 2H), 0.87 - 0.77 (m, 2H), | 610.2 |
| 139 | **Compound 163** | 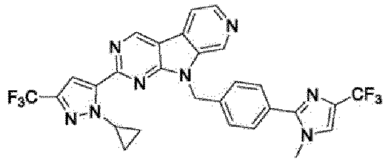 | $^1$H NMR (400 MHz, DMSO-$d_6$) δ 9.82 (s, 1H), 9.25 (s, 1H), 8.64 (d, *J* = 5.2 Hz, 1H), 8.35 (dd, *J* = 5.1, 1.1 Hz, 1H), 7.90 (d, *J* = 1.4 Hz, 1H), 7.71 - 7.65 (m, 2H), 7.57 - 7.48 (m, 3H), 5.96 (s, 2H), 4.92 - 4.82 (m, 1H), 3.72 (s, 3H), 1.22 - 1.14 (m, 2H), 1.13 - 0.99 (m, 2H). | 583.0 |
| 140 | **Compound 164** | 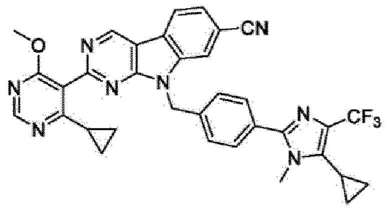 | $^1$H NMR (400 MHz, DMSO-$d_6$) δ 9.81 (s, 1H), 8.72 (s, 1H), 8.59-8.55 (t, *J* = 13.6 Hz, 1H), 7.87-7.85 (d, *J* = 8.0Hz, 1H), 7.60 - 7.52 (m, 4H), 5.84 (s, 2H), 3.88 (s, 1H), 3.65 (s, 3H), 1.79-1.73 (m, 2H), 1.07-1.02(m, 4H), 0.86-0.84(m,2H), 0.71-0.70(m,2H). | 621.6 |

**Example 141: 6-(4-cyclopropyl-6-methoxypyrimidin-5-yl)-8-(4-(1-isopropyl-4-(trifluoromethyl)-1H-imidazo 1-2-yl)benzyl)-2-methyl-2,8-dihydropyrazolo[4',3':4,5]pyrrolo [2,3-d]pyrimidine**

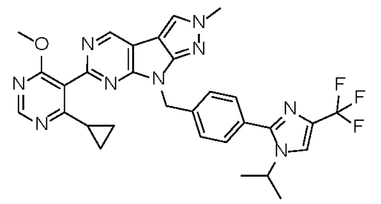

## Reaction route:

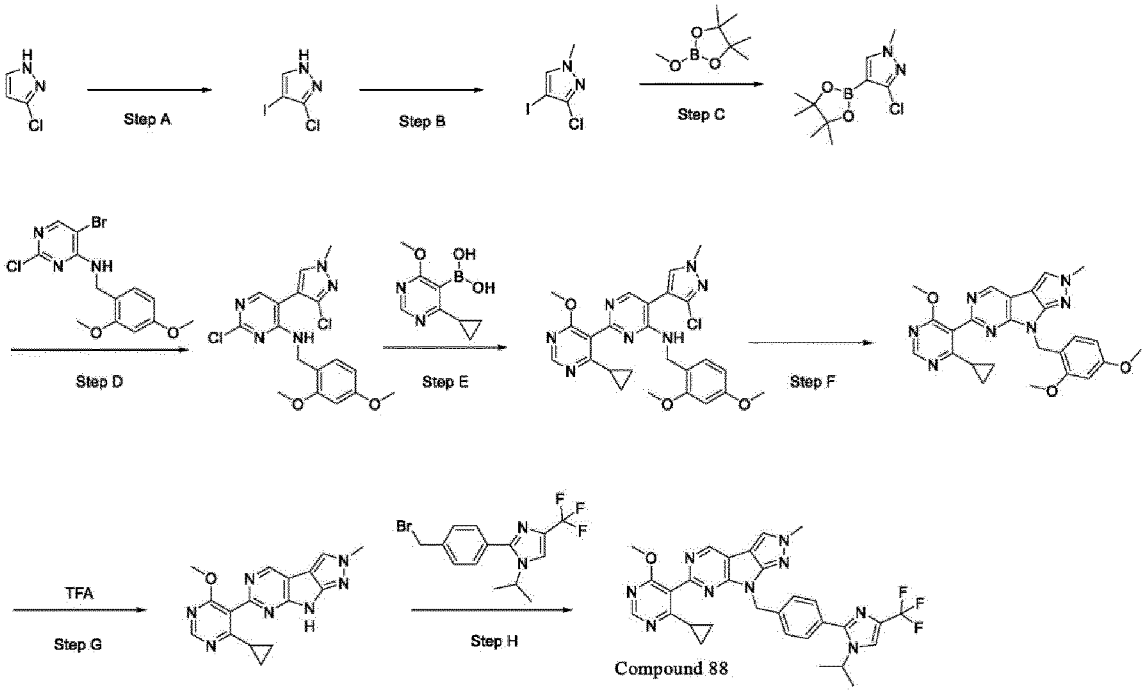


**Operation steps:**

**Step A:** 3-Chloro-1H-pyrazole (5 g, 49.0 mmol) was dissolved in N,N-dimethylformamide (50 mL) at 0°C. Subsequently, N-iodosuccinimide (14.3 g, 63.7 mmol) was slowly added to the above solution. The reaction system was then stirred at room temperature for 2 hours.

After the disappearance of raw materials as monitored by LCMS, ice water (200 mL) was added to the reaction solution for quenching. The mixed solution was extracted with ethyl acetate (50 mL × 3 times), and the organic phases were combined and washed with saturated saline (35 mL × 2 times). The resultant was dried with anhydrous sodium sulfate, filtered and concentrated under reduced pressure. The residue was purified by silica gel column chromatography to obtain 11 g of 3-chloro-4-iodo-1H-pyrazole.

MS(ESI) M/Z: 229.0 $[M+H]^+$.

**Step B:** 3-Chloro-4-iodo-1H-pyrazole (3.5 g, 15.35 mmol) was dissolved in dry tetrahydrofuran (77 mL) at 0°C under nitrogen protection. Subsequently, sodium hydride (content 60%) (1.2 g, 30.70 mmol) was slowly added to the above solution, and the resultant was stirred for 30 minutes. Then, methyl iodide (4.3 g, 30.70 mmol) was slowly added dropwise, and the mixture was heated to room temperature and stirred for 1 hour.

After the disappearance of raw materials as monitored by LCMS, ice water (150 mL) was added to the reaction solution for quenching. The mixed solution was extracted with ethyl acetate (50 mL × 3 times), and the organic phases were combined and then washed with saturated saline (20 mL × 2 times). The resultant was dried with anhydrous sodium sulfate, filtered and concentrated under reduced pressure. The residue was purified by silica gel column chromatography to obtain 2 g of 3-chloro-4-iodo-1-methyl-1H-pyrazole.

MS(ESI) M/Z: 243.0 $[M+H]^+$.

$^1H$ NMR (400 MHz, DMSO-$d_6$) $\delta$ 7.93 (s, 1H), 3.81 (s, 3H).

**Step C:** 3-Chloro-4-iodo-1-methyl-1H-pyrazole (1.6 g, 6.61 mmol) was dissolved in dry tetrahydrofuran (33 mL) at room temperature. Subsequently, 2 M isopropyl magnesium chloride (10.5 mL, 21.02 mmol) solution was slowly added dropwise into the above solution, and the resultant was stirred for 1 hour. Then, 2-methoxy-4,4,5,5-tetramethyl-1,3,2-dioxaborolane (4.43 mg, 28.03 mmol) was added, and stirring was continued for 1 hour.

After the disappearance of raw materials as monitored by LCMS, water (150 mL) was added to the reaction solution for quenching. The mixed solution was extracted with ethyl acetate (50 mL $\times$ 3 times), and the organic phases were combined and then washed with saturated saline (20 mL $\times$ 2 times). The resultant was dried with anhydrous sodium sulfate, filtered and concentrated under reduced pressure. The residue was purified by silica gel column chromatography to obtain 2 g of 3-chloro-1-methyl-4-(4,4,5,5-tetramethyl-1,3,2-dioxaboran-2-yl)-1H-pyrazole.

MS (ESI) M/Z: 243.2 $[M+H]^+$.

**Step D:** 3-Chloro-1-methyl-4-(4,4,5,5-tetramethyl-1,3,2-dioxaborolan-2-yl)-1H-pyrazole (2 g, 8.23 mmol), 5-bromo-2-chloro-N-(2,4-dimethoxybenzyl)pyrimidin-4-amine (1.5 g, 4.12 mmol) and sodium carbonate (0.87 g, 8.23 mmol) were dissolved in 1,4-dioxane/water (20 mL/2.2 mL) at room temperature. Subsequently, [1,1'-bis(diphenylphosphino) ferrocene]palladium dichloride dichloromethane complex (0.33 g, 0.41 mmol) was added to the above solution, and the reaction system was evacuated to remove air and purged with nitrogen for 3 times, and the reaction system was stirred at 90°C for 16 hours.

After the disappearance of raw materials as monitored by LCMS, ice water (50 mL) was added to the reaction solution for quenching. The mixed solution was extracted with ethyl acetate (20 mL $\times$ 3 times), and the organic phases were combined and then washed with saturated sodium chloride aqueous solution (20 mL $\times$ 2 times). The resultant was dried with anhydrous sodium sulfate, filtered and concentrated under reduced pressure. The residue was purified by silica gel column chromatography to obtain 780 mg of 2-chloro-5-(3-chloro-1-methyl-1H-pyrazol-4-yl)-N-(2,4-dimethoxybenzyl)pyrimidin-4-amine.

MS (ESI) M/Z: 394.2 $[M+H]^+$.

**Step E:** 2-Chloro-5-(3-chloro-1-methyl-1H-pyrazol-4-yl)-N-(2,4-dimethoxybenzyl)pyrimidin-4-amine (780 mg, 1.98 mmol), (4-cyclopropyl-6-methoxypyrimidin-5-yl)boric acid (386 mg, 1.98 mmol) and cesium carbonate (1.29 g, 3.96 mmol) were dissolved in 1,4-dioxane/water (10 mL/1.7 mL) at room temperature. Subsequently, chloro(2-dicyclohexylphosphino-2',4',6'-triisopropyl-1,1'-biphenyl)[2-(2'-amino-1,1'-biphenyl)]pall adium(II) (312 mg, 0.40 mmol) was added to the above solution, and the reaction system was evacuated to remove air and purged with nitrogen for 3 times, and then stirred at 90°C under microwave for three hours.

After the disappearance of raw materials as monitored by LCMS, ice water (50 mL) was added to the reaction solution for quenching. The mixed solution was extracted with ethyl acetate (20 mL $\times$ 3 times), and the organic phases were combined and then washed with saturated sodium chloride aqueous solution (20 mL $\times$ 2 times). The resultant was dried with anhydrous sodium sulfate, filtered and concentrated under reduced pressure. The residue was purified by silica gel column chromatography to obtain 650 mg of 5-(3-chloro-1-methyl-1H-pyrazol-4-yl)-4'-cyclopropyl-N-(2,4-dimethoxybenzyl)-6'-methoxy-[2,5'-bipyrimidine]-4-amine.

MS (ESI) M/Z: 508.2 $[M+H]^+$.

**Step F:** 5-(3-Chloro-1-methyl-1H-pyrazol-4-yl)-4'-cyclopropyl-N-(2,4-dimethoxybenzyl)-6'-methoxy-[2,5' -bipyrimidine]-4-amine (650 mg, 1.28 mmol), N,N'-dimethyl ethylenediamine (68 mg, 0.77 mmol) and potassium carbonate (353 mg, 2.56 mmol) were dissolved in acetonitrile (6.4 mL) at room temperature. Subsequently, cuprous iodide (73 mg, 0.38 mmol) was added, and the reaction system was evacuated to remove air and purged with nitrogen for 3 times, and then stirred at 120°C for 16 hours.

After the disappearance of raw materials as monitored by LCMS, the reaction solution was filtered and the filtrate was concentrated under reduced pressure. The residue was purified by silica gel column chromatography to obtain 180 mg of 6-(4-cyclopropyl-6-methoxypyrimidin-5-yl)-8-(2,4-dimethoxybenzyl)-2-methyl-2,8-dihydropyraz olo[4',3"4,5]pyrrolo[2,3-d]pyrimidine.

MS (ESI) M/Z: 472.2 $[M+H]^+$.

**Step G:** At room temperature and under nitrogen protection, 6-(4-cyclopropyl-6-methoxypyrimidin-5-yl)-8-(2,4-dimethoxybenzyl)-2-methyl-2,8-dihydropyraz olo[4',3':4,5]pyrrolo[2,3-d]pyrimidine (180 mg, 0.38 mmol) was dissolved in trifluoroacetic acid (8 mL). Then, the reaction system was stirred at 85°C for 16 hours.

After the disappearance of raw materials as monitored by LCMS, the mixture was concentrated under reduced pressure. The residue was purified by silica gel column chromatography to obtain 120 mg of 6-(4-cyclopropyl-6-methoxypyrimidin-5-yl)-2-methyl-2,8-dihydropyrazolo[4',3':4,5]pyrrolo[2,3-d] pyrimidine.

MS (ESI) M/Z: 322.2 $[M+H]^+$.

**Step H:** 6-(4-Cyclopropyl-6-methoxypyrimidin-5-yl)-2-methyl-2,8-dihydropyrazolo[4',3':4,5]pyrrolo[2,3-d ]pyrimidine (100 mg, 0.31 mmol) was dissolved in dry N,N-dimethylformamide (1.5 mL) at 0°C under nitrogen protection. Subsequently, sodium hydride (content 60%) (50 mg, 1.24 mmol) was slowly added to the above solution, and the resultant

was stirred for 0.5 hour. Then, 2-(4-(bromomethyl)phenyl)-1-isopropyl-4-(trifluoromethyl)-1H-imidazole (162 mg, 0.47 mmol) was added. The reaction system was heated to 50°C and stirred for 16 hours.

After the disappearance of raw materials as monitored by LCMS, ice water (30 mL) was added to the reaction solution for quenching. The mixed solution was extracted with ethyl acetate (10 mL × 3 times), and the organic phases were combined. The combined organic phase was then washed with saturated saline (20 mL × 2 times). The resultant was dried with anhydrous sodium sulfate, filtered and concentrated under reduced pressure. The residue was purified by preparative HPLC. 25.98 mg of 6-(4-cyclopropyl-6-methoxypyrimidin-5-yl)-8-(4-(1-isopropyl-4-(trifluoromethyl)-1H-imidazol-2-yl)benzyl)-2-methyl-2,8-dihydropyrazolo[4',3':4,5]pyrrolo[2,3-d]pyrimidine (compound 88) was obtained.

MS (ESI) M/Z: 588.3 $[M+H]^+$.

$^1H$ NMR (400 MHz, DMSO-$d_6$) δ 9.14 (s, 1H), 8.68 (s, 1H), 8.26 (s, 1H), 8.16 (d, $J$ = 1.2 Hz, 1H), 7.51 (d, $J$ = 8.4 Hz, 2H), 7.46 (d, $J$ = 8.4 Hz, 2H), 5.51 (s, 2H), 4.45-4.36 (m, 1H), 4.05 (s, 3H), 3.85 (s, 3H), 1.76-1.67 (m, 1H), 1.37 (d, $J$ = 6.4 Hz, 6H), 1.08-1.00 (m, 2H), 0.87-0.80 (m, 2H).

**Example 142: 6-(4-cyclopropyl-6-methoxypyrimidin-5-yl)-8-(4-(1-isopropyl-4-(trifluoromethyl)-1H-imidazo 1-2-yl)benzyl)-1,8-dihydropyrazole[4',3':4,5]pyrrolo[2,3-d]pyrimidine**

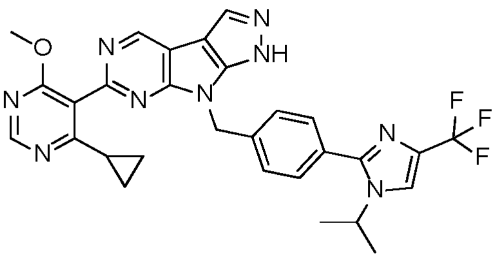

**Reaction route:**

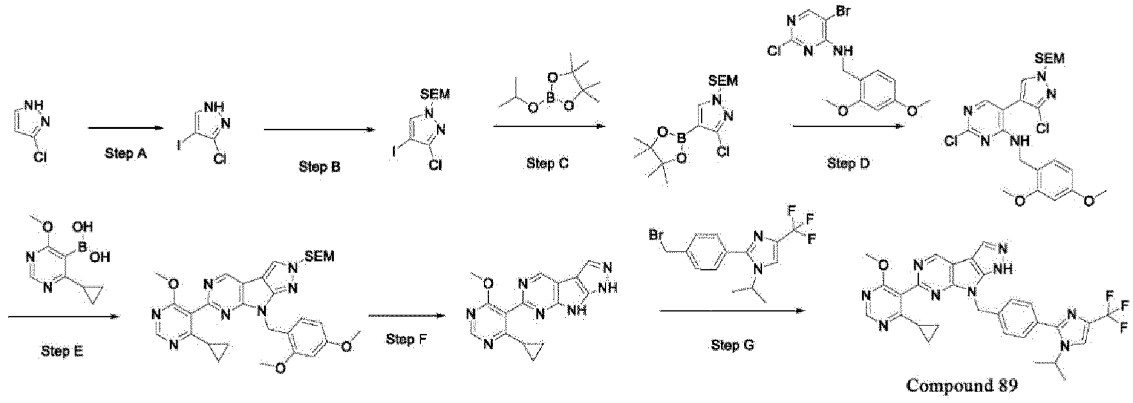


Compound 89

**Operation steps:**

**Step A:** 3-Chloro-1H-pyrazole (5 g, 49.02 mmol) was dissolved in N,N-dimethylformamide (50 mL) at 0°C. Subsequently, N-iodosuccinimide (14.3 g, 63.73 mmol) was slowly added to the above solution. The reaction system was then stirred at room temperature for 2 hours.

After the disappearance of raw materials as monitored by LCMS, ice water (200 mL) was added to the reaction solution for quenching. The mixed solution was extracted with ethyl acetate (50 mL × 3 times), and the organic phases were combined and then washed with saturated saline (35 mL × 2 times). The resultant was dried with anhydrous sodium sulfate, filtered and concentrated under reduced pressure. The residue was purified by silica gel column chromatography to obtain 11 g of 3-chloro-4-iodo-1H-pyrazole.

MS(ESI) M/Z: 229.0 $[M+H]^+$.

**Step B:** 3-Chloro-4-iodo-1H-pyrazole (4 g, 17.54 mmol) was dissolved in N,N-dimethylformamide (88 mL) under nitrogen protection at room temperature. Subsequently, sodium hydride (1.05 g, 26.32 mmol) was added to the above solution under ice-water bath, and the resultant was stirred for 30 minutes. Then 2-(trimethylsilyl)ethoxymethyl chloride (4.4 g, 26.32 mmol) was slowly added, and the mixture was heated to room temperature and stirred for 1 hour.

After the disappearance of raw materials as monitored by LCMS, the reaction solution was quenched by adding into ice water (500 mL). The mixed solution was extracted with ethyl acetate (80 mL × 3 times), and the organic phases were combined. The combined organic phase was washed with saturated saline (60 mL × 2 times). The resultant was dried with anhydrous sodium sulfate, filtered and concentrated under reduced pressure. The residue was purified by silica gel column chromatography to obtain 5 g of 3-chloro-4-iodo-1-((2-(trimethylsilyl)ethoxy)methyl)-1H-pyrazole.

MS (ESI) M/Z: 359.0 $[M+H]^+$.

**Step C:** 3-Chloro-4-iodo-1-((2-(trimethylsilyl)ethoxy)methyl)-1H-pyrazole (5 g, 13.97 mmol) was dissolved in dry tetrahydrofuran (70 mL) at room temperature. Subsequently, 2 M isopropyl magnesium chloride (22.5 mL, 44.70 mmol) was added to the above solution, and the resultant was stirred for 1 hour. Then 2-isopropoxy-4,4,5,5-tetramethyl-1,3,2-dioxaborolane (9.5 g, 60.07 mmol) was slowly added dropwise, and stirring was continued for 1 hour.

After the disappearance of raw materials as monitored by LCMS, the reaction solution was quenched by adding into ice water (100 mL). The mixed solution was extracted with ethyl acetate (50 mL × 3 times), and the organic phases were combined and then washed with saturated saline (60 mL × 2 times). The resultant was dried with anhydrous sodium sulfate, filtered and concentrated under reduced pressure. The residue was purified by silica gel column chromatography to obtain 4 g of 3-chloro-4-(4,4,5,5-tetramethyl-1,3,2-dioxaborolan-2-yl)-1-((2-(trimethylsilyl)ethoxy)methyl)-1H-pyrazole.

MS (ESI) M/Z: 359.2 $[M+H]^+$.

**Step D:** 3-Chloro-4-(4,4,5,5-tetramethyl-1,3,2-dioxaborolan-2-yl)-1-((2-(trimethylsilyl)ethoxy)methyl)-1H -pyrazole (4 g, 11.17), (5-bromo-2-chloro-N-(2,4-dimethoxybenzyl)pyrimidin-4-amine (2 g, 5.59 mmol), sodium carbonate (1.18 g, 11.17 mmol) and [1,1'-bis(diphenylphosphino)ferrocene]palladium dichloride dichloromethane complex (915 mg, 1.12 mmol) were dissolved in 1,4-dioxane/water (54 mL/6 mL) at room temperature. The reaction system was evacuated to remove air and purged with nitrogen for 3 times, and the reaction system was stirred at 100°C for 2 hours.

After the disappearance of raw materials as monitored by LCMS, the reaction solution was filtered and the filtrate was concentrated under reduced pressure. The residue was purified by silica gel column chromatography to obtain 3 g of 2-chloro-5-(3-chloro-1-((2-(trimethylsilyl)ethoxy)methyl)-1H-pyrazol-4-yl)-N-(2,4-dimethoxyben zyl)pyrimidin-4-amine.

MS (ESI) M/Z: 510.2 $[M+H]^+$.

**Step E:** 2-Chloro-5-(3-chloro-1-((2-(trimethylsilyl)ethoxy)methyl)-1H-pyrazol-4-yl)-N-(2,4-dimethoxyben zyl) pyrimidin-4-amine (3 g, 5.89 mmol), (4-cyclopropyl-6-methoxypyrimidin-5-yl)boric acid (2.3 g, 11.78 mmol), cesium carbonate (2.9 g, 8.84 mmol) and chloro(2-dicyclohexylphosphino-2',4',6'-triisopropyl-1,1'-biphenyl)[2-(2'-amino-1,1'-bipheny)]pall adium(II) (2.3 g, 2.95 mmol) were dissolved in 1,4-dioxane/water (54 mL/9 mL) at room temperature. The reaction system was evacuated to remove air and purged with nitrogen for 3 times, and the reaction system was stirred at 130°C for 16 hours.

After the disappearance of raw materials as monitored by LCMS, the reaction solution was filtered and the filtrate was concentrated under reduced pressure. The residue was purified by silica gel column chromatography to obtain 240 mg of 6-(4-cyclopropyl-6-methoxypyrimidin-5-yl)-8-(2,4-dimethoxybenzyl)-1-((2-(trimethylsilyl)ethoxy )methyl)-1,8-dihydropyrazolo[4',3':4,5]pyrrolo[2,3-d]pyrimidine.

MS (ESI) M/Z: 588.2 $[M+H]^+$.

**Step F:** At room temperature, 6-(4-cyclopropyl-6-methoxypyrimidin-5-yl)-8-(2,4-dimethoxybenzyl)-1-((2-(trimethylsilyl)ethoxy )methyl)-1,8-dihydropyrazolo[4',3':4,5]pyrrolo[2,3-d]pyrimidine (200 mg, 0.34 mmol) was dissolved in trifluoroacetic acid (3 mL). Then, the reaction system was stirred at 85°C for 5 hours.

After the disappearance of raw materials as monitored by LCMS, the reaction solution was concentrated under reduced pressure. The residue was purified by silica gel column chromatography to obtain 70 mg of 6-(4-cyclopropyl-6-methoxypyrimidin-5-yl)-1,8-dihydropyrazolo[4',3':4,5]pyrrolo[2,3-d]pyrimidin e.

MS (ESI) M/Z: 308.2 $[M+H]^+$.

**Step G:** 6-(4-Cyclopropyl-6-methoxypyrimidin-5-yl)-1,8-dihydropyrazolo[4',3':4,5]pyrrolo[2,3-d]pyrimidi ne (25 mg, 0.08 mmol), 2-(4-(bromomethyl)phenyl)-1-isopropyl-4-(trifluoromethyl)-1H-imidazole (28 mg, 0.08 mmol) and potassium carbonate (23 mg, 0.16 mmol) were dissolved in N,N-dimethylformamide (1 mL) at room temperature. The reaction system was then stirred at 50°C for 2 hours.

After the disappearance of raw materials as monitored by LCMS, ice water (30 mL) was added to the reaction solution for quenching. The mixed solution was extracted with ethyl acetate (10 mL × 3 times), and the organic phases were combined and then washed with saturated saline (20 mL × 2 times). The resultant was dried with anhydrous sodium sulfate, filtered and concentrated under reduced pressure. The residue was purified by preparative high performance liquid chromatography. 3.60 mg of 6-(4-cyclopropyl-6-methoxypyrimidin-5-yl)-8-(4-(1-isopropyl-4-(trifluoromethyl)-1H-imidazol-2-yl)benzyl)-1,8-dihydropyrazolo[4',3':4,5]pyrrolo[2,3-d]pyrimidine was obtained.

MS (ESI) M/Z: 574.2 $[M+H]^+$.

$^1H$ NMR (400 MHz, DMSO-$d_6$) δ 13.17 (s, 1H), 9.13 (s, 1H), 8.68 (s, 1H), 8.30 (s, 1H), 8.15 (s, 1H), 7.53-7.46 (m, 4H), 5.53 (s, 2H), 4.45-4.34 (m, 1H), 3.86 (s, 3H), 1.76-1.66 (m, 1H), 1.36 (d, *J* = 6.8 Hz, 6H), 1.07-1.00 (m, 2H), 0.91-0.80 (m, 2H).

**Example 143: 6-(4-cyclopropyl-6-methoxypyrimidin-5-yl)-8-(4-(1-isopropyl-4-(trifluoromethyl)-1H-imidazo 1-2-yl)benzyl)-1-methyl-1,8-dihydropyrazolo[4',3':4,5]pyrrolo[2,3-d]pyrimidine**

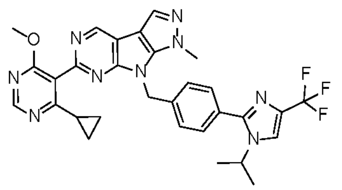

## Reaction route:

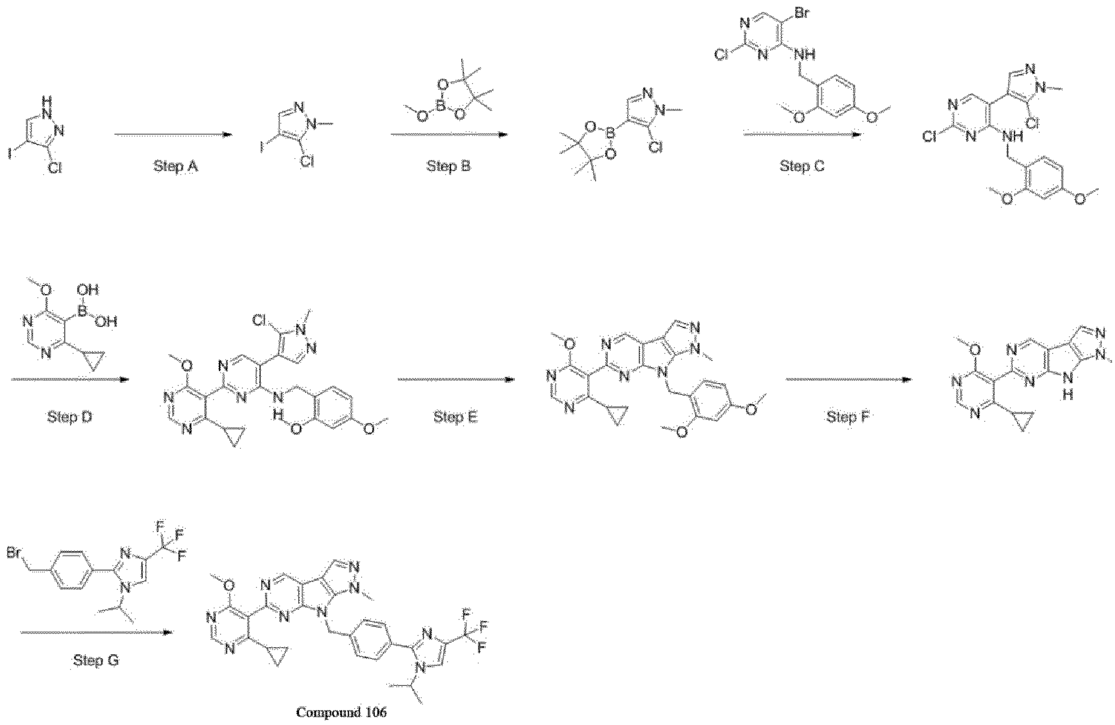


Compound 106

**Operation steps:**

**Step A:** 3-Chloro-4-iodo-1H-pyrazole (112 g, 491 mmol) was dissolved in dry tetrahydrofuran (1.4 L) at 0°C under nitrogen protection. Subsequently, sodium hydride (42 g, 1.05 mol) was slowly added to the above solution, and the resultant was stirred for 30 minutes. Then methyl iodide (104 g, 736 mmol) was added dropwise, and the mixture was heated to room temperature and stirred for 4 hours.

After the disappearance of raw materials as monitored by LCMS, ice water (5 L) was added to the reaction solution for quenching. The mixed solution was extracted with ethyl acetate (1000 mL × 3 times), and the organic phases were combined and then washed with saturated saline (600 mL × 2 times). The resultant was dried with anhydrous sodium sulfate, filtered and concentrated under reduced pressure. The residue was purified by silica gel column chromatography to obtain 23 g of 5-chloro-4-iodo-1-methyl-1H-pyrazole.

MS (ESI) M/Z: 243.0 $[M+H]^+$.

$^1H$ NMR (400 MHz, DMSO-$d_6$) δ 7.64 (s, 1H), 3.87 (s, 3H).

**Step B:** 5-Chloro-4-iodo-1-methyl-1H-pyrazole (3 g, 12.39 mmol) was dissolved in dry tetrahydrofuran (62 mL) at room temperature. Subsequently, 2 M isopropyl magnesium chloride solution (19.7 mL, 39.4 mmol) was added dropwise to the above solution and stirred for 1 hour. Then 2-methoxy-4,4,5,5-tetramethyl-1,3,2-dioxaborolane (6.72 g, 52.5 mmol) was added, and stirring was continued for 1 hour.

After the disappearance of raw materials as monitored by LCMS, water (150 mL) was added to the reaction solution for quenching. The mixed solution was extracted with ethyl acetate (50 mL × 3 times), and the organic phases

were combined and then washed with saturated saline (20 mL × 2 times). The resultant was dried with anhydrous sodium sulfate, filtered and concentrated under reduced pressure. The residue was purified by silica gel column chromatography to obtain 2.1 g of 5-chloro-1-methyl-4-(4,4,5,5-tetramethyl-1,3,2-dioxaborolan-2-yl)-1H-pyrazole.

MS (ESI) M/Z: 243.2 $[M+H]^+$.

**Step C:** 5-Chloro-1-methyl-4-(4,4,5,5-tetramethyl-1,3,2-dioxaborolan-2-yl)-1H-pyrazole (2 g, 8.23 mmol), 5-bromo-2-chloro-N-(2,4-dimethoxybenzyl)pyrimidin-4-amine (1.5 g, 4.12 mmol) and sodium carbonate (0.87 g, 8.23 mmol) were dissolved in 1,4-dioxane/water (20 mL/2.2 mL) at room temperature. Subsequently, [1,1'-bis(diphenylphosphino) ferrocene]palladium dichloride dichloromethane complex (0.33 g, 0.41 mmol) was added to the above solution. The reaction system was evacuated to remove air and purged with nitrogen for 3 times, and then stirred at 90°C for 16 hours.

After the disappearance of raw materials as monitored by LCMS, ice water (50 mL) was added to the reaction solution for quenching. The mixed solution was extracted with ethyl acetate (20 mL × 3 times), and the organic phases were combined and then washed with saturated sodium chloride aqueous solution (20 mL × 2 times). The resultant was dried with anhydrous sodium sulfate, filtered and concentrated under reduced pressure. The residue was purified by silica gel column chromatography to obtain 1.8 g of 2-chloro-5-(5-chloro-1-methyl-1H-pyrazol-4-yl)-N-(2,4-dimethoxybenzyl) pyrimidin-4-amine.

MS (ESI) M/Z: 394.2 $[M+H]^+$.

**Step D:** 2-Chloro-5-(5-chloro-1-methyl-1H-pyrazol-4-yl)-N-(2,4-dimethoxybenzyl)pyrimidin-4-amine (1.8 g, 4.57 mmol), (4-cyclopropyl-6-methoxypyrimidin-5-yl)boric acid (891 mg, 4.57 mmol) and cesium carbonate (3 g, 9.14 mmol) were dissolved in 1,4-dioxane/water (23 mL/2.3 mL) at room temperature. Subsequently, chloro(2-dicyclohexylphosphino-2',4',6'-triisopropyl-1,1'-biphenyl)[2-(2'-amino-1,1'-biphenyl)]p alladium(II) (644 mg, 0.91 mmol) was added to the above solution. The reaction system was evacuated to remove air and purged with nitrogen for 3 times, and then stirred at 90°C under microwave for 16 hours.

After the disappearance of raw materials as monitored by LCMS, ice water (150 mL) was added to the reaction solution for quenching. The mixed solution was extracted with ethyl acetate (50 mL × 3 times), and the organic phases were combined. The combined organic phase was washed with saturated sodium chloride aqueous solution (20 mL × 2 times). The resultant was dried with anhydrous sodium sulfate, filtered and concentrated under reduced pressure. The residue was purified by silica gel column chromatography to obtain 800 mg of 5-(5-chloro-1-methyl-1H-pyrazol-4-yl)-4'-cyclopropyl-N-(2,4-dimethoxybenzyl)-6'-methoxy-[2,5 '-bipyrimidine]-4-amine.

MS (ESI) M/Z: 508.8 $[M+H]^+$.

**Step E:** 5-(5-Chloro-1-methyl-1H-pyrazol-4-yl)-4'-cyclopropyl-N-(2,4-dimethoxybenzyl)-6'-methoxy-[2,5 '-bipyrimidine]-4-amine (800 mg, 1.58 mmol), N,N'-dimethyl ethylenediamine (278 mg, 3.16 mmol) and potassium carbonate (404 mg, 3.16 mmol) were dissolved in N,N-dimethylformamide (8 mL) at room temperature. Subsequently, cuprous iodide (300 mg, 1.58 mmol) was added. The reaction system was evacuated to remove air and purged with nitrogen for 3 times, and then stirred at 120°C for 16 hours.

After the disappearance of raw materials as monitored by LCMS, the reaction solution was filtered and the filtrate was concentrated under reduced pressure. The residue was purified by silica gel column chromatography to obtain 520 mg of 6-(4-cyclopropyl-6-methoxypyrimidin-5-yl)-8-(2,4-dimethoxybenzyl)-1-methyl-1,8-dihydropyraz olo[4',3':4,5]pyrrolo[2,3-d]pyrimidine.

MS (ESI) M/Z: 472.2 $[M+H]^+$.

**Step F:** At room temperature and under nitrogen protection, 6-(4-cyclopropyl-6-methoxypyrimidin-5-yl)-8-(2,4-dimethoxybenzyl)-1-methyl-1,8-dihydropyraz olo[4',3':4,5]pyrrolo[2,3-d]pyrimidine (520 mg, 1.1 mmol) was dissolved in trifluoroacetic acid (6 mL). Then, the reaction system was stirred at 85°C for 8 hours.

After the disappearance of raw materials as monitored by LCMS, the mixture was concentrated under reduced pressure. The residue was purified by silica gel column chromatography to obtain 250 mg of 6-(4-cyclopropyl-6-methoxypyrimidin-5-yl)-1-methyl-1,8-dihydropyrazolo[4',3':4,5]pyrrolo[2,3-d] pyrimidine.

MS (ESI) M/Z: 322.2 $[M+H]^+$.

**Step G:** 6-(4-Cyclopropyl-6-methoxypyrimidin-5-yl)-1-methyl-1,8-dihydropyrazolo[4°,3$^7$:4,5|pyrrolo[2,3-d]pyrimidine (50 mg, 0.16 mmol) was dissolved in dry N,N-dimethylformamide (1.0 mL) at 0°C under nitrogen protection. Subsequently, sodium hydride (25 mg, 0.62 mmol) was added to the above solution, and the resultant was stirred for 20 minutes. Then, 2-(4-(bromomethyl)phenyl)-1-isopropyl-4-(trifluoromethyl)-1H-imidazole (60 mg, 0.17 mmol) was added, and the mixture was heated to room temperature and stirred for 1 hour.

After the disappearance of raw materials as monitored by LCMS, ice water (50 mL) was added to the reaction solution for quenching. The mixed solution was extracted with ethyl acetate (15 mL × 3 times), and the organic phases were combined. The combined organic phase was washed with saturated saline (20 mL × 2 times). The resultant was dried with anhydrous sodium sulfate, filtered and concentrated under reduced pressure. The residue was purified by preparative high performance liquid chromatography. The purification conditions are as follows: preparative column: YMC-AcTus Triart C18 20.0 mm× 150 mm; mobile phase: water (containing 0.1% ammonium bicarbonate) and acetonitrile. 22.69 mg of 6-(4-cyclopropyl-6-methoxypyrimidin-5-yl)-8-(4-(1-isopropyl-4-(trifluoromethyl)-1H-imidazol-2-yl)benzyl)-1-methyl-1,8-dihydropyrazolo[4',3':4,5]pyrrolo[2,3-d]pyrimidine was obtained.

MS (ESI) M/Z: 588.2 $[M+H]^+$.

$^1H$ NMR (400 MHz, DMSO-$d_6$) δ 9.22 (s, 1H), 8.67 (s, 1H), 8.16 (d, *J* = 0.8 Hz, 1H), 7.95 (s, 1H), 7.54 (d, *J* = 8.0 Hz, 2H), 7.35 (d, *J* = 8.0 Hz, 2H), 5.85 (s, 2H), 4.49-4.33 (m, 1H), 3.97 (s, 3H), 3.85 (s, 3H), 1.74-1.65 (m, 1H), 1.37 (d, *J* = 6.8 Hz, 6H), 1.06-0.99 (m, 2H), 0.86-0.78 (m, 2H).

**Examples 144 to 166**

Target compounds in the following Table 2 were prepared by referring to the synthesis method of Example 142 above:

Table 2

| Example | Compound | Structural formula | $^1$HNMR | MS (ESI) M/ Z:[M+H] |
|---|---|---|---|---|
| 144 | **Compound 101** | 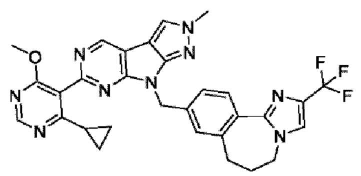 | $^1$H NMR (400 MHz, DMSO-*d6*) δ 9.14 (s, 1H), 8.68 (s, 1H), 8.26 (s, 1H), 7.96 (s, 1H), 7.62 (d, *J* = 7.9 Hz, 1H), 7.35 (s, 1H), 7.28 (d, *J* = 7.9 Hz, 1H), 5.47 (s, 2H), 4.04 (s, 3H), 3.96 (t, *J* = 6.6 Hz, 2H), 3.85 (s, 3H), 2.61 (t, *J* = 7.0 Hz, 2H), 2.28 - 2.15 (m, 2H), 1.76 - 1.65 (m, 1H), 1.09 - 0.99 (m, 2H), 0.89 - 0.77 (m, 2H). | 586.3 |
| 145 | **Compound 107** | 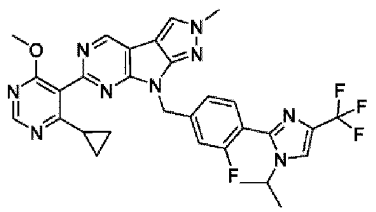 | $^1$H NMR (400 MHz, DMSO-$d_6$) δ 9.15 (s, 1H), 8.68 (s, 1H), 8.27 (s, 1H), 8.23 (d, *J* = 1.2 Hz, 1H), 7.51 (t, *J* = 7.6 Hz, 1H), 7.38 (dd, *J* = 10.4, 1.2 Hz, 1H), 7.27 (dd, *J* = 7.8, 1.4 Hz, 1H), 5.54 (s, 2H), 4.14 - 4.08 (m, 1H), 4.06 (s, 3H), 3.85 (s, 3H), 1.78 - 1.68 (m, 1H), 1.33 (d, *J*= 6.4 Hz, 6H), 1.08 - 1.00 (m, 2H), 0.89 - 0.78 (m, 2H). | 606.2 |
| 146 | **Compound 108** | 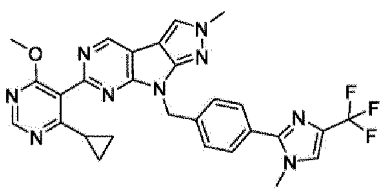 | $^1$H NMR (400 MHz, DMSO-$d_6$) δ 9.14 (s, 1H), 8.68 (s, 1H), 8.26 (s, 1H), 7.91 (d, *J* = 0.8 Hz, 1H), 7.66 (d, *J* = 8.0 Hz, 2H), 7.45 (d, *J* = 8.0 Hz, 2H), 5.50 (s, 2H), 4.05 (s, 3H), 3.86 (s, 3H), 3.73 (s, 3H), 1.76 - 1.68 (m, 1H), 1.08 - 1.01 (m, 2H), 0.88 - 0.81 (m, 2H). | 560.2 |
| 147 | **Compound 110** | 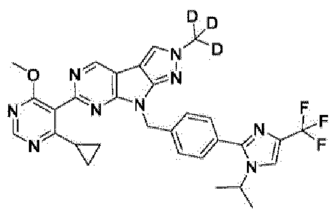 | $^1$H NMR (400 MHz, DMSO-$d_6$) δ 9.14 (s, 1H), 8.68 (s, 1H), 8.26 (s, 1H), 8.16 (d, *J* = 1.2 Hz, 1H), 7.51 (d, *J* = 8.0 Hz, 2H), 7.46 (d, *J* = 8.4 Hz, 2H), 5.51 (s, 2H), 4.45 - 4.35 (m, 1H), 3.86 (s, 3H), 1.76 - 1.68 (m, 1H), 1.37 (d, *J* = 6.4 Hz, 6H), 1.07 - 1.01 (m, 2H), 0.87 - 0.80 (m, 2H). | 591.3 |

(continued)

| Example | Compound | Structural formula | $^1$HNMR | MS (ESI) M/ Z:[M+H] |
|---|---|---|---|---|
| 148 | **Compound 114** | 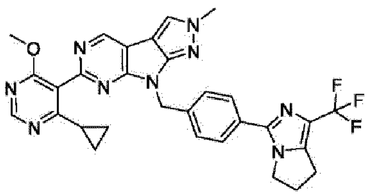 | $^1$H NMR (400 MHz, DMSO-$d_6$) δ 9.13 (s, 1H), 8.68 (s, 1H), 8.25 (s, 1H), 7.76 (d, *J* = 8.0 Hz, 2H), 7.43 *(d, J* = 8.4 Hz, 2H), 5.47 (s, 2H), 4.27 (t, *J* = 7.0 Hz, 2H), 4.04 (s, 3H), 3.86 (s, 3H), 2.92 (t, *J* = 6.8 Hz, 2H), 2.68 - 2.57 (m, 2H), 1.76 - 1.68 (m, 1H), 1.08 - 1.01 (m, 2H), 0.88 - 0.81 (m, 2H). | 586.2 |
| 149 | **Compound 115** | 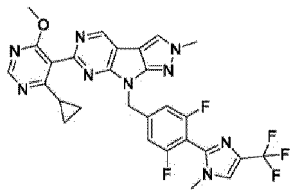 | $^1$H NMR (400 MHz, DMSO-d6) δ 9.16 (s, 1H), 8.68 (s, 1H), 8.28 (s, 1H), 8.05 (d, J = 1.2 Hz, 1H), 7.32 - 7.24 (m, 2H), 5.55 (s, 2H), 4.06 (s, 3H), 3.85 (s, 3H), 3.54 (s, 3H), 1.78 - 1.70 (m, 1H), 1.08 - 1.02 (m, 2H), 0.89 - 0.82 (m, 2H). | 596.2 |
| 150 | **Compound 118** | 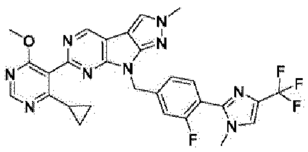 | $^1$H NMR (400 MHz, DMSO-$d_6$) δ 9.15 (s, 1H), 8.68 (s, 1H), 8.27 (s, 1H), 7.98 (d, *J* = 0.8 Hz, 1H), 7.55 *(t, J* = 7.6 Hz, 1H), 7.37 (dd, *J* = 11.0, 1.0 Hz, 1H), 7.27 (dd, *J* = 8.0, 1.2 Hz, 1H), 5.53 (s, 2H), 4.05 (s, 3H), 3.85 (s, 3H), 3.56 (d, *J* = 0.8 Hz, 3H), 1.80 - 1.66 (m, 1H), 1.09 - 0.99 (m, 2H), 0.90 - 0.79 (m, 2H). | 578.2 |
| 151 | **Compound 120** | 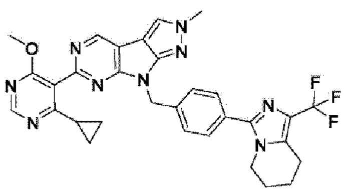 | $^1$H NMR (400 MHz, DMSO-$d_6$) δ 9.13 (s, 1H), 8.68 (s, 1H), 8.25 (s, 1H), 7.62 (d, *J* = 8.4 Hz, 2H), 7.44 (d, *J* = 8.4 Hz, 2H), 5.49 (s, 2H), 4.09 - 3.98 (m, 5H), 3.86 (s, 3H), 2.88 (brs, 2H), 1.87 - 1.76 (m, 4H), 1.74 - 1.68 (m, 1H), 1.08 - 1.01 (m, 2H), 0.88 - 0.81 (m, 2H). | 600.2 |
| 152 | **Compound 123** | 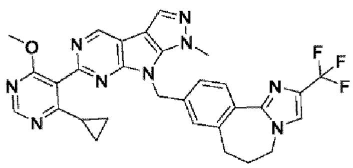 | $^1$H NMR (400 MHz, DMSO-$d_6$) δ 9.21 (s, 1H), 8.68 (s, 1H), 7.97 (s, 1H), 7.95 (s, 1H), 7.65 (d, *J* = 8.0 Hz, 1H), 7.28 (s, 1H), 7.16 (d, *J* = 8.0 Hz, 1H), 5.80 (s, 2H), 4.01- 3.92 (m, 5H), 3.85 (s, 3H), 2.62 (t, *J* = 6.8 Hz, 2H), 2.26 - 2.16 (m, 2H), 1.74 - 1.65 (m, 1H), 1.07 - 1.01 (m, 2H), 0.86 - 0.79 (m, 2H). | 586.2 |

(continued)

| Example | Compound | Structural formula | $^1$HNMR | MS (ESI) M/ Z:[M+H] |
|---|---|---|---|---|
| 153 | **Compound 131** | 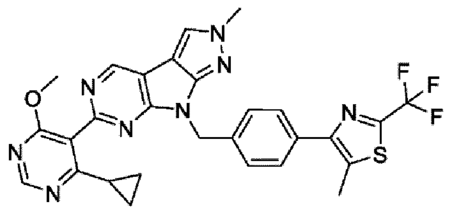 | $^1$H NMR (400 MHz, DMSO-$d_6$) δ 9.13 (s, 1H), 8.68 (s, 1H), 8.26 (s, 1H), 7.66 - 7.60 (m, 2H), 7.48 - 7.42 (m, 2H), 5.49 (s, 2H), 4.05 (s, 3H), 3.86 (s, 3H), 2.61 (s, 3H), 1.77 - 1.66 (m, 1H), 1.08 - 0.96 (m, 2H), 0.88 - 0.79 (m, 2H). | 577.4 |
| 154 | **Compound 133** | 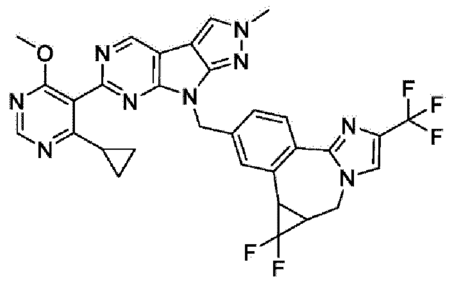 | $^1$H NMR (400 MHz, DMSO-$d_6$) δ 9.14 (s, 1H), 8.68 (s, 1H), 8.26 (s, 1H), 7.99 (d, *J* = 1.3 Hz, 1H), 7.68 (d, *J* = 8.0 Hz, 1H), 7.46 (d, *J* = 1.7 Hz, 1H), 7.40 - 7.33 (m, 1H), 5.60 - 5.38 (m, 2H), 4.65 (dd, *J* = 15.0, 6.5 Hz, 1H), 4.04 (s, 3H), 3.84 (s, 3H), 3.79 (dd, *J* = 14.7, 11.3 Hz, 1H), 3.26 (t, *J* = 12.4 Hz, 1H), 3.01 - 2.86 (m, 1H), 1.75 - 1.64 (m, 1H), 1.08 - 0.99 (m, 2H), 0.88 - 0.78 (m, 2H), | 634.3 |
| 155 | **Compound 138** | 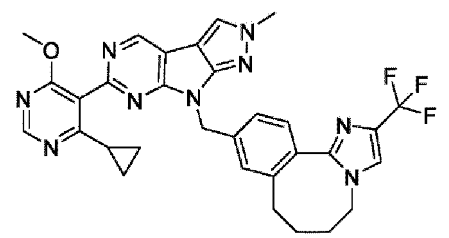 | $^1$H NMR (400 MHz, DMSO-$d_6$) δ 9.14 (s, 1H), 8.68 (s, 1H), 8.26 (s, 1H), 7.96 (d, *J* = 1.3 Hz, 1H), 7.57 (d, *J* = 7.8 Hz, 1H), 7.48 - 7.43 (m, 1H), 7.24 (dd,*J* = 8.0, 1.7 Hz, 1H), 5.50 (s, 2H), 4.21 - 4.11 (m, 1H), 4.05 (s, 3H), 3.84 (s, 3H), 3.72 - 3.59 (m, 1H), 2.79 - 2.66 (m, 1H), 2.50 - 2.36 (m, 1H), 1.87 - 1.75 (m, 1H), 1.73 - 1.62 (m, 1H), 1.11 (d, *J* = 6.9 Hz, 3H), 1.08 - 1.00 (m, 2H), 087 - 0.78(m, 2H). | 600.3 |
| 156 | **Compound 139** | 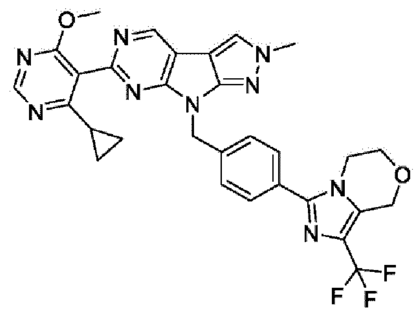 | $^1$H NMR (400 MHz, DMSO-$d_6$) δ 9.13 (s, 1H), 8.68 (s, 1H), 8.25 (s, 1H), 7.72 - 7.65 (m, 2H), 7.46 (d, *J* = 8.0 Hz, 2H), 5.50 (s, 2H), 4.98 - 4.92 (m, 2H), 4.15 (t, *J* = 5.1 Hz, 2H), 4.04 (s, 3H), 3.92 (t,*J* = 5.0 Hz, 2H), 3.86 (s, 3H), 1.78 - 1.67 (m, 1H), 1.09 - 1.00 (m, 2H), 0.90 - 0.81 (m, 2H). | 602.2 |

(continued)

| Example | Compound | Structural formula | $^1$HNMR | MS (ESI) M/ Z:[M+H] |
|---|---|---|---|---|
| 157 | **Compound 143** | 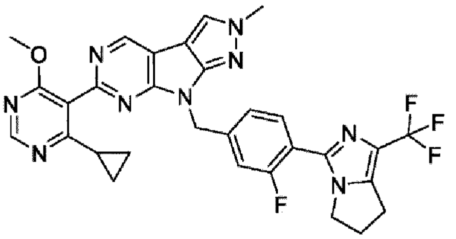 | $^1$H NMR (400 MHz, DMSO-$d_6$) δ 9.14 (s, 1H), 8.68 (s, 1H), 8.26 (s, 1H), 7.64 (t,.*J* = 7.9 Hz, 1H), 7.34 (dd, *J* = 11.4, 1.7 Hz, 1H), 7.26 (dd,*J* = 8.0, 1.7 Hz, 1H), 5.51 (s, 2H), 4.05 (s, 3H), 4.04 (t, *J* = 7.2 Hz, 2H), 3.86 (s, 3H), 2.99 - 2.90 (m, 2H), 2.64 - 2.52 (m, 2H), 1.78 - 1.68 (m, 1H), 1.09 - 1.01 (m, 2H), 0.90 - 0.81 (m, 2H). | 604.0 |
| 158 | **Compound 144** | 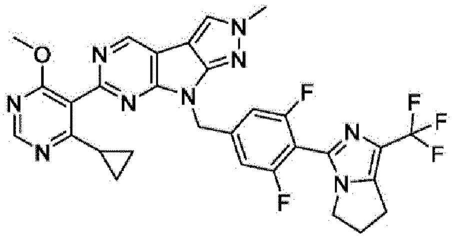 | $^1$H NMR (400 MHz, DMSO-$d_6$) δ 9.16 (s, 1H), 8.68 (s, 1H), 8.27 (s, 1H), 7.24 (d, *J* = 8.5 Hz, 2H), 5.53 (s, 2H), 4.06 (s, 3H), 3.93 (t, *J* = 7.2 Hz, 2H), 3.85 (s, 3H), 2.97 (t, *J* = 7.0 Hz, 2H), 2.59 (t, *J* = 7.1 Hz, 2H), 1.80 - 1.69 (m, 1H), 1.09 - 1.01 (m, 2H), 0.90 - 0.81 (m, 2H). | 621.8 |
| 159 | **Compound 145** | 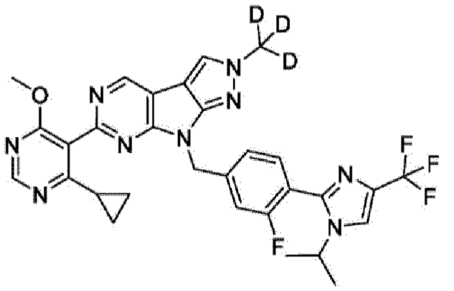 | $^1$H NMR (400 MHz, DMSO-$d_6$) δ 9.15 (s, 1H), 8.68 (s, 1H), 8.30 - 8.20 (m, 2H), 7.51 (t, *J* = 7.7 Hz, 1H), 7.38 (dd, *J* = 10.7, 1.6 Hz, 1H), 7.26 (dd, *J* = 7.9, 1.6 Hz, 1H), 5.54 (s, 2H), 4.15 - 4.04 (m, 1H), 3.85 (s, 3H), 1.78 - 1.67 (m, 1H), 1.32 (d, *J* = 6.6 Hz, 6H), 1.08 - 1.00 (m, 2H), 0.88 - 0.79 (m, 2H). | 609.3 |
| 160 | **Compound 147** | 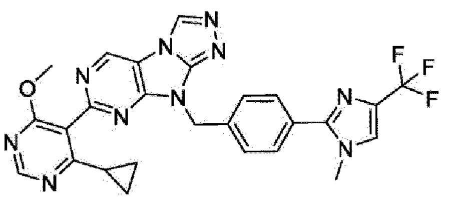 | $^1$H NMR (400 MHz, Chloroform-d) δ 9.07 (s, 1H), 8.75 (s, 1H), 8.70 (s, 1H), 7.81 - 7.73 (m, 2H), 7.65 - 7.57 (m, 2H), 7.30 (q, *J* = 1.2 Hz, 1H), 5.58 (s, 2H), 3.96 (s, 3H), 3.73 (s, 3H), 1.72 (tt, *J* = 8.3, 4.6 Hz, 1H), 1.32 - 1.24 (m, 2H), 0.97 - 0.88 (m, 2H). | 547.2 |
| 161 | **Compound 148** | 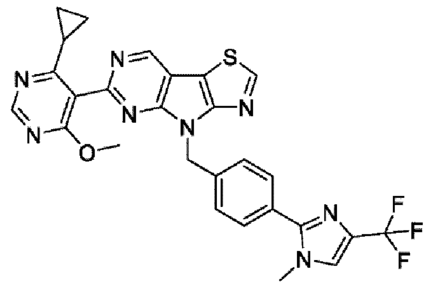 | $^1$H NMR (400 MHz, DMSO-$d_6$) δ 9.51 (s, 1H), 9.40 (s, 1H), 8.70 (s, 1H), 7.90 (d, *J* = 1.4 Hz, 1H), 7.69 - 7.62 (m, 2H), 7.52 - 7.45 (m, 2H), 5.80 (s, 2H), 3.86 (s, 3H), 3.72 (s, 3H), 1.77 - 1.66 (m, 1H), 1.10 - 1.02 (m, 2H), 0.89 - 0.80 (m, 2H). | 563.2 |

(continued)

| Example | Compound | Structural formula | ¹HNMR | MS (ESI) M/ Z:[M+H] |
|---|---|---|---|---|
| 162 | **Compound 149** | 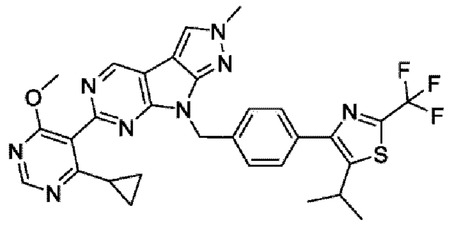 | $^1H$ NMR (400 MHz, DMSO-$d_6$) δ 9.13 (s, 1H), 8.67 (s, 1H), 8.26 (s, 1H), 7.53 (d, *J* = 8.2 Hz, 2H), 7.45 (d, *J* = 8.2 Hz, 2H), 5.50 (s, 2H), 4.05 (s, 3H), 3.85 (s, 3H), 3.45 - 3.38 (m, 1H), 1.75 - 1.67 (m, 1H), 1.29 (d, *J* = 6.8 Hz, 6H), 1.06 - 1.00 (m, 2H), 0.86 - 0.80 (m, 2H). | 605.4 |
| 163 | **Compound 156** | 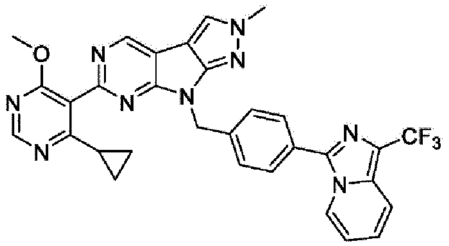 | $^1H$ NMR (400 MHz, DMSO-$d_6$) δ 9.14 (s, 1H), 8.68 (s, 1H), 8.49 (m, 1H), 8.27 (s, 1H), 7.82 - 7.76 (m, 2H), 7.72 (m, 1H), 7.57 - 7.50 (m, 2H), 7.20 (dd, *J* = 9.3, 6.5 Hz, 1H), 6.94 - 6.86 (m, 1H), 5.54 (s, 2H), 4.06 (s, 3H), 3.86 (s, 3H), 1.79 - 1.68 (m, 1H), 1.08 - 1.00 (m, 2H), 0.90 - 0.81 (m, 2H). | 596.6 |
| 164 | **Compound 162** | 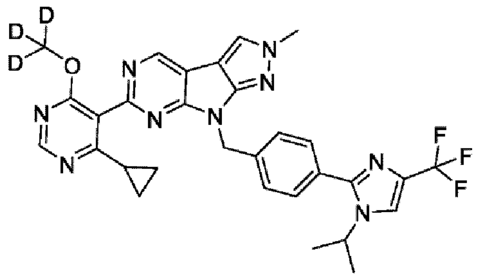 | $^1H$ NMR (400 MHz, DMSO-$d_6$) δ 9.14 (s, 1H), 8.67 (s, 1H), 8.26 (s, 1H), 8.15 (t,*J* = 1.4 Hz, 1H), 7.51 (d, *J* = 8.3 Hz, 2H), 7.46 (d, *J* = 8.4 Hz, 2H), 5.51 (s, 2H), 4.47 - 4.33 (m, 1H), 4.05 (s, 3H), 1.77 - 1.66 (m, 1H), 1.37 (d, *J* = 6.6 Hz, 6H), 1.08 - 1.00 (m, 2H), 0.88 - 0.79 (m, 2H). | 592.2 |
| 165 | **Compound 165** | 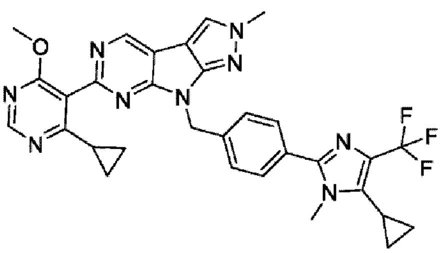 | $^1H$ NMR (400 MHz, DMSO-$d_6$) δ 9.13 (s, 1H), 8.67 (s, 1H), 8.25 (s, 1H), 7.60 - 7.44 (m, 4H), 5.49 (s, 2H), 4.04 (s, 3H), 3.86 (s, 3H), 3.66(s, 3H), 1.79-1.70 (m, 2H), 1.06-1.01(m, 4H), 0.87-0.82(m,2H), 0.73-0.70(m,2H). | 600.6 |
| 166 | **Compound 166** | 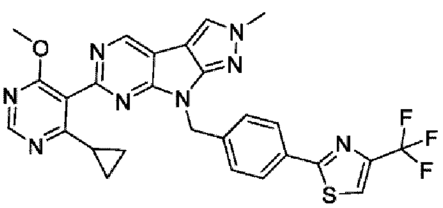 | $^1H$ NMR (400 MHz, DMSO-$d_6$) δ 9.13 (s, 1H), 8.67 (s, 1H), 8.53 (s, 1H), 8.25(s, 1H), 7.93 - 7.91 (m, 2H), 7.46-7.44(m, 2H), 5.50 (s, 2H), 4.04 (s, 3H), 3.85 (s, 3H), 1.71-1.68 (m, 1H), 1.05-1.01(m, 2H), 0.85-0.81(m,2H). | 563.4 |

**II. Biological Activity Test**

As stated in this application, room temperature refers to a temperature of about 20°C to 30°C.

**Test Example 1 Experimental Method of USP1 Enzyme Activity**

**1. Experimental scheme:**

USP1i compounds were screened by USP1 enzyme activity detection experiment.

**1.1 Experimental materials:** recombinant human His6-USP1/His6-UAF1 composite protein (R&D, catalog number E-568-050); Ubiquitin rhodamine 110 (Ub-Rho) (R&D, catalog number U-555-050); Fluorescent 384-well plate (Perkin Elmer, Art. No. 6007279).

**1.2 Test sample:** Compounds of table 3 in this application, whose structural formulae and preparation methods are shown in the above examples.

**1.3 Experimental process:**

(1) Preparing 1× detection buffer (improved Tris buffer, comprising 50 mM Tris-HCl (pH 7.8) (Sigma, article number: T2569-1L), 0.01% Tween-20 (Sigma, article number: P2287-100ML), 1 mM DTT (Sigma, article number: D0632-10G), 0.01% BSA (Sigma, article number: B2064-100G) and 0.5 mM EDTA (Invitrogen, article number: 15575020)).
(2) Diluting compounds: preparing a 10 mM (mol/L) solution of the compound to be tested with dimethyl sulfoxide (DMSO, with a purity of 100%); diluting the solution of the compound to be detected in a 3-fold gradient to obtain 10 concentrations, wherein the highest concentration is 10 mM; transferring the diluted solution of the compound to be tested to a fluorescent 384-well plate by Echo acoustic liquid handlers, double holes were set for each concentration, and the final concentration of DMSO is 1 vol%; the final concentrations of the solution of the compounds to be tested is 10000 nM, 3333 nM, 1111 nM, 370 nM, 123 nM, 41 nM, 13.7 nM, 4.6 nM, 1.5 nM and 0.5 nM.
(3) Preparing enzyme solution: preparing enzyme solution in 1× detection buffer.
(4) Preparing substrate solution: adding Ubiquitin Rhodamine 110 (Ub-Rho) into 1× detection buffer to form the substrate solution.
(5) Transferring 10 μL of the enzyme solution prepared in step (3) to the fluorescent 384-well plate.
(6) Incubating the resultant for 1 hour at room temperature.
(7) Adding 10 μL of the substrate solution prepared in step (4) into each well to start the reaction; wherein the final reaction system composed of 200 nL of the compound to be tested + 10 μL of the enzyme solution + 10 μL of the substrate solution; the final concentration of enzyme is 0.05 nM, and the final concentration of the substrate solution is 300 nM; and the reaction is carried out with centrifuging for 30s and shaking for 30s.
(8) Reading the plate on a multifunctional enzyme-labeling instrument SpectraMax Paradigm for 30 minutes, with an excitation wavelength of 480 nm and an emission wavelength of 540 nm.
(9) Collecting data regarding SpectraMax Paradigm.
(10) Curve fitting:

Using Equation (I) to fit data in Excel to obtain the inhibition value;

inhibition rate % = (maximum signal value - target signal value)/(maximum signal value - minimum signal value) × 100; Equation (1):

wherein, the maximum signal value represents the luminous signal intensity of the positive control hole without adding the compound of the application; the minimum signal value represents the luminous signal intensity of the negative control hole without enzyme; and the target signal value represents the luminous signal intensity of the Test samples; and
fitting the data in XL-Fit with Equation (II) to obtain $IC_{50}$ value;

$$\text{Equation (II): } Y=\text{Bottom}+(\text{Top}-\text{Bottom})/(1+(IC_{50}/X)\times\text{HillSlope})$$

wherein Y is the inhibition rate, X is the concentration of the compound; Bottom is the lowest inhibition rate; Top is the highest inhibition rate; and HillSlope is the slope.

**2. Experimental results:**

According to the determination, the compounds in the examples of the application have good inhibitory effects on USP1, and the $IC_{50}$ values are generally lower than 1000 nanomoles (nM). The $IC_{50}$ values of some compounds in the examples of this application are below 100 nM, and more preferably, the $IC_{50}$ values of the compounds in the examples of this application are below 50 nM or even below 10 nM. See Table 3 for the results of inhibition of USP1 by some examples of the compounds in this application.

**Table 3 Enzymatic inhibition results**

| Compound | $IC_{50}$(nM) | Compound | $IC_{30}$(nM) |
|---|---|---|---|
| **Compound 1** | 4.04 | **Compound 2** | 7.55 |
| **Compound 3** | 5.55 | **Compound 4** | 6.85 |
| **Compound 5** | 5.63 | **Compound 6** | 6.07 |
| **Compound 7** | 5.81 | **Compound 8** | 20.96 |
| **Compound 9** | 4.07 | **Compound 10** | 17.85 |
| **Compound 11** | 8.91 | **Compound 12** | 58.26 |
| **Compound 13-P1** | 6.01 | **Compound 13-P2** | 5.00 |
| **Compound 14** | 4.32 | **Compound 15** | 12.24 |
| **Compound 16** | 7.55 | **Compound 17** | 9.28 |
| **Compound 18** | 7.38 | **Compound 19** | 8.25 |
| **Compound 20** | 3.26 | **Compound 21** | 4.39 |
| **Compound 22** | 135.00 | **Compound 23** | 11.67 |
| **Compound 24** | 35.53 | **Compound 25** | 13.41 |
| **Compound 26** | 11.49 | **Compound 27** | 9.95 |
| **Compound 28** | 5.95 | **Compound 29** | 4.62 |
| **Compound 30** | 5.71 | **Compound 31** | 4.85 |
| **Compound 32** | 19.07 | **Compound 33** | 26.07 |
| **Compound 34** | 61.98 | **Compound 35** | 9.82 |
| **Compound 36** | 8.98 | **Compound 37** | 58.01 |
| **Compound 38** | 12.09 | **Compound 39** | 29.94 |
| **Compound 40** | 15.05 | **Compound 41** | 35.20 |
| **Compound 42** | 30.44 | **Compound 43** | 1.29 |
| **Compound 44** | 4.22 | **Compound 45** | 4.40 |
| **Compound 46** | 3.60 | **Compound 47** | 5.26 |
| **Compound 48** | 0.92 | **Compound 49** | 21.17 |
| **Compound 50** | 4.84 | **Compound 51** | 5.90 |
| **Compound 52** | 245.29 | **Compound 53** | 5.58 |
| **Compound 54** | 9.88 | **Compound 55** | 7.90 |
| **Compound 56** | 7.65 | **Compound 57** | 14.75 |
| **Compound 58** | 7.90 | **Compound 59** | 7.21 |
| **Compound 60** | 5.34 | **Compound 61** | 3.14 |
| **Compound 62** | 2.65 | **Compound 63** | 2.74 |
| **Compound 64** | 6.12 | **Compound 65** | 59.57 |
| **Compound 66** | 1.62 | **Compound 67** | 36.07 |

(continued)

| Compound | $IC_{50}$(nM) | Compound | $IC_{30}$(nM) |
|---|---|---|---|
| **Compound 68** | 39.19 | **Compound 69** | 516.62 |
| **Compound 70** | 190.71 | **Compound 71** | 108.13 |
| **Compound 72** | 42.90 | **Compound 73** | 4.90 |
| **Compound 74** | 4.13 | **Compound 75** | 38.53 |
| **Compound 76** | 25.26 | **Compound 77** | 99.09 |
| **Compound 78** | 4.39 | **Compound 79** | 2.04 |
| **Compound 80** | 6.55 | **Compound 81** | 7.15 |
| **Compound 82** | 4.45 | **Compound 83** | 4.62 |
| **Compound 84** | 58.96 | **Compound 85** | 73.51 |
| **Compound 86** | 6.96 | **Compound 87** | 8.56 |
| **Compound 88** | 4.50 | **Compound 89** | 4.70 |
| **Compound 90** | 3.65 | **Compound 91** | 4.16 |
| **Compound 92** | 25.95 | **Compound 93** | 2.56 |
| **Compound 94** | 3.69 | **Compound 95** | 138.33 |
| **Compound 96** | 4.89 | **Compound 97** | 2.10 |
| **Compound 98** | 1.26 | **Compound 99** | 3.13 |
| **Compound 103** | 12.35 | **Compound 104** | 10.15 |
| **Compound 105** | 5.17 | **Compound 106** | 4.56 |
| **Compound 107** | 4.65 | **Compound 108** | 7.52 |
| **Compound 109** | 11.86 | **Compound 110** | 9.50 |
| **Compound 112** | 23.90 | **Compound 113** | 7.73 |
| **Compound 114** | 1.89 | **Compound 115** | 3.03 |
| **Compound 116** | 6.48 | **Compound 117** | 3.54 |
| **Compound 118** | 3.57 | **Compound 119** | 17.77 |
| **Compound 120** | 1.97 | **Compound 122** | 36.00 |
| **Compound 123** | 13.72 | **Compound 124** | 38.53 |
| **Compound 125** | 38.00 | **Compound 126** | 7.10 |
| **Compound 127** | 10.15 | **Compound 128** | 4.80 |
| **Compound 129** | 36.00 | **Compound 130** | 23.90 |
| **Compound 131** | 4.68 | **Compound 133** | 21.99 |
| **Compound 134** | 13.00 | **Compound 135** | 4.40 |
| **Compound 136** | 1.34 | **Compound 137** | 1.54 |
| **Compound 138** | 1.80 | **Compound 139** | 23.51 |
| **Compound 140** | 7.29 | **Compound 141** | 4.00 |
| **Compound 142** | 6.20 | **Compound 143** | 4.67 |
| **Compound 144** | 3.17 | **Compound 145** | 5.91 |
| **Compound 146** | 11.60 | **Compound 151** | 1.00 |
| **Compound 152** | 11.00 | **Compound 153** | 8.50 |
| **Compound 154** | 6.20 | **Compound 155** | 25.97 |

(continued)

| Compound | $IC_{50}$(nM) | Compound | $IC_{30}$(nM) |
|---|---|---|---|
| **Compound 156** | 3.50 | **Compound 158** | 25.66 |
| **Compound 159** | 16.55 | **Compound 160** | 6.20 |
| **Compound 161** | 8.13 | **Compound 162** | 5.49 |
| **Compound 164** | 12.00 | **Compound 166** | 10.22 |

**3. Conclusion:**

It can be seen from the above experimental results that the compounds prepared in the examples of this application have a good inhibitory effect on USP1 and are effective USP1 inhibitors.

**Test Example 2 USP1 CTG Test Method**

**1. Experimental scheme**

NCI-H1693 cell killing experiment was used to verify the biological activity of USP1i compound *in vitro.*

**1.1 Experimental materials:**

NCI-H1693 cells were purchased from ATCC (article number: CRL-5866) and cultured in a cell incubator with 5% $CO_2$ (containing 5% $CO_2$ and 95% air by volume) at 37°C; 1640 complete medium: comprising 94 wt% RPMI-1640 liquid medium (Gibco, article number: 11875-093), 5 wt% FBS (Gibco, article number: 10099-141), and 1 wt% Pen Strep (Gibco, article number: 15070-063); Fluorescent 384-well plate (Perkin Elmer, article number: 6007279); Trypsin (Gibco, article number: 25200056); CTG Buffer (CellTiter-Glo® 2.0 Cell Viability Assay, Promega, article number: G9241).

**1.2 Test sample:**

Compounds of table 4 in this application; wherein the structural formulae and preparation methods of compound 58 and compound 88 are shown in the above examples;

comparative compound 1 represented by the chemical formula:

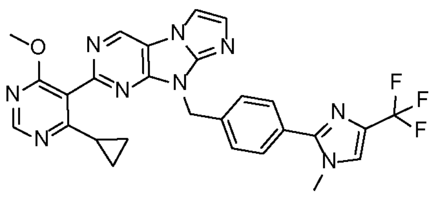

;

and

comparative compound 2 represented by the chemical formula:

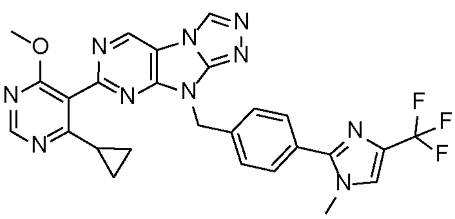

.

**1.3 Experimental process:**

(1) Digesting NCI-H1693 cells in a 75 $cm^2$ culture flask with 2 mL trypsin for 2 to 3 minutes, and then neutralizing the resultant with 2 mL 1640 complete medium; centrifuging the resultant at 1200 rpm for 5 minutes; removing the supernatant, and collecting the cell precipitate and resuspending with 4 mL of 1640 complete medium; taking 500 μL cell suspension and counting the cells with Vi-CELL-XR cell counter.
(2) Inoculating 500 NCI-H1693 cells per well (each well contains 40 μL 1640 complete culture medium) in a fluorescent 384-well plate with a Multidrop instrument, and adding drugs after 24 hours.
(3) Diluting the test sample (concentration: 3.33 mM, dissolved in DMSO) in a 1:3 ratio to obtain 9 concentrations (10000 nM, 3333 nM, 1111 nM, 370 nM, 123 nM, 41 nM, 13.7 nM, 4.6 nM, and 1.5 nM) using an ultra-micro sampler, with double-holes set for each concentration, and adding drugs; wherein 9 holes are set for each of the positive control and the blank control, the positive control being double-holes comprising 10 μM positive compound and the blank control being double-holes comprising DMSO.
(4) Putting the cells after adding drugs into an incubator with 5% $CO_2$ (containing 5% $CO_2$ by volume and the rest being air) at 37°C and culturing for 6 days; and after 6 days, adding 25 μL CTG buffer to each well for CTG detection, and reading the plate using an enzyme-labeled instrument.
(5) Analyzing the CTG readings:

setting the average inhibition rate of the positive control as the relative inhibition rate of 100%;
setting the average value of the negative control was set as the relative inhibition rate of 0%;
converting the CTG reading value into a relative inhibition rate, and converting the inhibition rate (inhibition%) of the compounds in each concentration to cells according to the following formula:

$$\text{Inhibition\%} = (b-x)/(b-a) \times 100\%;$$

a = CTG value (hole of maximum concentration)
b = CTG value (hole of blank control)
x = CTG value (x nM);

(6) Calculating $IC_{50}$ with GraphPad PRISM 8:
gathering the corresponding concentrations and inhibition rates of 10000 nM, 3333 nM, 1111 nM, 370 nM, 123 nM, 41 nM, 13.7 nM, 4.6 nM and 1.5 nM, and performing statistical calculation by Log 10 (compound concentration):

inputting data value into GraphPad PRISM 8, selecting "Analysis", "Nonlinear regression (curve fit)", "Log (inhibitor) vs. response-Variable slope", selecting the calculation formula, and calculating according to the following formula:

$$Y = \text{Bottom} + (\text{Top}-\text{Bottom})/(1+10\text{^}((\text{Log}IC_{50}-X) \times \text{HillSlope}))$$

X: Log10 (compound complex concentration);
Y: corresponding response;
Top and Bottom: corresponding response corresponding to the same unit of Y; and
it is noted that:

- if X is not the logarithm of dosage, data shall be returned and converted;
- if any baseline response is subtracted, the Bottom is set as a constant value of 0.0; and fitting the data to get the $IC_{50}$ value;

wherein the fitting conditions may be adjusted according to the specific conditions of the data; specifically, Constraint conditions of Bottom, TOP and HillSlope may be adjusted appropriately so as to achieve the curve that is most in line with the actual situation.

**2. Experimental results:**

See Table 4 for the results of inhibition of NCI-H1693 cells achieved by some examples of the compounds in this

application.

**Table 4** Inhibitory results of NCI-H1693 cells

| Compound | $IC_{50}$ (nM) |
|---|---|
| **Compound 58** | 13.0 |
| **Compound 88** | 9.2 |
| **Comparative compound 1** | 53.7 |
| **Comparative compound 2** | 174 |

**3. Conclusion:**

It can be seen from the above experimental results that the compounds of the present application have a significant cell inhibitory activity.

**Test Example 3 Pharmacokinetic Determination of the Compound of the Present Application in Male CD1 Mice**

**1. Experimental scheme:**

Male CD1 mice were used as test animals. After intravenous injection and oral administration, plasma samples were collected at specific time points. The concentration of the compound in plasma was detected by LC-MS/MS, and the parameters of drug metabolism and pharmacokinetics (DMPK) were calculated to evaluate the pharmacokinetic characteristics of the compounds in plasma of mice.

**1.1 Test sample:**

Compounds of Table 5 in this application; the structural formula and preparation method of compound 88 are shown in the above examples. The structural formula of comparative compound 1 is:

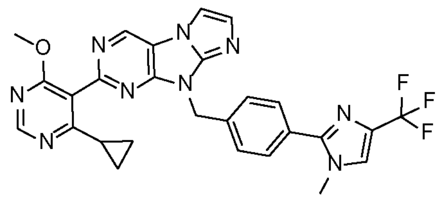

.

**1.2 Experimental animals:**

Male CD1 mice having a weight of between 20 g and 30 g; supplied by Vital River.

**1.3 Administration:**

Administration of the compound in this application: both group IV (intravenous injection group) and group PO (oral administration group) consist of 3 mice; for group IV, the dosage is 1 mg/kg and the administration volume is 5 mL/kg; and for group PO, the dosage is 10 mg/kg and the administration volume is 10 mL/kg. The administration solvent was 5 vol% DMSO+10 vol% Soltol (Kolliphor HS 15) + 85 vol% Saline (physiological saline).

**1.4 Sample collection:**

0.02 to 0.03 mL of blood was collected from the saphenous vein in group IV at 0.083, 0.25, 0.5, 1, 2, 4, 8 and 24 hours after administration and from the saphenous vein in group PO at 0.25, 0.5, 1, 2, 4, 8 and 24 hours after administration. The collected samples were placed in EDTA-K2 test tube; plasma was separated by centrifuging at 4°C for 44 hours, which was then stored at -80°C until analysis of the samples.

**1.5 Sample treatment:**

Mice plasma samples were treated by:

1) adding 200 μL acetonitrile to 10 μL plasma sample for precipitation, vortex mixing the mixture for 30 seconds and centrifuging at 4°C and 3900 rpm for 15 minutes; and
2) diluting the obtained supernatant with water for 3 folds, and analyzing the concentration of compounds to be detected by LC/MS/MS.

**2. Experimental results:**

Table 5

| DMPK \ Compound | Compound 88 | Comparative compound 1 |
|---|---|---|
| Administration mode/Dosage | PO/10 mg/kg | PO/10 mg/kg |
| Curve area | 44062 | 6558 |
| Blood concentration | 4803 | 2246 |

**3. Conclusion:**

It can be seen from the above experimental results that the compounds of this application have obvious pharmacokinetic advantages compared with the control compound.

**Test Example 4 Pharmacokinetic Determination of Compounds of the Present Application in Male Beagle Dogs.**

**1. Experimental scheme:**

Male Beagle dogs were used as test animals. After intravenous injection and oral administration, plasma samples were collected at specific time points. The concentration of the compound in plasma was detected by LC-MS/MS, and the DMPK parameters were calculated to evaluate the pharmacokinetic characteristics of the compound in dog plasma.

**1.1 Test sample:**

Compounds of Table 6 in this application. The structural formulas and preparation methods of compound 58 and compound 88 are shown in the above examples. The structural formula of KSQ-4279 is:

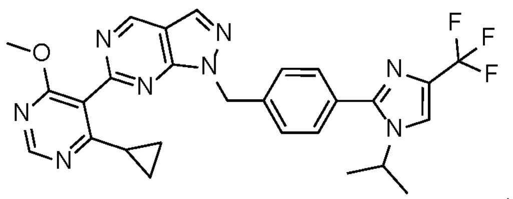

**1.2 Experimental animals:**

Male Beagle dogs having a weight of 9 to 13 kg, supplied by Beijing Mars Biotechnology Co., Ltd.

**1.3 Administration:**

Administration information of the compound in this application: both group IV (intravenous injection group) and

group PO (oral administration group) consist of 3 dogs; for group IV, the dosage was 0.2 mg/kg and the administration volume was 0.5 mL/kg; for group PO, the dosage was 3 mg/kg and the administration volume was 3 mL/kg. The administration solvent was 5 vol% DMSO+10 vol% Solutol (Kolliphor HS 15)+85 vol% Saline (physiological saline).

**1.4 Sample collection:**

0.5 mL of blood was collected from the head vein in group IV at 0.083, 0.25, 0.5, 1, 2, 4, 8 and 24 hours respectively after administration, and from the head vein in group PO at 0.25, 0.5, 1, 2, 4, 8 and 24 hours respectively after administration. The collected samples were placed in EDTA-K2 test tube; plasma was separated by centrifuging at 2 to 8°C and 2000 g for 10 minutes, which was then stored at -80°C until analysis of the samples.

**1.5 Sample treatment:**

Dog plasma samples were treated by:

1) adding 200 μL acetonitrile to 50 μL plasma sample for precipitation, vortex mixing the mixture for 30 seconds, and centrifuging at 4°C and 3900 rpm for 15 minutes; and
2) diluting the obtained supernatant with water for 3 folds, and analyzing the concentration of the compounds to be detected by LC/MS/MS.

**2. Experimental results:**

Table 6

| Compound / DMPK | KSQ-4279 | Compound 58 | Compound 88 |
|---|---|---|---|
| Administration mode/ Dosage (mg/kg) | IV/0.2 | IV/0.2 | IV/0.2 |
| CL (L/h/kg) | 1.7 | 0.95 | 0.90 |
| $AUC_{last}$ (h*ng/mL) | 124 | 190 | 236 |

Note: "CL" in Table 6 represents the overall clearance rate; "$AUC_{last}$" represents the area under the plasma concentration-time curve from 0 to the final quantifiable time point.

**3. Conclusion:**

From the above experimental results, it can be seen that the compounds of this application has obvious advantages in clearance level and drug exposure amount compared with the control compound.

**Test Example 5 Pharmacodynamic Experiment of OV0589 Patient-derived Xenograft (PDX)**

**1. Experimental scheme:**

Female BALB/c nude mice were used as test animals. After oral administration of the compound of this application, the tumor was measured and weighed regularly, and the tumor growth inhibition and tolerance in different administration groups were investigated. After the investigate, plasma and tumor were collected at a specific time point for pharmacokinetic analysis/pharmacodynamics (PK/PD) analysis.

**1.1 Test sample:**

Compound 58; structural formula and preparation method are shown in the above examples.
Structural formula of compound KSQ4279 is:

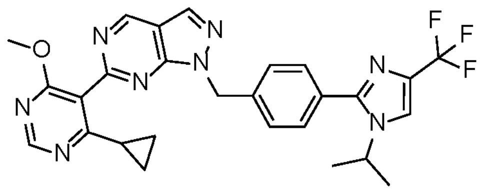

.

### 1.2 Experimental materials:

Tumor-bearing mouse tumor (R10P8) was purchased from Sino-American Crown Biotechnology (Beijing) Co., Ltd. Ubiquityl-PCNA (Lys 164) (D5C7P) mAb antibody was purchased from Cell Siganaling company, and the article number is 13439S. PCNA (PC10) antibody was purchased from Santa Cruz company, and the article number is sc-56.

### 1.3 Experimental animals:

Female BALB/c nude mice having a weight of between 21 and 25 g; supplier: Beijing An Kai Yibo Biotechnology Co., Ltd.

### 2. Experimental scheme:

Under a temperature of 20°C to 26°C and a humidity of 30% to 70% and alternate illumination day and night, when the tumor (R10P8) of the tumor-bearing mice grew to a diameter of about 1 cm (the tumor size reaches 50 to 800 $mm^3$), the tumor was collected and cut into tumor blocks of about 2 to 3 $mm^3$, and the tumor block (R10P8) was inoculated subcutaneously into the right anterior scapula of female BALB/c nude mice. The tumor growth in mice was observed regularly. After 35 days of inoculation, when the tumor (R10P8) grew to an average volume of about 150 $mm^3$ (the range of enrollment was 86.54 $mm^3$ to 262.96 $mm^3$), according to the tumor size and the weight of mice, StudyDirectorTM (version No.3.1.399.19, supplied by Studylog System, Inc., S. San Francisco, California, USA) was used for random grouping, and the administration was performed for these groups the next day.

Twelve mice were randomly divided into four groups, namely, G1 to G4. Group G1 is the solvent control group, and the solvent is: 10 vol% DMSO + 20 vol% Solutol + 70 vol% water + 5 vol% DMSO + 10 vol% Solutol + 85 vol% saline. Group G2 was orally administrated with KSQ4279 at a dosage of 100 mg/kg (mpk), and the solvent was 5 vol% DMSO + 10 vol% Solutol + 85 vol% saline. Group G3 was orally administrated with compound 58 at a dosage of 30 mg/kg, and the solvent was 10 vol% DMSO + 20 vol% Solutol + 70 vol% water. Group G4 was orally administrated with compound 58 with a dosage of 100 mg/kg, and the solvent was 10 vol% DMSO + 20 vol% Solutol + 70 vol% water.

The tumor growth in mice was observed regularly, and the tumor volume was measured (see FIG. 1), and the change of body weight of mice was observed regularly (see FIG. 2). After 36 days of administration, plasma and tumor were collected for PK/PD analysis.

PK detection: pharmacokinetics in mice was performed as described in the previous Test Example 3.

PD detection: tumor samples were collected 8 hours after the last administration, and the tumor samples were divided into 50 to 100 mg tumor specimens. 1×Tissue lysate was prepared by using dd$H_2O$, and then 20 μL of tissue lysate (Cell Signaling Cell Lysate Buffer (10×); article number: #9803) per mg of sample was added. Tumor samples were ground with a tissue grinder. The tumor sample lysate upon grinding was cracked on ice for 30 min, then centrifuged at 12,000 rpm and 4°C for 15 min. The supernatant was collected, and PD markers (Ubiquityl-PCNA/PCNA) in the samples were detected by protein blot test.

### 3. Experimental results:

FIG. 1 shows the tumor growth of mice in groups G1 to G4. From FIG. 1, it can be seen that compound 58 (dosage of 100 mg/kg) has the best inhibitory effect on tumor growth, followed by compound 58 (dosage of 30 mg/kg), and KSQ4279 (dosage of 100 mg/kg) is obviously worse than compound 58 (dosage of 100 mg/kg). FIG. 2 shows the changes of body weight of mice in groups G1 to G4. As can be seen from FIG. 2, the changes of body weight of mice in groups G1 to G4 are within the normal range and indicates a good tolerance.

Table 7 shows the statistical results of tumor growth inhibition rate of mice in groups G2 to G4. From Table 7, it can be seen that compound 58 (dosage of 100 mg/kg) has the best inhibitory effect on tumor growth, followed by compound 58 (dosage of 30 mg/kg), and KSQ4279 (dosage of 100 mg/kg) is obviously not as effective as compound 58 (dosage of 100 mg/kg) and compound 58 (dosage of 30 mg/kg).

**Table 7 Statistical results of tumor growth inhibition rate**

| Groups (N=3) | TGI% (Day 36 ) |
|---|---|
| **Group G2** | 85.7 |
| **Group G3** | 97.5 |
| **Group G4** | 107.7 |
| Note: "TGI%" represents the tumor growth inhibition rate; TGI% = [1-ΔT/C]×100%, ΔT/C = (mean (T)-mean (T0))/(mean (C)-mean (C0)), wherein T and C are the average tumor volumes of the administration group and the solvent control group on Day 38, respectively, and T0 and C0 are the average tumor volumes of the administration group and the solvent control group on Day 0, respectively. | |

**4. Conclusion:**

As can be seen from the above data, compared with control compounds, the compounds of the present application have obvious advantages in inhibiting tumor growth, and the dosage of administration is much lower than that of control compounds.

## Claims

1. A compound represented by formula (II'), or an isomer thereof or a pharmaceutically acceptable salt thereof,

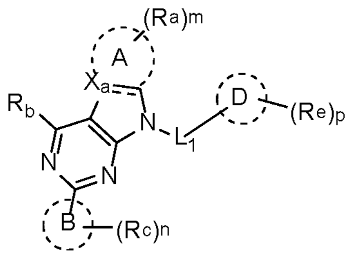

(II')

wherein,

$X_a$ is C or N;
ring A is selected from the group consisting of phenyl, 5-6 membered heteroaryl, $C_{5-6}$ cycloalkyl, and 5-6 membered heterocyclyl; $R_a$ are each independently selected from the group consisting of deuterium, halogen, $C_{1-4}$ alkyl, $C_{1-4}$ haloalkyl, $C_{1-4}$ alkoxy, oxo, cyano, amino, hydroxy, aminosulfonyl, $C_{1-4}$ alkylsulfonyl, carbamoyl, $C_{1-4}$ alkylamino, $C_{3-6}$ cycloalkyl, $C_{1-4}$ alkylsulfonylamino, dimethylphosphonoyl, -$C_{1-4}$ alkyl-OH, -$COOC_{1-4}$ alkyl, $C_{1-4}$ alkyl-$SO_2$-$NR_f$-, HO-$C_{1-4}$ alkyl-$SO_2$-$NR_f$-, 3-6 membered heterocyclyl, -$C_{1-4}$ haloalkyl-OH, ($C_{1-4}$ alkyl)$_2$P(O)-,

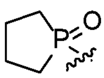 , 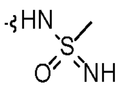 , 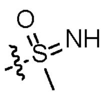 , 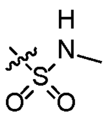 ,

deuterated $C_{1-4}$ alkyl, and 4-6 membered heterocycloalkyl; wherein C atom(s) in the $C_{1-4}$ alkyl and $C_{1-4}$ haloalkyl is/are optionally substituted with N or O; $R_f$ is $C_{3-6}$ cycloalkyl or $C_{1-4}$ alkyl; m is 0, 1, 2, 3, or 4;
$R_b$ is H or $C_{1-4}$ alkyl;
ring B is selected from the group consisting of phenyl, 5-10 membered heteroaryl, and 5-10 membered heterocyclyl; $R_c$ are each independently selected from the group consisting of halogen, $C_{1-4}$ alkyl, $C_{1-4}$ alkoxy, $C_{3-6}$ cycloalkyl, $C_{1-4}$ haloalkyl, $C_{1-4}$ haloalkoxy, and deuterated $C_{1-4}$ alkyl; n is 0, 1, 2, 3, or 4;
ring D is selected from the group consisting of phenyl, 5-6 membered heteroaryl, and 9-18 membered fused-heterocyclyl; $R_e$ is selected from the group consisting of deuterium, $C_{1-4}$ alkyl, $C_{1-4}$ haloalkyl, $C_{1-4}$ alkoxy, ring C,

and halogen, wherein ring C is optionally substituted with one $R_d$; ring C is selected from the group consisting of 5-10 membered heteroaryl comprising 1 to 4 N atom(s) and 8-10 membered fused-heterocyclyl comprising 1 to 4 N atom(s); $R_d$ are each independently selected from the group consisting of $C_{1-4}$ alkyl, $C_{1-4}$ haloalkyl, $C_{1-4}$ alkoxy, deuterated $C_{1-4}$ alkyl, and $C_{3-6}$ cycloalkyl; 1 is 1, 2, 3, or 4; p is 1, 2, 3, or 4;
$L_1$ is selected from the group consisting of $C_{1-4}$ alkylene, $C_{3-6}$ cycloalkylene and a chemical bond;
when ring A is 5-6 membered heteroaryl, structural unit

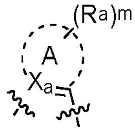

is not

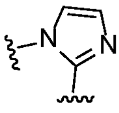,

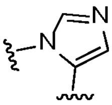, 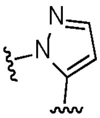, 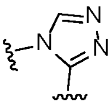, 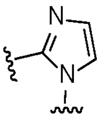, 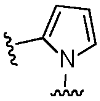, 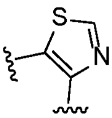, 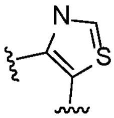, or 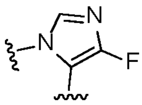;

and
when ring A is 5-6 membered heterocyclyl, structural unit

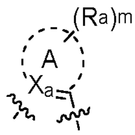

is not

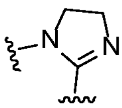.

**2.** The compound or the isomer thereof or the pharmaceutically acceptable salt thereof according to claim 1, having a structure represented by formula (I'):

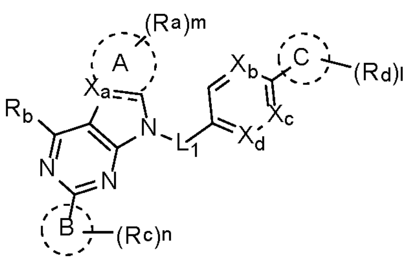

(I')

wherein,

$X_a$ is C or N;
$X_b$, $X_c$, and $X_d$ are each independently selected from the group consisting of CH, N, and CR', wherein R' is

selected from the group consisting of halogen, $C_{1-4}$ alkyl, $C_{1-4}$ haloalkyl, and $C_{1-4}$ alkoxy;
ring A is selected from the group consisting of phenyl, 5-6 membered heteroaryl, $C_{5-6}$ cycloalkyl, and 5-6 membered heterocyclyl; $R_a$ are each independently selected from the group consisting of deuterium, halogen, $C_{1-4}$ alkyl, $C_{1-4}$ haloalkyl, $C_{1-4}$ alkoxy, oxo, cyano, amino, hydroxy, aminosulfonyl, $C_{1-4}$ alkylsulfonyl, carbamoyl, $C_{1-4}$ alkylamino, $C_{3-6}$ cycloalkyl, $C_{1-4}$ alkylsulfonylamino, dimethylphosphonoyl, -$C_{1-4}$ alkyl-OH, -$COOC_{1-4}$ alkyl, $C_{1-4}$ alkyl-$SO_2$-$NR_f$-, HO-$C_{1-4}$ alkyl-$SO_2$-$NR_f$-, 3-6 membered heterocyclyl, -$C_{1-4}$ haloalkyl-OH, ($C_{1-4}$ alkyl)$_2$P(O)-,

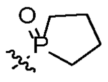, 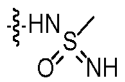, 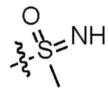, and 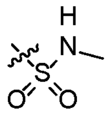;

wherein C atom(s) in the $C_{1-4}$ alkyl and $C_{1-4}$ haloalkyl is/are optionally substituted with N or O; $R_f$ is $C_{3-6}$ cycloalkyl or $C_{1-4}$ alkyl;
m is 0, 1, 2, 3, or 4;
$R_b$ is H or $C_{1-4}$ alkyl;
ring B is selected from the group consisting of phenyl, 5-10 membered heteroaryl, and 5-10 membered heterocyclyl; $R_c$ are each independently selected from the group consisting of halogen, $C_{1-4}$ alkyl, $C_{1-4}$ alkoxy, $C_{3-6}$ cycloalkyl, $C_{1-4}$ haloalkyl, and $C_{1-4}$ haloalkoxy; n is 0, 1, 2, 3, or 4;
ring C is 5-10 membered heteroaryl comprising 1 to 4 N atom(s); $R_d$ are each independently selected from the group consisting of $C_{1-4}$ alkyl, $C_{1-4}$ haloalkyl, $C_{1-4}$ alkoxy, and deuterated $C_{1-4}$ alkyl; 1 is 0, 1, 2, 3, or 4; and
$L_1$ is selected from the group consisting of $C_{1-4}$ alkylene, $C_{3-6}$ cycloalkylene and a chemical bond.

**3.** The compound or the isomer thereof or the pharmaceutically acceptable salt thereof according to claim 1, having a structure represented by formula (I'-1):

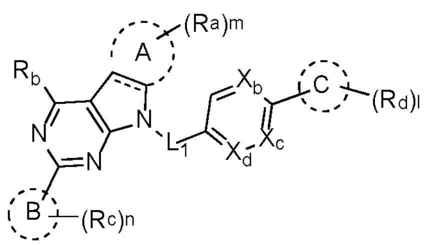

(I'-1)

wherein,

$X_b$, $X_c$, and $X_d$ are each independently selected from the group consisting of CH, N, and CR', wherein R' is halogen or $C_{1-4}$ alkoxy;
ring A is selected from the group consisting of phenyl, pyridinyl, and $C_{5-6}$ cycloalkyl; $R_a$ are each independently selected from the group consisting of deuterium, halogen, $C_{1-4}$ alkyl, $C_{1-4}$ haloalkyl, $C_{1-4}$ alkoxy, oxo, cyano, amino, hydroxy, aminosulfonyl, $C_{1-4}$ alkylsulfonyl, carbamoyl, $C_{1-4}$ alkylamino, $C_{3-6}$ cycloalkyl, $C_{1-4}$ alkylsulfonylamino, dimethylphosphonoyl, -$C_{1-4}$ alkyl-OH, -$COOC_{1-4}$ alkyl, $C_{1-4}$ alkyl-$SO_2$-$NR_f$-, HO-$C_{1-4}$ alkyl-$SO_2$-$NR_f$-, 3-6 membered heterocyclyl, -$C_{1-4}$ haloalkyl-OH, ($C_{1-4}$ alkyl)$_2$P(O)-,

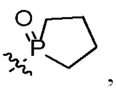, 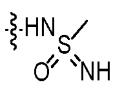, 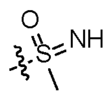, and 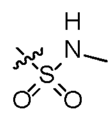;

wherein C atom(s) in the $C_{1-4}$ alkyl and $C_{1-4}$ haloalkyl is/are optionally substituted with N or O; $R_f$ is $C_{3-6}$ cycloalkyl or $C_{1-4}$ alkyl; m is 0, 1, 2, 3, or 4;
$R_b$ is H or $C_{1-4}$ alkyl;
ring B is 5-6 membered heteroaryl or 5-10 membered heterocyclyl; $R_c$ are each independently selected from the group consisting of $C_{1-4}$ alkyl, $C_{1-4}$ alkoxy, $C_{3-6}$ cycloalkyl, and halogen; n is 0, 1, 2, 3, or 4;

ring C is 5-6 membered heteroaryl comprising 1 to 4 N atom(s); $R_d$ are each independently selected from the group consisting of $C_{1-4}$ alkyl, $C_{1-4}$ haloalkyl, and $C_{1-4}$ alkoxy; 1 is 0, 1, 2, 3, or 4; and
$L_1$ is $C_{1-4}$ alkylene or $C_{3-6}$ cycloalkylene.

**4.** The compound or the isomer thereof or the pharmaceutically acceptable salt thereof according to claim 1, wherein ring D is selected from the group consisting of

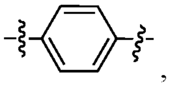,

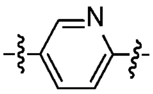, 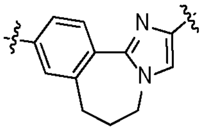, 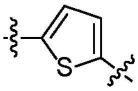, 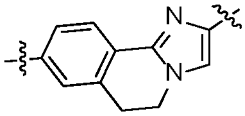, 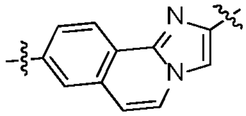,

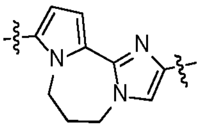, 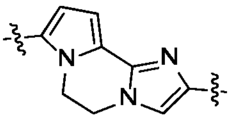, 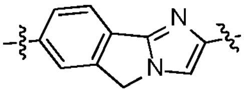, 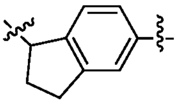, 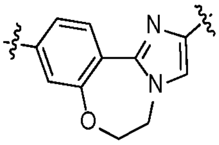,

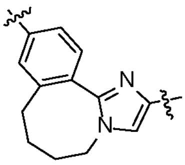, 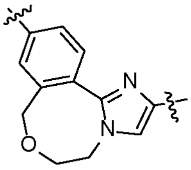, 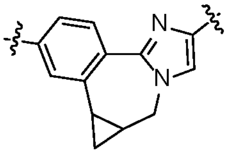, 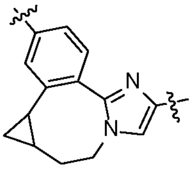, 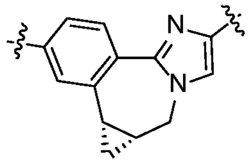,

and

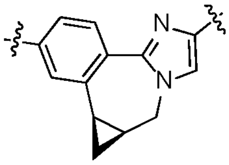.

**5.** The compound or the isomer thereof or the pharmaceutically acceptable salt thereof according to claim 1, wherein $R_e$ is selected from the group consisting of $-CF_3$,

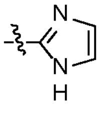, 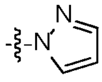,

$-OCH_3$, -F, -D, and $-CH_3$.

**6.** The compound or the isomer thereof or the pharmaceutically acceptable salt thereof according to any one of claims 1 to 5, wherein ring A is selected from the group consisting of

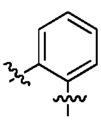, 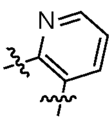, 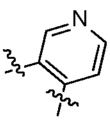, 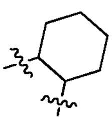, 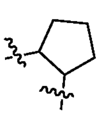, 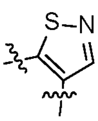, 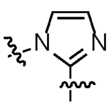, 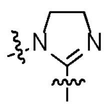, 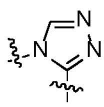,

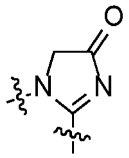, 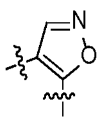, 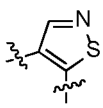, 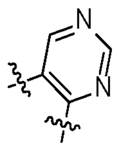, 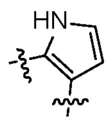, 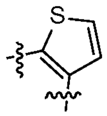, 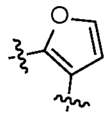, 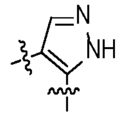,

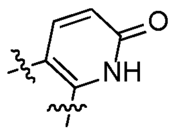, and 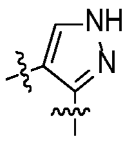.

7. The compound or the isomer thereof or the pharmaceutically acceptable salt thereof according to any one of claims 1 to 6, wherein $R_a$ is selected from the group consisting of -F, $-OCH_3$, $-CF_3$, $-COOCH_3$, $-C(CH_3)_2-OH$, $-CHF_2$, -CN,

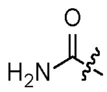, 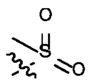,

**-Cl,** $-NH_2$,

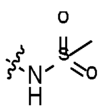,

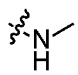,

$CH_3-NH-S(O)_2-$, $NH_2-S(O)_2-$, $-CH_3$, -OH, -Br, $-CH_2CH_3$,

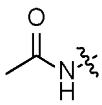, 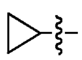, 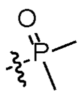,

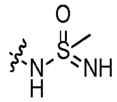,

$CH(CH_3)_2$,

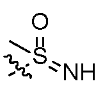, 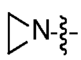, 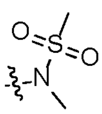, 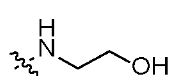, 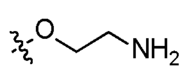, 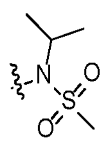,

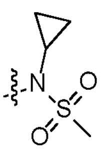, 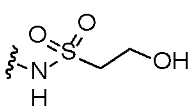, 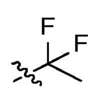, 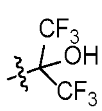, 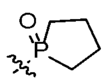, 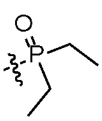,

$-OCH(CH_3)_2$,

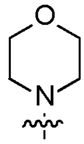,

D,

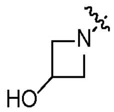, 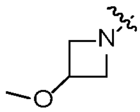, $-CD_3$, and 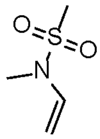.

**8.** The compound or the isomer thereof or the pharmaceutically acceptable salt thereof according to any one of claims 1 to 7, wherein structure unit

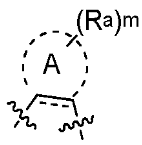

or structure unit

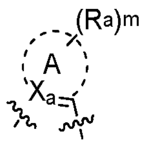

is selected from the group consisting of

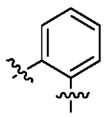, 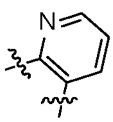, 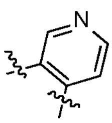, 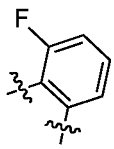,

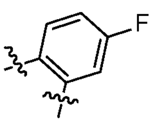, 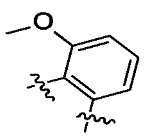, 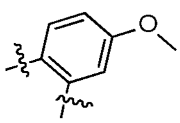, 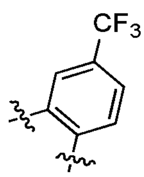, 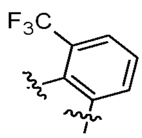, 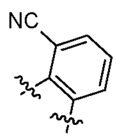, 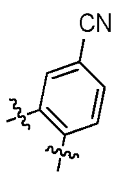,

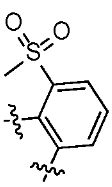, 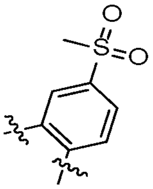, 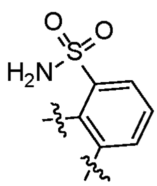, 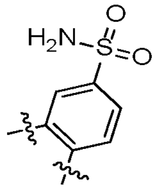, 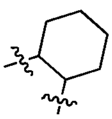, 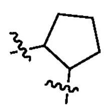, 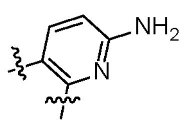,

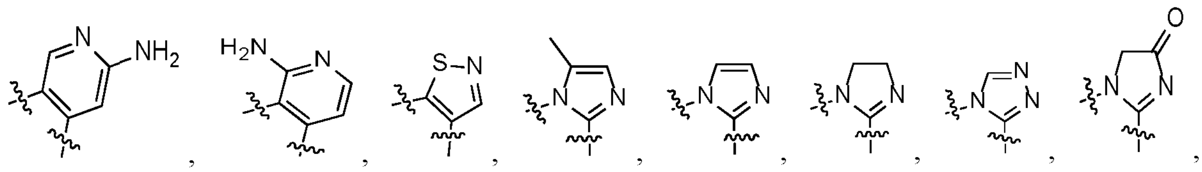
, , , , , , , ,

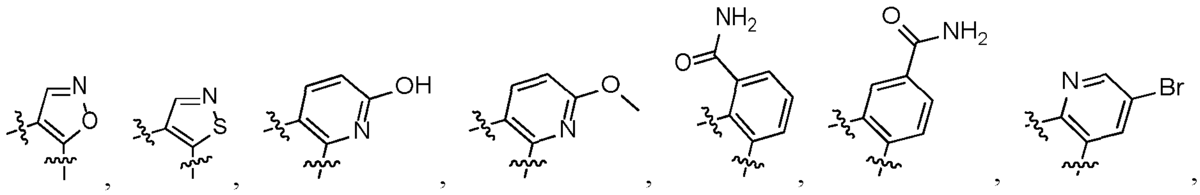
, , , , , , ,

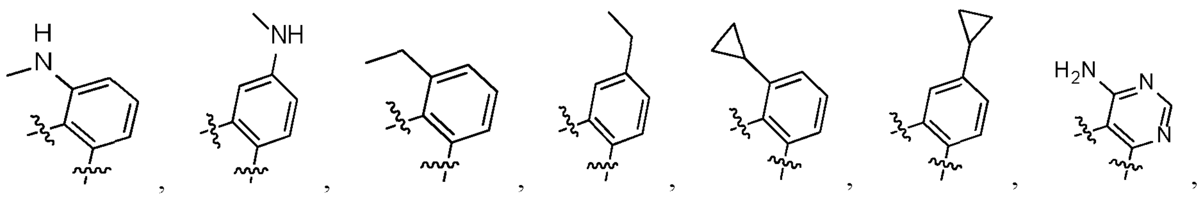
, , , , , , ,

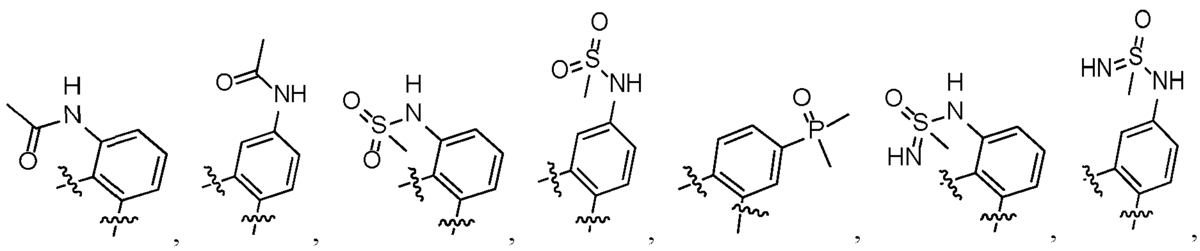
, , , , , , ,

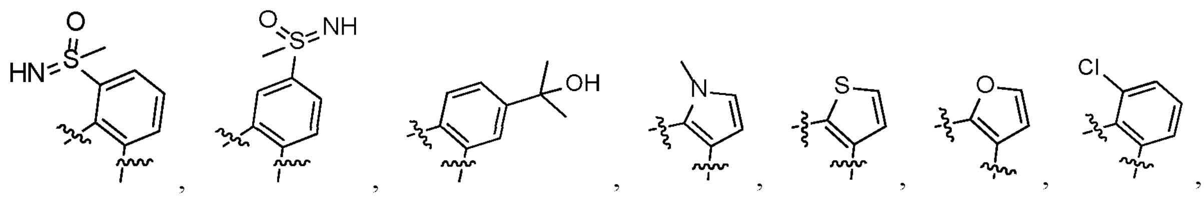
, , , , , , ,

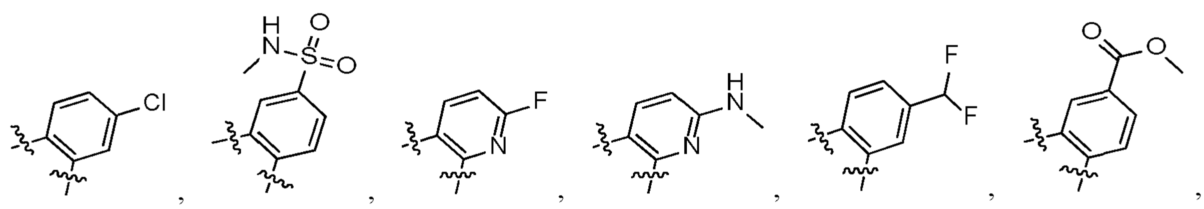
, , , , , ,

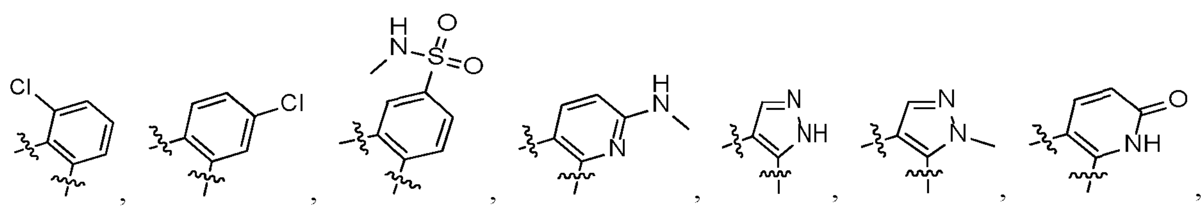
, , , , , , ,

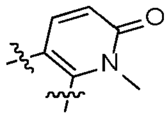, 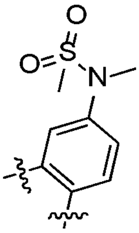, 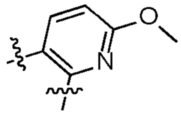, 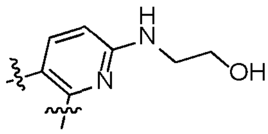, 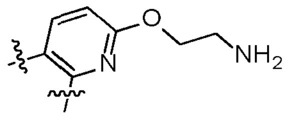,

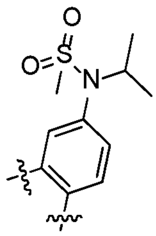, 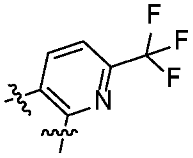, 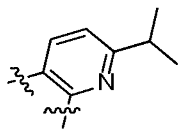, 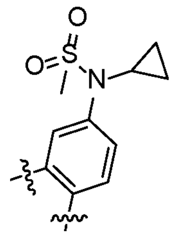, 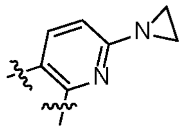,

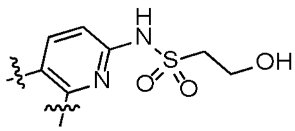, 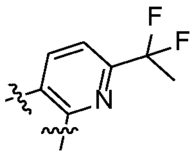, 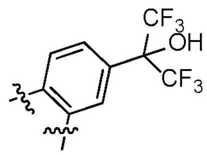, 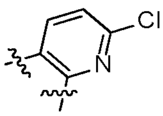, 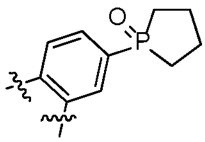,

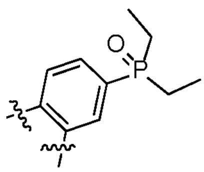, 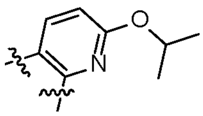, 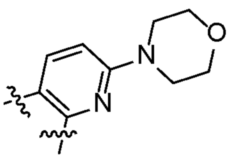, 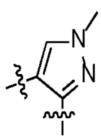, 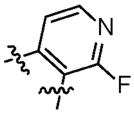, 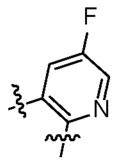,

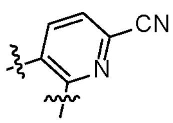, 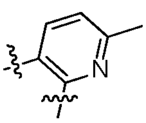, 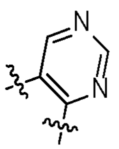, 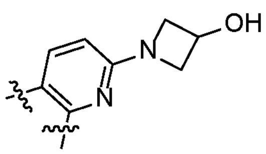, 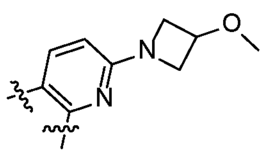,

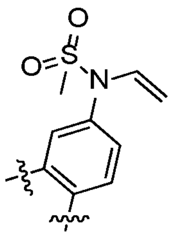, 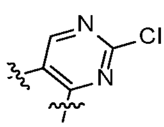, 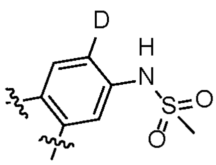, and 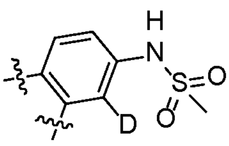.

**9.** The compound or the isomer thereof or the pharmaceutically acceptable salt thereof according to any one of claims 1 to 8, wherein,
structure unit

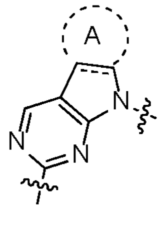

or structure unit

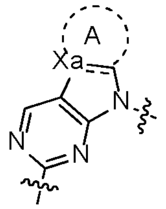

is selected from the group consisting of

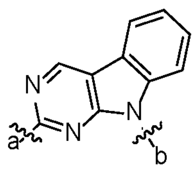, 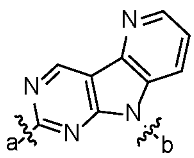, 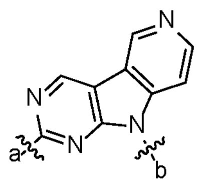, 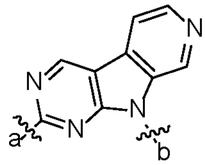,

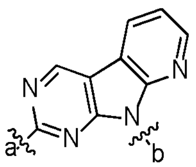, 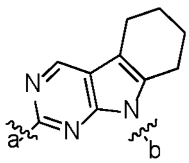, 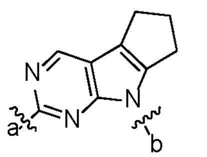, 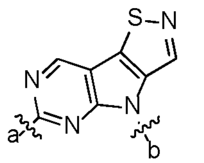, 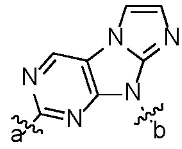,

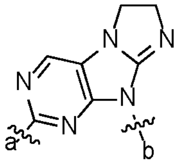, 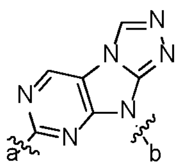, 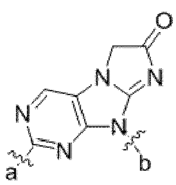, 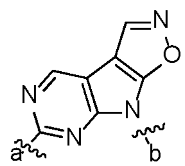, 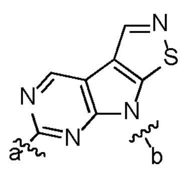,

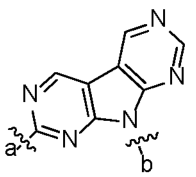, 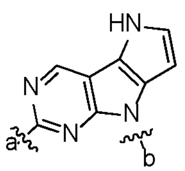, 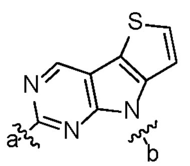, 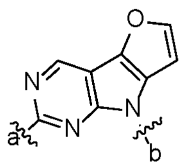, 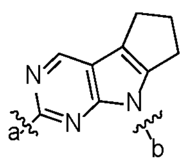,

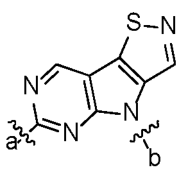, 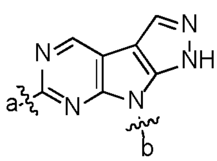, 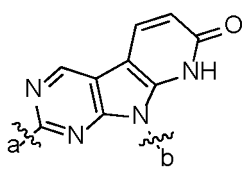, 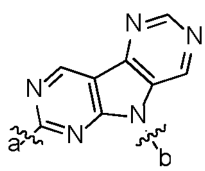,

and

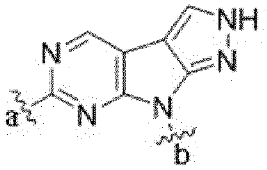,

wherein a represents the linking site with ring B, and b represents the linking site with $L_1$.

**10.** The compound or the isomer thereof or the pharmaceutically acceptable salt thereof according to any one of claims 1 to 9, wherein,
structure unit

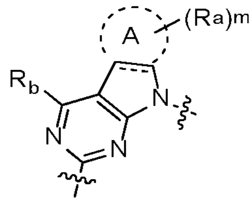

or structure unit

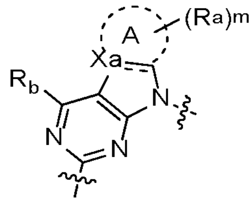

is selected from the group consisting of

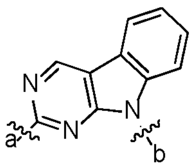, 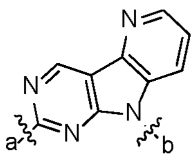, 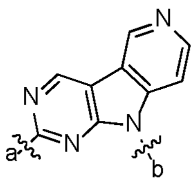, 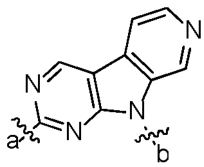,

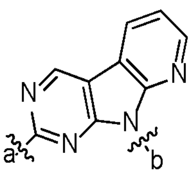, 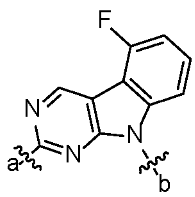, 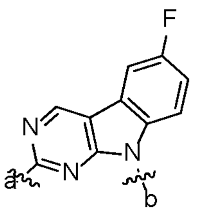, 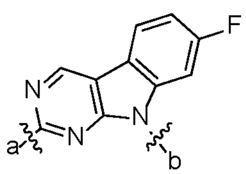, 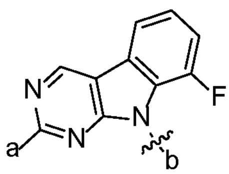,

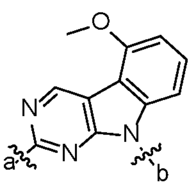, 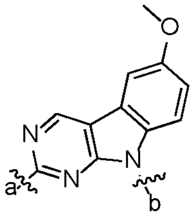, 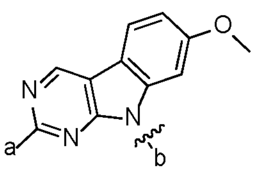, 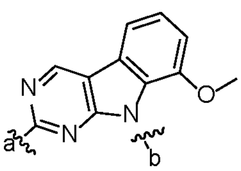, 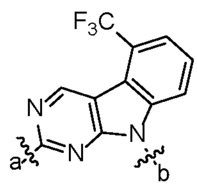,

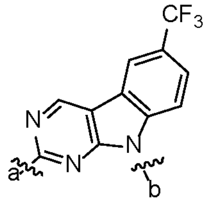 , 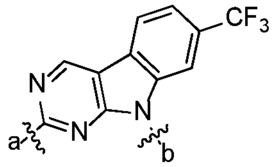 , 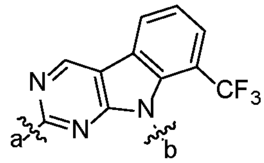 , 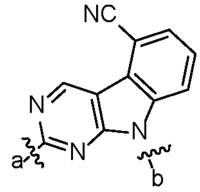 ,

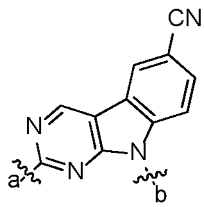 , 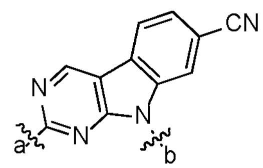 , 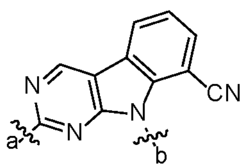 , 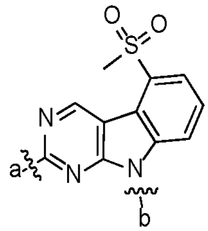 ,

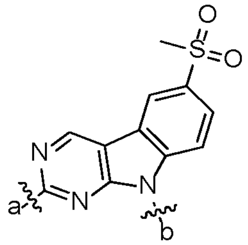 , 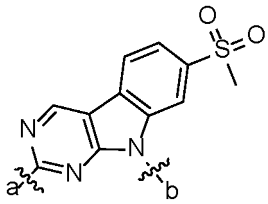 , 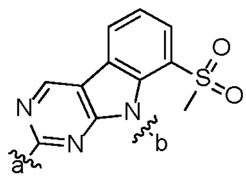 , 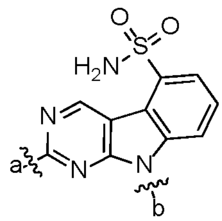 ,

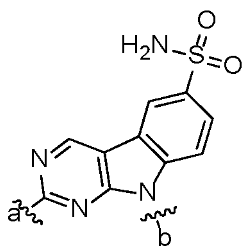 , 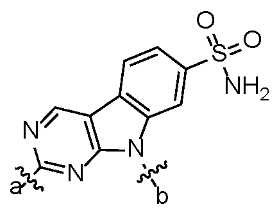 , 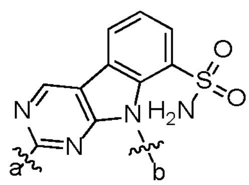 , 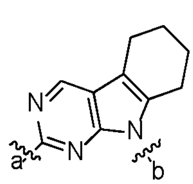 ,

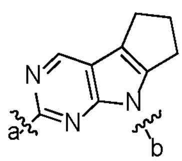 , 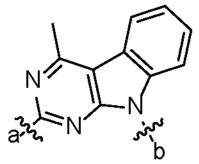 , 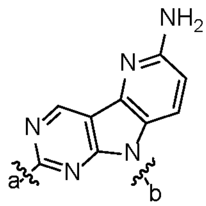 , 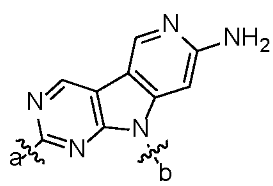 , 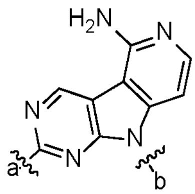 ,

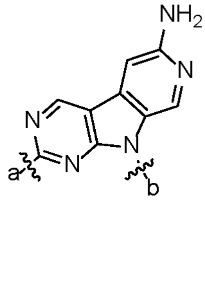 , 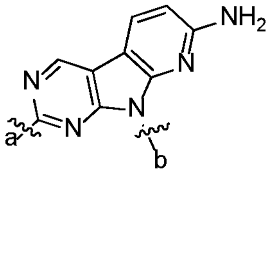 , 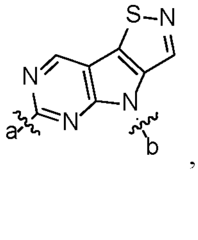 , 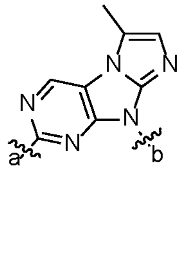 , 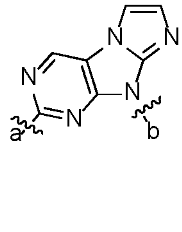 ,

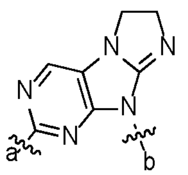 , 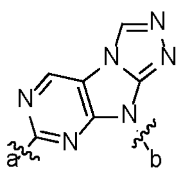 , 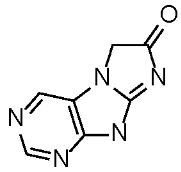 , 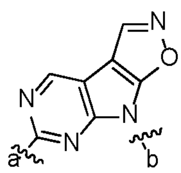 , 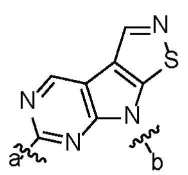 ,

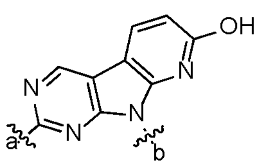 , 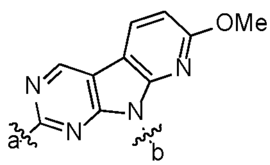 , 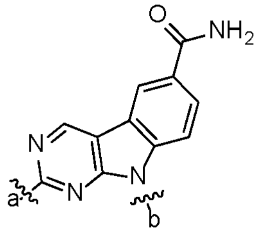 , 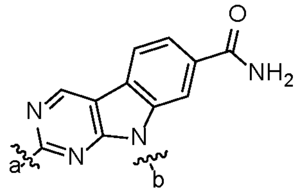 ,

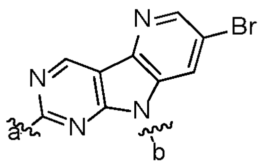 , 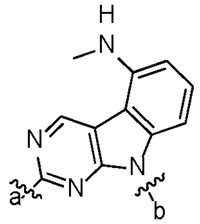 , 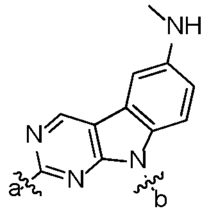 , 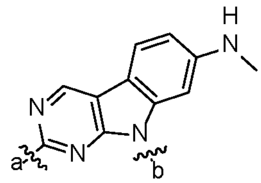 ,

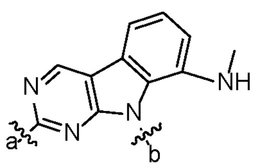 , 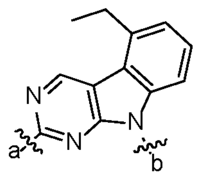 , 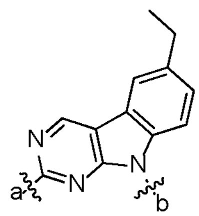 , 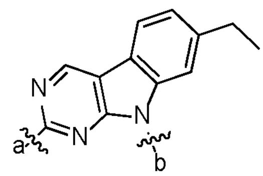 ,

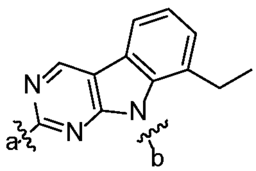 , 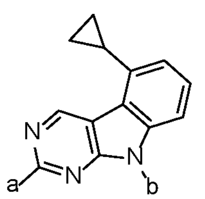 , 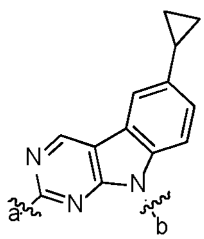 , 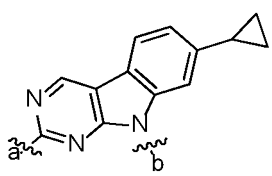 ,

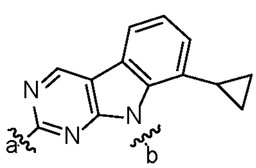 , 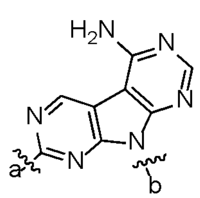 , 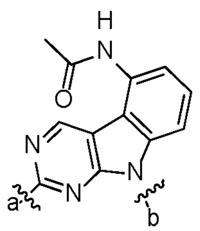 , 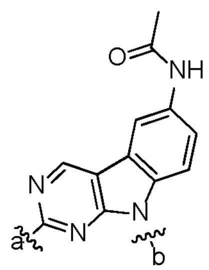 ,

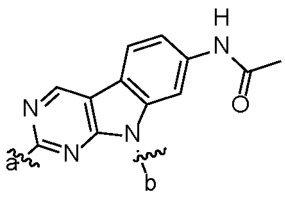,
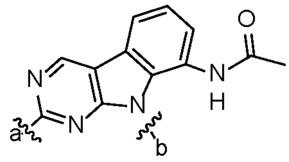,
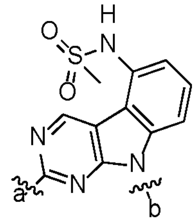,
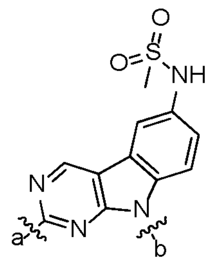,

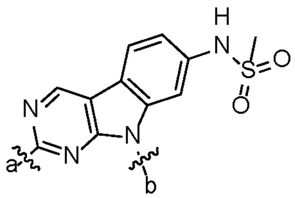,
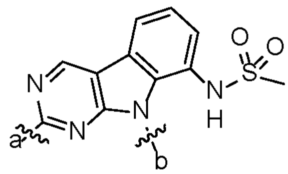,
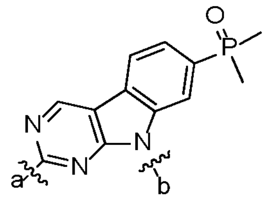,
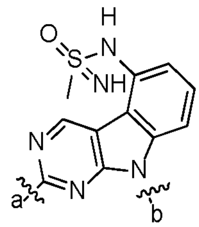,

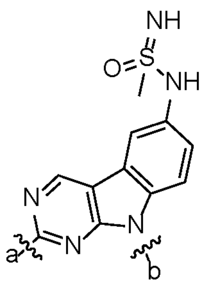,
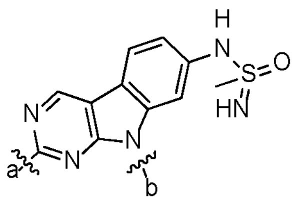,
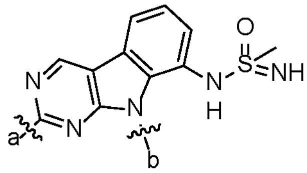,
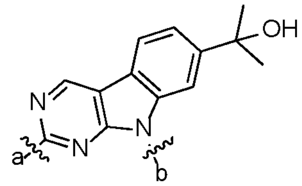,

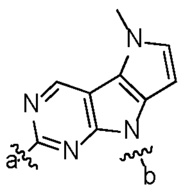,
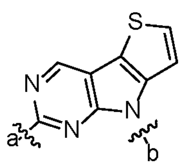,
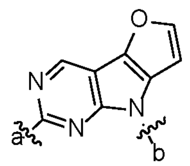,
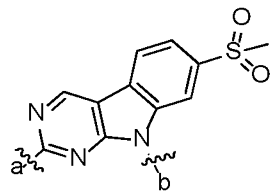,

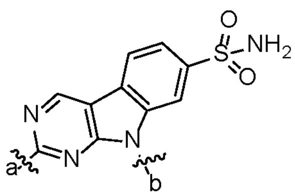,
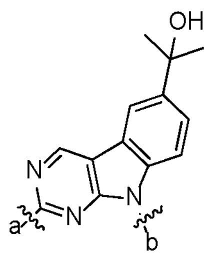,
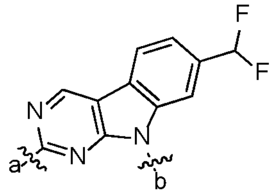,
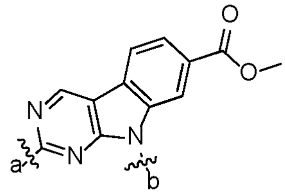,

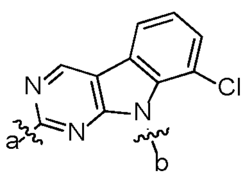,
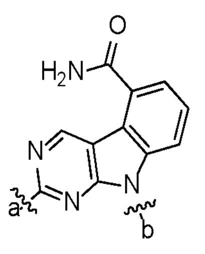,
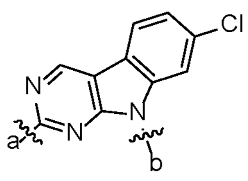,
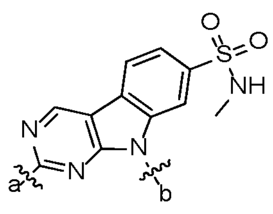,

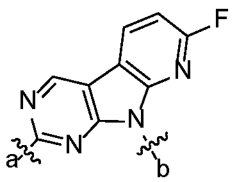 , 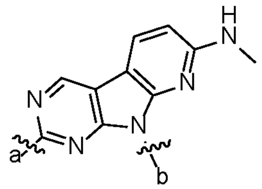 , 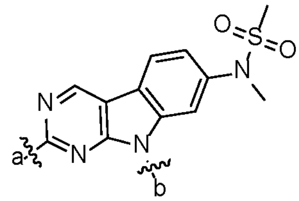 , 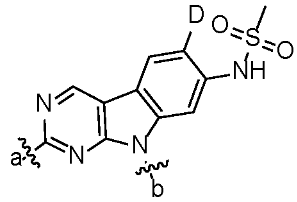 ,

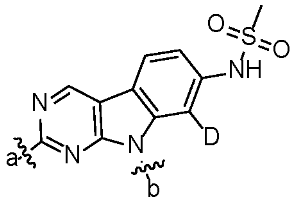 , 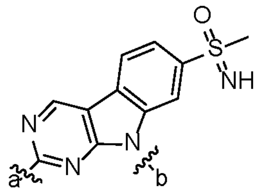 , 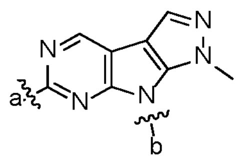 , 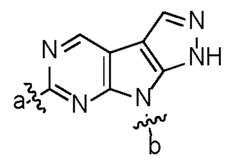 ,

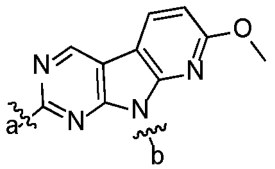 , 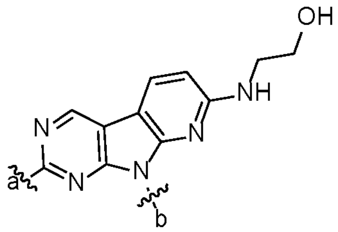 , 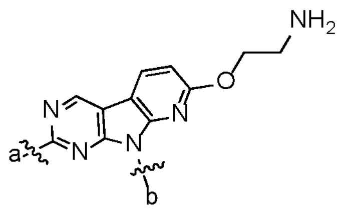 ,

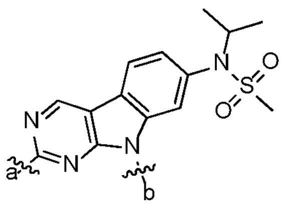 , 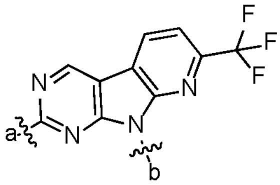 , 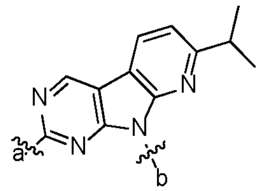 , 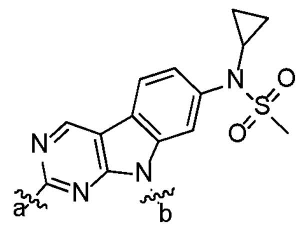 ,

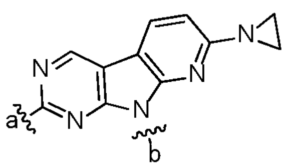 , 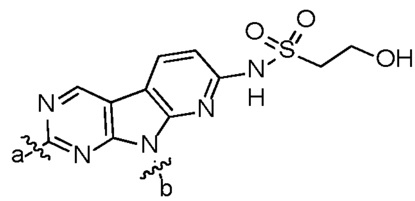 , 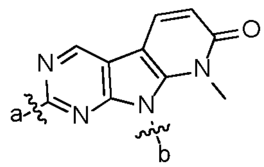 ,

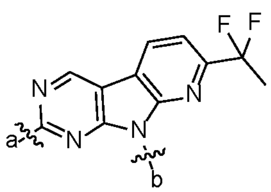 , 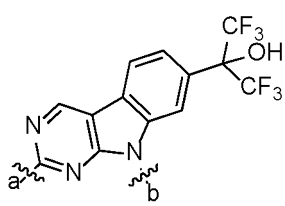 , 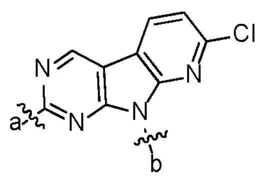 , 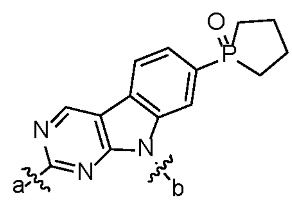 ,

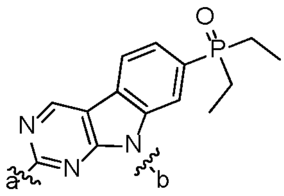 , 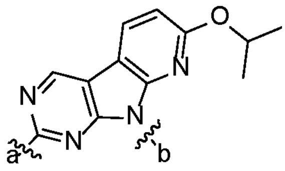 , 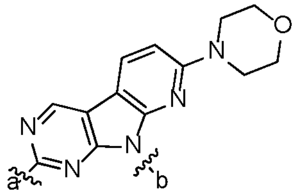 , 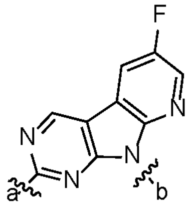 ,

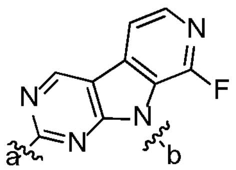 , 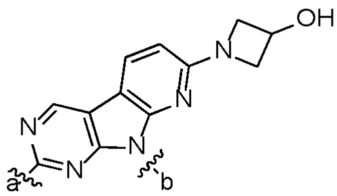 , 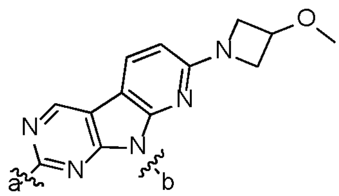 ,

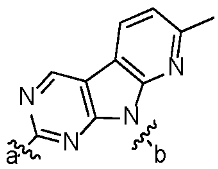 , 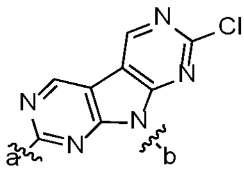 , 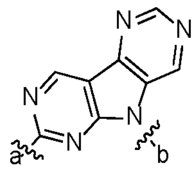 , 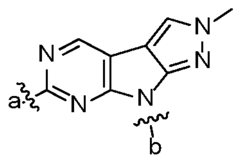 ,

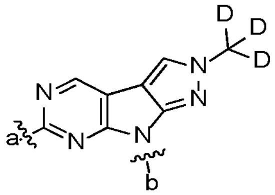 , 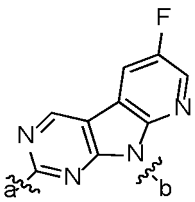 , 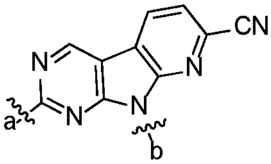 , 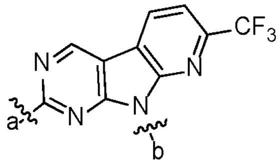 ,

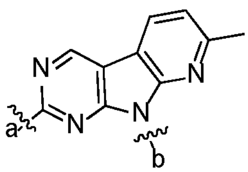 , 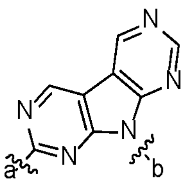 , and 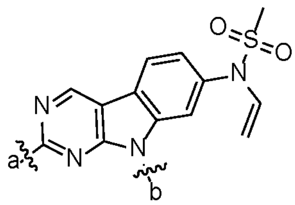 ,

wherein a represents the linking site with ring B, and b represents the linking site with $L_1$.

**11.** The compound or the isomer thereof or the pharmaceutically acceptable salt thereof according to any one of claims 1 to 10, wherein $R_b$ is hydrogen.

**12.** The compound or the isomer thereof or the pharmaceutically acceptable salt thereof according to any one of claims 1 to 11, wherein ring B is selected from the group consisting of

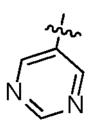 , 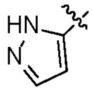 , 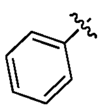 , 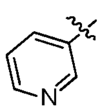 , 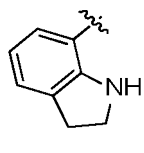 , and 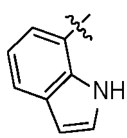 .

**13.** The compound or the isomer thereof or the pharmaceutically acceptable salt thereof according to any one of claims 1 to 12, wherein $R_c$ is selected from the group consisting of $-OCH_3$,

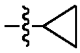,

$-CH(CH_3)_2$, -Cl, $-OCHF_2$, $-CF_3$,

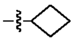,

$-CH_3$, and $-OCD_3$.

**14.** The compound or the isomer thereof or the pharmaceutically acceptable salt thereof according to any one of claims 1 to 13, wherein structure unit

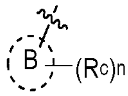

is selected from the group consisting of

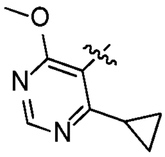, 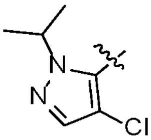, 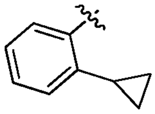, 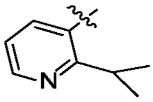, 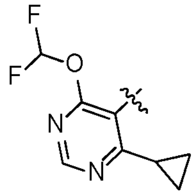,

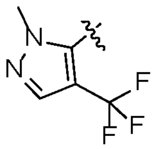, 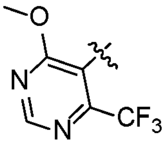, 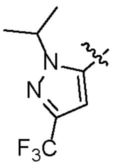, 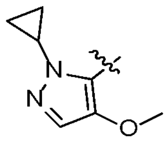, 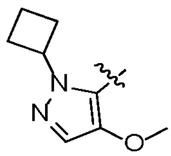, 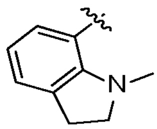,

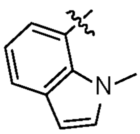, 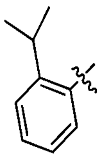, 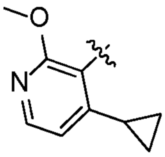, 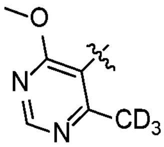, 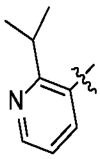, 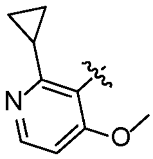, 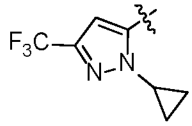,

and

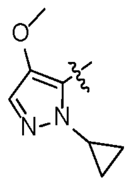.

**15.** The compound or the isomer thereof or the pharmaceutically acceptable salt thereof according to any one of claims 1 to 14, wherein ring C is selected from the group consisting of

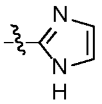, 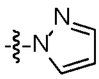, 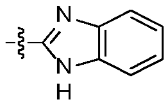, 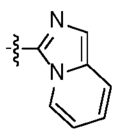, 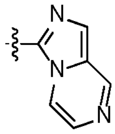, 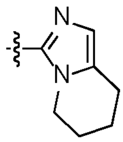, 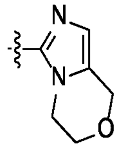, 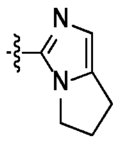,

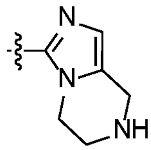, 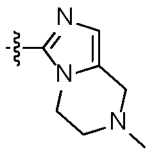, 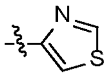, and 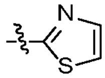.

**16.** The compound or the isomer thereof or the pharmaceutically acceptable salt thereof according to any one of claims 1 to 15, wherein $R_d$ is selected from the group consisting of $-CF_3$, $-CH_3$, $-CH_2CH_3$, $-CH(CH_3)_2$, $-OCH_2CH_3$, $-CD_3$, -Cl,

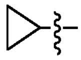,

-Br, and -F.

**17.** The compound or the isomer thereof or the pharmaceutically acceptable salt thereof according to any one of claims 1 to 16, wherein structure unit

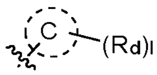

is selected from the group consisting of

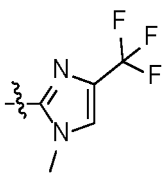, 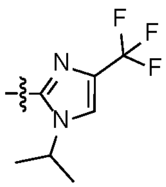, 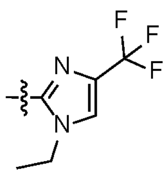, 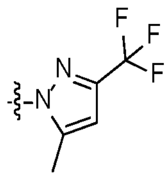, 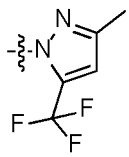,

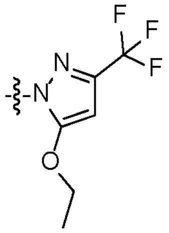, 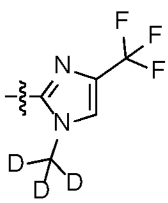, 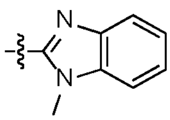, 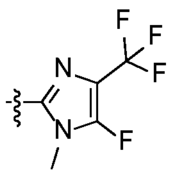, 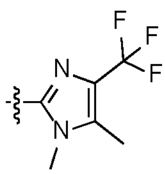, 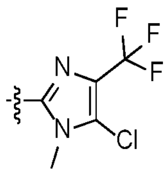,

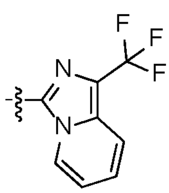, 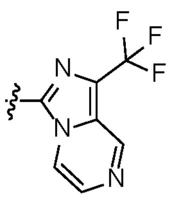, 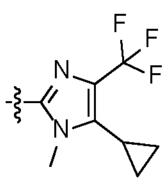, 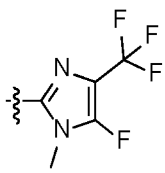, 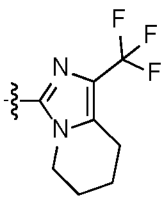, 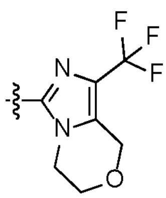,

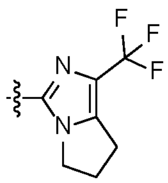, 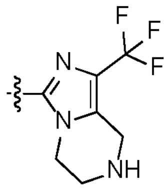, 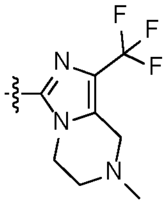, 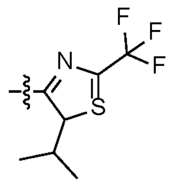, 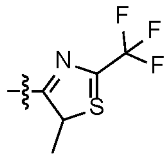, 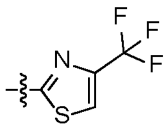,

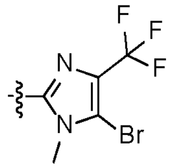, and 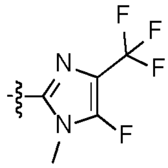.

**18.** The compound or the isomer thereof or the pharmaceutically acceptable salt thereof according to any one of claims 1 to 17, wherein $L_1$ is selected from the group consisting of $-CH_2-$, $-CH(CH_3)-$, $-C(CH_3)_2-$, $-CH(C_2H_5)-$,

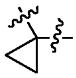,

and a chemical bond.

**19.** The compound or the isomer thereof or the pharmaceutically acceptable salt thereof according to any one of claims 1 to 18, wherein, the compound or the isomer thereof or the pharmaceutically acceptable salt thereof is selected from the group consisting of:

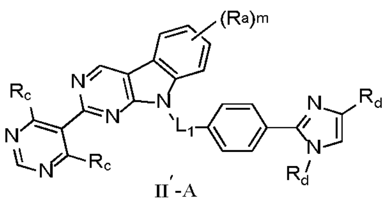
II′-A

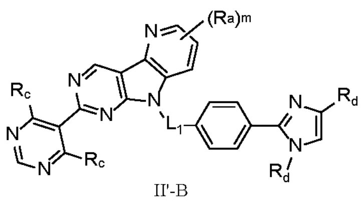
II'-B

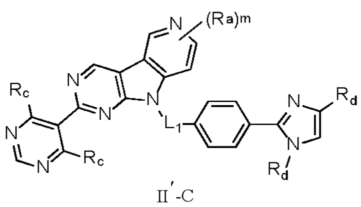
II′-C

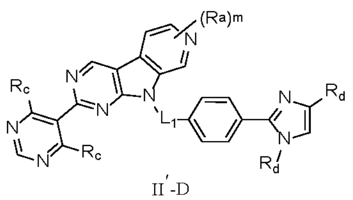
II′-D

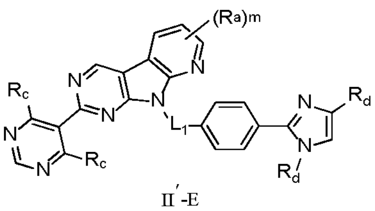
II′-E

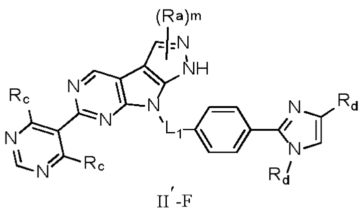
II′-F

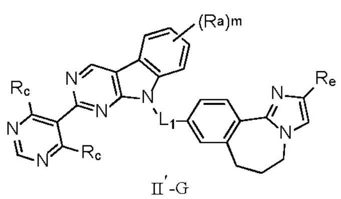
II′-G

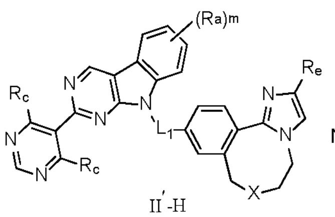
II′-H

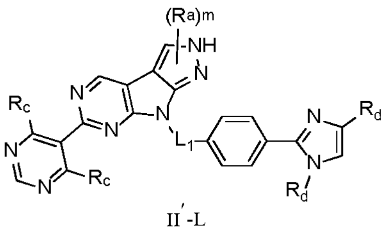
II′-L

,

and wherein $R_a$, $R_c$, $R_d$, $R_e$, $L_1$, and m are as defined in the preceding claims; and X is selected from the group consisting of C, N, and O.

**20.** The compound or the isomer thereof or the pharmaceutically acceptable salt thereof according to any one of claims 1

to 18, wherein, the compound or the isomer thereof or the pharmaceutically acceptable salt thereof is selected from the group consisting of:

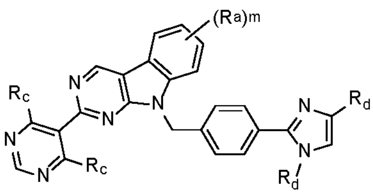

II'-1

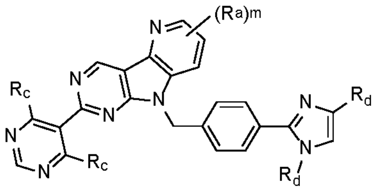

II'-2

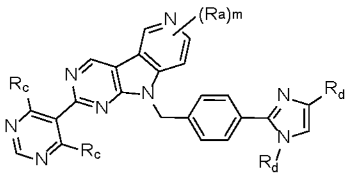

II'-3

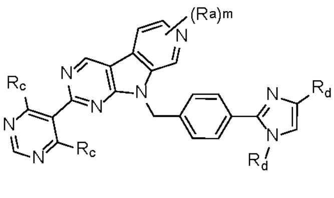

II'-4

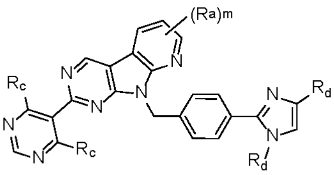

II'-5

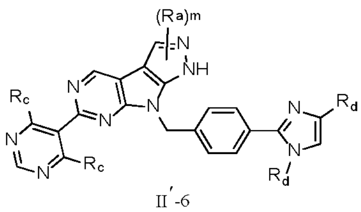

II'-6

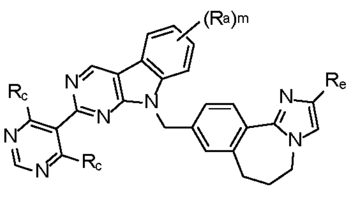

II'-7

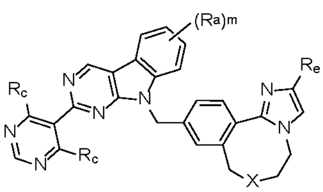

II'-8

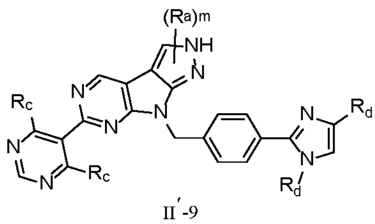

II'-9

,

and wherein $R_a$, $R_c$, $R_d$, $R_e$, and m are as defined in the preceding claims; and X is selected from the group consisting of C, N, and O.

**21.** The compound or the isomer thereof or the pharmaceutically acceptable salt thereof according to any one of claims 1 to 20, wherein, the compound or the isomer thereof or the pharmaceutically acceptable salt thereof is selected from the group consisting of:

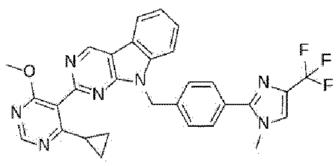
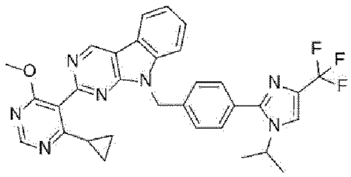
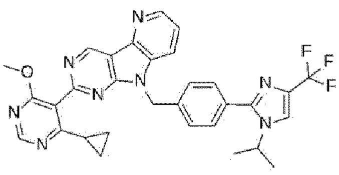
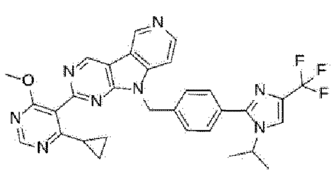
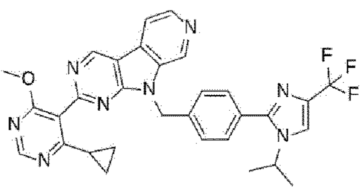
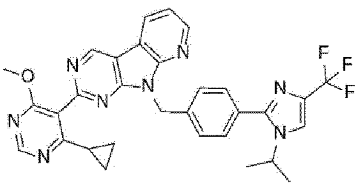
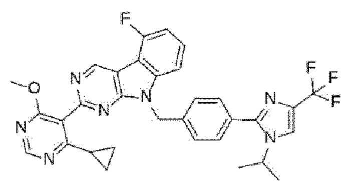
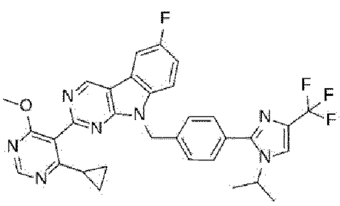
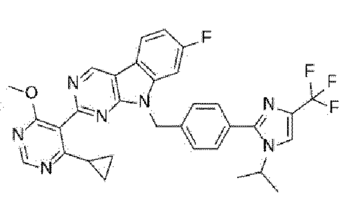

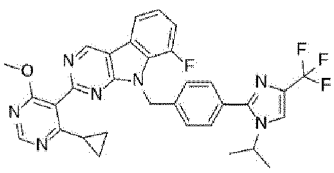 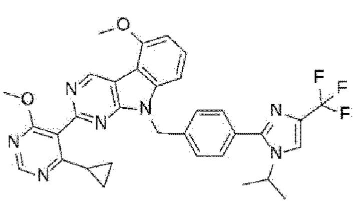 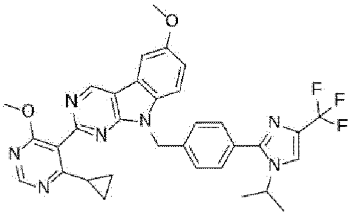

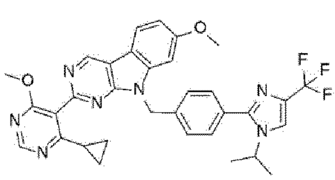 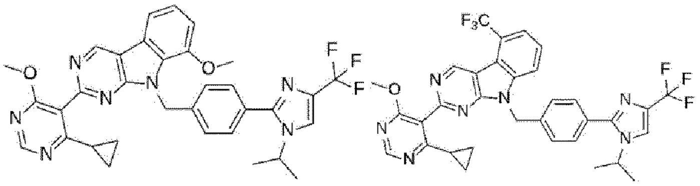

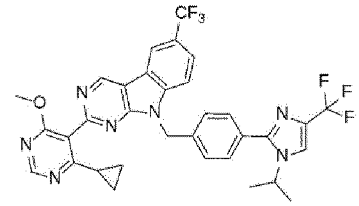 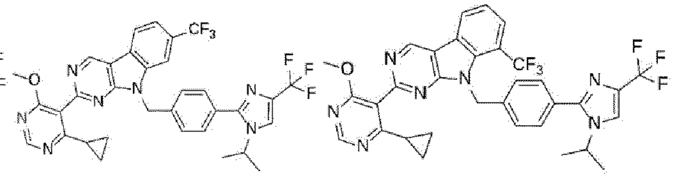

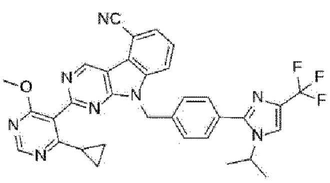 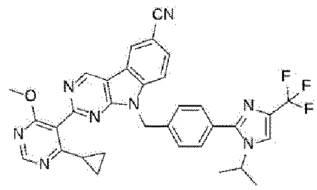 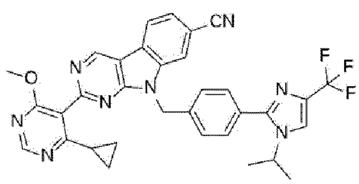

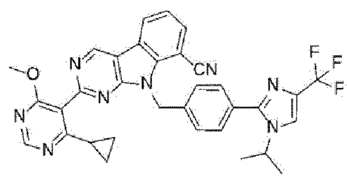 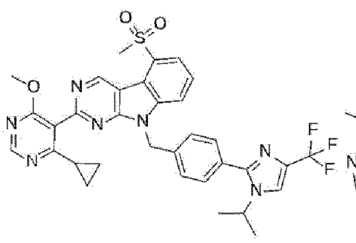 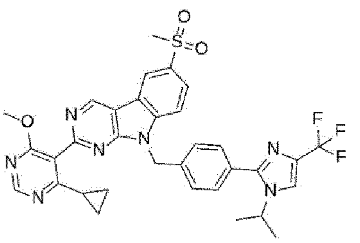

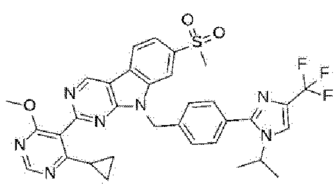 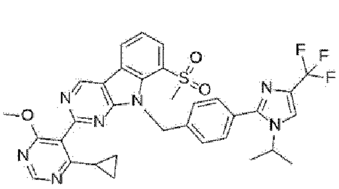 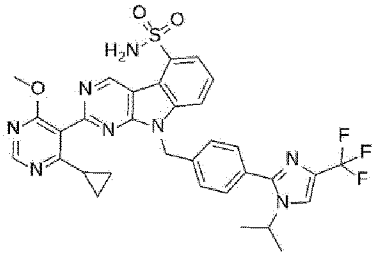

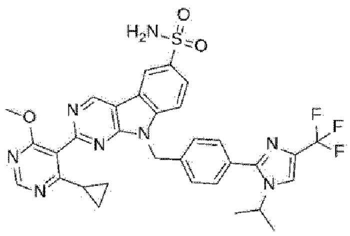 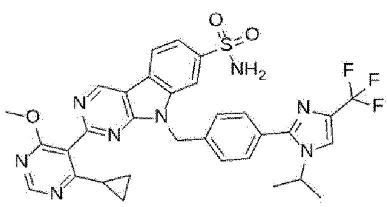 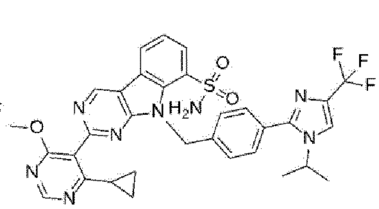

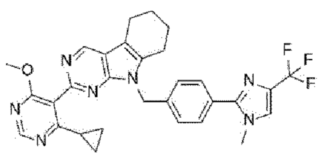
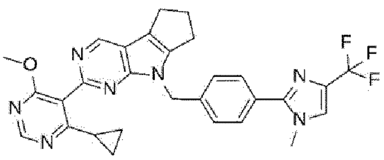
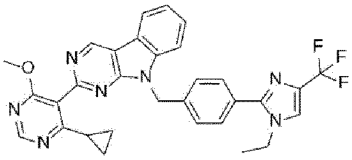

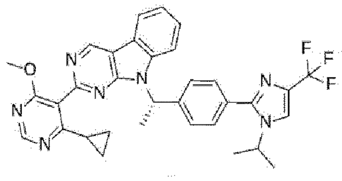
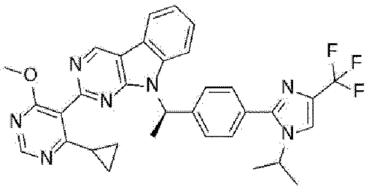
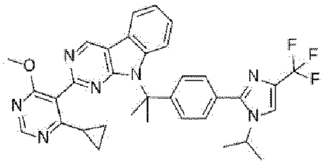

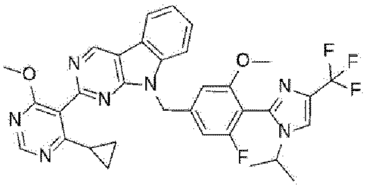
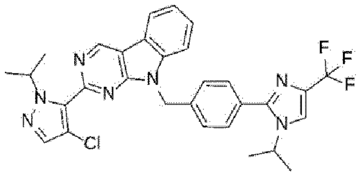
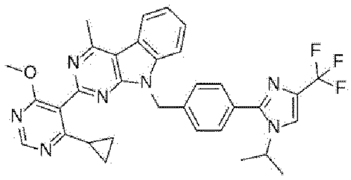

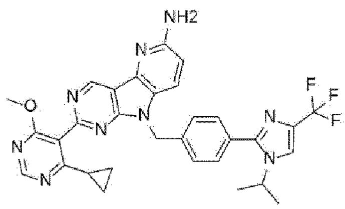
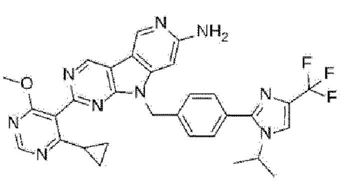
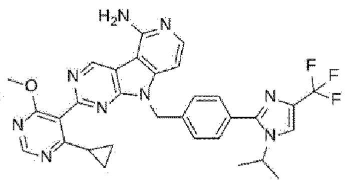

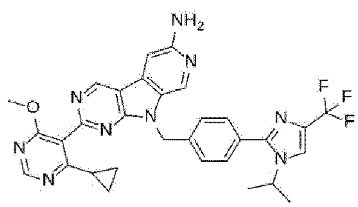
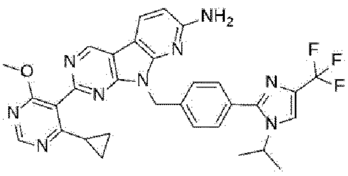
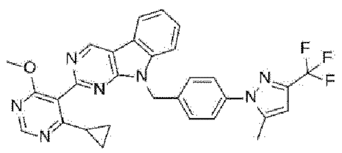

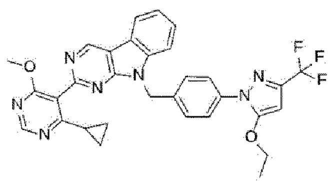
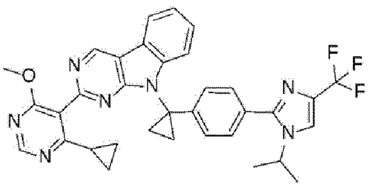
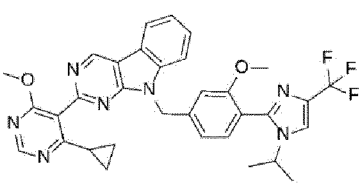

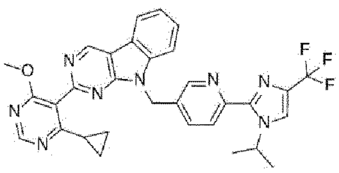
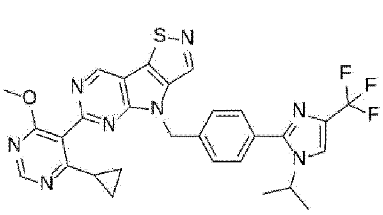
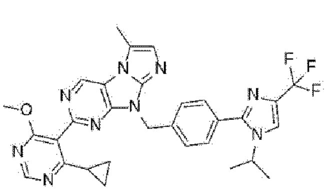

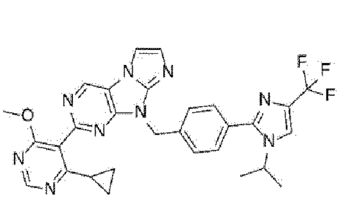
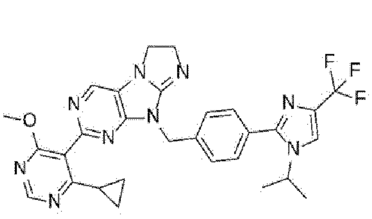
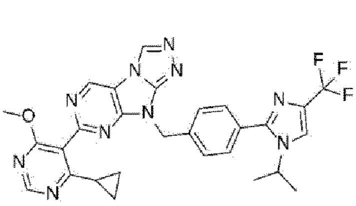

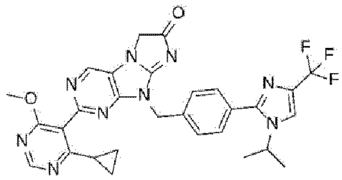
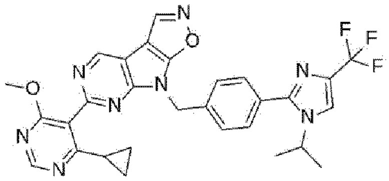
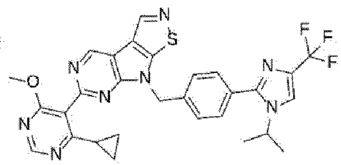

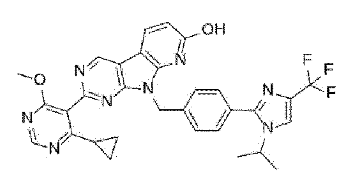
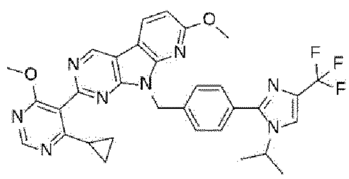
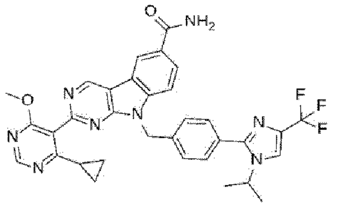

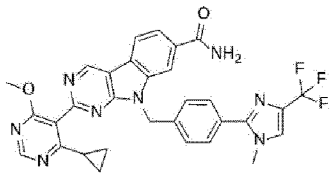
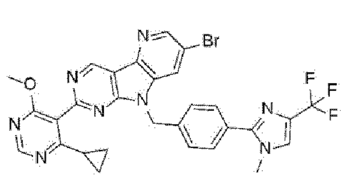
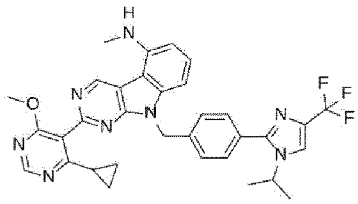

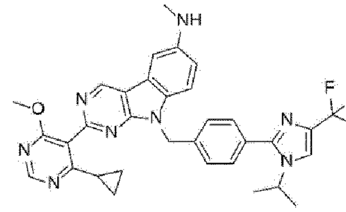
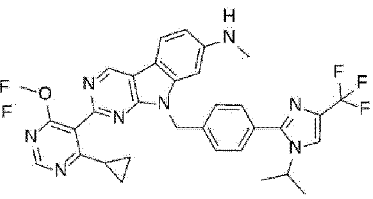
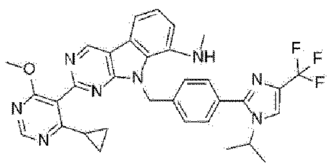

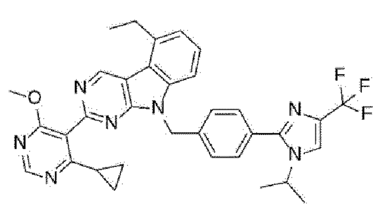
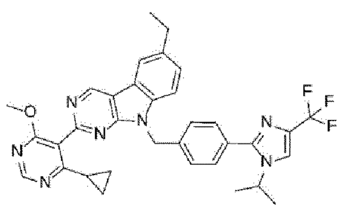
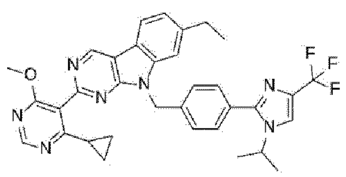

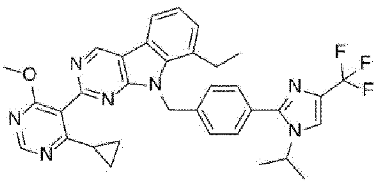
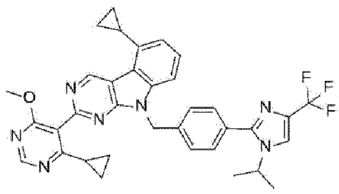
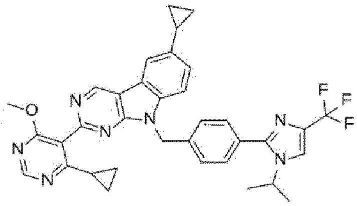

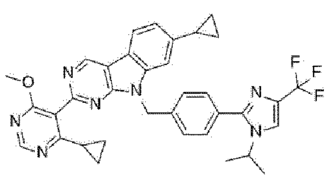
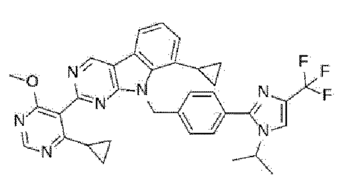
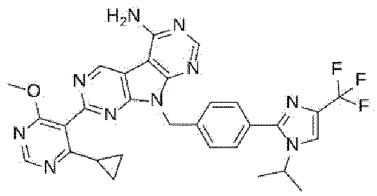

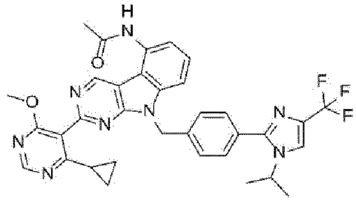
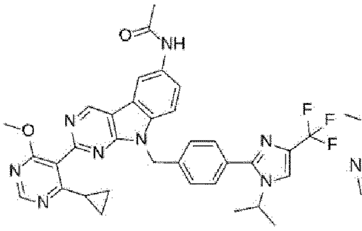
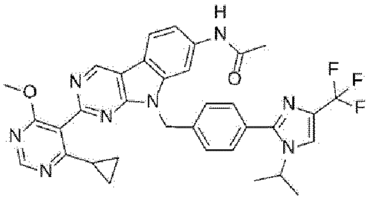

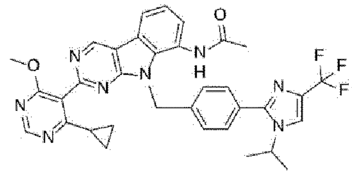
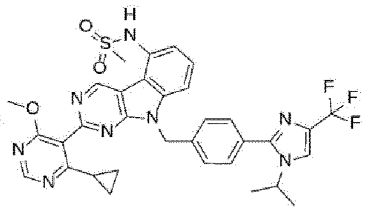
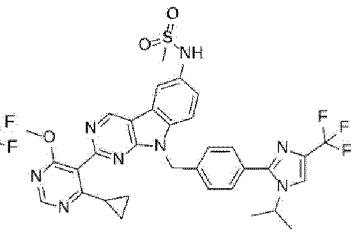

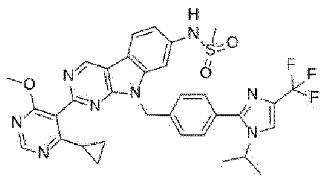
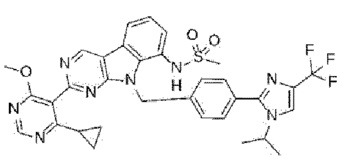
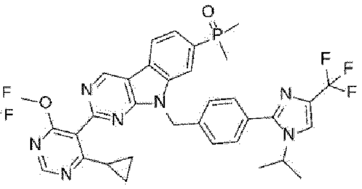

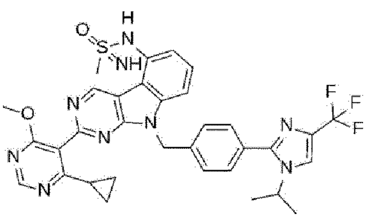
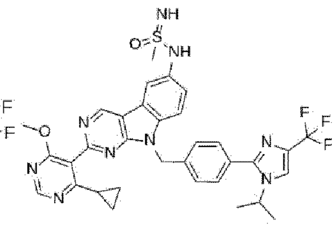
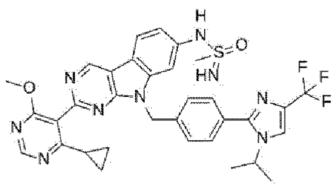

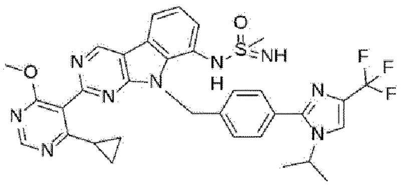
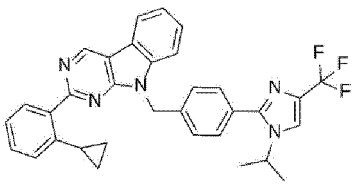
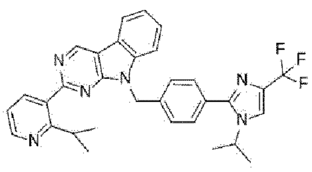

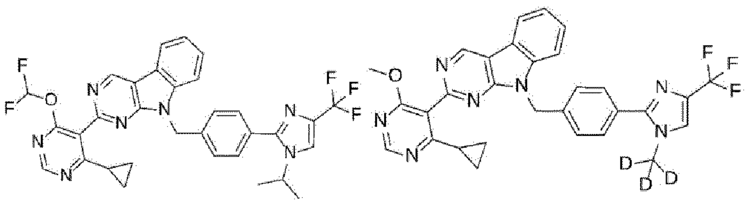
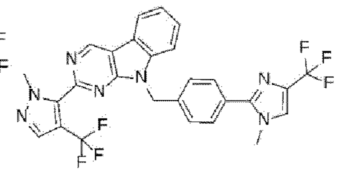

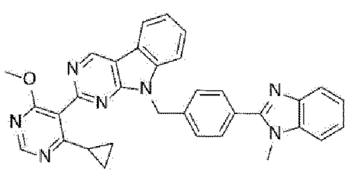
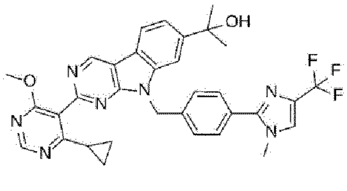
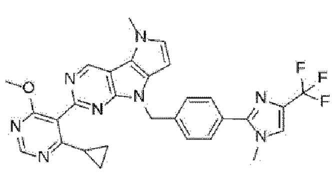

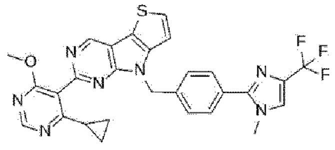
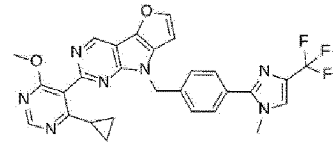
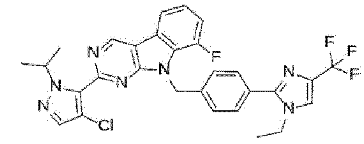

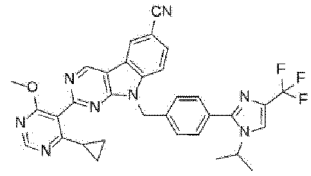
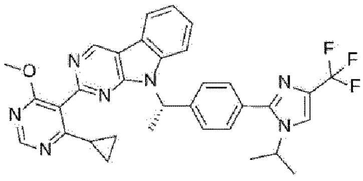
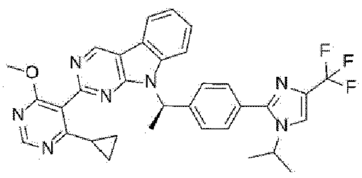

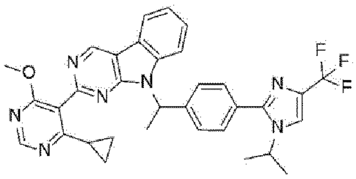
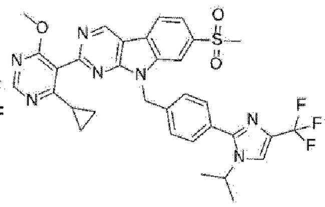
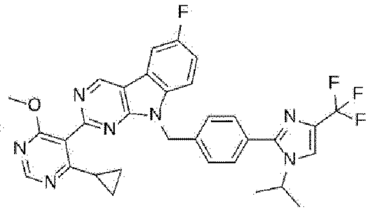

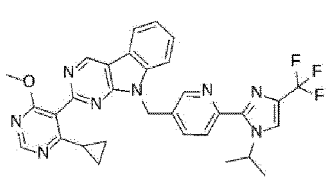
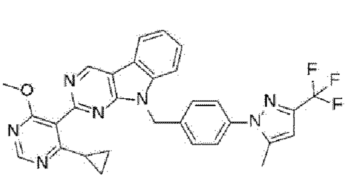
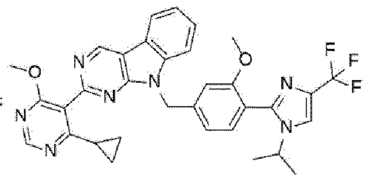

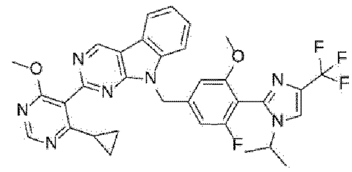
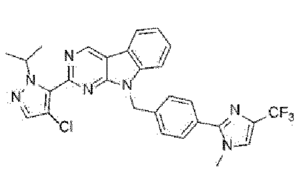
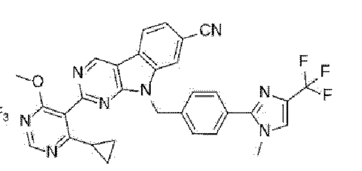

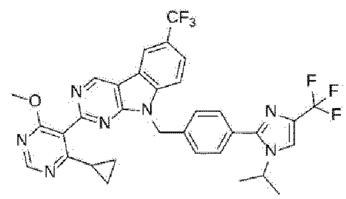
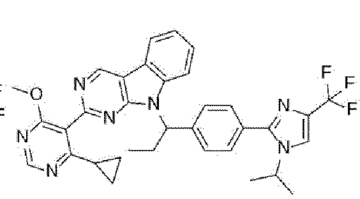
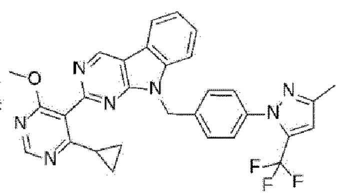

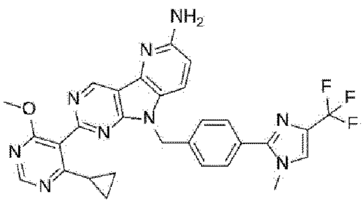
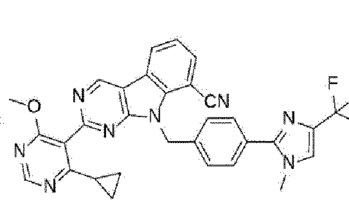
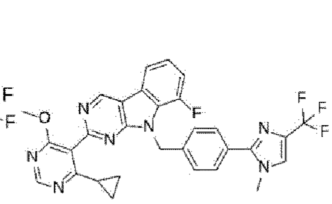

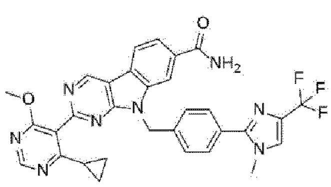
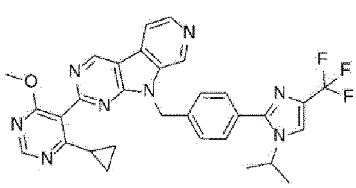
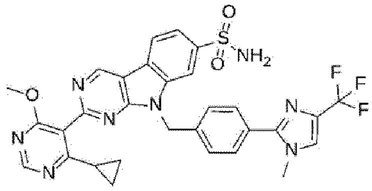

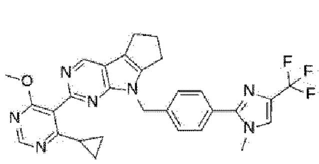
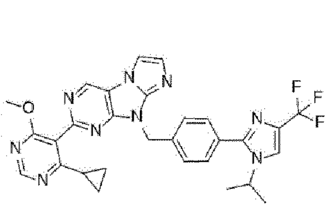
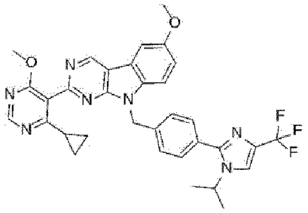

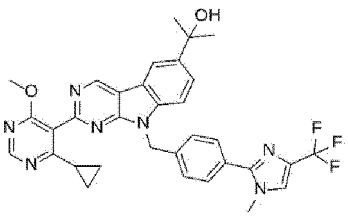
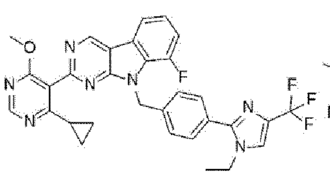
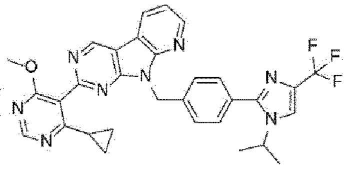

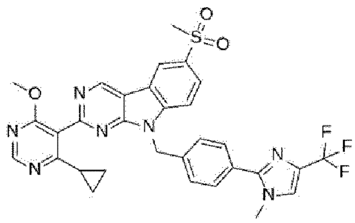
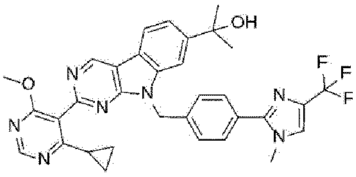
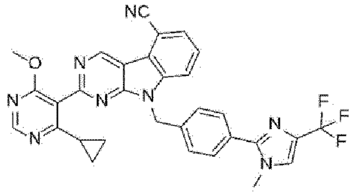

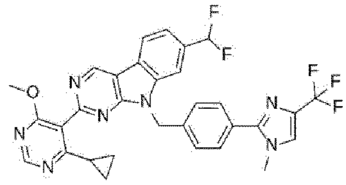
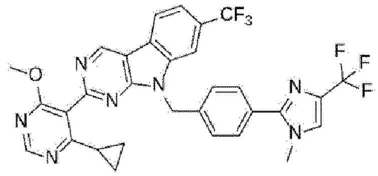
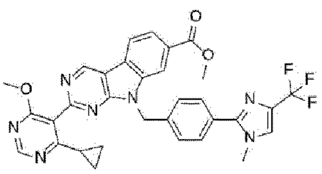

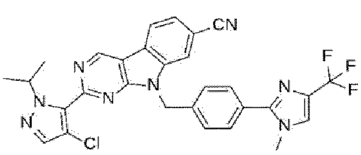
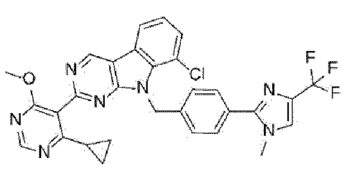
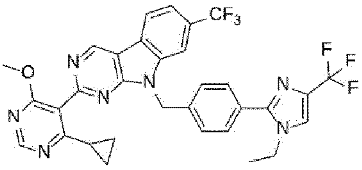

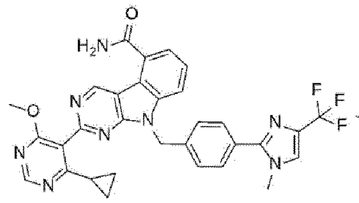
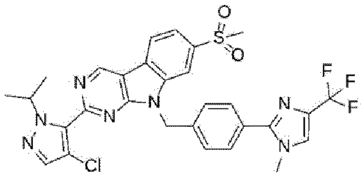
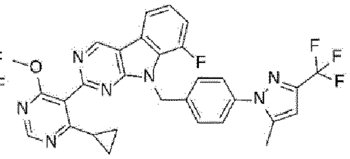

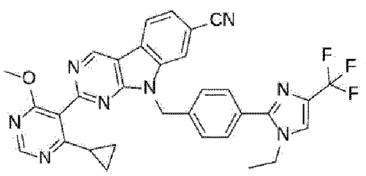
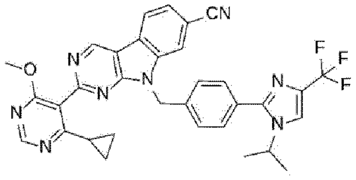
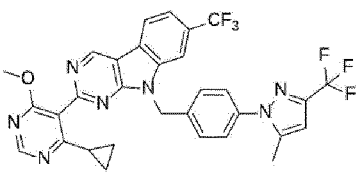

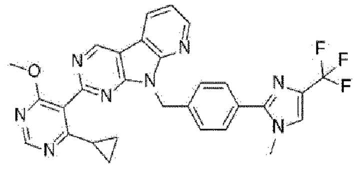
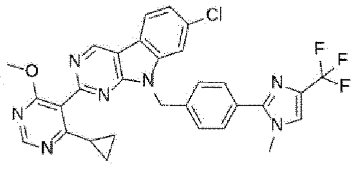
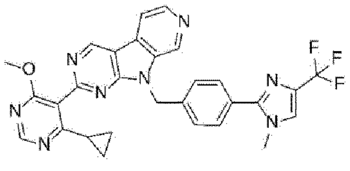

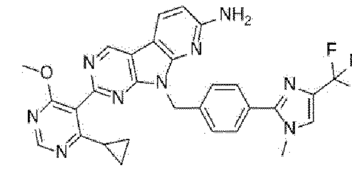
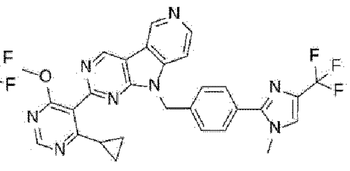
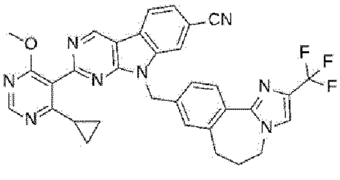

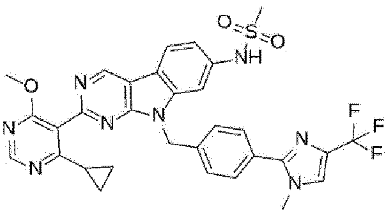
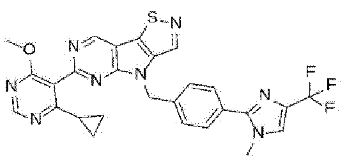
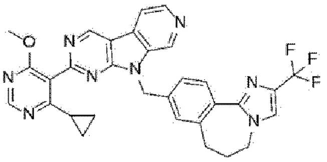

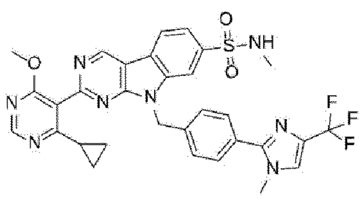
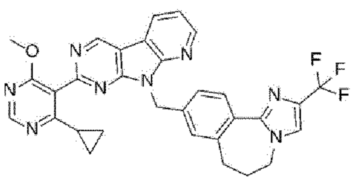
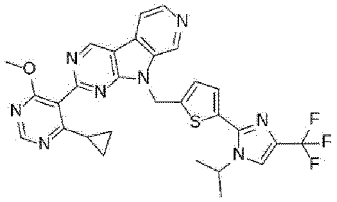

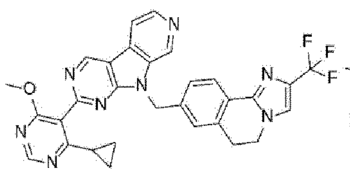
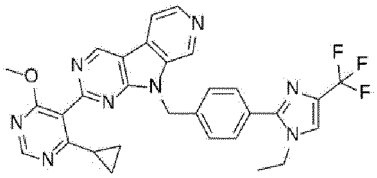
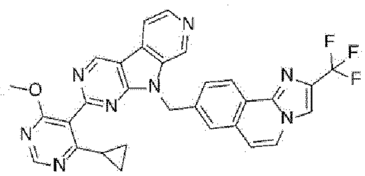

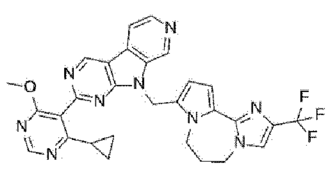
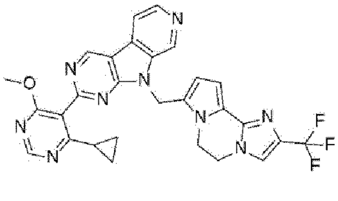
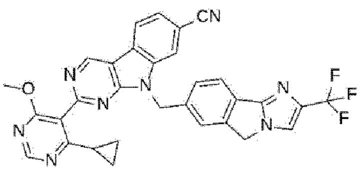

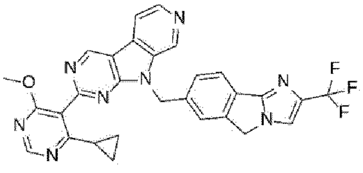
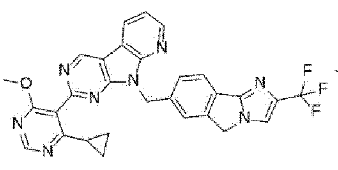
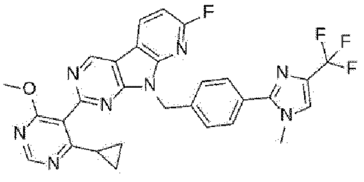

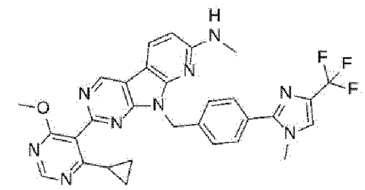
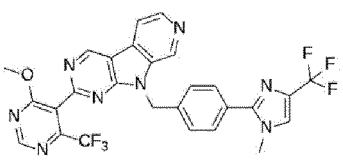
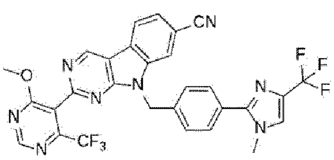

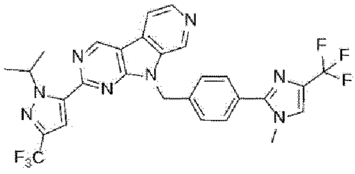
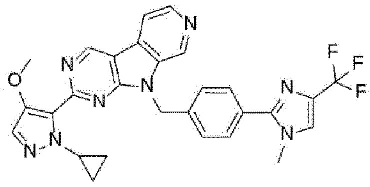
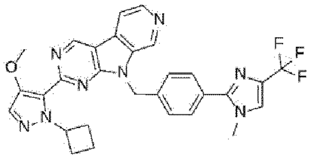

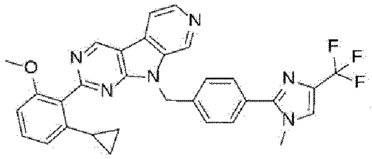
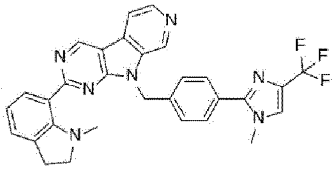
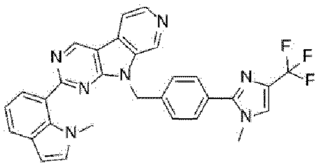

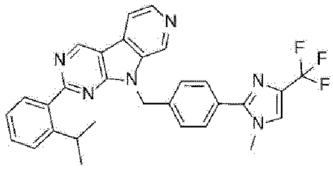
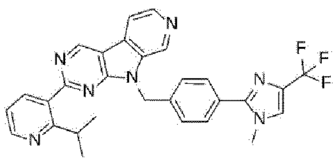
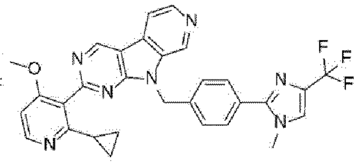

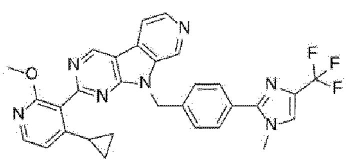
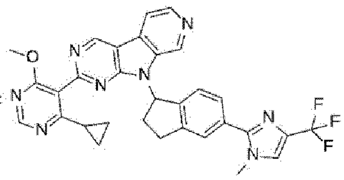
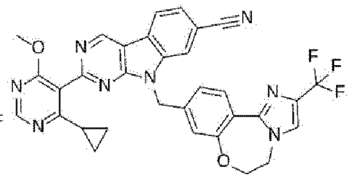

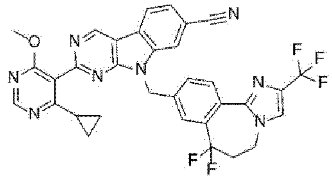
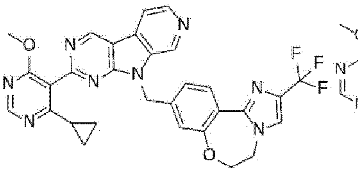
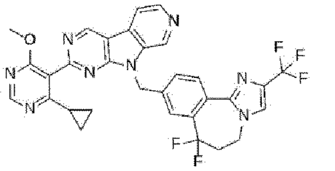

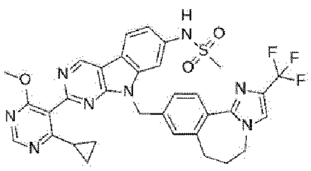
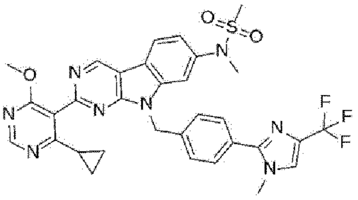
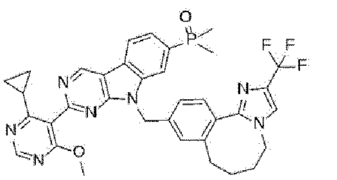

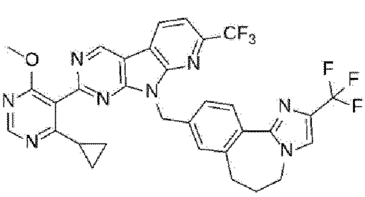
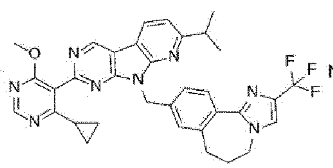
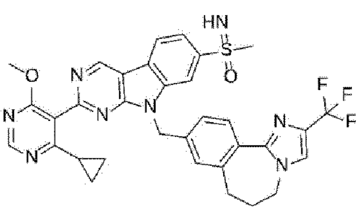

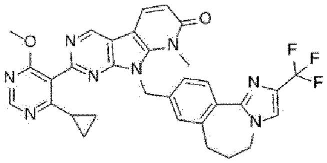
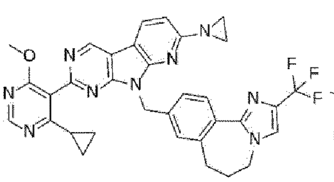
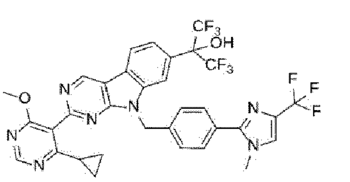

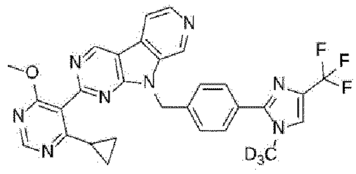
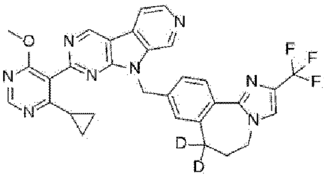
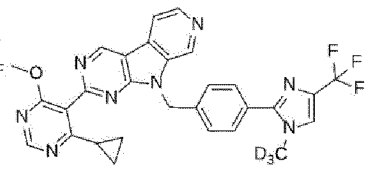

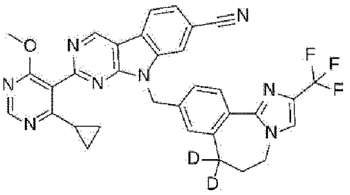
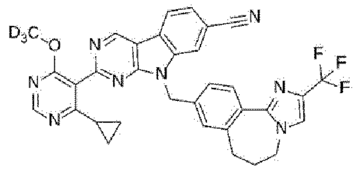
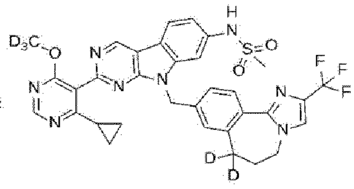

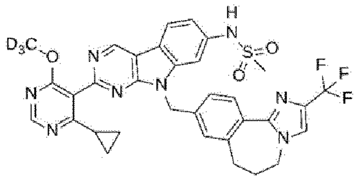
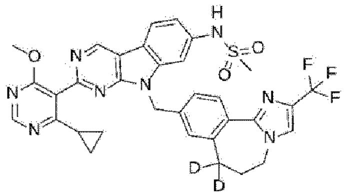
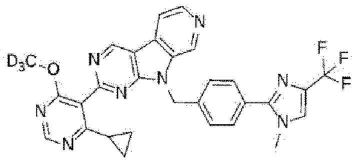

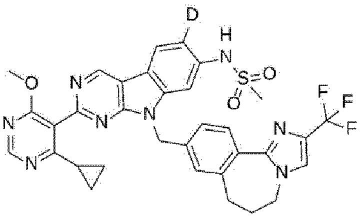
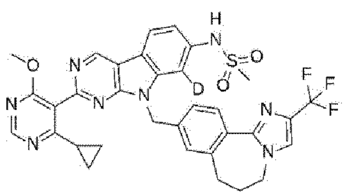
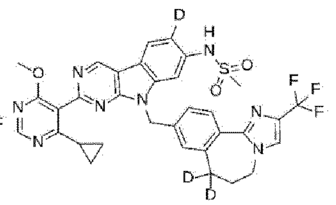

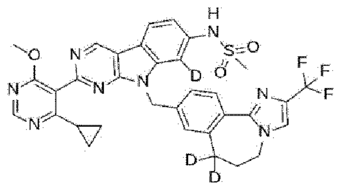
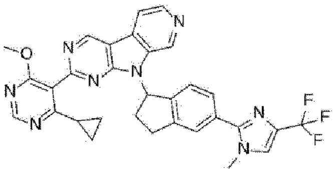
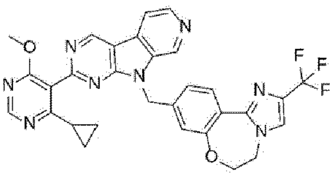

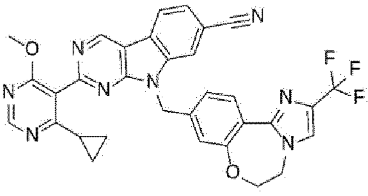
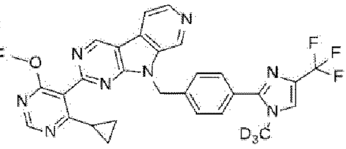
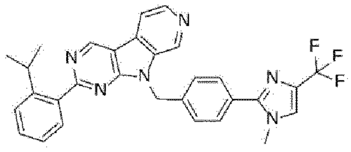

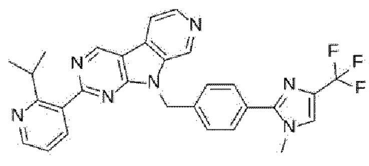
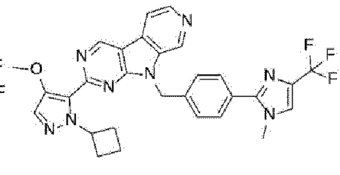
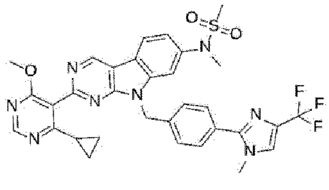

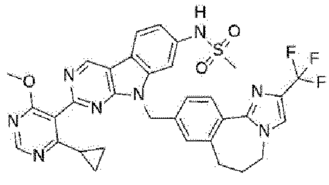
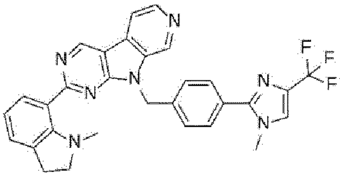
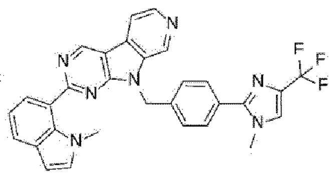

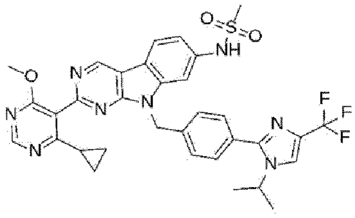
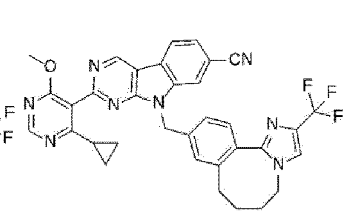
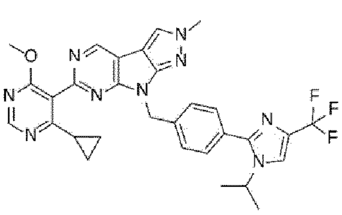

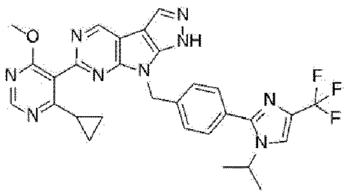
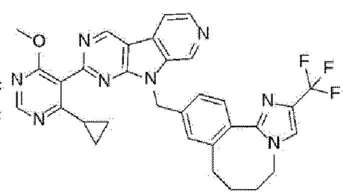
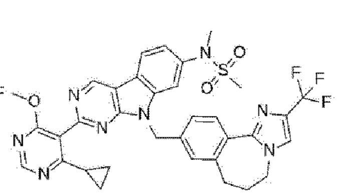

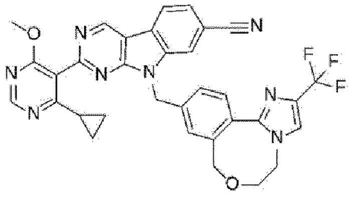
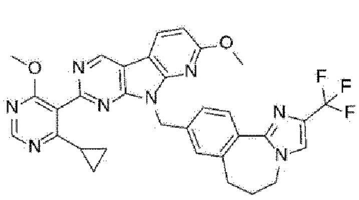
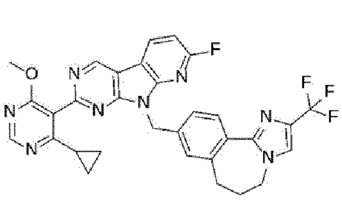

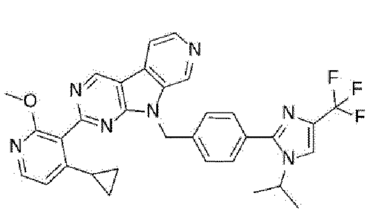
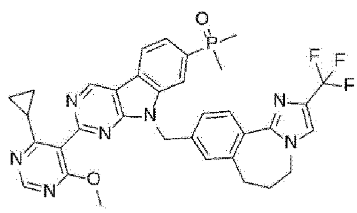
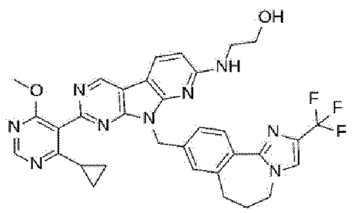

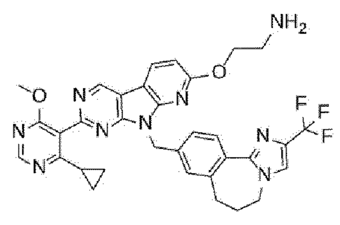
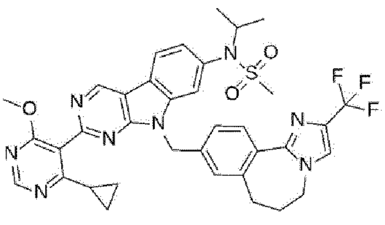
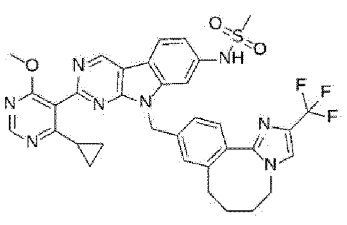

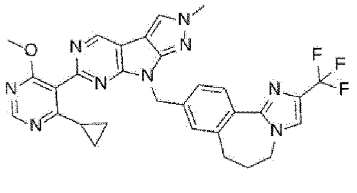
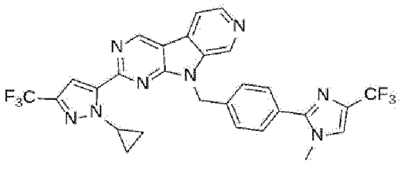
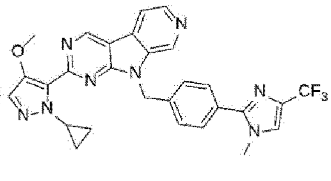

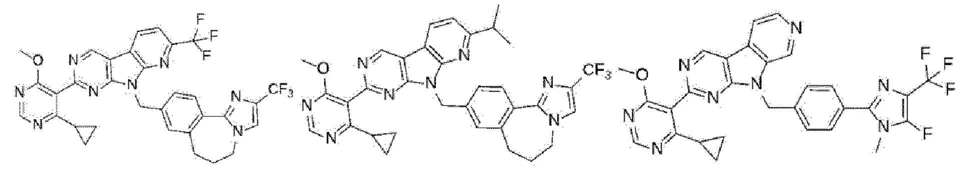

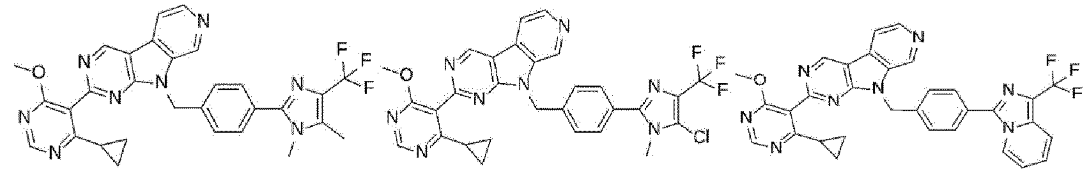

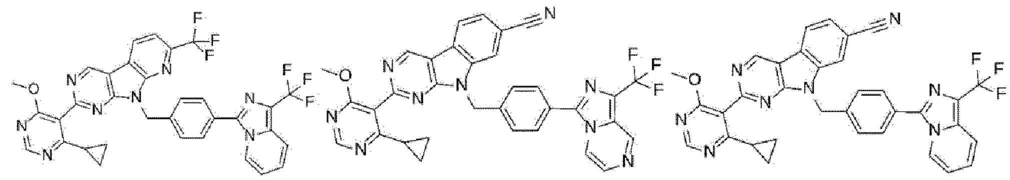

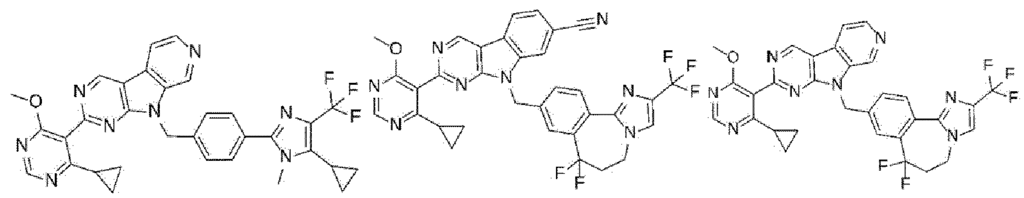

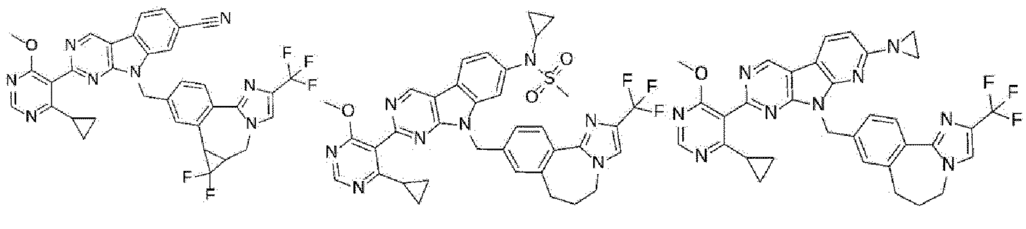

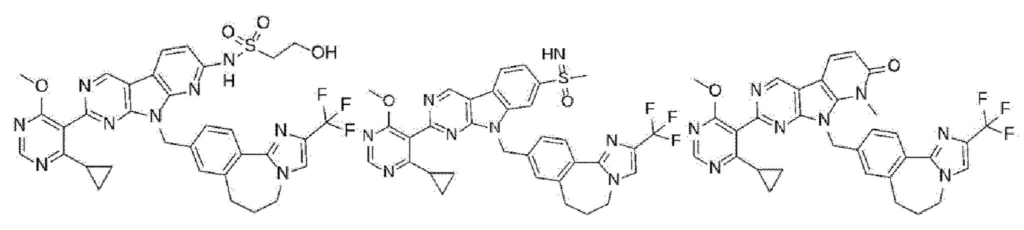

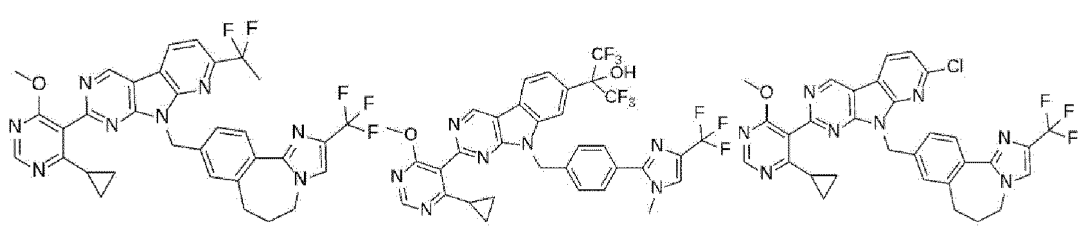

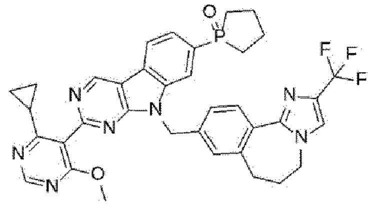
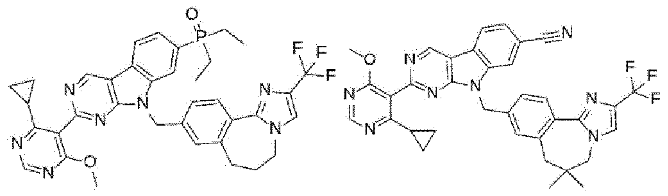

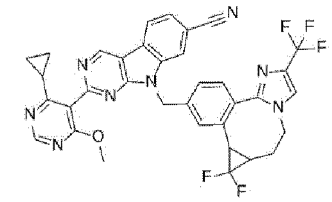
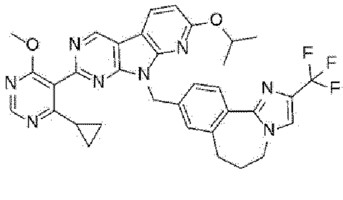
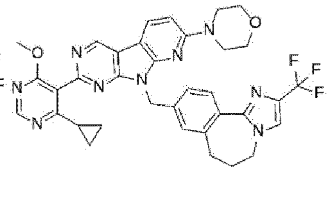

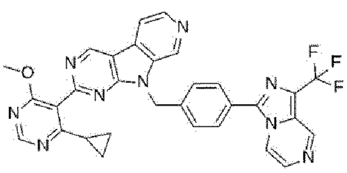
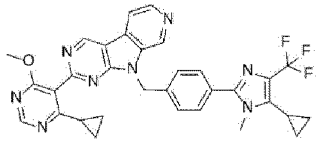
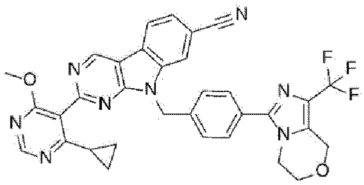

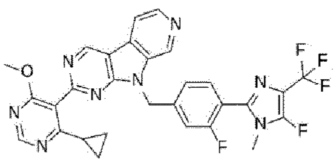
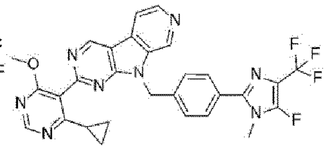
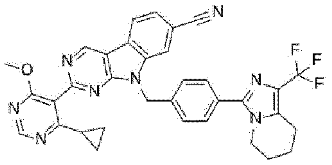

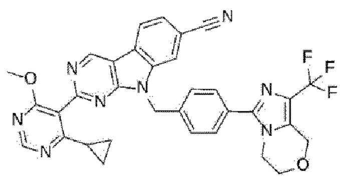
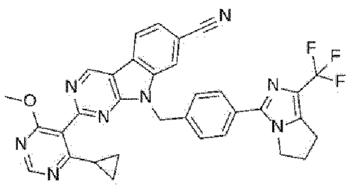
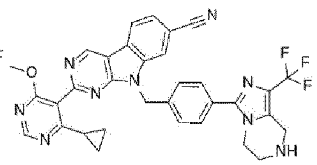

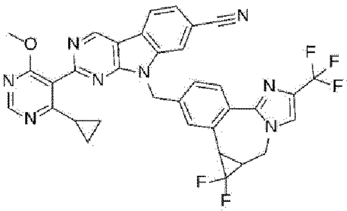
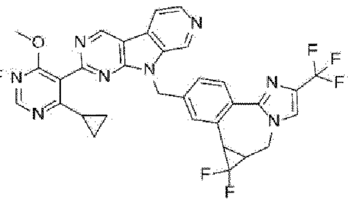
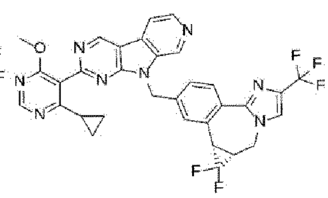

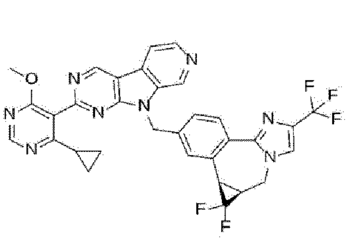
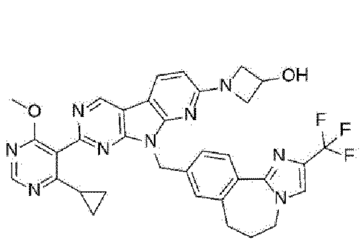
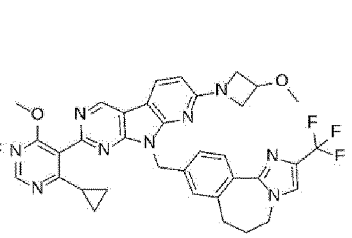

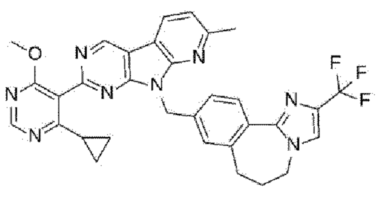
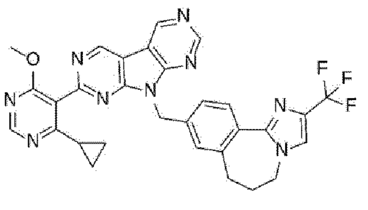
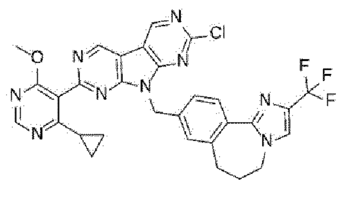

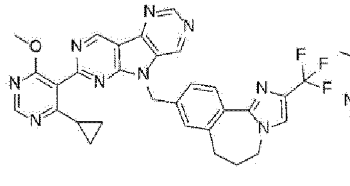
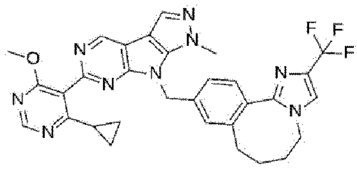
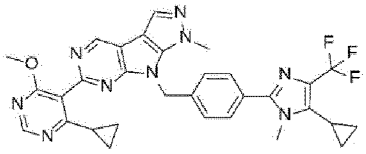

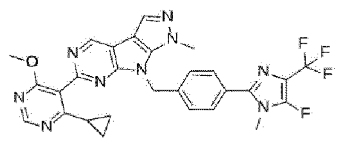
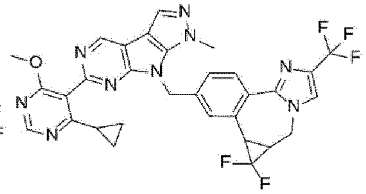
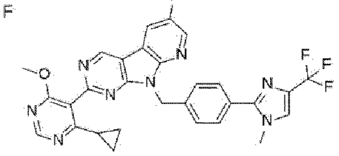

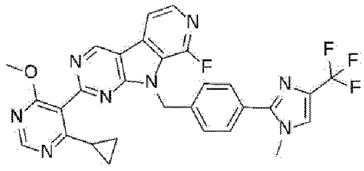
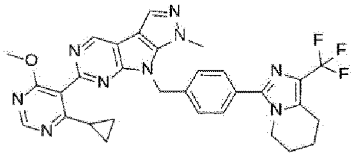
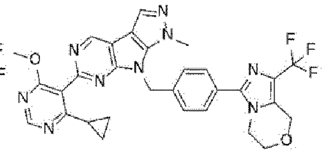

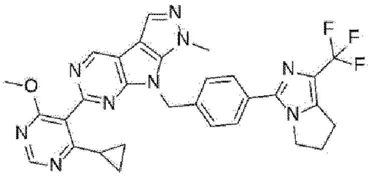
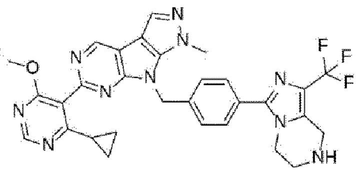
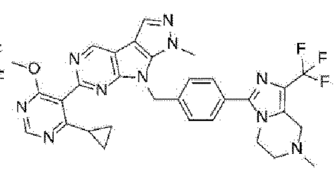

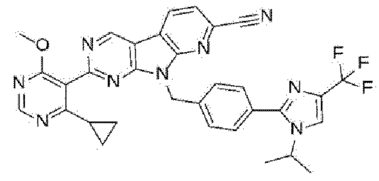
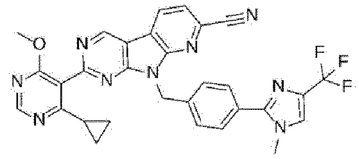
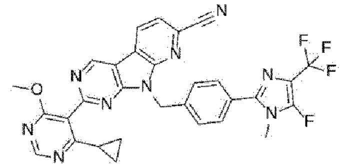

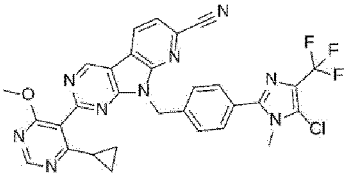
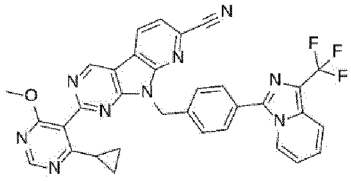
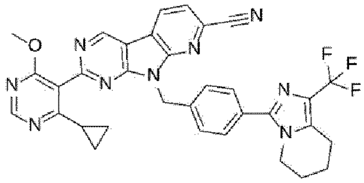

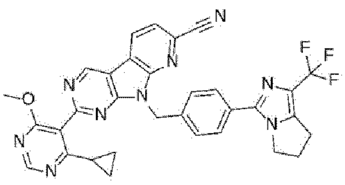
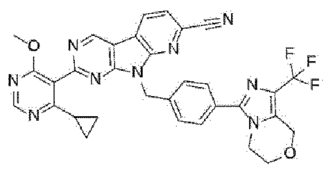
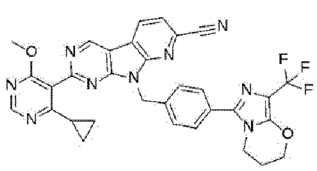

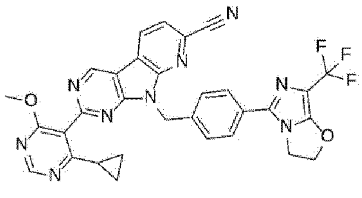
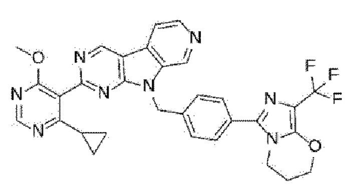
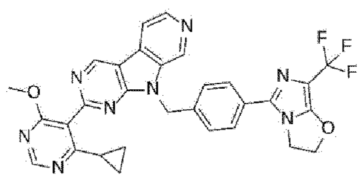

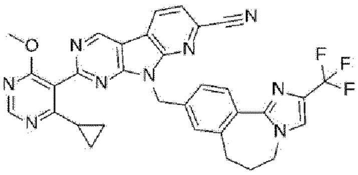
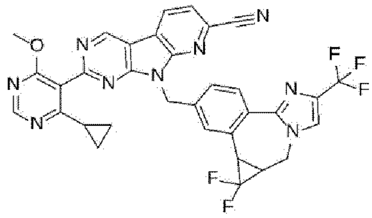
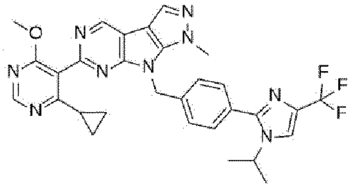

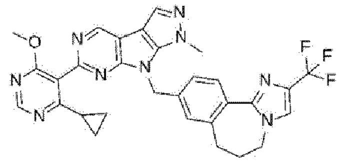
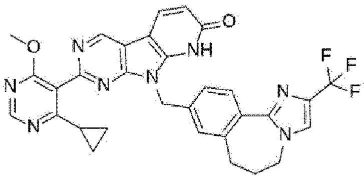
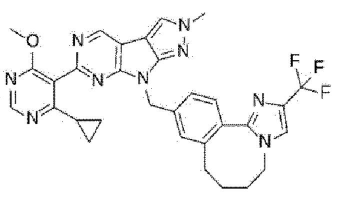

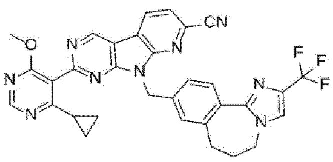
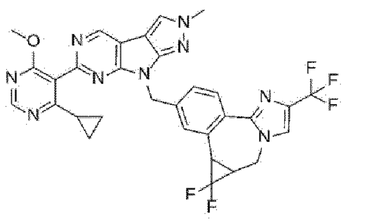
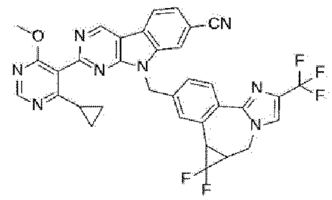

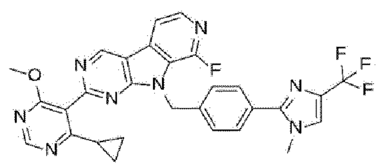
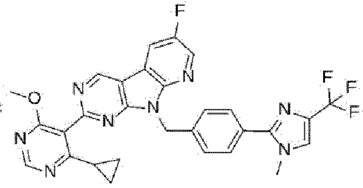
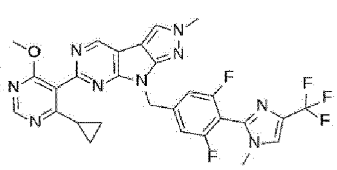

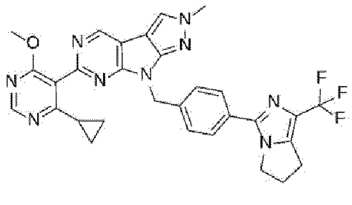
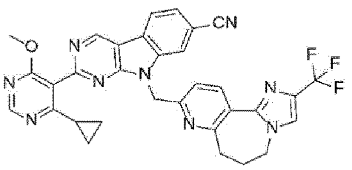
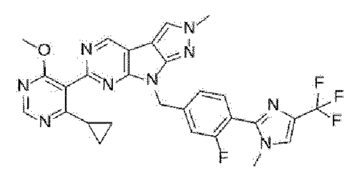

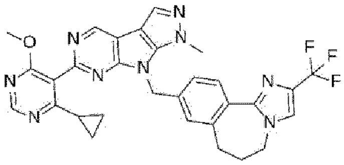
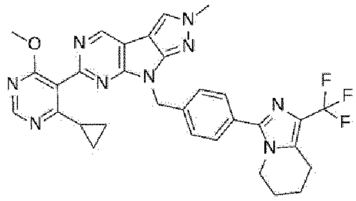
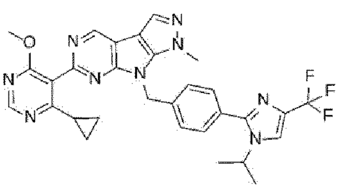

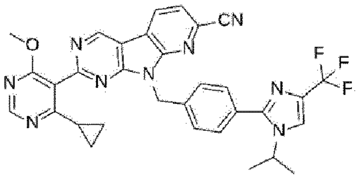
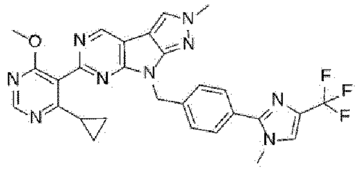
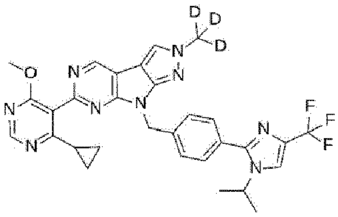

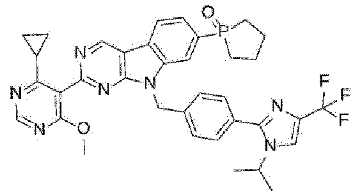
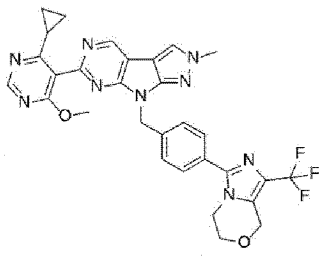
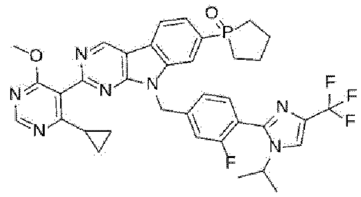

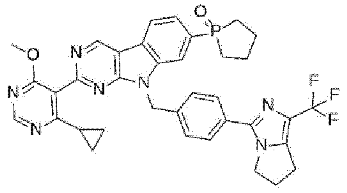
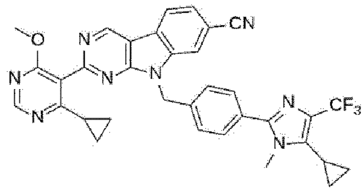
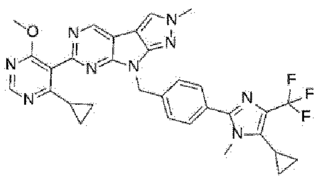

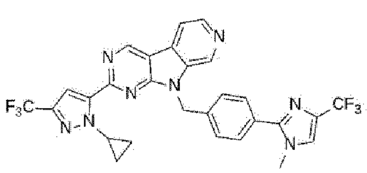
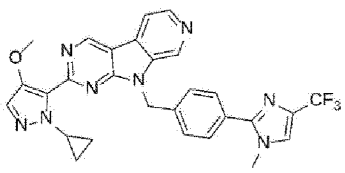
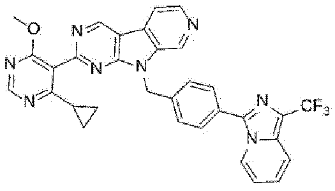

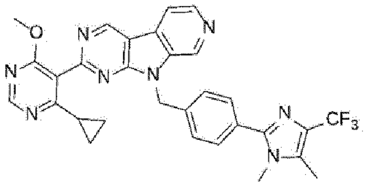
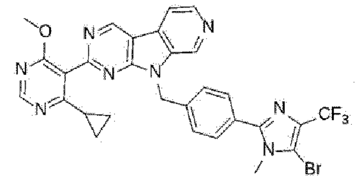
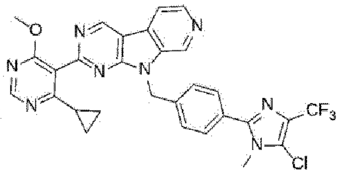

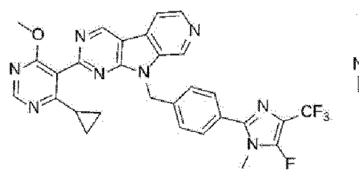
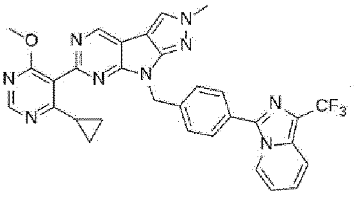
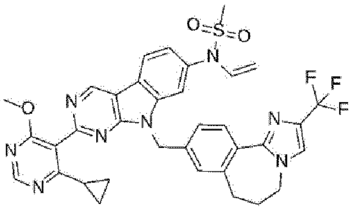

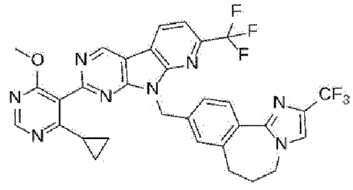
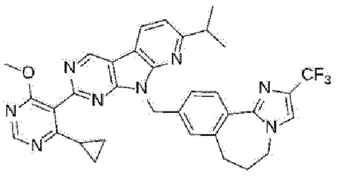
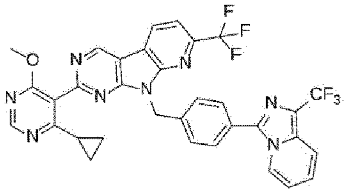

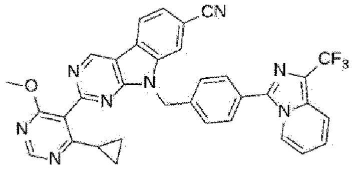
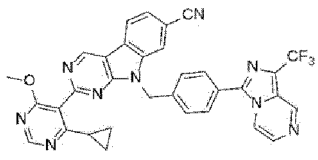
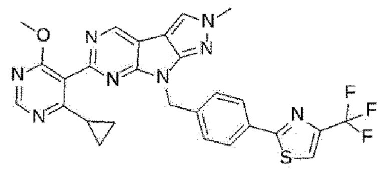

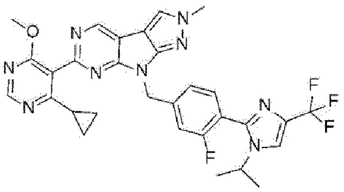
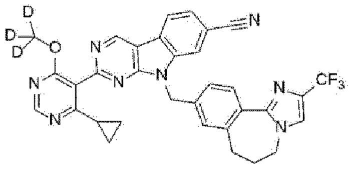
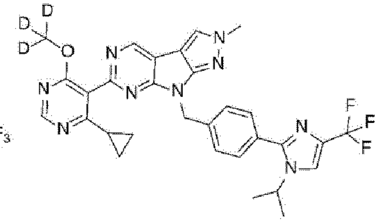

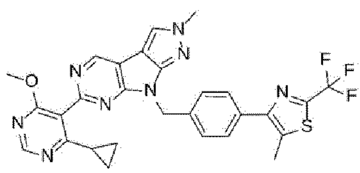
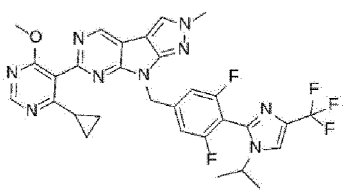
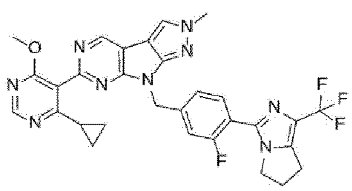

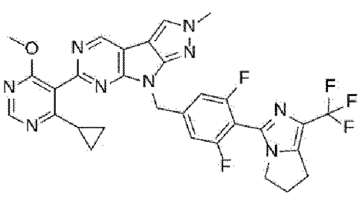
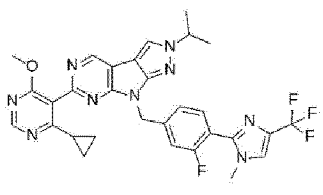
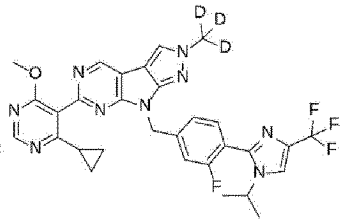

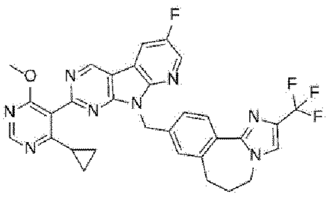
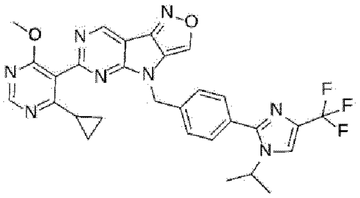
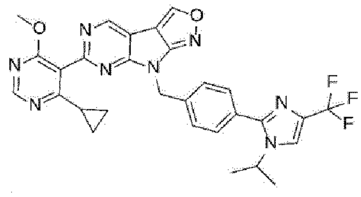

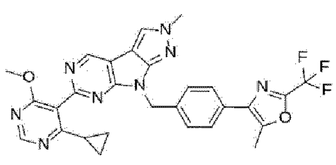
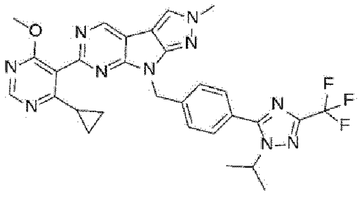
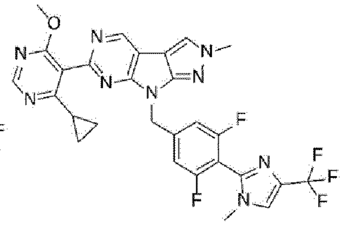

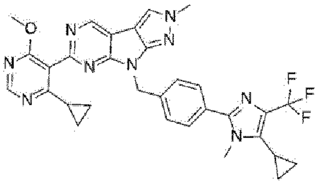
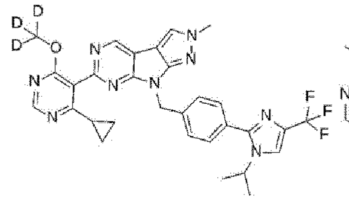
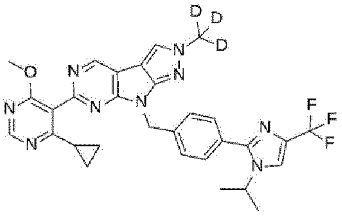

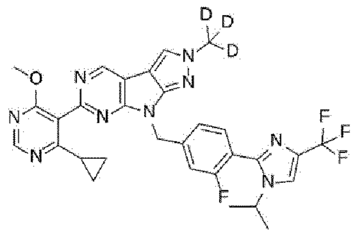
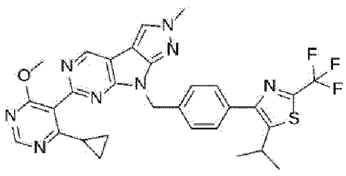
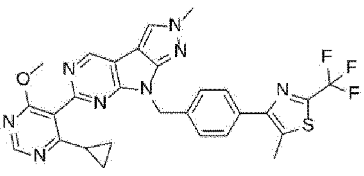

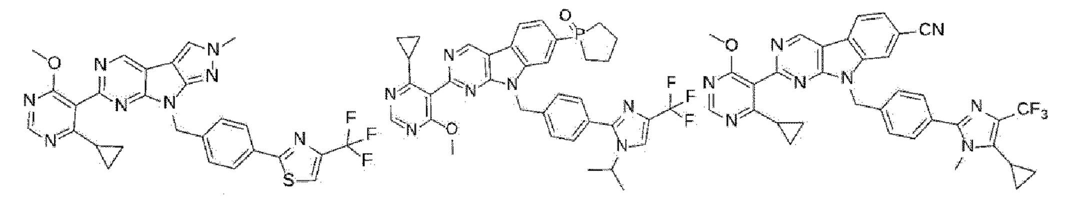

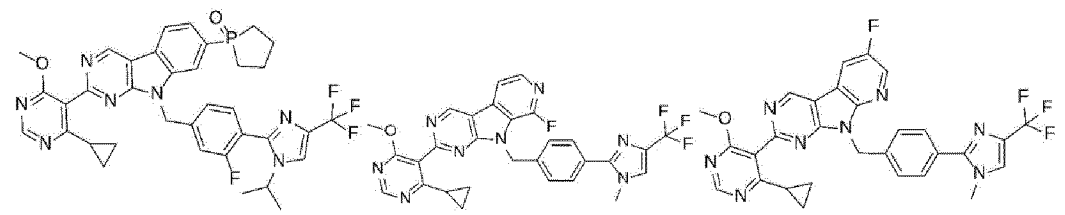

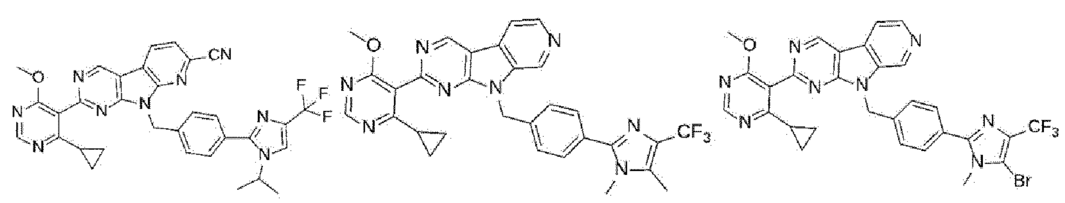

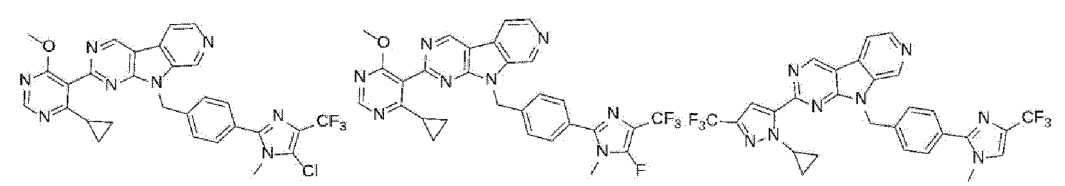

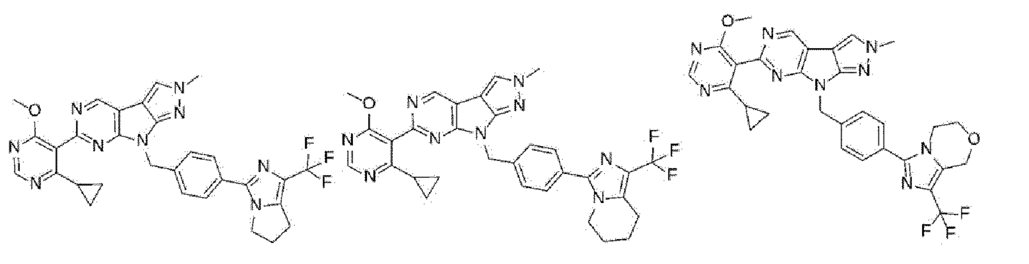

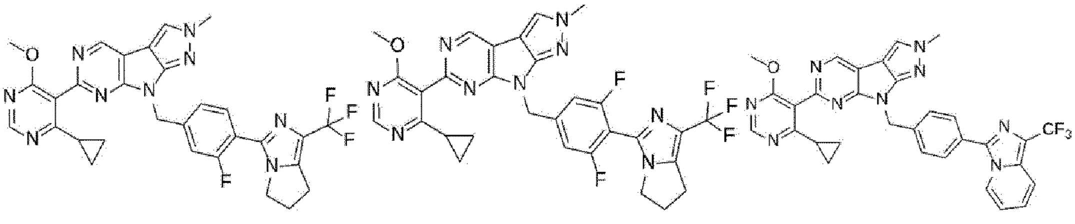

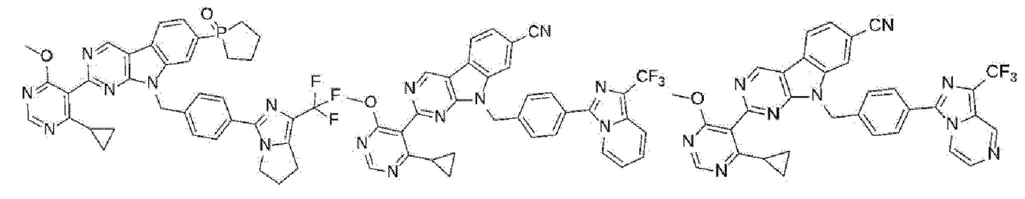

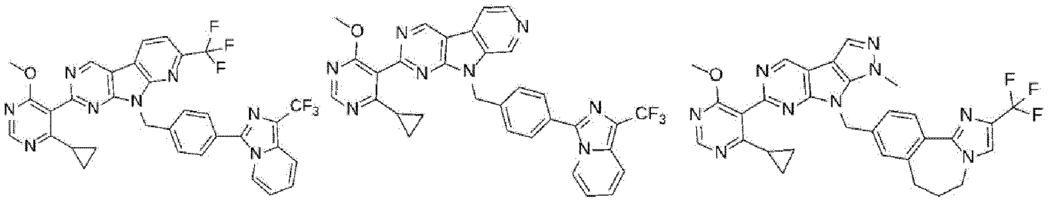

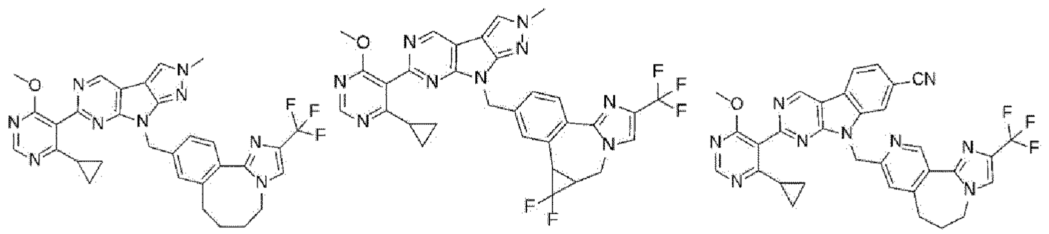

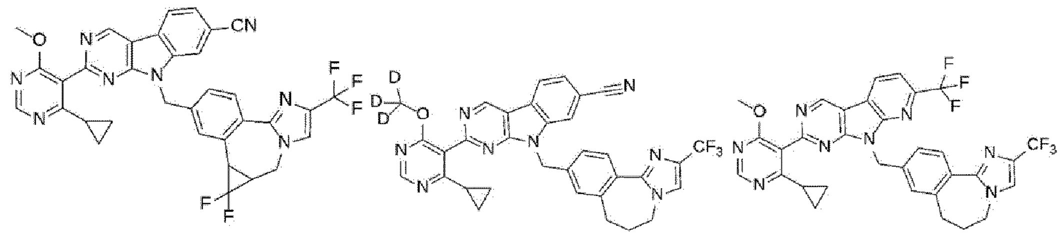

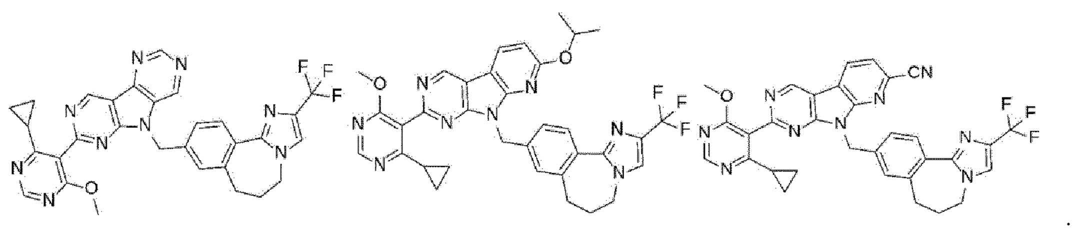

**22.** A pharmaceutical composition, comprising a therapeutically effective amount of the compound, or the isomer thereof or the pharmaceutically acceptable salt thereof according to any one of claims 1 to 21, and a pharmaceutically acceptable carrier.

**23.** Use of the compound, or the isomer thereof or the pharmaceutically acceptable salt thereof according to any one of claims 1 to 21 or the pharmaceutical composition according to claim 22 in the manufacture of a medicament for treating a USP1 target-mediated disease.

**24.** The use according to claim 23, wherein the USP1 target-mediated disease comprises a cellular inflammatory disease, a neurodegenerative disease, and cancer.

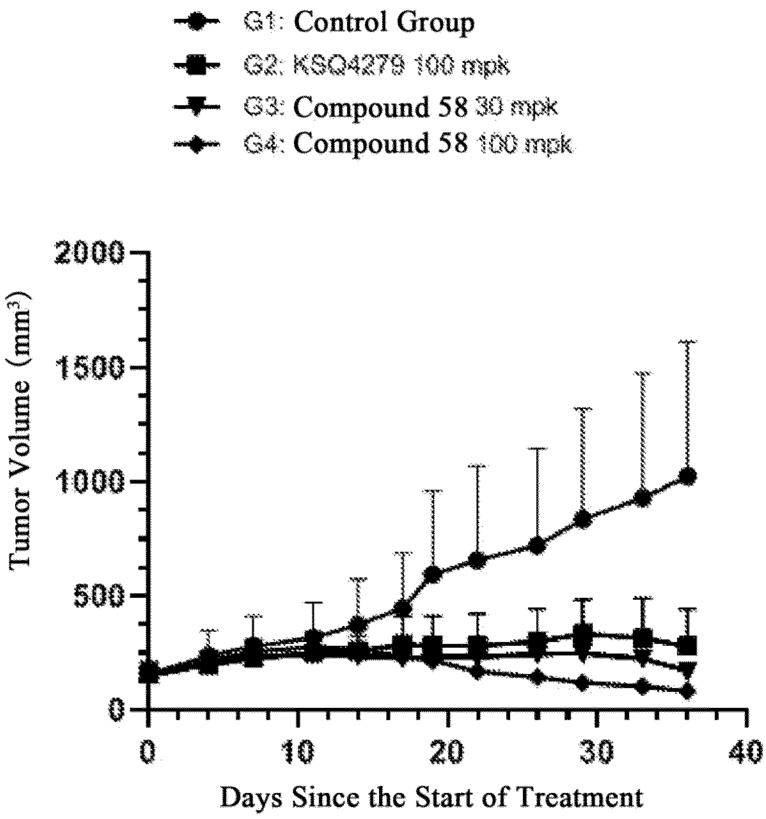
G1: Control Group
G2: KSQ4279 100 mpk
G3: Compound 58 30 mpk
G4: Compound 58 100 mpk
Tumor Volume (mm³)
2000
1500
1000
500
0
0
10
20
30
40
Days Since the Start of Treatment

FIG. 1

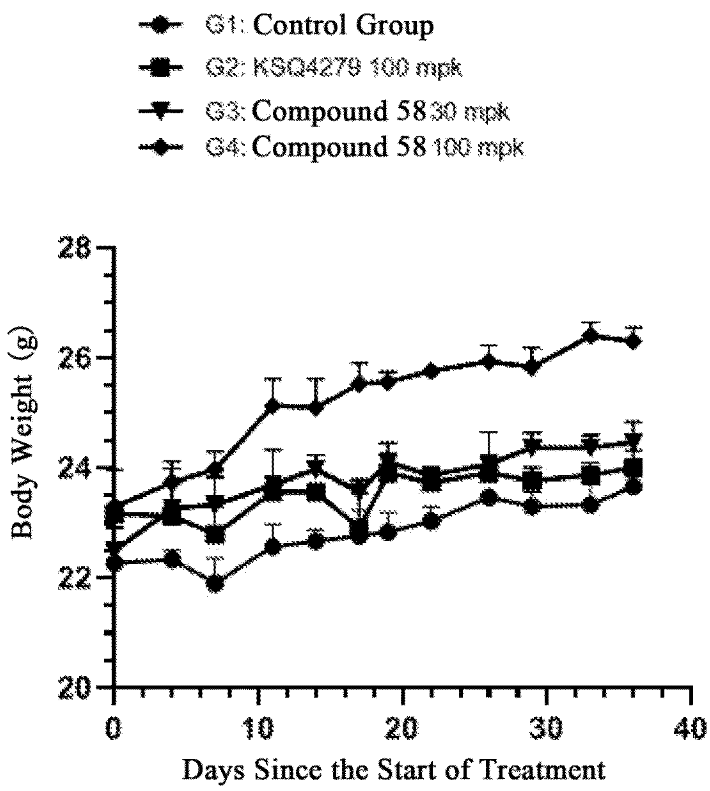
G1: Control Group
G2: KSQ4279 100 mpk
G3: Compound 58 30 mpk
G4: Compound 58 100 mpk
Body Weight (g)
28
26
24
22
20
0
10
20
30
40
Days Since the Start of Treatment

FIG. 2

**INTERNATIONAL SEARCH REPORT**

International application No.
**PCT/CN2023/116528**

**A. CLASSIFICATION OF SUBJECT MATTER**

C07D487/04(2006.01)i； C07D513/14(2006.01)i； C07D519/00(2006.01)i； A61P35/00(2006.01)i； A61K31/519(2006.01)i； A61K31/52(2006.01)i； A61K45/06(2006.01)i

According to International Patent Classification (IPC) or to both national classification and IPC

**B. FIELDS SEARCHED**

Minimum documentation searched (classification system followed by classification symbols)

IPC:C07D A61P A61K

Documentation searched other than minimum documentation to the extent that such documents are included in the fields searched

Electronic data base consulted during the international search (name of data base and, where practicable, search terms used)

CNABS; WPABS; CNTXT; CJFD; VEN; WOTXT; USTXT; EPTXT; CNKI; 万方, WANFANG; 超星读秀, DUXIU; ISI-Web of Science; STN-registry; STN-caplus: 齐鲁制药, 覃华, 石谷沁, 张宇星, 徐一鸣, 郝俊国, 陈昫, 冯昊, 祝盼虎, 付家胜, 孙维梅, 孙大庆, 泛素特异性蛋白酶, 三并环, 癌症, 肿瘤, 炎症, 神经退行, USP1, +tricyclic+, Cancer, Tumor, Inflammation, Neurodegenerat+, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2022228399 A1 (SHANDONG XUANZHUPHARMA CO., LTD.) 03 November 2022 (2022-11-03)<br>claims 1 and 10-13 | 1-24 |
| A | CN 113164485 A (KSQ THERAPEUTICS, INC.) 23 July 2021 (2021-07-23)<br>claims 1, 25-27, and 30-34 | 1-24 |
| A | WO 2022174184 A1 (TANGO THERAPEUTICS, INC.) 18 August 2022 (2022-08-18)<br>entire document | 1-24 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 November 2023** | **21 November 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/116528**

| Patent document cited in search report | Publication date (day/month/year) | Patent family member(s) | Publication date (day/month/year) |
|---|---|---|---|
| WO 2022228399 A1 | 03 November 2022 | None | |
| CN 113164485 A | 23 July 2021 | EP 3897652 A1 | 27 October 2021 |
| | | EP 3897652 A4 | 14 September 2022 |
| | | JP 2022511515 A | 31 January 2022 |
| | | KR 20210105887 A | 27 August 2021 |
| | | WO 2020132269 A1 | 25 June 2020 |
| | | CA 3122108 A1 | 25 June 2020 |
| | | US 2021115049 A1 | 22 April 2021 |
| | | US 11485736 B2 | 01 November 2022 |
| | | US 2023203046 A1 | 29 June 2023 |
| | | TW 202039501 A | 01 November 2020 |
| | | AU 2019405925 A1 | 29 July 2021 |
| | | SG 11202106232 A1 | 29 July 2021 |
| | | AR 117445 A1 | 04 August 2021 |
| | | BR 112021010715 A2 | 16 November 2021 |
| | | VN 81857 A | 25 November 2021 |
| | | IN 202117030155 A | 10 December 2021 |
| | | HK 40063629 A0 | 24 June 2022 |
| | | RU 2021114721 A | 06 April 2023 |
| WO 2022174184 A1 | 18 August 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211069731
- CN 202211272140
- CN 202310084635
- CN 202310416682
- CN 202310755391
- CN 202311082622